# FASCICULE DE BREVET EUROPEEN

(11) **EP 3 519 823 B1**
(45) Date de publication et mention de la délivrance du brevet: **12.05.2021**
(21) Numéro de dépôt: 17787640.6
(22) Date de dépôt: 30.09.2017
(51) Int. Cl.: A61P 31/12, A61P 31/18, A61P 37/04, A61P 43/00, G01N 33/569, C12N 5/0783

(54) **MARQUEURS MEMBRANAIRES**
MEMBRANMARKER
MEMBRANE MARKERS

(30) Priorité: 30.09.2016 FR 1659440; 15.03.2017 FR 1752126
(43) Date de publication de la demande: 07.08.2019
(73) Titulaire: Centre National de la Recherche Scientifique, 75016 Paris (FR); Université de Montpellier, 34090 Montpellier (FR)
(72) Inventeur: BENKIRANE, Monsef, 34980 Saint Gely du Fesc (FR); PETITJEAN, Gael, 34090 Montpellier (FR); DESCOURS, Benjamin, 34090 Montpellier (FR)
(74) Mandataire: LLR
(86) Numéro de dépôt international: PCT/IB2017/056055
(87) Numéro de publication internationale: WO 2018/060979

(56) Documents cités:
- WO-A1-2016/102829
- US-B2- 7 192 582
- PETITJEAN ET AL: "Isolation and characterization of HIV-1-infected resting CD4<+> T lymphocytes in breast milk", JOURNAL OF CLINICAL VIROLOGY, ELSEVIER, AMSTERDAM, NL, vol. 39, no. 1, 17 avril 2007 (2007-04-17) , pages 1-8, XP022032673, ISSN: 1386-6532, DOI: 10.1016/J.JCV.2007.02.004
- GREGORY M. LAIRD ET AL: "Rapid Quantification of the Latent Reservoir for HIV-1 Using a Viral Outgrowth Assay", PLOS PATHOGENS, vol. 9, no. 5, 30 mai 2013 (2013-05-30), page e1003398, XP055204081, DOI: 10.1371/journal.ppat.1003398
- M. DUVAL ET AL: "A Bispecific Antibody Composed of a Nonneutralizing Antibody to the gp41 Immunodominant Region and an Anti-CD89 Antibody Directs Broad Human Immunodeficiency Virus Destruction by Neutrophils", JOURNAL OF VIROLOGY, vol. 82, no. 9, 1 mai 2008 (2008-05-01), pages 4671-4674, XP055185624, ISSN: 0022-538X, DOI: 10.1128/JVI.02499-07

### SEQUENCE LISTING

<110> Centre National de la Recherche Scientifique
<120> Marqueurs membranaires
<130> BR90019
<150> FR1659440
   <151> 2016-09-30
<150> FR1752126
   <151> 2017-03-15
<160> 39
<170> PatentIn version 3.5
<210> 1
   <211> 300
   <212> PRT
   <213> Homo sapiens
<400> 1
   Met Val Leu Leu Trp Leu Thr Leu Leu Leu Ile Ala Leu Pro Cys Leu
   1               5                   10                  15
   Leu Gln Thr Lys Glu Gly Gly Lys Pro Trp Ala Gly Ala Glu Asn Leu
               20                  25                  30
   Thr Cys Trp Ile His Asp Val Asp Phe Leu Ser Cys Ser Trp Ala Val
           35                  40                  45
   Gly Pro Gly Ala Pro Ala Asp Val Gln Tyr Asp Leu Tyr Leu Asn Val
       50                  55                  60
   Ala Asn Arg Arg Gln Gln Tyr Glu Cys Leu His Tyr Lys Thr Asp Ala
   65                  70                  75                  80
   Gln Gly Thr Arg Ile Gly Cys Arg Phe Asp Asp Ile Ser Arg Leu Ser
                   85                  90                  95
   Ser Gly Ser Gln Ser Ser His Ile Leu Val Arg Gly Arg Ser Ala Ala
               100                 105                 110
   Phe Gly Ile Pro Cys Thr Asp Lys Phe Val Val Phe Ser Gln Ile Glu
           115                 120                 125
   Ile Leu Thr Pro Pro Asn Met Thr Ala Lys Cys Asn Lys Thr His Ser
       130                 135                 140
   Phe Met His Trp Lys Met Arg Ser His Phe Asn Arg Lys Phe Arg Tyr
   145                 150                 155                 160
   Glu Leu Gln Ile Gln Lys Arg Met Gln Pro Val Ile Thr Glu Gln Val
                   165                 170                 175
   Arg Asp Arg Thr Ser Phe Gln Leu Leu Asn Pro Gly Thr Tyr Thr Val
               180                 185                 190
   Gln Ile Arg Ala Arg Glu Arg Val Tyr Glu Phe Leu Ser Ala Trp Ser
           195                 200                 205
   Thr Pro Gln Arg Phe Glu Cys Asp Gln Glu Glu Gly Ala Asn Thr Arg
       210                 215                 220
   Ala Trp Arg Thr Ser Leu Leu Ile Ala Leu Gly Thr Leu Leu Ala Leu
   225                 230                 235                 240
   Val Cys Val Phe Val Ile Cys Arg Arg Tyr Leu Val Met Gln Arg Leu
                   245                 250                 255
   Phe Pro Arg Ile Pro His Met Lys Asp Pro Ile Gly Asp Ser Phe Gln
               260                 265                 270
   Asn Asp Lys Leu Val Val Trp Glu Ala Gly Lys Ala Gly Leu Glu Glu
           275                 280                 285
   Cys Leu Val Thr Glu Val Gln Val Val Gln Lys Thr
       290                 295                 300
<210> 2
   <211> 281
   <212> PRT
   <213> Homo sapiens
<400> 2
   Met Gly His Pro Pro Leu Leu Pro Leu Leu Leu Leu Leu His Thr Cys
   1               5                   10                  15
   Val Pro Ala Ser Trp Gly Leu Arg Cys Met Gln Cys Lys Thr Asn Gly
               20                  25                  30
   Asp Cys Arg Val Glu Glu Cys Ala Leu Gly Gln Asp Leu Cys Arg Thr
           35                  40                  45
   Thr Ile Val Arg Leu Trp Glu Glu Gly Glu Glu Leu Glu Leu Val Glu
       50                  55                  60
   Lys Ser Cys Thr His Ser Glu Lys Thr Asn Arg Thr Leu Ser Tyr Arg
   65                  70                  75                  80
   Thr Gly Leu Lys Ile Thr Ser Leu Thr Glu Val Val Cys Gly Leu Asp
   85 90 95
   Leu Cys Asn Gln Gly Asn Ser Gly Arg Ala Val Thr Tyr Ser Arg Ser
   100 105 110
   Arg Tyr Leu Glu Cys Ile Ser Cys Gly Ser Ser Asp Met Ser Cys Glu
   115 120 125
   Arg Gly Arg His Gln Ser Leu Gln Cys Arg Ser Pro Glu Glu Gln Cys
   130 135 140
   Leu Asp Val Val Thr His Trp Ile Gln Glu Gly Glu Glu Gly Arg Pro
   145 150 155 160
   Lys Asp Asp Arg His Leu Arg Gly Cys Gly Tyr Leu Pro Gly Cys Pro
   165 170 175
   Gly Ser Asn Gly Phe His Asn Asn Asp Thr Phe His Phe Leu Lys Cys
   180 185 190
   Cys Asn Thr Thr Lys Cys Asn Glu Gly Pro Ile Leu Glu Leu Glu Asn
   195 200 205
   Leu Pro Gln Asn Gly Arg Gln Cys Tyr Ser Cys Lys Gly Asn Ser Thr
   210 215 220
   His Gly Cys Ser Ser Glu Glu Thr Phe Leu Ile Asp Cys Arg Gly Pro
   225 230 235 240
   Met Asn Gln Cys Leu Val Ala Thr Gly Thr His Glu Arg Ser Leu Trp
   245 250 255
   Gly Ser Trp Leu Pro Cys Lys Ser Thr Thr Ala Leu Arg Pro Pro Cys
   260 265 270
   Cys Glu Glu Ala Gln Ala Thr His Val
   275 280
<210> 3
   <211> 287
   <212> PRT
   <213> Homo sapiens
<400> 3
   Met Asp Pro Lys Gln Thr Thr Leu Leu Cys Leu Val Leu Cys Leu Gly
   1 5 10 15
   Gln Arg Ile Gln Ala Gln Glu Gly Asp Phe Pro Met Pro Phe Ile Ser
               20                  25                  30
   Ala Lys Ser Ser Pro Val Ile Pro Leu Asp Gly Ser Val Lys Ile Gln
           35                  40                  45
   Cys Gln Ala Ile Arg Glu Ala Tyr Leu Thr Gln Leu Met Ile Ile Lys
       50                  55                  60
   Asn Ser Thr Tyr Arg Glu Ile Gly Arg Arg Leu Lys Phe Trp Asn Glu
   65                  70                  75                  80
   Thr Asp Pro Glu Phe Val Ile Asp His Met Asp Ala Asn Lys Ala Gly
                   85                  90                  95
   Arg Tyr Gln Cys Gln Tyr Arg Ile Gly His Tyr Arg Phe Arg Tyr Ser
               100                 105                 110
   Asp Thr Leu Glu Leu Val Val Thr Gly Leu Tyr Gly Lys Pro Phe Leu
           115                 120                 125
   Ser Ala Asp Arg Gly Leu Val Leu Met Pro Gly Glu Asn Ile Ser Leu
       130                 135                 140
   Thr Cys Ser Ser Ala His Ile Pro Phe Asp Arg Phe Ser Leu Ala Lys
   145                 150                 155                 160
   Glu Gly Glu Leu Ser Leu Pro Gln His Gln Ser Gly Glu His Pro Ala
                   165                 170                 175
   Asn Phe Ser Leu Gly Pro Val Asp Leu Asn Val Ser Gly Ile Tyr Arg
               180                 185                 190
   Cys Tyr Gly Trp Tyr Asn Arg Ser Pro Tyr Leu Trp Ser Phe Pro Ser
           195                 200                 205
   Asn Ala Leu Glu Leu Val Val Thr Asp Ser Ile His Gln Asp Tyr Thr
       210                 215                 220
   Thr Gln Asn Leu Ile Arg Met Ala Val Ala Gly Leu Val Leu Val Ala
   225                 230                 235                 240
   Leu Leu Ala Ile Leu Val Glu Asn Trp His Ser His Thr Ala Leu Asn
                   245                 250                 255
   Lys Glu Ala Ser Ala Asp Val Ala Glu Pro Ser Trp Ser Gln Gln Met
   260 265 270
   Cys Gln Pro Gly Leu Thr Phe Ala Arg Thr Pro Ser Val Cys Lys
   275 280 285
<210> 4
   <211> 504
   <212> **PRT**
   <213> Homo sapiens
<400> 4
   Met Glu Pro Ala Gly Pro Ala Pro Gly Arg Leu Gly Pro Leu Leu Cys
   1               5                   10                  15
   Leu Leu Leu Ala Ala Ser Cys Ala Trp Ser Gly Val Ala Gly Glu Glu
               20                  25                  30
   Glu Leu Gln Val Ile Gln Pro Asp Lys Ser Val Leu Val Ala Ala Gly
           35                  40                  45
   Glu Thr Ala Thr Leu Arg Cys Thr Ala Thr Ser Leu Ile Pro Val Gly
       50                  55                  60
   Pro Ile Gln Trp Phe Arg Gly Ala Gly Pro Gly Arg Glu Leu Ile Tyr
   65                  70                  75                  80
   Asn Gln Lys Glu Gly His Phe Pro Arg Val Thr Thr Val Ser Asp Leu
                   85                  90                  95
   Thr Lys Arg Asn Asn Met Asp Phe Ser Ile Arg Ile Gly Asn Ile Thr
               100                 105                 110
   Pro Ala Asp Ala Gly Thr Tyr Tyr Cys Val Lys Phe Arg Lys Gly Ser
           115                 120                 125
   Pro Asp Asp Val Glu Phe Lys Ser Gly Ala Gly Thr Glu Leu Ser Val
       130                 135                 140
   Arg Ala Lys Pro Ser Ala Pro Val Val Ser Gly Pro Ala Ala Arg Ala
   145                 150                 155                 160
   Thr Pro Gln His Thr Val Ser Phe Thr Cys Glu Ser His Gly Phe Ser
                   165                 170                 175
   Pro Arg Asp Ile Thr Leu Lys Trp Phe Lys Asn Gly Asn Glu Leu Ser
               180                 185                 190
   Asp Phe Gln Thr Asn Val Asp Pro Val Gly Glu Ser Val Ser Tyr Ser
           195                 200                 205
   Ile His Ser Thr Ala Lys Val Val Leu Thr Arg Glu Asp Val His Ser
       210                 215                 220
   Gln Val Ile Cys Glu Val Ala His Val Thr Leu Gln Gly Asp Pro Leu
   225                 230                 235                 240
   Arg Gly Thr Ala Asn Leu Ser Glu Thr Ile Arg Val Pro Pro Thr Leu
                   245                 250                 255
   Glu Val Thr Gln Gln Pro Val Arg Ala Glu Asn Gln Val Asn Val Thr
               260                 265                 270
   Cys Gln Val Arg Lys Phe Tyr Pro Gln Arg Leu Gln Leu Thr Trp Leu
           275                 280                 285
   Glu Asn Gly Asn Val Ser Arg Thr Glu Thr Ala Ser Thr Val Thr Glu
       290                 295                 300
   Asn Lys Asp Gly Thr Tyr Asn Trp Met Ser Trp Leu Leu Val Asn Val
   305                 310                 315                 320
   Ser Ala His Arg Asp Asp Val Lys Leu Thr Cys Gln Val Glu His Asp
                   325                 330                 335
   Gly Gln Pro Ala Val Ser Lys Ser His Asp Leu Lys Val Ser Ala His
               340                 345                 350
   Pro Lys Glu Gln Gly Ser Asn Thr Ala Ala Glu Asn Thr Gly Ser Asn
           355                 360                 365
   Glu Arg Asn Ile Tyr Ile Val Val Gly Val Val Cys Thr Leu Leu Val
       370                 375                 380
   Ala Leu Leu Met Ala Ala Leu Tyr Leu Val Arg Ile Arg Gln Lys Lys
   385                 390                 395                 400
   Ala Gln Gly Ser Thr Ser Ser Thr Arg Leu His Glu Pro Glu Lys Asn
                   405                 410                 415
   Ala Arg Glu Ile Thr Gln Asp Thr Asn Asp Ile Thr Tyr Ala Asp Leu
               420                 425                 430
   Asn Leu Pro Lys Gly Lys Lys Pro Ala Pro Gln Ala Ala Glu Pro Asn
           435                 440                 445
   Asn His Thr Glu Tyr Ala Ser Ile Gln Thr Ser Pro Gln Pro Ala Ser
   450 455 460
   Glu Asp Thr Leu Thr Tyr Ala Asp Leu Asp Met Val His Leu Asn Arg
   465 470 475 480
   Thr Pro Lys Gln Pro Ala Pro Lys Pro Glu Pro Ser Phe Ser Glu Tyr
   485 490 495
   Ala Ser Val Gln Val Pro Arg Lys
   500
<210> 5
   <211> 538
   <212> PRT
   <213> Homo sapiens
<400> 5
   Met Ala Arg Ala Ala Ala Leu Leu Pro Ser Arg Ser Pro Pro Thr Pro
   1 5 10 15
   Leu Leu Trp Pro Leu Leu Leu Leu Leu Leu Leu Glu Thr Gly Ala Gln
   20 25 30
   Asp Val Arg Val Gln Val Leu Pro Glu Val Arg Gly Gln Leu Gly Gly
   35 40 45
   Thr Val Glu Leu Pro Cys His Leu Leu Pro Pro Val Pro Gly Leu Tyr
   50 55 60
   Ile Ser Leu Val Thr Trp Gln Arg Pro Asp Ala Pro Ala Asn His Gln
   65 70 75 80
   Asn Val Ala Ala Phe His Pro Lys Met Gly Pro Ser Phe Pro Ser Pro
   85 90 95
   Lys Pro Gly Ser Glu Arg Leu Ser Phe Val Ser Ala Lys Gln Ser Thr
   100 105 110
   Gly Gln Asp Thr Glu Ala Glu Leu Gln Asp Ala Thr Leu Ala Leu His
   115 120 125
   Gly Leu Thr Val Glu Asp Glu Gly Asn Tyr Thr Cys Glu Phe Ala Thr
   130 135 140
   Phe Pro Lys Gly Ser Val Arg Gly Met Thr Trp Leu Arg Val Ile Ala
   145 150 155 160
   Lys Pro Lys Asn Gln Ala Glu Ala Gln Lys Val Thr Phe Ser Gln Asp
                   165                 170                 175
   Pro Thr Thr Val Ala Leu Cys Ile Ser Lys Glu Gly Arg Pro Pro Ala
               180                 185                 190
   Arg Ile Ser Trp Leu Ser Ser Leu Asp Trp Glu Ala Lys Glu Thr Gln
           195                 200                 205
   Val Ser Gly Thr Leu Ala Gly Thr Val Thr Val Thr Ser Arg Phe Thr
       210                 215                 220
   Leu Val Pro Ser Gly Arg Ala Asp Gly Val Thr Val Thr Cys Lys Val
   225                 230                 235                 240
   Glu His Glu Ser Phe Glu Glu Pro Ala Leu Ile Pro Val Thr Leu Ser
                   245                 250                 255
   Val Arg Tyr Pro Pro Glu Val Ser Ile Ser Gly Tyr Asp Asp Asn Trp
               260                 265                 270
   Tyr Leu Gly Arg Thr Asp Ala Thr Leu Ser Cys Asp Val Arg Ser Asn
           275                 280                 285
   Pro Glu Pro Thr Gly Tyr Asp Trp Ser Thr Thr Ser Gly Thr Phe Pro
       290                 295                 300
   Thr Ser Ala Val Ala Gln Gly Ser Gln Leu Val Ile His Ala Val Asp
   305                 310                 315                 320
   Ser Leu Phe Asn Thr Thr Phe Val Cys Thr Val Thr Asn Ala Val Gly
                   325                 330                 335
   Met Gly Arg Ala Glu Gln Val Ile Phe Val Arg Glu Thr Pro Asn Thr
               340                 345                 350
   Ala Gly Ala Gly Ala Thr Gly Gly Ile Ile Gly Gly Ile Ile Ala Ala
           355                 360                 365
   Ile Ile Ala Thr Ala Val Ala Ala Thr Gly Ile Leu Ile Cys Arg Gln
       370                 375                 380
   Gln Arg Lys Glu Gln Thr Leu Gln Gly Ala Glu Glu Asp Glu Asp Leu
   385                 390                 395                 400
   Glu Gly Pro Pro Ser Tyr Lys Pro Pro Thr Pro Lys Ala Lys Leu Glu
                   405                 410                 415
   Ala Gln Glu Met Pro Ser Gln Leu Phe Thr Leu Gly Ala Ser Glu His
               420                 425                 430
   Ser Pro Leu Lys Thr Pro Tyr Phe Asp Ala Gly Ala Ser Cys Thr Glu
           435                 440                 445
   Gln Glu Met Pro Arg Tyr His Glu Leu Pro Thr Leu Glu Glu Arg Ser
       450                 455                 460
   Gly Pro Leu His Pro Gly Ala Thr Ser Leu Gly Ser Pro Ile Pro Val
   465                 470                 475                 480
   Pro Pro Gly Pro Pro Ala Val Glu Asp Val Ser Leu Asp Leu Glu Asp
                   485                 490                 495
   Glu Glu Gly Glu Glu Glu Glu Glu Tyr Leu Asp Lys Ile Asn Pro Ile
               500                 505                 510
   Tyr Asp Ala Leu Ser Tyr Ser Ser Pro Ser Asp Ser Tyr Gln Gly Lys
           515                 520                 525
   Gly Phe Val Met Ser Arg Ala Met Tyr Val
       530                 535
<210> 6
   <211> 427
   <212> PRT
   <213> Homo sapiens
<400> 6
   Met Glu Trp Pro Ala Arg Leu Cys Gly Leu Trp Ala Leu Leu Leu Cys
   1               5                   10                  15
   Ala Gly Gly Gly Gly Gly Gly Gly Gly Ala Ala Pro Thr Glu Thr Gln
               20                  25                  30
   Pro Pro Val Thr Asn Leu Ser Val Ser Val Glu Asn Leu Cys Thr Val
           35                  40                  45
   Ile Trp Thr Trp Asn Pro Pro Glu Gly Ala Ser Ser Asn Cys Ser Leu
       50                  55                  60
   Trp Tyr Phe Ser His Phe Gly Asp Lys Gln Asp Lys Lys Ile Ala Pro
   65                  70                  75                  80
   Glu Thr Arg Arg Ser Ile Glu Val Pro Leu Asn Glu Arg Ile Cys Leu
                   85                  90                  95
   Gln Val Gly Ser Gln Cys Ser Thr Asn Glu Ser Glu Lys Pro Ser Ile
               100                 105                 110
   Leu Val Glu Lys Cys Ile Ser Pro Pro Glu Gly Asp Pro Glu Ser Ala
           115                 120                 125
   Val Thr Glu Leu Gln Cys Ile Trp His Asn Leu Ser Tyr Met Lys Cys
       130                 135                 140
   Ser Trp Leu Pro Gly Arg Asn Thr Ser Pro Asp Thr Asn Tyr Thr Leu
   145                 150                 155                 160
   Tyr Tyr Trp His Arg Ser Leu Glu Lys Ile His Gln Cys Glu Asn Ile
                   165                 170                 175
   Phe Arg Glu Gly Gln Tyr Phe Gly Cys Ser Phe Asp Leu Thr Lys Val
               180                 185                 190
   Lys Asp Ser Ser Phe Glu Gln His Ser Val Gln Ile Met Val Lys Asp
           195                 200                 205
   Asn Ala Gly Lys Ile Lys Pro Ser Phe Asn Ile Val Pro Leu Thr Ser
       210                 215                 220
   Arg Val Lys Pro Asp Pro Pro His Ile Lys Asn Leu Ser Phe His Asn
   225                 230                 235                 240
   Asp Asp Leu Tyr Val Gln Trp Glu Asn Pro Gln Asn Phe Ile Ser Arg
                   245                 250                 255
   Cys Leu Phe Tyr Glu Val Glu Val Asn Asn Ser Gln Thr Glu Thr His
               260                 265                 270
   Asn Val Phe Tyr Val Gln Glu Ala Lys Cys Glu Asn Pro Glu Phe Glu
           275                 280                 285
   Arg Asn Val Glu Asn Thr Ser Cys Phe Met Val Pro Gly Val Leu Pro
       290                 295                 300
   Asp Thr Leu Asn Thr Val Arg Ile Arg Val Lys Thr Asn Lys Leu Cys
   305                 310                 315                 320
   Tyr Glu Asp Asp Lys Leu Trp Ser Asn Trp Ser Gln Glu Met Ser Ile
                   325                 330                 335
   Gly Lys Lys Arg Asn Ser Thr Leu Tyr Ile Thr Met Leu Leu Ile Val
               340                 345                 350
   Pro Val Ile Val Ala Gly Ala Ile Ile Val Leu Leu Leu Tyr Leu Lys
   355 360 365
   Arg Leu Lys Ile Ile Ile Phe Pro Pro Ile Pro Asp Pro Gly Lys Ile
   370 375 380
   Phe Lys Glu Met Phe Gly Asp Gln Asn Asp Asp Thr Leu His Trp Lys
   385 390 395 400
   Lys Tyr Asp Ile Tyr Glu Lys Gln Thr Lys Glu Glu Thr Asp Ser Val
   405 410 415
   Val Leu Ile Glu Asn Leu Lys Lys Ala Ser Gln
   420 425
<210> 7
   <211> 461
   <212> PRT
   <213> Homo sapiens
<400> 7
   Met Ala Pro Val Ala Val Trp Ala Ala Leu Ala Val Gly Leu Glu Leu
   1 5 10 15
   Trp Ala Ala Ala His Ala Leu Pro Ala Gln Val Ala Phe Thr Pro Tyr
   20 25 30
   Ala Pro Glu Pro Gly Ser Thr Cys Arg Leu Arg Glu Tyr Tyr Asp Gln
   35 40 45
   Thr Ala Gln Met Cys Cys Ser Lys Cys Ser Pro Gly Gln His Ala Lys
   50 55 60
   Val Phe Cys Thr Lys Thr Ser Asp Thr Val Cys Asp Ser Cys Glu Asp
   65 70 75 80
   Ser Thr Tyr Thr Gln Leu Trp Asn Trp Val Pro Glu Cys Leu Ser Cys
   85 90 95
   Gly Ser Arg Cys Ser Ser Asp Gln Val Glu Thr Gln Ala Cys Thr Arg
   100 105 110
   Glu Gln Asn Arg Ile Cys Thr Cys Arg Pro Gly Trp Tyr Cys Ala Leu
   115 120 125
   Ser Lys Gln Glu Gly Cys Arg Leu Cys Ala Pro Leu Arg Lys Cys Arg
   130 135 140
   Pro Gly Phe Gly Val Ala Arg Pro Gly Thr Glu Thr Ser Asp Val Val
   145                 150                 155                 160
   Cys Lys Pro Cys Ala Pro Gly Thr Phe Ser Asn Thr Thr Ser Ser Thr
                   165                 170                 175
   Asp Ile Cys Arg Pro His Gln Ile Cys Asn Val Val Ala Ile Pro Gly
               180                 185                 190
   Asn Ala Ser Met Asp Ala Val Cys Thr Ser Thr Ser Pro Thr Arg Ser
           195                 200                 205
   Met Ala Pro Gly Ala Val His Leu Pro Gln Pro Val Ser Thr Arg Ser
       210                 215                 220
   Gln His Thr Gln Pro Thr Pro Glu Pro Ser Thr Ala Pro Ser Thr Ser
   225                 230                 235                 240
   Phe Leu Leu Pro Met Gly Pro Ser Pro Pro Ala Glu Gly Ser Thr Gly
                   245                 250                 255
   Asp Phe Ala Leu Pro Val Gly Leu Ile Val Gly Val Thr Ala Leu Gly
               260                 265                 270
   Leu Leu Ile Ile Gly Val Val Asn Cys Val Ile Met Thr Gln Val Lys
           275                 280                 285
   Lys Lys Pro Leu Cys Leu Gln Arg Glu Ala Lys Val Pro His Leu Pro
       290                 295                 300
   Ala Asp Lys Ala Arg Gly Thr Gln Gly Pro Glu Gln Gln His Leu Leu
   305                 310                 315                 320
   Ile Thr Ala Pro Ser Ser Ser Ser Ser Ser Leu Glu Ser Ser Ala Ser
                   325                 330                 335
   Ala Leu Asp Arg Arg Ala Pro Thr Arg Asn Gln Pro Gln Ala Pro Gly
               340                 345                 350
   Val Glu Ala Ser Gly Ala Gly Glu Ala Arg Ala Ser Thr Gly Ser Ser
           355                 360                 365
   Asp Ser Ser Pro Gly Gly His Gly Thr Gln Val Asn Val Thr Cys Ile
       370                 375                 380
   Val Asn Val Cys Ser Ser Ser Asp His Ser Ser Gln Cys Ser Ser Gln
   385 390 395 400
   Ala Ser Ser Thr Met Gly Asp Thr Asp Ser Ser Pro Ser Glu Ser Pro
   405 410 415
   Lys Asp Glu Gln Val Pro Phe Ser Lys Glu Glu Cys Ala Phe Arg Ser
   420 425 430
   Gln Leu Glu Thr Pro Glu Thr Leu Leu Gly Ser Thr Glu Glu Lys Pro
   435 440 445
   Leu Pro Leu Gly Val Pro Asp Ala Gly Met Lys Pro Ser
   450 455 460
<210> 8
   <211> 569
   <212> PRT
   <213> Homo sapiens
<400> 8
   Met Lys Val Leu Leu Arg Leu Ile Cys Phe Ile Ala Leu Leu Ile Ser
   1               5                   10                  15
   Ser Leu Glu Ala Asp Lys Cys Lys Glu Arg Glu Glu Lys Ile Ile Leu
               20                  25                  30
   Val Ser Ser Ala Asn Glu Ile Asp Val Arg Pro Cys Pro Leu Asn Pro
           35                  40                  45
   Asn Glu His Lys Gly Thr Ile Thr Trp Tyr Lys Asp Asp Ser Lys Thr
       50                  55                  60
   Pro Val Ser Thr Glu Gln Ala Ser Arg Ile His Gln His Lys Glu Lys
   65                  70                  75                  80
   Leu Trp Phe Val Pro Ala Lys Val Glu Asp Ser Gly His Tyr Tyr Cys
                   85                  90                  95
   Val Val Arg Asn Ser Ser Tyr Cys Leu Arg Ile Lys Ile Ser Ala Lys
               100                 105                 110
   Phe Val Glu Asn Glu Pro Asn Leu Cys Tyr Asn Ala Gln Ala Ile Phe
           115                 120                 125
   Lys Gln Lys Leu Pro Val Ala Gly Asp Gly Gly Leu Val Cys Pro Tyr
       130                 135                 140
   Met Glu Phe Phe Lys Asn Glu Asn Asn Glu Leu Pro Lys Leu Gln Trp
   145 150 155 160
   Tyr Lys Asp Cys Lys Pro Leu Leu Leu Asp Asn Ile His Phe Ser Gly
   165 170 175
   Val Lys Asp Arg Leu Ile Val Met Asn Val Ala Glu Lys His Arg Gly
   180 185 190
   Asn Tyr Thr Cys His Ala Ser Tyr Thr Tyr Leu Gly Lys Gln Tyr Pro
   195 200 205
   Ile Thr Arg Val Ile Glu Phe Ile Thr Leu Glu Glu Asn Lys Pro Thr
   210 215 220
   Arg Pro Val Ile Val Ser Pro Ala Asn Glu Thr Met Glu Val Asp Leu
   225 230 235 240
   Gly Ser Gln Ile Gln Leu Ile Cys Asn Val Thr Gly Gln Leu Ser Asp
   245 250 255
   Ile Ala Tyr Trp Lys Trp Asn Gly Ser Val Ile Asp Glu Asp Asp Pro
   260 265 270
   Val Leu Gly Glu Asp Tyr Tyr Ser Val Glu Asn Pro Ala Asn Lys Arg
   275 280 285
   Arg Ser Thr Leu Ile Thr Val Leu Asn Ile Ser Glu Ile Glu Ser Arg
   290 295 300
   Phe Tyr Lys His Pro Phe Thr Cys Phe Ala Lys Asn Thr His Gly Ile
   305 310 315 320
   Asp Ala Ala Tyr Ile Gln Leu Ile Tyr Pro Val Thr Asn Phe Gln Lys
   325 330 335
   His Met Ile Gly Ile Cys Val Thr Leu Thr Val Ile Ile Val Cys Ser
   340 345 350
   Val Phe Ile Tyr Lys Ile Phe Lys Ile Asp Ile Val Leu Trp Tyr Arg
   355 360 365
   Asp Ser Cys Tyr Asp Phe Leu Pro Ile Lys Ala Ser Asp Gly Lys Thr
   370 375 380
   Tyr Asp Ala Tyr Ile Leu Tyr Pro Lys Thr Val Gly Glu Gly Ser Thr
   385 390 395 400
   Ser Asp Cys Asp Ile Phe Val Phe Lys Val Leu Pro Glu Val Leu Glu
                   405                 410                 415
   Lys Gln Cys Gly Tyr Lys Leu Phe Ile Tyr Gly Arg Asp Asp Tyr Val
               420                 425                 430
   Gly Glu Asp Ile Val Glu Val Ile Asn Glu Asn Val Lys Lys Ser Arg
           435                 440                 445
   Arg Leu Ile Ile Ile Leu Val Arg Glu Thr Ser Gly Phe Ser Trp Leu
       450                 455                 460
   Gly Gly Ser Ser Glu Glu Gln Ile Ala Met Tyr Asn Ala Leu Val Gln
   465                 470                 475                 480
   Asp Gly Ile Lys Val Val Leu Leu Glu Leu Glu Lys Ile Gln Asp Tyr
                   485                 490                 495
   Glu Lys Met Pro Glu Ser Ile Lys Phe Ile Lys Gln Lys His Gly Ala
               500                 505                 510
   Ile Arg Trp Ser Gly Asp Phe Thr Gln Gly Pro Gln Ser Ala Lys Thr
           515                 520                 525
   Arg Phe Trp Lys Asn Val Arg Tyr His Met Pro Val Gln Arg Arg Ser
       530                 535                 540
   Pro Ser Ser Lys His Gln Leu Leu Ser Pro Ala Thr Lys Glu Lys Leu
   545                 550                 555                 560
   Gln Arg Glu Ala His Val Pro Leu Gly
                   565
<210> 9
   <211> 532
   <212> PRT
   <213> Homo sapiens
<400> 9
   Met Ala Pro Ser Ser Pro Arg Pro Ala Leu Pro Ala Leu Leu Val Leu
   1               5                   10                  15
   Leu Gly Ala Leu Phe Pro Gly Pro Gly Asn Ala Gln Thr Ser Val Ser
               20                  25                  30
   Pro Ser Lys Val Ile Leu Pro Arg Gly Gly Ser Val Leu Val Thr Cys
           35                  40                  45
   Ser Thr Ser Cys Asp Gln Pro Lys Leu Leu Gly Ile Glu Thr Pro Leu
       50                  55                  60
   Pro Lys Lys Glu Leu Leu Leu Pro Gly Asn Asn Arg Lys Val Tyr Glu
   65                  70                  75                  80
   Leu Ser Asn Val Gln Glu Asp Ser Gln Pro Met Cys Tyr Ser Asn Cys
                   85                  90                  95
   Pro Asp Gly Gln Ser Thr Ala Lys Thr Phe Leu Thr Val Tyr Trp Thr
               100                 105                 110
   Pro Glu Arg Val Glu Leu Ala Pro Leu Pro Ser Trp Gln Pro Val Gly
           115                 120                 125
   Lys Asn Leu Thr Leu Arg Cys Gln Val Glu Gly Gly Ala Pro Arg Ala
       130                 135                 140
   Asn Leu Thr Val Val Leu Leu Arg Gly Glu Lys Glu Leu Lys Arg Glu
   145                 150                 155                 160
   Pro Ala Val Gly Glu Pro Ala Glu Val Thr Thr Thr Val Leu Val Arg
                   165                 170                 175
   Arg Asp His His Gly Ala Asn Phe Ser Cys Arg Thr Glu Leu Asp Leu
               180                 185                 190
   Arg Pro Gln Gly Leu Glu Leu Phe Glu Asn Thr Ser Ala Pro Tyr Gln
           195                 200                 205
   Leu Gln Thr Phe Val Leu Pro Ala Thr Pro Pro Gln Leu Val Ser Pro
       210                 215                 220
   Arg Val Leu Glu Val Asp Thr Gln Gly Thr Val Val Cys Ser Leu Asp
   225                 230                 235                 240
   Gly Leu Phe Pro Val Ser Glu Ala Gln Val His Leu Ala Leu Gly Asp
                   245                 250                 255
   Gln Arg Leu Asn Pro Thr Val Thr Tyr Gly Asn Asp Ser Phe Ser Ala
               260                 265                 270
   Lys Ala Ser Val Ser Val Thr Ala Glu Asp Glu Gly Thr Gln Arg Leu
           275                 280                 285
   Thr Cys Ala Val Ile Leu Gly Asn Gln Ser Gln Glu Thr Leu Gln Thr
       290                 295                 300
   Val Thr Ile Tyr Ser Phe Pro Ala Pro Asn Val Ile Leu Thr Lys Pro
   305 310 315 320
   Glu Val Ser Glu Gly Thr Glu Val Thr Val Lys Cys Glu Ala His Pro
   325 330 335
   Arg Ala Lys Val Thr Leu Asn Gly Val Pro Ala Gln Pro Leu Gly Pro
   340 345 350
   Arg Ala Gln Leu Leu Leu Lys Ala Thr Pro Glu Asp Asn Gly Arg Ser
   355 360 365
   Phe Ser Cys Ser Ala Thr Leu Glu Val Ala Gly Gln Leu Ile His Lys
   370 375 380
   Asn Gln Thr Arg Glu Leu Arg Val Leu Tyr Gly Pro Arg Leu Asp Glu
   385 390 395 400
   Arg Asp Cys Pro Gly Asn Trp Thr Trp Pro Glu Asn Ser Gln Gln Thr
   405 410 415
   Pro Met Cys Gln Ala Trp Gly Asn Pro Leu Pro Glu Leu Lys Cys Leu
   420 425 430
   Lys Asp Gly Thr Phe Pro Leu Pro Ile Gly Glu Ser Val Thr Val Thr
   435 440 445
   Arg Asp Leu Glu Gly Thr Tyr Leu Cys Arg Ala Arg Ser Thr Gln Gly
   450 455 460
   Glu Val Thr Arg Lys Val Thr Val Asn Val Leu Ser Pro Arg Tyr Glu
   465 470 475 480
   Ile Val Ile Ile Thr Val Val Ala Ala Ala Val Ile Met Gly Thr Ala
   485 490 495
   Gly Leu Ser Thr Tyr Leu Tyr Asn Arg Gln Arg Lys Ile Lys Lys Tyr
   500 505 510
   Arg Leu Gln Gln Ala Gln Lys Gly Thr Pro Met Lys Pro Asn Thr Gln
   515 520 525
   Ala Thr Pro Pro
   530
<210> 10
   <211> 1163
   <212> PRT
   <213> Homo sapiens
<400> 10
   Met Thr Arg Thr Arg Ala Ala Leu Leu Leu Phe Thr Ala Leu Ala Thr
   1               5                   10                  15
   Ser Leu Gly Phe Asn Leu Asp Thr Glu Glu Leu Thr Ala Phe Arg Val
               20                  25                  30
   Asp Ser Ala Gly Phe Gly Asp Ser Val Val Gln Tyr Ala Asn Ser Trp
           35                  40                  45
   Val Val Val Gly Ala Pro Gln Lys Ile Thr Ala Ala Asn Gln Thr Gly
       50                  55                  60
   Gly Leu Tyr Gln Cys Gly Tyr Ser Thr Gly Ala Cys Glu Pro Ile Gly
   65                  70                  75                  80
   Leu Gln Val Pro Pro Glu Ala Val Asn Met Ser Leu Gly Leu Ser Leu
                   85                  90                  95
   Ala Ser Thr Thr Ser Pro Ser Gln Leu Leu Ala Cys Gly Pro Thr Val
               100                 105                 110
   His His Glu Cys Gly Arg Asn Met Tyr Leu Thr Gly Leu Cys Phe Leu
           115                 120                 125
   Leu Gly Pro Thr Gln Leu Thr Gln Arg Leu Pro Val Ser Arg Gln Glu
       130                 135                 140
   Cys Pro Arg Gln Glu Gln Asp Ile Val Phe Leu Ile Asp Gly Ser Gly
   145                 150                 155                 160
   Ser Ile Ser Ser Arg Asn Phe Ala Thr Met Met Asn Phe Val Arg Ala
                   165                 170                 175
   Val Ile Ser Gln Phe Gln Arg Pro Ser Thr Gln Phe Ser Leu Met Gln
               180                 185                 190
   Phe Ser Asn Lys Phe Gln Thr His Phe Thr Phe Glu Glu Phe Arg Arg
           195                 200                 205
   Ser Ser Asn Pro Leu Ser Leu Leu Ala Ser Val His Gln Leu Gln Gly
       210                 215                 220
   Phe Thr Tyr Thr Ala Thr Ala Ile Gln Asn Val Val His Arg Leu Phe
   225                 230                 235                 240
   His Ala Ser Tyr Gly Ala Arg Arg Asp Ala Ala Lys Ile Leu Ile Val
   245 250 255
   Ile Thr Asp Gly Lys Lys Glu Gly Asp Ser Leu Asp Tyr Lys Asp Val
   260 265 270
   Ile Pro Met Ala Asp Ala Ala Gly Ile Ile Arg Tyr Ala Ile Gly Val
   275 280 285
   Gly Leu Ala Phe Gln Asn Arg Asn Ser Trp Lys Glu Leu Asn Asp Ile
   290 295 300
   Ala Ser Lys Pro Ser Gln Glu His Ile Phe Lys Val Glu Asp Phe Asp
   305 310 315 320
   Ala Leu Lys Asp Ile Gln Asn Gln Leu Lys Glu Lys Ile Phe Ala Ile
   325 330 335
   Glu Gly Thr Glu Thr Thr Ser Ser Ser Ser Phe Glu Leu Glu Met Ala
   340 345 350
   Gln Glu Gly Phe Ser Ala Val Phe Thr Pro Asp Gly Pro Val Leu Gly
   355 360 365
   Ala Val Gly Ser Phe Thr Trp Ser Gly Gly Ala Phe Leu Tyr Pro Pro
   370 375 380
   Asn Met Ser Pro Thr Phe Ile Asn Met Ser Gln Glu Asn Val Asp Met
   385 390 395 400
   Arg Asp Ser Tyr Leu Gly Tyr Ser Thr Glu Leu Ala Leu Trp Lys Gly
   405 410 415
   Val Gln Ser Leu Val Leu Gly Ala Pro Arg Tyr Gln His Thr Gly Lys
   420 425 430
   Ala Val Ile Phe Thr Gln Val Ser Arg Gln Trp Arg Met Lys Ala Glu
   435 440 445
   Val Thr Gly Thr Gln Ile Gly Ser Tyr Phe Gly Ala Ser Leu Cys Ser
   450 455 460
   Val Asp Val Asp Ser Asp Gly Ser Thr Asp Leu Val Leu Ile Gly Ala
   465 470 475 480
   Pro His Tyr Tyr Glu Gln Thr Arg Gly Gly Gln Val Ser Val Cys Pro
                   485                 490                 495
   Leu Pro Arg Gly Trp Arg Arg Trp Trp Cys Asp Ala Val Leu Tyr Gly
               500                 505                 510
   Glu Gln Gly His Pro Trp Gly Arg Phe Gly Ala Ala Leu Thr Val Leu
           515                 520                 525
   Gly Asp Val Asn Gly Asp Lys Leu Thr Asp Val Val Ile Gly Ala Pro
       530                 535                 540
   Gly Glu Glu Glu Asn Arg Gly Ala Val Tyr Leu Phe His Gly Val Leu
   545                 550                 555                 560
   Gly Pro Ser Ile Ser Pro Ser His Ser Gln Arg Ile Ala Gly Ser Gln
                   565                 570                 575
   Leu Ser Ser Arg Leu Gln Tyr Phe Gly Gln Ala Leu Ser Gly Gly Gln
               580                 585                 590
   Asp Leu Thr Gln Asp Gly Leu Val Asp Leu Ala Val Gly Ala Arg Gly
           595                 600                 605
   Gln Val Leu Leu Leu Arg Thr Arg Pro Val Leu Trp Val Gly Val Ser
       610                 615                 620
   Met Gln Phe Ile Pro Ala Glu Ile Pro Arg Ser Ala Phe Glu Cys Arg
   625                 630                 635                 640
   Glu Gln Val Val Ser Glu Gln Thr Leu Val Gln Ser Asn Ile Cys Leu
                   645                 650                 655
   Tyr Ile Asp Lys Arg Ser Lys Asn Leu Leu Gly Ser Arg Asp Leu Gln
               660                 665                 670
   Ser Ser Val Thr Leu Asp Leu Ala Leu Asp Pro Gly Arg Leu Ser Pro
           675                 680                 685
   Arg Ala Thr Phe Gln Glu Thr Lys Asn Arg Ser Leu Ser Arg Val Arg
       690                 695                 700
   Val Leu Gly Leu Lys Ala His Cys Glu Asn Phe Asn Leu Leu Leu Pro
   705                 710                 715                 720
   Ser Cys Val Glu Asp Ser Val Thr Pro Ile Thr Leu Arg Leu Asn Phe
                   725                 730                 735
   Thr Leu Val Gly Lys Pro Leu Leu Ala Phe Arg Asn Leu Arg Pro Met
               740                 745                 750
   Leu Ala Ala Asp Ala Gln Arg Tyr Phe Thr Ala Ser Leu Pro Phe Glu
           755                 760                 765
   Lys Asn Cys Gly Ala Asp His Ile Cys Gln Asp Asn Leu Gly Ile Ser
       770                 775                 780
   Phe Ser Phe Pro Gly Leu Lys Ser Leu Leu Val Gly Ser Asn Leu Glu
   785                 790                 795                 800
   Leu Asn Ala Glu Val Met Val Trp Asn Asp Gly Glu Asp Ser Tyr Gly
                   805                 810                 815
   Thr Thr Ile Thr Phe Ser His Pro Ala Gly Leu Ser Tyr Arg Tyr Val
               820                 825                 830
   Ala Glu Gly Gln Lys Gln Gly Gln Leu Arg Ser Leu His Leu Thr Cys
           835                 840                 845
   Asp Ser Ala Pro Val Gly Ser Gln Gly Thr Trp Ser Thr Ser Cys Arg
       850                 855                 860
   Ile Asn His Leu Ile Phe Arg Gly Gly Ala Gln Ile Thr Phe Leu Ala
   865                 870                 875                 880
   Thr Phe Asp Val Ser Pro Lys Ala Val Leu Gly Asp Arg Leu Leu Leu
                   885                 890                 895
   Thr Ala Asn Val Ser Ser Glu Asn Asn Thr Pro Arg Thr Ser Lys Thr
               900                 905                 910
   Thr Phe Gln Leu Glu Leu Pro Val Lys Tyr Ala Val Tyr Thr Val Val
           915                 920                 925
   Ser Ser His Glu Gln Phe Thr Lys Tyr Leu Asn Phe Ser Glu Ser Glu
       930                 935                 940
   Glu Lys Glu Ser His Val Ala Met His Arg Tyr Gln Val Asn Asn Leu
   945                 950                 955                 960
   Gly Gln Arg Asp Leu Pro Val Ser Ile Asn Phe Trp Val Pro Val Glu
                   965                 970                 975
   Leu Asn Gln Glu Ala Val Trp Met Asp Val Glu Val Ser His Pro Gln
               980                 985                 990
   Asn Pro Ser Leu Arg Cys Ser Ser Glu Lys Ile Ala Pro Pro Ala Ser
   995 1000 1005
   Asp Phe Leu Ala His Ile Gln Lys Asn Pro Val Leu Asp Cys Ser
   1010 1015 1020
   Ile Ala Gly Cys Leu Arg Phe Arg Cys Asp Val Pro Ser Phe Ser
   1025 1030 1035
   Val Gln Glu Glu Leu Asp Phe Thr Leu Lys Gly Asn Leu Ser Phe
   1040 1045 1050
   Gly Trp Val Arg Gln Ile Leu Gln Lys Lys Val Ser Val Val Ser
   1055 1060 1065
   Val Ala Glu Ile Thr Phe Asp Thr Ser Val Tyr Ser Gln Leu Pro
   1070 1075 1080
   Gly Gln Glu Ala Phe Met Arg Ala Gln Thr Thr Thr Val Leu Glu
   1085 1090 1095
   Lys Tyr Lys Val His Asn Pro Thr Pro Leu Ile Val Gly Ser Ser
   1100 1105 1110
   Ile Gly Gly Leu Leu Leu Leu Ala Leu Ile Thr Ala Val Leu Tyr
   1115 1120 1125
   Lys Val Gly Phe Phe Lys Arg Gln Tyr Lys Glu Met Met Glu Glu
   1130 1135 1140
   Ala Asn Gly Gln Ile Ala Pro Glu Asn Gly Thr Gln Thr Pro Ser
   1145 1150 1155
   Pro Pro Ser Glu Lys
   1160
<210> 11
   <211> 351
   <212> PRT
   <213> Homo sapiens
<400> 11
   Met Glu Thr Asn Phe Ser Thr Pro Leu Asn Glu Tyr Glu Glu Val Ser
   1 5 10 15
   Tyr Glu Ser Ala Gly Tyr Thr Val Leu Arg Ile Leu Pro Leu Val Val
   20 25 30
   Leu Gly Val Thr Phe Val Leu Gly Val Leu Gly Asn Gly Leu Val Ile
           35                  40                  45
   Trp Val Ala Gly Phe Arg Met Thr Arg Thr Val Thr Thr Ile Cys Tyr
       50                  55                  60
   Leu Asn Leu Ala Leu Ala Asp Phe Ser Phe Thr Ala Thr Leu Pro Phe
   65                  70                  75                  80
   Leu Ile Val Ser Met Ala Met Gly Glu Lys Trp Pro Phe Gly Trp Phe
                   85                  90                  95
   Leu Cys Lys Leu Ile His Ile Val Val Asp Ile Asn Leu Phe Gly Ser
               100                 105                 110
   Val Phe Leu Ile Gly Phe Ile Ala Leu Asp Arg Cys Ile Cys Val Leu
           115                 120                 125
   His Pro Val Trp Ala Gln Asn His Arg Thr Val Ser Leu Ala Met Lys
       130                 135                 140
   Val Ile Val Gly Pro Trp Ile Leu Ala Leu Val Leu Thr Leu Pro Val
   145                 150                 155                 160
   Phe Leu Phe Leu Thr Thr Val Thr Ile Pro Asn Gly Asp Thr Tyr Cys
                   165                 170                 175
   Thr Phe Asn Phe Ala Ser Trp Gly Gly Thr Pro Glu Glu Arg Leu Lys
               180                 185                 190
   Val Ala Ile Thr Met Leu Thr Ala Arg Gly Ile Ile Arg Phe Val Ile
           195                 200                 205
   Gly Phe Ser Leu Pro Met Ser Ile Val Ala Ile Cys Tyr Gly Leu Ile
       210                 215                 220
   Ala Ala Lys Ile His Lys Lys Gly Met Ile Lys Ser Ser Arg Pro Leu
   225                 230                 235                 240
   Arg Val Leu Thr Ala Val Val Ala Ser Phe Phe Ile Cys Trp Phe Pro
                   245                 250                 255
   Phe Gln Leu Val Ala Leu Leu Gly Thr Val Trp Leu Lys Glu Met Leu
               260                 265                 270
   Phe Tyr Gly Lys Tyr Lys Ile Ile Asp Ile Leu Val Asn Pro Thr Ser
           275                 280                 285
   Ser Leu Ala Phe Phe Asn Ser Cys Leu Asn Pro Met Leu Tyr Val Phe
       290                 295                 300
   Val Gly Gln Asp Phe Arg Glu Arg Leu Ile His Ser Leu Pro Thr Ser
   305                 310                 315                 320
   Leu Glu Arg Ala Leu Ser Glu Asp Ser Ala Pro Thr Asn Asp Thr Ala
                   325                 330                 335
   Ala Asn Ser Ala Ser Pro Pro Ala Glu Thr Glu Leu Gln Ala Met
               340                 345                 350
<210> 12
   <211> 317
   <212> PRT
   <213> Homo sapiens
<400> 12
   Met Thr Met Glu Thr Gln Met Ser Gln Asn Val Cys Pro Arg Asn Leu
   1               5                   10                  15
   Trp Leu Leu Gln Pro Leu Thr Val Leu Leu Leu Leu Ala Ser Ala Asp
               20                  25                  30
   Ser Gln Ala Ala Ala Pro Pro Lys Ala Val Leu Lys Leu Glu Pro Pro
           35                  40                  45
   Trp Ile Asn Val Leu Gln Glu Asp Ser Val Thr Leu Thr Cys Gln Gly
       50                  55                  60
   Ala Arg Ser Pro Glu Ser Asp Ser Ile Gln Trp Phe His Asn Gly Asn
   65                  70                  75                  80
   Leu Ile Pro Thr His Thr Gln Pro Ser Tyr Arg Phe Lys Ala Asn Asn
                   85                  90                  95
   Asn Asp Ser Gly Glu Tyr Thr Cys Gln Thr Gly Gln Thr Ser Leu Ser
               100                 105                 110
   Asp Pro Val His Leu Thr Val Leu Ser Glu Trp Leu Val Leu Gln Thr
           115                 120                 125
   Pro His Leu Glu Phe Gln Glu Gly Glu Thr Ile Met Leu Arg Cys His
       130                 135                 140
   Ser Trp Lys Asp Lys Pro Leu Val Lys Val Thr Phe Phe Gln Asn Gly
   145                 150                 155                 160
   Lys Ser Gln Lys Phe Ser His Leu Asp Pro Thr Phe Ser Ile Pro Gln
                   165                 170                 175
   Ala Asn His Ser His Ser Gly Asp Tyr His Cys Thr Gly Asn Ile Gly
               180                 185                 190
   Tyr Thr Leu Phe Ser Ser Lys Pro Val Thr Ile Thr Val Gln Val Pro
           195                 200                 205
   Ser Met Gly Ser Ser Ser Pro Met Gly Ile Ile Val Ala Val Val Ile
       210                 215                 220
   Ala Thr Ala Val Ala Ala Ile Val Ala Ala Val Val Ala Leu Ile Tyr
   225                 230                 235                 240
   Cys Arg Lys Lys Arg Ile Ser Ala Asn Ser Thr Asp Pro Val Lys Ala
                   245                 250                 255
   Ala Gln Phe Glu Pro Pro Gly Arg Gln Met Ile Ala Ile Arg Lys Arg
               260                 265                 270
   Gln Leu Glu Glu Thr Asn Asn Asp Tyr Glu Thr Ala Asp Gly Gly Tyr
           275                 280                 285
   Met Thr Leu Asn Pro Arg Ala Pro Thr Asp Asp Asp Lys Asn Ile Tyr
       290                 295                 300
   Leu Thr Leu Pro Pro Asn Asp His Val Asn Ser Asn Asn
   305                 310                 315
<210> 13
   <211> 290
   <212> PRT
   <213> Homo sapiens
<400> 13
   Met Gly Ile Leu Ser Phe Leu Pro Val Leu Ala Thr Glu Ser Asp Trp
   1               5                   10                  15
   Ala Asp Cys Lys Ser Pro Gln Pro Trp Gly His Met Leu Leu Trp Thr
               20                  25                  30
   Ala Val Leu Phe Leu Ala Pro Val Ala Gly Thr Pro Ala Pro Pro Lys
           35                  40                  45
   Ala Val Leu Lys Leu Glu Pro Gln Trp Ile Asn Val Leu Gln Glu Asp
       50                  55                  60
   Ser Val Thr Leu Thr Cys Arg Gly Thr His Ser Pro Glu Ser Asp Ser
   65                  70                  75                  80
   Ile Gln Trp Phe His Asn Gly Asn Leu Ile Pro Thr His Thr Gln Pro
                   85                  90                  95
   Ser Tyr Arg Phe Lys Ala Asn Asn Asn Asp Ser Gly Glu Tyr Thr Cys
               100                 105                 110
   Gln Thr Gly Gln Thr Ser Leu Ser Asp Pro Val His Leu Thr Val Leu
           115                 120                 125
   Ser Glu Trp Leu Val Leu Gln Thr Pro His Leu Glu Phe Gln Glu Gly
       130                 135                 140
   Glu Thr Ile Val Leu Arg Cys His Ser Trp Lys Asp Lys Pro Leu Val
   145                 150                 155                 160
   Lys Val Thr Phe Phe Gln Asn Gly Lys Ser Lys Lys Phe Ser Arg Ser
                   165                 170                 175
   Asp Pro Asn Phe Ser Ile Pro Gln Ala Asn His Ser His Ser Gly Asp
               180                 185                 190
   Tyr His Cys Thr Gly Asn Ile Gly Tyr Thr Leu Tyr Ser Ser Lys Pro
           195                 200                 205
   Val Thr Ile Thr Val Gln Ala Pro Ser Ser Ser Pro Met Gly Ile Ile
       210                 215                 220
   Val Ala Val Val Thr Gly Ile Ala Val Ala Ala Ile Val Ala Ala Val
   225                 230                 235                 240
   Val Ala Leu Ile Tyr Cys Arg Lys Lys Arg Ile Ser Ala Asn Pro Thr
                   245                 250                 255
   Asn Pro Asp Glu Ala Asp Lys Val Gly Ala Glu Asn Thr Ile Thr Tyr
               260                 265                 270
   Ser Leu Leu Met His Pro Asp Ala Leu Glu Glu Pro Asp Asp Gln Asn
           275                 280                 285
   Arg Ile
       290
<210> 14
   <211> 1479
   <212> DNA
   <213> Homo sapiens
<400> 14
   gtcaggttca tggttacgaa gctgctgacc ccaggatccc agcccgtggg agagaagggg      60
   gtctctgaca gcccccaccc ctccccactg ccagatcctt attgggtctg agtttcaggg     120
   gtggggcccc agctggaggt tataaaacag ctcaatcggg gagtacaacc ttcggtttct     180
   cttcggggaa agctgctttc agcgcacacg ggaagatatc agaaacatcc taggatcagg     240
   acaccccaga tcttctcaac tggaaccacg aaggctgttt cttccacaca gtactttgat     300
   ctccatttaa gcaggcacct ctgtcctgcg ttccggagct gcgttcccga tggtcctcct     360
   ttggctcacg ctgctcctga tcgccctgcc ctgtctcctg caaacgaagg aaggtgggaa     420
   gccttgggca ggtgcggaga atctgacctg ctggattcat gacgtggatt tcttgagctg     480
   cagctgggcg gtaggcccgg gggcccccgc ggacgtccag tacgacctgt acttgaacgt     540
   tgccaacagg cgtcaacagt acgagtgtct tcactacaaa acggatgctc agggaacacg     600
   tatcgggtgt cgtttcgatg acatctctcg actctccagc ggttctcaaa gttcccacat     660
   cctggtgcgg ggcaggagcg cagccttcgg tatcccctgc acagataagt ttgtcgtctt     720
   ttcacagatt gagatattaa ctccacccaa catgactgca aagtgtaata agacacattc     780
   ctttatgcac tggaaaatga gaagtcattt caatcgcaaa tttcgctatg agcttcagat     840
   acaaaagaga atgcagcctg taatcacaga acaggtcaga gacagaacct ccttccagct     900
   actcaatcct ggaacgtaca cagtacaaat aagagcccgg gaaagagtgt atgaattctt     960
   gagcgcctgg agcacccccc agcgcttcga gtgcgaccag gaggagggcg caaacacacg    1020
   tgcctggcgg acgtcgctgc tgatcgcgct ggggacgctg ctggccctgg tctgtgtctt    1080
   cgtgatctgc agaaggtatc tggtgatgca gagactcttt ccccgcatcc ctcacatgaa    1140
   agaccccatc ggtgacagct tccaaaacga caagctggtg gtctgggagg cgggcaaagc    1200
   cggcctggag gagtgtctgg tgactgaagt acaggtcgtg cagaaaactt gagactgggg    1260
   ttcagggctt gtgggggtct gcctcaatct ccctggccgg gccaggcgcc tgcacagact    1320
   ggctgctgga cctgcgcacg cagcccagga atggacattc ctaacgggtg gtgggcatgg    1380
   gagatgcctg tgtaatttcg tccgaagctg ccaggaagaa gaacagaact ttgtgtgttt    1440
   atttcatgat aaagtgattt tttttttttt aacccaaaa                           1479
<210> 15
   <211> 1455
   <212> DNA
   <213> Homo sapiens
<400> 15
   gccgagccag ccccttcacc accagccggc cgcgccccgg gaagggaagt ttgtggcgga 60
   ggaggttcgt acgggaggag ggggaggcgc ccacgcatct ggggctgact cgctctttcg 120
   caaaacgtct gggaggagtc cctggggcca caaaactgcc tccttcctga ggccagaagg 180
   agagaagacg tgcagggacc ccgcgcacag gagctgccct cgcgacatgg gtcacccgcc 240
   gctgctgccg ctgctgctgc tgctccacac ctgcgtccca gcctcttggg gcctgcggtg 300
   catgcagtgt aagaccaacg gggattgccg tgtggaagag tgcgccctgg gacaggacct 360
   ctgcaggacc acgatcgtgc gcttgtggga agaaggagaa gagctggagc tggtggagaa 420
   aagctgtacc cactcagaga agaccaacag gaccctgagc tatcggactg gcttgaagat 480
   caccagcctt accgaggttg tgtgtgggtt agacttgtgc aaccagggca actctggccg 540
   ggctgtcacc tattcccgaa gccgttacct cgaatgcatt tcctgtggct catcagacat 600
   gagctgtgag aggggccggc accagagcct gcagtgccgc agccctgaag aacagtgcct 660
   ggatgtggtg acccactgga tccaggaagg tgaagaaggg cgtccaaagg atgaccgcca 720
   cctccgtggc tgtggctacc ttcccggctg cccgggctcc aatggtttcc acaacaacga 780
   caccttccac ttcctgaaat gctgcaacac caccaaatgc aacgagggcc caatcctgga 840
   gcttgaaaat ctgccgcaga atggccgcca gtgttacagc tgcaagggga acagcaccca 900
   tggatgctcc tctgaagaga ctttcctcat tgactgccga ggccccatga atcaatgtct 960
   ggtagccacc ggcactcacg aacgctcact ctggggaagc tggttgccat gtaaaagtac 1020
   tactgccctg agaccaccat gctgtgagga agcccaagct actcatgtat aaatgccatg 1080
   tggagataga gccccagatg tttcagccat ctcagcccag gcaccagaca agtgggtgaa 1140
   gaagccacct tggacatgta gccccagcag atgtgatata gagaagaaac aggaaacttg 1200
   gctatattag tttcctaggg ctgcctgtga taaattatta caaactttat aaactaacac 1260
   attgtgtgcc tatatcaaaa catcatggaa ggacaggcac agtggctcat gcctgtagtc 1320
   ctagcacttt gggagggtga gaaaggaaga tctcttgagc tcaggagttc aagatcagcc 1380
   tgggcaacac agtgagacct catctccact aaaaataaaa aaaaattggc tggaaaaaaa 1440
   aaaaaaaaaa aaaaa 1455
<210> 16
   <211> 2618
   <212> DNA
   <213> Homo sapiens
<400> 16
   tccacccaag agcaacctgg aactaagtta ttcggcaacg aactgttcca ctttgttgtg      60
   aggcaataga tgtggaaatt ccctgacgag gggctctgtc ctcatacttc ctgcggagct     120
   tattgtcgta agaatatctg tcatcctgct aatgtgcatt gaaaggagag caacggggct     180
   gaggccgtgt cagcacgatg gaccccaaac agaccaccct cctgtgtctt gtgctctgtc     240
   tgggccagag gattcaggca caggaagggg actttcccat gcctttcata tctgccaaat 300
   cgagtcctgt gattcccttg gatggatctg tgaaaatcca gtgccaggcc attcgtgaag 360
   cttacctgac ccagctgatg atcataaaaa actccacgta ccgagagata ggcagaagac 420
   tgaagttttg gaatgagact gatcctgagt tcgtcattga ccacatggac gcaaacaagg 480
   cagggcgcta tcagtgccaa tataggatag ggcactacag gttccggtac agtgacaccc 540
   tggagctggt agtgacaggc ttgtatggca aacccttcct ctctgcagat cggggtctgg 600
   tgttgatgcc aggagagaat atttccctca cgtgcagctc agcacacatc ccatttgata 660
   gattttcact ggccaaggag ggagaacttt ctctgccaca gcaccaaagt ggggaacacc 720
   cggccaactt ctctttgggt cctgtggacc tcaatgtctc agggatctac aggtgctacg 780
   gttggtacaa caggagcccc tacctgtggt ccttccccag taatgccttg gagcttgtgg 840
   tcacagactc catccaccaa gattacacga cgcagaactt gatccgcatg gccgtggcag 900
   gactggtcct cgtggctctc ttggccatac tggttgaaaa ttggcacagc catacggcac 960
   tgaacaagga agcctcggca gatgtggctg aaccgagctg gagccaacag atgtgtcagc 1020
   caggattgac ctttgcacga acaccaagtg tctgcaagta aacacctgga ggtgaaggca 1080
   gagaggagcc aggactgtgg agtccgacaa agctacttga aggacacaag agagaaaagc 1140
   tcactaagaa gcttgaatct actttttttt ttttttgaga cagagtctgg ctctgtcacc 1200
   caggctggag tgcagtggag caatctcggc tcattgaacc tcttgggttc aagtgattct 1260
   tgtgcctcag cctcccaagt agctggaatt acaggcacat accactgcac ccagctaatt 1320
   tttgtatttt tagtagagat ggggtttcac tgtgttggcc aggctggtct cgaactcctg 1380
   acctcaggtg atccacccac cttggcctcc caaagtgctg agattatagg catgagccac 1440
   cacgcctggc cagatgcatg ttcaaaccaa tcaaatggtg ttttcttatg caggactgat 1500
   cgatttgcac ccacctttct gcacataagt tatggttttc catcttatct gtcttctgat 1560
   tttttatatc ctgtttaatt tcttccttca ttgttcttct ctttttttat ttattttatt 1620
   tatttttatt tttattttta tttgagacag agtctcactc tgttgcccag gctggagtgc 1680
   agtggcacga tctcggctca ctgcaacctc tgcctcctgg gttcaagtga ttctcctgcc 1740
   tcggcctccc aagtagctgg gattgcaggc tcccaccatc acgcccagct acttttacag 1800
   tatttttagt agagacgggg tttcatcaca ttggccaagc tggtctcaaa cttctgacct 1860
   cgtgatctgc ccgcctcggc ctcccaaagt gctgggatta cagatgtgag ccactgcgcc 1920
   cagccttctt tttatatttt taaatgtgtc ttccccaaat ataaatggtt ggtaagcatg 1980
   ccaaatatat tcaataaccc ccctccttta tttttttttg ttgaagtgag gctctcccta 2040
   tgttgcctaa gctggtcttg aactcctggt ctcaagcaat cctcctacct cagcctcctg 2100
   ctgtgttcat ctacaaattg ataagagtga aagtcataat cctacaggag gattacccta 2160
   tttatttcac aaaccctatt tctaccggat tttcatacaa ggaatacagg catgtgtttc 2220
   acctcattaa tttatttttt cacttagttt tgatgatatt cacatatatt atcaagtgtg 2280
   caaacattaa attcttgtgt acaaaactca aatggtcttc caaataattc cccattcttt 2340
   tttcttataa actttcacag ctttaccctt gacagacttt actcaaggaa atctaagttg 2400
   gtcatatgtg gctctttcac tgattgctat ttacttcatt gtccagtagc ttatgtatga 2460
   aaatataatt ataaaatgta agggtcctac ttccagtgaa actgaaggga cttaggccca 2520
   cttttatcct ttactgagag cttatctcta cttgataaaa tttctactgt attcttggct 2580
   taactcaggt cctgtgatta aaaaaaaaat gcaaagta 2618
<210> 17
   <211> 4201
   <212> DNA
   <213> Homo sapiens
<400> 17
   tccggcccgc acccaccccc aagaggggcc ttcagctttg gggctcagag gcacgacctc 60
   ctggggaggg ttaaaaggca gacgcccccc cgccccccgc gcccccgcgc cccgactcct 120
   tcgccgcctc cagcctctcg ccagtgggaa gcggggagca gccgcgcggc cggagtccgg 180
   aggcgagggg aggtcggccg caacttcccc ggtccacctt aagaggacga tgtagccagc 240
   tcgcagcgct gaccttagaa aaacaagttt gcgcaaagtg gagcggggac ccggcctctg 300
   ggcagccccg gcggcgcttc cagtgccttc cagccctcgc gggcggcgca gccgcggccc 360
   atggagcccg ccggcccggc ccccggccgc ctcgggccgc tgctctgcct gctgctcgcc 420
   gcgtcctgcg cctggtcagg agtggcgggt gaggaggagc tgcaggtgat tcagcctgac 480
   aagtccgtgt tggttgcagc tggagagaca gccactctgc gctgcactgc gacctctctg 540
   atccctgtgg ggcccatcca gtggttcaga ggagctggac caggccggga attaatctac 600
   aatcaaaaag aaggccactt cccccgggta acaactgttt cagacctcac aaagagaaac 660
   aacatggact tttccatccg catcggtaac atcaccccag cagatgccgg cacctactac 720
   tgtgtgaagt tccggaaagg gagccccgat gacgtggagt ttaagtctgg agcaggcact 780
   gagctgtctg tgcgcgccaa accctctgcc cccgtggtat cgggccctgc ggcgagggcc 840
   acacctcagc acacagtgag cttcacctgc gagtcccacg gcttctcacc cagagacatc 900
   accctgaaat ggttcaaaaa tgggaatgag ctctcagact tccagaccaa cgtggacccc 960
   gtaggagaga gcgtgtccta cagcatccac agcacagcca aggtggtgct gacccgcgag 1020
   gacgttcact ctcaagtcat ctgcgaggtg gcccacgtca ccttgcaggg ggaccctctt 1080
   cgtgggactg ccaacttgtc tgagaccatc cgagttccac ccaccttgga ggttactcaa 1140
   cagcccgtga gggcagagaa ccaggtgaat gtcacctgcc aggtgaggaa gttctacccc 1200
   cagagactac agctgacctg gttggagaat ggaaacgtgt cccggacaga aacggcctca 1260
   accgttacag agaacaagga tggtacctac aactggatga gctggctcct ggtgaatgta 1320
   tctgcccaca gggatgatgt gaagctcacc tgccaggtgg agcatgacgg gcagccagcg 1380
   gtcagcaaaa gccatgacct gaaggtctca gcccacccga aggagcaggg ctcaaatacc 1440
   gccgctgaga acactggatc taatgaacgg aacatctata ttgtggtggg tgtggtgtgc 1500
   accttgctgg tggccctact gatggcggcc ctctacctcg tccgaatcag acagaagaaa 1560
   gcccagggct ccacttcttc tacaaggttg catgagcccg agaagaatgc cagagaaata 1620
   acacaggaca caaatgatat cacatatgca gacctgaacc tgcccaaggg gaagaagcct 1680
   gctccccagg ctgcggagcc caacaaccac acggagtatg ccagcattca gaccagcccg 1740
   cagcccgcgt cggaggacac cctcacctat gctgacctgg acatggtcca cctcaaccgg 1800
   acccccaagc agccggcccc caagcctgag ccgtccttct cagagtacgc cagcgtccag 1860
   gtcccgagga agtgaatggg accgtggttt gctctagcac ccatctctac gcgctttctt 1920
   gtcccacagg gagccgccgt gatgagcaca gccaacccag ttcccggagg gctggggcgg 1980
   tgcaggctct gggacccagg ggccagggtg gctcttctct ccccacccct ccttggctct 2040
   ccagcacttc ctgggcagcc acggccccct ccccccacat tgccacatac ctggaggctg 2100
   acgttgccaa accagccagg gaaccaacct gggaagtggc cagaactgcc tggggtccaa 2160
   gaactcttgt gcctccgtcc atcaccatgt gggttttgaa gaccctcgac tgcctccccg 2220
   atgctccgaa gcctgatctt ccagggtggg gaggagaaaa tcccacctcc cctgacctcc 2280
   accacctcca ccaccaccac caccaccacc accaccacta ccaccaccac ccaactgggg 2340
   ctagagtggg gaagatttcc cctttagatc aaactgcccc ttccatggaa aagctggaaa 2400
   aaaactctgg aacccatatc caggcttggt gaggttgctg ccaacagtcc tggcctcccc 2460
   catccctagg ctaaagagcc atgagtcctg gaggaggaga ggacccctcc caaaggactg 2520
   gagacaaaac cctctgcttc cttgggtccc tccaagactc cctggggccc aactgtgttg 2580
   ctccacccgg acccatctct cccttctaga cctgagcttg cccctccagc tagcactaag 2640
   caacatctcg ctgtggacgc ctgtaaatta ctgagaaatg tgaaacgtgc aatcttgaaa 2700
   ctgaggtgtt agaaaacttg atctgtggtg ttttgttttg ttttttttct taaaacaaca 2760
   gcaacgtgat cttggctgtc tgtcatgtgt tgaagtccat ggttgggtct tgtgaagtct 2820
   gaggtttaac agtttgttgt cctggaggga ttttcttaca gcgaagactt gagttcctcc 2880
   aagtcccaga accccaagaa tgggcaagaa ggatcaggtc agccactccc tggagacaca 2940
   gccttctggc tgggactgac ttggccatgt tctcagctga gccacgcggc tggtagtgca 3000
   gccttctgtg accccgctgt ggtaagtcca gcctgcccag ggctgctgag ggctgcctct 3060
   tgacagtgca gtcttatcga gacccaatgc ctcagtctgc tcatccgtaa agtggggata 3120
   gtgaagatga cacccctccc caccacctct cataagcact ttaggaacac acagagggta 3180
   gggatagtgg ccctggccgt ctatcctacc cctttagtga ccgcccccat cccggctttc 3240
   tgagctgatc cttgaagaag aaatcttcca tttctgctct caaaccctac tgggatcaaa 3300
   ctggaataaa ttgaagacag ccagggggat ggtgcagctg tgaagctcgg gctgattccc 3360
   cctctgtccc agaaggttgg ccagagggtg tgacccagtt accctttaac ccccaccctt 3420
   ccagtcgggt gtgagggcct gaccgggccc agggcaagca gatgtcgcaa gccctattta 3480
   ttcagtcttc actataactc ttagagttga gacgctaatg ttcatgactc ctggccttgg 3540
   gatgcccaag ggatttctgg ctcaggctgt aaaagtagct gagccatcct gcccattcct 3600
   ggaggtccta caggtgaaac tgcaggagct cagcatagac ccagctctct gggggatggt 3660
   cacctggtga tttcaatgat ggcatccagg aattagctga gccaacagac catgtggaca 3720
   gctttggcca gagctcccgt gtggcatctg ggagccacag tgacccagcc acctggctca 3780
   ggctagttcc aaattccaaa agattggctt gtaaaccttc gtctccctct cttttaccca 3840
   gagacagcac atacgtgtgc acacgcatgc acacacacat tcagtatttt aaaagaatgt 3900
   tttcttggtg ccattttcat tttattttat tttttaattc ttggaggggg aaataaggga 3960
   ataaggccaa ggaagatgta tagctttagc tttagcctgg caacctggag aatccacata 4020
   ccttgtgtat tgaaccccag gaaaaggaag aggtcgaacc aaccctgcgg aaggagcatg 4080
   gtttcaggag tttattttaa gactgctggg aaggaaacag gccccatttt gtatatagtt 4140
   gcaacttaaa ctttttggct tgcaaaatat ttttgtaata aagatttctg ggtaataatg 4200
   a 4201
<210> 18
   <211> 2869
   <212> DNA
   <213> Homo sapiens
<400> 18
   agaattcttt ggcaggggcg accttagaat cctggggagg agcgagaatg gaatcccggg 60
   gaggaacagg ggtggaatcc ggggggcggg gtcagaacgc caggaggggg cggggccgga 120
   gccagggtcg gcttgactcg ggggagcagc gggtggatcc tgtgacgtca gcgggttcga 180
   accgccggag ctgagcgaga ggccgggggt gccgagccgg gcggggagag ctgggccggg 240
   agagcagaac agggaggcta gagcgcagcg ggaaccggcc cggagccgga gccggagccc 300
   cacaggcacc tactaaaccg cccagccgat cggcccccac agagtggccc gcgggcctcc 360
   ggccgggccc agtcccctcc cgggccctcc atggcccggg ccgctgccct cctgccgtcg 420
   agatcgccgc cgacgccgct gctgtggccg ctgctgctgc tgctgctcct ggaaaccgga 480
   gcccaggatg tgcgagttca agtgctaccc gaggtgcgag gccagctcgg gggcaccgtg 540
   gagctgccgt gccacctgct gccacctgtt cctggactgt acatctccct ggtgacctgg 600
   cagcgcccag atgcacctgc gaaccaccag aatgtggccg ccttccaccc taagatgggt 660
   cccagcttcc ccagcccgaa gcctggcagc gagcggctgt ccttcgtctc tgccaagcag 720
   agcactgggc aagacacaga ggcagagctc caggacgcca cgctggccct ccacgggctc 780
   acggtggagg acgagggcaa ctacacttgc gagtttgcca ccttccccaa ggggtccgtc 840
   cgagggatga cctggctcag agtcatagcc aagcccaaga accaagctga ggcccagaag 900
   gtcacgttca gccaggaccc tacgacagtg gccctctgca tctccaaaga gggccgccca 960
   cctgcccgga tctcctggct ctcatccctg gactgggaag ccaaagagac tcaggtgtca 1020
   gggaccctgg ccggaactgt cactgtcacc agccgcttca ccttggtgcc ctcgggccga 1080
   gcagatggtg tcacggtcac ctgcaaagtg gagcatgaga gcttcgagga accagccctg 1140
   atacctgtga ccctctctgt acgctaccct cctgaagtgt ccatctccgg ctatgatgac 1200
   aactggtacc tcggccgtac tgatgccacc ctgagctgtg acgtccgcag caacccagag 1260
   cccacgggct atgactggag cacgacctca ggcaccttcc cgacctccgc agtggcccag 1320
   ggctcccagc tggtcatcca cgcagtggac agtctgttca ataccacctt cgtctgcaca 1380
   gtcaccaatg ccgtgggcat gggccgcgct gagcaggtca tctttgtccg agagaccccc 1440
   aacacagcag gcgcaggggc cacaggcggc atcatcgggg gcatcatcgc cgccatcatt 1500
   gctactgctg tggctgccac gggcatcctt atctgccggc agcagcggaa ggagcagacg 1560
   ctgcaggggg cagaggagga cgaagacctg gagggacctc cctcctacaa gccaccgacc 1620
   ccaaaagcga agctggaggc acaggagatg ccctcccagc tcttcactct gggggcctcg 1680
   gagcacagcc cactcaagac cccctacttt gatgctggcg cctcatgcac tgagcaggaa 1740
   atgcctcgat accatgagct gcccaccttg gaagaacggt caggaccctt gcaccctgga 1800
   gccacaagcc tggggtcccc catcccggtg cctccagggc cacctgctgt ggaagacgtt 1860
   tccctggatc tagaggatga ggagggggag gaggaggaag agtatctgga caagatcaac 1920
   cccatctatg atgctctgtc ctatagcagc ccctctgatt cctaccaggg caaaggcttt 1980
   gtcatgtccc gggccatgta tgtgtgagct gccatgcgcc tggcgtctca catctcacct 2040
   gttgatccct tagctttctt gccaaggatc tagtgccccc tgacctctgg ccaggccact 2100
   gtcagttaac acatatgcat tccatttgtg atgtctacct tggtggctcc actatgaccc 2160
   ctaacccatg agcccagaga aattcaccgt gataatggaa tcctggcaac cttatctcat 2220
   gaggcaggag gtggggaagg tgcttctgca caacctctga tcccaaggac tcctctccca 2280
   gactgtgacc ttagaccata cctctcaccc cccaatgcct cgactccccc aaaatcacaa 2340
   agaagaccct agacctataa tttgtcttca ggtagtaaat tcccaatagg tctgctggag 2400
   tgggcgctga gggctccctg ctgctcagac ctgagccctc caggcagcag ggtcccactt 2460
   accccctccc caccctgttc cccaaaggtg ggaaagaggg gattccccag cccaaggcag 2520
   ggttttccca gcaccctcct gtaagcagaa gtctcagggt ccagaccctt ccctgagccc 2580
   ccacccccac cccaattcct gcctaccaag caagcagccc cagcctaggg tcagacaggg 2640
   tgagcctcat acagactgtg ccttgatggc cccagccttg ggagaagaat ttactgttaa 2700
   cctggaagac tactgaatca ttttaccctt gcccagtgga ataggaccta aacatccccc 2760
   ttccggggaa agtgggtcat ctgaattggg ggtagcaatt gatactgttt tgtaaactac 2820
   atttcctaca aaatatgaat ttatactttg accaggaaaa aaaaaaaaa 2869
<210> 19
   <211> 4006
   <212> DNA
   <213> Homo sapiens
<400> 19
   tgccaaggct ccagcccggc cgggctccga ggcgagaggc tgcatggagt ggccggcgcg      60
   gctctgcggg ctgtgggcgc tgctgctctg cgccggcggc gggggcgggg gcgggggcgc     120
   cgcgcctacg gaaactcagc cacctgtgac aaatttgagt gtctctgttg aaaacctctg     180
   cacagtaata tggacatgga atccacccga gggagccagc tcaaattgta gtctatggta     240
   ttttagtcat tttggcgaca aacaagataa gaaaatagct ccggaaactc gtcgttcaat     300
   agaagtaccc ctgaatgaga ggatttgtct gcaagtgggg tcccagtgta gcaccaatga     360
   gagtgagaag cctagcattt tggttgaaaa atgcatctca cccccagaag gtgatcctga     420
   gtctgctgtg actgagcttc aatgcatttg gcacaacctg agctacatga agtgttcttg     480
   gctccctgga aggaatacca gtcccgacac taactatact ctctactatt ggcacagaag     540
   cctggaaaaa attcatcaat gtgaaaacat ctttagagaa ggccaatact ttggttgttc     600
   ctttgatctg accaaagtga aggattccag ttttgaacaa cacagtgtcc aaataatggt     660
   caaggataat gcaggaaaaa ttaaaccatc cttcaatata gtgcctttaa cttcccgtgt     720
   gaaacctgat cctccacata ttaaaaacct ctccttccac aatgatgacc tatatgtgca     780
   atgggagaat ccacagaatt ttattagcag atgcctattt tatgaagtag aagtcaataa     840
   cagccaaact gagacacata atgttttcta cgtccaagag gctaaatgtg agaatccaga     900
   atttgagaga aatgtggaga atacatcttg tttcatggtc cctggtgttc ttcctgatac     960
   tttgaacaca gtcagaataa gagtcaaaac aaataagtta tgctatgagg atgacaaact    1020
   ctggagtaat tggagccaag aaatgagtat aggtaagaag cgcaattcca cactctacat    1080
   aaccatgtta ctcattgttc cagtcatcgt cgcaggtgca atcatagtac tcctgcttta    1140
   cctaaaaagg ctcaagatta ttatattccc tccaattcct gatcctggca agatttttaa 1200
   agaaatgttt ggagaccaga atgatgatac tctgcactgg aagaagtacg acatctatga 1260
   gaagcaaacc aaggaggaaa ccgactctgt agtgctgata gaaaacctga agaaagcctc 1320
   tcagtgatgg agataattta tttttacctt cactgtgacc ttgagaagat tcttcccatt 1380
   ctccatttgt tatctgggaa cttattaaat ggaaactgaa actactgcac catttaaaaa 1440
   caggcagctc ataagagcca caggtcttta tgttgagtcg cgcaccgaaa aactaaaaat 1500
   aatgggcgct ttggagaaga gtgtggagtc attctcattg aattataaaa gccagcaggc 1560
   ttcaaactag gggacaaagc aaaaagtgat gatagtggtg gagttaatct tatcaagagt 1620
   tgtgacaact tcctgaggga tctatacttg ctttgtgttc tttgtgtcaa catgaacaaa 1680
   ttttatttgt aggggaactc atttggggtg caaatgctaa tgtcaaactt gagtcacaaa 1740
   gaacatgtag aaaacaaaat ggataaaatc tgatatgtat tgtttgggat cctattgaac 1800
   catgtttgtg gctattaaaa ctcttttaac agtctgggct gggtccggtg gctcacgcct 1860
   gtaatcccag caatttggga gtccgaggcg ggcggatcac tcgaggtcag gagttccaga 1920
   ccagcctgac caaaatggtg aaacctcctc tctactaaaa ctacaaaaat taactgggtg 1980
   tggtggcgcg tgcctgtaat cccagctact cgggaagctg aggcaggtga attgtttgaa 2040
   cctgggaggt ggaggttgca gtgagcagag atcacaccac tgcactctag cctgggtgac 2100
   agagcaagac tctgtctaaa aaacaaaaca aaacaaaaca aaacaaaaaa acctcttaat 2160
   attctggagt catcattccc ttcgacagca ttttcctctg ctttgaaagc cccagaaatc 2220
   agtgttggcc atgatgacaa ctacagaaaa accagaggca gcttctttgc caagaccttt 2280
   caaagccatt ttaggctgtt aggggcagtg gaggtagaat gactccttgg gtattagagt 2340
   ttcaaccatg aagtctctaa caatgtattt tcttcacctc tgctactcaa gtagcattta 2400
   ctgtgtcttt ggtttgtgct aggcccccgg gtgtgaagca cagacccctt ccaggggttt 2460
   acagtctatt tgagactcct cagttcttgc cacttttttt tttaatctcc accagtcatt 2520
   tttcagacct tttaactcct caattccaac actgatttcc ccttttgcat tctccctcct 2580
   tcccttcctt gtagcctttt gactttcatt ggaaattagg atgtaaatct gctcaggaga 2640
   cctggaggag cagaggataa ttagcatctc aggttaagtg tgagtaatct gagaaacaat 2700
   gactaattct tgcatatttt gtaacttcca tgtgagggtt ttcagcattg atatttgtgc 2760
   attttctaaa cagagatgag gtggtatctt cacgtagaac attggtattc gcttgagaaa 2820
   aaaagaatag ttgaacctat ttctctttct ttacaagatg ggtccaggat tcctcttttc 2880
   tctgccataa atgattaatt aaatagcttt tgtgtcttac attggtagcc agccagccaa 2940
   ggctctgttt atgcttttgg ggggcatata ttgggttcca ttctcaccta tccacacaac 3000
   atatccgtat atatcccctc tactcttact tcccccaaat ttaaagaagt atgggaaatg 3060
   agaggcattt cccccacccc atttctctcc tcacacacag actcatatta ctggtaggaa 3120
   cttgagaact ttatttccaa gttgttcaaa catttaccaa tcatattaat acaatgatgc 3180
   tatttgcaat tcctgctcct aggggagggg agataagaaa ccctcactct ctacaggttt 3240
   gggtacaagt ggcaacctgc ttccatggcc gtgtagaagc atggtgccct ggcttctctg 3300
   aggaagctgg ggttcatgac aatggcagat gtaaagttat tcttgaagtc agattgaggc 3360
   tgggagacag ccgtagtaga tgttctactt tgttctgctg ttctctagaa agaatatttg 3420
   gttttcctgt ataggaatga gattaattcc tttccaggta ttttataatt ctgggaagca 3480
   aaacccatgc ctccccctag ccatttttac tgttatccta tttagatggc catgaagagg 3540
   atgctgtgaa attcccaaca aacattgatg ctgacagtca tgcagtctgg gagtggggaa 3600
   gtgatctttt gttcccatcc tcttctttta gcagtaaaat agctgaggga aaagggaggg 3660
   aaaaggaagt tatgggaata cctgtggtgg ttgtgatccc taggtcttgg gagctcttgg 3720
   aggtgtctgt atcagtggat ttcccatccc ctgtgggaaa ttagtaggct catttactgt 3780
   tttaggtcta gcctatgtgg attttttcct aacataccta agcaaaccca gtgtcaggat 3840
   ggtaattctt attctttcgt tcagttaagt ttttcccttc atctgggcac tgaagggata 3900
   tgtgaaacaa tgttaacatt tttggtagtc ttcaaccagg gattgtttct gtttaacttc 3960
   ttataggaaa gcttgagtaa aataaatatt gtctttttgt atgtca 4006
<210> 20
   <211> 3682
   <212> DNA
   <213> Homo sapiens
<400> 20
<210> 21
   <211> 5170
   <212> DNA
   <213> Homo sapiens
<400> 21
   gtggccggcg gccggagccg actcggagcg cgcggcgccg gccgggagga gccggagagc      60
   ggccgggccg ggcggtgggg gcgccggcct gccccgcgcg ccccagggag cggcaggaat     120
   gtgacaatcg cgcgcccgcg caccgaagca ctcctcgctc ggctcctagg gctctcgccc     180
   ctctgagctg agccgggttc cgcccggggc tgggatccca tcaccctcca cggccgtccg     240
   tccaggtaga cgcaccctct gaagatggtg actccctcct gagaagctgg accccttggt     300
   aaaagacaag gccttctcca agaagaatat gaaagtgtta ctcagactta tttgtttcat     360
   agctctactg atttcttctc tggaggctga taaatgcaag gaacgtgaag aaaaaataat     420
   tttagtgtca tctgcaaatg aaattgatgt tcgtccctgt cctcttaacc caaatgaaca     480
   caaaggcact ataacttggt ataaagatga cagcaagaca cctgtatcta cagaacaagc     540
   ctccaggatt catcaacaca aagagaaact ttggtttgtt cctgctaagg tggaggattc 600
   aggacattac tattgcgtgg taagaaattc atcttactgc ctcagaatta aaataagtgc 660
   aaaatttgtg gagaatgagc ctaacttatg ttataatgca caagccatat ttaagcagaa 720
   actacccgtt gcaggagacg gaggacttgt gtgcccttat atggagtttt ttaaaaatga 780
   aaataatgag ttacctaaat tacagtggta taaggattgc aaacctctac ttcttgacaa 840
   tatacacttt agtggagtca aagataggct catcgtgatg aatgtggctg aaaagcatag 900
   agggaactat acttgtcatg catcctacac atacttgggc aagcaatatc ctattacccg 960
   ggtaatagaa tttattactc tagaggaaaa caaacccaca aggcctgtga ttgtgagccc 1020
   agctaatgag acaatggaag tagacttggg atcccagata caattgatct gtaatgtcac 1080
   cggccagttg agtgacattg cttactggaa gtggaatggg tcagtaattg atgaagatga 1140
   cccagtgcta ggggaagact attacagtgt ggaaaatcct gcaaacaaaa gaaggagtac 1200
   cctcatcaca gtgcttaata tatcggaaat tgaaagtaga ttttataaac atccatttac 1260
   ctgttttgcc aagaatacac atggtataga tgcagcatat atccagttaa tatatccagt 1320
   cactaatttc cagaagcaca tgattggtat atgtgtcacg ttgacagtca taattgtgtg 1380
   ttctgttttc atctataaaa tcttcaagat tgacattgtg ctttggtaca gggattcctg 1440
   ctatgatttt ctcccaataa aagcttcaga tggaaagacc tatgacgcat atatactgta 1500
   tccaaagact gttggggaag ggtctacctc tgactgtgat atttttgtgt ttaaagtctt 1560
   gcctgaggtc ttggaaaaac agtgtggata taagctgttc atttatggaa gggatgacta 1620
   cgttggggaa gacattgttg aggtcattaa tgaaaacgta aagaaaagca gaagactgat 1680
   tatcatttta gtcagagaaa catcaggctt cagctggctg ggtggttcat ctgaagagca 1740
   aatagccatg tataatgctc ttgttcagga tggaattaaa gttgtcctgc ttgagctgga 1800
   gaaaatccaa gactatgaga aaatgccaga atcgattaaa ttcattaagc agaaacatgg 1860
   ggctatccgc tggtcagggg actttacaca gggaccacag tctgcaaaga caaggttctg 1920
   gaagaatgtc aggtaccaca tgccagtcca gcgacggtca ccttcatcta aacaccagtt 1980
   actgtcacca gccactaagg agaaactgca aagagaggct cacgtgcctc tcgggtagca 2040
   tggagaagtt gccaagagtt ctttaggtgc ctcctgtctt atggcgttgc aggccaggtt 2100
   atgcctcatg ctgacttgca gagttcatgg aatgtaacta tatcatcctt tatccctgag 2160
   gtcacctgga atcagattat taagggaata agccatgacg tcaatagcag cccagggcac 2220
   ttcagagtag agggcttggg aagatctttt aaaaaggcag taggcccggt gtggtggctc 2280
   acgcctataa tcccagcact ttgggaggct gaagtgggtg gatcaccaga ggtcaggagt 2340
   tcgagaccag cccagccaac atggcaaaac cccatctcta ctaaaaatac aaaaatgagc 2400
   taggcatggt ggcacacgcc tgtaatccca gctacacctg aggctgaggc aggagaattg 2460
   cttgaaccgg ggagacggag gttgcagtga gccgagtttg ggccactgca ctctagcctg 2520
   gcaacagagc aagactccgt ctcaaaaaaa gggcaataaa tgccctctct gaatgtttga 2580
   actgccaaga aaaggcatgg agacagcgaa ctagaagaaa gggcaagaag gaaatagcca 2640
   ccgtctacag atggcttagt taagtcatcc acagcccaag ggcggggcta tgccttgtct 2700
   ggggaccctg tagagtcact gaccctggag cggctctcct gagaggtgct gcaggcaaag 2760
   tgagactgac acctcactga ggaagggaga catattcttg gagaactttc catctgcttg 2820
   tattttccat acacatcccc agccagaagt tagtgtccga agaccgaatt ttattttaca 2880
   gagcttgaaa actcacttca atgaacaaag ggattctcca ggattccaaa gttttgaagt 2940
   catcttagct ttccacagga gggagagaac ttaaaaaagc aacagtagca gggaattgat 3000
   ccacttctta atgctttcct ccctggcatg accatcctgt cctttgttat tatcctgcat 3060
   tttacgtctt tggaggaaca gctccctagt ggcttcctcc gtctgcaatg tcccttgcac 3120
   agcccacaca tgaaccatcc ttcccatgat gccgctcttc tgtcatcccg ctcctgctga 3180
   aacacctccc aggggctcca cctgttcagg agctgaagcc catgctttcc caccagcatg 3240
   tcactcccag accacctccc tgccctgtcc tccagcttcc cctcgctgtc ctgctgtgtg 3300
   aattcccagg ttggcctggt ggccatgtcg cctgccccca gcactcctct gtctctgctc 3360
   ttgcctgcac ccttcctcct cctttgccta ggaggccttc tcgcattttc tctagctgat 3420
   cagaatttta ccaaaattca gaacatcctc caattccaca gtctctggga gactttccct 3480
   aagaggcgac ttcctctcca gccttctctc tctggtcagg cccactgcag agatggtggt 3540
   gagcacatct gggaggctgg tctccctcca gctggaattg ctgctctctg agggagaggc 3600
   tgtggtggct gtctctgtcc ctcactgcct tccaggagca atttgcacat gtaacataga 3660
   tttatgtaat gctttatgtt taaaaacatt ccccaattat cttatttaat ttttgcaatt 3720
   attctaattt tatatataga gaaagtgacc tattttttaa aaaaatcaca ctctaagttc 3780
   tattgaacct aggacttgag cctccatttc tggcttctag tctggtgttc tgagtacttg 3840
   atttcaggtc aataacggtc ccccctcact ccacactggc acgtttgtga gaagaaatga 3900
   cattttgcta ggaagtgacc gagtctagga atgcttttat tcaagacacc aaattccaaa 3960
   cttctaaatg ttggaatttt caaaaattgt gtttagattt tatgaaaaac tcttctactt 4020
   tcatctattc tttccctaga ggcaaacatt tcttaaaatg tttcattttc attaaaaatg 4080
   aaagccaaat ttatatgcca ccgattgcag gacacaagca cagttttaag agttgtatga 4140
   acatggagag gacttttggt ttttatattt ctcgtattta atatgggtga acaccaactt 4200
   ttatttggaa taataatttt cctcctaaac aaaaacacat tgagtttaag tctctgactc 4260
   ttgcctttcc acctgctttc tcctgggccc gctttgcctg cttgaaggaa cagtgctgtt 4320
   ctggagctgc tgttccaaca gacagggcct agctttcatt tgacacacag actacagcca     4380
   gaagcccatg gagcagggat gtcacgtctt gaaaagccta ttagatgttt tacaaattta     4440
   attttgcaga ttattttagt ctgtcatcca gaaaatgtgt cagcatgcat agtgctaaga     4500
   aagcaagcca atttggaaac ttaggttagt gacaaaattg gccagagagt gggggtgatg     4560
   atgaccaaga attacaagta gaatggcagc tggaatttaa ggagggacaa gaatcaatgg     4620
   ataagcgtgg gtggaggaag atccaaacag aaaagtgcaa agttattccc catcttccaa     4680
   gggttgaatt ctggaggaag aagacacatt cctagttccc cgtgaacttc ctttgactta     4740
   ttgtccccac taaaacaaaa caaaaaactt ttaatgcctt ccacattaat tagattttct     4800
   tgcagttttt ttatggcatt tttttaaaga tgccctaagt gttgaagaag agtttgcaaa     4860
   tgcaacaaaa tatttaatta ccggttgtta aaactggttt agcacaattt atattttccc     4920
   tctcttgcct ttcttatttg caataaaagg tattgagcca ttttttaaat gacatttttg     4980
   ataaattatg tttgtactag ttgatgaagg agtttttttt aacctgttta tataattttg      5040
   cagcagaagc caaatttttt gtatattaaa gcaccaaatt catgtacagc atgcatcacg     5100
   gatcaataga ctgtacttat tttccaataa aattttcaaa ctttgtactg ttaaaaaaaa     5160
   aaaaaaaaaa                                                            5170
<210> 22
   <211> 3249
   <212> DNA
   <213> Homo sapiens
<400> 22
   caagcttagc ctggccggga aacgggaggc gtggaggccg ggagcagccc ccggggtcat        60
   cgccctgcca ccgccgcccg attgctttag cttggaaatt ccggagctga agcggccagc       120
   gagggaggat gaccctctcg gcccgggcac cctgtcagtc cggaaataac tgcagcattt       180
   gttccggagg ggaaggcgcg aggtttccgg gaaagcagca ccgccccttg gcccccaggt       240
   ggctagcgct ataaaggatc acgcgcccca gtcgacgctg agctcctctg ctactcagag       300
   ttgcaacctc agcctcgcta tggctcccag cagcccccgg cccgcgctgc ccgcactcct       360
   ggtcctgctc ggggctctgt tcccaggacc tggcaatgcc cagacatctg tgtccccctc       420
   aaaagtcatc ctgccccggg gaggctccgt gctggtgaca tgcagcacct cctgtgacca       480
   gcccaagttg ttgggcatag agaccccgtt gcctaaaaag gagttgctcc tgcctgggaa       540
   caaccggaag gtgtatgaac tgagcaatgt gcaagaagat agccaaccaa tgtgctattc       600
   aaactgccct gatgggcagt caacagctaa aaccttcctc accgtgtact ggactccaga       660
   acgggtggaa ctggcacccc tcccctcttg gcagccagtg ggcaagaacc ttaccctacg       720
   ctgccaggtg gagggtgggg caccccgggc caacctcacc gtggtgctgc tccgtgggga       780
   gaaggagctg aaacgggagc cagctgtggg ggagcccgct gaggtcacga ccacggtgct 840
   ggtgaggaga gatcaccatg gagccaattt ctcgtgccgc actgaactgg acctgcggcc 900
   ccaagggctg gagctgtttg agaacacctc ggccccctac cagctccaga cctttgtcct 960
   gccagcgact cccccacaac ttgtcagccc ccgggtccta gaggtggaca cgcaggggac 1020
   cgtggtctgt tccctggacg ggctgttccc agtctcggag gcccaggtcc acctggcact 1080
   gggggaccag aggttgaacc ccacagtcac ctatggcaac gactccttct cggccaaggc 1140
   ctcagtcagt gtgaccgcag aggacgaggg cacccagcgg ctgacgtgtg cagtaatact 1200
   ggggaaccag agccaggaga cactgcagac agtgaccatc tacagctttc cggcgcccaa 1260
   cgtgattctg acgaagccag aggtctcaga agggaccgag gtgacagtga agtgtgaggc 1320
   ccaccctaga gccaaggtga cgctgaatgg ggttccagcc cagccactgg gcccgagggc 1380
   ccagctcctg ctgaaggcca ccccagagga caacgggcgc agcttctcct gctctgcaac 1440
   cctggaggtg gccggccagc ttatacacaa gaaccagacc cgggagcttc gtgtcctgta 1500
   tggcccccga ctggacgaga gggattgtcc gggaaactgg acgtggccag aaaattccca 1560
   gcagactcca atgtgccagg cttgggggaa cccattgccc gagctcaagt gtctaaagga 1620
   tggcactttc ccactgccca tcggggaatc agtgactgtc actcgagatc ttgagggcac 1680
   ctacctctgt cgggccagga gcactcaagg ggaggtcacc cgcaaggtga ccgtgaatgt 1740
   gctctccccc cggtatgaga ttgtcatcat cactgtggta gcagccgcag tcataatggg 1800
   cactgcaggc ctcagcacgt acctctataa ccgccagcgg aagatcaaga aatacagact 1860
   acaacaggcc caaaaaggga cccccatgaa accgaacaca caagccacgc ctccctgaac 1920
   ctatcccggg acagggcctc ttcctcggcc ttcccatatt ggtggcagtg gtgccacact 1980
   gaacagagtg gaagacatat gccatgcagc tacacctacc ggccctggga cgccggagga 2040
   cagggcattg tcctcagtca gatacaacag catttggggc catggtacct gcacacctaa 2100
   aacactaggc cacgcatctg atctgtagtc acatgactaa gccaagagga aggagcaaga 2160
   ctcaagacat gattgatgga tgttaaagtc tagcctgatg agaggggaag tggtggggga 2220
   gacatagccc caccatgagg acatacaact gggaaatact gaaacttgct gcctattggg 2280
   tatgctgagg ccccacagac ttacagaaga agtggccctc catagacatg tgtagcatca 2340
   aaacacaaag gcccacactt cctgacggat gccagcttgg gcactgctgt ctactgaccc 2400
   caacccttga tgatatgtat ttattcattt gttattttac cagctattta ttgagtgtct 2460
   tttatgtagg ctaaatgaac ataggtctct ggcctcacgg agctcccagt cctaatcaca 2520
   ttcaaggtca ccaggtacag ttgtacaggt tgtacactgc aggagagtgc ctggcaaaaa 2580
   gatcaaatgg ggctgggact tctcattggc caacctgcct ttccccagaa ggagtgattt 2640
   ttctatcggc acaaaagcac tatatggact ggtaatggtt acaggttcag agattaccca 2700
   gtgaggcctt attcctccct tccccccaaa actgacacct ttgttagcca cctccccacc 2760
   cacatacatt tctgccagtg ttcacaatga cactcagcgg tcatgtctgg acatgagtgc 2820
   ccagggaata tgcccaagct atgccttgtc ctcttgtcct gtttgcattt cactgggagc 2880
   ttgcactatg cagctccagt ttcctgcagt gatcagggtc ctgcaagcag tggggaaggg 2940
   ggccaaggta ttggaggact ccctcccagc tttggaagcc tcatccgcgt gtgtgtgtgt 3000
   gtgtatgtgt agacaagctc tcgctctgtc acccaggctg gagtgcagtg gtgcaatcat 3060
   ggttcactgc agtcttgacc ttttgggctc aagtgatcct cccacctcag cctcctgagt 3120
   agctgggacc ataggctcac aacaccacac ctggcaaatt tgattttttt tttttttcca 3180
   gagacggggt ctcgcaacat tgcccagact tcctttgtgt tagttaataa agctttctca 3240
   actgccaaa 3249
<210> 23
   <211> 4720
   <212> DNA
   <213> Homo sapiens
<400> 23
   atgctgacaa tcttcttcct tcccctggcc acctctctgc ccacttgctt cctcagtacc      60
   ttggtccagc tcttcctgca acggcccagg agctcagagc tccacatctg accttctagt     120
   catgaccagg accagggcag cactcctcct gttcacagcc ttagcaactt ctctaggttt     180
   caacttggac acagaggagc tgacagcctt ccgtgtggac agcgctgggt ttggagacag     240
   cgtggtccag tatgccaact cctgggtggt ggttggagcc ccccaaaaga taacagctgc     300
   caaccaaacg ggtggcctct accagtgtgg ctacagcact ggtgcctgtg agcccatcgg     360
   cctgcaggtg cccccggagg ccgtgaacat gtccctgggc ctgtccctgg cgtctaccac     420
   cagcccttcc cagctgctgg cctgcggccc caccgtgcac cacgagtgcg ggaggaacat     480
   gtacctcacc ggactctgct tcctcctggg ccccacccag ctcacccaga ggctcccggt     540
   gtccaggcag gagtgcccaa gacaggagca ggacattgtg ttcctgatcg atggctcagg     600
   cagcatctcc tcccgcaact ttgccacgat gatgaacttc gtgagagctg tgataagcca     660
   gttccagaga cccagcaccc agttttccct gatgcagttc tccaacaaat tccaaacaca     720
   cttcactttc gaggaattca ggcgcagctc aaaccccctc agcctgttgg cttctgttca    780
   ccagctgcaa gggtttacat acacggccac cgccatccaa aatgtcgtgc accgattgtt     840
   ccatgcctca tatggggccc gtagggatgc cgccaaaatt ctcattgtca tcactgatgg     900
   gaagaaagaa ggcgacagcc tggattataa ggatgtcatc cccatggctg atgcagcagg     960
   catcatccgc tatgcaattg gggttggatt agcttttcaa aacagaaatt cttggaaaga   1020
   attaaatgac attgcatcga agccctccca ggaacacata tttaaagtgg aggactttga    1080
   tgctctgaaa gatattcaaa accaactgaa ggagaagatc tttgccattg agggtacgga 1140
   gaccacaagc agtagctcct tcgaattgga gatggcacag gagggcttca gcgctgtgtt 1200
   cacacctgat ggccccgttc tgggggctgt ggggagcttc acctggtctg gaggtgcctt 1260
   cctgtacccc ccaaatatga gccctacctt catcaacatg tctcaggaga atgtggacat 1320
   gagggactct tacctgggtt actccaccga gctggccctc tggaaagggg tgcagagcct 1380
   ggtcctgggg gccccccgct accagcacac cgggaaggct gtcatcttca cccaggtgtc 1440
   caggcaatgg aggatgaagg ccgaagtcac ggggactcag atcggctcct acttcggggc 1500
   ctccctctgc tccgtggacg tagacagcga cggcagcacc gacctggtcc tcatcggggc 1560
   cccccattac tacgagcaga cccgaggggg ccaggtgtct gtgtgtccct tgcccagggg 1620
   gtggagaagg tggtggtgtg atgctgttct ctacggggag cagggccacc cctggggtcg 1680
   ctttggggcg gctctgacag tgctggggga tgtgaatggg gacaagctga cagacgtggt 1740
   catcggggcc ccaggagagg aggagaaccg gggtgctgtc tacctgtttc acggagtctt 1800
   gggacccagc atcagcccct cccacagcca gcggatcgcg ggctcccagc tctcctccag 1860
   gctgcagtat tttgggcagg cactgagcgg gggtcaagac ctcacccagg atggactggt 1920
   ggacctggct gtgggggccc ggggccaggt gctcctgctc aggaccagac ctgtgctctg 1980
   ggtgggggtg agcatgcagt tcatacctgc cgagatcccc aggtctgcgt ttgagtgtcg 2040
   ggagcaggtg gtctctgagc agaccctggt acagtccaac atctgccttt acattgacaa 2100
   acgttctaag aacctgcttg ggagccgtga cctccaaagc tctgtgacct tggacctggc 2160
   cctcgaccct ggccgcctga gtccccgtgc caccttccag gaaacaaaga accggagtct 2220
   gagccgagtc cgagtcctcg ggctgaaggc acactgtgaa aacttcaacc tgctgctccc 2280
   gagctgcgtg gaggactctg tgacccccat taccttgcgt ctgaacttca cgctggtggg 2340
   caagcccctc cttgccttca gaaacctgcg gcctatgctg gccgccgatg ctcagagata 2400
   cttcacggcc tccctaccct ttgagaagaa ctgtggagcc gaccatatct gccaggacaa 2460
   tctcggcatc tccttcagct tcccaggctt gaagtccctg ctggtgggga gtaacctgga 2520
   gctgaacgca gaagtgatgg tgtggaatga cggggaagac tcctacggaa ccaccatcac 2580
   cttctcccac cccgcaggac tgtcctaccg ctacgtggca gagggccaga aacaagggca 2640
   gctgcgttcc ctgcacctga catgtgacag cgccccagtt gggagccagg gcacctggag 2700
   caccagctgc agaatcaacc acctcatctt ccgtggcggc gcccagatca ccttcttggc 2760
   tacctttgac gtctccccca aggctgtcct gggagaccgg ctgcttctga cagccaatgt 2820
   gagcagtgag aacaacactc ccaggaccag caagaccacc ttccagctgg agctcccggt 2880
   gaagtatgct gtctacactg tggttagcag ccacgaacaa ttcaccaaat acctcaactt 2940
   ctcagagtct gaggagaagg aaagccatgt ggccatgcac agataccagg tcaataacct 3000
   gggacagagg gacctgcctg tcagcatcaa cttctgggtg cctgtggagc tgaaccagga 3060
   ggctgtgtgg atggatgtgg aggtctccca cccccagaac ccatcccttc ggtgctcctc 3120
   agagaaaatc gcacccccag catctgactt cctggcgcac attcagaaga atcccgtgct 3180
   ggactgctcc attgctggct gcctgcggtt ccgctgtgac gtcccctcct tcagcgtcca 3240
   ggaggagctg gatttcaccc tgaagggcaa cctcagcttt ggctgggtcc gccagatatt 3300
   gcagaagaag gtgtcggtcg tgagtgtggc tgaaattacg ttcgacacat ccgtgtactc 3360
   ccagcttcca ggacaggagg catttatgag agctcagacg acaacggtgc tggagaagta 3420
   caaggtccac aaccccaccc ccctcatcgt aggcagctcc attgggggtc tgttgctgct 3480
   ggcactcatc acagcggtac tgtacaaagt tggcttcttc aagcgtcagt acaaggaaat 3540
   gatggaggag gcaaatggac aaattgcccc agaaaacggg acacagaccc ccagcccgcc 3600
   cagtgagaaa tgatcccctc tttgccttgg acttcttctc ccccgcgagt tttccccact 3660
   tacttaccct cacctgtcag gcctgacggg gaggaaccac tgcaccaccg agagaggctg 3720
   ggatgggcct gcttcctgtc tttgggagaa aacgtcttgc ttgggaaggg gcctttgtct 3780
   tgtcaaggtt ccaactggaa acccttagga cagggtccct gctgtgttcc ccaaaggact 3840
   tgacttgcaa tttctaccta gaaatacatg gacaataccc ccaggcctca gtctcccttc 3900
   tcccatgagg cacgaatgat ctttctttcc tttctttttt tttttttttc ttttcttttt 3960
   tttttttttg agacggagtc tcgctctgtc acccaggctg gagtgcaatg gcgtgatctc 4020
   ggctcactgc aacctccgcc tcccgggttc aagtaattct gctgtctcag cctcctgagt 4080
   agctgggact acaggcacac gccacctcgc ccggcccgat ctttctaaaa tacagttctg 4140
   aatatgctgc tcatccccac ctgtcttcaa cagctcccca ttaccctcag gacaatgtct 4200
   gaactctcca gcttcgcgtg agaagtcccc ttccatccca gagggtgggc ttcagggcgc 4260
   acagcatgag aggctctgtg cccccatcac cctcgtttcc agtgaattag tgtcatgtca 4320
   gcatcagctc agggcttcat cgtggggctc tcagttccga tttcccaggc tgaattggga 4380
   gtgagatgcc tgcatgctgg gttctgcaca gctggcctcc cgcgttgggc aacattgctg 4440
   gctggaaggg aggagcgccc tctagggagg gacatggccc cggtgcggct gcagctcacc 4500
   cagccccagg ggcagaagag acccaaccac ttctattttt tgaggctatg aatatagtac 4560
   ctgaaaaaat gccaagacat gattattttt ttaaaaagcg tactttaaat gtttgtgtta 4620
   ataaattaaa acatgcacaa aaagatgcat ctaccgctct tgggaaatat gtcaaaggtc 4680
   taaaaataaa aaagccttct gtgaaaaaaa aaaaaaaaaa 4720
<210> 24
   <211> 1921
   <212> DNA
   <213> Homo sapiens
<400> 24
   gaagcacaca ggaaaaggag cttagctgct ggtgctgctg gcaagatgga aaccaacttc 60
   tccactcctc tgaatgaata tgaagaagtg tcctatgagt ctgctggcta cactgttctg 120
   cggatcctcc cattggtggt gcttggggtc acctttgtcc tcggggtcct gggcaatggg 180
   cttgtgatct gggtggctgg attccggatg acacgcacag tcaccaccat ctgttacctg 240
   aacctggccc tggctgactt ttctttcacg gccacattac cattcctcat tgtctccatg 300
   gccatgggag aaaaatggcc ttttggctgg ttcctgtgta agttaattca catcgtggtg 360
   gacatcaacc tctttggaag tgtcttcttg attggtttca ttgcactgga ccgctgcatt 420
   tgtgtcctgc atccagtctg ggcccagaac caccgcactg tgagtctggc catgaaggtg 480
   atcgtcggac cttggattct tgctctagtc cttaccttgc cagttttcct ctttttgact 540
   acagtaacta ttccaaatgg ggacacatac tgtactttca actttgcatc ctggggtggc 600
   acccctgagg agaggctgaa ggtggccatt accatgctga cagccagagg gattatccgg 660
   tttgtcattg gctttagctt gccgatgtcc attgttgcca tctgctatgg gctcattgca 720
   gccaagatcc acaaaaaggg catgattaaa tccagccgtc ccttacgggt cctcactgct 780
   gtggtggctt ctttcttcat ctgttggttt ccctttcaac tggttgccct tctgggcacc 840
   gtctggctca aagagatgtt gttctatggc aagtacaaaa tcattgacat cctggttaac 900
   ccaacgagct ccctggcctt cttcaacagc tgcctcaacc ccatgcttta cgtctttgtg 960
   ggccaagact tccgagagag actgatccac tccctgccca ccagtctgga gagggccctg 1020
   tctgaggact cagccccaac taatgacacg gctgccaatt ctgcttcacc tcctgcagag 1080
   actgagttac aggcaatgtg aggatggggt cagggatatt ttgagttctg ttcatcctac 1140
   cctaatgcca gttccagctt catctaccct tgagtcatat tgaggcattc aaggatgcac 1200
   agctcaagta tttattcagg aaaaatgctt ttgtgtccct gatttggggc taagaaatag 1260
   acagtcaggc tactaaaata ttagtgttat tttttgtttt ttgacttctg cctataccct 1320
   ggggtaagtg gagttgggaa atacaagaag agaaagacca gtggggattt gtaagactta 1380
   gatgagatag cgcataataa ggggaagact ttaaagtata aagtaaaatg tttgctgtag 1440
   gttttttata gctattaaaa aaaatcagat tatggaagtt ttcttctatt tttagtttgc 1500
   taagagtttt ctgtttcttt ttcttacatc atgagtggac tttgcatttt atcaaatgca 1560
   ttttctacat gtattaagat ggtcatatta ttcttcttct tttatgtaaa tcattataaa 1620
   taatgttcat taagttctga atgttaaact actcttgaat tcctggaata aaccacactt 1680
   agtcctgatg tactttaaat atttatatct cacaggagtt ggttagaatt tctgtgttta 1740
   tgtttatata ctgttatttc actttttcta ctatccttgc taagttttca tagaaaataa 1800
   ggaacaaaga gaaacttgta atggtctctg aaaaggaatt gagaagtaat tcctctgatt 1860
   ctgttttctg gtgttatatc tttattaaat attcagaaaa attcaccagt gaaaaaaaaa 1920
   a 1921
<210> 25
   <211> 2429
   <212> DNA
   <213> Homo sapiens
<400> 25
   ctcttttcta agcttgtctc ttaaaaccca ctggacgttg gcacagtgct gggatgacta      60
   tggagaccca aatgtctcag aatgtatgtc ccagaaacct gtggctgctt caaccattga     120
   cagttttgct gctgctggct tctgcagaca gtcaagctgc agctccccca aaggctgtgc     180
   tgaaacttga gcccccgtgg atcaacgtgc tccaggagga ctctgtgact ctgacatgcc     240
   agggggctcg cagccctgag agcgactcca ttcagtggtt ccacaatggg aatctcattc     300
   ccacccacac gcagcccagc tacaggttca aggccaacaa caatgacagc ggggagtaca     360
   cgtgccagac tggccagacc agcctcagcg accctgtgca tctgactgtg ctttccgaat     420
   ggctggtgct ccagacccct cacctggagt tccaggaggg agaaaccatc atgctgaggt     480
   gccacagctg gaaggacaag cctctggtca aggtcacatt cttccagaat ggaaaatccc     540
   agaaattctc ccatttggat cccaccttct ccatcccaca agcaaaccac agtcacagtg     600
   gtgattacca ctgcacagga aacataggct acacgctgtt ctcatccaag cctgtgacca     660
   tcactgtcca agtgcccagc atgggcagct cttcaccaat ggggatcatt gtggctgtgg     720
   tcattgcgac tgctgtagca gccattgttg ctgctgtagt ggccttgatc tactgcagga     780
   aaaagcggat ttcagccaat tccactgatc ctgtgaaggc tgcccaattt gagccacctg     840
   gacgtcaaat gattgccatc agaaagagac aacttgaaga aaccaacaat gactatgaaa     900
   cagctgacgg cggctacatg actctgaacc ccagggcacc tactgacgat gataaaaaca     960
   tctacctgac tcttcctccc aacgaccatg tcaacagtaa taactaaaga gtaacgttat    1020
   gccatgtggt catactctca gcttgctgag tggatgacaa aaagagggga attgttaaag    1080
   gaaaatttaa atggagactg gaaaaatcct gagcaaacaa aaccacctgg cccttagaaa    1140
   tagctttaac tttgcttaaa ctacaaacac aagcaaaact tcacggggtc atactacata    1200
   caagcataag caaaacttaa cttggatcat ttctggtaaa tgcttatgtt agaaataaga    1260
   caaccccagc caatcacaag cagcctacta acatataatt aggtgactag ggactttcta    1320
   agaagatacc tacccccaaa aaacaattat gtaattgaaa accaaccgat tgcctttatt     1380
   ttgcttccac attttcccaa taaatacttg cctgtgacat tttgccactg gaacactaaa    1440
   cttcatgaat tgcgcctcag atttttcctt taacatcttt tttttttttg acagagtctc    1500
   aatctgttac ccaggctgga gtgcagtggt gctatcttgg ctcactgcaa acccgcctcc 1560
   caggtttaag cgattctcat gcctcagcct cccagtagct gggattagag gcatgtgcca 1620
   tcatacccag ctaatttttg tattttttat tttttttttt tagtagagac agggtttcgc 1680
   aatgttggcc aggccgatct cgaacttctg gcctctagcg atctgcccgc ctcggcctcc 1740
   caaagtgctg ggatgaccag catcagcccc aatgtccagc ctctttaaca tcttctttcc 1800
   tatgccctct ctgtggatcc ctactgctgg tttctgcctt ctccatgctg agaacaaaat 1860
   cacctattca ctgcttatgc agtcggaagc tccagaagaa caaagagccc aattaccaga 1920
   accacattaa gtctccattg ttttgccttg ggatttgaga agagaattag agaggtgagg 1980
   atctggtatt tcctggacta aattcccctt ggggaagacg aagggatgct gcagttccaa 2040
   aagagaagga ctcttccaga gtcatctacc tgagtcccaa agctccctgt cctgaaagcc 2100
   acagacaata tggtcccaaa tgactgactg caccttctgt gcctcagccg ttcttgacat 2160
   caagaatctt ctgttccaca tccacacagc caatacaatt agtcaaacca ctgttattaa 2220
   cagatgtagc aacatgagaa acgcttatgt tacaggttac atgagagcaa tcatgtaagt 2280
   ctatatgact tcagaaatgt taaaatagac taacctctaa caacaaatta aaagtgattg 2340
   tttcaaggtg atgcaattat tgatgaccta ttttattttt ctataatgat catatattac 2400
   ctttgtaata aaacattata accaaaaca 2429
<210> 26
   <211> 2114
   <212> DNA
   <213> Homo sapiens
<400> 26
   agaacatttc tttttcactt cccctttcag actccagaat ttgtttgccc tctagggtag 60
   aatccgccaa gctttgagag aaggctgtga ctgctgtgct ctgggcgcca gctcgctcca 120
   gggagtgatg ggaatcctgt cattcttacc tgtccttgcc actgagagtg actgggctga 180
   ctgcaagtcc ccccagcctt ggggtcatat gcttctgtgg acagctgtgc tattcctggc 240
   tcctgttgct gggacacctg ctcccccaaa ggctgtgctg aaactcgagc cccagtggat 300
   caacgtgctc caggaggact ctgtgactct gacatgccgg gggactcaca gccctgagag 360
   cgactccatt cagtggttcc acaatgggaa tctcattccc acccacacgc agcccagcta 420
   caggttcaag gccaacaaca atgacagcgg ggagtacacg tgccagactg gccagaccag 480
   cctcagcgac cctgtgcatc tgactgtgct ttctgagtgg ctggtgctcc agacccctca 540
   cctggagttc caggagggag aaaccatcgt gctgaggtgc cacagctgga aggacaagcc 600
   tctggtcaag gtcacattct tccagaatgg aaaatccaag aaattttccc gttcggatcc 660
   caacttctcc atcccacaag caaaccacag tcacagtggt gattaccact gcacaggaaa 720
   cataggctac acgctgtact catccaagcc tgtgaccatc actgtccaag ctcccagctc 780
   ttcaccgatg gggatcattg tggctgtggt cactgggatt gctgtagcgg ccattgttgc 840
   tgctgtagtg gccttgatct actgcaggaa aaagcggatt tcagccaatc ccactaatcc 900
   tgatgaggct gacaaagttg gggctgagaa cacaatcacc tattcacttc tcatgcaccc 960
   ggatgctctg gaagagcctg atgaccagaa ccgtatttag tctccattgt cttgcattgg 1020
   gatttgagaa gaaaatcaga gagggaagat ctggtatttc ctggcctaaa ttccccttgg 1080
   ggaggacagg gagatgctgc agttccaaaa gagaaggttt cttccagagt catctacctg 1140
   agtcctgaag ctccctgtcc tgaaagccac agacaatatg gtcccaaata accgactgca 1200
   ccttctgtgc ttcagctctt cttgacatca aggctcttcc gttccacatc cacacagcca 1260
   atccaattaa tcaaaccact gttattaaca gataatagca acttgggaaa tgcttatgtt 1320
   acaggttacg tgagaacaat catgtaaatc tatatgattt cagaaatgtt aaaatagact 1380
   aacctctacc agcacattaa aagtgattgt ttctgggtga taaaattatt gatgattttt 1440
   attttcttta tttttctata aagatcatat attactttta taataaaaca ttataaaaac 1500
   aacattctgt ttaccttttc aaggctgtat tggttggagt gtagactgaa ctgcctgggg 1560
   tctgtttctc ttcagtgatg agactcttag gaaggcagga atggatagga tagggggagg 1620
   agaggagaga tggggattta gaatgtagag tgagtgcccc ttttcttaaa actgaataca 1680
   gtcacgcacc acataatgat gtttagttca acaacagact gcatatatga tggtgatccc 1740
   ataaaattat aataccatat ttctattgta ccttttctat tcctatgttt agatatatga 1800
   gtacttacca ttgtgttaca attgcctaaa gtattcagta cagtagcatg ctgtacaggt 1860
   ttgtagccta ggggcaatag gctatacgct acagcctagg tgtgtagtag gccacaccat 1920
   ttaggtttgt ataagtacct gctatgatgt tcacacaaca aaattgcctg catttctcaa 1980
   aatgtatccc catatttcaa caatgcatga ctgtactctt ctgccaatga ccttgtattc 2040
   ttgtttccat gtcttcttct ctttcctcct atggcaaata aaacactgtt ttgcaacaca 2100
   aaaaaaaaaa aaaa 2114
<210> 27
   <211> 46115
   <212> DNA
   <213> Homo sapiens
<400> 27
<210> 28
   <211> 24252
   <212> DNA
   <213> Homo sapiens
<400> 28
<210> 29
   <211> 17328
   <212> DNA
   <213> Homo sapiens
<400> 29
   tccacccaag agcaacctgg aactaagtta ttcggcaacg aactgttcca ctttgttgtg      60
   aggcaataga tgtggaaatt ccctgacgag gggctctgtc ctcatacttc ctgcggagct     120
   tattgtcgta agaatatctg tcatcctgct aatgtgcatt gaaaggagag caacggggct     180
   gaggccgtgt cagcacgatg gaccccaaac agaccaccct cctgtgtctt ggtgagtttc     240
   agagtaaaag tgggttagag gggaagatag agaaatccca aaataatcag ggtgtctctt     300
   aacagtgtga ctaggagatt ttagtggctg ccaaggagat tctgatctcc ttagtggaaa     360
   ggccgtcttt gtcaatgtat ctataacttt gtctctaccc aagcccaagc tagcttgtgg     420
   ggctcaaggt ttaatatttg tattaaacct atagtgtgtt atctgggatt catgatggtc     480
   ccaaggttct tatcaaggag agacttagag gctggaatct gaaaggtaaa aataaagaat     540
   gaacctcaaa actgtgattg ttgtggaagg aaaacatatg atagaacccc atatagaaat     600
   atggttacta gtattttgtt gaagattttt gcatttatgt tcaacaaaga tattatccag     660
   aagttttctg tttttgttgt atctctgcca cattttgtta tcaggataat gttggcctca     720
   tagaatgagt tggggaggag tccctcctcc aggatttttt tcaatagttt cagtaggaat     780
   aatactagct cttcttggcc gggcgcagtg gctcacacct gcaatcccag cactttggga     840
   ggccaaggca ggcggatcac aaggtcagga gatcaaaacc atcctggcca acatggtgaa     900
   accctgtctc tactaaaaat acaaaaaaat tagccaggcg tggtggcggg cgcctgtagt     960
   cccagctact cgtgcggctg aggcaggaga atggcatgaa cctgggaggc agagcttgca    1020
   gtgagccaag atcatgccac tgcactccag cttgggcgac acagcgagac tctgtctcaa    1080
   aaaaaaaaaa aaatgccagc tcttctttat atatctggtg ggattgagct gtgaatccat    1140
   ctggtactgg tctttttctg gtctgtcatt acagagggtg atttgtcgta aaggttggaa    1200
   atggaagctt gatttttcat aaatctctct cttccagtgc tctgtctggg ccagaggatt 1260
   caggcacagg aaggtaagtg tcctgtaaat ctctcccagc ccctttagac cctcttggga 1320
   gctctaggat aaagaaattg aagaatagcc tgaagcacca ttcttatttt aatccccatt 1380
   ctagttgttt ctgctgtgct tctcttgcat aatttctatc tcactttgtt atctccaaac 1440
   ccttcagact cattaatgct caggcctgga tttatagtta gtccttgcct gtgttagact 1500
   gtccatgaag gatctgtaat ttactgaatg ctcaaactgc aagaatgagg aagtcaggag 1560
   tcatctgccc aatatccttc cttatgctga ttctattttg ttttagcaac ccacttcctc 1620
   ccgtcacttc atttaaaagg atgctgccat agtctaaccc tactgaacac tctagcattc 1680
   tgtagtacta ctgcagtact aagcatgagg cagtcttagt gtactactga atattctgcc 1740
   accccaacta ctactgcctt agcctcctaa tgggtgtgag ccccacgtcc atccatgtct 1800
   tctctcttcc agctccttct aaagcctgaa ttatttgtgt gttgaacaat actcattctt 1860
   cctatccatg agcatggaat gtttttccat ttgtttgtgt catctatgat ttctttgacc 1920
   agtgttttgt agttctcctt gcagagatct ttcacctccc tggttagctg tattcccagg 1980
   tattttattc tttttgcagt aattgtgaat ggattctatt cttgatttgg ctctcagctt 2040
   agatgttttt ggtgtatagg aatgctactg atttttatat attgattttg tatcctggaa 2100
   ctttgctaaa gttgtttatc agattaagaa gtgtttgggc agagactgtg gttttctagg 2160
   tatagaatca tatcacctgc aaacagggat agtttgactt cctttcttcc tatttggatg 2220
   ccttttattt ctctcttgcc tgattgctct agctaggacc tccagtacta tgttgaacag 2280
   aagtagtgag agacggcatc catgtctttt gccagttttc aaggggaata cttccagctt 2340
   ttgcccattc aatatgatgt tgactgtgag tttgtcatac atcattctta ttattttgaa 2400
   atatgtttct tcaatgccta gtttgtcgag gttttttagc ataaagggat gttgaatttt 2460
   atcaaaagct ctattgagag gattatgtgt gtggggaggg ttgttctatt tatgtgatga 2520
   atcatattta agatttgtgt atattggccg ggcactgtgg ctcatgcctg taatcccagc 2580
   actttgggaa gccaaggctt gtggatcatg aggtcaggag atcgacacca tcctggctaa 2640
   cacggtgaaa cctcgtctat actaaaaaac acaaaaaaat tagccaggcg tggtggtggg 2700
   cacctgtagt cccagctgct cgggaggctc aggcaggaga atggcgtgaa tccaggaggc 2760
   ggagcttgca gtgagccaag ttcacgccac tacactccag cctgggcaac agagcgagac 2820
   tcctatatcg aatcaacctt gcatcccaga aataaagcct acctgatggt ggtggattag 2880
   ctttctgatg tgctgctgga tagtttgcta gtattttgtt gaggattttt gcatttatgt 2940
   tcaacaagga tattgtcctg aagttttctg gttttgttgt gtctctgcca tgtttttgca 3000
   tcaagatgat gctggtctca tagaatgagc tggggaggca ttcctcctcc tgaatatttt 3060
   tggaacgttt cagtaggtat agtaccagct cttctttata tatcagatgg gattcagctg 3120
   tgagtctgtc tggtactggg ctttttctgg tctgtaggat ttttattact gattcaattt 3180
   tggagctcat tattggtctg ttcatgtatt caatttattc ttggtttgat ctcaggaggg 3240
   tgtatgtgtc caggaatttc tccatttatt ctggattttc tagtttgtgt gcatagaggt 3300
   gttcataata ttctctgatg attgtatttc tgtggggtga gtggtaatat accctttgtt 3360
   gtttctaatt gtgtttatcc ggatcatctc tcttttcttc tttattagtc taactagtca 3420
   tctgtcttac taattttttc aaaaattcta ctcctcgatt tgctgatctt ctgaatgctt 3480
   tttcgtgtct caatctcctt cagctcagct ctgattttgg ttatttcttg tttcctatga 3540
   gctttggggt tgatttcctc ttggttctct tagttcctct tgttatgatg ttaggttgtt 3600
   aatttgaact ttttctagct ttttgaagtg ggagtttagt gctataaact tcccccttaa 3660
   cactgcctta gctgtgtccc agagattctg ctatatttac ccaaaaattc cagaacagac 3720
   tgcttaattt ccatgcattg tacagttttg agtggttttc ttagtattta ttcctatttt 3780
   tattccactg tgctctgatt tcgcttttct ggatttgcta aggattgttt tttttttttt 3840
   tgaaatggag tcttgctctg tcgcccaggc tggagtgcag tggcgcaatc taggctcact 3900
   gcaagctccg cctcccaggt tcacaccatt ctcctgcctc agcctcctga gtagctgaga 3960
   ctacaggtgc ccgccacccc gcccggctaa ttttttttgt atttttagta aagacggggt 4020
   ttcactgcgt tagccaggat ggtctcgatc tcctgacctc atgatccgcc agccttggct 4080
   tcccaaagtg ctgggattac aggtgtgagc caccgtgccc agcctgctaa ggattgctgt 4140
   atgtctgatt gtatgattga ctttagagta tgtgccatga ggcaatgaaa acaatgtaga 4200
   ttctgttgtt ttgggggtgg agagttctgt agatgtctgt caggtccatt tgatccactg 4260
   ctgagttcag gtcctgaata tctctgtttg cctcaatgat ctaatactgt cggcgggatg 4320
   ttaaagtctc cccctattat tgtgtggttg tctaagtctc ttcgttggtc tctcagaaca 4380
   cgctttatga atccgggtgc ttccatgtta ggtgcatata tatttaggat agttaggtct 4440
   tcatgctctt tttttaattt tttttttctt tttctttttg attcagcagt tgggctatta 4500
   cacactcctt agcagattcc gacttccgtg gccactgtcc tgctatggtc ttcatgttga 4560
   attgaaccct ttaccatgat ttaatgccct tctttgcctt ttttgatctt tgttggtata 4620
   aagtctgttt tgtctgaaat tttaatagca gctcctgcat tttttttttt ttttggcttt 4680
   ccatttgctt ggtagatttt tctccatttc tttactttga gcctatggat gtcattgcat 4740
   atgagatggg tttcttatag gcagcataat gttgagtctt gctttttttt tttttttttt 4800
   ttttgagatg gagtctcact ctgtcaccca ggctggagtg cagtggtatg atcttgactc 4860
   actgcaactt ctgcctccca ggttcaaatg attctcctgc ctcagcctcc caaatagctg 4920
   ggattacagg tgtgtaccac cacgcccagc tatttttttt ttttattaga gatgggattt 4980
   catcacattg gccaggctaa tgtcgaactc ctgacctcaa atgatccacc cacctcagcc 5040
   tcccaaagtg ccagggttac aggcgtgagc cactgcacct ggcctcttgc ttctttatcc 5100
   aacttgccac tctctgcatt ttaattagga caattagtcc atttacattc aaagttagca 5160
   tttacatgtg cagatttttt cctgtcatca tgttgttagc tggtttggtt attatgcaga 5220
   cttgtttgtg tggttgtttt atagtgtcac tggtttatgt acgtaagtgt gttttctatt 5280
   ggctggtgat ggtcttttct ttccatattt agcgttcctc ttaggacctc ttgtaaggca 5340
   ggcctgatgg taataaattc cctcagcatt tgcttgtctg taaaggatct tatttctcct 5400
   tcacttatga aacttagttt ggctgggaat gagattcttg gttggaaatt cttttcataa 5460
   gaacattaaa tataggcccc caatctcttc tggattgtag agtttctgct gaaaggtctg 5520
   ctgttagctc gatggcattc cctttgtagg tgacctgccc cttctttttt gctgcctttt 5580
   cacatttttt tttttttttt tgagactgag tcttgctctg ttgcccaggc tgccaggctg 5640
   gagtgcaatg gcgtgatctc ggctcactgc aagctccgcc tcccggattc acgccattct 5700
   cctgcctcag cctccccagt agctgggact acaggtgccc accaccacgc ccagctaatt 5760
   ttttatattt ttttagtaga gatggggttt caccatgtta gccaggatgg tctcaatctc 5820
   ctgacctcgt gatccgcccg ccttggtctc ccaaagtgct gggattacag gcgtgagcca 5880
   ccgcgcccgg ccgacatttc tttctttcat ttctaccttg aagaatctga tgattttgtg 5940
   tcttggggat gctcttcttg tgtagtattt tgcaggggtt ctctgtttcc tgcatttgat 6000
   tcttggcctc tctagtgacg ttggggaagt tttcatgaac aatacactga aatatgtttt 6060
   ccatgttcct tgctttctcc ccatctcttt cggggatgcc aatgggctat ttggtctctt 6120
   ttcatgatcc catatttctt agaggttttg ttcattaatt tttattcttt ttcttcattt 6180
   ttgtctgact gaattagttc agagagccag tattcatgct ctgagattct ttccttattt 6240
   tgctttattt tgccattaat acttgtgatc gcattatgaa atctcgtagt gtgtttttca 6300
   gctccatcag atccgtttgg ttctttcaaa atgaccatct catagattag cccctctgtc 6360
   attttattgt aatctttagg ttccttgcat tgggtttcaa ctttctcctg aatcttgatg 6420
   accttaattt ctatccatat tctgaattct atttctgtca tttcagccag gtaaagagcc 6480
   cttgctggga agcttgtgtg gtaatttgga ggaaggaaga cactgttgct ttttgagttg 6540
   atggagttct tgctcagttc tttctcatcc atgtgggcta atgttccttt gagtgtgctg 6600
   caatttgaat ttttttcttt tttcttttaa ccgtgatgta atttgagcac agtcagtaga 6660
   cttcttttct ggatggtttc agagggctgg ggcttcgcac agggtcttta tttatagcta 6720
   aattcttgtc cttggtttca cagggaggta tattagcgag catttttggt gttgaagttt 6780
   gggctgcaat ccggtaaatg atgcttcagc acaacggcca gtaggtcatt cctcatgatt 6840
   gccgctgtgc tccctctcac gctctgaaag tgcgggctcc tctcccaccc aagtgctggc 6900
   tgcagatctg ggctcggcac tcccaggctg cacatcacag ctctggggtg agctcagact 6960
   ttatgttccc tccgtggctt gggggcagca ggggaaggga ccttagcagc ggttgtggca 7020
   gacggccttt cacttgtccc ttggaactcc accccagaga gatgtggagc cactatcagt 7080
   gcgatgagcc aagagtgagg cgactgcact gtgggtccaa gctaggggcc ctgcctagtg 7140
   atgagcaggg gggacaggtg ggtcacaggg gtgacagact ggcctcttct ccttagggca 7200
   gtttgccgga ggtgtggttg aagcactcag agtctttgct ccttccccag tctgagggca 7260
   gcaaggccag taccaccgca gtggcagcgg cagagtgacc ttccgttgcc tctgggagct 7320
   ccgccacaga gaaacgcaga ctcacagctg ctgggaacgc tccgccagag ggtggggctg 7380
   ttgtgccgcg gaatcaagct ggggcttgtt gaagagcagg gggttgaggg ctcacaggga 7440
   gaggagactg agctcctctc cgtatggcga ctgcggtgtg ctggaagcat gaatgaaggc 7500
   cgggcgcggt ggactcacgc ccggaatccc agcactttgg gaggccgagg cgggcggatc 7560
   acgaggtcgg gagatcgaga ccatcctggc taatctggtg aaaccccgtc tctactgaaa 7620
   acacaaaaaa tgagccgggc gtggtggcgg gcgcctgtag tcccagctac tcgggaggag 7680
   gatgaggctg aggcaggaga atggtgtgaa cccgggaggc gcagcttgca gtgagccgag 7740
   atcgggccac tgcactgcag cgtgggcgac agagcgagac tccgtctcaa ataaataaat 7800
   aaataaataa ataaataaat aaataaataa ataaataaat tgaagcatga atgaaggccc 7860
   caggctcctt gcttcttccc cagatcacgg gcagcagagg cagaaccctt gccatggcag 7920
   tggcagaggg gctgtcagtt gcctctggga gccactcccc agggaaacac gagccaccac 7980
   cagtgagtgt gctgagggcg gggcagctgc tctgcactcc cgagctgggg gctctgcctg 8040
   gtaaagtggg ggtgggagct cacggggaag agagactgga ctcctctctg tctgatggct 8100
   gtggcataat gaccgggccc ccacacatga aaaagaattc tgggaactca aaaagccagt 8160
   gtgtccccac catggacccc ttggattgtg tttcaaattt ctcctcaatt tcgaagagcg 8220
   tcctggccat ccagattctg aattctataa ccctcgtttc attcatctca atgtagctaa 8280
   gaaccagatt tctggggaac tatcgagttg ccagagttct tgtgttgatt cttttttttt 8340
   tttctttttt ttttcttttt tgtggcagag tcttactctg tcgcccaggc tggagtgcag 8400
   tggcacgatc tcagctcact gctgcaacct tcacctcccg ggttcaagcg attctcctgt 8460
   ctcagcctcc tgagtagctg ggattacagg tgtgtgccac cacgcccggc taatttttgt 8520
   atgtttagta gagcccgggt tttgtcacgt tggtcaggct ggtctcaaac tcttgacctc 8580
   cggtgatctg cccacttcag cctcccaaag tgctgggatt acaggtgtga gccaccgcgc 8640
   ccggccttgg tgttgattct ttctcttgtg tgagggctgg tgttccttta actgtgatgt 8700
   cggttgagta cagtcgcttg gcttcatttc tgggtgtttt cagatgccag gactctgcac 8760
   aggatcttta tttgtggctg aatttttccc ttcattgtat actggcaaaa tttttcagtg 8820
   ttgtattttg aagtgtgatc cagtaggtgg cacttaaaag ggttggccag catacaggat 8880
   cttagccaca aggctctttt gtagttttgt ttcgtttttt gtttcgtttt ttgacacagg 8940
   gtcttgttct gtcgcccagg ctggagcaca gtggcacaat ctcggctcac tgcagcctct 9000
   acctatcagg ctaagtgatc ctcctgcctc agcctcctga gtagctggga ctacaagcac 9060
   gcagcaccat aaagagaaaa tttttgtaat tttttttttt tttttgtaga gatggggttt 9120
   caccatgttt accaggctgg tctcaaactc ctgggctcaa gcaatctgcc tgccttggcc 9180
   ccctaactct tgtattttga caaagtcggc agtagtgctc tgtggttgtg aggaggggtg 9240
   actccctcgc ctggtccatt cttgggcctt ggaggagcct cctacagtca ctggctctgc 9300
   acccactgtt tcctttgtta ggattgttct gcccacgggg ctccctcagg cagggcatgg 9360
   tgggcagaca ggctgtatcc ttccccggcc agccctatgg agggaggacc accccgcacc 9420
   tctgcaggct gatgaaatca ggtgtttcac ccctctgaac gttctgagaa tgagggctcc 9480
   tcacggcttg gtcgccacct aacgtggtga gtccttctca gcaagggtga ttggagccac 9540
   atgatctgcc atctcagtgc ttcccagggg aacacagagc tactgggcat ggtggctcac 9600
   acgtgtaatc ccagtacttt aggaggccga gatgggcaga ttgcgaagtc aggagactga 9660
   gaccatcctg gataacatgg tgaaaccccg tctctactaa aaatacaaaa aaaaattagc 9720
   caggcgtggt ggcgggcgcc tgtagtccca gctactcggg aggctgaggc agaagaatgg 9780
   tgtgaacccg ggaggcggag cttgcagtga gccaagatca caccactgca ctccagcctg 9840
   ggcaacagag tgagacttca tctcaacaaa aaaagaaaag aaaagaaaaa cacagagctg 9900
   cacaccccac agagttcagg cagaaggggg tctgcagcgc tggaagaccc agcaagcctg 9960
   gcccgtctgg ctgcaagtgg caggggtggg tggagtcacc cacttcacca tctgggtgct 10020
   ttccagggaa gcatgcagcc acgaccccgg gcagagttca ggcagaagct gggccactat 10080
   gctggaaact ggccttgagc cttgtggagt aacggcaggt ggagccatct cactgctccc 10140
   acgcaccatg cccgtggcct ctgcgggggc tgtggtaacg gcacccgact gctctggggt 10200
   caatgcctgc ggaggtcccc ctggcttcag tgttgcctct gcaaaaaccc cagttgcagc 10260
   caggtgcggt ggctcacgct tgtaatccca gcactttggg aggccgaggc aggtggatca 10320
   cttgaagtca ggagttcaag accagcctgg ccaacatggt gaaaccccgt ttctactaaa 10380
   aatacaaaaa ttatccaggc atggtggtgg gcacctgtaa tcccagctac tcggaaggct 10440
   gaggcagcag aattgcttga acccgggagg cggaggagct gagattgcac cactgcactg 10500
   cagcctgggc gacaaaacaa gactctgtct cgaaaaataa taacaataaa aaataaagat 10560
   ggcaaccata gacactggag actactagat gggggggaag aaagggggtt gaaaaactgc 10620
   ctattgggta ctatgctcag tacctgggtg acaggatcaa tcgtactcca aacctcagca 10680
   tcacaaatta tttaaatttt tctctttttt taattttttt gttgttgttg ttgagacgaa 10740
   gtctcactct gttgcccagg ctggagtgca gtggtgtgat ctcggctcac tgcaagctct 10800
   gcctcccagg ttcacgccat tctcctgccc caatctcccg agtagctggg actacacgcg 10860
   cccgccacca cgtcctgcta attttttgta ttttcagtag acacggggtt tcaccgtgtt 10920
   agccaggatg gtcttgatat cgtcaccttg tgatccaccc gcctcggcct cccaaagtgc 10980
   tgggaataca ggcgtgagct accgcacccg gcctaaattt ttttttaaat aaagaatggt 11040
   aggttcttca caccctaatg tatttttact tctcccacag agaaggaaag gaatggcttc 11100
   cccatggcaa gccacctcag tctgggcttt cttttcttcc aggggacttt cccatgcctt 11160
   tcatatctgc caaatcgagt cctgtgattc ccttggatgg atctgtgaaa atccagtgcc 11220
   aggccattcg tgaagcttac ctgacccagc tgatgatcat aaaaaactcc acgtaccgag 11280
   agataggcag aagactgaag ttttggaatg agactgatcc tgagttcgtc attgaccaca 11340
   tggacgcaaa caaggcaggg cgctatcagt gccaatatag gatagggcac tacaggttcc 11400
   ggtacagtga caccctggag ctggtagtga caggtaagga aacatccagg gtccacagcc 11460
   ctggtgtgat ttttttctta tttttaatag agtatttttc aagaagtttt agatttacaa 11520
   acaaaaaaaa attgatgatt gcttcagaga gttctcagcc atctggcacc ccacttcccc 11580
   cagagttaac atcttacatt agtatggcac atttcttacc attaatgaac aaatatcgac 11640
   acattcccag ctacagtcta cagtttattt acattttctt agtttttacc tgatagtctt 11700
   tctctgttcc aggatcccat tcaagatttc acattgcggc tgggagtggt ggctcacgcc 11760
   tgtaatccca acacttaggg aagccgaggc gggtggatca cccaaggtca ggagttcgag 11820
   accagcctgg ccaacatggt gaattccccg tctctactga aaatgcaaca atcgctgggc 11880
   gcggtggctc acgcctgtaa tcccaacact ttgggtggct gaggtgggtg gatcacctga 11940
   ggtcaggagt tcgagaccag cctggccaac acagtgaaac ctcgtctcta ctaaaaatgg 12000
   aaaaaattgg ccaggcctgg tggcacacgc ctgtaatccc agctacttgg gaggctgagg 12060
   caggagaatc gcttgaaccc aggaggcaga ggttgcagtg agccaagatc acaccactgc 12120
   actccaggct gggcgacagg gcgagactcc atctcacaca cacacacaca aaaagatttc 12180
   acattgcatt caggtgtcat gtatctttat tttttttttt tttttttttt ttgagatgga 12240
   gtcccgctgt gttgcccagg ctggagtgca gtggcacaat ctcggctcac tgcaagctcc 12300
   aacctcccgg gttcacgcca ttctcctgcc tcagcctccc gagtagctgg gactacaggc 12360
   gcccgccacc acgcctggct aattttttgt atttttagta gagatagggt ttcactgtgt 12420
   tagccaggat ggtctcaatc ttctgacctc gtgatccgcc cgcctggcct cccaaagtgc 12480
   tgggattact ggcgtgagcc accacgcccg gcccccgaaa atgctgggat tacaggcatg 12540
   agccaccgca cctggcctcc caaagtgctg ggattccagg cgtgagccac cgtgcccggc 12600
   aggtgtcatg tatctttagg tttgtcttgg ctgtcacagc ttctcagatg ttgctggttt 12660
   tccatgacct tgtcagtttt gagggtagtg gtccattatt ttcaagggta ctcccactac 12720
   tggaaattgt ccgatgtttt gctcatgact agactgagtt atgggtcatt gcaggcaaga 12780
   ccacagaagc aaagtgccat ttcatctcct catagcaaag gtttaaactg tccatgggaa 12840
   catgactgtg gatgttgagc tggctgttgt tgaaagcctg gctgaagtag taactgtggc 12900
   cagacaccgt ggctcgtgcc tgtaatccca gcactttggg aggctgggcg ccgtggctca 12960
   cgcctgtaat cccagcactt tgagaagccg agatgggcag atcacttaag cccaggagac 13020
   cagcctgggc aacatagtaa gaccccatct gtacaaaaaa tcaaaaaatt agctgggcat 13080
   ggtggcaccc acctgtagtc tcagttactt gagaggctga gatggtagga tcacctgagc 13140
   ctgggaggtc gaggctgcag tgagccgtga ttatgccact gccctcagcc tgggcgacag 13200
   agtgagaccc tctctaaaat aaataaattc taaaaaagaa aaaagaggct gggcactgtg 13260
   gttcacgcct gtaatcccag cactttggga ggctgaggca ggtggatcac ctgaggtcag 13320
   ggattcaaga ccagcctgac caacatggag aaacctcatc tttactaaaa atacaaaaat 13380
   tagctgggcg tggtggcggg tgcctgtaat cccagctact cgggaggctg aggcaggaga 13440
   ctcacttgaa cctcggaggt ggaggttgca gtgagctgag atcgtgccac tgcactgcag 13500
   cctcagtgac agagtgagac tccatctcaa aaaacaataa taggctgggc acagttgctc 13560
   atgcctgtaa tcccagcact ttgggaggcc aaggtgggca aatcacctga ggtcaggagt 13620
   tcgagaccag cctgaccaac atggagagac cccgtctcta ctaaaaatac aaaaattagc 13680
   tgggcgtggt ggtacgcacc tgtaatccca gtttctcggg aggctgaggc aggagaattg 13740
   cttgaacccg ggagacggag gttgcagtga gctgagatca cgccactgca ctccagcttg 13800
   ggcaataaga gcgaaactcc atctcaaaaa aatatataat aataacaata ataagaagaa 13860
   gaaaagaata aaggagaaaa ggtctttcta atagctcact cttttctctc ttaggcttgt 13920
   atggcaaacc cttcctctct gcagatcggg gtctggtgtt gatgccagga gagaatattt 13980
   ccctcacgtg cagctcagca cacatcccat ttgatagatt ttcactggcc aaggagggag 14040
   aactttctct gccacagcac caaagtgggg aacacccggc caacttctct ttgggtcctg 14100
   tggacctcaa tgtctcaggg atctacaggt gctacggttg gtacaacagg agcccctacc 14160
   tgtggtcctt ccccagtaat gccttggagc ttgtggtcac aggtaggtac cgcccagtcc 14220
   agccctgtgt ctgggttggc tgtccagggc cttgccaccg ggcaggaata tgaagacgtg 14280
   cactgagagt gaagtgaaga gaggcaaagg ctctcactcc aggacagtgg agagagaaag 14340
   gcttccccac cacactttcc gctttcactt cctcgctaga gttctccaga cagggttcat 14400
   tgaaaactta gtctgtggag aacagaaggg ctaactcagt ttgtttcatt ttatttattt 14460
   cattttattt tccgggatag agtcttgctc tttcgccaag gctggagtgc agtggcacga 14520
   tctcgactca ctgcaacctt cgcctcccag gttcaagcaa ttctcctgcc tcagcctcct 14580
   gagtagctgg gaccacacag acagggtttc accatgttgg ccaggctggt ctcgaactcc 14640
   cgacctcagg tgatccacct gcctcggcct cccaaagtgc tgggattaca ggcgtgagcc 14700
   accgcgcctg gccaggctgc acacattctt attaggattc caccttgttc tggtgttgta 14760
   gagatgtgat taggtattta gtgaattcac caagtgagga gagaatgaaa agaaaacaca 14820
   acctgcctgg ccgggcgtgg tggcgtgagc ctgtcgtccc agctactcag gaggctgagg 14880
   caggagaatc acttgaaccc aggaggcagc tgttgcagtg agccaagatc acgccattgc 14940
   actccagcct gggtgacaga acgagactcc acctcaagaa aaaaaaaaaa aacatggttg 15000
   ggcacgatgg ctcacgcctg taatctgagc acattgggag gctgaggcag gtggatcacc 15060
   tgaggtcggg agttcgagac cagcctggcc aacatagtga aaccccatct ccactaaaaa 15120
   tacaaaaatt aaccaggcgt ggtggtggtg ggcgcctgta atcccagcta cttgggaggc 15180
   tgaggcagga gaatcacttg accagggagg cggaggttgc agtgagccga gatcacgcca 15240
   ctgcactcca gcctgggcaa cagagtgaga ctccatctca aaaaaaaaaa aaaaaaaaaa 15300
   cacacacaac ctgcccataa tcacctcctt cccagtttat agcacttccc tgggaagcac 15360
   agttccttgc ccgtgaacac agtcttgctg actgatcagt gtggtgctgg cgaagcatga 15420
   gctcattgag gggatgcttg agggagtccc attttggcaa gcgaaaagga aaatgagctc 15480
   ccgtttcagg gctctggggt tgggatggaa tggaacacaa ccaccaacca ttcatctcct 15540
   tgaattgtgt ctccagactc catccaccaa gattacacga cgcagaactt gatccgcatg 15600
   gccgtggcag gactggtcct cgtggctctc ttggccatac tggttgaaaa ttggcacagc 15660
   catacggcac tgaacaagga agcctcggca gatgtggctg aaccgagctg gagccaacag 15720
   atgtgtcagc caggattgac ctttgcacga acaccaagtg tctgcaagta aacacctgga 15780
   ggtgaaggca gagaggagcc aggactgtgg agtccgacaa agctacttga aggacacaag 15840
   agagaaaagc tcactaagaa gcttgaatct actttttttt ttttttgaga cagagtctgg 15900
   ctctgtcacc caggctggag tgcagtggag caatctcggc tcattgaacc tcttgggttc 15960
   aagtgattct tgtgcctcag cctcccaagt agctggaatt acaggcacat accactgcac 16020
   ccagctaatt tttgtatttt tagtagagat ggggtttcac tgtgttggcc aggctggtct 16080
   cgaactcctg acctcaggtg atccacccac cttggcctcc caaagtgctg agattatagg 16140
   catgagccac cacgcctggc cagatgcatg ttcaaaccaa tcaaatggtg ttttcttatg 16200
   caggactgat cgatttgcac ccacctttct gcacataagt tatggttttc catcttatct 16260
   gtcttctgat tttttatatc ctgtttaatt tcttccttca ttgttcttct ctttttttat 16320
   ttattttatt tatttttatt tttattttta tttgagacag agtctcactc tgttgcccag    16380
   gctggagtgc agtggcacga tctcggctca ctgcaacctc tgcctcctgg gttcaagtga    16440
   ttctcctgcc tcggcctccc aagtagctgg gattgcaggc tcccaccatc acgcccagct    16500
   acttttacag tatttttagt agagacgggg tttcatcaca ttggccaagc tggtctcaaa    16560
   cttctgacct cgtgatctgc ccgcctcggc ctcccaaagt gctgggatta cagatgtgag    16620
   ccactgcgcc cagccttctt tttatatttt taaatgtgtc ttccccaaat ataaatggtt    16680
   ggtaagcatg ccaaatatat tcaataaccc ccctccttta tttttttttg ttgaagtgag    16740
   gctctcccta tgttgcctaa gctggtcttg aactcctggt ctcaagcaat cctcctacct    16800
   cagcctcctg ctgtgttcat ctacaaattg ataagagtga aagtcataat cctacaggag    16860
   gattacccta tttatttcac aaaccctatt tctaccggat tttcatacaa ggaatacagg    16920
   catgtgtttc acctcattaa tttatttttt cacttagttt tgatgatatt cacatatatt    16980
   atcaagtgtg caaacattaa attcttgtgt acaaaactca aatggtcttc caaataattc    17040
   cccattcttt tttcttataa actttcacag ctttaccctt gacagacttt actcaaggaa    17100
   atctaagttg gtcatatgtg gctctttcac tgattgctat ttacttcatt gtccagtagc    17160
   ttatgtatga aaatataatt ataaaatgta agggtcctac ttccagtgaa actgaaggga    17220
   cttaggccca cttttatcct ttactgagag cttatctcta cttgataaaa tttctactgt    17280
   attcttggct taactcaggt cctgtgatta aaaaaaaaat gcaaagta                 17328
<210> 30
   <211> 45779
   <212> DNA
   <213> Homo sapiens
<400> 30
   acttccttct tttccttcac ctcggcccgg ctctgaaagg atcgggcccc tccggcccgc 60
   acccaccccc aagaggggcc ttcagctttg gggctcagag gcacgacctc ctggggaggg 120
   ttaaaaggca gacgcccccc cgccccccgc gcccccgcgc cccgactcct tcgccgcctc 180
   cagcctctcg ccagtgggaa gcggggagca gccgcgcggc cggagtccgg aggcgagggg 240
   aggtcggccg caacttcccc ggtccacctt aagaggacga tgtagccagc tcgcagcgct 300
   gaccttagaa aaacaagttt gcgcaaagtg gagcggggac ccggcctctg ggcagccccg 360
   gcggcgcttc cagtgccttc cagccctcgc gggcggcgca ggtccgggcc cggcagggac 420
   ggggaggggc agagcgggac cggtcggggc tggggaccag ccgcgcgggc ttctgaacgg 480
   gcacgctgtc ggagccggag gctcgtctag tcccgagccc cagcgggccg cggcggctcg 540
   cggggccgcc ttcccgggac agcagccggg gcccgggcgc cggcgcgggg gaggacgctg 600
   ccctccccgg ccgggcgcgc agctcgcagc cctcgctccc tcccccgggg cgcgcaggcg 660
   ggcgctcgct cgcagagaag gtgccggcgg ggccggagcc ccgaagcctc cccgggcggc 720
   gggaagacgg gaggagggga aagggaagga gggagggggt cgggctgccg ggagggcggg 780
   ggcagcggcc gggactgcgg gccgggagcg agcccagccg cccgcgccgt gtggagcccg 840
   ggcggggcag gtgggcagcg gcgggggcgg ctccgcgcgg ccgggctcgg ccgtgctgcc 900
   cctcgcccct gctcgcgcgg tcctctctcc cgcgcggtct gtcggtctgc gcgccgcctc 960
   gctcctcgct cctcgctctc cccctctttc tccccctctc gcctctccgt gtctctggct 1020
   ctctgcctgg ctcccttggg tctgtttctc tctcctgccg cctctctctg gccgcccctg 1080
   gctttatttc tcgcgcgctt ggggtctctc ccagtctccg tctctccatt tctcctgggg 1140
   ggcggggagg gggggtctcc aaaaaccgcg gcggcggcgg cggccgctcc aggcgcccgt 1200
   tccggagtcg gggggaggcc cagccgggag gggggaaggg ggggagcctt agtcatttcc 1260
   ccgctccagc ctgctcccgc ccgagcgcgc actcacggcc gctctccctc ctcgctccgc 1320
   agccgcggcc catggagccc gccggcccgg cccccggccg cctcgggccg ctgctctgcc 1380
   tgctgctcgc cgcgtcctgc gcctggtcag gtaagcaccc ccccgctccc caccgctgca 1440
   ctccccaaac tgcgggctca ggcctctcag actggcactg ggaccccttc ggctaatgcc 1500
   gagcagtaat agggggagag acgcctgtaa ccagggttat agatgcagaa actgaggccc 1560
   agagagggcc agcgacatgc ccaaggtcac acagccaagg gtagaaccca agtctcttct 1620
   atgagagttt gtctctgtgg actctgactc tgtgcactgt gctgggcact gagacaaaag 1680
   agccgagttg cttcactcag cccctgcccg gtccaatggg gaaggctgac tgacagtggc 1740
   tttcagagca gggccgtccg gcttccccag aatctgtccc catggacata cgactttcca 1800
   catgtttgac ttcagggtgt gaggggagga ggtgagggca gaggtcctcc cgatgctgcc 1860
   tggaatggaa cagagacaag ctgttgcaat tagggaagag tgagccctcc ctctggtgcg 1920
   cagagtaaac cggtgttagt gtgtgcctgg gagagaggga gccgggaggc cgcggtaacc 1980
   ccagagcgag ctgctgcgag caacagatcc ttatgtaaaa acacggtgga ttgtccctgt 2040
   ggttgccaaa gcctctccct ggtggtcttg ctccttgagg cgctcccagc tcccccttcc 2100
   tcctgccttc cccgagcttc caggcagctc cctgcagttc aggtgctccg cggggatgtt 2160
   acctgtggct ctgaccatgt ctttctgttg gcctacatca aacacctggc tgtcgccggc 2220
   aggcgggctc tgaaggctac tgtgcatgtt caccaaaggg tgaggtcacc attcaggagt 2280
   cttgcttctt aaatgggttt tgcaaaacca tgggtctcct gggagggcac cgggtggggg 2340
   gcggggggag cagagaaatg gcattaaggg agttgggagg gagagaaaaa tggggatctt 2400
   gttttttgtc gtcttgaaac tgtgttgtag gcagtggggt ctctctgtac ctcccaccct 2460
   tgaggaggga ggtaaagatg agctcagtgt ccaacacccc cttttgcgtt tgactagaga 2520
   tggtaaccat ctgatagcag ggagaaaggg agaactagca aagagtgggt ggaaggagaa 2580
   gtatgggggg tagacagctg cttctctctg ccctgcagtt tcccctgaaa cccaggaatt 2640
   cacataggca tccccccacc ccaccccacc ctccaccccc agttcttttc agttatagtt 2700
   gcgtccttgc tgggctgaag ggagcactga tacagttcac gtctctccag tgagtggcat 2760
   ctcctatctg ctccttcctg cctgagctgg ccatttgtac cctgcaaaag tccgagttcc 2820
   tgccctgcca tccttcctgg cctggaaatg cacaagtgtc actcacaagt gcttaatttt 2880
   cctgaagcag ggaattgggc ccgagttaat ttctaaatgt ctattccaga cccttctccg 2940
   accaggaggc tgtggttcag tccctggcaa acgagcagtg acgctgatta gcaagtgctc 3000
   cgatgggcag cctgcctttc ctggggcacc ggctgggaac accaacacct ttcttgtgag 3060
   cacatctccc ccttttgcct ataaaaacag tctataagat ggtaaagatt gggccataaa 3120
   acttccgttc caatccattc agcatcaata gcctgaaaaa gtgggagcag cagaggggcc 3180
   aagattagct ttgaaataaa taaatcttag gaggtaagaa tgaatttctt ggtcgagggt 3240
   ggtggggaag ggatgaggtg cgaccatcat agctcaagtc tcccattatc cttcagtatc 3300
   tgttcttcgg ggtcctggtc acaccatggg cctgggcagg gaagtcacct ggtttggctg 3360
   aaggatgggg cctcaggccc ctccccctat tgctccagac atggaattta caacaaagaa 3420
   gtagagagac aggatatgag ggtaggaagg tacaaggagg cagttgtcca attttctaag 3480
   aaaatggatt tgttcgagtg agaaactaaa atgtcaggtt taggaaggcg gctttggatt 3540
   gaatgactac tagtgtctca ggcagaactg tcagcctggg aggtgtcaca tgattcctgt 3600
   gtcttcacat gatcactgtt ttgggggggt ggggccaatc atgtgaatta tcctgggtgg 3660
   gggaggcaag agaagaagtt tctaaagatt gcttttagca caaatgctgc caaagggaaa 3720
   ttctaaaaag atcccagtga agtcaccaac ctatgttttt ggacccaatg tgaggcctgt 3780
   ttcctaaaac aactgcactg agggagattc tgaggctggg agaagggcaa gggactgagg 3840
   cctggggaca cctggcacct gattttgtgt cagcagggcc tgacgctgaa cccgcaggat 3900
   gcctgcttgg ccccaggtag gccttgagac ctctgagtca ccccccaggc cgacctcaga 3960
   taaactccca ttcatttctt ctctctggcc tggaggaggc caagccaagt tgcagttcag 4020
   gctctgggga gcagcggggc cccccagtga acctctcacc gtttggtaga gggtggcact 4080
   ctgggggctc ctctgtactt tcctaaatgg taaagttaat ctcacttcct ctttcgtaag 4140
   aagagaaaac ggggcgggag tgctttggct caacacatca ggctgttgga agttccttta 4200
   aagccatagt tttctctgag tactttctgg cctttaccaa cccctcggaa actcacggta 4260
   attcacgtct gtacttgcag tcatgcagca cctatgcagt gctggtgtga accatgtgtt 4320
   gagagaaggg aggaaaacca acattcatta gatagttaat ggcgttccag gccggatgct 4380
   gctcctgcgt cgtctcaccc gcaagacatg gattggctct tctccccatt ctgcagatgc 4440
   agaaaatcga gtcccaaact ggggaactaa cttataagtg atagagagga gccaggattt 4500
   tggatgctgg ccttcctgct gggtggcttc tagggaagaa gctcaatgac ttaatcagga 4560
   tgatttagtc caagctggag agggagtgag agaggcccct ttctggcctt gggtggagga 4620
   ctgtagaaga gttctttctc tgggcagaag gttttgatga agtcacaggc atttctctga 4680
   aagcctgctg agggtggaaa tactgacagc cccttggtcc tcaccagtaa caacacatga 4740
   gaggtgatgt ggctcctgtg agagcccccc atctgggctg ctgagctctg gagcccccag 4800
   agctggaata ttcctccctg catcttcctg gtgccctttg cgacagatgg agagggggtg 4860
   tgcgagagga agggagtcta aaagtgttgt acggatccca cacgcgctcg cttgctgctt 4920
   ccaatggctc cgtgcgacag gttattttct tatttgtagt cttggactga cggaaggttt 4980
   cccccctctc tctctctttt taaatgtgct cagatttctg gcctgtcaac acaactcaga 5040
   tcatgtcact ccccagctca aaactgttga gtgggtccaa gttttcttta aaaaaaaaaa 5100
   acaaaaaaac acaaagctgc cgtgaggcct gggcttcctc cctgccaggc ctgatttcca 5160
   gccacatgtt ttccaacaag ctgacctgtt gagatgctgc aggataacca ggcttcctcc 5220
   ctgcagacca gagctttgct ggctactcag ccacacgagc accccaaaga ccctgagaag 5280
   ccctgcaatg tagaatcccg agctgggtca catttactcc agtgttccct gaacttatga 5340
   acgagaggca tttgatttgt agtatgcccg ttagcatgtg gcctctctcc gtttggtaaa 5400
   cggttttcta cagatccatg ccctcagctg aactctcagc tctgtgcctt ggcccgtgct 5460
   gttcctgcca tcagaaatgc cctccctgta cctccttgtc ttcctgtgtc tgaatcctac 5520
   tcatcctccg aggttcaatt taatgtgcct cctggggaga ggtcagagga gcctctccac 5580
   gcagacacac accctggcct agcgctaaga tggtgctaag acccctgggt gttgatctga 5640
   gccctgctac tgacttggac aatttgcagg aatctgtgtc tcattttgct catctgtaaa 5700
   agaggaatgg tattaaccac tctattgcaa ggattaagtt aattaatgcg tgtataatgc 5760
   tggaagccat gccttgtatg tagtaagcac tcaatacata ttaaccagtg ttgtaattag 5820
   catggcttta ttctggtttg attcctcttt tttaacaatt gggacggatt ttcataaaac 5880
   atctaacaca gttgcttaca catagggggt acttaaaaaa cagcaaaggc tgttcagtga 5940
   acaaatgtcc ttcagcttga tattttcctt gtagcttttt ttttttcttt tttttttttt 6000
   tgagatggag tttcactctt gttgcccagg ctggagtgca atggcgtgat ctcggctcaa 6060
   cgcaacctcc gcctcctggg ttcaagcgat tctcctgcct cagcctcccg agtagctggg 6120
   attacaggga tgcgccacca tgcctggctg attttgtatt tttagtagag acaaggtttc 6180
   tccgtgttgg tcaggctggt ctcgaactcc tgaccccagg tgatccacct cccaaagtgc 6240
   tgggattaca ggctgagcca ccgcaccacc cagctgtcct tgtagctttt atgctgttgg 6300
   gggatggaca agttgagtag aaacccaggc tagagtgatt taattgcctg ttactaggga 6360
   ctgagtgctg tgggcacagc ctgcctctag gtgtctctat ctggatccac aggtctgcag 6420
   ttgtggattc ccggacctgc aaagcagtag ggggcctgag agagccctct gtgctttcat 6480
   ccagtgcctt atggtgcggc cagaagaaca gagagaaaaa aactggccgg gcccacgcgg 6540
   gagatccatg gcaaggcccg gctggaacgc caagcctcct gtggtccagg ccaaggcttt 6600
   taaaaactgg gctgtgctgg aactttccac tgtttcagcc agaaatctga caggatcccg 6660
   aattccccgg gaaaacacta aagtaatact aataggaacc atctgggttc tctgcttcct 6720
   gggctcagca cttggtgtgt gtgaccctgg tggtactcac agccacctgg cgatgcacgt 6780
   cgttatcttc ggacccgttg gatctgtgag gaaactgagg ctctgaatgg tgaagcagct 6840
   tgcccaaggc cacaccgtaa gcgccagatg tggataacca ctgtcggcct aatcacagag 6900
   cctatgtggt taactgttgt gccattcctt agccgtcact gaggcttgct tgacaggtag 6960
   ggaaactgag gcccagttgt gctggcaccc tgctccctta gtgctcctct catttttgtt 7020
   tttgtttttt cttcctttct ttctttcttt tttttttttt tttttttttt ttgagacgga 7080
   gtcttgctct gtcgcccagg ctggagtgca gtggtgcgac ctcggctcac tgcagcctct 7140
   gcctcctggg ttcaagcaat tctcctatct cagcctcccc agtagctagg actacaggcg 7200
   cacaccgtca cgcccagcta atttttgtgt ttttagtaga gactgggttt caccatattg 7260
   atcaggctgg tctcaaactc ctgacctcag gtgatcctcc cgcctcggcc tcccaaagtg 7320
   ctgggattac aggcgtgagc cactgcaccc ggcctcctct cgtatttcta tcagtgcacc 7380
   ttcctatgag gtgggggcag tgtgtgtccc caaggtgtgt ccctgtcccc atactagaga 7440
   aggaagcatc tattttcaca gctgaaactg ctttcctgtt aactgctggt gttcctggga 7500
   tgggtattct gatgaagccc tccacgagat ggattttccc tgccaggtgt cttgccaggc 7560
   cctcagtcca gagagatgca aaacagctga ggtggaagtc ggaaatgctg cagaattggt 7620
   tctttgtgtc tgtaagattt cctggcacat tcgttcgggg gcgcagggtg ttagatggct 7680
   gcagattagc gccaagagcc caaggagatc aggtgctcgg atcaggaggg aaattggaag 7740
   ccgggagtga gcgatgggcc tcccagccag gagggacggc tggagcaggt gggcggtgat 7800
   accagcgccg tttctacagc tccattcttc aggccgaccg tcggtgttct ccagaaatgg 7860
   aagcccgtgg tggaaggaaa tactaggtac acgatgtgct cggcacaggc tgagccttga 7920
   acatgtgtta gctcttcctt atctttttat tttggaaaat ttcaaacata tgcaaaatga 7980
   gaaggaatga tgtaatgagc ctcagctttg acactgatca actctatacc atgcttgtcc 8040
   cctgccccta tttttgctag ggagaggaga atcggggagt atataaagca agtgctaaac 8100
   atggggtatc atttcaccca ccgatactct ctccacatcc cttcatgtga gctgttacct 8160
   gaccctcacc acgagccaaa cagtatggcc cttcatcccc actttacaga taaggcaact 8220
   gaggcttccc aaggccaagt ggggctcatc cttaggccac acagccagtg agaggtaaag 8280
   ccaggctcca ctcctcactt tacccttaac cactttgcag tcattattag ggtcatttta 8340
   attgtaaata ttcattcaac aaatattttt gagcacctac tatgtgccag ggatggctct 8400
   aggtgcttgg aggatacaaa tgtgaacaag acagacaaaa acacctgctc tcgtgcagca 8460
   gacattctag tgaggaagag aaaaacacac ccaagtaaac aaatgatgcc aggtagctgg 8520
   ggagaaaagc aggaggaggg aagggagaag gttagtcagg acagagggag ggccgctcta 8580
   aggagcagag gcctcaagaa aatgtacaat taaggccagg tgcggtggct cacgcctata 8640
   atcccagcac tttgggaggc tgaggtgggc agatcacctg aggtcaggag tttgagacca 8700
   gcctggccaa catggcgaaa tcccatctcc actaaaaata caaaaattag ccgggcatgg 8760
   tggcgtgcgc ctggaatccc agctactctg gaggctgcgg caggagaatc gcttgacccc 8820
   aggaggcaga ggttgcagtg agccgagatt gcaccactgc actcccgcct gggtgacaga 8880
   gcaagactct gtctcaaaaa aaaaaaaaaa aaaaaagaaa agaaagaaaa gaaaatggag 8940
   gagtagccac gggggatagc aagagccctc catgtgggag gaatggcaga tgcaaaggcc 9000
   ctgaggcagg aatgagcttg tatgttccag gaattgcaaa gaggccagtg gggctggagt 9060
   gaaagtagaa gaggtggggg agtgagcaga ggtcagggcc agaccagcag gatctggctc 9120
   attgagcacc taacatggag catgtctggc ttgtgagagg atcattatgc attctctcat 9180
   ttaattctca ccacgggttc tgtaagatgg gaaataccgt taatccattg gacagatgaa 9240
   gaaactgaga ctcagaaagg taaagtgact tgcctaaggt cacacagctt ttatgtgcag 9300
   aactaggatt taggtccctg aatgctgcag catccccatt gctgctttcc gcagccgtcc 9360
   ttgccccctt cgggctcttc acccatagca gctggagtga atcaagtcat ctttggttct 9420
   gctggaaacc tttttcggtt tccagcatgg catttctgga ccacgtctgt ctcttcatcc 9480
   tcacctctta ccactctcct gcctccttgg gcttcagcct tggcagcgtc ctttctggcc 9540
   cttcaccagc ttctttccca cccgagggcc tttgcactgg ctgtctcttc tacgggagca 9600
   tcttccctgg tcttcacttc ctttgggtct cagctgccac ctcccttcct tggatgctgt 9660
   cccaaccacc ccattccaaa agaggttatc gcttgtgatt ctctatcact gggccaaatc 9720
   tgcctgttgc ctagtgctcc tagccctttc tatattaatt catttcatta tttcagccct 9780
   gtctcctcca ctaggatata agctgcacga gagatgggct atgtctctta ctcactgttg 9840
   ggcctcccag gacctcagca cagggcctga cacacagtag gtgggttaat agatactgat 9900
   tgaatgaaag aacaaggtgg gtactaaaat ccccctttta aagaggaggc ttggagttat 9960
   gaggttgtac gagcaaagct tattccctaa ctaccacagt cccccccctg cagccagggc 10020
   cgggatcata ccacaattcc ctgcccatca agctgtcatg aatagcccta agatatcagg 10080
   aagcacaaag atatcaatgc caggagggat aggaggaggc ggccaagaaa ccccggaatt 10140
   tgtacttaaa attctggcta cgcaaactgc ttgcccaact ttagacacca tgatgaatga 10200
   tcaggtgctg caaccatgaa acatgacgta attagtgctg cactcttggc tgggggttgg 10260
   cttggaggcc ccacctcgag gttagcctcc cccagagggt cactgcacga ggcagtctgg 10320
   tgcggggtgg gggttacaac agagggaggt ggaatcagac cactcggcag ccctgtgacc 10380
   atggccaaat ggttgtacct cccaaagctt cacatctggt cctccgtgaa atgggggtgg 10440
   cgacagccct gtcttactgg gctcctgcag aggttccatg aggcctgtct tgctctgggc 10500
   agcgtcgatg cttattagct attgttactg ctgttatgtg attgtcatcg ttttatggtt 10560
   tgggaatttt aaaagctcca tcttttgcca gcggggagac ctggaaccca ctcctctgcc 10620
   ccctcagcct cagtttcccc ttctgtgacc gggaggcttg gctcggctcc tctcttgcag 10680
   gtcccttcca gccctgatgc tccagcctgc atgtcctcct ggccctttgt cctgatgact 10740
   ccacagactc ctctgggagg agaggccatg gaggggcttc tgctctgggg cacattcctg 10800
   aattggagtc cagcatgtgc cgcttactgg ctgtgtgctc tggggcaggt gtcttcccag 10860
   ctctgagcct ccgtttcccc atctgcaaaa tggaaccgct aacactgttt cctcccaaat 10920
   gggtgactgt tgagggttaa ggaattaaga gccgtggaaa gtgcttttgc aagctgcaaa 10980
   atgctctata aaaggaatca gtgaatagtg tcagcttggg gaaaaatccc gtcagtccgg 11040
   agactgtctg ctgccggcag tgtgctgaaa ccctggcaaa tgtcgtttaa taaatgcgtg 11100
   ttgcttttaa gagggccttt atctcagctg ggaaaatgtg tgcgcatcct tggcaacctg 11160
   agaatccaga agcctgaact ggaggggacc tgtcacaggg agaggcgatc gcatggggtg 11220
   gctccagcct ccagtgttgg aatcccacct ctgccactta cctacctctc cagactctgc 11280
   ttcctcatct gtaggagggg acagtgacaa tagcacctgc cactctgggc caggatgtcc 11340
   tgagggctaa atgaaggctt ctggttatct ggcctaaccc atgcatttcc cccatgaaag 11400
   tgtggctgtg cagcaccagg gcctgtctca gggcaatggt tggggcctgg agattgggag 11460
   gacctcccaa cctcagcctc tccaggcctt actccaggcc tcagtttctt cctctgcaaa 11520
   atgaggagga ggaaaggtcc ttaggttgtc aaggcaccaa gcctagtaga tgtcaatgtc 11580
   ctctacctgc tctctcagcc tctgccggtc ccccgaagcc tgttaattct aaaggatatt 11640
   tacacagatt tgcagttctt ttcctgccac tttgcaaaca gggtcacact gcaagcatcg 11700
   cttcatcacc tccaggtttc atctgaaccc agcaaatgct ccatagggct cccagtgccg 11760
   cctgtccccg ctgcctcctt gccagcagtg tgctcagcct gcttacaggc ccatgccaga 11820
   ggggcctcat gcatgcatca aggcagagct ggttctgcct cggagaggat cccgagcctc 11880
   tcaggcttaa ggacgtggtc atctgtcgcc cccttcatct ttagacaccc atctgcatgg 11940
   tgacagctgc tctgcattga gtacttactc tgtgccaggc acgctagccc gtttcatcct 12000
   cagaacaacc ctagtattgt tatcatcatc atcatcatca gacccattct acagataaag 12060
   agccagaggt ccagagtggg cgagacattc cctggagtct catgggtagc agagttggaa 12120
   tccaaatcaa aacatacttt cagccagtgc caggcactgc tccaaccctg gggaagaaaa 12180
   ctcagttcct gctctcaaag gacttataag tgagtctatg acagggagat gagcgctttt 12240
   aaaaaattaa caaggaaatg cgtagctttt tgagtgagga tgacatatcg aacaatgaag 12300
   cagagccagg gacattgagg tgggtagtat tttagacaga atggccaagg aaggcctctc 12360
   agataggcct gaaggagagg agcgagccag acatgtagat atttagggat gagagtgcca 12420
   ggcagaggca atggcgagtg caaaggtcat gtggtgagtg gagaatctgg cacgattggg 12480
   ggaaagcaag gctgtaggcc cctggggcca ctgtaaaaat tctgggtttc actctgagtg 12540
   cagtgggggc tgtgggaggg ttttgcacaa aggaggaacg tcacctgact taggttttag 12600
   aggcttctct ggctgctggg ggaatgtggc tggtagagag gtgaggcaga ggcagggagg 12660
   tcagcgtgga ggttcagtca gggtggtaag aagaggtcag ggtctggcca tgtgttggaa 12720
   gttgagccag caggatctac taacagatgg gttgtggaac acggatgaaa gggagtcaag 12780
   gaaatgtctc attctgagtc cagcacttct aaccacagag cctctgacct ggggagggag 12840
   ggggctgcat ttcctgttta caggtgagga aacaggctta gagaaaagca actttgccaa 12900
   gcaccgtgct acagacaaga cataaaggtc atctggcccc agaacctgag ctctggacag 12960
   tcttagcacc ttagaagttt acctactgca tggatggggc caccagagtc tgccccttcc 13020
   ctgcagggat gtggcacagc actgagttct cagagctacc cagggcaagg gtgaaggagt 13080
   ggggaccctg agcccctggg ccagctgtca gcttatttac caggccacac gcatccccat 13140
   ttttggtggc ttcaatgcag gtagcagttt ctatcacagc acctggtttg tccctgccca 13200
   atgggtgtat ccagtggtgc cggcagaatg ttcccagtgc tggcctgctc ttcagatggg 13260
   caaccgaaac catccttcag ggcgctaggt tagcccagtc ccaggtcctc cccatctcag 13320
   cttccttaca gccagtgagc atgttccaag gactctaccc tgagtcctgt cacctctgcg 13380
   tctttgctca tgtggttccc tcctcttgga atgccttcct tcatcccagc ctctgtcagc 13440
   ctttaagacc tagctcaggg gccaccagct ccaggaagtc ttcctgggtt tcactctctt 13500
   ccagaacccc atccctaaac ctcctcttgc aacaccgatc gccatgtgcg tggtcattgc 13560
   tcatgtccag gacggcccca catgctaaga tagtctcaag ggcaagggcc tgtcttactt 13620
   ggctctgttt tctgtggtat ctaggacagg gcctggcagc agagtgaggt ttgagagggc 13680
   atgaatgaat cattctgact ccagaggaaa tcactgtgta gtcctggctg ttggagcccc 13740
   aagctctctt cttgggaact gtggcatcca tgcagccatc cactggtgca gccccggcca 13800
   tgaatgcagc cccagctacc tttcgccttc ctgcatgggg tcatgggctc cagcagatgg 13860
   ttctgggcta gcctgttgac agctgtgccc tgtggccagc acatagtaag taggtgctct 13920
   ggaaaggtta gcacctccta tcaagggcac gaccctagac agataccaca cagggtgttg 13980
   accgccagga gcccatggca gacttgaggg aagtgtgtct cctcacatcc tccatcaccc 14040
   ttgtgcttgt ggaaacttac acagcaacct catctattcc aggcacatgc ccagctggac 14100
   acactcccga acacaggcac acggacacct ggggtgcaca caaatagtgg catgtgcata 14160
   cctccctgct tgtcaatgca cacaccctca tagtcacatt tacgcataca gcactctgtc 14220
   acacacgtgc caccccccac acacaccctc tacacacaca tacacaccac actcacgtgc 14280
   atgccgctcc tgcatcactc tcccacacac tcacaccctc ccacacatgc ccaggccacc 14340
   atgattcaga cactctcctg cacacaattc acatgcccac gcccatgatc cctagccccc 14400
   cattcagaca cagtcctgtg ttgcactctg acatattccg gcacacaggc accgtctctt 14460
   tcacactcct ccttgtgccc acccatgtgt acatgtgtgt gcagatgctt atgctctgta 14520
   cacgcataca tgtatgtaca cacgtgtgtt ctcgtgtgca tactcttatg caccctcagc 14580
   acactcatgt tcacatgttc cattgtacag ccctgtacta catcctgtgt gcatccgctt 14640
   taatgtacac tcatgttcac gtgttctatt gcacagccct gtactatatc ctgtgtacat 14700
   cctctttaat gcactcttaa tgttcacatg ttctctgcat agccctttat tacatcctgt 14760
   ttgcatccgc cttaatgcac atttatgttc acatgtgcta ttgcaaggtc ctgtactaca 14820
   tcctgtgagc accctcttta atgcacactc ttgcactgca tactctggca cacagcatta 14880
   aatcacaccc agaagatagt agaggcagcc cccgatgagg agctgctata agtgcggggt 14940
   ggaaaatcgc agccttctga ggatggtgaa taacaataat gatgagggaa gaagggagcc 15000
   gggtatcact gcagccactt tagaatgttg agccctgtgt actgccagag ctccagccat 15060
   agcctcccag accctggggg tccttgagta caaacaaaga atctacaaag aatccaaaca 15120
   ggtggcctcc tgcctgtccc tgtggcttgt gctgtggtga cccactgctt ccgggggatg 15180
   ggcaagctcc catgcagtgg cagtgagcac ccgcctgggc aaggctggtt cttgggaaat 15240
   gtgcctgttg gcttccttct ccccacccct tgagacaggc ttctggaaga ctcacacccc 15300
   agggtaaggc agccctcccc atccccgcct caagcttcag ttctaagttg attttttttt 15360
   ctattagaaa aaacatccat gtgggcaggg cgcagtggct catgcctgta atcccagcac 15420
   tttgggaggc tgaggcgggc agatcgcctg aggtcaggag tttgagacca gcctggccaa 15480
   catggtgaaa ctccatctct actaaaaata caaaaattag ccaggtgtgg tggcaggcgc 15540
   ctgcaacccc agctacttgg gaggctgagg caggagaatt gcttgaaccc gggaggtgga 15600
   ggttgcagtg agccgagatt gtgccattgc actccagcct gggtgatgag agtgagactt 15660
   cgtctcaaaa gaaaaaaaaa gtatccatgc taattttttt caccctttat gaaatatttt 15720
   catcttttaa tctcaaaagc cacacacatg aacattgttt tttaagaaat caaatatatt 15780
   ttctaaatcc cagtggtcag ttctcagccc ccatcttatt gcatctctca gtggcattca 15840
   atgcaggtgg tcacaccctc cttggccaat tcaaagaaga gaaaggagat gtggcgggag 15900
   aagcaggtca caggaagggg ccgggagatg ggatctaaga tgccagcagg agcctcttgc 15960
   ccaggggggt taggtacatt ccagatgtaa tcatttgagt gatttctttg aaatccatct 16020
   ttgcctcgag ggtggaagct ccatgaggcc agggacacag ccatttcaca gcccttagct 16080
   cagggcctgg cacagggaag gatccagaat gcagcactga ccgcacttgc tgtggacact 16140
   gagttcctct tacagcaaac atatggtgac cacccactgt gctgactgct ccccttagga 16200
   aacgtgttcg tgtgttttgt ttcattactt tttaactttt ttccaaaacc aataaatgtt 16260
   cactgcaaaa aaatacaata tatgatgtat tataaagtaa taagaaaaca tgccgacccc 16320
   atcctacctc agtcccattt cccagatgca acacaccaca gcatgccatg tttaacttat 16380
   catccttgca gacctttttc tctagacatg cgattgacca atcaacaaag atttatcgag 16440
   cgtctacact gtgccaacca cagtagcagg agctggggaa acagtgaaca aaacagacta 16500
   aatccctgga ggagaggttc atttaaacag tacacatacg gtaacataca tcattacatg 16560
   tacaggtttt ttaaaaaaca aaatgagata ctataaatat tcttttgtgt tcatccaatt 16620
   tagagaagct gaaacaatgc ccaaagtagg aggggaaaaa aagagaaaaa cctcatgagg 16680
   tttagattca tgatccaaag agagttacat tttatagctt tgtttaatac cctccagttg 16740
   tttatatata gttttatttt acaaagtcat gatcatacag agggtataga tttttctcca 16800
   tctttttttt tcttacaaaa gaaatgcaaa cagaaaacat aaacaagagc aaaatcccat 16860
   gtaaatctac cactcaatca tggctaatct ttaatatcat tgtttttttc taaaattggg 16920
   agtttgggat cacactggat ctactacttg gtaacagact ctgatcttgt aacacatttc 16980
   cattgtaatt ctataccatc attttcatgg ctatagactt ggcctatagc tgtgaaagta 17040
   gattcaccat aatgggatca gccaatctcc tttccttgga tattaggata ttttcaattt 17100
   tttattttca tacagacact taagtgaatg aagcaaaatg tctgcacata ttcttagtta 17160
   cctaaaggta tgatgttaga ttgtttttct tcactcatat ttttactaca tcagaatgtt 17220
   gattacagtt atctttgatg gtatgatgtg agattattac tttgtttcca tacttatttt 17280
   ctctaaatgc cttgcaatga acaggtttta tttgcataaa cacaatatta tttaaaatga 17340
   tgccatgaaa atttctatat gaccaccgtt ttcatggtaa tggcttgtta cggctgcctg 17400
   taattccaca taggggatag gccgtaattc acttaaccat cccctatcat tgaccggctg 17460
   ggctttttct aatgtcttta agatcgttat acaggaaacc tactccatat ctggggttac 17520
   gtcctcaggg agcagcccag caaaggctgg tgaacagatt tcacaccttt ggttcatcct 17580
   gccaagctgc tttcccgaaa agatgttcca atttcccctc ccaccggcag tgcgtgggaa 17640
   tgcctgccca gccgccctcc tctccctgag acggttcagg cccctggaca gtcgcatcct 17700
   ctgctaggag ggcaagagaa agcaggaaat aatttggcca gcaagtgacc ccagacccag 17760
   agccaggagt gatttttaac aagcctgatt ctcagaacag aacctgcaga gaaaaagaaa 17820
   gcaagcaagg aaaaaaaaaa aaaaacccct ctctactgga aaaaacatac acatacacag 17880
   attaaaataa tatttggcac ttacgtcgca cttttaaaat tcttcaaacg ctttataaac 17940
   actagctaat taattaagcc gcctaaatct ggctgcagtc ggtggagaga gctcgagagg 18000
   agctaaaaaa aaaatggggg agccagcgct acacaactcc agattttatt gtcgcctgcc 18060
   tggggtgtga ttacaggcct ccggctgcac cacaagaata gccctgctcc aacatgctgc 18120
   acgcctgact ccgcagcctc tcctgcgcct cctgctccct caccaactgc tgactcacag 18180
   ccccttgtgg tgttattgat aaggataata ataactgcaa gccaggctga gtcccgggct 18240
   ccgggcctgc accctcccct ggatatgcca gtatcaactc cacctaacac atgaagagag 18300
   aggagatttg cccagggtca tggagccaac gacatccttc cttccttcat tcattaccgc 18360
   attcattctg catattttta tcgatgacac tgtgttttag gcaggaggtt ggaaacagtc 18420
   ccttgtggat cctacatcct actattggga atgtgacaat ccagagaaaa tcaaatatgt 18480
   gaataataca gctatgtgat aataatggga ataagaatta aaacttaccc agcacctatg 18540
   cagcaggcat accaccgtgt cagggtcctc atgactgtgc tggggtggat gccggaaccc 18600
   cacttcatgg atgatggaat gggggtccag ggaaggtggg tacagaggtg gctaagaggg 18660
   gagccatgct gtcacccagg cctgctggcc ctagcaactg tgcttccccg aggcagcctt 18720
   tccagttggt ccagtctcga ggctgaaata cagacctatc cggaaagttc ctagctcagt 18780
   gctgctggtg ctgcctctga tgtccccgcc tggatgggcc tcccgggcct cccacacatt 18840
   tggccaccag cctcacccct ctcaggccgt ctgatgctgg ctgggcctct gtccactgtg 18900
   ggttctgtct gtccactgtg ggttctgttt ttttctcctg tgccatgaag atcttgccat 18960
   gttcaacttc aaatggaaat gaaatgagaa gctggacttc ctgataataa aagcagttga 19020
   ggtcttaagt ggttgatcag gaaaaggaag tcgaaatggt aatatttaca cccaaagcag 19080
   gtggagtggt cttaggcact ctgagactcc actcacctgc catgagaacc gggccccaag 19140
   ggtgatccct tgccagagtc aagactgggt gaggctggga atgggagaaa gaggcaggac 19200
   aatgggcaga tagccctggg cttcctgcag tgagggcaga taggggtctg tgagcctctt 19260
   tccctggctg ccctgcccct gccagtgggg tggtgccagg ccatgggttg gtgggcaaat 19320
   tcacagggtc tctctcctcc ccagccagag ccagaggggc acagcagctc tggacatctg 19380
   ccgcagcctt gtctgatgta caaggcaaag gtggcccagg tcagggcagg acaaggtcac 19440
   atgaagggtc agtggagagc ccaactgggg ccccaggtca cctgtcacct gcataccagg 19500
   tagctgccac tcctctgtct acctggttct ctccttctcc cctattctag ctcgctctct 19560
   cactctgcgt ctgtctctca ggcatgtgtt tctctgaggt ctttatgatc atgatctttc 19620
   ttttttttgt ttcttgtacc tcaccttgtt cagaatcgag gccctggctc tgccttgctc 19680
   tgaactgttc tcacttggtc cctatccatc ttcaacgcat ctcatgacaa gctcgtcctg 19740
   ccctcttcct gtctgggctc cccccgtgca ccccaatctc tccacccagg gtccctgtct 19800
   tgtgtgcccc agtgtctctg attggggctc aggctcctgc agccaacctc tacaattcct 19860
   tccagctgcc catttgggag cccttgaggc ctggactggg tccagctcca gtgtgtcccc 19920
   atcacagtga ggcctggatc acagggcatc agcaggtgtt ggtgagagtg aagtttgagc 19980
   cacagccacc tccttgttta ttcaatgtat agtaacaaca acttcctcag ggcccatctc 20040
   agggctaagt gacttctatg tgtgatctcg ttttcttttc agaacaaagc agaggcctgg 20100
   gggctgttct acccatcctg tagataagaa aactgagact caaaactgat ggataaatta 20160
   aggtcccaca agaacaaggg gcgagccagg tttcaaacca ggtccgatga ctctaaagac 20220
   cagatgctgc atttggacgc tgtccccagc cttggcttat tttatcctcc cagagatcca 20280
   tcgaggtggg gtcatagtaa ctattttaca gatgaagaaa ccaaggctga gagcagtaag 20340
   ggcaaactaa gggcagctct tgcatttgtg tgacactttg ctagccaggc tcagtcctgg 20400
   gttccaggtg gggactcaaa cacactggca cgagtctaca ttggatattc ttggttacct 20460
   gagtggtggc ccaaggacac agctataggc cactgatcca gaactcaaac tacattctcc 20520
   tactgcccaa accgctgagt acagcctccc ttctcggggc ctgcatcctg ggccaggcca 20580
   gaaacagaat cttaacacct tgtacagccc cagatatgcc aggcccctac ctctgagtct 20640
   ttgcccaggc tgttccctct gtctggaata ccaggctccc ttctggctca ttcagattag 20700
   aattagccca gctcagatgt gactcttcca gagagggtcc aggcattcaa acccactctc 20760
   tgccatgtca tttagctttc tgtgcctcag tgtccccact tagaatacag gctcatgttg 20820
   caggtttgtt gtgagggtca aatgagatga tacatgcact atactgatct cacagcctgc 20880
   ttctggtgtg catccagtca atgaacgtca ttgataaaca cttgaggaaa cacagaggat 20940
   cacgtaagga tgaaaaaatg actgctttgt gctcctttcc aggagtggcg ggtgaggagg 21000
   agctgcaggt gattcagcct gacaagtccg tgttggttgc agctggagag acagccactc 21060
   tgcgctgcac tgcgacctct ctgatccctg tggggcccat ccagtggttc agaggagctg 21120
   gaccaggccg ggaattaatc tacaatcaaa aagaaggcca cttcccccgg gtaacaactg 21180
   tttcagacct cacaaagaga aacaacatgg acttttccat ccgcatcggt aacatcaccc 21240
   cagcagatgc cggcacctac tactgtgtga agttccggaa agggagcccc gatgacgtgg 21300
   agtttaagtc tggagcaggc actgagctgt ctgtgcgcgg tgagtacagc gtgggcctcc 21360
   tttgcctctg gtttgtgaca gtaactcaat aataacaccc tccattcttt gagcaatgat 21420
   caggtgtggt ggtaggtgct cctttccatg aattgatgtc tccccctttt acaaataagg 21480
   gaagttgagg cctgaagacg tcatgctatt tgctgaaggc tccacagctg gtaaagggtg 21540
   ggaacatctg catccttcat ctgcctggct gtgtagccct ggcctcttca aagctgaccc 21600
   agccctttca ttccacagat gaggaaactg acagatagtg tgacttgccc aggcacagga 21660
   ccagacctca cctgctgcct ccagagagca cttccctctc agggcagccg agtctcctct 21720
   atgtgacagg cactcactga gaacctactg tgtgccaggc tccgccaagc ttgtgttctc 21780
   atcccttcca ataagagatc cacccagggc agggagcaga gagggtgtgt ttcctttatt 21840
   tcatcaccaa acaccaactg agaagattcc taaaaggcac agatttactg tttaatctga 21900
   tggccctggt gagaggagaa gccagagggt tgggcatttt ctgtgggtct tttagaaaaa 21960
   cacttgtgtg atttagggac tgattcatgc gtcactaaag gctctgaaca aagctgggtc 22020
   atcatagaat gtctctcacc ccagggagga ggtctccctt ctaggtgact ttgggttcct 22080
   ccgttaaggc agcaaagatg ctcattttgt atttactgcc agagccccag gatccttgtt 22140
   ctaactccga atcctgggac attagtacag gaaggaccct cagaccatcc agcccaaccc 22200
   ctagttacag gtggaaactt gaagtcagga gatgacaggg gccttgtcca cagtcatagg 22260
   cccagttagt gacaggatct ggaccagagc ccacatctga ttccagggag gctccttcat 22320
   tagccattgt ttacatagaa gagcctggtg catcataggt gccgactgaa agctccttga 22380
   acagaactgc taagcaggaa gccagcactt ctcctttcca tcagtttcct aaaggttcag 22440
   aatattcaca ttcagaccgt taggcccact ggtgattgga aaaccgctcc caaatgtaag 22500
   ccatatcatt tgatactgag atttcatatc aggatagaga cctgctccat tgtgtaatag 22560
   ctacagaata ctccagctta tggaaatacc agatttattt taccatcttc cattgatgga 22620
   tacgtaactt gtccccaaac ttgaactatt aaaacagtcc tgcagtggat gaccttatac 22680
   atgtgtgacc ttactcaggt gggagacgcc cactgcaagt gccattgcta gatcagaggg 22740
   ggtgtgtgtt ttaatgtgtg tggatatggc ctaatttccc tcctagacat tataacaatc 22800
   aagtctcccc acaaccgctg gacataggca gggagggtgg ctttatcctg attgtgcaga 22860
   tgttacagga aaagacgttg aagttcccag atgatgcatc tcgtgcctag acaatcacag 22920
   caaatacgtg gctcaggtgt gacccagatc tgctgactcc tagcagagtg ctcactcctc 22980
   tttgccatgc tgtcagccta aatgatgccc tggatgagaa aggagggagg tgggaggggg 23040
   ccagggagca gcagctggat tggatcctca tcctggcact ttctcctttt ataacaagta 23100
   cttatgaagg aagagccatt tcttgccccc tttacagata aggactctga ggctcagaga 23160
   ggagacctgt gtggtgagcc agtgggggag ccaaggtttg atccccacca gcctgactca 23220
   cagcccctgt ctctgtaaca ctcagctatg cttgtcaaag tgtggagaaa tgctccaccc 23280
   tactcattcc aaactcttgc agttctatag caataagcat aggctgaggc aggagaatgc 23340
   ttgaacccag gagacggaga ttacagtgag ccgagatcgc accactgtac tccagcctgg 23400
   gtgatagagc aagattctgt cttgaaagaa aaaacaaaac aagtcctatg ggagtaggag 23460
   ggactggccc ccatcagaaa ggtcctgcag atgtctcatg atatgggaac ttgttcctaa 23520
   aggacaggtc tcagcacatc actgagtagc atccatggag cagagttggg cctcctgcag 23580
   cccagcagac cacgtgccca cctactcacg gtgctgagga ctaaggatga ggagatgagg 23640
   catttcctgg tgggtgcttg gggctcattg gagcaagagt ttatattatc ccctcaggct 23700
   gtcaggagcc cactgacttc agtttatggc tatagggtaa ccagatctgg gtaccctgga 23760
   gggaggcttc tccctagaac tgcatgaagt cagggatctc aaagggaaaa agggaaggtg 23820
   gtggtcagtg tcagggttca tgaagttcag gcacttgctc aattgattga catgagagct 23880
   tggggaacac tggaaatgcc cattgggtac atgagggatg aggagacagt gccttcgctt 23940
   ttctcagcct cagtttcctc atctgtagaa tggtgataat cagggactcc tttggcagag 24000
   tggaaaggat ttgagttcct gtgtataaaa cacccaatgc agagtagaca cccccaaatc 24060
   aaggtttttt gtttgtttgt ttgttttttc tttttttttg agatggagtt tcactcttgt 24120
   tgcccaggct ggagtgcaat ggcaccatct cggctcactg caacctccac ctcctgggtt 24180
   caagcgattc tgcttcaacc tcccaagtag ctgggattac aggggcaccc accaccacac 24240
   cagctttttt tttttttttt tttttttgta tttttagtag agacatggtt tcatcatgtt 24300
   ggcccggctg gtcccaaact cctgacctca ggtgatcccc ccacctcggc ctcccaaagt 24360
   gctgggatta caggtgtgag ccactgcgcc ctgccatcaa ggtgttattt taagatgtgt 24420
   taccttgggc aacccagttc cccttgctga gcctcagttt ccccatgcgt agaacgtcga 24480
   taattgtacc aacctttcca ggatgttatg aggatgcaat gagcctgggg cgacgataga 24540
   ccttagtaag caatggggtt ttcccaaggt catgtgatct tgggcaaatc ctggcttccc 24600
   ttgagtcctc agcttccctg tctatgaaat gatgatgatg attgccaacc ctcacaggtt 24660
   ctgtgaggat tggagaggat gtacagagcc tcggaatggt ggcggtgttc gggttatcac 24720
   tgagctcctg cagaagtgtg gttaggagag tgtggccaga gtccaactgc ctgggccaca 24780
   tcccagctct gccacctcct ggctctgtga cctgggacaa ggcactccac caccatgggc 24840
   ctcagtgtcc tcacttgtca gtgataacac caacctcaca gaccaggagt tgggaccgaa 24900
   tgagttaaat cctgtagcgt gcttagggca gcaggcgcct ggcctgcagt caatgttcaa 24960
   cagatgtgag ctgtgtgatg ctgaagccat gcaacctcag atgggttagt gctcctccag 25020
   gcctcaagga taaatggtcc ttaggatgta ctcaggtagt tttcaatctt ggctgctctt 25080
   taggatcatc tgggaaacat tgaaataaat cccaggctcc accacagaaa tgcctcttca 25140
   attggcctgg catgggccta ggcatctaaa gtttttaatt aatccagaag attctaatac 25200
   agagtcagga ctgaaaaccc ctgggataga cacccagagg tcacggagga caagttggaa 25260
   aagatggtcc tcttggggtg ccagatggga gaacagcttt tcagggtgtg gatgcttctg 25320
   aacaacgtgc acccgggaca gctctttgga ggccccagtg gcctggccag accccctctt 25380
   atgatacagg cctcgggggc caaggtaggg gccccagaca aagccctgct cagatccagc 25440
   ctcagtgtgg ggaaaccttc catcctgaaa atgtttttgt ctaaatgacc gccggaatcc 25500
   cagcaaatcc ttccaattcg gccccagaac cctccctgcc tcgacacacc aagatgtcac 25560
   ggaggagtct gttccctcct cctttaaaat gcttccagct tctgtaaatg gctcttctgg 25620
   agggaggcgc ctgtgatctt gtgttttgaa agccaattcg ccaaacttgt ttcctgaggg 25680
   gattgtttag aaaaggggag agaatcctca cttggtggtt tctgctcctt gtatcatttg 25740
   ggtttgtcat gagccacagg gagcagggtt tgagcctttt cttggtgttt ctgctctgct 25800
   ttgttctgct gcctgaagga taaatgtgta gattcggaag cagtaatgac cagacctcct 25860
   gataacagtc acaggggtca tttattaagc acctgctgca taccaggctc tgtgcttgca 25920
   ttcatcctcc ctcttcttaa cagggtgggg tttgtatcca cttttggccc tgggcctctc 25980
   tgagctttgt cctaaaaggg gctcacacga ggatcccctc acagagatgg agtgaggatt 26040
   aaagagctga cgggcatgga gggagcagcc agtgtccagc tccagagaag ccctggataa 26100
   atgggaactg attgcagccc ctcagccctg tggggacatc caggccgctg gcctcaatga 26160
   cttgacttct tcaagtgaca ccctccttac tgtgccctca aaatgtttga tgggtgtgag 26220
   ggagaagtta atatttacaa atgcaagtat gtgtgatatg tgcttcctgg tgactgcgat 26280
   aggaggtatg gggtaatgat tagcaatttg gagacacgtt tacgacccat ctgcagggag 26340
   aaacagcccg cgttctgatt cagccatgtg tgtgctttca ggacccagga agtgcttctg 26400
   cctgcttggg cgccctgtgt ctgtgtaatt aattgataat taaccacttt ggctttctcc 26460
   acacttggtg atctttagtg tctatctaaa actgacacca aagaagaccg cagattcaga 26520
   aaagtatccc ttcctttcaa atttagcact tggcacagat tagaccctgc tggaatattt 26580
   gtggagaaaa tgaaaaatat gaagtaaatt cataaatacc tgaagagaga gctttggata 26640
   tgaataaagt ctgggttaaa gttagatctg tttttgctta tttttgtgaa gcatcttctg 26700
   tgtttatcca aagcaaaact gcttctgttg cctcatttaa tcctcgctgc atcccagaaa 26760
   ggagaaatgc tgctgttcca ttttacagac aaggagctgg gtgtgccagg ccgcccctct 26820
   catgggctga gcggcctggt tccagctctc atggtgatgc cacgttgctg cttactccct 26880
   gcactgacct ctataaacgc tctctctgag gttggcactg ttgccagggg aggcagggta 26940
   gaaacagtta ataccaagga tgagatggat cgttaatttt gatcctgtgc ccaaattgct 27000
   ggaagtcacc ctgtgtggag tggagaagtt tgaactcagg cctggacttc cctctgggcc 27060
   ctgcccctac actgtctgac ctcacatggg tcctcacctc tcccaacctt tgcatcctgc 27120
   tctgtaaaat gacaataagg acaccgccct cattaatcct tccacattat tggaaggata 27180
   aaaaataatg tctcagaagg ttcttaacgt gtcacacact tatcgttagt gattgtcatc 27240
   tgagtcatgt ctcctgatta tcgatggtcc ttttgtagcc aaaccctctg cccccgtggt 27300
   atcgggccct gcggcgaggg ccacacctca gcacacagtg agcttcacct gcgagtccca 27360
   cggcttctca cccagagaca tcaccctgaa atggttcaaa aatgggaatg agctctcaga 27420
   cttccagacc aacgtggacc ccgtaggaga gagcgtgtcc tacagcatcc acagcacagc 27480
   caaggtggtg ctgacccgcg aggacgttca ctctcaagtc atctgcgagg tggcccacgt 27540
   caccttgcag ggggaccctc ttcgtgggac tgccaacttg tctgagacca tccgaggtag 27600
   aagaccctca cccagcccaa gcccacacct gaccgccaag cccacccccc accaccctcc 27660
   actcatccag ctctactctt gctccagggc ttgaaatgcc tggaacctaa attcctaact 27720
   gcccacctcc catgccccta gatgtgccag ccacttacta acattttaat ggcagtggct 27780
   gggtgaccac accacccacc aggtgccaag gactgtgcta gccgtttcat ttatttcact 27840
   gatagcaaac acagttaatg agtacctact atgaactaag cctctatgtg cttggtgatt 27900
   ttctttattt tgctacagtt acaacattcc aactgcatgc caggggtggt gcttatagat 27960
   ttccctcatt cttagcctag taggagctac cagtccttga gcacctactg tgtgctgggc 28020
   gttgtgctta gtaatttcat tcatatgcaa agagcaccct caatgtctga gcacctgatg 28080
   cctgcctagc tctgtcctgg tgatgacctc accgtggtgg gggagctacg ttatggagcc 28140
   ctccctgtga tctgtctgtg ccacaaggtc agagcttctg ccctgtgctg tttcagttcc 28200
   acccaccttg gaggttactc aacagcccgt gagggcagag aaccaggtga atgtcacctg 28260
   ccaggtgagg aagttctacc cccagagact acagctgacc tggttggaga atggaaacgt 28320
   gtcccggaca gaaacggcct caaccgttac agagaacaag gatggtacct acaactggat 28380
   gagctggctc ctggtgaatg tatctgccca cagggatgat gtgaagctca cctgccaggt 28440
   ggagcatgac gggcagccag cggtcagcaa aagccatgac ctgaaggtct cagcccaccc 28500
   gaaggagcag ggctcaaata ccgccgctgg tgaggcctct atttcagctg acccagcttt 28560
   tttaaacttt tagttttgtg ggttttttaa atgttaaaag tagtaattca tgcttattat 28620
   agaacaatct agaagtctat aaatataaag tagaatttta atgtcagctc catttcccca 28680
   aatcccacac tccaagagtc accactggta agagtttgat gcacatcttg atagatcttt 28740
   tgacatagat ttatatcaca ggaggaaaga gggagagagg gggcccatct gttcatggtg 28800
   tcctgcctct tgccttcttc acctgacagt tgtcatgctc cccattcctc actggcccag 28860
   cccctcttcc ctactctttc tagcccctgc ctcatctccc tggctgccat tgggagcctg 28920
   ccccactgga agccagggtg gagtcattca gggtgtttgc agcttgtctc acatacagtc 28980
   agtgcttcag tgaacatcct tgtgcatgtt ccatcccaca tgtgactatt tctgtgtgat 29040
   tgcccaagga acactttagg ccaaaatgca tactcatgta aggaggagat aatgctgtcg 29100
   ctcccctgag agagtgtgag ggtgtcaagg tatcattttc tcagcccttg caacgtggtg 29160
   ttggtgtaca tctcttttgt tttcaaatgg tggcacctga ttggcatttc agtgcagcca 29220
   tggaatattt catcacctgt tatcaatgtg gctgatgccc tgtacatagt agaacccttg 29280
   aatggattag aatggaagcc atttctgtcc ttcatgcacc cttcagggaa gctctcaaag 29340
   acagccttgg ggctgtgtca aagtggccct ttggtttcac caatgccagg ttttcttagg 29400
   atgtgtaggg catcggccaa tgctgggttt tcatagtacg tgtggagcat tggccaatgc 29460
   cgggttttcc taggacgtgt agggcatcgg ccaatgccag gttttcatgg gatgtgtagg 29520
   gcattgggca atgctaggtt ttcatgggat gtgtagggca ttgggcaatg ccaggttttc 29580
   atgggatgtg tagggcattg ggagacagga tgtgtggctt tccacgccag ctatgttgca 29640
   tacttggctg tgtgaccttg gacaagtcag tgcctctctc tgggcctcca cttgcttctg 29700
   gctgtgttga aggggatgag cttcatgacc cctcagctct gagtctcagc tggtaggggc 29760
   catgattatc ttgctcagca ttgtagcttc agggcctgac cacagtaagc actcagtaaa 29820
   tgctttttga gtgaaagagg ataaaagagt cagttggttg gctggttggt tggttggttg 29880
   gttggttggt tggttggttg gttggttaca taatccttag ttagccaatg aggtagagat 29940
   gactgttggc cccagtaaca gaagaagcag ccagggtttg gagggaggct taactgactt 30000
   gtccagggtc acacagctac ttagtagcat agatttgaaa gcacttttct gtggcttaga 30060
   gccctgcttc tgatgaacca ctgtgttcta gtctatgacc cccttacctg tgctgtgggc 30120
   agtcaagcct ccattgtatt ttcaagagtc ctggttcaga gcccctatgc taactcctga 30180
   gcccagtgtg catggcccag gtgctgggca cagaccggtc gtcagcccct gagctgtgca 30240
   gagcctgctg gccgcactgc ccttcttcca gctgctgatt cagtttccag tggatctggg 30300
   gtgccctcat gaccgctctg tggttcactc cagatgagaa ggaacgtttc tgttcctcag 30360
   gagtgagatt tctgcctgta tcctgcagca ggggttcctc cgtagctgtc agctacaaat 30420
   atattccccc ctggcacctc agcatggttt ttgtgctgtt catggatgag tctcgtgggc 30480
   aagtgtgtac agacccatga gtgtgcccat gtggctcgag acatctaaga aggtccagcc 30540
   agatgttctc agttaatgat gcctgcttag tggtgaaaag cagtggtggg tttggttttc 30600
   tgtttttaat ctgcatacgt gaagcctcta ttccatgtgg tccctagaga acactggatc 30660
   taatgaacgg aacatctata ttgtggtggg tgtggtgtgc accttgctgg tggccctact 30720
   gatggcggcc ctctacctcg tccgaatcag acagaagaaa ggtgggtgca ttcccctctt 30780
   cctccctaag ggtttgtccc tggactgtcc tcggagggag acgccattag gtgctttggg 30840
   ttaaggacat cagcttctgc cagtagcaag aagtccagag gtagtggtgc tagggctggg 30900
   gcagtggcct caccatgtta gagtctgggt gggtcactct gtggcttccc cctcatggtt 30960
   acaagatgtt catcactgcc ccaggccccg tgtcctcaag gggccactac tgagcaaggg 31020
   agctgccagc ctgggaagag aggaggcagg gactcttctt acctgtctca gggaggacat 31080
   cgtctttccc aggagcctcc cggcagactt ctgctttcct ggtacaagtc taggttacat 31140
   gaccagtccc tggacaggag gccagaggga atggagataa tagcgccacc atttgattgg 31200
   gaggacttaa caagtgattg catgggaaat gttagggaag taacctggca cacaagtgct 31260
   caatgtatgt cagccctgat tattacccgt attgtatgtg agcccagaaa tatgagccac 31320
   ctccctagaa agccccctca cagggcaagg tgcatagcct ttgactgcac agcaggaggg 31380
   cacagggtca aacccaggac accgcttccc cttgtttgtg tgcacgtgtt acgtgtgtgt 31440
   ctgtgtgtgt gtgctggttg ttgggagggt tggttgttgc tgtggcaggg cctctgactt 31500
   tgaagtcaga caaaccaggg ttccagaccc tggttatata ctgtaaacaa tcaatttggc 31560
   ctctacaagc ctcagtttcc ccatctgtaa aatgggttta gcaacgcctg tcttggagaa 31620
   gttacaagat ccaaataagc ccatgtgaaa taacgggtct gacacattct aggatcgcca 31680
   taaatacatg ttctctttcc cactcttgga gaagacaatt ttaagctggt atttctgcaa 31740
   ccttttttta ttacaagaga tagaaataca tttcacatct caacccaaca catacacaca 31800
   ccctttgtat ataaaattaa aactgatatt ttcttttttg tcatattctt ttttctgttt 31860
   aaaatgctgg ttgtgaccca ccgcctccaa agggacaagt gctgtcccga gctgcctttc 31920
   tgtggcctgg cacagctgag cttggttggg cacattgtac actaatcacg catcccttca 31980
   gtagctcctt cacctctgcc tcccaagttt ccctccctcc tgggctggct ctgagatact 32040
   ggccaagcct gatgcttctg tctgggaggg ttggagtcag gccctgcttc tccagaatct 32100
   gcaagacgtc ccaccaagac gtctacccaa tgggttccct aaagttcctg gagagcccca 32160
   cacagggagg actcctcctt ggatggggaa gcaggtccct tccccaccaa gggtgacagc 32220
   ccatcccatg ggatgaagca ttcatagcag ccgatagtga aatcctggcc atcctttctg 32280
   agtctcaggc ccaggcagca cgccagctcc tggcacacgt gagctcatta ctgtgggctt 32340
   aggtgttgca gaggaatgga gtttgcaggg ctgcagctct ggtaaaggcc acccagcccg 32400
   agtggcaggt ctgtgtgcag agcctagctc tcaaacacag gctccagtga cagggacggc 32460
   tagtatcccc cagcagttcc aatgtgccag gcactgctca ctgtgctttc cctgtagtaa 32520
   cagtcagtgc tcaccacaga cctcatgggt gacattacta accccacttt acagatgagg 32580
   aaactgaggc atgggaaagt tataaaactt gttcagggcc tcacagccag gaggtggtgg 32640
   ggccagaatt taaacccaag gaccatgtct aggaccacct tgcacactac ctccctccgc 32700
   cataactaga ccccccttct ccctcattag gccctggagg ggtctgccca gcctctcaat 32760
   gctcacagtg aaatgtcctg atttcaactc aaccactacc cagctgtgtg accctgagga 32820
   gtcacctagt ctctctggtg taaaaggggg tgactggtgt cctatcactg gattcccaga 32880
   gatgacttgt gtccagcact ttgcaacctg tcagcctcca ctcagacttt cggtgctgag 32940
   cacagcttcc aactggtaca cctcatctaa agatccagga cctgcccagg cagcaagcgt 33000
   taacccagca gcaagaattc ttgcactgtg gcagcctcca gcctattaaa gtgcggtgca 33060
   gagtgcattt gggtgtgcat gttgcttttt aattacaatt ttacagcaaa atgggagtca 33120
   aacggctgct gaaccctgga ggcttctctg tgggttaccc catatcacag gtttaaaacc 33180
   tctgaaccag atggacactt tctttccttc caagatcctt tttttggaaa gtggtggcat 33240
   gtaaataaat ggatgctcag cccaaccctg caagttatag aagctgagag agaggaagtg 33300
   acccacccgg ggtcacatgg tgagtgagtg ggtagcagac ccggggttca cacatgatcc 33360
   ccgatttgca gccctgctag aatctgtttt caagccacga agggcactga ttgggaccta 33420
   ggcttgttgg agggtctccg ctgaggtttg tggttgaatg gaggtgagac ccagacagag 33480
   gtagggcggt tgtcgcctcc caggctgaag gctgcttttg gacccctggc aacttccaga 33540
   agtggaaaag caggaccatc tagcttactc cttcccaggc ttccttggtg gaagaggatg 33600
   cccactgggt gggcatgggg gtctcctgtg gttccaagga tgtgattaca gcatttcctc 33660
   tccatgtccc tggaggcaaa ccttttgcca aaaaatagtt acataagaaa agtgtgcttc 33720
   tagttaaaca actggctttt gtttcttttg tctttcagcc cagggctcca cttcttctac 33780
   aaggtaagtg catcattggt ccagaccctc ttgcagttat tatttggtta tttgacagcc 33840
   ccccagacta caaagcataa tccatgtcca ctgacctcac caatgtgttg gtcaacatgt 33900
   ctctttctct cctttgtaac ctcctcacac tgggtcacgc tgtttttaat tattgtccca 33960
   aatgaggggt ttttttggtg cacagaggtg ctaatttctt gcctgctctt cccagacaac 34020
   gagacccttg tctaatccca tgtggactgg ggcagagacc tcccgggctt tctgtcctca 34080
   cagacatcct gcacacgcca ccggatgcat cctcccagat ccaagttgta tactttactc 34140
   tttcctttgt ttagtaaata tttattgagt accttggcgt gctgtgaccc tggaatgccg 34200
   ggatgagtag gatagaatct gtgccctctg caagctcaca gcctggtcag aggctcagac 34260
   ggtgacatta catcactgat gtacgtcacc gatggcactt tagtttgttg attcacattt 34320
   ttagtgactt gagtccctac tatgtgccag ccctgctcag gaccagctgg tgatatagca 34380
   gtaaacaaga atcagcccag gctttcatgg ggctcagacg ccggtgagag aaacaggcat 34440
   aaacgcagaa ataaagtgat tcatggtgtc atatcaggtg gtgacaactc ctaccctgca 34500
   aggagatgga ggtgaatggt ggggggctgt cttaggcaga ggaagggact gcctctgaaa 34560
   aggggatgca aacgggtgcc tgaaactagt gagctatgtt ggacaggagc atgctgggcc 34620
   agagaggagg cctgtgtgct gggccagagg gaaggagagg aagaagataa ggtcaggaag 34680
   acccaggtcc tcggcatccc ggtgtccttt gcagatgccc ctgcagtcag aggtgttacg 34740
   aactattctg tgaaaagcct ttatcggctt tttggaaggc ctctgatctt catccccagc 34800
   agagcagagg cagcacacca tcatctgcca tgtgaaccca tctgcataga accatgtgga 34860
   catccgtgct aagagtgata aataaaacta taaatagctt catgttggtt cacaccaggt 34920
   tttgccagca actgcatagt gccaatcttt tgatggttta gagctttctg ggtttcagaa 34980
   ttacagaata ggacatgatg atctggagtg gggcctgtgg gtgttagaag gactcgggat 35040
   tttatccgca gtgggatgag gaaccaaaag atggacacga tctgactttt gttttaaaag 35100
   tgtcgcctgg ctacatgtgg agcccagact acaggaggtg aggatgtgta ggtgaccata 35160
   gcggtactgg tccaagcaga aaaagatggt gacctggacc aaagtggtgg gatgggctga 35220
   gaagtgctca gattctggat atattttgaa ggtagacaca agaggacttg ctgatagatt 35280
   ggctgtaggg ttaaggacca cctcaaggtt gggggcagtg gagtgctgta tcctgagttg 35340
   gggggtgcag gtgatgggtg gagtgatggg gcaaatatca ggcaaagcag ttgcatatag 35400
   caggagcagc tcttctgcca gagaaggctc tgggaagcat caggtgcccc gctcctgccc 35460
   agcagtgtga gtccaaggta ctcttgggat aaagccttgc ttcttgctct gatctccaca 35520
   gggggcctcc tggtctgagc cctgcccagc ctcctggcct tttcctcccc tgaacaagca 35580
   gagccgcctc tccagcaaaa ctaggctctt cactgccttc tgggcacatg tgttttcctt 35640
   ttgtcatgct tgtgcctgtc acactcccac acctaggtca ctcttccaag ggcaagactc 35700
   gtttatatcc tttgccaaaa aaccttcagt ggctctacct gtccctccag ataaagtcca 35760
   gatgcccccc tgaggctcct gggccccttg tggtccagtt gccttgaaca gtttcacttc 35820
   tagccacccc ctgtcctcac atttcacggc tcaaccacac tgtgctcccc ccttccctcc 35880
   tcccacagtc tgccctcctc ccccttgcac actgccctca ctgctggggt gtgctccctg 35940
   cccctgctca tgcactaccc agggtccccc agctccaggg gtggcaggca cagtgggctc 36000
   tgtgaatggc accccctgga gttatgcaat gtggtagcta gagtgggacc cctcctgtca 36060
   cctgctcacc tcctgcttct cctgagatca gcaagggcac gacatcctcc agggtcggag 36120
   ggcttcctgt gaaccctcct ggtgccgcag cgtggacacc ctggtgtgtt caaggtgtct 36180
   ctgcacctgt cccatttcat aacccacccc tcaaggccag ggccaatgtc cctgaggttc 36240
   actgcaaata gctgctaaac ttggcaccat gcctggaatg ggataggtgc ttagtaaagg 36300
   tttactgact gaacaagtga acaaacgaat gaatgaatgg atcagtgcat accatcctaa 36360
   accactgtag cacgtcccac tctgccttct ctgaatccct aaaatgctgc ttcccagtgg 36420
   ctacacagcc ccatctagga ggatctctct tttttgtttt ttttgttgtt atttttttat 36480
   tttttatttt tgagacggag ttttgctctt gttgcccatg ctggagtgca gtggtgcgat 36540
   cttggctcac cacaacctcc gcttcctagg ttcagatgat tctcctgcct cagcctcctg 36600
   agtagctggg attacaggca tgcgccacca cccccggcta attttgtatt tttagtagag 36660
   atggggtttc tccttgttgg tcaggctggt cttgaactcc cgacctcagg taatccacct 36720
   gcctcggcct cccaaagtgc tggaattaca ggcgtaagcc actgcgcccg gccctgttgt 36780
   cattattttt tacttgtctt acctccccga ttagacaatg agtttctgga agaataggac 36840
   attgtcgata atagtaacag caatagcagc taccatttat tgagcactta ctgtgctcct 36900
   ggcactatct atgcctttat gtgtattaac ccacttgtga ttttaattct tgcaggaacc 36960
   ctatgattta tcactgtccc aagttcatag aacagtgttt ttcaaacttg agtgtgctac 37020
   agaatcccca aaggacttgt taaaacacag attactgggc cccaccccca aagtccctga 37080
   ttgagaatct ctatttccac aagttctcag gtggtgctga tgctgttgtt ccccagccac 37140
   actttgggaa cccctgttct agaaggttct tattccctat cctatggttg aggaaattga 37200
   ggctcagaga agtcgtgaca ttcccgaggt cctgcggcta ggaagcaccg gaatgatgac 37260
   ttcagcctcg acatctcttg tactaaagct agtgccttca tcactgttcc catgcttctt 37320
   gctgtctatt tcttcttccc cagagaaacc aaccgttttc tgcctgtttt ttatttttcc 37380
   agcgtgagcc taccaaggga gccatgagat cacttgctcc ctttcatctc tctaaaccaa 37440
   agcaatagct agtgagtgct aatggtggtt tcccatgtag cagccagctt cggctttggc 37500
   ttgctgggat cagacatttt ccagaggacg ttggtgtgag taacagctga gcacctcagg 37560
   gaggctgcaa gcggggaggc tgccactttg agctcaaggg gttcttcagg ttgcagggct 37620
   ggtggaggga ttggctctct ctaactccct gggttaggaa tgtacagaca gaatattgtc 37680
   tgtaatcttc cccagctcag atgagagaaa aatgtgtctc tgctccctta atggggcact 37740
   gatcattcga tcagccattt attcacttgt cagacattca ctgcatttat tcttggaata 37800
   tttagggctt ccctgagtct ttcatttatt cgtcgggact gagcctaact cagtgccaag 37860
   ccttgagcag agggttgcaa aggggagcag aaaaggccca cccctggtct catggcactt 37920
   aagagccagt gagaaagatt cattcattca ttcattcatt cattcattca gcaaatacat 37980
   atggactgct gacgatgtgc cagcccagtt cttggtgctg gggcttcagc agtgaatgag 38040
   actggcccag ttcctccctg tgtgaagctc agtgtccgct gaggagacag gttaccagac 38100
   aagctggaaa gttaataagg gatgtggtgc agctgctgac tagataaaat gaagccaggt 38160
   atgggataga gcagatcaag gaggtggggt ctggggcgag cagggagggc ttgtctgcaa 38220
   ggtgacattt agaagatcac tgaaggatga ggaggttcca gccaagggaa tatctgggac 38280
   aagactgcac caatcaaagg gaacagccag tgccaagacc ctgagacggg agcagtcaga 38340
   tgcatgcaca gaacgcagag gaggccagag gagctggagc agagcagagg caggagaggg 38400
   atggagccta gagcagttgg ccgagctgag ccttggagct ggtgtcactt gaccaccttg 38460
   gagtggttat agaggggttg agcattctga gcagaaaagt aacatgacat cacttatgca 38520
   tttcagatag ggagaatgga gggcagggaa gcaggaatag aggtggggag atcaaggagg 38580
   aggttcttca ggaatcaggg gagttcggat ggtggagagg atggggtggc tggctttgat 38640
   ggatattttg gaggtggagc cgacgagccc taggttggat tagatgagag ctgtaaagga 38700
   agagagagaa gaatcaggac tgactcccac ttctccagcc tgagcacagg atgaccgatg 38760
   gtagagctat tcactgaaat aggaaaatcc aggggagaca cagatatggg gaaaatgata 38820
   attggttatt gtctgaaatt gctttgaaag agaagaaccc aatgttttga gaatggagtt 38880
   tgggggttca gtgaatattt ccccaagaaa aggctgttta aaaatagcat ctgaaggaag 38940
   aaaaggatta aagcagatta agaggcagca gcatggagca tgcaaaggcc ctggggcagc 39000
   ctgactggca ttcattccaa gtcactgttt ttgagcacat gtggtgagga agcatggcag 39060
   gcaggcaggc agacagcaat agaaaccaga tatgcagata gacaggagat ctgagattgt 39120
   catggagggg cagcccagcc agcgcagcac tgtcagcaga gccatcacaa ggtgacacca 39180
   tagttgagtc ttgaaggaaa caggaaagtt agccaagagg aaaaatgatc aaaggggtcc 39240
   ttcgaggctg ctattgaagc atgaagtaca taacatcaaa tgccaccccc cccccgaaat 39300
   atctggtggg cagatgagtg ggtacatgag ggcagggcag tgaattagcc tggaggcagc 39360
   acgtcagacc acgtgggact ctgagcaggg cgatgagttg gacttgaacc agccagccag 39420
   tgggaagctc caggagggct ggaggccagt agtgtgggga cagatctgcg tgatcttccc 39480
   cagcagacgt gcccctgctc ctggagggtt ctttctgtgg ccacgtgggg cttgtcatgt 39540
   ctcagaactg ctttcagagc caagcactca tgccaaatcc attaataacc ctgctgccaa 39600
   gccccactgc tcatttctga cattatccat gctaacatcc ccggggttgc cggcttgaga 39660
   atttacacaa cttctgccaa gccagtggct ctggtttgct catcaccttg accgtgaccc 39720
   tccagtgagt caagtcatga cctccaatta cagaagcaag tgcacgaaca acttctttga 39780
   ttaacttgga catcttggtc tttttctttt ctttctttaa tgatgataca tagatgttta 39840
   taatacagaa catttggaga atatgggaaa ctaaaaagga gaaaaaaaat accatgcaaa 39900
   gaccactgca gtttgcattt tgctatgttt ccttccagtg ttatccacac atttttcata 39960
   attgagataa tcctacagag aattttcctt tttcacttat cattttaatg taagcaattt 40020
   taacttaaaa aaaaaacctc ctcataaacc ttttaaatct ttatctgtgg atttatccta 40080
   atttccctaa ccagtcccaa ttggcagagc cctaagacat aaacctcctc ccccatccag 40140
   cccttgacaa atgcctttgt gttgaaagct ttttctatat tttgaattcc cccaaaaaaa 40200
   agtgcaatta tgagtcaaag gatgaggaca tcttccaagc tcttgatttt gccagctgct 40260
   tcccaaaagg attgtactgg tttgcctctc ccaccagtga cgtatgaaag ctccagttca 40320
   tttcaccata gcttccccag caccaagtat tataattttt aaagatcctt gccaatagag 40380
   taggttaaaa aaatgatagt gcattgctta gaattccttt ttagtgagat tgaccccttt 40440
   gtccaaacat ttgatatgca gttgtatttc tgttatgagt ccttcgttca tgtcctcaac 40500
   ccatatagaa aatggagcct aaatgttatt cttatcagtt tgcatgagca cttgagatag 40560
   tgagggtatt tttatctgtg tgtctctttc ctttttaggt tgcatgagcc cgagaagaat 40620
   gccagagaaa taacacaggt acagtccttg gtgagatgcc cttcctggga aactccgtgg 40680
   cgtggttgct tcacatcaac cggaattgca gatctggttc taaattaaga ctccttgtgg 40740
   ggtggagggt ggaggatctt acactcctag ctttccccat gtcctgtgca tattccttct 40800
   ctctactgca gccaagagtg gctgttgcag cctcatgact cagtgcgacc cattgcacaa 40860
   ctccccaggg gcaccattca caatccgtcc tatgaaaatg gtgccctgga gtcaggctgc 40920
   agctccctta gtgtggatcg tgccttccca tctctctaca tttgcccagg cccatccctc 40980
   taccaggagc gccactccca gctcactgcc cgctcctcac agccaggctt ttctatgctg 41040
   gaactgcaaa gcccagcctg ccagggcctc tcccaccttt gctgatggaa agaccccatg 41100
   tcattaatta cctcacacaa gggcacactc actcaagctg gaggtttctg gtcagcattg 41160
   ctgagatgat tttgcagcaa cacggtttct gagaaaccta gccctgctta tcaccggctg 41220
   atgctgtcag accttgaagg cagagggctg ctcagcatct tgtaacaaga caggccctgc 41280
   tctgaacccg gaacgagagc gcgccagctg tccttgacat ttccaaggca gttgtcatct 41340
   ttttagatgt aatctgttga aggagtcggg ctggtgaaag gcgcagcatt tgtagaccaa 41400
   aatgccttgg ccctgccctt gtgacccatg gggtcccccc gcagccccgg acatgagctc 41460
   gagttgtaat taagaaaaaa caatttccag tcatgagcga gggagatctt ctcccccatc 41520
   attttacttc tggacttcct cctggaatct tctgggggaa aaaaatgatg gtgagaagaa 41580
   tgggtcatgg aaacctctac gaaattcaca gcagagactt actgggcacc cactgtgtgc 41640
   cacgcactgg acagtgagcc agacagtcac agcccagctg gcagagtgcc tgcgattcca 41700
   gtgccaggtg ccagccctgg ccagggaggg agttgcacgc tgtcaacacg gatttcccag 41760
   acagaagcaa atgagagcac gcagggctca ggacagagga gctgtgtgct aagagcacag 41820
   tcgtagagtc caacacactt tggttctttg ccagccggac aactttgtcc actttattga 41880
   acctcccttg gtctcagtgt cttcagccgt aaagtggaat agataatcct tcccaagcag 41940
   ggttgtggta agagtggatg gggtgatcta tgtgattgtg tgctgcttgt aagccctgag 42000
   aactgaaatc agccattacc gtgcaccaac taggaggtgt gccggttggt ggtagcttta 42060
   ttctgttcta tcacgcaggg gccagtcaca gagggagcaa agtttatcat tcatgatggg 42120
   cgcttttgag gcggcagtgg caggttgata ggattagcac atgggaggta tccaagtttg 42180
   ctaagtggtt aatgattgcg aacatttatt gaagacagat gctcaatagg taccaggctc 42240
   tgttctaaac gctttacctg gataatctca ttccatcctc acagcaaccc tggcagtaga 42300
   cactattatc atcccctctt tacagatgga gaaactgagg cccagagagg ttaagtgaat 42360
   tgctcaaaat ccctgagcta gaaccaggat tcaaacccag gctttctgac tccagaacgt 42420
   ttcccgcttc actaactggc gatctcgttc ttatactgcc cctgttctct gtctccctgt 42480
   ccccagcttt gccctgagtg aaggtgaaat tattgccatc cactcatata ttcattcaat 42540
   atggatcaga cttcttatgt gccagccttg gtttctgaat cataacaaat gcccagtgaa 42600
   tatttgtgga ataaacgcat acattaatga atgtccccac acgtaccacc atcagcagca 42660
   cctcctcagg cccccaaaca gacatggtcc tgggtagcgg gccctgtgat ggaaaacaaa 42720
   catgggctga ggaagcacaa agaaggaaca tggaccctgc caggagaggg gagagccagg 42780
   gagggtctta gtatgagcca tgcttcatct gaaatttaca tggtggattg ggatttgtta 42840
   caaggagaag caggacagac accctcgagg gatgaacatg agaaatgggt gaagactgca 42900
   ggtggttcag gctaggagga ggcaggaacg ggaggaggtg acactgaggc ggggctgtga 42960
   ggagactgca ctgtgcgggt cagaaagacc cttcaggcag ggtgaggagg agggcagatg 43020
   ggaaggggca aggctggagg caagtaacgt tggcaagaga tgaggggaac atgacttatg 43080
   gctgagccag tgtgggccga gaggacacag aagcatccag acttggtatt cagtttgagt 43140
   gagtgggcca ggggctatag aatgaccttc tcatgacttt ctctttgggt atcttaaatc 43200
   caggacacaa atgatatcac atatgcagac ctgaacctgc ccaaggggaa gaagcctgct 43260
   ccccaggctg cggagcccaa caaccacacg gagtatgcca gcattcagac cagcccgcag 43320
   cccgcgtcgg aggacaccct cacctatgct gacctggaca tggtccacct caaccggacc 43380
   cccaagcagc cggcccccaa gcctgagccg tccttctcag agtacgccag cgtccaggtc 43440
   ccgaggaagt gaatgggacc gtggtttgct ctagcaccca tctctacgcg ctttcttgtc 43500
   ccacagggag ccgccgtgat gagcacagcc aacccagttc ccggagggct ggggcggtgc 43560
   aggctctggg acccaggggc cagggtggct cttctctccc cacccctcct tggctctcca 43620
   gcacttcctg ggcagccacg gccccctccc cccacattgc cacatacctg gaggctgacg 43680
   ttgccaaacc agccagggaa ccaacctggg aagtggccag aactgcctgg ggtccaagaa 43740
   ctcttgtgcc tccgtccatc accatgtggg ttttgaagac cctcgactgc ctccccgatg 43800
   ctccgaagcc tgatcttcca gggtggggag gagaaaatcc cacctcccct gacctccacc 43860
   acctccacca ccaccaccac caccaccacc accactacca ccaccaccca actggggcta 43920
   gagtggggaa gatttcccct ttagatcaaa ctgccccttc catggaaaag ctggaaaaaa 43980
   actctggaac ccatatccag gcttggtgag gttgctgcca acagtcctgg cctcccccat 44040
   ccctaggcta aagagccatg agtcctggag gaggagagga cccctcccaa aggactggag 44100
   acaaaaccct ctgcttcctt gggtccctcc aagactccct ggggcccaac tgtgttgctc 44160
   cacccggacc catctctccc ttctagacct gagcttgccc ctccagctag cactaagcaa 44220
   catctcgctg tggacgcctg taaattactg agaaatgtga aacgtgcaat cttgaaactg 44280
   aggtgttaga aaacttgatc tgtggtgttt tgttttgttt tttttcttaa aacaacagca 44340
   acgtgatctt ggctgtctgt catgtgttga agtccatggt tgggtcttgt gaagtctgag 44400
   gtttaacagt ttgttgtcct ggagggattt tcttacagcg aagacttgag ttcctccaag 44460
   tcccagaacc ccaagaatgg gcaagaagga tcaggtcagc cactccctgg agacacagcc 44520
   ttctggctgg gactgacttg gccatgttct cagctgagcc acgcggctgg tagtgcagcc 44580
   ttctgtgacc ccgctgtggt aagtccagcc tgcccagggc tgctgagggc tgcctcttga 44640
   cagtgcagtc ttatcgagac ccaatgcctc agtctgctca tccgtaaagt ggggatagtg 44700
   aagatgacac ccctccccac cacctctcat aagcacttta ggaacacaca gagggtaggg 44760
   atagtggccc tggccgtcta tcctacccct ttagtgaccg cccccatccc ggctttctga 44820
   gctgatcctt gaagaagaaa tcttccattt ctgctctcaa accctactgg gatcaaactg 44880
   gaataaattg aagacagcca gggggatggt gcagctgtga agctcgggct gattccccct     44940
   ctgtcccaga aggttggcca gagggtgtga cccagttacc ctttaacccc cacccttcca     45000
   gtcgggtgtg agggcctgac cgggcccagg gcaagcagat gtcgcaagcc ctatttattc 45060
   agtcttcact ataactctta gagttgagac gctaatgttc atgactcctg gccttgggat 45120
   gcccaaggga tttctggctc aggctgtaaa agtagctgag ccatcctgcc cattcctgga 45180
   ggtcctacag gtgaaactgc aggagctcag catagaccca gctctctggg ggatggtcac 45240
   ctggtgattt caatgatggc atccaggaat tagctgagcc aacagaccat gtggacagct 45300
   ttggccagag ctcccgtgtg gcatctggga gccacagtga cccagccacc tggctcaggc 45360
   tagttccaaa ttccaaaaga ttggcttgta aaccttcgtc tccctctctt ttacccagag 45420
   acagcacata cgtgtgcaca cgcatgcaca cacacattca gtattttaaa agaatgtttt 45480
   cttggtgcca ttttcatttt attttatttt ttaattcttg gagggggaaa taagggaata 45540
   aggccaagga agatgtatag ctttagcttt agcctggcaa cctggagaat ccacatacct 45600
   tgtgtattga accccaggaa aaggaagagg tcgaaccaac cctgcggaag gagcatggtt 45660
   tcaggagttt attttaagac tgctgggaag gaaacaggcc ccattttgta tatagttgca 45720
   acttaaactt tttggcttgc aaaatatttt tgtaataaag atttctgggt aataatgag 45779
<210> 31
   <211> 43093
   <212> DNA
   <213> Homo sapiens
<400> 31
   agaattcttt ggcaggggcg accttagaat cctggggagg agcgagaatg gaatcccggg 60
   gaggaacagg ggtggaatcc ggggggcggg gtcagaacgc caggaggggg cggggccgga 120
   gccagggtcg gcttgactcg gggagcagcg gggtggatcc tgtgacgtca gcgggttcga 180
   accgccggag ctgagcgaga ggccgggggt gccgagccgg gcggggagag ctgggccggg 240
   agagcagaac agggaggcta gagcgcagcg ggaaccggcc cggagccgga gccggagccc 300
   cacaggcacc tactaaaccg cccagccgat cggcccccac agagtggccc gcgggcctcc 360
   ggccgggccc agtcccctcc cgggccctcc atggcccggg ccgctgccct cctgccgtcg 420
   agatcgccgc cgacgccgct gctgtggccg ctgctgctgc tgctgctcct ggaaaccggt 480
   gagacgagcg gacggccccc tgccctcgcg cggaccccct ccaaccttac cttccgagct 540
   ggggaagccg agcaccttcc ccgcccaccc ccggctcccc gagccccctc tcgcgtgccc 600
   ccttcctggc tggccccaca gactccgacc ccctacacgt ccgattctct cgaggagact 660
   ctgacccctc ctttctgcat ctctctcacc accaccccca tccttcttaa tccaattgcc 720
   catcctacgc ctgcttgtct gtccccctcc ccatttctcg gattcccgcg ccgaaagggt 780
   taaatcccag gaaggcctgg tgccccctcc ccaccccgcg ccgagtagtt cacccggatc 840
   tgggtctctg tctcctctac ccctctcagg aatgtgggga atgttccccc cacctaccgc 900
   agtaaggtct cgaggaagag gcgagaccac ctccctcccc tatttttcct tcttcccggc 960
   ttgggcgtct gaggcccgga gagaactgag gacaagcgga cctgattcgg gtcccttgga 1020
   gggctaagga taagagttct ggcgacttag ggggaagggc ctgagctcgg agctccaacc 1080
   ccgaacctca gggagttggg ggttctgact ccgtggactc cagaaaaatt aggggccggg 1140
   ggctccgtct tcagggtccc tggtagaatg gaggctaggc gctaagtcta actgggatct 1200
   aggtaaaact gggggcgaga gaggatcaaa cttgtagctt ctggatttaa ttgtgggcag 1260
   ggggcttcgt tttcagggtt cccggcagaa ccgggggaca ggagactcga tctcgtgggc 1320
   ttcagactta attgtaggca gaggaacgtc ttctgagatc caaacagaag tgcgagcagc 1380
   gggccccgtc tcttgggctc cagatatgat caagggtcga aggctctgtc ttgaggctgg 1440
   agtctcagtc gcctgagtcc cagatagaac gggaggcccc gagtcccaga ggctgggaga 1500
   ctcctccctt tccctatgaa tggtcggggg cgacggagac gtcgccgggg aagttttcct 1560
   ggtttcactc tcgccgagcc agggaggggc ggggcgggcg ctcctggctc cgggttccgg 1620
   actccccggt ttcgggacct tatctatcct ctggtagcgc ctttgtcctc agagccatag 1680
   caggaggtgg acgagtggag ccgccgccct tatcccccgt ccctcccatt gctatgcctt 1740
   ggaccccctg gcgtccttcc atccacccca ctaggggagg agagcggccc ccctggggag 1800
   ggaggggctg ggagggaacc cttgaaagag gcgctggccc cgccccttgc aggtgacccg 1860
   gttgtgtatt cccagcgaca aactctggga tgctagagtt tcccgagggt ttgcgcagaa 1920
   gagagctcag ggggatcttg ggggaacccc ggccacctct ctggcctcat ttcccccagg 1980
   atggctctca ctggcctccc agctttgacc cagagctggg aactccgctt ccatcccctg 2040
   cctctccctt caggcagaaa taggcagatg agatcagaac tggcacctct gggtgaattc 2100
   tccgaaccca ccagaccctg agcctactgt tagcttggtg catgatctca ccgaaggctc 2160
   cagcttgccc tgggaaacag gtcctgtttc tgtttccatc ttagagatga ggaaacaggc 2220
   tcagcaaagg caagcagcct tctcaaggtc tcacagcagg tcccggggaa gccgggctcc 2280
   ggttcttcag agccccgcac cgggtgggct ccaggaagtg cctctgagtt tccatttcct 2340
   ccctcgtaaa aaaactgaga taacaatcct ttccctgcgg accatccgga aggctcccag 2400
   gccccactga ttccctgtga aacctcttag cccctggaga aggagctgtc ggcccctttc 2460
   ttgaccaccc ccaccccaat ccttcgcctc tgctgtccac tggctggccg gctggcatgt 2520
   gctcgctctc cctctctctc tctctcccct ccccctccct gttttcctcc ttccctcact 2580
   ttctttcccc ctcttcatct ctcttcgttt ctcacccttt ctctctccat ccctctccaa 2640
   gctctttctc tcttctcctc tccccatctc tctctctcct ctccgtcttt ctctgtatac 2700
   ctgtctagct ccactgcctc ccccgcttgg cctcctcggg gaatgcgggt gcaccacctg 2760
   ggcccccagc tgggccggct cccctccccc agctttcttc cctgcccatc cgtgtgtcca 2820
   tgtccgccag gcagccccac gtagtggagg gggaggggag cagagggacg gcagcctggc 2880
   tgtctcctct cattagggaa gggggactgg gcagaggaga agttgggggg tgggtatata 2940
   gagaggctgg agctacaagg atgggtacag aggccaatag agggaagcgg tcagacacag 3000
   atttaggaat ggggtcagag acacgtgtag gaatggaatc agaggataga aggggagtga 3060
   aatgccggca cagggtcggg ggagtgaaat accgacacag ggatgaggct gaaagacact 3120
   ggaatgggac ggaggagggt gggggaaggg agctagacat agataccaga tgaaggcagg 3180
   gacaggaaaa ggaaaggggt ttggtgacag gcagggagcc ccaaagactc cagctgtggg 3240
   acagcagagg tgccgtgacc cacggaggtc agggtacagg gggcctcaag gccagcgaca 3300
   tgtgggggcc acagggagtg atgaacacag aagggacagg gagctgggca catgggccag 3360
   ctcccccgcc cctgcctggg acgctgttac atttttaatg tcccagcagt gctgacaaat 3420
   ggagcaggaa ggggaggaga agaggggacc cagcaagatg ggccaggctg tgagaaggtg 3480
   tgtcagaaat atggggagac agggcactgg agacccagga ggcagggaga ctccacagtc 3540
   agagacagaa acagagacgt agaccagaag gcttgtgaga agagaacccc aggaatcaag 3600
   gcagagagcc aggttccaga cacagggaga cccagacgga gaaagagacc ggagatgtca 3660
   gacatacaga ggaaagagga agagagtgac taacggggac agggtatctt ctcaggctaa 3720
   tgaaaatgtt ctataattgc tcgggtgatg gatggctaac tctgaatata caaaagccac 3780
   tgaactgtac actttaaatg ggtagattgt atggtatggg aataatgtct caataaagct 3840
   gttacaaaga gacagagaat ataagaggca cagagcagag accagagact ctgggggaca 3900
   cagataagag tgacagaggc caggcacatt ggctcatacc agtaattcca gcactttggg 3960
   aggctgacgc aggagaatcg tttgaaccga gttcaaggct gcagtgagcc ctgattgcac 4020
   tactgcactc cagcctgggc cacagagcaa gaccctgtct caaaagagaa aagagtaaca 4080
   gagaccccaa gagagaccct gggaattgga gacacagagg gacagggacc ctaagaacta 4140
   gacacagaga gagatagaag aggccccgga agacacagat aagagacgct aagagacagc 4200
   agggatacca ttaaaagact ttcaagaagc caggcatggt gactcatgcc tataatccta 4260
   gcattctggg aggtccaggc aggtgtatca cttgagctca ggagttcgag accagcctgg 4320
   gcaacacggc gaaaccctgt ctctgcaaaa aatttaaaaa ttagccaaag gtggtggtgt 4380
   gtgcctatag tcccagctac ttggaaggct gaagtgggag gatcacatga gccaggaagg 4440
   ttgaggctgc agtgagctga gatcacacca cagtactcca ccctgggcaa caaagcaaga 4500
   tcctatctca aaaaaacaaa acaaaacaca cggattagag gaggccaggg ggcaggtgca 4560
   gaaatccagg cacagacaga cagccctaca tcctggctgc tgcagccgct gctcactccc 4620
   tgaaccagct cctggatttc cgctgtgggg gttgggcact gtgattaata caattaatta 4680
   actgacaagg tgactgaggc agcagctgag gccagtactc aaagcctctg gccttcaagg 4740
   gccttctcta gtcccgacgt gccttgccat gggaggctgg gtcgctgtgc tttatagatc 4800
   tgcgggggaa gccaggccct ggggagccag ccaggcagga gtccaggagc ccagctcctc 4860
   ctcccctaga cccaggtgtc cagcttcagg ccccgcctcc ctcagaccca gaagcccagg 4920
   ccccagcctc tcctccctca gacccaggag tccaggcccc agccctcttc cctcagaccc 4980
   aggagtccag gcccccatcc ctaatccctc agacctggga gttcaggccc ccatccctgc 5040
   tctctcagac ccaggagtcc aggccaccag tgcttcctcc ctcagaccca ggagtccagg 5100
   cccccatcct tcccctctca gacccaggag tccaggccct tagtccctcc tccctcagac 5160
   ccaggggtcc agcctcagtc ccctgctccc tcagacccag gagcccaggc cccctgctct 5220
   tcctccctca aacctgggag tctaggcctc cagctcctcc tccctcagac ccaggaatcc 5280
   aggcccccag ccccctcctt tctcagaccc aggagtccaa gcccccaacc tctcctccag 5340
   ggttccatag gtcactctgc accctcctcg ctgccttggt ctacccatcc ctggatctcg 5400
   gggtctcaag ctgtgtctgt ccccattgtt ctcccatggg ctcacactac accctgggca 5460
   gcctccactc tgccaccaca ggaagcctgc aggtgcagtc cacagggaga ggaagtgtgt 5520
   ctggggaccc agctcacact tcatgaagct gtcaccagtc tggcttgccc agtgacactg 5580
   agggaggctg gtgttcccat ggccacagtc ccctggctat ggtgctggcc atgtcctggc 5640
   caagttcatg gcacagcccc tgcctagcag tgtgctggat gatgggggct cactccaggc 5700
   tgaccaacct cctggctgta tctcccttgc ctccacctga catgcagagc gtctgggcag 5760
   cggccctcac ccctgtcccc tttacctccg cagccctgtc agcctccaag tcccaaccct 5820
   cccagcttct ccctgtcacc cccaccgctg gtcctgcttt cccattccaa gttctgagtg 5880
   tgtttccaat ctcgccatcc tccgtcccca cagccccgac cctgatcccg gcgcctcccc 5940
   ccgagacccc tgccccactg agggctcttt cttttttctt ttttttcttt ttttgagaca 6000
   agagtctcac tctgttgccc aagctggcaa tagtgtctca ctctctcact ctgtcttcca 6060
   agctggtaca gtggtgtgat cttggctcac tgcaacctcc gcctcccagg ttcaagcaat 6120
   tcttgtgcct cagcctccca agtagtgggg attacaggca ggtgccacca tgcccagcta 6180
   atttttgtat ttttagtaga gacgggctct caccgtgttg cccaggctgg tctcctgagc 6240
   tcaggtgagc cactgcaccc agcctgtgag ggttttttct ttctcttttt gttttttttg 6300
   tttttttttt gagacatggt agccaccacc tgtaatccca gctactcagg aggctgaggc 6360
   aggagcccga gacagagcaa gactccgtct caaaaaaaaa aaaaaacaga aaagacttgg 6420
   gcttgtttcc tgagggctct gtgcagagga gggcgtgttc tgcactttct agcaggatcc 6480
   ttctagctgt gcataggaac aaaccacaag gacaactgca gtcctccagg tgggaagtgg 6540
   cagtggatta gaccagggca gggactgggg aggcgtcaga ggtggtctaa ctagataaat 6600
   gtcaaagaag gaggtgacag ggtttcctga tagattgctt atgaggtgag agagaaagag 6660
   gtgaaaatgc cttaaaaatg gactgtaggc aggacgcggt ggctcacgcc tgtaatccaa 6720
   gcactttggg aggccaaggc aagaggatcg ctttagttca ggagtttgag aacagcctag 6780
   gcaacaaaca gtgagacccc ttctctacaa aaaaaaattt tttttaatta gtcaagtatg 6840
   gtggtgcccg cctgtagtcc caactactga gaaggctgag gtggtaggat tgcttgaacc 6900
   caagaggttg aggctgcggc aagctgggat cctgcactcc accctgagca acggagtgag 6960
   atcctatccc agaaaaaaaa aaaaggaatg ttaaccccac agggcctgaa ctatcctatt 7020
   cattcatttg gaattagaaa ttcagaatgg ggaacttaca gcaatgggat tttttttttc 7080
   agacggagtc tccctctgtc cccccagctg gagtgcagtg gcgcgatctc agctcactgc 7140
   aagctctgcc tcccagattc acgccattct cctgcctcag cctcccgagt agctgggact 7200
   acaggggcct gccaccacgc ccagctaatt tttatatttt taatagagac gtggtttcac 7260
   cgtgttagcc aggatggtct caatctcctg acctcgtgat ctgcctgcct tggcctccca 7320
   aagtgctggg tttacaggcg tgagccacca cagctggccc tttttttttt tgagatggag 7380
   tctcactctg tcccccaggc tggagtgcag tgctgcaatc tcagctcact acaacctccg 7440
   cctgctaggt tcaagtgatt ctcctgcctc agcctcctga atagctggga ttacgggggc 7500
   ccgccaccac gcccggctaa tttttgtatt tttagtagag acggggtttc accatgttgg 7560
   ccaggctggt ctcaaactcc tgacctcgtg atccgccctc ctcggcctcc caaagtgttg 7620
   gaattacagg cgtgagccat tgcgcccagg gattttagaa cctcattcat tcgttattca 7680
   gcaagtactg agcatctact aggtaccaat ctctcttgca gatactgagg gtacaggttg 7740
   tgagcaaaac agacagaaga tccctgccct cgtggcgctg gcagtcagtt tccaggagga 7800
   tgtgatatat ctagtgtgtg agatggtgag aagcactatg cagacagaga acgtaacagg 7860
   gcaaaagggc ggaagagcag tcaaccaggg aggggagagg aggtggcagc tgaggtggag 7920
   tatctcgggg taaaagaacc ccccgaggag tgtgccccac aagaagctaa aagtggtggg 7980
   tttgtttgag accgagtcct cactctgtca cccaggctgg agtgcagtgg cgcaatctca 8040
   gctcactgca acctccacct cctgagttca agcgactctc cctaaagtag ctggaattac 8100
   aggcatgcag ctaatttttg tatatttagt agagacaggg tttcactgtg tcggccaggc 8160
   tggtctcgaa ctcctggcct caagtgatcc atccaccttg gtctcccaaa gttctgggat 8220
   tacaggcttg agccaccgcc ccatcctaaa gtcggttttt ttgttttaat cctccaaatc 8280
   cagtagtgct cagatgccat cctaaatgga ggattggagg ggagcctccc ttagaaggaa 8340
   gatctgggcc cctggaagcc acaggactgc tccttaagtc aagaactctg aatctggcca 8400
   ggtgcagtgg cttatgccta taatcccaac actttgggag gccgaggcgg gtggatcact 8460
   tgaggtcagg agtccgagac tagcctggct gacatggcga aaccccatct ctactaaaat 8520
   tacaaaaatt agctgggtat ggtggtacat gcctgtaatc ccagctactt gggaggctga 8580
   gacaggagaa tcacttgagc tggggaggcg gaggctgtag tgagcccaga ttgcgccact 8640
   gcactccagc ccgggcaaca gagtgagact ccacctcaaa aaaaattttt ttaaaaagaa 8700
   ctctgagttg ccgggcgcgg tggctcacgc ctgtaatccc agcactttgg gagtcccagg 8760
   cgggcggatc acgaggtcag gagatcgaga ccatcctggt taacatggtg aaaccccatc 8820
   tctactaaaa aaattagccg ggcgtcgtgg cgggcacctg tagtcccagc tactcgggag 8880
   gctgaggcag gagaatgggg tgaacccggg aggcggaggt tgcagtgagc cgagatcgcg 8940
   ctgctgcact ccagcctggg tgacagagca agactccgtc tcaaaaaaaa aaaaaagaac 9000
   tctgagtcag cctggcgcag tggctcacac ctgtaatctc agcactttgg gaggccgagg 9060
   cgggcagatc acgaggtcag gagatcgaga ccatcctggc taacacggtg aaaccccgtc 9120
   tctactaaaa atacaaaaaa ttagcagagc gtggtggcgg gcgcctgtag tcccagctac 9180
   tcgggaggct gaggcaggag aatggcatga acctgggagg cggaggttgc agtgagccga 9240
   gatagtggca ctgcactcca gcctgggcga aagagtgaga atctgtctca aaaaaaaaag 9300
   aaaaaaactc tgaatcccag agacccaggg acttggagga ggtgaagggt gtggggctcc 9360
   agggacctgg ggctcagctt ttcaggtctc ctcctccagg aagctcttct ttcgttcatc 9420
   gtccctccct ccttcagaca ggtcaagcct cttcaggttc ctcccatacc acccctactc 9480
   cgtcttccag gcattcttgg tgataattat ttgtgcaata tttgtacccc ctgcaatctg 9540
   gatgtcaacc ctggaaaaca agcacctgat ttgtcctgtt acttctgcag catccacact 9600
   tagcaacatt cattcacttg gcagtgttta ctgagcacct actacatgct aggccctgtt 9660
   gtaggtgctg gggatacagt aggggaaaaa cacagacaaa cctcagggct ggagagggca 9720
   gaccctctag cacccaggac ctgtttgttg agtaactatt tgttttttgg atttgaccta 9780
   taagatgtga aagaacagcc agattcttcc aggttctgcc ttttcctgat ccctccctct 9840
   ctccctacta ccaaatccct tccattttaa aattttaaat cctggtgggg cacagtggct 9900
   cacgcctata atcccagcag tttgggaggc taaggtaggc agatcacctg aggtcgggag 9960
   ttcgaggcca gcctggccaa gatggtgaaa ccctgtctct actcaaaata caaaaattag 10020
   ctgagtgtgg tgacatatgc ctgtaatccc agctactcgg gaggttgagg cagaattgct 10080
   tgaacccaga ggcagaggtt gcagggagcc aagatggcgc cactacactc tggcctgggt 10140
   gatacagcga gaatgtgttt caaaaaaata aaatcctaag acctattagc ctcctcccca 10200
   acactcagtc tcagtcttaa cctcttgttc ttttcattgc caactttttg tgtcattttg 10260
   ctgtctggtc atctatctct gctcagccca ccaccttccc ctcaggcctc cttggatgaa 10320
   gttaagtcat tctctatttg aagaacccgc aacggctccc acttgccaca aagaggaaac 10380
   taacttcctc ggccccagcc tactttgctg tctctgcagt tcacacaggc ccaaatggct 10440
   ttccttgacc ctcattgagt ggccttcccc ccttgcctag ctaggaaggg gccagagcgc 10500
   ttaaccaccc tgatcccaac cttagtgcct ttgggggcca ggcgggtgat aagtcccagc 10560
   acctggggaa tctggaggag ccggaaggga gtgtgccttg tgggaggggg gtgatgtcct 10620
   tcctcagccc cacccagctg ttgcccccag gagccacata tccggacttc tgtctggagc 10680
   tttagacagc aatctccagg ccttctgtgt gggctgctac ttcccaactg aaataacact 10740
   ctgtgggcca aagcaaacat gcaccacagt cagatctggc ctgcagcagg cggctgaccc 10800
   ctgctgctct gctcacctgg gccacgcccc gtgaacatcc cccatgagca agtccttccc 10860
   tggaactggc acaagggacc ctggagggtg gctgaaacat ccccatttta cagatggagg 10920
   aagttgggct tggagacaag gctgccagct ggaaagggtg tcagcgttaa ataacaagat 10980
   tcagaaaata ggattaagta tagagtttat tcaagcctaa agcttgagga tggccacccc 11040
   ggagtgtgga ttcaagttgc cccaaataaa ccctttgact agctgtgatt acaagtggac 11100
   tttttttttt tttttttgag acagggtctc accctgtcac tcagtctgga gtgcagtgtg 11160
   tgatcatggc tcactgcagc gtcagtttct cgggctcctg gccttgagtg attctcctgc 11220
   cacagcctcc ctagtagctg ggactacagg ccctagccat cttgcccagc tatttttttt 11280
   ttttgagacg gagtcttgct ctattgccca ggctggagtg tagtggcaca atctctgctc 11340
   actgcaacct ccacctcccg ggttcaagca atcctcctgc ctcagccccc ctagcagcta 11400
   ggattacagg cacctgccac catgcccggg taatgtttat ttttttagta gagatgggat 11460
   ttcgccatgc tggccaggct ggtctcgaac tcctgacctc aggtgatcca cctgctttgg 11520
   cctcccaaag tgctgggatt acaggcgtga gccatcgtgc cgggtttttg tttttgtttt 11580
   ttttttttta agagatgaag tctcactata ttgtccaggc tggtctcaaa ctcctggcct 11640
   caagtgatcc tccacccact tcggcctccc atagtgctgg gattacaggc atgagccaac 11700
   gcacccagtc tgcaagtgga tttttaagga acaaagaaga gagagttcct aaattgttac 11760
   caagaattta cattgaaaca gcataaggta tctgcaggtc tggctcagaa aaaaagataa 11820
   cagaaggtat tgattggcta tgcactgttt attgtatcac aaattccagg aacaagaagc 11880
   taatggggag ggcagctgtg caggaaccac tgcctcgggc atgggtggag gaggcgtgtg 11940
   accgaagtcc cctattcact gtacagtctc cctgggcctg atacatttcg catagctcag 12000
   actgatctga gctatttttc ttttctcaag ttgggtgggg gtggcccctc tggactggaa 12060
   aagcctcatt tttcccccaa gtgacacacc tgagtttgtt tgacttgttt cccccaagtg 12120
   acacacctga gtttgtggga cttgtgtgga gatgatgtat ttatgtcaca cttcagtctt 12180
   cgctgatggc tttttttttt atatttattt acattttttc ttttaaggta ggcagggcct 12240
   cattctatcg cccatgttgg agtgcagtgg cgaggtctct gggttcaagt gattcccctt 12300
   cctcagcctc ccaagtagct gggaatacag gggcccgcca ccacgcccag ctaatttttg 12360
   tatttttggt agagacgggg tttcaccata ttggccaggc tggtctcgaa ctcctgacct 12420
   cagggtgatc catccgcctc ggcctcccaa agtgctggga ttacaggcat gagccgcagc 12480
   acccagcctt ttttctttct ttcttttctt tttgtttccc ctcccgagac cgagtctctc 12540
   tctgtcaccc aggctggagt gcagtggtat gatctcagct tactgcaacc tccacctccc 12600
   gggttcaagc aattctccca ccttagcctc ctgagtagct gggattacag gtgcctgcca 12660
   ccacacccag ataatttttc tatttttagt agagatgggg ttttgccatg ttggccagtc 12720
   tggtctcaaa ctcctggccc caagtgagcc tcctgcctcg gcctcccaaa gtgctgggat 12780
   tacaggtgtg agccaccaca cctggcctta ttttattttt aatttctgtt cttgtattgc 12840
   tataatttac tgatgatttc ttacccaaag atcctcggcc tgtccccacc caaggggact 12900
   gccttccacc ggggacccct cataagagtt actcattagg atggtgttcc ctggggttcc 12960
   tgtgtgtatg acgccagggc ctgtgcaccg cctccatggc ccatctccat cagacagagc 13020
   tgggcagtgg tgttggccct cgaaatgagg cttgtgccct ggcctttcct ggcctggggg 13080
   ctgagtgcca aggccacctg taattaaact gagcttccta tgtttactta gcaagcactg 13140
   acctctcatt tcctggggcc ctatgctgag tgttccacag ctattcttta atctttaaac 13200
   agcccttggg acgcaggcac tattattaac ctcacatcac agctggggaa actgaggcac 13260
   agagaggtta agtggcctgc acaaggtcgc agagccagga ttcaaaccca tgaactatgg 13320
   gccactcagt cgccatgctg tactgccgtg cacagcagag gaagatttgt tcttgttctt 13380
   gtttatatta aaagtgagaa aacatgggcc acacgccgtg gctcacgcct gtaatcccag 13440
   cactttggga ggccaaggcg ggtggatcac ctgaggtcag gagtttgaaa ccagcctgac 13500
   caacatggtg aactctggtc tctactaaaa attcaaaaat cagccggacc gtagtggctc 13560
   gcacctgtaa tcccagctac tcaggaggct gaggcaggag aatcgcttga acccaggagg 13620
   cagaggttgc agtgagttga gaccgtgcca ctgcactcca acctgggtga cagagcaaga 13680
   ctccgtctcc aaaaaaaaaa aaaaaaggtg agaaaccact taggctggct gagaactgca 13740
   aacatgcgtc cacataaaaa cttgaacttg catgatccca gcagctttat ttatcagagc 13800
   caaaaagttg aaacaacctc aatgcctatc agcggctgaa tggataaaca aaatgtggtc 13860
   cgtccatgta gtggaatatt attgagcctt aaagaagagt gaagcgccga cacgtgttac 13920
   agatggatga acctggaaaa caagatgcca agtgaaagaa gccagacaca aaggctacat 13980
   agtctattac atgaaatgtc cagaacaggc acatcaacag agacagaaag ttgcatcgtg 14040
   gctgggggaa tgggaagtga ctgcttaatg gataaggggg ttacttaggg aattataaaa 14100
   atgcccaggc tgggcgcagt ggctcatgcc cgtaatccga acactttggg aggccgaggc 14160
   aggtggatcg cttgagctca ggagcctaag caacatgatg aatccccatc tctacaaaaa 14220
   atacaaaatt agctgggttt ggtggctcgc acctgtagtc tcagctactt ggggggctga 14280
   ggtgggagga tcacttcggc cccagagacg gtttgcagtg agcagaaatc gcgccattgc 14340
   acgccagcat ggacagagtg agatcctgtc tcaaaaaata ataccttaaa actgattata 14400
   gtaacagatg taaaactcca aatatactta aagccactga attgtactgt attttttatt 14460
   ttactttttt ataaaaaaat tatatatata tattgttttt tcttttcttt tcttttttca 14520
   agacagagtc tcactttatc acccaagctg gagtgcagag gcgcgatctc ggctcactgc 14580
   aacctccgcc tcctgggttc aagtgaacac gtccggctaa tttttatatt tttagtagag 14640
   acagggtatt tcaccgtgtt ggccaggctg gtctcaaact cctgacctca agtggcccac 14700
   ccacctcggt ctcccaaagt gctggaatta caggtgtgag ccactgcacc cagccttaat 14760
   tgtatacttt aaatgggtga attgtgtagc gtaagaattt atatctcgat aaagctgttt 14820
   aaaaaaaaaa aaaactacgt aaaaaatgag caaaggaccc gaagagacat ttttctaaag 14880
   aagacataca ggcagccaac aaacatgaaa aaatgctcaa catcattaat catcagagaa 14940
   atgcaaattt aaaccacaat gaggtgccat ctcacctcag tcagaatgtc tgttactaaa 15000
   aagtctaaaa aaacagatgc tggcaaggat ttggagtaaa gggaccgctt acccactgtt 15060
   gatgggaatg taaattagta cagcctctat ggcaaacagc atggagattt ctcaaagaac 15120
   taaaaataga actaccattt ggtccagtat tccactgagt atcctcccaa aggaaaagac 15180
   atcattatat caaaaagaca cctgcgcttg tatgtttatt gcagcactat ccacaacagc 15240
   aaagatatgg aatcaactta agtgtccatc gatgaatgga ggattggata aagaaaatgt 15300
   ggtctatatc tacaaagatt tcatgacgaa attgccaaaa gcagtgcagc aaaagcaaac 15360
   attgacaaat gggatctaat taaactaaaa agcttctgca cagcaaaaga aactatcctc 15420
   agagtgaaca gacaacctac agagtgggag aacatttttg caatctatcc atctgacaaa 15480
   ggtctaacat ccagaaccta caaggaattt aaacaaattt acaagaaaag aaaaacctaa 15540
   aaaagtgggc aaaggacacg aacagacact tctcaaaata agacagtcat gcagccaaca 15600
   aacatgaaaa aaatgctcaa catcactgat cgttgagaaa tgcaaatcaa aacaatgaga 15660
   taccatctca caccagtcaa aatggccatt atcagaaagt caagaaacaa cagatgcagg 15720
   caaggttgtg gagaaatagg aatgctttta cactgttggt gggaatgtaa attagttgaa 15780
   ctattatgaa gacagtatgg cgattcctca aagatctaga accagaaata ccatttgacc 15840
   cagcaatccc attactggta tatacccaaa agaatataaa tcattctatt acaaagatac 15900
   atgtatgcgt atgttcattg caggactatt cacaatacca aagacatgga ctcaatccaa 15960
   atgcccatca tggatagact ggataaagaa aatgtacagg ccgggcactg tggctgacgc 16020
   ctgtaatcct agcactttga gaggccgagg cggcggatca cgaggtcagg agatcgagac 16080
   cattctgggt aacacggtga aaccccgtct ctactaaaaa tacaaaaaaa ttcactgtgt 16140
   gtggtggcgg gcacctgtag tcccagctac tcgggaggct gaggcaggag aatggtgtga 16200
   acccaggagg cagagcttgc agtgagccga gatggcacca ctgcactcag gcctgggcaa 16260
   aagagtgaga ctccgtctca aaaaaaaaaa agaaaagaaa aaagaaacct gcacatgtat 16320
   cctggaacaa taaaaaaaaa aaagaaagaa aatgtggtat atgtctacca tggaatacta 16380
   cacagccata aaaaaggatg ggatcatgtc ttttgcacca acacggatgg aactggaggc 16440
   cattatcctg agtaaaatag aaagtcaaat accacatgtt ctcacttata agtggaatct 16500
   aaacagcaga tacacaaaga ttggaataat ggacagtgga gactacaaaa ggtgggaggg 16560
   agggaaacag gtgagggttg aaaaattaac tgttggaggc tgggcacagt ggcgcacacc 16620
   tataatccca gcactttggg aggccgacac gggtggatca cgaggtcagg cgttcgaggc 16680
   cagcctggcc aacatagtga aaacccatct ctattaaaaa tattaaaaaa ttggccgggc 16740
   gcggtggctc acgcctgtaa tcccagcact ttgggagccc gaggcggcag atcacgaggt 16800
   caggagattg agaccatcct ggctaacatg gtgaaacccc gtctctacta aaaatacaaa 16860
   aaaaaaaaaa aaaaaaatta gccgggcatg gtagtgtgcg cctgtaatcc cagctactcg 16920
   ggaggctgag gcaggagaat tgcttgaaat tgggaggtgg aggttgcagt gagccgagat 16980
   cacaccactg cactccggcc tgggcaacag tgcgagagtc tgtctcaaaa aaaagaaaaa 17040
   ttgactgttg gaagccaggt gcattggtgc atgcctataa tcccagcact ttgggaggct 17100
   gaggcgggct gctcactttg agttcaggag tttgagacca gcctgggcaa catggcaaaa 17160
   ccctatctct acaaaaacaa aaattagccg ggcactggtg gcacatgcct gcagtcccag 17220
   ccactcaagt tgcagtgagc caagattgtg ccactgcact tcagcctggg agacagagtg 17280
   agaccctgtc tcaaaaaaga aaaagaaaaa ttaactgttg ggtacgctgt ttgctattca 17340
   ggtgatagat atgctaaaag cccagacttc acctctatgc aatatatgta tgtaagaaac 17400
   tggcacttgc acctgctaaa aatataataa ttttaaaatt tttattttaa taaaaaaata 17460
   gaaacttagg gccaaggggg gagctgatca tttgaggtca ggagttcgag accagcctgg 17520
   ccaacgtggt gaaagcccat ctctactaaa aatacaaaaa ttagccaggc atggtggcgg 17580
   gcgcctgtaa tcccagctac tcaggaggct gaggcacaag aatcccttga accagggatg 17640
   gaggttgcag tgaccaagat tgctccactg caccccagcc tgggcaacag agcgagactc 17700
   agtctcaaaa aaaaaaaaga aacttgagaa aaaatgctat gtaggaggtc tgtaggtttc 17760
   ataggaaggt gagagaaagt atattactga ggggggatga agacaatact cagcctttaa 17820
   atgttttaaa atgtatttta tgaaagtttt tttttattga tttattttca gaggattccc 17880
   cccccccaaa aaaaaatctt atgaaaattt tggggtgggc tgggtacagt ggctcacgcc 17940
   tataatccca gcactttgag aggctgaggt gggaggatca cttgagtcca ggagttcaag 18000
   accagcctgg gcaacatagc gagacattgt ctctaccaaa acaaacaaac aaaaaataca 18060
   aattagccag gtgtggtggt acatgcctgt agtcccagct tgaacctggg aggtcaaggc 18120
   tgcagtgagc tgggtggccc actgcactct agcctgggtg aaagtgaggc cctgtctgag 18180
   aaaaaaaatg tagttgaaag aaaagaaaac ttttattttt ttattttctt ttttaatttt 18240
   aaaggcaggg tctcactctg ttgcccaggc tgttcttgaa ctcctaggct caagcattcc 18300
   tcccaccttg gcctcccaaa gtgctgagat tgaagtcttg agccactacc ctggccatca 18360
   tgtcaacttt taaacataca aatacaggta agaatggcct ggtggccggg cgcggtggct 18420
   cacgcctgta atcccagcac tttgggaggc tgaggcaagc ggatcacgag gtcaggagat 18480
   tgagaccatc ctggctaaca cagtgaaacc ccatctctac taaaaataca aaaaagaaaa 18540
   aaattagcca ggcatggtgg caggcgcctg tagtcccagg tactcaggag gctgaggcag 18600
   gagaatggcg tgaacccagg aggcggagct tgcagtgagc cgagatcgcg ccactgcact 18660
   ccagcctggt caacagagca agactccgtc tcaaaaaaaa aaagaagaat ggcctggagc 18720
   ctccctcacc ttctccacct agacccactt gacaatgggt gacatatttt ggtttttgct 18780
   gaaccattta ggagtaactt gagatcatga cattgcgact tcatatcttc tgggaaaaat 18840
   agtgtgtgca cagccacaat ttaattatca cacctcggaa catgaacagc aattccaaaa 18900
   tcatatgtaa tatgaactca tagtacacgt tatagttgta taatagaata gtcattttag 18960
   tcttccctaa aatgtctttt ccaggtggct tttttggaat caggatgctg cgaagctgcc 19020
   tacgttgcat gcggagcctg catttcccgt ggggccccct tcgtggtggc cctgcctgga 19080
   ggtgtctggg tccctccccc acccgactac ttcactctct gtcctctctg cccaggagcc 19140
   caggatgtgc gagttcaagt gctacccgag gtgcgaggcc agctcggggg caccgtggag 19200
   ctgccgtgcc acctgctgcc acctgttcct ggactgtaca tctccctggt gacctggcag 19260
   cgcccagatg cacctgcgaa ccaccagaat gtggccgcct tccaccctaa gatgggtccc 19320
   agcttcccca gcccgaagcc tggcagcgag cggctgtcct tcgtctctgc caagcagagc 19380
   actgggcaag acacagaggc agagctccag gacgccacgc tggccctcca cgggctcacg 19440
   gtggaggacg agggcaacta cacttgcgag tttgccacct tccccaaggg gtccgtccga 19500
   gggatgacct ggctcagagt cataggtgag cagggtaagg gcgggaggca aggaggtggg 19560
   agggccgcgg tgtgggagca tccccttgcg ggtcagtttc tctcttggct tcagctgtga 19620
   ggttcacatt ctctgtgggt ttccatccat cagcaaatgt tcattaagca cctaccgcgt 19680
   gccagatgct tttctgtgtg ctgaggatgt ggcaggcaac caggcattct tgggaaaaac 19740
   aaaaaaataa gtattggctg ggcgcagtgg cccacccctg taatcctagc actttggaag 19800
   gccgagatgg gcagatcgcc tgaggtcagg agttcaagac cagcctggcc aacatggtga 19860
   aaccctgtct ctacgaaaaa taaaaaatta gctgggtgtg gtggcccgtg cctataatcc 19920
   cagatactca ggaggctgag tcaggagaat cgcttgaacc caggaggcag aggttgcagt 19980
   gagccgagat tgtgccacca ctgcactcta gtctgggtga cagagcaaga ctcggtctaa 20040
   aaaaaaaaaa agtattgagg ttgggtgtgg tggctcacga ctataatcca agcactttgg 20100
   gaggctgagg caggtagatt gcttgagccc atggtggtgc gcagatgtgg tcccagctac 20160
   ttgggaggct gaggtgggag gatcgcttga gaccaggaat ttgaggctgc ggtgagctgt 20220
   gatcatgcca ctacactcta gcctgggtga cagagtgagg ccctatctct aataaataaa 20280
   taaaagagta agtcaggcag ttaaaggggc catggaagtt ggagagggcc aagttgcaat 20340
   tctagctaag gaggtctggg gatatttagg cagaaactag gagaggagga gccctctggg 20400
   tatctaaggg aagagcgttc caggcagacg gaatggcaag tacaaaggcc ctgagacagg 20460
   aatacctgga gtatttgagg gccaggaagg aagtcagggt ggctggagac atatgagtgg 20520
   gggaaagtag gaagcaataa agtcaaggag aggatgggga cagattggca aaagccttgt 20580
   ggactataag aagcctttgg cttttgccca gggaggataa gagctgtagt aggttcttag 20640
   cagcagacag gtttgatctg gcttgggttc tcaccagctc ctcctggctg tgttgggagg 20700
   ggaaagaatg ggagctgaga aaccacaggg taaggcaact acaaagtccg ggtggggccg 20760
   ggtgctgtgg ctcacgccca taaccccagc actcagggag gccgaggtgg gaggatccct 20820
   tgagcctagg aatttgagac catcctaggc aacatagtaa gactcttgtc tcaatgacaa 20880
   aaaaaatttt tttttttttt gagacagagt tttgctcttg ttacccaggc tgtagtggag 20940
   tggcgcgatc tcggctcact gcaacctccg cctcccaggt tcaaccaatt ctcctgcctc 21000
   agcctcccaa gtagctggga ttacaggctc ccaccaccac acctagctaa atttttttgt 21060
   atttttagta gagacggggt tgcaccatgt tggccaggct ggtcttgaac tcccgacctc 21120
   aggtgatccg tccacctggg cctctcaaag tgctgggatt ataggcatga gccactgagc 21180
   ctggccagga aagaaaaaaa aattgtttta attaaccagg ctcagtgact tacctgtaat 21240
   ttcagctact cgagagactg aggtgggagg atcacctgag cccaggaggt tgaggctgca 21300
   gtgagctgtg attgtgctac tgcactccat cctggacaag agagcgagac cctgtcttaa 21360
   aacaagtcca gtccgggcag gaagtaaaac ggcttagaag taggggacat agtggcctca 21420
   ttgagatagg atgtgcaggg aaggcgtctt agtcaagtcc tggagtctga gaaggagcta 21480
   cctttgggca tagcagaggg gcacgggcac agaaacctgg gaggtgggca tgagatggga 21540
   gctgggcgcg ttccaggaat ggaaaggggg ctggtgagga taggcaaggg cttgggtgac 21600
   cctcagcggg gccacattac acgggcttca caggtggagg cgaggaggct ggactatgcc 21660
   ctcagcatgg ttaagagcca tgggaggtgt caagccacag gagagatctg gacaaacgtt 21720
   tgaggttgca aaaaatgacc ggcagcagta tgaggagtga ttggaggagt ggacggaggt 21780
   agaagggagc tgggacgaga ggggagaaga ggcattgctg ggaagacgga tgaggcagtg 21840
   gggggcagag ggaagcgggc ggcatatagt ggtgttttta gaagatagta ggtgctgggc 21900
   ttgcccatgg attgaggggg aagcaggatc agagaggagt cccaggcctg gtgcggtggc 21960
   tcacgcctgt aatcccagca ctttaggagg ccaaggtggg cagatcgctt tgaggtcagg 22020
   agtttcagac cactctgggc aacatggtga ggccccatct ctacaagaaa aaaaaaaatt 22080
   agctgggcat ggtagcacac acctgtagtc ccagctaccc aagaggctga ggtgggagga 22140
   tcgcttgggc ctgggaagca gagattgcaa gtgagccaag attgcaccac tgcactccag 22200
   cacgagaccc tgtctcaaaa aaaaaaaaaa aaaaatagga ctcccagcgg gaaggtgact 22260
   gatacttggc aaaaatagag aaggcaggga agattttttt tgaaggatgc ttttttttct 22320
   tttccttttt cttttttttt ttttttcttt ttttgagaca aagtctcact acattgtcca 22380
   ggctagactc gaactcctgg gctcagggga tcctcctacc tccgcctcct gcatagctgg 22440
   gattacaggc atgagtcacc acatccagct acgaatgctc tttctttaaa tcttgaggct 22500
   gggcgtggtg gctcactcct gtaatcccag cactttggga ggccgaggcg ggcggatcac 22560
   gaggtcagga aatcgagacc atcctggcta acacggtgaa accccatctc tactaaaaat 22620
   acaagaaatt agccaggcgt ggtggcgggt gccttagtcc cagctactcg ggagattgag 22680
   gcaggagaat ggcttgaacc cgggaggcgg agcttgcagt gagccgagat cgcgccacta 22740
   tactccagcc tgggcgacag agtgagactc tgtctggaaa aaaaaaaaaa tatgttggaa 22800
   gtcccctggg agcccacctt ccctaggcaa cttcccagaa gccacgtcac tctggatgcc 22860
   ttttcttccc ccttctcagc ccagagcgtc tgagaaggtc accctgtgct atccttgagt 22920
   aaacacttcc ctttccagta acacatgaaa ccacagccgt tttccataat acaaaggaca 22980
   gctcttctcc tccccaccct cacaaacgcc tctcttcttg tgcaatcatt tgttctcatc 23040
   cagagataac ttcagggctg ggcacggtgg ctcacacctg taatcccagc actttgggag 23100
   gctgagaggc aacaagagca aaactctgtc tcaaaataaa taaataaata atgagactca 23160
   gaaaatatga ttaagggcca ggcgcagtgg ctcacgcctg taatcccagc actttgggag 23220
   gccgaggcag gcggatcacg aggtcaggag attgagacca tcctggctaa cacggtgaaa 23280
   ccctgtctct actaaaaata caaaaaaatt agccgggcgt ggtggcgggt gcctgtagtc 23340
   ccagctactt gggaggctga ggcaggagaa tggcatgaac ccaggaggcg gagcttgcag 23400
   tgagccgaga tcgcatcact gcactccagc ctgggcgaca gagcgagact ccatctcaaa 23460
   aaaaaaaaaa aaaagaaaag aaaatatgat taagtgtagg gtttaatcaa gcccaaagct 23520
   tgaggatgac cacctgggag catagattga agttgccctg aatatacact tcaagcaggg 23580
   gccaaggcaa gcagctcttg caagtgagct tttgtttgtt tgtttgtttc ttggagagag 23640
   ggtctccaaa actacaaata aaaattagct aggaatggtg gcgcacacct gtaatcccag 23700
   ctactcagga ggctaaggca ggcgaattgc tgaaaccagc aagggcaggt tgcagtaagc 23760
   tgagattgca ccactgcact gcatcctggg tgacagcaag actctgtctc aaattaataa 23820
   aaaaaaaaga gagagagaga gataacctca agagcttccg tatacacact ggtgcagaag 23880
   taaggaacaa aacagccaaa gcctctgtct tcatgatgcg tgagccacca aggaatagtt 23940
   ggccagagtc ggcggtcagg aaagtgccct tgacgaagtg gggcttggag tggggtctgg 24000
   agaaggaggc caaagcaatc caggcacaaa atcaccaacc aaaaccccaa agtgggtgga 24060
   ggcagagact gaggagggcg tggatgggga gcatgtggag tggccagggg aggtggccat 24120
   ggggaagtgg tgggctcggg ccctgagtca ctattctaac agcttggagg gcggccagga 24180
   ggatgtgggg aggcttgttg gatgctccac gatggtcagg ggagaaggcg gtgggaaggg 24240
   agagctggga agaagaggag agtcagaccg tgttgagaag ttcagaggtg gatacccagg 24300
   tccagaaagc aacagaagat gtgagtctgg gtgggggaca cacatcataa gcatgagctg 24360
   ctattcttcc tggagttttg tttttgtgat ttttttccat gttttccaag agaatgaggt 24420
   ggaaagggta taaaaaaaag aaacggcttt gagattttct gcctttatca cacttccagt 24480
   cattcaatca tctaatggtg attacaggaa aaggaacagg gggcctttcc agctggacct 24540
   ttgaaccaca gtactcagtt actcgctcgg ccttgatttg cttttttttt ttcctttttt 24600
   ttttttttct tttggagaca gagtctcact gtcacccagg ctggagtgca gtggcacgat 24660
   ctcggctcac tacaacctct gcctcccagg ttcaagcaat tctcttacct cagcctcccg 24720
   agtacctggg attacaggca cctgccacca cgcccagcta atttttgcat ttttagtaga 24780
   gatggggttt caccacgttg gccacgctag tctagaactc ctgacctcag gtgatctgcc 24840
   cacctgggcc tcccaaagtg ctgggattac aggcatgagc cactgcgccc atcctaattt 24900
   ttgtattttc agtagagatg agttttcacc atgttggcca gactggtctc aaattcctgg 24960
   cctcaagtga tccaccctcc tcagcctcct aaagtgctgg tattgcaggt gtgaaccacc 25020
   acgcccgacc agcttttttt taaatctcag cctcctctag gcttttggac cctgtctctg 25080
   catctctggc agggcgaaga gacagtatac cgaaatggcc aggccaggcg cctgtaacct 25140
   cagagctttg gggagccgag gtgggaagat ggcttgtgct caggagtttg agatcagcct 25200
   gggcaacata gcaagaactc atctctacaa aaaaatttaa aaattagccg ggcatggtgg 25260
   tgcatgccta tggtcctagc tactctggag gccaaggcgg gaggatcact tgagcctagg 25320
   aggtgcaggc tgcagcaagc tatgatcaca ccactgcact cgagaccagg tgacagagcg 25380
   ggcccctgtc tctaaaaaat aaataaagta aaataaaatg ggcaacactt ggatagggca 25440
   gtgcaggcca gacattaccc taagccattt acctatcaat gtattattta attcacacag 25500
   caatcctagg ggtgggtact ttcattatcc ccattttaca aaacaagaaa cagctacaga 25560
   gttgcaaaac cacttatccc aagccaggcc gctgataagc agcagagctg ggattggaac 25620
   cccggcagtt agggcctaac caccctgctc ctctgctgag tgtttgttga atgactgccg 25680
   gtgaggagtg acgcaccccc tctcctcctc tccccagcca agcccaagaa ccaagctgag 25740
   gcccagaagg tcacgttcag ccaggaccct acgacagtgg ccctctgcat ctccaaagag 25800
   ggccgcccac ctgcccggat ctcctggctc tcatccctgg actgggaagc caaagagact 25860
   caggtgtcag ggaccctggc cggaactgtc actgtcacca gccgcttcac cttggtgccc 25920
   tcgggccgag cagatggtgt cacggtcacc tgcaaagtgg agcatgagag cttcgaggaa 25980
   ccagccctga tacctgtgac cctctctgta cgctgtgagt gtatcggggg tgacccctcc 26040
   cccatcaaaa ctgcctacac tggcttctct aaagcactgt ctacattgtc tctgaatgtt 26100
   gtctacgtgg gttccctgaa gccaaattct gcactacctt cctgccattg tctacactgg 26160
   ctcccatggg caaatctaca tacttccccc tcattgtcta cattggcttt actgggttcc 26220
   tgcatgctgt gcctagtctg tggtaacctg ggatgggctt gcaagagcca gtttgataca 26280
   tttctttcca attccacatc caatgaagtc ttctcggtag cttgaaatca accttgggga 26340
   atatttacac caaggaaatt ggcaaaccct agaaatgggg gcttctttta ttccccagag 26400
   agttggttta ccagcaaaga gctgccccca acagtgtctt ttctggctcc catggacccc 26460
   ggttgacact gcccctggac cgttagatca atcatgctgg gccatgtcca catgtctaca 26520
   tgtctacact gcccaaccta acatcgtctc cacaccttcc catacttctg ccttgacttc 26580
   cctaggtgcc atctatactg agtctcccag agtctctaca ccgacccctg atccttgata 26640
   ttatttacat tgtatttatt atttattatt tattattata tttttatata ttatatatta 26700
   tataaaatat ataaattata tattatatat taacatatat tttgtcatta tttattattg 26760
   ttatttaata taatataatt acattatatt atatattaca ttacaggctc caccccccgg 26820
   gccctgttga tcctgacccc tcccactggg ttttttgcta gctcttccag aaactgccta 26880
   tactcacacc tcgtaatgtt acccagagag gccaccctgg cactcttcac acagaatccc 26940
   tcaaatactg gcttaaccac ctcttccacc actgtccaaa atgaccacat atcccaactc 27000
   aatgttgtct acactagctc cccaggcaat gcctccactt acacagattc cctaagccaa 27060
   gtctacacca gctcccccag gcaacatgca tcacgtgcgc ccataagtgc aacagctccc 27120
   ctgagcctcg tctgtgccac tttcctcccg ggacctacac tggttgccca aaccctgttt 27180
   tgacatcctc cctccatgcc aaaaacacac acacaggccg ggtgcggtgg ctcacacctg 27240
   caatcccagc actttgggag gccaaggcgg gcagatcact tgaggtcagg agttcgggac 27300
   tagcctgggc aacatggtga aaccctgtct gtactaaaaa taccaaaatt agctggacgt 27360
   ggtggtacat gcctgtaatc ccagccattt gggaggctga ggcggaggga tggcttgagc 27420
   ccaggacgtg gagtttgcag taaaccagga tcaccccact gctctccatc ctgggcaaca 27480
   gagcaagatc ctgtctcaaa aacacacaca cacacacaca cacattattt gtagtggctc 27540
   ccttaggcac tgccagcact gaacaataac tcccacaaaa ttatccacag tggtttccct 27600
   gaacagtgtc tacactgaca tcagcacaca acagcctgga ctgatttgcc ccagacagtt 27660
   ttgactgctg tacctcctgc caacccgccc cgtttcttta gcattcctgt ctatctgcta 27720
   acttgtccac ccgctctggg attctcctcc ttgctgatcc agtcccccca tttcccccag 27780
   accctcctga agtgtccatc tccggctatg atgacaactg gtacctcggc cgtactgatg 27840
   ccaccctgag ctgtgacgtc cgcagcaacc cagagcccac gggctatgac tggagcacgt 27900
   gagtcacgtg gtctcagaac cctgggtctg agggaggcgg ggctgggggc ctggactcct 27960
   ggatctaagg gaggaggggc tgggggcctg gactcctgga tctgagggag gaggggctgg 28020
   gcctaaattc ctaagtcctc caggatgaag ggagcttgca ttttggcaat ttggggtctc 28080
   cgggagtact taggtccctg gagcttggct cctggtgggt gtatctttag ggatgaggcc 28140
   tgtgctcccc tctcgacctt ggtatccctc tcacctgacc ccacacccct ccaggacctc 28200
   aggcaccttc ccgacctccg cagtggccca gggctcccag ctggtcatcc acgcagtgga 28260
   cagtctgttc aataccacct tcgtctgcac agtcaccaat gccgtgggca tgggccgcgc 28320
   tgagcaggtc atctttgtcc gaggtgagtg ggtcttgggg gccgtgtgtg gagacctggg 28380
   tccgctcccc tggagttctg cccttcagga cttggggctg cactggggga ggctgcgccg 28440
   ccgacctggg agggatgcag acctgcctgg ctgaggggag atgagggttg gccttcccct 28500
   cgtggcctca gactggggtc tggatttggg gtgtcggggt aggtgaaggc agcagagttg 28560
   gggggttgag gactttgctc ggggttccta gaaagtagag gcctgggtag ggtcctcacg 28620
   aatagacccg aggaagctgc cctgggctcc agctcctgag aaagacgcac acctagagtc 28680
   aggcatgcaa acttcaaaga gacagaaacc aagagctccc acccccagaa acacaatccg 28740
   gccccaaggc acggagccaa agaggaaaag cacaaagcca gaccatcacc cagtgatgga 28800
   aactgggtca aactcacaac tgcagggcca cacacctaga gcgcggccgg gactgttaac 28860
   agagccatgc ttcctgggac agagagaccc ctgggtcctg gagatagggc cagagacccc 28920
   tcctgtcaga gccacagctg gcaggatcca cagccacaga cactccctgt gctgagaaat 28980
   tctgttgcag agatgcatca gaatccagac actcacctgg atggtaaccc tgcacgctca 29040
   aacccattgt caccaagaca cacacccagg aggacagtgg caccagccaa gatagactga 29100
   acgaagccag atgacacctg gaaagggaca ttcttttttt ttttttttga gatggagtct 29160
   cgctctgtcg cccaggctga agtgtagtgg cgcaatctca gctcaccgca acctccgcct 29220
   cccgggttca agcaattatc ctgcctcagc ctcctgagta gctgggacta tatgcgctca 29280
   ccaccaaacc cggctaagtt ttgtattttt agtagagaca gggtttcacc atattggtca 29340
   ggctggtctc gaactgctga cctcaggtga tccacccgcc tcggcctcac gaagtgctgg 29400
   gattatatgc atgagccact gcgcctggcc agaaaaagac attcttagaa acacgccagg 29460
   gcgacggtcc atgcagcaca cctgtgcaaa cacgcaatgt gcaggccccc cagaaacatg 29520
   actgaaggtg tagggacaaa ctcccagaca ggggctgtcc ctgtcatgca gacagactag 29580
   ccacggaggg acagaagcag acccccttac tgagtcataa tcttagaatg cacacacact 29640
   tgcagggaca cccgagggaa aacagacacc tcttcaggaa tccagataca gccagggaga 29700
   taggacaccc ctacgggaaa cactgtcaca gacagcaaac ccagagagac agctgtcccg 29760
   ctctgggctc gcctggcgcg tgaggatgca cataagtccc cggaggaaga cgtcacacag 29820
   acacgctggg ggcaaaacta atccaggaca gcaacggctg cacacggagg aatgtcatcc 29880
   ctcactcaca gaccctcaga cggaaaatca aagacatcct gggaggagct tcttcccagg 29940
   aaccctcccg aaactccccg aaactcctcc gaaattatcc agaaagtccg ttagggagat 30000
   tctcattcaa caaatatgtg tttcctgttt gctaggcact cttgctgagg ttgggaatac 30060
   aatggtgaat gagccacagt ccctgccctc agtagcacac ccaggggagg gtcagctcca 30120
   cccgcccaga cagacaccga actggaaata cacccctaac ctctacccca gatacacctg 30180
   acaagctgat cccctgccca cacacccagc aggtggctcc aaggtcagag cctgcagaac 30240
   acagacgcaa tagagaaaga ctcagcgctg tggaaagaca cccccaccta cccacgacag 30300
   tggccacagg gccaccccca gccaataaca ccattacaaa gacacgtgcc ttcagataat 30360
   gcctgcagaa acgtgcatgc tccaagcact ctagaaatcc cgtctcaagc agtgagtgcc 30420
   ccgacagcca cagccagagg agacacactc aaagctcctg gggtcactct cagagggtct 30480
   ctgagccacg aacccacatc caggcacaca cagagtcgtc acagagacac agtatggagt 30540
   ccacagcagc acgaacatag gatacgaacg ccgcaccaca ggacagatct atctttaggg 30600
   agactcagag aactttttat ttttaataga gacagggtct cgctatgttg gccaggctgg 30660
   tcttgaactc ctggcctcaa agcattcctc cagctttggc ctcccaaagt gctgggatta 30720
   caggtatgag ccagcacact aagcctgatt cagagctttg aaccctagac atatgaacag 30780
   actcagcttc gagttccaaa acaccaatgc cacctccaga aacagagaca agcctcccaa 30840
   gataggctca acctggtcca gttagtgacc agcacaggag aaaaatctgt ttcactcctc 30900
   aacccagtgt atagttagag acatatatac caccctaccc cctaccaaaa aagattttat 30960
   cctccttttt ttaatgctat ttgtgagtca ctaagctgat ttgacagccc gctgggtcat 31020
   gaccccctgt ttgaaaacaa cagaaacaga tgtccttcct gggtctcagc ccctcctgtg 31080
   tgtcgaggtg tagaggtggg gtcaggtgtg ggtgaacccc ccacccccgt ctgtcccagt 31140
   tatgccttgc atgcagggat gggggaagtg gatgtgggta ggttctggtt acattgactt 31200
   ctgtccctgg cttggggccc aggagcccca tccctccctg ctcacactgc cctcctcagc 31260
   acttcatggc ttcctctctc tctccgcctc cccactacct cccatccccc atccccacct 31320
   gctcagcccc tgcagggagc actaaggaag tcccactgtc ccggtctgtg agaaccccat 31380
   tttacacact ggggataagt ggcctagaga tgaggcagcc acggcacttg gaataaatgc 31440
   tcccagccac tcaagagggc agaattatca ggaatctgtg gccctctgca gagagctttg 31500
   ggatcagaca gcctcccact gaaacccagc tgtcctcccc cgagggggtg tggcccccaa 31560
   gctcatagct ttgtgaggac cccacagcac attcagggag ggctgcgaag tcagatggca 31620
   ccagggtcct ggccagtgac cgggcggcca gatctattgc atccaaatag ccagcttcca 31680
   ctcggctacg gcgatcgcca gacatgggag tttggtgttg ctagatctga tttgtccccc 31740
   taagagaaac ctggattttt aagtgaaatc gcctgatttt tgaaaggttg gctagcaatt 31800
   taaattttgc aaaacactaa cacctgggga ccaccggttg gcatccccct ccttgtacaa 31860
   agtggtgctc aggctgtagc gaagggcgga ggggcttctg cttccccacc cccctcctcc 31920
   tcctcctcca ttcttctgcc cagagtcacc ccctcattct ggtcactccc gctccctcca 31980
   tccccgccta tcctgcatct cccccacccc ctcactcccc agagcgatcc tcgtgatctt 32040
   gtgtctccct ttctctctct ctcacccctc cttctctctc tcctcagaaa cccccagggc 32100
   ctcgccccga gatgtgggcc cgctggtgtg gggggccgtg ggggggacac tgctggtgct 32160
   gctgcttctg gctggggggt ccttggcctt catcctgctg agggtgagga ggaggaggaa 32220
   gagccctgga ggagcaggag gaggagccag tggcgacggg ggattctacg atccgaaagc 32280
   tcaggtgttg ggaaatgggg accccgtctt ctggacacca gtagtccctg gtcccatgga 32340
   accagatggc aaggatgagg aggaggagga ggaggaagag aaggcagaga aaggcctcat 32400
   gttgcctcca cccccagcac tcgaggatga catggagtcc cagctggacg gctccctcat 32460
   ctcacggcgg gcagtttatg tgtgacctgg acacagacag agacagagcc aggcccggcc 32520
   ctcccgcccc cgacctgacc acgccggcct agggttccag actggttgga cttgttcgtc 32580
   tggacgacac tggagtggaa cactgcctcc cactttcttg ggacttggag ggaggtggaa 32640
   cagcacactg gacttctccc gtctctaggg ctgcatgggg agcccgggga gctgagtagt 32700
   ggggatccag agaggacccc cgcccccaga gacttggttt tggctccagc cttcccctgg 32760
   ccccgtgaca ctcaggagtt aataaatgcc ttggaggaaa acatcagggg tgtcttgtaa 32820
   tacatcagac cccctccttg catgttgggg aaagagaggg tgggacaggg actcctaggt 32880
   cccagggctg ggaagagctg atccacgccc ccggggaacg agagcctggc aggggttaga 32940
   ctggggaagg ctgtggggct cggaccactt ggatccttga aagacgcatt ggatggccga 33000
   gaaccgcaag gctcgtgcaa ctaggattca ggtcactgga ttggggactc agaggcctgg 33060
   acatttggac tcctgggtcc caggacagag gctgcctttc tggatggtgt ggccagaccc 33120
   atcagaggca ggagttctct ggactcctgg gccctcgggt ggaggtgggg gtggggacag 33180
   caggggctca gccaggttcc ctgcgtcctg agagaatgag gccagagggc tgtgatccag 33240
   tggagcctca gacctcaact cccccaggtt gctgggggtc tcgggagggg agggctgctc 33300
   cccactccca ttcttagggg ccctgagggc atggggcagg cggggggtgc tggggaagtc 33360
   ccacctctgg ccaccccgac tgtggctccc ctccttctcc atccttgccc ccagggatca 33420
   ccccagggga cttttcagtg gctccctggg agtggcggca gctccctcca gctcctctca 33480
   actggaaaag caaagacggg gggaggggcg gcccttggtc cttgaactcc caggctcagt 33540
   aagggacagg tttttacccg cgtcacctct gctctcccaa gcctccatgc ctcctctgta 33600
   acacggggca ggtgtttcca tcggcctcac aaggctgtcg tgggacccag taaggacatg 33660
   cccgtgatgc cctcatgcag cctcattgac ctccacagac cccaccaagc cctgtgccag 33720
   gcagtgctgg ggctgcagct gtggcctgca cagacccagt gccgtcctcc ggtgtgcacg 33780
   gccataatga ccacaagaat agcagcagta tgtttaggat gaaggtgtgt gtgtggaccg 33840
   aggggcagtc tctctctctc actcccccac cctctgaagc cctccagagg atgccaggct 33900
   gtggaacggg agcagtggag atccaacagt ccagcgtcct ctggaagctt cctcctcatc 33960
   tggagagtga ggccgatgca tgcgtgaccc ccacgggccc ttccatactt gttacttcct 34020
   ctggttgttt gttgtctgta ctcacccacc caccccaccc ggacaaccgg agggcagggg 34080
   cggggaattg ggtccgcctg gcacacagtg gatccttcag tgccatgctg agtcctcctt 34140
   acacacccag caaacaccta ctgtgtgccg aacctcaggc acacagtcta gtatggaaat 34200
   tggcaagtaa acgggtaacc actccaaaaa ctcttccatt aggaaagact tgagaaatgg 34260
   aaacgggagg tgctagcaag gaaggagagg cccaggagca aacaggcttc tcgcttctcg 34320
   accccttttc ctgtcttgcc tttttttttt tttttttttt tttttttttt gagacggtgt 34380
   ctagctctgt tacccaggct ggagtgcagt ggtacgatct cggctcactg caccctccac 34440
   ctcccaagtt caagcaattc tcctgtctca gcctcttgag taacgcaccc agcctttttt 34500
   tttttaagaa gtggggtggt ctccctatat tacccagatt gggctcaaac tcctgggctc 34560
   aagcgatcga tcctcccact tcagcctcca aaagtgctgg gatgacaggt gtgagtcact 34620
   gtgcctggcc cctggctaat tttttttttt tttttttaag acggagtttc actcttatcc 34680
   aggctggagt gcaatggcgc gatcttggct cacggcaacc tccgcttccc aggttcaagc 34740
   aattctcctg cctcagcctc ctgagtagct gggattacag gcgcgcgcta gcatgcccag 34800
   ctaacatttt gtatttttag tagagacagg gtttctccat gttggtcagg ctggtctcaa 34860
   actcccaacc tcaggtaatc cacctgcctc ggcctcccaa agtgctggga ttacaggcgt 34920
   gagccaacgc gcccggctag cccccggcta atttttaaaa ttttttctag agatgaggtc 34980
   ttgtgctcgt tgccctggct ggtcttgaac aatccgcccg cctcgccctc ccaaagcgct 35040
   gggattacag gcgtcagtca ctgccccaag ccctaacccc cttcctgata agtggaagga 35100
   gattctagtc cccagaggtg attctggatc ccagaattct gtccccgcat tctctgtctt 35160
   ctagccgccc ctggtcctgt ctcctctcct ctcatcttcc cactctgccc cgcctcattc 35220
   tgccccagct acacagtcta cttgctgttc tttaaaaaca atacaaggca ggccgagcac 35280
   ggcataatcc cagaacgttg ggaggccgag gcgggaggat cacttgagcc caggagttca 35340
   agaccagcct gggcaacatg gcaaaacccc atctctacaa aaaaattcaa aaaaaaaaaa 35400
   aaaaagctgg gcctggcggc acatgcctgt agttccagcc actccggagg gtgaggtggg 35460
   aggatcgctt gagcccagga gattggggct acagttggtg gtgatcatgc cactgcactc 35520
   tagcctgggt gacaaagcga gaccgtctca aaaaaaacca tagagcccca caagcctcct 35580
   gcagggccgc tggctgtttg cgctgcctgc gataccccca gacactctca tgactcatcc 35640
   ccggtgtctc tttcaggccc ttgctcagtt gtcaccttac agtaaggtag aggccctccc 35700
   actaccttcg gggaccccac tttgcacctc ctatcacttc cgcactgtat atttttgtat 35760
   cccccgtgac ttactcccat ctggcatgtg agctattgta ctggtttgct tactgttgtt 35820
   ctccctgccc aaccagaatc aaagttccat gaaggtagac actttgtatg ttttgttctc 35880
   cactcatctc ccatgcgggg cacacatcct cgctccttcc tcatgctcag cagtttgagt 35940
   tgaatgaaag aatgtcccct gcccatggga gaggacatag acaggcaggc gatgatgtgg 36000
   ggtgggatgg tcgcttggaa taaggagctg tggcttcctc ctggagaccc cctcacgctg 36060
   tccctctcct tgcagagacc cccaacacag caggcgcagg ggccacaggc ggcatcatcg 36120
   ggggcatcat cgccgccatc attgctactg ctgtggctgc cacgggcatc cttatctgcc 36180
   ggcagcagcg gaaggagcag acgctgcagg gggcagagga ggacgaagag taagtgatgg 36240
   gccctgagac ggggatggga gaggggtcga agaactgagg agtatggggt aggggtgagg 36300
   gtgggagaaa attctccata atggctgtgt gaaacagaca gacctaaagg aactggggtc 36360
   taaatggagt agggaggtat gggtttctct ctgctataaa gtatggcatt gaatttaaaa 36420
   attagctggg ggccaggcac agtggcttac acctgccatc ccagcacttt tggaggctga 36480
   ggtggggaga tcgatcgttt gagcccagga atttgagccc agtctgggta acataggaag 36540
   actaccccca tctacaaaaa aaaaaaaaaa aaaaaaaaaa tggccacgca gggtggctct 36600
   gaccgctccg ctctgcaaag actcctcgga gcccaggatc ctactacatt tttttttttt 36660
   ttttgtgacg gagtctcact ctgtcaccga ggctggaata cagtgatgca atctcggctc 36720
   actgcaagct ctgcctccca ggttaaagca attctcctgc ctcagcctcc ggagtagctg 36780
   ggaatacagg cgcccaccac catgcccagc taattttttg gatttttagt agagacaggg 36840
   tttcactgtg ttagccagga tggtctctat ctcctgacct tgggatcccc cctcctcaga 36900
   ctccaaaagt gctgggatta caggcgtgag ccaccatggc tggccagttg ttaataaaag 36960
   aagaaagggc ggttcctaaa ttgttatgga gaatttacat taaaataacg taagctattg 37020
   attggctatc cattgctctt tgtatcacaa attccagaaa cctgaagata aagagtaaca 37080
   ctggtcaggc agggtgggtc aggcagggtg gctcctgcct ctaatcccag cactttgttt 37140
   tgtttttgtt tttgtttaag atggagtctt ggtctgtcgc cctggctgga gtgccaaggc 37200
   gccatctcgg ctcactgcaa cctccgcctc cctggttcaa gcgattctcc agcctcagcc 37260
   tctcgagtag ctgggattac agatgcgcgc caccacgccc ggctaatttt tgtattttta 37320
   gtagagacgg agtttcacca tgttggccag gctactcttg aactcctgac ctcaagtgat 37380
   ccacccgcct cggcctccca aagtgctaag atcacaagca tgagccactg cgcccagcca 37440
   atgccagcac tttggaaggc caaggctctt gttgctagtg gttcgagacc agactgaaca 37500
   tcaaagtgag acaccatctc taaaaaaata aaacaaacaa aattagccag gcaaagtggt 37560
   gcacacctgt ggtccagctc ctcaggaggc tggggtggga ggtcacttga gcccaggagc 37620
   tcaagtctgc aatgagctat gatcatgcca ctgcactcta gcccaggtga cagagcaaga 37680
   ccctgtatct taaaaaataa aagactgggc acggtgcctc acacctgtaa tcccagcact 37740
   ttgggaagcc aacccaagta gataacttga gctcaggagt tcgagaccag cctgggcaac 37800
   atggcgaaac ctgtctacaa aaaaaataca aaaattaccc agacctggta gcccatgcct 37860
   gtagtcccag ctacttgggt gggctgaggt gggggtgggg gcatgtggag gctgcagtga 37920
   gctatgatca caccactgta ctccagtctg gccaacagag tgagaccttg taaaaaaaaa 37980
   aaaagaaaag aaaaatagct cagagcagtc tgaggtatgc gagctatgca aaatttgttt 38040
   ttttgagaca ggatctcact ctgtccctca ggctagagtt cagtggtata atctgagctc 38100
   actgcaacct tcgcctcctg agctcaacca atcttcctac cgcagcctcc caagtaaatg 38160
   ggaccacgta ccaccacacc tagctagttt ttgtattttt gtagagacag ggttttgcca 38220
   tgttgcccag gctggtcttg aactcctgac ctcaggtgat ccacccacct cagcctccca 38280
   aagtgctcgg attacaggcg tgagccacca tgcccagcct ggaatctgta tttaccacaa 38340
   gctctctaag tttttctgat gcacaggaag gtctgttaca ccactgtctt aatcatctag 38400
   ttagttcaaa ccaataactt gctgctgatc tcctctgtgt gcagctccat gctgtgaccc 38460
   actgtgtccg agacggcctg gagctcacaa tatggaggag gagacaaaac tgtctccaga 38520
   tagtgatggt gtagcatgac cagtgatggg atagggcagt tgagcaagct ctgagaagcc 38580
   agagggggca tctgacccag gctggatggt caggtagggc ttcctggaag aggggacatc 38640
   tgagctgaca gaatgcatcg taagcacctg tcattactgt atgctttggt ctgtgggtgt 38700
   gtagaatgag ggacaggaac tttaacctaa tttgagggac aggaaggcac ttcatttatt 38760
   cattcattct tttcacaaat atttactgaa ctcctcatag tggattctgg agacaccttg 38820
   ctgacgaccc agatctttct tttttttctt ttcttttctt ttcttttttt ttctttttga 38880
   ggtggagttt cgctcttttt tcccaggctg gagtgcaatg gcgcgatctc ggctcaccac 38940
   aacctccacc tcccgggttc aagcaattct cctgcctcag cctcctgagt agctgggatt 39000
   acaggcatgc gccaccacgc ccggctaatt ttgtattttt agtagggaca gggtttctcc 39060
   atgttggtca ggctggtctc acactcctga cctcaggtga tctgcccacc tcggcctccc 39120
   aaagtgctgg gattacaggc gtgagccacc acgcctggct gatctttctt tctttttttt 39180
   tttttttttt ttttaatttg agatggcatc tcactctatt gcacaggctg gagtggagtg 39240
   atctcatctc actgcaacct ctgcctcccg agttcaagcg attcttgtgc ctcagcctcc 39300
   tgagtagctg ggattacagg cgtgcaccat catgcccagc taatttttgt atttttgggg 39360
   gagatggcat ttcaccatgt tggccaggct ggtctcgaac tgctgacctc agatgatccg 39420
   cccgcctcag cctcccaaag tgctgggatt acaggcgtga gccactgccc ccagcccaac 39480
   acagatcttt tttccctccc agagctcaca gtggggcatg gtaattataa ataacatttc 39540
   aagtacaaat tcagtaagtg ccctgagggg gaagtacagc taggtatgtc cgtgtaacag 39600
   gaattcctga tccactgggg aaacctgaag gcttccggga aaaagaggaa ttcatggaat 39660
   gaataaaaac tcttactcca ggcaagaaaa gggggcaggg gaaagggaga cccaggtaga 39720
   gggaacagca tgtgccataa ccccggagtc agagggatgc ctgggtctta atctccactt 39780
   gtcctacctc ctaccccaca gcctggaggg acctccctcc tacaagccac cgaccccaaa 39840
   agcgaagctg gaggcacagg agatggtgag cactttccct ggagcccaag ctatccccac 39900
   ctccacaccc accccagggc tgggaggggc ctggcaggga gaagctggct gggtggtggg 39960
   ggcagttcct gacctccccg cccctctccc actacagccc tcccagctct tcactctggg 40020
   ggcctcggag cacagcccac tcaagacccc ctactttgat gctggcgcct catgcactga 40080
   gcaggtagga gctcatggga agacaaaggt gggttggggg tctgggttga agggccaggg 40140
   gtcaggcctg gcaaaagagg tcaaagacta aaggtcataa ctcaaggtca agggtgtgtc 40200
   cccggttggt gcttaatgca ttgagatgtg ttcaggattc catgggggag gtcaggggtg 40260
   aaaaggccca aggtcacaag gttagaattt aaagtcactt gggggctggg cacggtggct 40320
   cacgcctata atcccagcac tttgggaggc caaggcagga ggatcacttg gggtcaggag 40380
   ttcgagacca gcctgtgcaa catggtgaaa ccccgtctct actcaaaaca caaaaaatta 40440
   gttgggtgtg gtggtgcgag cctgtaatct tagctacccg gggcgctgag gaggaagaat 40500
   cgcctgaacc caggaggccg aggttacagt gagctaagat cacaccactg cactccagcc 40560
   tgggcaacag agcaagattc cgtctcaaaa aaataaataa aaccaaataa ataaaatcac 40620
   ctgggccggg catggtggct ctggtctata atcccagtac tttgggaggc caaggtagct 40680
   ggaccccttg agcctaggag ctcgagacca gcgtgggcaa catgggctac ccagcaacat 40740
   tttgcgtcta caaaaatact gaatttacct ggatgtggtg gcaaatgcct gtaggctgag 40800
   gtgggcggat tgcttaagcc caggtggttg gggctgtgcc actacactcc agcctgggca 40860
   tcagggtgag accctatctc aaaaaaaaaa aaaaagagga aaagtcatta ggtcaatgaa 40920
   aattgattat ctggtcaggc atggtggctc acacctgtaa tcccagcact ttgggaggct 40980
   gagacgggca gatcacctga cgtcaggagt tccagaccac cctggctaac aaggtgaaac 41040
   cccttctcta ctaaaaacaa aaataagcca ggcatgggtg acatgctcct gtaatcccag 41100
   ctattcagga ggctgaagca ggagaatcgc ttggacccag gaggcagaag ttgcaatgag 41160
   ctgagaccac gccactgcac tccagcctag gcaacagagc aagactccgt ctcaaaaaag 41220
   aaaaaggccg ggtgcggttg ctcacgcctg taatcccagc actttgggag gccaaggcag 41280
   gtggatcacc tgaggtcagg agttccaaga ccagcctggc caacatggtg aaaccctgtc 41340
   tctactaaaa attacaaaaa ttagctgggc gtggtggcgg gcgcctgtaa tcccagctac 41400
   ttgggaggct gaggcaggag aatcgcttgg acccgggagg cagaggttgc agtgagccca 41460
   gattgcacca ttgcacaaca gcctgggaga caagagcaaa actccgtctc aacaacaaaa 41520
   aaaaacagaa tgaaagaaaa ttgattatct acatttgctg gatactgtgc ctgaagctgg 41580
   gacaagacaa atcaagtcac tcccctcagt ggtctactgg aggtcagggg tcagacactg 41640
   aggttgcgct caaggttcaa aggactgttt agagcccaag gttaagtatt tgaggtcaag 41700
   actgaggtga aaggttcatg gggaaagatt tgcagagatc gagtgtcaaa agtgagtccc 41760
   aggattaaca gggtgagttg cacgagctgg gctttgggtt aaggaagggc tgaatgaatg 41820
   tcccagtgca atatggcctc aggggacaag gagtgaggtg gcatcttgtt ggcatgggga 41880
   gagagcggtc aggattttgg ggtcaagagc agattggtaa tctgtgtcgt gcgtaggaag 41940
   tgatcttgag ttcctgtcct ctctctacct ccaggaaatg cctcgatacc atgagctgcc 42000
   caccttggaa gaacggtcag gacccttgca ccctggagcc acaagcctgg ggtcccccat 42060
   cccggtgcct ccagggccac ctgctgtgga agacgtttcc ctggatctag aggatgagga 42120
   gggggaggag gaggaagagt atctggacaa gatcaacccc atctatgatg ctctgtccta 42180
   tagcagcccc tctgattcct accagggcaa aggctttgtc atgtcccggg ccatgtatgt 42240
   gtgagctgcc atgcgcctgg cgtctcacat ctcacctgtt gatcccttag ctttcttgcc 42300
   aaggatctag tgccccctga cctctggcca ggccactgtc agttaacaca tatgcattcc 42360
   atttgtgatg tctaccttgg tggctccact atgaccccta acccatgagc ccagagaaat 42420
   tcaccgtgat aatggaatcc tggcaacctt atctcatgag gcaggaggtg gggaaggtgc 42480
   ttctgcacaa cctctgatcc caaggactcc tctcccagac tgtgacctta gaccatacct 42540
   ctcacccccc aatgcctcga ctcccccaaa atcacaaaga agaccctaga cctataattt 42600
   gtcttcaggt agtaaattcc caataggtct gctggagtgg gcgctgaggg ctccctgctg 42660
   ctcagacctg agccctccag gcagcagggt cccacttacc ccctccccac cctgttcccc 42720
   aaaggtggga aagaggggat tccccagccc aaggcagggt tttcccagca ccctcctgta 42780
   agcagaagtc tcagggtcca gacccttccc tgagccccca cccccacccc aattcctgcc 42840
   taccaagcaa gcagccccag cctagggtca gacagggtga gcctcataca gactgtgcct 42900
   tgatggcccc agccttggga gaagaattta ctgttaacct ggaagactac tgaatcattt 42960
   tacccttgcc cagtggaata ggacctaaac atcccccttc cggggaaagt gggtcatctg 43020
   aattgggggt agcaattgat actgttttgt aaactacatt tcctacaaaa tatgaattta 43080
   tactttgacc agg 43093
<210> 32
   <211> 66938
   <212> DNA
   <213> Homo sapiens
<400> 32
   tgccaaggct ccagcccggc cgggctccga ggcgagaggc tgcatggagt ggccggcgcg 60
   gctctgcggg ctgtgggcgc tgctgctctg cgccggcggc gggggcgggg gcgggggcgc 120
   cgcgcctacg ggtgagtgcg accctcgggg cccgaggggc ggccggaggg ctgagggcgg 180
   tgagggtcac ggctgaaagg ccccggaggt caacgccggg cggagggccg aagctggggt 240
   gggtgctggg ggctgccctc gcgaggcccc gcgctcggcg gctgcggctt cccgcgcggg 300
   atctctgggg cccggtactg gggccggttc gaggtgggac ccgggagctg cggggttttc 360
   ccgaactccg ggtgcggcgg aggtggcggc agcgaagtaa gagggctgcg acttgtagtg 420
   ggaagagccc aggaggctgg cgtccaggcg tgcgggggcg gggggcccga aacatgccca 480
   cggcgtcaga gggccgtggg tcagcgcccg gaatgtcgcc tgtgagcccg ggaggaaatg 540
   aggcgccaga gccggcgggg accacgaccg aagtccaggc gagctgagaa cgcgccgggg 600
   ctctttgccc gaggccgcgc caatcctcac tgtctaaggg tggctcttgc gagtggaagt 660
   tgaagggaac ctctaaacat ctcagtcctt gttgaaaaag gacgtgacgc tcatgacctc 720
   ctctggcgtc gaagaaatcc tttgggaaat ccagaatagc atcccggcag atgctccccc 780
   caccccactc tctttaataa accttatatt ttagggtaat tgtagattta tggaaaagtt 840
   gcaaagatac ttcagagagt tgtgtatatc cctcaccctg tttggtaact tctagttaat 900
   caacctggca gttgtgaaaa attgggagtt aatgacttcc ttaggagcag ctcctggaag 960
   gaatggcctt ctggacgttt ttggtgacaa tgaaagcccc aaactgtata ggttttatta 1020
   tcacattgac acaaacactt atatatgctt attgcatttt taccaatgtg ctttgaaatc 1080
   caagttgaaa tgttgattag gaaaccttaa tgccctctat ggattcagtc aataaaatac 1140
   atgctgccag gcagtgttta ggggctgagg atgcagcagt gagcaaaaca gatgaggtgc 1200
   ctgccctcag acatatcggg gggtgggggt aaaatatatg atatgtctga gggtgttaag 1260
   tagagaaaaa cagagcaggt gggaaggggt ttgccattgt aaaaagggtg gatcgagaag 1320
   aaaacaaatg ttgctgaaag gtggcatttg agtagagacc tgagggagac ttgaagcttg 1380
   tcagtcaata attattcagg tgtctgcctt atttagtact gagtcaggcc ctctaggttg 1440
   acttagaacc tgagagtgta tagcaaaatt cttgccccaa aagggcctct agtttagttg 1500
   ggtacacctg atttccgtaa tcatatgggg acattgcagt ccagagaagg gaaggaactt 1560
   gtctaaagtt acccgtctca ttagtctcct gattttcaca gtgccactcc agcaccctcc 1620
   ctcctacaga ctcactccca cacacacctg aaacaacagc tggccatcta gagaatactg 1680
   gcaactccca acttcatcag tcctgtcaca atggtttgtc gttagcttgc ttatttagaa 1740
   aacaaacttc tttccccatt aaaagtattg atagaattgc attgaggtat ggtgggaaaa 1800
   aattagatgt ttccagtgag acttgagttt tagtggttct gatatttacc acctgtggga 1860
   ctctgggctg gtcatttcct ctcttttgtg ctgcagttag ttttctcatc tgtgaaatga 1920
   ggaggttgaa ctgcataaat tctaagagct cctccagctc taatcctcag agtgtttata 1980
   aaagtgatta gattactagc ttagtttaac ctgtcccact caatggccca cattgtggca 2040
   tgtcttaatt ataactagca tgtatatagc atttatgccc agcagttttc tagctgcttt 2100
   acttgtacta tttcatttaa tctttaccaa taatataact tgtaggtgct gttattagtc 2160
   ccattttgta cacgaaaaag ttgaggcaca gtgcgaccaa gacccttgcc caaattcgca 2220
   tacctggcaa agtggaagag ctaggtttcc aaccctggca gcctgattcc agaggccatg 2280
   cccttaacca ctgtactcat tgcttctgcc cagcagaaaa gacgagagtc cttagctgga 2340
   cacctcccag ggtagcaggg caggacggct tgggacccac gaaagtatgt gcaagccatt 2400
   tgttaagaag ctaatgatac tatattaggc cgggtgcagt ggctcatgcc tgtaatccca 2460
   gcactttagg agactgaggc agatggatca cttgaggcca ggagtttgag cctggccaac 2520
   atggcaaaac cccgtctact aaaaatacaa aaattagccg agtgtgggat tacatgcctg 2580
   taatcccagc tactcaggaa gctgaggcat gagactcatt tgatcctggg aggcagaggt 2640
   tgcagtgagg gaagatcagg ccactgcact tcagcctggg cgacagagca agactgtctc 2700
   aaaacaaaaa aagaagcgaa tgatactaca ttggagactg gctttatagt ggctgggctg 2760
   tgtggtccaa acaactagta ctataggcat tcaaaagaga aaaagctgac ggttggtagt 2820
   gttatggaag aggtaaaact tgagctagat cttctggatg actgggattt gagtaggtgg 2880
   agaatttatt ccaggtgggg gcaatagcag gagcagagac ccagaaatga gggcaagagt 2940
   gctcctttgt ggtcaagaag gaacctggct tggcaggaga gtgcattggt aaagtcaatc 3000
   caggcagaat agcttgattt gcccttgaaa gctctgagtc cggcacagca tgggaaaaat 3060
   aacacagaag gaggaagcaa gcagggagac ttcactgggt aggggagccc ctggagtgtg 3120
   aagctacata agaatctatg gttgcaggtc tccactagaa aaatgtgctg cgaacccttc 3180
   ctgtccagga ccatgactta ctggtatctt cccacctggt ggggcacaac agagcatatt 3240
   tgcatgattg gggtaactaa gaaagatgtt ggaagaaaaa aacgaacagg agttgatgcc 3300
   tgagtggatg taagggaagg gacagatgac tcatatatga ctcttgtgat ttccttaact 3360
   tttcccaaaa tgtgcatgcc tctgctagct gctgtgattg gacaggcgag agcaaaagct 3420
   ataagctaag caaaatgcct tcttatgaaa tagtagattg tgttaactct taatttaaat 3480
   aggatgttgt gctagatgac tgtacagact tgggatttga ggtttagata cagacatact 3540
   tgggtccctt cctactccct tttctgaagt cagtggtgca atccccaaga catggttgaa 3600
   attaggtgcc taagggaagc ttatcaggta gaagggatac ttgttctgtc tcaaaggcca 3660
   gggacacata tcaattcaac agaaaaccaa agcaaccctg gccaggcaca gtggttcaca 3720
   cctgtaatcc cagcactttg ggaggccgag gtgggtggat cacttgaggt caggagttcg 3780
   agaccagcct ggccaacatg gcgaaaccca gtctctacta aaaatacaaa aattagccag 3840
   gcgtggcagt gcacacctgt aatcccaact actcggaagg ctgaggcagg agaatcgctt 3900
   gaacccagga ggcagagttt gcagtgagct aagatcacac cagcgcactc cagcctgggt 3960
   aacagagcga gactccgtct ccaaaaaaaa aaaaaaaaag caactcttta gaattgtttt 4020
   gtcttaaaga aataaagcag atagctaaca catagcactt gctgcctgtg ggcactattc 4080
   taagcacatt gcttgtatta actcatttaa tcctcataat aaccctttgc aagaggttac 4140
   tattattatc ctcatattag agatgagaaa actgagaaat agagaggtta agtaatttgc 4200
   ctgctggcaa gtagtagagg tgggatataa actcaggcag tcaggctcca gagtctgtgc 4260
   tcttaaacac tacactatgt tgaagttata cacgaagagc tgttagagac ctttacaagt 4320
   agtataatca tcacttaatc atatatatgt ctaggcatgt tgtatacaca cacagttcac 4380
   atgtatttgt atatatttaa acatttggag attaaagttt cagtgcctgt gtttttttca 4440
   attttttatt ataaaataca cataaaattt accatcttaa tcatttttaa gtacacagtt 4500
   cagtgttacg aaatacattc ataatgctgt gcaaccatca tcaccatcca tccccataac 4560
   tctttttatc ttgtaaaact gaaactctgt acccattaaa caataactcc tcattttccc 4620
   ctgcccctag cccctggcaa acaccattcc actttctatc tctatgattt tgactactct 4680
   aagtatctca tatgaatgta atgatatact atttgtcttt tttgtgactg gcttatttct 4740
   gttagcatag tgcccttatg gttcatggtg tatcatatgt cagaattttt ccttttttaa 4800
   atttatatat atatatatat ttattatacc ttaagttcta gggtacattg cacaacgtgc 4860
   aggtttgtta catatgtata catgtgccat gttggtgtgc tgcacccatt aactcgtcat 4920
   ttacattagg tatatctcct aatgctatcc ctcccccatc cccccacccc acaacaggcc 4980
   ccggtgtgtg atgttcccct tcctctgtcc aagtgttctc aatgttcaat tcccacctat 5040
   gagtgagaac atgcagtgtt tggttttttt gtccttgcga tagtttgctg aaaatgatgg 5100
   tttccagttt catccatgtc cctacaaagg agatgaactc atcctttttt atgactgcat 5160
   agtattccat ggtgtatatg tgccacattt tcttaatcca gtctatcatt gttggacatt 5220
   tgggttggtt ccaagtcttt gctattgtga gtagtgctgc aataaacata cgtgtgcatg 5280
   tgtctttata gcagcatgat ttatactatt tgtctttttt gtgactgact tatttctgtt 5340
   agcataatgc cctcatggtt catgttgtat catatgtcag aatttttcct ttttaaaagc 5400
   tgactaatat tccattgtat gcatatacca tattttgctt atctgtttat ctgtcaatgg 5460
   acacttaggt tgcttccatg ttttagcaat ttcacaatac tattatgaac ctgggtgtag 5520
   aatatctctt tgagaccttg ttttcacttc ttttgggtat atacccagaa atggaattat 5580
   tgtatcatat gataattcta tttgtaattt ttttgaggaa ccaccatact gttgtccata 5640
   gtgaaatttt aaatttccac caactgtgca ccggggtttg aatttttcca tatcattgcc 5700
   aacatttatt ttctgttttt tgatagtagc cattctaatg ggagtgaggt gaaatctcat 5760
   tgtggttttg gtttgcattt tcctaaatat tagtgatgat gaacaccttc atgtgcttat 5820
   tggctatttc cttatctttg ttggtgaaat atctatttaa gcctttgcct atttctgaat 5880
   tgaatttttt tttattgttg agttttaggc attctctgta tattctggat attaatccct 5940
   tatcagatat atgatttaca catatttttc cattctgtga gtcacttttt tctgtgaata 6000
   ttttgatgca cacgttttaa ttttttccat gaaatccaat tcgtcaattt tttcttttgt 6060
   tacctacatt gttggtgtca tatccaagaa attgttgctg aatccaatgg tatgaagctt 6120
   ttgtcttatg ttttcttcta aaaattttat agttttagtc ttaaaatttc agtctctgat 6180
   ccattttgaa tttttttttt acttggtatt gggtaagcgt ccaatttcat tctttttcat 6240
   atggatatcc agttttccca gcaccatttg ttgaaaagat tgtctgcatt gagtggtctt 6300
   ggcactgttg tcaaaatcat ttgaccatac atgtgatggt ttatttctgg actgtctatt 6360
   ctattacatt gatcagtatg tctgtcttta tgccagtaca cactgttttg attattgcag 6420
   ctttgtagta agttcagaaa tcaggaagtg tgagtcctac agctttgttt ttccgtttca 6480
   agactgtttg gctattcagg gttccttgag atttcataag aattttagga tggatttttc 6540
   tatttctgca aaactatgcc attaagattt tgataaggat tgaattgaat ctatatatca 6600
   ctttggatag tattgacatc ttaacaatat taagtgttct aatacgtgaa catgggatgt 6660
   gtttccatat catctacaaa cagagataat tttacatctt attttaactg gatgtctttt 6720
   atctcttttt cttgcctaat tgctctgggt agaacttcaa gtactatgtt gaataggagt 6780
   ggtgaaagtg ggcatctttg tttcattcct gaccctaaag gaaaagcttt cagtctttca 6840
   ccattaaggg tgatgtttac tgtgggtttt tcatatataa cttttaagtc aattttctta 6900
   cactcctagt ttgttgagtg tttttatcat gaaaagtaaa ttttgtctaa tgctttttct 6960
   gtatcaattg agatgatcag gtagtttttt cccctttatt ctgttaatgt ggtatattac 7020
   attgatcaag ttttgtatgt taaaacatct ttgtattcca ggaataaatc ccacttggtc 7080
   atggtctata atccttttga tatgcccgtg tattcacttt actgctattt tgttgagaat 7140
   ttgtacatca atgttcataa ggcatatggg tctgtagttt tcttttcttg tagcgtcttt 7200
   gtctggcttt ggtatcaggt aatgctggcc tcgtagaatg agttacgaag tttgagaagg 7260
   tttggtacta gttctttaga tgtttggtag aattttatct gtgaagctat caggtctagg 7320
   gcttttcttt gttgagagat ttttgattac tgattcaatc tccttactac ttataggtct 7380
   gtttagattt cctatttctt tgtgatttag ttttggtggt tttgtgtttc tggaaatgtg 7440
   tccatttcat ctaggctgtc caatttgtta gtgtacggtt gttcatagta ctctcttata 7500
   acccttttta tttctctaga atcagtagtg attctagaga aatactcatt ttcatttttg 7560
   aaaatgagta aagttctcat tttcattttt gattttagta atttcagtct tctttcttat 7620
   tagtccatct agctaaaggt ttgtccgttt tgttgatctt ttcaagaagc ttttagtttc 7680
   attgattttc tctatttttc tattcccttt ttcttttatc tctggtctaa tcttcattat 7740
   ttccttcctt atgatagctt aagattagtt tgttcttttt ctagttacgc aagttgtaaa 7800
   gttaaggttg ctgatttgag atctttcttg tttcttaata tgtgtttata gctataaatt 7860
   tttccaatag tactgctttc gctgcatcgt ctacatttta gatgtttata tttacgcctt 7920
   tcatcaaatt tgggaagttg tcatccatta tttctttatt ttcttttttt gagacagagt 7980
   ctcactctgt cacccaggct ggagtgcagt agcacgatct cagctcactg caacctctgc 8040
   ctcccaggtt caagcgatgc tcctgcctca gccacccaag tagctgggat tacagatgtg 8100
   caccaccaca ctcagctaat ttttgtattt ttagtagaac tggggtattg ccatgttggc 8160
   taggcttgtt tcgaactcct ggacccaggc aatctgcctg ccttggcctc ccaaagtgct 8220
   gggatgagcc accacacccg gctcattatt ttttcaagta ttctttcttc ctctttctct 8280
   ctcttctcct ggaaccccca taatgcatac actggtctgc tgatggtgta ccacaggtct 8340
   cttagactct gttcactttt cttcaatctt tttttctttc tgttcctcgg actcaataat 8400
   ttttattgcc ctaatttcaa gtttattgat tctttcttct gcctgctcaa atctaccttt 8460
   gattcttttt agtgaatttt tcatttcagt tgtgcttttt ctgctccaga gtttcttttt 8520
   ggtctctttt taggttttct atctctttat tggtattttc attttgctta catatcattt 8580
   tcttgactct ctccacatct tcctttagtt ctgtgaacat ctgaagatat ttgtttcaga 8640
   atctttcttt aatatatttg ccatcaggtc tttttcaggg acaggtttgc tgttgtttaa 8700
   ctttttttct ttgggccata gtttcctggt ttttttgttt tgttttgttt tgtctgtctt 8760
   gtgattcttt gttagaaact ggacatttaa atctggtttc tctggaaacc agattccccc 8820
   ttcccccagg atttgctgtt ttttgttatt gttttgttta ttgtctttat ttttttgatt 8880
   attgtaggct gtctgtgtgt tgagcatcaa cctgaagtgt aaacttagtc ttctcaggtt 8940
   tttttctggg ctttccctga gcatgtgtgg tcactttctg tttttctctg tatatatagt 9000
   tttgtttcat tttgttttgt tttgtttttg agacctagtc tcgctctgtt gcccaggcta 9060
   gagtgcagtg gtgtgatcac agctcactgt aacctcgaat ccctgggctc aagtgatcct 9120
   cccacttcag tctcctgagg agctggaact acagattcat gccaccacac ctagctaatt 9180
   taaccaaaca aacaaaaaaa attgttttgg tggagatggg atctcgcagt gttgccaggg 9240
   ctgatcttta actgctggcc tcaagtaatc ctcctgcctc ggcctaccga agtgctggga 9300
   ttataggtgt gagtcaccat gcccagcctc tgtagttgtt taagaatttc taatctttaa 9360
   tgcctggctc ccaaaagtgg aaaaggagaa aaatgaggcg acaagggaag agcgccattc 9420
   ttttacatct tctggaaatc actttagcta gatagggagg gcttgcaaca gtggggatag 9480
   gtgcaaccac aatggctgcc agcctctttg tctgtacctc tgtgatcaga agcagcaatc 9540
   agtaatcaga atacaaatcc ctgatatttg gaggacagag tacttctgcc cacatagccc 9600
   acaagctgtg ggcagagggc tccaggaaca tgtgcacagc cgcctgccat ggggttgggg 9660
   atgaaggatg ggcagctact actatgtaaa agctgacatt gattgaaatt tatggcaatt 9720
   tgttgttcaa gcctttcctg ggaaattgca agccttcaat gtaagtgttt cctgatataa 9780
   ctctgcagtc ttccaaaatt gttacatcaa acagactctg ccactaccat tgttatctag 9840
   gtgaggagat agatgtctgg tgcttcctac tcttgcctta ttcccagaat ccatgtgtgt 9900
   gtgtttttaa agtgtaggaa gtagaaaaat caaaagatat ggaggaaacc agaagagaaa 9960
   aagaaaagga tagcagatag ccccaggaag cccaaaatgc aaaccagtta gaagatctga 10020
   ccaaaggaca cttcaaaaca gatgggagaa tattaatccc atttgaaagg ttgaatatag 10080
   ggcaggccat tgttttattt caaaactgag gttaaagtag gttttaaatt accttgaaaa 10140
   taaattggag tacaattgaa atgaaccaca aataggtata tttttattat cagggcttta 10200
   atatttgtgt gggagtggga ggtaggtggg tagggagagt actggaagtg ctaaactagg 10260
   tttcttggat gtgtttctgg gaactgcaga ataaggcaga taatcctaca gtaaattgta 10320
   gtctccagta ggaacaatgt ttttattgga ggctctctac ctgacaaatg atttgtcttc 10380
   aaaaatatca ttgatatggt gttagaaagg ccaaaatgga atagccatac atgcttttaa 10440
   tcacttaaaa tcctaacttt agcactgttg cctgagccat tggtgctccc tggtactagc 10500
   tcagttttaa tgcagtgcaa taggaattct ttgtttccac aaggccttgt cctgctgtgg 10560
   gctgctcctt cccacagttg gaccttggga aaaggtagag ttagaagagg gaatgatttt 10620
   ttttatttca aattttatta tagattaaag ggtatatgca cagatttgct acatgggtaa 10680
   actgcatgac gctgagactt ggggtccaac aatctgatca cccaggctgt aaccatggta 10740
   cccaacaggt ggttcttcgg cccccaaacc cacctccctc cctcccttgt ctagtgatcc 10800
   ccagtgtcta tcatttccat ctttatgatc atgtgtattc agtgtttaac acccacttat 10860
   gagtgagaac atgcggtatt tggttttcta ttcttgcatt agatcactta ggataaaggc 10920
   ctccagctcc acccatgttg ctgcaaaggg catggtttca ttctttttat ggctgcgtag 10980
   tattccatgg tatatatgtg ccacattatc tttatccagt ccactgttgg tgggcactta 11040
   ggttagttcc gtgtccttgc ttttgtgaat agtgctgcag tgaacataca ggtgcatgtg 11100
   tctttatgat agaatgagtt attttccttt gggtatatat caagtaatgg aattgttggg 11160
   ttgaatggta gttctatttt aagtcctttt tttttttttt tttttttttt gagacatagt 11220
   cttgctctgt tgcccaggct ggggtgcagt ggtgcaatct tggctcactg caacctccac 11280
   ctcctgggtt caagtgattc ttatgccaca gcctcctgag tttctggtat tacagttgta 11340
   caccaccatg cctggctaat ttttgtattt ttagtagaga cggtgtttcg ttatgttgtc 11400
   caggctggtc ccgaactcat gatctcaagt gatccaaccg cctcagcctc ccaaagtgct 11460
   gggattatag gcgtgaacca ctgtgcctgg cctattttaa gttctttgag aaatctctaa 11520
   agtgctttcc acggtggtgg aaccagtttg catttccacc aacagggtat aagcattccc 11580
   tattttccac agcctcatca acatctgttt tctgactttt aaataatcac cagggaatga 11640
   atgttgaggg tcaagggcct ttgtagactg ctgaccaagt agctgctatg tataaaattg 11700
   actgttagag aaacctcaac tacagagtgt ctgtgagttt gatgttttga tgttttggga 11760
   caaatggaat tttttgctca gttagggagg aaggtaacag tataaatatt gctgattatg 11820
   taacaggccc agtgcactcc aaagtattag catagagtac aagatttaac tctatcattt 11880
   ggtaaattac tattactata atccttaagt aattaataat ggttccgttc atcactttag 11940
   aaaatttggg ggaattattt tcaaataatt ttaataaact tctatgttga aataattttt 12000
   gatttacaga aaagttgcaa aggtgggaca gagggttcct gtatacccat cacccagttt 12060
   ctgggggatg attttgatag tctcaggaga acttctcagg aaagtttaaa atgatgtttc 12120
   acagttcctc ctctttctct tccctcttca gattagtcaa agctctagct ccagctgttt 12180
   agtgtcagac ctcaggggaa caagggctgc taagtctctt tctcagtact tctttggaaa 12240
   agctataaaa aaacaattac caggtcatgt tagcaagcag aattaaagat gagcttctta 12300
   aacttactta tagaactgct gagattttga acaatctgat tttttgttgt tgtcattgtt 12360
   tttgagacag ggtcttgctc cgtcacccag gctagagtgc agtggcatga tcttggctca 12420
   ctgcagcggc atgatcttgg ctcactgcag cctcaaactc ccgggctcaa gtcatccttt 12480
   tgcttcagcc ccccagtacc tgggactata ggcatgtacc agcatgtttg gctaactttt 12540
   gtaacttttt tgtatagatg ggattttgct atgttgccgg ggctggtctc aaactcctgg 12600
   gctcaagccg cttggtctcc caaagtgcca ggattacagg tgtgagccgc cgtgcccagt 12660
   ctgaagagtc tgtatggagt gaggtgtgga tcccaaggtt agggaacccc aaattcctgg 12720
   aagagaacaa cttggaaata ttagtattta agaatatcac tcttctctat ggaagtggtt 12780
   aagaatgtga ccttggagcc acacttttca agtgacactc ccagctctgc cgtttactgt 12840
   agccatggga tatggaacaa attacttcac ctgcttgttg cttcagttgc ctcatcttta 12900
   acatgaggcc aataggccag gtgtggtggc tttatacctg taatcccaga actttgggag 12960
   gccaaggtgg gcggatcact tgagctcagg agttcgagac cagcctggcc agtgtggcaa 13020
   aaacccatct ctactaaaat tacaaaaatt agccaggcgt ggtggcatgt gcctgtagtc 13080
   ccagtaacag gctaggtgaa tcacttcacc ccaggaggca gaggctgcag tgagctgaga 13140
   tcgtgccact gcactccagc ctgggttaca aagtgagatt ctatctcaaa aaacaacaac 13200
   aacaaaaaca tgaggctaat actagtccct atctccaaga ttgttgtgag gattaaatgg 13260
   attaatatgt gtggaatatt tagaatagtc cctggcccag taaatgttag atgttgttaa 13320
   ctcagttatt actgtcttgt cctctaggac ttgggagaat taagcaaacc attaaaaata 13380
   ctgttgataa attcattttt ttttgtcctt gatttgaaca gaaactcagc cacctgtgac 13440
   aaatttgagt gtctctgttg aaaacctctg cacagtaata tggacatgga atccacccga 13500
   gggagccagc tcaaattgta gtctatggta ttttagtcat tttggcgaca aacaagataa 13560
   ggtaagtttt ctgcaacatg ttacattgat gaggtaaagt gaacactatg aattggagcc 13620
   aagaataagc caaattctag tcttacgtca aagtggaaat tgtattttag ggtgtgttta 13680
   aatttcagtt ggggaaatta tagaagcaac tattgacaca agtgaacact taaaaatatt 13740
   tgctaattat ttgacaatta aatgcaattg agattgctct gacaggaaat actagatatc 13800
   tttttctcta aggtactcaa taaataatta tgttattgtt aaaatttgtt cctatgttcc 13860
   tgcctgtagc tgtattgcct tgtatctata taacacttaa ttttggggaa tttttacatt 13920
   taccatcttg ctaatcacat ggacaaatag atgtacaggc actatgtggt gtaaaagtaa 13980
   acggccatat ggaaagtgaa aaagtaagtt gtcagtggca ggtgtgtttg agtagagggt 14040
   tctgtaatgc ccgcggaaaa aggaggaaga ctttcaagat ttctagtcaa ctgatataag 14100
   tgaatttttg ttaataggtt tggataattt caggtccttt atgataatct tattcaactt 14160
   ttttttcaaa atctttttat accttctttc ggtgtgtatg tgtttgttgc agggtggcgg 14220
   cggtgagtgg gttgggtgtt tggggttgaa tttgtccagg attttgagta ggcagaatga 14280
   gaagttatta gcaacagtgc tttgcatcag tgaattgtag aagcaccaag ttcaggcagc 14340
   tttggtggca ggaagtcagg agaactagct tctgattggc actgtcactc ttagctgact 14400
   ctatggcctg gaataaattg cttaacctct ctgagcgttt gtttccttat gtataaagta 14460
   tggctttact cacttcgctg gcttgctggg acacagaaaa taatgtatat atacaaaagt 14520
   ttaatagtca ggatggaaca tgctcttcct ctggagggaa atttgctaat ctgccttcct 14580
   ctcccttgtc ctgctgaggt gttacttatg gtttaggcgt taattgataa agaattgagg 14640
   cttatcagaa gtatttgcat tttaaaagag gtcttctaaa tgtggaagcc aatgatacta 14700
   ctagcagtga gactggagtt tgcctttaca tactgtcggc ttcctcttgt tcctcctaag 14760
   gaagggcctt aggcatttgg agtcttagtg gtgatttttg gtaattgagc cttgagaaat 14820
   tcacagaaag accctaagtg cctctggctg taagccctgt aatcacccaa gtgtctaact 14880
   gaaaccctga gcagaggttg cttttcaggt cccttttcct taagttgtca aggatcaatc 14940
   aggtagccag gctggtgttc tagcctagaa atggggctat ttcctcagag tatgtgcaga 15000
   aatctagagt taaatgtaac tttaggagac agtagaatca gatagaattg tgcttgaaat 15060
   tttatctctt cccgtttcaa gctgtttgat tttgggcaaa tcacttaacc tctctgagct 15120
   cccattttct tgtttataaa atatggggtt gttttggcgg ggggcgggta ataatagtac 15180
   caactgaata tggatggtgt gtgcattaaa ggagatgagg tttgtaatgc acctgacaca 15240
   gagttagtgc taggtaattg ttagctttta ttattgttac tgttgttagg ttgagccatg 15300
   tgaaattacc acttttatgg gtcaaaatgg ttgaatgttg tcagtattat ttgtcagcct 15360
   aatattatca tttaatcttg tttcctccat tttacccctg aagaaaccag atagagacca 15420
   aaaagggtga cctctgccta aggccagctc attcattgta gccatttgac agcattgatg 15480
   gccatcagtc ctacatttgt gatgaacacg gattattcct tccagtcagt gataggagat 15540
   atcctgtctg gacctttttc aagtttatca tcaggaggct gaaaatgaat ggcagaagta 15600
   attttcctct cctctttctg tcctttgggt acagcattag catcattggg ccaaactgag 15660
   gtatccctga agcatattga agcaatggca ttatgatagt ttgtacatta gggggcttag 15720
   gaaaatgttc aaaggtccca agttgctaaa tgagataaac ccttgaggtc attttcaatc 15780
   ttaagattgg aggctcatca agttgagaga cagggatttg cagaggattt ttggaaaaga 15840
   aaagattagt gtaaatggca gccctccacc attttttagg cagtgtctaa tagttcctca 15900
   gctggcaagt agaaggagga tctagcatca gattttagat atctttcatc actaagagat 15960
   gggcaagtcc cattaggcaa actaatcaaa aggccaggca tccaaatagg aagatgctga 16020
   agctaactcc ctttcttttt gagaaaggaa ttctggagtt gagaaaggtg cagtccagcc 16080
   aagtgaacat tggtctgggg agatcagttc aggactcttg ccttggtttt tgataaattt 16140
   tcaaagggaa tcagattccc tgcagggtgg agcataacat catacagcag ctaccagtgg 16200
   gaatgattca ccaaagccaa aagtcacatg ggagccatca cacatacctc atcaagatct 16260
   gatgggaggg ttgaacgtgg tgactcatgc ctgtaatccc agcactttgg gaggccgagg 16320
   cgggctgatc acctgaggtc aggagttcaa gaccagcctt gccaacatgg tgaaaccctg 16380
   tctctactaa aaatacaaaa gttagccagg cgtggtggtg tgcacctgta atcccaacta 16440
   cttgggaggc tgaggcagga gaatcgcttg aacccaggag gcagtggttg cagtgagccg 16500
   agatcatgcc actgcactcc agcctgggca acagagcaag actgtttaaa aaaaaaatct 16560
   gatggaagga atttaggaat ttagtttctt gtgtgataca gagaggtgta cttgacttgg 16620
   cagttgttga taatctgggt tcctacttta ttagtgccag tttaatattg tttaccttga 16680
   actttgaaag aaattaaaaa aaaattattt gaaaaaattt tccctctact gtcccttttc 16740
   tatgttatta ttcatatgcc tgaagcataa ggttcggatt tagatactaa ggcatttttt 16800
   aattttttat gttattttat tttgaaatgg agtctgctct gtcacccagg ctggagtgta 16860
   gtggtacgat ctcagctcac tgaaacctct gcctcctaag ttcaagcaat tcttgtgcct 16920
   tagcctgcca aatagctgga actacaggtg catgccacca cgaccagcta attttttttg 16980
   tatttttagt ggagacagtg ttttgccatg ttggccaggc tggtttccaa ctcctgacct 17040
   taggtgatcc tgacctcagg tgaccttggc ctcccaaagt gctgggatta caggtgtgac 17100
   ccaccacgcc tggccactaa ggcatttttt aaattaaaaa ttccattgtg ataatttttg 17160
   tgtactaaaa ctccgtaacc ctctaactgg aacaatgtcc attataatat gaaaactggg 17220
   attatcttca tgatgattct aatttgactg tatttgactg tacagtctaa tttgacattt 17280
   gactgtacat tttcccctga tgtcttcaaa gggcaaatat agcttccaaa caagaattgc 17340
   catggatcct tggataaggg tggcttcttc aaagatatat gggacttgtg cttagtaaaa 17400
   ttgtgaggtt ggtaaaattt gtattcgaag ccttcctgcc accactacca tgatcctgga 17460
   ggcaggtctt cagcttcaca tactcagaaa cttcatttcc tcagcacaca tgcatctctc 17520
   aggggctgat tttttacttt gttgtgaaaa tggaatggta atggtgaagc attctaggaa 17580
   gatttctgtt agcataactg gtgcatttca tccctgtcgg cagagcctat atgggaagcc 17640
   aaatgttaag taggtttctt ggtgaggaat cagaaattag ttgtaaaaat atacttgtct 17700
   aagaatagct ggaggcccta ctgagagtaa acagctttgc cagtgggaca gaaaaattca 17760
   atgcccacat tcctgtatca ccctcttttt cctgcctgtc aggattttgc tcacagacat 17820
   ttctttttct gttcttccag ggctgaccaa ctcccttttc agagtactgt cttgactgga 17880
   cctaagagct attctggttc tataaagtga ttttgttttt gtgtccttgc tatgatttat 17940
   tcattgttga cttggtagtt acttctcaga ggccatccat atgtcctggt tttgggggta 18000
   tctcacagtt gccctcatgc tcagctggca gcaggcatgt gacatggcag aatagctctt 18060
   tagggtagca gatagaacta actgtggcaa attttcctct tcatggccgt caacatgtag 18120
   atgaaaggaa atactatctc tgacttgagt cagaagctat tgcaaaacag gaggtgctct 18180
   ccttggagct ctgattaaaa gcagctccat tcttaattat tccaactctg tcagtttggg 18240
   aaatgttaat ttcccggaga gcccattaat cttattattc tcagtcatag taaccagact 18300
   ggtgatttat gtctctatga tgtctataaa ttggcttcct atggttccac aaagcttatc 18360
   tacttttcag aatgagagtg tatatactca gcacatatag caatgtttga aatttataca 18420
   tatatatgag gtagtcaaaa tagtcaaaca gtagagaaag aggtttaatg aaaagtctct 18480
   cttcagccct caggccctcc tcctttactt ccttacccag aagtaaccat gattactgga 18540
   tttttttttt gagacagggt ctcactgcat tgtccaggct agagtgcaat cttggctcac 18600
   tgcagcctcc cgctcccagg ctcaagtgat cctcccacct cagcctcatg agtagctggg 18660
   actataggca tgagccacca tacctggcca attttttttt gtacagatgg ggttttgcca 18720
   ttttgcccag gctggtctcg aactcctggg cttaagtgat ctgcctgcct cagcctccca 18780
   aagtgctggg attataggca tgagccactg agcctggcat gattcctgga ttcttaaagc 18840
   atcccttttt aaccttaatt ttcctggtac agtgcatcta cttctaagtg aattttgata 18900
   atcatattgt ttcctttaaa attgctttta gagagttgat atgttagcat tgtaggttta 18960
   taggtaaatt tcatgtttat acctttaaaa tgtgggaaat tgttgtatct acaccagggt 19020
   ttctcaatgg tgacactagt gacatttgct gaagggtttg ttgtaggagg aggggggctg 19080
   ttgtgtgcat tgtaggatgt ttagcagcgt ccttgacctc tacttactag atgctggtaa 19140
   cctccccctc ccaagttagg acaataaaaa gtgttcgata ttaccaaatg tctctgagag 19200
   cggtagggag aacaaaatca gccccaattg agaatcactg atctatacca tttgataact 19260
   tttttgataa aaaaagcacc acatttagac atgctaatgt ttcattatat aagtgtgtgt 19320
   tagctgaagc aatgcctttt tcaattttct aaccttagaa aatagctccg gaaactcgtc 19380
   gttcaataga agtacccctg aatgagagga tttgtctgca agtggggtcc cagtgtagca 19440
   ccaatgagag tgagaagcct agcattttgg ttgaaaaatg catctcaccc ccagaaggta 19500
   acaactgaaa gcgctatctc ttggtgttta gagaataaat ctgaatgctc caatgctttt 19560
   gtgcattgtt cattaattaa ttcaattctc aaacattttc tgagtctaat aagtgccagg 19620
   cattgtacta ggtgctagca gctcaatgaa aatatgagta tgtctctgcc ttgaagaagc 19680
   ttacaaacaa cttgaataac aatgcctaat agttatataa aatttaataa tttaaaaaat 19740
   gatatacaca tgcacgcgcg cgcacacaca cactcacaca cacacacacg cacacccctc 19800
   ctttagatcg agagtcacaa gcttatcttg tgaagtgagt aaggcatgta ttattaccct 19860
   gatccatttt atgaggaaga aaggaactgc gtgacttgct gtatgtcttc acatttcagg 19920
   cccagtgctt taaaaataaa acacaaaata ctctcttacc cattatgtgt gatacttagt 19980
   ttgatatgca ggatatattt gggaaaagat gttgtttaaa gagaaaaata ttatgctggc 20040
   ctggctccaa tacaagtcta gacaagatag tacaagacaa ccttaggaaa agctgtcaag 20100
   tagtgaggat gatcattgtt ccccaatccc acctctccca atgtccttct ggctcttatg 20160
   ttacatacag ggagtagcat atactgatta agagcagggc taccagaaac caactgtcta 20220
   actttaaatc ccagctctgc cagctactag ctgtgtaacc ttgggcaaat ttcttagccc 20280
   ctctgcacct tagtttcctc atctgtaaac tgggcatgat gatagtatct atctcctaga 20340
   attattggga tgattacgta agtcagtata tgtgaggtac ttagaatagt gcctggcaaa 20400
   gagtgaatgt tgtgtgtgtg tgtgtgtgtg tgtgtgtgtg tgtacacaca caaaaaacat 20460
   atatatataa cacatatata tactatatat aatatatatt atatacatta tatgtaatat 20520
   atataatatt atataatata taattatata taatattatg ttgtcccttg gttgggtagg 20580
   gctgggcccc atctcttaac ctccagtcct tagctctatg tgccctttgc tctaagccat 20640
   tccatttacc tgctttgtac tgatcacaaa cacatgaagt gctgtttgta aaattataaa 20700
   attcaggact gccaggagat ttgaaacaac ttacctgccc tattctcctt tccttaggag 20760
   ttaacgaaaa aaaaaaaaaa aaaaacggct agatgcagtg gctcacacct gtaatcccag 20820
   cactttggga ggccaaggtg ggcgaatcac ctaaggtcag gagtttgaga ccagcctggt 20880
   caacatggag aaaaataata ataattagcc aggtgtggtg catgcttgta gtcccagcta 20940
   ctcaggaggc tgaggcagga gaatcgcttg aacctgggaa gcagaggttg cagtgagcca 21000
   agatcacatg actgcactcc agtctgggtg acagagcaag actctgtctc aaaaaaataa 21060
   taattactat actatgacat aaaatggttg atgatggctc agtgtagtac cccttcaagg 21120
   atcatgaggg ataacagatt tacattttaa acagaggtgt ttaatttaaa gacattatac 21180
   cttaactaaa atggctttat aaagatagag attttaggtc agaaagatac tagggctctt 21240
   aaagtcatga cacttaagag gcaactacta agagttaaat taggcttacc tttccccaga 21300
   attgcttcag gaggaatgtt tgtttgtttg tttatttaga gacagagtct tgttctgttg 21360
   tccaggctgg agtgcagtgg caagatcttg gctcactgca acctccaccc ccctggttta 21420
   agcaattctt gtgcctcagc ctcccgagta gctgggacta aagatatgtg ccaccacacc 21480
   tggctatttt ttgtattttt agtagagaca gggttttgcc gagttggcca ggctgttctt 21540
   gaactcctga cctcaaatga tccacccttg ttggccttcc aaagtgctgg gaatacaggc 21600
   gtgagccacc ctgcctggct gaatgtttat tttttaaaaa atgtgtcagt attgatcaaa 21660
   agacagaaag ttaaattgta cctcaataat tttttaagac agatatatat aagggttgtt 21720
   tgtatatcca cattcattta ggaattgtgt agatattgtc tatgattgaa atagtttttg 21780
   atcttttatc tctccctcca gttaggaata ttagttaggg tccctcagtg atagggaaga 21840
   tgttttcttt gtatcaggag gtaagagaga aggttgtgaa caaattagat ttgagtttcc 21900
   tccatcctgg gtgacttctg ggtaaaatgg ggtgagagat gaagcatttg ctttctgaca 21960
   aagcagcatg aagcactctc atcatcccca tgccaaagag tgatttgcta ctttgcatta 22020
   caaggaaaga agggtcttaa actcttggcc tggatattct aggtgatcct gagtctgctg 22080
   tgactgagct tcaatgcatt tggcacaacc tgagctacat gaagtgttct tggctccctg 22140
   gaaggaatac cagtcccgac actaactata ctctctacta ttggtgagta tgtacagtac 22200
   aattactgga agactgattg taattgtgtt gggagccttt ggatccaacc tagtcaaaaa 22260
   aaaaagaact gtttaatttt ttctgcaaac tcaacaagat ggacagtttg cttcaatgaa 22320
   aaatgcatct gtctttggtg tctacttttt aaataaactt tctattttta aatatagatc 22380
   ctgtatatta ccagaaaaaa tgtacagaga gatcccatat actccactca gtttctccca 22440
   gtggtaacat cttgtgtaac tatagtgcat tatcaaaacc agaaaattgg cattggtaca 22500
   atccaaaaac ctcagtccta tttcaacagt tttacatgca ctcgtgtgtg tgtatatgta 22560
   gttgtatgca attttatcac atgtgaagat atacaatggt tgcatcacca taaggagttg 22620
   ctcttttata gccacactca cctccgtctt aaaccatctc tccttgtaca cccctctccc 22680
   ccaatcctct aacctctggc aaccactaat ctgttctcca gctctttaat tttattattt 22740
   caagaatgtt actgaaatgg aatcctatag tatataacca cttaagactg gcttaaaaaa 22800
   aaaaaactca gcgtaatacc ctggagatct atacaagtag ttgtatatca cgaggccact 22860
   cctttttata gctcagtagt attccatgat atggatgtac cagtttttta aaccattcat 22920
   tcattaaagg atatctcagt tttttccagt tttttgctat tatgaataaa gctactgtaa 22980
   atattcacat acagattttt atatgaacat aacttttcat ttctctggaa taaatgccca 23040
   agagtgcaat tgctgggtca tatagtaatg gcttatttag tttttttttt taatgccaaa 23100
   ctgttttcta aagaagttag ttcaccttac atttccacta gcaatgcatg agttattgat 23160
   gtctactttt aaaaaggaaa atgatatgtc cggggtcaaa aagttgtttg tttgtttatt 23220
   tatgtttttg agatagagtc ttgctctgtc acccaggcta gagtgcagtg gtgagatcta 23280
   ggctcactgc aacctctgcc tcctgggttc aagtgattct cctgcctcag cttcccaagc 23340
   agctggaatt acatgcatgc accaccatgc ctggctaatt tttgtatttt tggtagaaac 23400
   agggtttcaa cgtgttggcc aggctggtct cgaactcctg gcctcaagtg atctgcccat 23460
   ctcggcctcc caaagtgctg ggattacagg tgtgagccac cacacccagc ctcaaaaagt 23520
   agtttagctt cctgcaaaac aactttgata ttttgatatt tgatacttta gtatttggta 23580
   tttgtgacta tatctactac aaagtttgtt aaatttctaa gttagtcttc catcattccc 23640
   tttagagcct agaattatta gcaacagaac aaggaagaaa gaccagatta atccagttta 23700
   attcagtaaa cctttattta gaacactttg tggataaacc actgaatgca cagcagagac 23760
   aaaaaacata tctcttctct cctaaagctc aaaatataga ggagaagata gtgcctgata 23820
   cagtgacctg cattaaatta agtaagacag tgtatgctga aggctgttgc agagggatag 23880
   atagattcct gtgggaagtt aggttggagc ttggggtggt ggtcgagggg aggtggatgg 23940
   gtggatggag aggggactga gaggctttga tgaaggagga agactgccta gatgggggag 24000
   tttctctgat gctggcctct ggggaagagc attttgagaa atggaggtat ttaatcgttg 24060
   tgatgtcagg gtggagaagg ggagacctag cataggcagt ctgcctctac atggccttgt 24120
   tccctgactg tcatttgtta agacttcttc tgagagaatg ggatgtggag agccatgtcc 24180
   aagacttgtg aagctactgg ataaacatgg cctttcatgc tagagtgtct attttgcctg 24240
   atgacatgat ggaaagcatt tttgatacat taaaacactc atggctatat catggaatag 24300
   aatgcaaaaa aaaaaaaaaa aaaagtaaca cacatttttg gatgaaaacc tccaaagttg 24360
   ccccaggatt cacatccgca ctcctccctg gtggaaggat cctcctggca aagttgggaa 24420
   atcctgcagg tggagccaga agttctgtgt ggttatgtct gaggtctgtt tctttttccc 24480
   cccctgatgt aaatctcatt tgaggttttg tagtttcacc tgctcactct tggtacctgt 24540
   tgtttctcct ttacttaaaa cccttcagtg gctccctggt ggctttaggt taaagtccaa 24600
   actctccagc atgacagaca aatcgcttta tgacagacct ggcccctgtc aacctctcca 24660
   gactcggttg catctgcctc catgcacccc cgcccctctt gcagcctacc attcgcccgc 24720
   agcaaacttc tttcagttcc tcagactgct ttcttcaaat actcaaacac aacactagat 24780
   ttcaaacaga atattcaaac agaatagtgg aaacattctg catcctcttg tagcctaggc 24840
   ctttcctttt catttggatg cctcttattt gtctttatcc ttatttcagc ccagacacca 24900
   tttcctctcc agaaagcctt ccttgacctc tcaagtctag acatgtgtcc cttcctcttg 24960
   tctcacggca ccttgcaatt tcttgtgtca tactacccag cacattgtgt tgtcatttcc 25020
   tgttacttgg tggtagcctg ctacaggctt cagtttctct tgagagcggg agctgtgtct 25080
   tgtttctcct ggtgcctgat atatagtagg tattccataa ataagttgtg aatgtttggt 25140
   aggatccaaa gctagagaat attcagggtt gcggtgatga cttatgaggt ataagcaact 25200
   gaggaatatt atggttcatg aataaaaaga gagaaggatt gagtagatcc agctccctaa 25260
   agaattgcta agaaggggcc aaagaactaa gccttactaa gaaggagtag cctggctgtt 25320
   gatgttgtag taaagtttag ggagagctgt tacacaggct tttaagatgt cttgtgagtt 25380
   ggcagcatcc atgtgtttat tatgctatta acccctcttg ttttccctgg aatcattatt 25440
   ggtaaaaccg cagggaaaac ctttaaagaa tctatgtcta tataagggac tggaaaagaa 25500
   gggaagaagt cttaaaactg tatctcattc catggcagac tgggtagaaa attatcccag 25560
   ctattagaat cagggtggca ctggaggcag atggtagcct agtccttggg gttcttctaa 25620
   gacaaaaata gggaagtgcc ttggggatat actagcaaaa acaaagtctc aatgcaaatt 25680
   gttaaaagaa tgtttcctct gggtgaatga attacaaaaa aacctggact gaccaatttg 25740
   aaaaagcatt cctcttcctc tgggctgaag caggctctca ccaaagctga gagcagatgg 25800
   tacttgggct ggctttcagc cctcagttga tgtctttttg cagggaacaa tctgcacagc 25860
   cactggaggt cgctcaagat gcaattccaa gatagtggtg ggtcactagc atccaagggg 25920
   gcagatatca aggatcctac ttttttttgc gatagggtct tgctctgttg cccaagctga 25980
   agtacagttg gtgtgatcat tgcccactgc ggcttcaacc acccaggccc aagccatccc 26040
   cccatgtcag cctcctgagt tgctgggatc acaggtgtgt accaccatgc ccagctaatt 26100
   tttgtgtttt ttgtagagat ggggttttgc tatgttgccc aggttgctct cgaactcctg 26160
   ggctcaagcc cgtctcggcc tcccaaagtg ctgggattat aggcatgagc caccgcacct 26220
   ggcctacttc ttgtcatata cagtcccaga gctggtcatc agcaggggat ctttctgtag 26280
   tgtagttctg tgccatccag tataggagcc actggctaca tgtggctagt tacattttaa 26340
   tttaaatcaa ttaaaattga atcaaattaa aacttcagtt gcatagtctc gccaaccact 26400
   ttcacaagtg atgagtggct cttgtattgg acagcacaga tagcgaccat ttccataatc 26460
   ccagaaagtt ctgttggatg gtgctggtat agattctgta tcttatcccc ttaccacccc 26520
   acagcttatt ctttctggag ccacactgct tgggttgaaa tcctggctct cttccttact 26580
   ttagtgtgat tttgggtaag tcccttctct ctcagcctta gtttcataat ctgtgaaatg 26640
   gagatagtaa taatagtgcc tacttcttaa ggttgttgtg agggctagtg tttagagcca 26700
   tgactagctg ctagcatatg tagagtaagt gctcaatcca taatagctat tattactatt 26760
   ggccaggcgt ggtggctcac gcctgtaatc ccagcacttt gggaggctga ggcaggtgga 26820
   tcatgaggtc aggagatcca gaccatcctg gctaacacag tgaaaccccg tctctactaa 26880
   aaatacaaaa aaatagccag gcgtggtggc gggcacctgt agtcccagct actcaggagg 26940
   ctgaggcagg agaatggtgt gaacccggga ggcggagctt gcagtgagcc gagatcccac 27000
   cactgcactc cagtctgggc aacagagcaa gactccatct caaaaaaaaa atactattta 27060
   ttattattat tattgttatt atccccactc ctttgcagat gcagtcagta cacagcgctg 27120
   gcctcttccc gtctcacaga attagtacct aacccatatc tccttctttc ttgctctcct 27180
   ctagatagat cctttctaat caaaaccaca aaagaaaggg agtcaagaca cagtgctcat 27240
   tagcaagcac cagaaatggt ttaggtacca gtataaatca catcaaacaa actgccagag 27300
   actgttttcc ctaaccccca accataaaat acggagtctt tctttttttt ttttttcttt 27360
   tttttttttt tggagacaga gtctcattct gtcgccaggc tggagtgcag tggtgcgatc 27420
   tcggctcact gcagcctctg cctcccgagt tccagtgatt ctccagcctc aacctcctga 27480
   gtagctggga ttacaggcac gtgccaccac acctggctaa tttttgtatt ttcagtagag 27540
   acagggtttt accatgttgg ccaggccggt ctggaactcc tgacctcaag tgattcaccc 27600
   acctcagcct cctaaagtgc tgggattaca ggcgtgaccc atggcgcctg gcctggagtc 27660
   attttttgaa atcaaaataa ttcattgaag ccctagggca atgacttgtg tccctgtagg 27720
   gacagtctgg cattgaacag accccggcct gaagagcttc agactagtca tgccagcgaa 27780
   ttttagggat tttttttttt tttaccacat taatcatctt ctgagttggg agaatagtct 27840
   ttaatttgtc aatttccttc tcatatctgc agtgtgactc tgcagatgaa ttcagtatat 27900
   aaattttaac aggaatcacc tgctgtttta ggttaatgtt tcacaagaat tcaagaaatg 27960
   ttatctattc agaaccagta ttttcccttc tataattatt tctaaattac aacaaaattg 28020
   ttttaaaatt tcattatctg agatccatga gcatatgcag aatactcagg gtacagaaat 28080
   gaaaaaggga tcaagaagat tgtgcattcg aggagttcct ttcatcatta aaaacaattt 28140
   tacacatggc tttctttagt tatgtaagaa aaatgtcatt aaatttcttc agtttctgtt 28200
   tttggccatg ggcggaaaac attccaggac agtctgatcc aaaaactagt catgacaata 28260
   acttcatttt tacaacactg ttttcttcat aaaccacagg gcgctgcaac tagggggaaa 28320
   agcttatgta ctacctattg aaggaaaaaa gcattttcta tatcgaagca ttcctttgtg 28380
   cagcctttga aggaataaat acattagctc tgagtttttt tttttttaaa ctgacttgca 28440
   gcagaatctc ctggagaatt gctgttcaaa tgctggttcc taggtcctgg caaaaccttc 28500
   taaatcagat tttctgggag agggtttgag aaacctgcat tttaaagtag tacagaggat 28560
   tctgaggtaa caggttaagg atttgggact ctttactctt agttcatttt tcttaaaatt 28620
   gtcttccttt tctgcaaaac ctttgtgcaa ctccctttgt ggctgatgat gtaagacttg 28680
   actctgtctt ctgccagtta ctacaaaaca gttctctgat agcctagact aaaaaaaaaa 28740
   gtgtaataaa cacattgtct gcctgcgctg ggctaggtgc tgatgatctt tggggaaagg 28800
   tatgtgtcga ggagaaatgg gaaataaggt acctgtaata ctgcttatgg aagacagtta 28860
   aagccacaca gtggagtgag acacttagaa agccattgtg ttttagaaca ttgacctggc 28920
   agtgattgga gggcactaat aatgaaggtc agggaggtca atctgaagaa gcaatggagc 28980
   ctgtagtcat tagggtatga atctcagggt gtgggcaatg gatgtatcat aaaagacgag 29040
   gatccatgaa gctttgcaaa ggaagaaaga acagaatttg gtgatagaat acagggaaca 29100
   gaggtggggg aaaaggtgat tttaatttaa attacctaat ctaggagccc aaagaaaatg 29160
   agctctggct gataatggga aggttggaag aaaaaacagt ttggtggaga agagggtgca 29220
   tgatgtgttt tggacactgg atttgaggca atggtagaac actcaaatgg agcagttgga 29280
   gatctgggat ttgttctgag gtgagatgtc aggattagga attaccagca tagaaactgt 29340
   agttgaagga tgagggagtg ataaactgga tgtgagtgga tgagggagtc agaaagaggg 29400
   aagaatgtga gtagtcctcc tactccccct ctgctggaag agagaggagg gaacagaaca 29460
   agccaagaaa caaggaagga gcagggcaga gggaccagag gaggaatcgt agacttcagc 29520
   agataggcat tttcaagaag catgaaactt ctaatcccag ccttagaatg tcttctctta 29580
   tacagaagaa ataggcctcc tgtagtctct atagctgctg acagtaccag tgttcatcaa 29640
   aaaggaacaa tctaggctgg gtgcaggggc tcacgcctgt aatcccagca gtttgggagg 29700
   ccgaggcggg tggatcactt gtggtcagga gttcgagacc agcctggcca acatgctgaa 29760
   cccgtctcca ctaaaaatac aaaaattagc cgggcgtagt agtgtgtgcc tgtaatccca 29820
   gctactcagg aggcatgaga ttcactggaa ctcgggaggc ggaggctgca gtcagctgag 29880
   atcgtgccac tgtactccag cctgggcaga cagagtgaga ctctgtctca aaaaaaaaaa 29940
   aaaaaaaaag cagaaaacag tcttttcttc ctagcatatg aaatgagtca cattgtacaa 30000
   tagggttttt ttaaaaaaag ttatcttgcc cattgcaaat attgcttttc atggacatct 30060
   cagtcattaa aaagaattct gccttttttt tttttttttt tttttttttt tttgtggaag 30120
   ggtcttactc ggtcacccag gctggagtgc agtggtgcaa tctcagttca ctgcaacctc 30180
   tgcctcccag gttcaagaga ttctccttcc tcagcctcct gagtaggtgg gattacaagc 30240
   acctgccacc acacccggct aatttttgta tttttggtag agacagggtt tcaccatgtt 30300
   ggcaaggctg gtttccaact cctgacctca agtgatccgc ctgcctcagc ctccaaaagt 30360
   gctgggatta caggtgtgag ccaccgcgcc cagcctgctc ttatttcttg aatactctgt 30420
   tgaatataat gcagaagatt gtctcatttt atcttgtagg cacagaagcc tggaaaaaat 30480
   tcatcaatgt gaaaacatct ttagagaagg ccaatacttt ggttgttcct ttgatctgac 30540
   caaagtgaag gattccagtt ttgaacaaca cagtgtccaa ataatggtca aggataatgc 30600
   aggaaaaatt aaaccatcct tcaatatagt gcctttaact tcccgtggta agttttagaa 30660
   gtcctctaaa acagtatgga taaaaagtgt gttatatctt ttgcacaatc aatacaaata 30720
   ataaaaaatg atcattctgg gtttaagatc ttctgaaaga agggactata ttgatgtata 30780
   actttctttg atagatcctt tttaaagaag taaacttgga aattcttttt attgaacaca 30840
   tgtgccagat gctttatcct tgtttagagt acactgagag ttagaacagt tagttgtaga 30900
   accaacattg gaattgtctt agttcatttt ttgtttggta ttttctggta ttctgttgct 30960
   tataacagaa taccagagac tgggtaattt ataaagaaaa ggaatttatt tcttagagtt 31020
   ctggaggcag ggaagtggaa ggtcaagggg ccaagtctga tgagtgcctt cttgtttgtg 31080
   gggactttct gcaaagtccc aagacagcac agggcatcac atggttaggg agttgagcat 31140
   gctagctcag gtctctcttc ctctttgtat acatccacga gtcccactcc catgataatc 31200
   cattaatcgg tcaatagatc aatcaattca tgaaggcaga accctcatga cccaattatc 31260
   tctttaaagg tccaacctct ccatactgcc acattgggga tgaagtttca acatgagttt 31320
   gggagaagat aaacattcaa atcatagcag gaacccaggc catctgactc taaggtctag 31380
   tctcttgata cttaaagagg atgatactca taatgatcat atgtagttcc taaataatct 31440
   gtctcttaga aaaggcatag acttaaggta gtgtaatcgg gtgatattca aaatatttaa 31500
   cattctgtat ggcatgggca ctgatcagtt agaaaaacca gtatagcctt aactgtgggt 31560
   gccagctgca tgtcagcctg ttagaaagca ccctcagctt gaacacagat tatctgatta 31620
   ggttctaact ccttcacttt gggcaaggca tataaacccc cttcaaatat ttccttatct 31680
   gtgaaatgga atatataatc tccctctatc taaaaaaatt gtcataaggt ccaaatgaca 31740
   taaagcgtat taacacattg tgtaaattac taaagcaaat tccattagcg taatggttta 31800
   tttgttgaaa tatcccaact gggagctcag attctggctt agatagtcat agaagtgaat 31860
   tctaataggg taccaaagtc agcttacttt gtaggccaag ggagtgaggt tggagtattt 31920
   tgcctagtgg ggtaagctga agcagagccc ttctctttgt ttggcagatt atcttagatg 31980
   atcttttaag tcttattttg ctgttttcct ctctggatat taatggtgac ataaaggaag 32040
   aaggaagatc cttctcagat agaagatgat atcttcaagt tgttttctgt taattaccca 32100
   ttatcttcta ttgcttggtc ttaagtatag ctgactcctg aaaaagtaaa ttgaccaggc 32160
   taatgcattt ttaaaaataa attatttttt tctttttctg agacagggtc tctgttgccc 32220
   aggctggaat gcagtggcgt gatcacagct cactgcagcc ttgacctccc gggctcaagc 32280
   tatcctccca tctcagcctc cctagtacct gggaccacag gcatgtgcca ccacgccagg 32340
   ctaatttttg tattttttgt agagacgggg ttttgccatg tttcgcaggc tggtctcaat 32400
   ctcctgagct caagcgatct acccaccttg gctcccaaag tattttactc ttaattgtgg 32460
   ttataaacac acaacattaa aattaccatc ataaacattt taagtgtaca gttcggtagt 32520
   gttaagtata ttcacattgt tatgcagcag ctctttagaa ctttttcatc ttgcaaaacc 32580
   gaactctgtt cattaaacat taatttcccc tcccctccgc ccttagcaac cacctttcta 32640
   ctttctgttt ctatgaattt gactacttaa gatacctcat aggagtggat tcatacaata 32700
   tttgtctttt gtgactggct tattttacct agcataattt ccttgaagtg catttatgtt 32760
   gcagtgtgtg tcagagacta agccaatgtt ttaatggtgg tctttgattg gcattaataa 32820
   gtgacaacta gggaagcccc ttgtatctgg tgccttatta ctcatcaggt ggaagtacat 32880
   ctggatccaa gcctttcttt attctctatg ttgcgtctag atgtcctgat tataccctgg 32940
   ccattttcta accagatgca ggtcagagtt catatcatct gctagatacc ccaaatatgt 33000
   tccttaaatt cattttacta ggcagtttta aataaacttc ttctataatt gtggctaaat 33060
   agaaattcta aaactatgta tctgatcatg aaaggccact aaatgcatca gctctgtttt 33120
   gatttaactt tatgaaccct gcatatatct gcagttgtct ctcagtattt gtgagggatt 33180
   gcttccagga acttccaagg ataccaaaat ccatggatgc tcaagtccct gataaaaagt 33240
   agcatagtac ttgcatataa cctatgcata ttttctcaca tactttaaat catgtctaca 33300
   ttacttataa tacctaatac aatgtaaatg ctatgtaaat agttgttata ttatctaggg 33360
   aataaatgag aagaaaaaac tctgtacatg ttcagcacag atgctatttt ttaaaaaata 33420
   ttttcagttc atggttggtt gaattcatga atgtggaatc catggatatg gagggccgac 33480
   tgtgtgtttg tgtgtataat caagctttgg aagcttactt gtgttctatt tttgtttttc 33540
   agtgaaacct gatcctccac atattaaaaa cctctccttc cacaatgatg acctatatgt 33600
   gcaatgggag aatccacaga attttattag cagatgccta ttttatgaag tagaagtcaa 33660
   taacagccaa actgagacac ataatgtttt ctacgtaagg ttttaaaatt attgttttta 33720
   tttggctatt tttcttttac ttgaatttct tacattgaaa tatcaatgaa acatagctct 33780
   tacatttgtc accttatgat gatgatggta atgaaacttc ttgtctaaaa taaggcatgg 33840
   tggaagattt gaagatttat ggtatgatag tatcagctgc aagtttcaag ccatttattc 33900
   atctgtaagc ttcttagaaa ggcctattaa acgataattc atttgctcaa cacgttctta 33960
   ttaaatatcc accacatgcc aagcactctt ttgtgtgcta gggataaggc agtgaacatg 34020
   acacctgaca agtttttttc ttcatgaaac ctacataata gaaaacgtaa gtaaataagc 34080
   aagctaattt cagaggatga gtgctgtgaa gaaaataaaa tgggctgatg tagtagagtg 34140
   actgggagaa cgagagggaa tattaggtag ggaaatcatc tgaggtaata acatttgaga 34200
   tgagaccaga atgatgagca gacgctagcc atgtgaagat ctgggtgtag ggggtcagca 34260
   gatacagact ctgaggcaaa aatgagctta gcatgtgtga gagaacagaa aggagggtgg 34320
   tgtgaacaaa atgtagcagg aaaagatgtc taagcagtag gcagacatca gatcttgcgg 34380
   acactttgga ggctctgata ataccattct gttttcttgg agtttgtggc aactaaagta 34440
   atcagatttg agtagtcaat atttgttgga ttatgtgcct gtatgctatt cagaatcaaa 34500
   tgcagaacat gctaattaga ggccctgtag gacactgacc agagtgtact ataatctcag 34560
   gacatcagtc cagttgttct gctttagggt aaaggtcaca aactagggcc tcacactttt 34620
   ttgtgtggaa cacaaagtcg tgttttgttt gtttgtttgt ttactagatt tgaatttttt 34680
   gcccagattt taaatcatag tactttaaga gtgtgtgtcc ataagatggc gccttaagac 34740
   catgaagact gcaccactca cttgtctgct acctgcctgg accctttgtg tgtttgagtt 34800
   ttagagatta gaagtgaagt gttttatatt ggcttttaaa ggagcctagc atctcgcacg 34860
   tgggtaaatt ccttggtcca gttgttttag ctcatcatgg aacttccaaa atgcttgcta 34920
   aaataagcca gtgttgacag gctataattc caactccaga gggagtgacc aagtccacag 34980
   cagaacagaa cagcaaatgc tcttctgggc atttgcatta tgaatccttc tgctgaaata 35040
   taataatcat tactagtacc agaaattaag tgccccatga aatgcagcaa agggaaaaat 35100
   ctaagggctt tcatgtggaa gggggctggt acttgaattt acaacaaaag gagttatcag 35160
   aaagctgaaa tacacttgtt tactccagtc tttactgttc tctcctgtct gaaatcctga 35220
   gatgcatgtc tgaaatcctg agatagtatt cattttgcca ctcaattatt ttctgctgtt 35280
   taacctatca catgtatgca ttttctctcc actgaaaata tagactccta cagaaaacca 35340
   aatgccacat gttctcactt ataagtggga gctggacaat gagaacacat ggacacatgg 35400
   aggtgaacaa cacatactgg ggcctgtggg ggctgtggtg ggaggaggga gagcatcagg 35460
   aagaacaact aatggatact ggccttaata cctgggtgat gggttgatct gtgcagcaaa 35520
   ccactatggc acacttttac ctatgtcaca aacctacaca ccctgcacat gtacctcaga 35580
   atttaaaagt tgatattttt taaaaaaaga aaatatagac ttctgtagga aggagtttgg 35640
   tgctgtctac tttgtgcagt tcctgctact aaatagcagc taacctgtat tgagtgatta 35700
   ctatgtgcta ggtatcttaa taagcactat atctgaattg tttcatttaa tcttcacaat 35760
   aaccctaagg atctaggtcc tgttattttg tctccatttt acaagataag aaaacttagt 35820
   tactcctaag gtcacatttt tagtaagtgg tagaatcaac atttaaccaa agttgtaacg 35880
   ctaagacttg gattcttaac cgctagagaa cacctcaata cttcctctta atagcaatag 35940
   ctatcattca ttaagtgttt atggcatgct acaaagatgc tcttctatgt gcttcttaat 36000
   acagtagctc atagcaaccc atgaaataga aactatttta acctacattt tattgaaaaa 36060
   actaaggctc agagaggtta agtaacttgc tacaagccac acagctaata agtcagtaac 36120
   acttcaaagg ttggttggtc tccatgtatt taactttgac caagaatact tagcataatg 36180
   tcatgtgcac aatcatcatt taataaatta ttattgaatt gagttacttt tttttttttt 36240
   acaaacttct gaactgaaga gaacacatac actatggaag ttatctacct aaatcagaac 36300
   tattttctat ttggaattaa ctctgtttgt acacttgagt ttaatattct tcctcctagc 36360
   agatgtagaa tgtcatcagt tttttgtgtg ctcacagatt ttgaactctg ctgcttagat 36420
   ttttcttttt ttttcctttt tgaggaatta ttttgcctgg tgttacactt aagaggggga 36480
   agtaagcggg aacatgaact ggtaccaaca catgggggct gtggtcagag gtagctcatg 36540
   acagaattat gaagtaagga acaacttgtc tgtgaccacc ccccaccccc cacccccacc 36600
   ttcccagcag actatcttct tggtgccctg gctttccttc tcttagtggt ctctgaagag 36660
   ccagtaaact atgcaggggg atttacattt gaacaagagc ctagagacgt aaatctacag 36720
   gttgattcgg caagtttgga atttctatat atgggagatg gtccagggga ggagggacat 36780
   caatttgcaa cttgatccct cactggtagt tgacgcttgt gattccccaa caggtctacg 36840
   attctaaatg gccagtaata gggtatacta gccccctgag tggggttttt tttttctcaa 36900
   cctctgtatg aaatattgaa aggatgtccc ttcatcattt gcttggcctc tcagctcttt 36960
   acacaggctg gtaggaacta tttagtgaat gacattgaat gtcaaaaata atatctatgt 37020
   gttctggtct tggttccaag tttaaacgtc tctttgatag aagttgacta tttaagcaac 37080
   agattttcat gtgtttttta ctccattttg gaaatacttg catttaagga aattagtatt 37140
   ctctttacat tagacagcaa gtgactttta ttgtgtggag gagaattttg cattacactt 37200
   tcttttctcc aacccagtca agttttatat cttatactct caacaaggga cactggtttc 37260
   tcgctccctg tctcttttta aaaagttttt actgagttat aatacatgaa caaaattaca 37320
   caaatcataa cctatagctc aatgattttt cacaaatgat tttcacccta tgtaaccagc 37380
   accacagaag cccccttcag ttgctgtcct cccaaagagc agctactctc ctggcttcta 37440
   atcccataga ttgattttgt tgctacttta aaactttata taaatggaat tatatagaac 37500
   gtactttttt ttttcgagac agagtcttgc tctgtcaccc aggctggagt gcagtgcagt 37560
   ggcgcaatct cagctcactg caacctccac ctcccgggtt caagcgattc tcctgtctta 37620
   gcgtgcggag tagctgggat tacaggcaca tgccatcaag cccagctttt tttttttttt 37680
   tgtatttttt agtagagacg aggtttcgcc atgttaggca ggctcctggc tctcgaactc 37740
   ctggcctcat gtgatccacc ctcctcagcc tcccaaagtg ctgggattac aggcgtgagc 37800
   cactgtgcct ggcccagaat gtactttttt gtgtgtttgc cctttttttc actcaacatt 37860
   atgtttatga gattcaccaa tgttgtttct tatagctgta gatggcacgt tctcactaca 37920
   ttcattccat tgtgtgaata taccatactt tatccattct cccattgatg gacgtttgga 37980
   tagtttccca tttggtttta tgaatggagt tcttatgaac gttcacatac ttgtctcttg 38040
   gtacacatgt gcgtacattt gtatggggca tttatatagg catatgtggg tcataaggtt 38100
   acatatgatt gtctctagta gataataaca gttttcctgg atggttgtgc cagtttacac 38160
   tactaagcag aaattgtgag ttctagatgc ttcatatcct cacaacactt gttgttgtca 38220
   ggtaagtgtg tgtattgtta tcttgtggat ttaatttgca tttttctcat gaccagtgat 38280
   gttgaacact ttttcagatg tttattcgca acttgcttat cctcttatga agtgcctgtt 38340
   caagtctttt gccagttttt ttaaaattgg gttttctgtt tttgccttat tagtagggta 38400
   agaaaaaata catatgcaag tcctttgtca gaaattgcaa atatgttctc acactctatg 38460
   cattgcctgt ttatttccct ataaggtgtc atttgatcaa caaatgtttt taattttaat 38520
   atggatgact ttattaaaaa attctcttta tggttaatat gggaggtttt tttggtcttg 38580
   tttaagaaat atttcccttc ttcaaggtca ttaagatatt ctctgttttt atctaaaagc 38640
   ttgctatagt tatgtattta ttaattcatt ttatcttaca catttaaatc tgcaatatat 38700
   caggaactga ttttgtgaat agtatgaggt agggcgtcaa gaatcttttt ttttttcata 38760
   tgcatatgta attgacccag caccatttat taaaaaaggc caaccttttt tcactgtacc 38820
   tgatcatctt ttaatgggat aaaaggaaag cttgttagaa atgactaggt gatagaaaat 38880
   gggtttttaa acatttagga ctcattagtt tatttattta tttttatttt caaggtccaa 38940
   gaggctaaat gtgagaatcc agaatttgag agaaatgtgg aggtcagtaa attcaacatt 39000
   agctatttgg gtttagactt agagcagttg ggggtggagc atagcctaaa tatgagctgc 39060
   tttgagaaaa acaaattacg ttgttccgtg agttattgac aattttttcg aaaagtcaaa 39120
   gataatttga attgcaatcc aagtcttaat catagacaga atttggcaga taattgtatt 39180
   atgattcaat aacagtgaaa aagaataaac tggtaatatt ccttgtgtat ttttatttta 39240
   tgcctaagaa tacatcttgt ttcatggtcc ctggtgttct tcctgatact ttgaacacag 39300
   tcagaataag agtcaaaaca aataagttat gctatgagga tgacaaactc tggagtaatt 39360
   ggagccaaga aatgagtata ggtaagagaa cagaattttt attaaaattt aaaacactga 39420
   tgtataaaaa ctaagctgaa gcagaaaata gactggggaa agggactgtc atgctctctt 39480
   gatgtcagga aaagagtact tacaattgaa atttattttc ttcttaatca ctttcaaacc 39540
   acccagggtt aattgacagt atacagttat ttagaaaacc ataatccaaa tggttttatg 39600
   ttcttttcta tctcatttcc atttttatta acaaactgaa caaaaatata gaaagcaggc 39660
   ataggctgca cacagtgact cacgcctgtg atcccagcac tttgagaggc tgaggcagga 39720
   ggatcgcttg agcccaggag ttggagacca gcctgagcaa catggtgaaa ccccatctct 39780
   acaaaaaata caaaaattaa ccagatatgg tggtgcacgc ttgtggtctt ggctacccaa 39840
   gaggcagaga tgggaagatc tcttgagcct gggaggcgga ggttacagtg agctgagatt 39900
   gtgccactgc cctccagcct gggcgacaga gtgagaccgt ctcaaggaag aagaaaaaaa 39960
   aaaggcaggc ataataaaat ttgcacattg tacaatacta gagactgtcc tacatgctgg 40020
   ttggcagaaa caagagtgga gccatttaat agaaacctgt acctaagttt gaaaaaaatc 40080
   atttattaat tgaaaggctg agcaagctag gctcaagtac aggactggaa agacctgtgc 40140
   tttatggatg tgtgtgtggt ggggtgggat acacggcttt ataagtgcaa tgtgactcaa 40200
   cattgtaatc ttaaatgtgt caaaaaggca tatacaatct taggagtgat aagtgcagca 40260
   cccagaaagg aatgctgcag gttggggcac agagctatgg tcacatcaga attgtgcagt 40320
   attccattct gagcaccaca ttaaaggagt tgccttggca gactggaatc tatccttgaa 40380
   aggataatca ggatgttgaa gaatctcaaa acttggcaag gaacaattgg taaagtaact 40440
   ttgaggagtc atgattgctg atttctaata tttgaggtat tgtcaggtac aagaaggaat 40500
   aggctttact ttttgtgtct ccaaggggca acacagaacc aacttaagaa ctttttaaac 40560
   agctgtatat tggtcaaaca ggttgtcgag gtagccaatt ccctattaat tgagaagctc 40620
   aacaagttat gtgccaggca tggttgtatt cgtttattta ttgttgtata acaaattact 40680
   ccaaagttta atggcttgaa ccaacataca tttaccatct cacagtttcg gtgagtcagg 40740
   agtctagcca cggcttagct ggaggtctca tgaggttgca gtcaagctgc aatgagaact 40800
   gaaatatgtc tgaaggcttg actggggaca gagcatccat ttccaagttc actcatatag 40860
   ttgttggaaa gcctcagtac ctcactggtt gctggatgga ggctgttgca ggttgaattg 40920
   tatctcccaa aaaggtatgt tcgtaggttc caaccccggt acctgtgaat gtaaccttat 40980
   ttggaaatag gatttttgca gatataatca agtgaggatg aggtcatgct ggattaaagt 41040
   ggacagttaa tctaatgact agtatcctta taaaaagaga agccacacag agacacacag 41100
   acacacaggg aagaaagcca tgtgaagatg gaggcagaga ttgcagagat gcagctacaa 41160
   ccaaggaaca acaaggattc ccaggagcta ccagaagcta ggaagaggca aagagggatt 41220
   cttctctaga gccctcagag ggagcatagc cctgctgaca ccttcatttt ggatttctag 41280
   cctccaaaac tgtgagataa tacatttctg ttgttgtaag ccattcagtt tgtggtaatt 41340
   tgcttccctt agttccttgc cacttggacc tccccatagg gctacttcct gacatggaaa 41400
   ctggctttcc caagtgagta atccaagcaa gaaacagtgt gagagaacac gcaatatggg 41460
   aaccatggtc ttttataacc taaccttaga aatgacatcc cattactgct gctgttctgt 41520
   tttttagaag tgagtcaatt agtctagccc atgctcaagg agaggggatt acaccaggga 41580
   atgaatatca ggaggcagga atcatgtgga gaaatcttag tgactgccta aaacaatggt 41640
   gccaggcctt gatgatgctt tctagatgtt catgacctag tgggaaaaat gaatttgtat 41700
   atagacaaat gacagtctat atgttagatg tagcaaggca ggctttggaa ttggacagtg 41760
   gaactggaat ttgaggcctg atgccattat ttactaaatc cttccggatg acttactaaa 41820
   cctctctgag ccttatcttt ctcatctgct taaatgggag ataatgctat cattatagca 41880
   tatatatgag ataagcatgc aaaaccacct agtgtggtat cagacatata gtaggtgctt 41940
   atttaagaat aatagtagtt gttaggatta ttttaggtat tttagtagtg gtaagtaacc 42000
   agtaaaatat gtagtcactg ttaataagat tacatattta atatagcatc tttgttaata 42060
   gtatttaaaa ctagttaaga tcttctgatt tacctgagcg ggtggggaaa cccaccccat 42120
   gagcatcccc tgggctcacc cagtgcacag agggaggcct cccgtggctg ggcccctctc 42180
   agtcagccca tgtggctgcc atgacctgga gcaccacagc cgggggcgcc caccagctca 42240
   acaccaccac attcacgtgg cagcacctcc ctgcctggca accgctgctg ttggccagca 42300
   ttaggctgca gctcttcttc tacgtgggcc tggccttcat cagcctggac ctctattact 42360
   cctccaccag catcaaggag ctggagtaca actacaccgg cgacccgggc accagcaact 42420
   gctcggtgtg tgctgtggct ggccagggct gtgtgccact gcccatctgc tcatgcgcct 42480
   ggtacttctc actgcctgag ctcttccagg gccctgtgta cccctactac gtgctgacca 42540
   acttctacca aaacaaccgg cggtatggag tgtccgcgac aacgcgcagc tgagcgggct 42600
   gcccagcacg ctgcaccatc cagtcaatga gtgcacccac tgcgccgcct gcccatcgtg 42660
   caccctgcaa tgtcatcacc aacagcctct tcaacgactc ctcgctgtgg caccagtgct 42720
   ggcccggcga gccctacgtg gaggtgccgc gctaccgcac tgcacctgca tcacccggta 42780
   gaccaactac cccatcaagt tctgcaaccc accactggtc aacggcagcc tggcactggc 42840
   cttccatggc acagcacccc tgcccaactg gcgctggctg gtctacgaca agctcagccc 42900
   catccccaac aacaacggct tcatcaacca ggacttcgtg gtgtggatgc gcatggcagc 42960
   gctgcccacg ttccgcaagc tgttccgcaa gctgtacggg cacatccgcc agggcaacta 43020
   ctcagctggg ctgccgcggt gtgtctactg tgtcaacatc acctacaact acctggtgcg 43080
   tgcatttggc ggccacaggc tccgcatctt cagcagcatc ttgtggatgg gtggcaggaa 43140
   ccccttcctg tgcatcgcct atgtggtggt cagctccctc tgcatcctta ctggctttgt 43200
   catgctggtc atctacatta gctaccagga cctaccagaa agatgatgac aatgaggagg 43260
   aggggtgatt ccagctttcc aacgaccctt cctgcttctt accaaaattc tgcaagcttt 43320
   tttcagtctc tcccctttgc ctctcatcct attccaacct ctcctcactt ttcctccctt 43380
   tgtgaatgag ggggccagag aagggaatta cccacttact cttgggggct gctaaactct 43440
   gttgcctggt ggcggagggt tgactgcaga gtgaaacatc cttgcaaact ctccccacct 43500
   ccttcatgat gctgagatgc catgttaggt tctccaagtc caagagtgga agggatcctt 43560
   atagagactc attttcaacc cctaccagag gaagagattg agagacctag caacatcaaa 43620
   taactcaatc aagatcaggc agctggcaag tggttcctga cgctaaggct agagcttttg 43680
   ccactacagt acagtggttt tcttttcttt gtggggaggg tgggttggga tggagagtga 43740
   gacccacaaa ttatctgcag aggcatggag gggtgtggga gaacatattt gttaaatatg 43800
   cagatagatg aggaggcacc aaccccagag attctgacac agtaatgctg gggtgagatg 43860
   cttgaaactg tgttttaaca aactcctctg gagattcctg tattctctca aatttggaaa 43920
   tcactaccct gaacctggtt gggcctgaag cagtcacctg agtccagttt ttcagatagt 43980
   aatttgttcc cagggacagt gacaccataa tgttgcaggt ttggtctggc actctgcctt 44040
   aaatatagta tgctctaatg acttgtggaa tgaattttta aaaaatcact atctctgggc 44100
   tgggcgccat ggctcatgcc tataatccca tcactttggg aggctgaggc gggtggatca 44160
   cttgaggtca ggagtacaag accactctgg ccaacatggt gaaaccctct ctctactgaa 44220
   atataaaaat aagctgggca tggtggcaga tgcctgtagt tctagctccc gggaggctga 44280
   ggcaggagaa tcgcttgaac ccgggaggcg gaggttgcat tgagtcgaga tcgtgccact 44340
   gcactccagc ctgggtgaca gagtgagact ctgtctcaaa aaaattaaat taaaaaaaat 44400
   cactatctct ggaaaactag ttggtgtaca gatttgtagg ccagggtttg tgtcgattta 44460
   tttgatattt tagtataaca gcaaacaggc aaactttacc agatactgtg tagaaatttg 44520
   aaaatgtgag gccagtttgt acagttcaga gggaatgctt tttaactgta gaatcagata 44580
   gctggaaggg atctttgagg gaaagtaatt tccctagtca catctatgtc tcctggctca 44640
   gtcttctttg tcatttaatc tgtgtgtgtg ggtgtgtgcg tgcgcacacg tgcgtactta 44700
   caaagggctt cattcatacc ttttctcttg gatctggaaa aaattttttt atgttttcca 44760
   atgagataca tttgtagtaa tcatatttga aacaatgcta aggtatatat agtcttatat 44820
   gtaaactaga atcttaacat tatttgaatc ttggagataa tgttaaaagt cttagctaaa 44880
   atgattccat tgattttatc gattgactaa catcaatcaa tatgtttaaa gttattctaa 44940
   gaagcaatat ttttatttaa aaaaaccttt ctttcatggc aaaatagctt aaaagaactt 45000
   taaaaaaaaa ccctatgtga tatgttccag tttatttgct aaaagcaaac agttatttgg 45060
   tatttgtcag acagaattct tcagtacctg ctttctatta tagctatttt ccagttacca 45120
   ctcattcgtt tatactcctt cctttaaagc agcacaagat cttgaagttt tttttttatc 45180
   agttaagaaa tattggattg tattggatag tatacatagt ttagtctgtt agttctctga 45240
   aagttactga acaatggaca atgtgcatgt cactgacatc cgtctcagat cttctgggac 45300
   aactatgatt taagagattc tatctcatta ttacagatac caaaaaaata cttgggtaat 45360
   tccagtgtgt tggctttcca actcctgaac actacacaat tttattatca gtttttgtat 45420
   ggtgatttta ggctatgaaa acattatttt atgtacatag agaaattttt attatgaatg 45480
   tttgagctca ctagcccagc cctcctctat tttgaaaaag agagtttact acatttttta 45540
   ctatgtagtt gaaaacaaca tgtattttgt catttttaga atggtcagtc tatttctata 45600
   aaattttaaa tgtggctggg cgcagtggct catgcctgta atcccagcaa tttgtgaggc 45660
   tgaggcggac agaacacttg aggtcaagag tttaagacca gcctggccca aatagggaaa 45720
   ccccgtctct actaaaaata caaaaattaa ctcggtgtgg tggtgtgcgc ctgtagtccc 45780
   agctactcag gaggctgagg caggagaatc tcttgaaaca tggaggcgga ggttgcagtg 45840
   agccaagatc gtgctattgc actccagcct gggtgacaga gtgagagact ctgtctcaaa 45900
   acaaacaaac aaacaaaagt ttaaatgaga ccatcaaatt taagggattc tgatccagat 45960
   taaattaatg aatatggttc caggtcctag tctctgagtt tttgaaagag aataaagctt 46020
   atgtttgtct tttaaaaaat atcctaattt caggtaatct tttaggtatt tgctcgccta 46080
   gggaagttca ggcaccagag gaggactttt tttttttaaa ctattaattt aacagaagat 46140
   aagataattg aagagcttgg gaaaataggt tgggggaggg agcagaaata tttgtgatct 46200
   tactagctaa cagaattgct gcttggttta ttactaaaag ggttttattt ctcaagtctt 46260
   tctgtttgct tttcattctc caggtaagaa gcgcaattcc acactctaca taaccatgtt 46320
   actcattgtt ccagtcatcg tcgcaggtgc aatcatagta ctcctgcttt acctaaaaag 46380
   gtaaggtagc tgcccaggca ggttttgcag tttctgatag cctgtttgct gtggttatta 46440
   cttttagtgc ttgtgtttta tgtctgccca gtgtattaga agcatgttgc ctgtaagtgg 46500
   ctttcgggag tatgacatga caaggaagta ttaccacgcc cactttcttt tcttttcttt 46560
   tttttttttt tttcctgaga tggagtctct gttgcccagg ctggagcgca gtggtgtgat 46620
   ctcggctcac tgcagcctcc acctcctggg ttcaagcaat tctcctgcct cagcctcccg 46680
   agtagctgcg attacaggtg cccaccacca cgcccggcta atttttgtta tttttagtag 46740
   acagggtttc accatgttgg ccaggctcgt ctcaaactcc tgacctcaag tgatccaccc 46800
   tcctcagcct cccaaagtgc tgggattata ggcatgagcc actgcaccca gccgctatgc 46860
   ccactttcta atgaaaacca tgttggatta gttttccagg gctccctgct caagcactgc 46920
   tgcctttgca tgtgtttagg attgttgaat aggtcctatg ttcacataat actctttcaa 46980
   atgcctaatc tatcaagtta gtcagtagtt cttagaatga agttttagaa atggagacct 47040
   ttactaataa aatgacttgt ctggtattaa tgcaaagtag gctactactt aatattgtgg 47100
   gaaagagaac ttcatcaagt tgtcatgaat gtatggtatc cattccttga gaatatttta 47160
   ttttctctat ctcattatag gactgttatt tgctcttgta gtaaataact aaggttgaaa 47220
   taaggttatt aagttggatc cagaaccaat attttatcat tatttcaaca tttattcaac 47280
   atatatatat actctctcta tatatgtata ttttttgagt gcctatttgt gaaccaggta 47340
   ctgttctaag tgctaaagtt acagcagtga acaggacaaa gtcctcatct acattctaga 47400
   gggaaagata cacacatgca catacacaca cacacacaaa ataaataata aatactataa 47460
   tttcacttag aagtataatt gacagtagtg gggtatggct aggaggtgat aggaggtgca 47520
   ttttatgtag gatgtcacca agaaagtgac atttgaggag agactcccag taatgtaaag 47580
   gagtgaatca ttcagatatt cgaggaaaga gcactccagg ccatgggaac agcaaatgca 47640
   aagtccccaa ggctgaaatg cacttggttt gtttggctgg agtggagagt gcagggtaga 47700
   gagtggcagc gagggaggac agaggttggc caaccacatc acgcaggcca tggcaaggaa 47760
   ctgagatttt attctaagca tgatgaggag cattggagag ttttgagcag agtaggcaca 47820
   tggtctgagt tactgtcata aaggattatt gtagttgctg agtgaagaac tgacttgggc 47880
   aaaagtggaa gccgatggag tagttcggag gcattgcagt gttttggcag aaagatgagg 47940
   ataacctata ctagggggtt agcaatggag atagcaagaa gtggtcagaa atagtatata 48000
   ttttgagagt agaattgata agacttgctg ttagttgcct gtctgtggag gtgtgaaggt 48060
   aattaaggat tcagagatac ctcttagatt tttggcttga gcaactgagt gaatgctggt 48120
   gccatgtact gagacagaga aggctgtgag gagagctggg tttgagggaa agataaggac 48180
   ttcagttttg catatgttaa gtttgagatg cttattagat atccaaataa ggatgttaaa 48240
   taagcgattt tgtgcatgaa tctggagttg aagggagagg tctgggctga agatctaatt 48300
   ttgggaatcc tcggcatgtg aatggattta aagcctaaag accgaaagga gtgctcataa 48360
   agtgaatgag atagacaaga agacaaaaaa ctgaatcctg gggcacatcc atggacactc 48420
   actgggagat ggaggcaagc tataagattg aaaaatggag agaagtagga aggaaaaaga 48480
   aacagaggaa tgtatcctgt tctcagatta ttcttttcaa aattatacag agcaagcgcc 48540
   catctggtct gttaaaagta gagtagcata ttatttagat gatagggtat aagtaattca 48600
   tttattctga tggtccctgt gctccaccta tgggcaagag tagaacttaa tgcccttaga 48660
   acattgattt ttgtgtaccc tattttagaa atttcttcac aactgccttg tcaataaaga 48720
   tgacttgtgt accatgtctt ttgtccatgc cttcttttaa tatggtggta tttatgtggg 48780
   acactcctat ggactggaag gtttgaatca aatgtctctg ttttcttaaa ggctcaagat 48840
   tattatattc cctccaattc ctgatcctgg caagattttt aaagaaatgt ttggagacca 48900
   gaatgatgat actctggtaa gaacagattt catggggctt gaaatgtttt tttttttttt 48960
   tttttttgga agctatggca ttttttaaaa aagtgtggta aggtatccat aacataaaat 49020
   ttattatttt aagcattttt aagtatacag ttcaggctgg gtatggcagc tgacgcctgt 49080
   aatcccaaca ctttgggagg ctgaggcagg aggatcactt gagcccagca gtttgagacc 49140
   agcctggaca gcatgccact gcacccagct aatttttata tttttttgcg agatctcatt 49200
   tccacaaaat atataaaaat tagccgggtg cagcggcatg cacctgtggt cccagctact 49260
   caggagattg aggtgggagg atcccttgag cccaggagtt caaggttgca gtgagccatg 49320
   atcatgccac tgcattccag cctggatgac agagcgagac tctctaaaaa ataaaatata 49380
   tatgtgtgta tatgtgtgtg tgtatatata tatatatata tatatacaca catacagttt 49440
   agtgtcatta aatacattta taagttgtac aaccatcgcc actatctctt tccagaaatt 49500
   tttcatcatc ccaaatagaa actgtggctg ttaaacaata attccccatc ccctcattcc 49560
   tcccacccct ggtagcctct attctacttc ttgtctctat gaattaaata ttctagatac 49620
   ctcctatatg tgaaatcatg caataatttg tccttttgtg tctggcttat ttcatttacc 49680
   ataatgtttt aaaggttcat tcatgttgta gcatgtatca gaatttcctt ttgaaattgg 49740
   ttcttctaaa gtacaattta tttttaaaaa tttgaaggag agctgattta aaatatagta 49800
   tctgaggcca agcataccag ttaaaaagtc ttcagaatac tttgctcatc ttatataact 49860
   gggctccatt ctctctctgg ttaggagaca tattttatca aaacatgcag gaagaagggc 49920
   ctcctttatg aagctagcct aatgagcagg ccttctgact cttggataat ttaaaaaact 49980
   tgatgtgggg aaatataaac atagggtaag caattgaaat gcattgtgtt catgttgttt 50040
   cttggaaggc tagactccaa agatatagaa atggagcagc cctaccccaa ctgttatagt 50100
   tggtttcctg cagattctca tgctagaatt tgtggccaga aaacctaaga cattatggga 50160
   acaaaagggt ttgaggcttc taactttagt tggatttatc atccacccta ctgaaataat 50220
   aaagacacta aatgaaataa atattattat cattgtgtat atcttaaagg aataataaca 50280
   agcatagggc agttaattta gggaagtagt actgcatagt agataatagc ctgatttctg 50340
   gagtcagatt gtctggattc atatccctgc tgtgtcattt accatctttt tgaccttgga 50400
   taattttctt agcttctctg tgactcaatt ttttcattag taaaatggag gtaacaatag 50460
   cacctgcctc atagtgttgt tatgtggatt aacttaatac ccaacagtta attgaatgga 50520
   aactgcttag cctacctggt accttgtaag tgcttaataa gtgttagtga ttagtattac 50580
   tattgataat aatttatttt tatctttaat tcccaaataa ttattgtaat aatgggtgat 50640
   tctcaatggt agtgatgata tttggttagg gaaggaaggc agagaggcaa atatgctatc 50700
   agaagaggat tcatttggat cactttttaa ccaggtcatc ttcacagaca gtagataact 50760
   ctgccagacc ctgggagtca gggcatgtag acccagaagt taggtacctg atgaggaaat 50820
   tgtatgccgt atctggagcg agtatgatag ctatggttct ccaactttag ttgagtcaca 50880
   gtcaactgga gtgcttacaa gaaatacaga tttctggacc ctacttccaa ttccctgacc 50940
   tgacctgatt tggcaggtca gggacgagtt ctaggactct gcatatttaa taggagccca 51000
   cagatgatcc ctgtcctgct aagagagtct gtggaccaca ctttgaaaat cagtgataaa 51060
   gatatttacc cctgtctctt acttcatttc accagctgtg atagatgctg gaggttggga 51120
   attgtacaag taccctaaca actttcacca gctattgaaa gtctactatg tgtcagacaa 51180
   tgtattaagt gctttgccta tattacctta ttgaatcttc ataaccaccc tatggagaag 51240
   atgctgttgg tattatttta cagatgagga aatggagccc caatgaggtt aagcaattta 51300
   gctgcagtca cacagctagt tggtggtaag gtagaatttg acccagggct gtcttaatgc 51360
   tagacttctc ttggtctttc tactaggctg cattgcctct tgtaagatag aatgcttgtt 51420
   ctgaagaacc tcaccatctt ctaagacatc tgtatgacag aagtagaggg ttggcatagg 51480
   ggatttctga gaaaaagatt tagggttgta ttatgaatag ctttaaatgg gaagatattg 51540
   gcatgtcttg tatatattaa tggcactttt catgcactgc tggtgggcct gtaaaatgat 51600
   agatatcctt tctggagggc aaactggtaa aatgtaacag gaatctttga gcctatgatt 51660
   atacttttag gaaccaattt tgaggaaatg atccaacaat ggcacaagag tggtcaatgc 51720
   ggcatttgct tttaacatga aaaattagaa gccaatgaaa cataacaatt gagaattagt 51780
   ttaaaaaaat atgttaggtt ttcaaagcat ttaatgttat aacaaaatac tcaaaataca 51840
   ggagtaagaa acttaccata aacatgtagt atattacaat cataattttt atcaaaaatg 51900
   cattaatata tacatagaac gaggggaagg aaatgggtcc gaatgaatga tcatttctag 51960
   gcaaagggat tgtgggtaac tgatattact ctctctctac ctttctaaaa tttccagatt 52020
   gcctatagtg agcatgtact acttttataa taaaagaaac cctgtacaag tcattaaggg 52080
   aaaaagaaaa tactgtagtc atccaggtct gagagataat aggaacttga acttaggtgg 52140
   tagcagagga gattgacgta agagacatta ttaaaggaaa tcaactcatt atgacttggt 52200
   gagtctgtgt gggtgatgag aggtagggag agtaaaaaat aatttcaagt tttcaagtct 52260
   gagtaataga gtgaatgatg gtaacattga cagcaaaatc agatcaatta tctttctcag 52320
   agatgggaaa attgcttttt tcaatttgct gtctagagga taatatgttg tttttgtccc 52380
   gatgagtcag ataattctgt aatttggtga cgcaaatata ctgacaatat ggaccattaa 52440
   aagcacaagg tatttttgtt ttgtccattg cagaccatta aacaaaatca gttctttaaa 52500
   actaaaagta taataaaaat tccagatatg gggaagggta agagaacaaa tttttatgtc 52560
   ggcttttaac caaatgaaaa aaatctgctt tcttcacatg gcatcaattc tcacaaattg 52620
   aactgcatat gctgcatact cttttacttc tagcatcatt gaattattga atgaacatag 52680
   ccctagaggt catctaatga ggtggatgtt gacatgttta attatttccc tttaattata 52740
   aatgttcaaa actcgagatt ctcactcatc tccctctctt tagagatggg gacatgagga 52800
   gccacagaga agtttctcaa ggactgtcac tgtgttaagc caatcttgta ttgctataaa 52860
   gacatacctg aaactgggta atttataaat aaaagaggtt taattagctc atggttctgc 52920
   aggcttcaca ggaactgtgg tgccagcatc tgcttctggt gagtatctca ggaagcttac 52980
   agtcatggtg gaaagtgaag ggggagcagg catcttacgt ggtgagagtg ggaacaagag 53040
   ggggtaggag gtgccataca cttttaaaca gctcttatga gaactcactg ttgtgatgac 53100
   agcattaagc catgagggat ccacccctat gacccaaaca cttcccacca ggccacactt 53160
   ccaacgttgg ggattacaat tcagtatgaa atttggcagg gacatatatc caaactgtca 53220
   gtcacataga gaggcaactg caaaacctgg actagaaccc atatagccca aagttaccaa 53280
   cttccaatcc aaataaataa tcttgtcctt ttttaaacac tgtcttcttc cacactgcac 53340
   cattaatagt tgttcttata aaaacttatt tagttatggc catgaataag ttaaatcttc 53400
   agtatagttt tccttaaagt tactcgtgtt ttgatgatgt tttgttcatt actatagtaa 53460
   tgttttggcc aacagcattt ctttctgttg ctttgctatt tgccttttcc ttgtgactaa 53520
   ttgggcttct ggccatattg aggggaggta aggggagttg cagagggaat tgcttctcct 53580
   ctctatctgc atttctctta tccataaaaa aaaaatctgt cctccccctg cttcttggaa 53640
   accaattctt attttcagca ttgtgccatt tttatattct tgttcagaaa ggagcttttt 53700
   aaacatgggg ataacatcca aatactgctg attgttattt tttacttcag tgtgtatctt 53760
   ttttttttcc tttttgagat ggagtctcat tctatcactc aggctggagt gcagtggcac 53820
   gatcttggct cactgcaaac tttgcctccc aggttcaagt gattctcctg cctcagcctc 53880
   ccgagtagct gggactacag atggtgccac catgcctggc taatttttgt aattttagta 53940
   gaaatggggt tttgccatgt tggccaggct ggtctcgaac tcctgacctc aggtggtccg 54000
   cccgccttgg cctcccaaag tgctgggatt acaggcgtga gccaccacgc ccagcctcaa 54060
   tatttatctt taaaagctaa gaatgctttc ctacatagcc aaaatagcat tactgtactt 54120
   taaaaattat taattcctta atttcatcta atactcagtc catattcaag tatttccaac 54180
   tgcccaaatt gtcaggagac agatattatc cagtcatctc accactgacc actaagacag 54240
   tggttgagat tgaccactat atttaaggtg atacaacagc atgtcccctc ctgccatctg 54300
   cacttatttc cccattgctt caacacagat aatctgtgta gtgttatgct agcaccttgt 54360
   caagtcttca gctaatgctt tttacatcag ttgatatcct tgcttgaatc agtaatttcc 54420
   ttaggggttg ggttgaatat tacagttttt ctctttctat catttctaca tttacttttc 54480
   tttttttttt cttttgagac aggatcttgc tctgtcaccg aggctggagg gcagtgatgc 54540
   aacctctgct cactgtagcc tcgacctcct gagctcaagc agttttcctg cctcagcctc 54600
   ctgaatagct gggactatag tcacgtgcca ccacactcag ctaattttta aatttctttt 54660
   gtagagacaa ggtctcacta tgttgcccag gctggtcttg aatttctagg ctcaagcgat 54720
   cctcttgcct cagcctccca aagtgctggg actacaggca tgagtcaccg tgcctggccc 54780
   atttctacac ttattagctg acaggtatct ataaagcgct gctttcagtc atcaactgag 54840
   ctctttagtt gttctgaaat tcagttccta ctaaaaaggc aagatgcata tttaatcatt 54900
   tccctttatt tatccattaa ggtgaggagt tggattaata gacacctcat agtaccaagt 54960
   tattcctctc cataccacca cattcaactt ggtgtttttt tgtttttgct tttttttttt 55020
   gagacagcgt ctcactgtgt tgcccaggct ggaatatagt ggtgcaaaca tggttcactg 55080
   cagcctcaac ctcctgggct gaggcaatct tctgatctca gcctcccaag tagctgggaa 55140
   tacaggtgtg cactaccacg cccagctaat aatttttttt tttttttgta tgaatgggct 55200
   tttgccatgt tgcccaggct agtctcaaac tcctgggctc aaatgatctg cctgccttgg 55260
   cctcccaaag tgctgggatt acaggcatga gctaccgtgc tgagcctcaa cttgttcttt 55320
   ttcaagttgc aatgatttta aggtgtggag atctacaatt cattttgtat aactttctgt 55380
   tttgaatgat aatctgaaga gagactgggg catgaaatag gtagacttgt ataccaaagg 55440
   ccagtgtttt tgcatggggc taaggtacat gaagctcact tcacttctgt agacaatcca 55500
   ctctggaaaa tatcttaacc aaatcttgaa agtaatctta agttgagact tgatatttca 55560
   ttgtactacc aaatttatgc ctttaaatta taaaaaccta tataacatgc cgtttttcta 55620
   ggctcacata attaccaact aaaagccagt tgtgtaagcc ccctaatcac tgattttctt 55680
   catctatgat gtttttcatc tttggccatt actctgttgt tgattacttt gtttttaaaa 55740
   ttggcagtaa ggattgaatt ttgggctggt aattgtgtgg atttgtttat ttttttctac 55800
   aagtggtttt aaaattagac aggcatataa ttcacagaat tgaataatag gaaagagcca 55860
   ttgcatttat ttcttttacc ttacatggac atattcatag aagcagtact caggaaaagg 55920
   ccactgtgtt gataaagggt ggaaatgaga gtaattcaaa ttttggggat gaaaaaaggc 55980
   aatcagaaag attaatgtat tctgagaagc cctttctgtt gtagtgttca cctcattaaa 56040
   acaaattgct tttttcttgt ccagcacagc caagtgttgg tctaatgacc ggaaacaaaa 56100
   gtaattttca gggtgcattt ttaatagtta tacagttttt atttgtgaaa aatccatatt 56160
   acacacacac acacacacac ttcaccacta tacattcatg cattttttaa attaaaactt 56220
   tttttttttt tctgatcact tgggaggatg aggcaagcgg atcacctgag gtcaggagtt 56280
   cgagaccaac ctggccaaca tggtgaaacc ccgtctacta aaaatacaaa attgaccagg 56340
   catggtgatg catgcctgta atcccagcta cttgggaggc tgaggcagga gaatcgctta 56400
   agcctgggag gcggaggttg taatgagccg agattgagcc actgcactcc agcctgggca 56460
   acaaaaatga gactctgtcg ccaaaaaaaa aaaaaatata tatatatata tatacgtata 56520
   tatgtatata tatgtatata tatatatata tacgtatata cacatatata cgtatatata 56580
   ttcttttaaa agcaatatac atttagtata gacaaattag aactacagat agtcaagaga 56640
   gaaagacaaa atcactctta atcctgtcac ccagatataa ctatctttaa tggtatgtga 56700
   acaacatagg gaaactgaaa tacctttagt gataaatgga gtaatagcaa ccacaataat 56760
   aatagttcat acttatggaa cctttactat gtgtcttaat aacactgtca tattgaattg 56820
   tatttgaatg taacaaatga gaattaaaag aatttctgaa cttttgatat aaaataaatt 56880
   ttaatgatga ttgaaaagat atttttccat atgctctccc catttgctcc ccatttttat 56940
   agttgttgaa catctccatt gtgtcctcat atagagccct catttagtct tacttcagtg 57000
   tgtggagata cacttgatgc tcactaccat tttttatgtc aacattgctc cagtcatttg 57060
   ggttgtttga agctggaaag ttttctcagg aagtattcat gggaccggca tttcctgaat 57120
   tatttacaaa ttgatggaat ttgctacatt tatcattgaa agtcattttg gcaaaatata 57180
   aaatccatgg ctcataggtc ctgcgttgcg tatcttaaat gtgtcatcca ttttcttctg 57240
   gcataatatg ttttgtcaga gtcagcagac aatataattt tcctcctgta ttaaaacact 57300
   tggtcttttt ggctggttgt caaatgattt ttttcttcaa agtccaataa ttttaccaga 57360
   atatgtgttg gcgttgtgat tttttgcagt gtatggtgtg ctttctcagt atgtaatttt 57420
   agatcttttt ttttttaatt ttagtaaaag tttcttcact tacagatttt agttattctc 57480
   ttcccatgtt ttctttgacc tttcacatct tttttgtttt ggcacagggt cttgctctgt 57540
   tggctgggct ggagtatggt ggcaccatga tagcttattg cagactccaa catctgggct 57600
   caagtgatcc tcccaactca gcctcctgaa tagctgggac aggcatgtgc caccatgcct 57660
   ggctaatttt taaatttttt ttagagatgc agtcttgctg tgttgcccag tctggtcttg 57720
   aactactggc ttcaagttat tcccccacct ggaacttcca gagttcagga attataggtg 57780
   tgagccgctg cacccagccc ttccagccct ttcacatcct cttgaataat tttcatccac 57840
   cttcatttca tttttgttaa aaaatcctac tttttaatct atttaagatc ttatctgatg 57900
   atatgttgtg tttatttgcc ctgtgtttct tctagcttag tcttcaattt tgaaatgatt 57960
   attttccttt acttttattt tgttttattt tttgagatgg agtctcactc catcgcccag 58020
   gctggagtgc agtggtgcga tcttggctca ctgcaaactc cgcctcccag gttcaagtga 58080
   ttctcctgcc tcagcctccc aagtagctgg gattacaggc acatgccacc atgcctggct 58140
   aattttgtat tgttattaga gatggggttg caccatgttg gccaggctgg tctcgaaccc 58200
   ctgacctcaa gtgatccacc caccttgacc tcccaaagtg ctgggattac aagcatgagc 58260
   cactgcgccc agccttcttt tacttttaat tcttctgtga gttttgtcat ccaatttctg 58320
   agttgtttat attgactttt aatatattct tctacattga ttactttttt tatatcatgt 58380
   aactttttaa atatacttca gctagttttg aaatattagg ttgtagtttt catctatttt 58440
   gtggatatat cgttctgtta tgttttaatt ctgtaagagt attattctgt ggctcatttt 58500
   tattttattc tttaatttta aacatttttt attttaaaaa taattgtcac tttgggaggc 58560
   tgaggcagac agatcacaag gtcaggagtt caagaccagc ttggccaaca tagcaaaacc 58620
   ccgtctctac taaaaataca aaaaaattag ccaggcttgg tggcgggtgc ctgtaatccc 58680
   agctacttgg gtggctgagg caaggagaat cgcttgaacc tggaggcaga agttgcagtg 58740
   agccaagatc acgcctctgc actccagccc gggcaacagt gtgagactcc atctcaaaaa 58800
   aaaaaaaaat tattattttt gtagaggtgg ggtttcacca tattgcccag gctggtctca 58860
   aattcctggg ctcaagcaat cctcctgcct tggcctccca aagtgctggg attataggcg 58920
   tgaacaacca cacttggctt ctgttgttca tttttgtatg aaatcagttt cctgaacttt 58980
   tagaaggagg cttgattgag acaagttgtt tctttcccta attttatggc tgtagagctc 59040
   catcctctaa tgaattacat tctgaaatct cctggccctt tctgtctacc tttctgcttt 59100
   gatctaggct ttcttttttg tcgctcttgt ccctgtcctg actaatttgt aatccagttt 59160
   caccagtttc tcctcagagt gcagctttgc cctggaaggg attaaggatt agttttgagt 59220
   ttctggaggc ccaggctgct ttcagtctag accacggact ccttgcactc aatcactgtt 59280
   tgaaaatgca aacccttccc tttatcagtt gttcataaat ttgcttgctg aagttgagtt 59340
   attttacagt tctcagatct ataggatgcc atgttgagtc cgtttatata ctcttccaca 59400
   gcttatacca ttttggtcct gtaactatct ctaatttgtc cacattcact cttactttga 59460
   ggttcatggg ggaaatgttt tcatcttgtt ttgttgtaga tttcatcttg tgttggtaga 59520
   ttttggattt actttcctat tgggggaacc agcccccaat atttcaacgt aagttctttt 59580
   ctattttccc taagtgtcag ccggtctgat aaataaaggg aaagagtaca aaagagagaa 59640
   attttaaagc tggatgtctg ggggagacat cacatgtcag caggttccat gatgccccct 59700
   aagccgtaaa accagcaagt ttttattagc aattttcaaa ggggagggag tgtacgaata 59760
   gggtgtgggt cacagagatc acatgcttca agggcaacaa aatatcacaa ggcaaatggg 59820
   cagggcaagg tcacaaggcc agggcgaaac tagaatttcg cctggttcct ggtcctgctg 59880
   tgcatgcatt gtcattgata aacatcagga aacagggttt gagagcagac aaccattctg 59940
   actaaaattt actaggcaga aatttcctaa tcctaataag cctgagggtg ctgcaggaga 60000
   ctagggcgtg tttcatctct atctatgact gcataaggca gacactccta gagcagccat 60060
   tttagaggcc tcctcctggg aatgcattct tttcccaggg ctgttaattg ctaatattcc 60120
   ttactgggga aataattcag tgatatttct cgtacctgtt tttggcaata agagaaatat 60180
   gactgtcctg cccggctcca ggcagtcaga cctaatggtt atctcccttg ttccctgaac 60240
   attgctgtta tccttttctt ttttcaaggt gcccagattt catattgttc aaacacacat 60300
   gctttatgaa caatttgtgc agttaatgca atcatcacag gggcctgagg cgacatacat 60360
   cctcagttta tgaagatgac gggattaaga gattaaagta aagataggca taggaagtta 60420
   taagagtatt gattggggaa gtgataaatg tctatgaaat cttcacaatt tatgttcaga 60480
   gattgcagta aagagaggcg taagaaatta taaaagtatt aatttggaga actaataaat 60540
   gtccatgaaa tcttcacaat ttatattctt ctgtcatgac ttcagcaggt ccctccgttc 60600
   ggggtccctg acttcacgca acactttcct atttatattt ccaacttgtg tttttttatg 60660
   gtgatatttg ggaatattca aaaacgatgc tgctaccacc atcttcttcg cagaattttt 60720
   ctgcaaacta tagaccaagg ggcaaattct gcctgccact tgtttttgta cagtctttaa 60780
   cctaagaatg gtttttgtgc ttctaaatag ttgggaaaaa aatgtaagaa tactatttca 60840
   tggcacatga aattaagatt tcagtgtcat acagtttcgt tggccaggcc cattcattta 60900
   tatattgtct gtggctgctt tcatgctaca acagcagagt tgttttatag gcacagagac 60960
   tatatggccc ccgaagctga aaatacttct tatctagcca ttttcaggaa aagttagtca 61020
   accctggtcc cctataatat agtttttagt ggctaaataa tattccattg tgtgcacata 61080
   ttataatgta cttaacaaaa ttcctattgg catttggatg aatcctaaat ttcattgtat 61140
   aggcatcatt tcacagctaa gtataagcat atgtattaat attttattac tgtcttagga 61200
   taaattgtta caagccaaat tgcatgatca agggggaggg tatatgtatt ttaaactctt 61260
   tgactgatac aggctgagta ttcctaatct gaaaatctaa aatccgaaat gctcccaaat 61320
   ctgaaacttt ctaacatgac gttcaaagga aatgttcatt gcaacatttg gaatttcaga 61380
   tttttgcgtt agtgatgctc agctggtaag tatatagtgc aaatattcca aaagctgaaa 61440
   cacttctggt cccaagcatt ttgcataagg gatattctac ctgtattgac aaattgtcat 61500
   ccagaaaggt tgtacaaatt aaattcccat taaggaaact ggctaaataa gttatggtgc 61560
   attcatgcaa tgaaatatta tgtagaggtt aaaatgaggt agatcacttt tactgatgta 61620
   taaaaatata acatacactg ttaggtgaaa cacccaggtt acatatggag agtatgattc 61680
   ctttgtgtta aggaaactgg ggcattaaaa tgcacaaaca aattttttca taagaagcat 61740
   agattacaac taagttgtgc cagttcatat acctaccagc agtatgttcg actggttgtt 61800
   tgaccatact tttgccaata tgatattacc taattttaaa attcttgcca agttaataga 61860
   tgagaaaaga gcgtcttaat tttacagtca ggttttgtac tttttcccaa gtttattggc 61920
   ttgaattaat tcttttgtga aattcttgtt ccacgtccat ttttccttgt tgtattcatc 61980
   ttttggtggt ttgtaaagct ctttacgtat tagatattaa ccctctggct tcttatccat 62040
   gttttccttt gtattgtatt tagggtgatt aatgatgtac aaaaggtttt tcctttttta 62100
   tgcagtcaaa tcttcctgta tttttaggtg taaagagccc tttccacctt agatttatat 62160
   agttgttcct ctacatattt ttctagtact tatttgattt tttttaacat tgagatcttt 62220
   aatccatcag ggatttattc atgtaggcta tgaggtaggg tttagcattg ttttcaaatg 62280
   cttatattac ccacttgtct tagtgtcatt tattaaataa gccatttttt caaggttata 62340
   taattttttc aaattgtttt tattagaaat ctatacattt cacatgtttt aaaatcaagt 62400
   ttgaggtgta atttacaata aaatttactc ttttttgagt gttcagttag aagaatgtta 62460
   gcaaatatat gcaattttgt aaccactgct ccattaaaaa tatagaatat ttccatcatc 62520
   tcctaaaagt tatcttgtag tcctttgcag tcaaccccct ctgttaatcg ccttgcaaac 62580
   actgatcagt tttctgtccc taaagtctta caagatgtcc tataaatgaa atcacacagt 62640
   acatagcttt ttgagtttgg cttcttttac tcagcataat gtgtttaaga gacatccaca 62700
   ctgtggtgtg tgtcagtagt tcatttcctt ttattgctga gcagtattct ataatatggt 62760
   tgtaaccaca gtttatttaa ccattcactt gttcaaggac atcagagttg tatctagttt 62820
   ttaatctatt attagtgaaa atgctataaa cattcatgta gaagtctttg tgtgacatgt 62880
   tttcatttct cttgggtaaa tacctgggag tgggatttct gggtcatgtg gtaagtgtat 62940
   gtgttatttt ataaccaagt gtcagactgt tttccataat gattgcacta tcttattttt 63000
   accaacaata catagagatc tagtttctct gcgttcttat gagcgcttgg aattgtcggt 63060
   atttttaatt ttagccattc tagtaggtgt gtcagggtta tataatttgt ttagaataat 63120
   ttgcatattt tgagcagtgt ttcccaactc atcccctcat ctcttaactt gcccagtcca 63180
   tcttcagttg cacaacctga ggcagctctt gtttctgttt ttcttctttc catttctttt 63240
   ctaagaacat ctgtgcagaa aacgagctta gacataacct aatgcagggt ttggtagact 63300
   tttttctgta aagggccaga taataaatga ttttgttttt gcaagccatt cagtctctgt 63360
   cacaactcaa ctctgccatt ataacatgaa tgcaggtgaa ggcaatatga aacaaagacc 63420
   agcattcagc cggatttgac ctgtggacca tagtttgctg acccctggtt tagtataaca 63480
   actttatttt acagatgaga ggaccgagct catgtttcac cctttcctct gatttacaag 63540
   taaaagtaaa aaaaaggcaa ggaccaggat attggctttt accataagag acatgcttga 63600
   attgggttca agataagtgt gctttatgtg taatttcctg ttctagcaaa tgatgttaga 63660
   tgaagctctt aatatagggt ctttcggcaa tttcgattgc agtaagactt taggaacatc 63720
   ttatgtttta ccattcatct caatgctact tgactttttg aacctattat gtctctggag 63780
   caatgagcat taagaaaata aaggtaataa aggtctcagc aaaacagttg gagtaagaac 63840
   ggcatgacat ccttttattc ttcttaatag attttatttc cagtggtgga aatgtccaaa 63900
   tttgtttatt tctggagata gtcattgtac atcatgtaac tatctcatga ttcccacctc 63960
   catttaatat taattcctga ttgttctgtt tcattaatca ttcagataga taaagattct    64020
   taggtcaaaa aaaaaaaaaa aacaggaaat cataccccta cggttccatc cacacaaacc    64080
   taaaaataag cattggcact aagtactcag ggaaagaaca ctgtttagat atgtcattgt 64140
   gtatgtgttt ttttcctccc ttctagcact ggaagaagta cgacatctat gagaagcaaa 64200
   ccaaggagga aaccgactct gtagtgctga tagaaaacct gaagaaagcc tctcagtgat 64260
   ggagataatt tatttttacc ttcactgtga ccttgagaag attcttccca ttctccattt 64320
   gttatctggg aacttattaa atggaaactg aaactactgc accatttaaa aacaggcagc 64380
   tcataagagc cacaggtctt tatgttgagt cgcgcaccga aaaactaaaa ataatgggcg 64440
   ctttggagaa gagtgtggag tcattctcat tgaattataa aagccagcag gcttcaaact 64500
   aggggacaaa gcaaaaagtg atgatagtgg tggagttaat cttatcaaga gttgtgacaa 64560
   cttcctgagg gatctatact tgctttgtgt tctttgtgtc aacatgaaca aattttattt 64620
   gtaggggaac tcatttgggg tgcaaatgct aatgtcaaac ttgagtcaca aagaacatgt 64680
   agaaaacaaa atggataaaa tctgatatgt attgtttggg atcctattga accatgtttg 64740
   tggctattaa aactctttta acagtctggg ctgggtccgg tggctcacgc ctgtaatccc 64800
   agcaatttgg gagtccgagg cgggcggatc actcgaggtc aggagttcca gaccagcctg 64860
   accaaaatgg tgaaacctcc tctctactaa aactacaaaa attaactggg tgtggtggcg 64920
   cgtgcctgta atcccagcta ctcgggaagc tgaggcaggt gaattgtttg aacctgggag 64980
   gtggaggttg cagtgagcag agatcacacc actgcactct agcctgggtg acagagcaag 65040
   actctgtcta aaaaacaaaa caaaacaaaa caaaacaaaa aaacctctta atattctgga 65100
   gtcatcattc ccttcgacag cattttcctc tgctttgaaa gccccagaaa tcagtgttgg 65160
   ccatgatgac aactacagaa aaaccagagg cagcttcttt gccaagacct ttcaaagcca 65220
   ttttaggctg ttaggggcag tggaggtaga atgactcctt gggtattaga gtttcaacca 65280
   tgaagtctct aacaatgtat tttcttcacc tctgctactc aagtagcatt tactgtgtct 65340
   ttggtttgtg ctaggccccc gggtgtgaag cacagacccc ttccaggggt ttacagtcta 65400
   tttgagactc ctcagttctt gccacttttt tttttaatct ccaccagtca tttttcagac 65460
   cttttaactc ctcaattcca acactgattt ccccttttgc attctccctc cttcccttcc 65520
   ttgtagcctt ttgactttca ttggaaatta ggatgtaaat ctgctcagga gacctggagg 65580
   agcagaggat aattagcatc tcaggttaag tgtgagtaat ctgagaaaca atgactaatt 65640
   cttgcatatt ttgtaacttc catgtgaggg ttttcagcat tgatatttgt gcattttcta 65700
   aacagagatg aggtggtatc ttcacgtaga acattggtat tcgcttgaga aaaaaagaat 65760
   agttgaacct atttctcttt ctttacaaga tgggtccagg attcctcttt tctctgccat 65820
   aaatgattaa ttaaatagct tttgtgtctt acattggtag ccagccagcc aaggctctgt 65880
   ttatgctttt ggggggcata tattgggttc cattctcacc tatccacaca acatatccgt 65940
   atatatcccc tctactctta cttcccccaa atttaaagaa gtatgggaaa tgagaggcat 66000
   ttcccccacc ccatttctct cctcacacac agactcatat tactggtagg aacttgagaa 66060
   ctttatttcc aagttgttca aacatttacc aatcatatta atacaatgat gctatttgca 66120
   attcctgctc ctaggggagg ggagataaga aaccctcact ctctacaggt ttgggtacaa 66180
   gtggcaacct gcttccatgg ccgtgtagaa gcatggtgcc ctggcttctc tgaggaagct 66240
   ggggttcatg acaatggcag atgtaaagtt attcttgaag tcagattgag gctgggagac 66300
   agccgtagta gatgttctac tttgttctgc tgttctctag aaagaatatt tggttttcct 66360
   gtataggaat gagattaatt cctttccagg tattttataa ttctgggaag caaaacccat 66420
   gcctccccct agccattttt actgttatcc tatttagatg gccatgaaga ggatgctgtg 66480
   aaattcccaa caaacattga tgctgacagt catgcagtct gggagtgggg aagtgatctt 66540
   ttgttcccat cctcttcttt tagcagtaaa atagctgagg gaaaagggag ggaaaaggaa 66600
   gttatgggaa tacctgtggt ggttgtgatc cctaggtctt gggagctctt ggaggtgtct 66660
   gtatcagtgg atttcccatc ccctgtggga aattagtagg ctcatttact gttttaggtc 66720
   tagcctatgt ggattttttc ctaacatacc taagcaaacc cagtgtcagg atggtaattc 66780
   ttattctttc gttcagttaa gtttttccct tcatctgggc actgaaggga tatgtgaaac 66840
   aatgttaaca tttttggtag tcttcaacca gggattgttt ctgtttaact tcttatagga 66900
   aagcttgagt aaaataaata ttgtcttttt gtatgtca 66938
<210> 33
   <211> 42218
   <212> DNA
   <213> Homo sapiens
<400> 33
   gcgagcgcag cggagcctgg agagaaggcg ctgggctgcg agggcgcgag ggcgcgaggg 60
   cagggggcaa ccggaccccg cccgcaccca tggcgcccgt cgccgtctgg gccgcgctgg 120
   ccgtcggact ggagctctgg gctgcggcgc acgccttgcc cgcccaggtg ggtgactcgc 180
   gcggcccacg ggggacagcc gccccgcatg tccacccggc tggtgcgcag ccttcgggtg 240
   cccgggccgc gctctcccgg ggcgctgtca cgggctggga ggctgggagt cccagtcgcc 300
   gccccccatc cgcatcagac acgcgcgcct ctggggaccc gctggggact ccgggcccgg 360
   cacacgtgcg ctcggggcac aactctggcc cccgaagccc ctcttacccg cgtcgtgaca 420
   ggggcgcacc ctggttcctc cgcgagcgca ggccggggca tttggggctg agcagcgggt 480
   cctgggcaga cccccgctag acccgggacc cctcttccct caccccggcg actgccgcga 540
   cagcccttcc ctcccacgcg gtggagaagg ggctgtgccg gggcgctgcc ccactgccca 600
   cgccgggcac cctcttcaaa agacttcctt ttcctctggg cgacggtttt ccgatttctt 660
   agaaatacca gggtcctgtg tctgaagagc aggaccaggt gtgtgtcagg tccactgagg 720
   gcacagctgg agggcgagct gcctgtctgc ttcctccgat gcgtccgggg gctactgcag 780
   agtctgtttt ctgcggcctc tggggacagg gcaagcgagg gaggtctggg ctggtaatcg 840
   agcaaggcaa atgaagtctc cgggggccgc aggaaaatgg agacagcaga aaccctaggg 900
   ctcctattca atatgaacgt cactcccaca gtagcaacgg gcgtacaagg gactgtgcac 960
   cctttgcctg tttttgactg tctcctggtc tggtaacata ggataacccc atttttgttt 1020
   tactgtctcc atggtttggt aagataagat aaaccatttt ttagagaagg aaaactgagg 1080
   ctcagagaac ttgtatttca tggaagggtc tggggggaac tcttcctcca aagggggcct 1140
   ctcctgcctt cggcccctgt gccctgaggc tgcggggaag gtgggcgttc atccttcctc 1200
   agagccgccc ctgatggaca catggcccat ctgcccccat ctgacatctt gaccgaggag 1260
   gaaggtggga ggtggcactg gcttctgtgg tgacataagg tgacttgttg atctgaggcc 1320
   agagagtcca gttcttgtgg gtggaagatg ggggctgtca gactagaatt gactcaccga 1380
   cagtgggtgt caggagtggg tcctggagaa ctgccccaga gtaagtcctg ggtggccttt 1440
   gggtcaggag agtccccact ctagggccga gtgtgaatgg ggacgaccgt ggtccccagc 1500
   ttgactttgg ggtcatgcca tatactctgg cctcagaggg ggtcttcctg ccgctgagct 1560
   tgggcctgcc tgggtgaact ctggggtcat gtgagcccct ggatgggaga ggggtgtcag 1620
   gcacagggct cagcaggaca ccaggctgca gtcctggagt agctggagga ggttccctcc 1680
   cacttccttc ccttcctgac cagctcctgc ggcagctcat attccctcct ccctcatccc 1740
   tgctccccac gaccccaccc ggactattgc cgcagccttg tagctggtct cccggccctg 1800
   ccccctctcc cgctgatcct tggcagagag acccttctca agcatgaggc acctctctgt 1860
   ttgcctgggg agcctcctgt ggctcccaat ggttgaggca gaaactcccc tggagatccc 1920
   aggccagagt aaggtacctg aactcttcct cgcgcctccc tgcacatgtg ctcccccgcc 1980
   tctgtagaac tgatgatggt catcgtgtgt gtcattgtgc aaaaagcatt gtccgtgcca 2040
   tcccctctcc aagggacact tcttacccct atccttcaag gccttctgca aatgccattc 2100
   tctccaggaa agcctttccg cattctcagt tggatttgat cttctcttcg aactcccagg 2160
   cccttttgtt ccctttgagg acagattgca ttctgctttt taatctagct gtgttttatc 2220
   cccctcagtg gatggaaggt gtcttcaggg ccagaactgt gggttcctct tccttctcat 2280
   cagtagcatc gccatagctc ctgatttctg aacacccact aaacaccagg cactgggcta 2340
   ggtactttgt cacaaaattt cactgaaacc tcccttacag caactcccaa gaggtgggtg 2400
   gtgccgtttg tctttagaga tgtggaaact gaggcgtttc ccaggatcac agagctgggg 2460
   gccggggagc tgggggtggg atttgaatgc aggtctgcct gattcccaag ctcctttcac 2520
   tgcaccctcc ccgtgcccgt atctgcactc atgctcccag ccaggccctc acacacagtg 2580
   ggttctcagg atggaagtca actgagctgc agaagaagcc cccaggagac ctgagctagc 2640
   catggacgca gccgactcac tgccagcagg gcttttgccc cgtttaaaag tcttatttgc 2700
   cagctggtat ggaaaacatc attcattcat ttagccattt attcatttga caaagaaatg 2760
   ccagtaaacc tggcagtagg tgagtgcttg gtgccctctg atggggatga gcagggtctg 2820
   ggctgtctgg ggaggaggat gaggggtctc tgcctgatgg gcctggccca cctacccctg 2880
   gaagtcttgg tgctgaggtg ccactatttg cctcctcatc acccagggcc tggggccagt 2940
   ttgggttagg ggagaggttg ggggctccca ggctggatgt caaagaggag ttgggtgatc 3000
   tggggtacct gctggccctg ggggctcttc cctccttcca ccacgtctct gtctctttgc 3060
   cctctgtctg aaatcccagt ggggaccctg tgtgttgact gagggtgtgg tctgttggtc 3120
   aggcacgtct tcagagggca atgggtaggt ggccaggcac cctgaggcag ggccattcat 3180
   tcattcattc attccccaaa gttcatggac acacctagtc caagcccagc ccagtgcctg 3240
   gctctgggga cctcaagatg actccagctc caatcctcta ggagctacac taccaatagg 3300
   tcacagacct cacagacctg ggctctgtcc caactgccct tatgagctta gctgagctag 3360
   taagcctatg ttctcatctg tggttgtgga aagtattccc tgcctggcct ttgtcacaga 3420
   gccatgggtg tgaaaggctg agaacctgcc gggcattctt acgtcagagg tgaccctgcc 3480
   atcctggcac ctggcacagt ggctccccgt agcactgtcc ctccccacgg ctgggtgacg 3540
   ctttatggtc cttactatgc aatgttttct ggaggagtag cagggacggt tgtgcgtggg 3600
   aaggggaaga gttttgccgg agccatggca tgtatgttac acccgactct acaccaactc 3660
   acttcttact ttttttctga gatggagtct tgctctgttg ctcaggctgg agtgcagtgg 3720
   cgacatctct gctcactgca gtctccatct gcctcttggg ttcaaacgat tctcctgcct 3780
   cagcctccag agtagctggg actacaggca tgcaccacca tgcccaacta attttttttt 3840
   tttttgtatt tttagtagag tcggggtttc accatattgc ccaggatggt cttggacccc 3900
   tgacctcaag tgatcctccc gcctcagcct cccaaagtgc tgggattaca agcatgtgcc 3960
   actgtgcctg gcccgttctc acttcttctt ctttttttct atttaatttt tttttattga 4020
   gatagagtct tactctgtca cccaggctgg agtgcagtgg cccaatctct gctcactgca 4080
   acctccacct cctgggttca agcaattttc ccacctcagc ctcccaagta gctgggatta 4140
   caggtgtgtg ccaccatgcc cggctaattt ttcctttttt tttttttttt tagtagagac 4200
   cgggttttgc catgttggcc aggctggtct cgaactcctg aactcaagtg atccacctgc 4260
   ctcggcctcc cgaagtgctg ggattatagg cgtgagccac cccacccagc cccattctca 4320
   cttttaagcc atgcttttct gtcctcagat gtgctgcaca cacacctgct ccagggcctt 4380
   tgcacttgct ttccccatca cctccaatac tcctcctgct gacgtctctt ggtctccttc 4440
   cctcgcttcc ttctgctgaa gtagacaatc ctgcctgtag gtgctccctg ccaggcactc 4500
   tcactccctg acctgctttg tattcctgta taatgctctt gacctattct atttatttca 4560
   cttttattat gtcttcccac aagattgtaa gtaccgtgag gacagggact cattcaccac 4620
   tgtagactct agcctagaag agggtttttc agtcagtgcc tctgacttta attctttgtt 4680
   gtggggctgt cccgtgagct gtaggatgtt tagcgacatc cctggcctct acttattgga 4740
   tggcagtagc gccaccctcc ctcgtgacaa aaatatctcc agatattgct aagtgtcctt 4800
   tggggttggg ggattgcccc tgattgagaa ccactggcct aggacagtgc ctggcacata 4860
   gtaagtgttc aaaaaatatt catctgacct tgaccttgat aatggccctg ttttacagat 4920
   gagacggggg tttgaaaagt cagtggtggc agggtggggt tctgtttgcc tccatctctt 4980
   ggggtatttt tctgggctcc tcttcccatt gagtggtgtc tgctgaggtt aggggtcctc 5040
   tccccaaatt gctcaccccc tcgacatccc tcgtgtagtg agggatgggg ccactcactg 5100
   acggtgactc agggccccag aatgcatgtg atttacccag aagcgggcaa ggagggaagc 5160
   tggggtttgg ggtgccgggg atgaacagga acaaaggaga ggcgctgtgg ctccccgttc 5220
   tggaccctcg tctggccaca gggaagcagc actgatgact ctggggaaag gccccggcct 5280
   ctgaatcaga taaacctcaa gtcgctgctc tgctgtttta ctggctgtgt gacctgggca 5340
   agtctttcta tccctcggag cctctgtgtt cttatctgta aaatgggagt aatcctagcc 5400
   tcacgggctg gctgtcagga ttaaaagtgc tttctaatcc tggcattggc agctatttga 5460
   gccattggtg gctttgcagg tctgacccga ctctctgggc catggggtgg tgctcgggcc 5520
   taggtgggga cccctgtgga gtcagactgt ctgctctgag ccccagggcc ctgaagcttg 5580
   ggtggcagcc ctgcagttgc ccagagttcc tgtctaagtg gctttgttgc ccatcatctc 5640
   agaaagcaac ttctttcctc cctgcctttt cctgtctgtg cttctgcccc agtgccccct 5700
   ggctgggctc tcaaacccac tgcttgacag cctggtctta ggacactgta ttgtggcaaa 5760
   ggctgtgggg tcaggatatc ccctcccctc cctttgccac cccttttagt gagtaacagg 5820
   tgctgtgtgt tttggggatg agtgaagggt acatttggtg aagcacagag agagtgcaca 5880
   aggcttcagt ttgcaagatg ggcacgggaa gattccaggc catctccaaa acctcttcca 5940
   tctccaacct ctgtgctgct gaaggttctg tttacttgtg accctgagaa ggggatgccc 6000
   tatgggggct cttctgagat catgctgtcc attttcctgc ctccaagtga cttctaagac 6060
   agcatggctc tgaggaagga gcactgactt gggtgttggg agatcacagt tcccatctga 6120
   gcaccgtcac tggcttgctg tgctaccttg cttgggttac ttaacctctc tgggcctaag 6180
   catccctatc tatgaaacct aacaaccctg gttccaatgc tttcctagaa tattggggaa 6240
   gtgtcggctt tgagtatact tgacaaaggc cacagattgg gggtggggta tataaaagtc 6300
   tacaggaagg agagcccagg actccccact gtcaatatct tggttcccca gcttctcttc 6360
   ttcttgctga cctcaatctc acttgctttg ggtggattga tttcctgact caggctccag 6420
   ggccatcaag aattcacggc tgcctttcag ccttcatgaa ggacatggtg gagctcctta 6480
   ggcagaactg gggtttgtgg atgtcctgcc tctctgcccc ttggtaccta ggtggcacct 6540
   tttcaagttc gggagctaga tcctttcccc ttccccacac tgtaggaaag tccttactgt 6600
   aggcaggcgc tgcagagcct gctgtctggg aacccacagg ttcaaggaag ccttgagcag 6660
   gaagggcgtt ttcctggaag aagccctttg tcgctggtaa tgggtgcagg ctgctctgac 6720
   ccggttctga gtcctgcccc cttccaggcc tagggctttg ggccttgatc acctctgctg 6780
   agtagctgac tgcggggctg gggctctgat gctcaggacc cacctctctg ggacccacag 6840
   tctttttcca ctgtggcgtg tagtgatgtc acaggtggca gtgatgtcac tgtggtttga 6900
   ggtacttggc tgtgagcccc ggaggaggaa gtgtctgttc gctgatgggg ggttggaaga 6960
   gatcattgac ttctgcccca agcgtgagcc ccaagtgtgc aggggggagt gcggggggag 7020
   ggctgttggc ggcgcatccc agggctctgg ctctgccctt gcatctagcc tgtctttcct 7080
   gtgggctgtg acaagccact ctgcatctct gagactccat ttcttcttct tcttcttctt 7140
   cttcttcttc ttcttcttct tctccttctc cttctccttc tccttctcct tctccttctc 7200
   cttctccttc tccttctcct tctccttctc cttctccttc tccttctcct tctccttctt 7260
   cttctgatgg agtctgactc cgttgcccag gctggagtgc agtggcgtga tcttggctca 7320
   ctgcaacctc tgcctcccag gttcaagcta ttctccagcc tcaaccttcc aagtaactgg 7380
   gattacaggc atgcaccacc acacccggct agtttttgta tttttagtag agatggggtt 7440
   tcaccatgtt ggccaggctg gtctcgaact cctgacctca agtgatctgc ccgccttggc 7500
   ctcccaaagt gctggaatta caggcgtgag ccactgcgcc cagcttccat ttcttctttg 7560
   aaaagtagga ggggttggaa ttgtcaccct ggaggttcat ggttgctaga ttattgattc 7620
   tgttgctgga ttcaggagac ctgttagctg gctagttcac agcaagtatt gggtgtttgg 7680
   aggggggctg cagagcccct tctcaggccc caggagggcc agctgccctc cctaccccct 7740
   cttttggaca ctagtgggca tgttctgctg ggaaaacaga cagtgtaacc tgacttcgag 7800
   ggctgcaggc tgaatctctt tcagatgtac tggctccttg agaggcctag cagatctcta 7860
   ctctgtgggt ccctttcgga gctggggctc aggtttgacc cagccactct gaccggagac 7920
   agcgcagaaa tcaccaggag catttgtttg ttttcctttg ctgtccagac agtggcccac 7980
   cgtctgcttc agtctgagcg tggccctgca ctagtgagac tgatgtggcc acaggttcag 8040
   agaatcagtg cgcctgagtg ggagagcaga gcgggagggg atttggaggc aggcgggctg 8100
   tggacaggga gggggaggga catcttccga gctgtcagcg ggaggccttc ttgaaggtct 8160
   ttcaggagca aggccagagg ccgtgggagt gggcggggcc tggggctggg agtcaggggt 8220
   ctgaggcctc atcttggccc ggcctctgct tccgggagaa cttggccaat tcccttctcc 8280
   tttctgaacc ttggtggccc cagctgtcac ttgagattag caaggacctc cctggtccca 8340
   gcaggagtga gtggctttgg gcagggggtg gagtgcagat tgcactgcca cgtcacccag 8400
   ctcagaaggc ccaggtgtgg tccagctgtg gacttgattg tgaactcctg gggaagggct 8460
   ctggtccttt gcctcagtta gcgggacccc caccagtgcc ctgggtgccc ctgctggccc 8520
   ctcctctcgc tgaccccagc cccagtttct ggctgcaact cacagaggcc ccaggctcct 8580
   gtggccccat caccagcagc tcctgctgtc atttcctgag cctcgcaagc ccctaatcct 8640
   gctccttcct gtcctggccc ccagctccct caagccccac tcctgcatcc cattctccct 8700
   ggacatcacc cctcccaccc cttggctcaa tttccttcct gtcgtggctt ggtcctttgg 8760
   tcattgccct ggtcttttct tgtccccttc ctgattctgc ccttctccag gcccacacgg 8820
   ggaacctcct aaccgctgag ccctgaaagg taggcgggtt taggatgagt ggctacaggg 8880
   aggagaatgg tgtggggaga gagatatgga ggaaagaata ttaggggaca ggccgggcac 8940
   ggtggctcac gcctgtaatc ccagcacttt gggaggccaa ggcgggcgga tcacttgagg 9000
   tcaggagttg gagaccaccc tggccaacat ggtgaaaccc tgtctctact gaaaaaaaaa 9060
   aaaaaaatta gccaggcgtg gtggtgtgca cctgtagtcc cagctacttg ggaggctgag 9120
   gtatgagaat cacttgaacc ccagaggcgg aggttgccgt gagctgagat cgcaccattg 9180
   cactccagtc tgggtgacag aacgagaccc tgtctcaaaa aagcaaaaca aaacaaaaca 9240
   aaaaccgaat attagaggac aatgttagga ctgtcccagc tgtcctagct ggagccaaag 9300
   gtttgttggg gacagtgaca gcagcacacg aggctgtaag tttagggcca gtttatggag 9360
   gagtagtacc aattacattg agaaaactgt agcaactata atttgggagg aattttctaa 9420
   atactttaca tgtgttattt aatcttccca tcagccccag gaggtatgtg catttatcag 9480
   tcccattcta cagatgaaaa gactgaggtc tatgaactcc tttatttaaa aacaaaacaa 9540
   aaccccctca aggctgcctg ctaagagggg aggagtgtgg attcaagcca gcatccttat 9600
   tccagtgtcc atgctctcag ccaccacaca cacactccta tcaggagttg gggcagtggg 9660
   gagccattat agtttttgag caagagagtg acagggcaaa gatcactgtg gcctcagtgt 9720
   gaagcacaga tgagatggag agactggagg cagggaggcc catgaggagg ctgtcaacat 9780
   ggttcaggtg acagctctgg gttggtgaag aagcagagaa caggaaggaa ggggcgagag 9840
   gcttgaggga ggaggaggaa gcctttggac tgggccgtgg atatagggtg ggaacatagg 9900
   aggtgaggag catgctctct ccacctgctt tttgcaacat gcggcctctc tggccttagc 9960
   tctgcctgtc ctgagccctg agccctgagc ccaatggacc gattctgttt tttttttgag 10020
   atggagtctt gctcttgttg cctaggctgg agtgcagtgg tgtgatcttg gctcactgca 10080
   acctctgcct tctgagttca agcgattctc ccacctcagc ctcccaagta gctgggacta 10140
   caggcgtgcc ccaccacgcc tggctaattt tttgtatttt ttttagtaga gatggtgttt 10200
   cgccatgttg gcccggctgg tcttgaactc ctgacctcag gtgatccacc tgccttggcc 10260
   tcccagagtg ctgggattac aggcgggagc caccgtgccc ggccccagtg gactgattct 10320
   ggtcagtgtc aaccccattt gtccctggag cttttgcagt cggtcacagg gccagcccgg 10380
   tcctggtcac cgctggctgg ttaccccggc tgggttgggc aggagtctgg gcagggctgg 10440
   ttgctcatct caggtatgaa attggctgca cccccactag tgtgggggag caggattcca 10500
   gggcctgcct ggcgctgcta cccctgtgcc tccagacccc tcgagctcct cccagacctt 10560
   tggcttcgtt gactgtgagg tgaaaacaaa agggctgctc tctgtcactt cccctagggg 10620
   cgggcagatg ctggcagggc acagagagcc gagggctaag agctgacatt tagcctggct 10680
   actcaggaga ctgaggcagg agaatcgctt gaacctggga ggcggagttt gcagtgaacc 10740
   gagatcgcgc cattgcactc cagcctgggt gacagggtga gattctgtct ccaaaaaaaa 10800
   aaaaaaaaga agagctgaca tttagcgggt gcttcttggg tgcaatgcgc aagagaccga 10860
   tgcattaact acacagagtg cccttccttg tcacagctac ctgggggcag gggaacatca 10920
   tccccatttt acagaagagg cctttgaagc acagagatta actgacgtac ttagggtttt 10980
   gcagctcact agaggcagag ccaagactcc accccaggtg gccaggctcc tgagcgctat 11040
   gcctacctca ccctccaatg aggtggtggt ggaggagacc gtgagccgct gtcacctgaa 11100
   cactggtgca ctcagtctgc tgctctgagg acgccgcccc ctctctttgt aagtccattt 11160
   cctgcccatt tctcatgcta gctccagctc atttcaacta attgaaaatt aaaactttgc 11220
   ttctgttgag caattcggca tcgatgagac gccagctgtg ggctgtgtgc tggctgtgtg 11280
   tagggtgatg agtagcagaa gcacgggtga cactgagctc tttccactgc cgggcacggt 11340
   gccaagcacg ccacgtatac tgacttcatg aatcataaag ccctgtgaag ggggcactgt 11400
   ttctgtcgcc attttataga tgataaagtg gaggtggggg aatggcagaa ctgggatcaa 11460
   agcccacgtg tctgtgtctg gctcagcgcc catacaaggg tgtcaaggtt gcggggaggc 11520
   acgagggaac caataattaa ccaaaactgg tcgggtgtgg gcagtataga gatgcacccg 11580
   ggttgtcaca ggagccccta ggagggacct ctgacctccc tgaaatgatg gcagagggaa 11640
   ctcttgaaag aagctgagaa gtcagcactg ccaggtcggg ggtggcgggt caggacgttt 11700
   gggcagaggc agcagcagta cgggcatgga gaagggggcg gaggagccag gtgctgcttt 11760
   gtgttgtgac agcaacaggt gagtgatgaa tacaggtggg agatgctggc aggggctaag 11820
   gaggttgcgc aggtggtggc agccgctgga agcttttaag gagagtcagg cttgattgta 11880
   ggaagggggc ttaggtccat ctgaaaggga cagaaggctg ggaaccaaga ccaaggagcc 11940
   tgcagctggc aggagatgct gagggcagcg aggaggaggg gtcgtgggag atggagcagg 12000
   cgacatcagc aggccccttg gagggtgacg gtgggagagg ctggggcgtc tctcattaga 12060
   ctcagggttc cttgcaggca gggaccatgc ccggcgtctt tgtaccctgg catttagcac 12120
   aaagcctggc tccaagaacc ggataccagc ccagtcttcc tgtctgcccc cagctcattt 12180
   ctaaagcaac atcacaggca gcgagtcggg agtgggatgt gtcacccccg ggttgcctgc 12240
   ctgtgatgtg gtcagtcctt cctaacgcag atgccattcc tctccgtgac ggcagacggg 12300
   cctccgctgt cactggccct ggcgttgctg tgacgacctg gcctgatgta gctgcaccct 12360
   gcagtccatg gggctcctgt gaacccccaa ggttgagacc caaatgcctc tgctgcctcc 12420
   caccacgccc taggggcccc gcgacccgcc atgcaaatga cgtggtttcc gcattgcttg 12480
   aagagggttt cctctgtgtg ggggtccctg ggtccttggg cagatacctc tgcccctccc 12540
   cacagctttg gcccctgccc taagaccctg gagacccttt tgcctgggag agtcactgaa 12600
   ccacctgccc ctgctcacag tgtgttcaca gaagagtcac tcccctctgt gggctccttg 12660
   tctgtaaaat gagggagcag gacaagatca cagggtacaa aactgtcaga gggcacagag 12720
   gctgatgttg ctgccatcag ctcctggagt gccgctcact ggctgcatga ccttggacaa 12780
   tttgcacgaa atctcccaag cctcaatgtc tgcatctgta aaatggatca tcctgacctc 12840
   attgcgttag tgctaggata aaataaagag aaggtatatg aacgtgccca gcactgtgcc 12900
   tgcacacagt aggtactttt cctggatctg aacagtctct gttcttccag ctctgtgtaa 12960
   aggcttgatc tcgaccccta aagcctgaag cttctccctg tggccacctc cccaccgcat 13020
   catcttccgt tctcagcggg tagatccctg cccagggggg accccctggg gaaggctggg 13080
   agcatgacct cttcccaagg gtacggcatc taaaagggat ctccggaaca ttgaaaagcc 13140
   atgaaggccg ggcacagcgg ctcacgcctg taatcccagc actttgggag gctgagggag 13200
   gaggatcgct taaggccagg agttcaagac cagtctggac aacatagcaa gacccggtct 13260
   ctttctataa atgaaaaaat aataataaag ccatgaagat ccccaaagaa ggctgtgcta 13320
   atggcggccg gaagagcaga gggcaccaca gatgccgtgc tttcagcttt ttccttctcc 13380
   tccaggcctc ccttctttcc ttcctctctc gctatttata aataagagaa ggcattgagg 13440
   gcccccacct cgggtacttg cagggccttg gcctgggaac aggacaagca gcaagtcagt 13500
   tgacccgggg ttgctcaatt ccccaagagc tgacgcaaac tccagaatca gacctgccct 13560
   ggccacctgg ccctgcccct taccagctaa gggctccagc agagtcctag tcttgagcga 13620
   atcttatacc tgcagccctg tccaccagct gtgcttcctg agtcctccag ggtgaggggg 13680
   cgtggggtca gaagagaggg gacggtgtgc agggagggtg gaacctgact gaccgggtca 13740
   gccttactct tccagcgggg tcttttcttc ctggagctcg gccctcggca gctctcagaa 13800
   gctcaccttc ttgcaggctc tcacagccgc atcgtcctct ggctgggctg ctcacagacc      13860
   ccggcccctg ggttttgtcc actcacccag actctgtttt ggggtcacat tacccttcca      13920
   gtgttcctct gagcaaagtc cctgtgagtt agcccaaggt ccctgtcaag gaggaccaca 13980
   tctggcctgt tgcactcttc tcctttgccc caccgaagcc tctgtcacac tgtcacagca 14040
   gcgggcctgc catggtctcc ccatgtaagg cttcctgagg aacgggcctg gtgccagcct 14100
   gggggtgaac tgccagcccc aggagtcaca ggtcagcttc cccacactta ccctggaggc 14160
   aggatggagg ccccctcctt tgcaaggccc acacgggcca tctaacaccc cactaaagcc 14220
   gggcagttcg ctgccctggg gtgtgttctc gccttgccag ttcttggcta aaaccaaggc 14280
   aggaagagcc cagttcccat gttctgtaac atgggtctat gaccagggcc tttgcaggcc 14340
   ccttgcagcg cccccatgga gcccccagga gtctgggaga agatgatgaa cccaagtgag 14400
   gacctcccca ggggcctttg ctcctcacaa gaacccctgg aggagacagg gcaaggagta 14460
   ccactcccat tttacaggtg acaatgccga ggctcagaga aagtccatga cacccccaaa 14520
   tcccccggac ccactaccct gacctcctgg cggtgcacag agcatgccac gcacctctcc 14580
   atatgcacat agaccctcta acctaagtca gcgccccttc tacaaggccg gtgtccagca 14640
   cacctgggaa aggggacatt tctggccctt tcctctcaga agtcgcctgc tcgagctgcc 14700
   ctccctgggg ggaaggggat gggggcagaa gctgagcatg gaagcccctg ctaatccacc 14760
   ctctgctctc tccagaggcc caggaccagc ccaactctgc cccacatccc agcccccacc 14820
   tcccagctcg tgctgtttct ccacctctca tgtggatggg ccacacacca cccagcaccc 14880
   ctgaggtctg gtgagggact gagccttcga cacccatcct taagaaccca gggctggtcc 14940
   ttttgcttag agcatcaacc aggcccacac tctcatttta caggcagaga aactgaggca 15000
   cagagaacct ggttactgcc cacagcagag gagacacttg agtgaggaca cagaggatct 15060
   ttggggtagg ggcgggcctt ggggttgaga cccagtcttc ctggccaggg aggaaacaca 15120
   ggactggagg tgaggaggcc tctttgaggc tagtttcttc atctgtaact tgggaataac 15180
   aataggactg gcctcagggg gaccagggga ggattcagag aggctatgat gctgggacag 15240
   ctcttcgtga tgactggcat gaagcaggct tctagtagat gctctactgc tgctttcgtt 15300
   ctcaccctca cccacccgct ctgggcccac tgccctgacc tcctgaccaa gcactgagtg 15360
   tgccaagggc atctccctgt ctccccggcc ccgcaccttg tgtcaccctt ttccttagct 15420
   cttaccacca ccttgaattc tctattgaac gtgcatgtgt ctccccagct aagatgacaa 15480
   cttcccgggg gcagggattt gatttgttca atgctactgt ttacccagca cctacaacac 15540
   tgggtgctca gcacgtctgt tgaatgaatg aatgaatgaa tcactgcttt cacctttgcc 15600
   cctgactttc tccatggacc acggacaagt gacttgatct ccctgagctg tgaaatgggg 15660
   ccattcacac cagcttttta gggtggaact aggattttat acagtgcctg ccatctggcc 15720
   tggcacatag tagttgctta gcaaatatcc cattgttttc cttcctcccc agagggccaa 15780
   agccaagacc tggaggagca acagggtggg ggtctaaccc ctgggtgaca gagacagctc 15840
   ccatctggga tggaggctgc atggtgggag gagctgctga ggttgggcca ttcagctttg 15900
   gggcccttgg agtcctgggt ctgtgtggag ggggaaggga agctctctgg gccccaagag 15960
   aagggaggca ggattggggg tggcaaggat tgcagctctt ctgggatagc cttgggcaca 16020
   ctgctttgtc tccctgagca cctgcgaatg tcagtgagaa gaccagacag tgggtctaac 16080
   acgcacagtt gcccctggga ggtagcaggt gctcggtacc gatgagctac cgctaactgc 16140
   aggtgctacc tgtcttagag gagggagccc aatctggagg aggggaaatg ggagctgtgg 16200
   acccagatgg gaggcggctc agagactgtg ggtgggccct gatgagcttc ttatccatag 16260
   tcctctatcc gcttgccgcc atcctggccc tggcatggcc caaagtctct gggagacgag 16320
   gggaggtggc tttgcctttt tccatctctg ttatctcatc tgtgaaatgg gcacaatcag 16380
   acctgcctca ccaattggaa tatagattta ggtgcagttt tcttttttct ttccttttat 16440
   ctattctctc tctctctctc tctctctctc tatgtatata tgtatagcta tctattgata 16500
   ttttctatca tccatctatt tatcttgtat ctactttcta ttatctctat catctattta 16560
   tctaatctat catctatcta gctatctttc tattttatct atctatctat ctgtttgtct 16620
   gtctgtttta tctatctagc tattttatct attttatcta tctatctatc tatctatcta 16680
   tctatctatc tatctatcta tctatctatt ctatctacct atctatctat ctatctatct 16740
   atctatctag ctagctagct agctagctat ctattctatc tacctaccta tctatctatc 16800
   tagctatcta tctattctat ctacctatct atctatctat ctatctatct acctacctac 16860
   ctacctacat atctatccat cctctccgat tccctcacca ctgggtctca gattccctgc 16920
   ctgttaaata aatggtcagt taaccctcaa cagtgatttt caatttttca cttgaggccc 16980
   ccatattttg gaagattgta gtttgctgaa cacgctgact gaatcaagta ctatccaact 17040
   tatttcctca ttttaacatg tcaaagatcc cctgtggggt gagtggggtg tgaattggag 17100
   cctccccaat ccaaggcaca ggaactggaa gcttctgtag ccctgcattt cctagagaat 17160
   gcttgccatg ttttggggac ctcctggggg gcccctgtac tgaccagagg ccggctccca 17220
   gggctgtggt ttgggcccag ccccctgctc ctggggactg ctgctgtgga gggagtgacc 17280
   ttttgttctg ctttgccaag gggctttctg ttcctcattg tttagccatg acaaggctgt 17340
   ggattttttt ttttttttct gtccaagaga ataaacaggc ctgagattgc tcaagggagc 17400
   cgaccccctc tgccagctac tgggggctgg aggctcacag agccagggag gaagtcctac 17460
   cccctctgcc ggctaccggg ggggctggag ccacacagag ccagggagga agtccaggga 17520
   cggtcactca cagcaaagcc tcagacaaga gaggaacctg gaggggagga ggtgggaggc 17580
   caccactcag ccggcctggg ggccatggga gtccagctgc ctgccgttag tggccaggag 17640
   ccctcgtcca ttgcggctct tgagagccca aacttggcca cggcagggct ctttcccatc 17700
   aggccctttg agccctgatg ggaaaccctt ccccagccag ggtgggattt cttgctgccg 17760
   cctggagccg ccctggccct gtggttctca ggccctggag gagtgagggg ctcccctggt 17820
   taccccaagg gcactcgtgg ggatgaagcc cgcgggatgc aggattccca cgagacctgt 17880
   gctggccact gtgcggggtg gggggctgcc agggaagagg tgcagcccct gcctgcctca 17940
   gggatgcgat ctgagggaag aggcagctgt gtctgggagg ccgttcggtc ggagggggat 18000
   aggcacagcc cctgcccttg gggaagcgcc cagcagcagc cttcagggaa ggcagtttgg 18060
   gggtttgccg agggccagga agaccctctg ccgcagttag tattcgatgg aatggcttgt 18120
   gcagagggag ctgcagaagc tgccatttct ctgggttagg ggctcccaca gcctttggat 18180
   gccgggtctc ggccatgagc cccctccgcc acccctcccc caggtcacct tgctcaatcc 18240
   aggaggccgg atgatcagag tgtgttctgc aaaggagcgt gagaatcact ggggagattt 18300
   cctttttctt tttctttctc tttttttaat ggtacagctt cctgggccca cccctgagat 18360
   tctgcacttg tttggggagt gggtgttggc ttcatgacag gcacccctgg gtgctttgga 18420
   cacaggcagt gagctccccc cacactgtgt gaacccctga gaaggcttct ccttttgtct 18480
   agggtgcctg ggagtctgct ccagcagcaa accggggagg ggaggggtac tgggaccgag 18540
   tgtgtgtggc agcaccgtgt ccaggcaaac tgaggctcca gacgggggtt acttcttcag 18600
   accctgttat gcagcatggt tcccactgcc cgtgcccaga attcaccata gctgatgggc 18660
   ctggggtggg cctccggggc aggccagggt ggaaagtgca tctgtgtttg agctttgcgg 18720
   ccctggcgct ggagttagat ccctacagga aaggacattg taacccagcc cgtggttgag 18780
   actagcagct gctgagcacc tacgccctgc taggctgtgg ggcaacagca aagggtaacc 18840
   cccagcctgc agcctcaggg gggcttccca tctagcgagg ggcccaggga ggccatgagg 18900
   agagccatga gggaggaagg tggggactct gggcctgagt gactccttca gggtggggtg 18960
   cgggcaggga gccttcagga gggagggtac tgcctgagca gggctcttaa gggacacgtg 19020
   gaatttgtta caggaagcac caggccattc caggcataga aatcagctgg ggtgcaaagg 19080
   cctgggggtc agaagagtgg cagattgctc catggcagga agccccagct cctctgggtc 19140
   gttcccgctg agggattcca gctgttggca ccgaggggtg cagccagggc agggtggtgc 19200
   tgcctgccag gctgaggtgg gagacagctg ggtttctagc cctgcttgtc actcgctata 19260
   ggtgcagcca aacgctcagc aggcccaaac tctgggcagt cctgactccc aggcagtgct 19320
   gtgaggaagg ggaggaggag gcacactgaa cccacctgct acgttggctg gtggaaattt 19380
   ggccaggtct gcactggccc agcggtgctg ggtgtcgtga ctggtgtctg ctctgactgg 19440
   cttgctgaca gctgaagtcc aggataggtc catggccacg ttgtgctggt ttgttaaata 19500
   tttttagtat ctcccctgct cctatatgtt aggtactgga gtccagagag gttaaggcac 19560
   ttgcctaagg ccacacagcc gctaagtggt ggagtgagga tttgaaacca gttctttctg 19620
   actccctgta tccttatcta tggagaggcc ccatgactcg ttcactaaag gttatggatc 19680
   tcgatcttgt actggccaca tgactgcagc tggcagtaca gtggggagtg cgccagccct 19740
   ggctctgacc tcctagaact gccctttgtt catttccagg tgacgcatct gtgggggccc 19800
   ctgaaggact tggggtgtgt gtgtacaccc tctttgcttg tacagatgtg ttcacaccca 19860
   tgtttgccta catgtgaatg tctccattct gagcttgctc tcagcctggt agaggtggct 19920
   aattaatgct tgtccctgaa agcccggact gccaacccca gtctttcagg agagagggca 19980
   ggactcaggg gcttgctccc gggggtcctg ggaaggcaca atggtgacag tgctgcagct 20040
   ctgcactcct ggagggtcac tcagagaccc gagagaggag ggctctgcgt ctgctcctct 20100
   gtccagggct gtagcttctc tgggtgcctt tgcttttctc ttttctcccc tctttttttt 20160
   tttttttcga gatggagttt tgctctgtca attaggctgg aagtgcagtg gcatgatctc 20220
   agctcactgc aacctcctcc tcccaggttc aggggattct cctgcctcag cctccagagt 20280
   agctgggatt acaggcacac gccactatgc cgggataatt ttgtattttt agtagagaca 20340
   gggttttgcc ttgttggcca ggctggtctc gaactcttga cctcaagtga tccacctgct 20400
   tcggcctccc aaagtgctgg gattacaggt gtgagccacc acacctggcc ctctctctct 20460
   tttcattcat cacattgttc atctcttccc ggaggcatcc agcacagtct ttgagactgt 20520
   gagctgtgaa gtcagacagg acccaggtcc aaacccttct ctgtcactta ccaggtctgg 20580
   gaccttgggc aggtgacctg ccctctcagg cctcggtttc ctgttctata aaatggctgc 20640
   agtagggaaa ccgtctccat cttcattggg tggttgggaa gtcttgctga gaccacacac 20700
   ctgtctcagg tagggtcttc cagaagcaga gcctgaggca gggatctttg tgaaagtgat 20760
   ttgctaggga gggctcccag gagaagcagg cagggcaggt caggggaggg ggcttagcag 20820
   ggacgttgtt caccagcttc agcccaacac cacgggggtg gctctggacc atgaattgta 20880
   ccagggtggg tcccaccttg aggctgtgga gcattcatta tcagccaaca cccacagcag 20940
   ctgggggagc ggtgccctgc tggcagcagg ggtctgagtg gggtgcaaca gcacccacca 21000
   attcgagcag cagaaacgct tagaacagtg cctggtacct aggcagtgct tcaaaaatgg 21060
   caacgattgt catggtcata accaacattc agctgatgag tgccggctct acggtgggtc 21120
   ccatgtgaga gattgagggt ctagagatga caaggctgcc acctctgctg tctgcatcac 21180
   atgagtgtgt gtgtacacat gtgtgtgcat acatacccac agggggtgga aggacaagca 21240
   agtaaccatt ccagaacaat gagatgactg ttagaggggt cataacccac gtttagagag 21300
   agccgagagg aggaggagga ggagcttgaa ttagccttgg gtagtcaggg agggcttccc 21360
   agaggtggca gcactcatct tgaaagacaa agggatgttt caggtgcagg agggaaggag     21420
   aggtatcttc cttcctcagc agagggcagc tttgtgctga gaacccccgt tctctgtgac     21480
   caaggccact gtcttctgga tatctgtccc atgcagtgtt aggggtcacc cagcactgga 21540
   gaaggccagc agagtcagtg cttctgcctg cagacatgac tagggtacac tgaggtgggg 21600
   aggcagggga ggtaaaagaa ggcacgagct ctccttcctg taccctgctc cagggggaga 21660
   aacctcccca gccatcatca gtgcagactg gcagggggag ggccaaacat ttgcagggcg 21720
   gggacctggg catcaggcat ggcagaaccc aggggcggcc ctgttgatgg cagtcttccc 21780
   ttcttccttc caggtggcat ttacacccta cgccccggag cccgggagca catgccggct 21840
   cagagaatac tatgaccaga cagctcagat gtgctgcagc aaatgctcgc cgggtgaggg 21900
   cagccacggg ggcactcggg gcccatgccc tggaggagcg tgtgtgtaca ggggctgggg 21960
   cgcaagagca tagcccttga ttcttactga actcctagtt ctatgcactg gcccatgctc 22020
   cctgctgctt ctgggcctgt gaacatgctg ttccctcttc ctggcacact tcccaactcc 22080
   ctttccctga ttaactccaa cttgcccttt agacctcagc tgaagatgtc ccctcttcca 22140
   ggaaggctgc ctgccccata ctgcccagcc ggtggaggag ggggaggagg agcctaaatt 22200
   agctcctggg ggagtcaggg agggctgcct ggaggaggca gcactcagct tgaaagacaa 22260
   gtgctgcctc ctttgagctc tcactggcct ctgatgttcc catggaagct ctttccttgc 22320
   tgtgggatta tagcccggtc ctgcttcctg ttgcctccac tgtgtgctcc tctagggcag 22380
   agattgtggc ttgttttctg ctgaatcccc agcacgtggc gcaatgccat gtaggtgctc 22440
   agtgcatatt tgactgactc tgtgaatgct tacccatttc acagatggta gacctgaggc 22500
   ccagccacag ttggtggagc taggtagcct gactcctggt gccctccatc tattcatttg 22560
   tgtctgtatt cattcatgtc atgattataa ggtatctgct gtgtgttcta agtgctggga 22620
   atacacagag agcagggcag acaaaaccct tgccctcgtg gagtctgcat tttaacggtg 22680
   gatgcagaca gatacattag gagatgtgtg gtcctgtcta tctgtaagtg ctgtagaaag 22740
   acaaggtcag gtaaggcggg ggagacagaa cgggacatac tgttttacac agggtggtct 22800
   gggaaggtct ctctgaggag gtgacatttg ggcagagccc tgcaggaggt gagggagcag 22860
   gtcatgcatg tatacaggga acagtgtgcc aggaggaagc aacagcatgt gcgaaggcct 22920
   tgaagtcgga gcctgtttgg tgagttggag gaacagcagg gccagcatgt gtggcccagg 22980
   atgagcgaag gcgagtacgg taggggagga ggaggaagga gtgagcaggg accatggtaa 23040
   ggagtttaga ttttattctg tctgggagcc aatggagggc tttgagccgt taccttgaga 23100
   tgtaggtggg catatgagta cctgtcaccg tccctagcca ttttaagaca tcctagggaa 23160
   agtgactctc ttctttcttt tattaactgg aagggtctct ttaagaagtt actagagagg 23220
   ctgaggtggg cggaccaccg gaggccagga gtttgagacc agcctggcca acatggtgga 23280
   acccgtctct actaagaata caaaaaaata gctgggcata atggcaggca cctgtaatcc 23340
   cagctacttg ggaggctgag gcaggagaat tgcttgagcc tgagaggcag aggttgcagt 23400
   gagccgagat tgtgccactg cactctagcc tgggccacag agtgagattc tgtctcaaaa 23460
   aaaaaaaaaa aaaaaaaagc agctactgag tcaacttttt tttaaggttt ccgttggtgt 23520
   tctctgtccc tggaatgcct gctttccagg cccatgccct tgtccttgat aggataccag 23580
   ggactacatt cttactacct gttcccccta tcattcatgc ccctagctgg agaatattag 23640
   ccatctatca agaaggaaga aactggcaag ctagttctta ctaaagtgtg attgactgat 23700
   aatagggctc cagtggtgga ggttcaagca tctggaaaga gcagagagga atctattttt 23760
   gagcagaagg ggttcagaaa tgacgtgata gctggtacaa tactagatgg gagatgattc 23820
   tgaggaattg gaactccaca gaatagcctt cccagctggg ctttagaact ctggactttg 23880
   tggggacagt ggatgagccc agggtcctgg cagaaggctc gcccagctga gacctctggc 23940
   ccttgtttcc tcaggccaac atgcaaaagt cttctgtacc aagacctcgg acaccgtgtg 24000
   tgactcctgt gaggacagca catacaccca gctctggaac tgggttcccg agtgcttgag 24060
   ctgtggctcc cgctgtagct ctggtgagta ggttcagaga aaaagggggc ccttacaccc 24120
   ctgcctccaa cttcccccgg caactccagc ctctttggct tccagctgtc tggagttacc 24180
   ccaggctggt tgttggaagt ggcacaggtg cagctgttta cccctaccac tggcattttc 24240
   ctcctctgtc tcaccaaagc ctcttcacag ccccacgggg caggcggtgg gagaactgtg 24300
   cccacgtgag ggttgaggag gtggtgcgtg ggagagtggt gcgcatgctc gtgctgcgag 24360
   gagcaggact gcggggagga gcgaaactgc tgggaagagc gggactgcgg ggaagagcgg 24420
   gacttcgggg aggagcggca ctgcggggag gagcaggact gcagggagga gcgggactgt 24480
   ggggaggagc gggacttcgg ggaggagcgg gactgcgggg gacgagcgcg actgcggggg 24540
   acgagcagga ctgcggagag tagcgggact gcggagagta gcgggactgc ggagagtagc 24600
   aggactgccg gtcctgcccc tggactctgg ccggtgttgt gtgtgcccca tgctgaggcg 24660
   gtccgccagc ctcctggaga tccgctgtct gagagtgctg ggctgtctgg gagggcagcg 24720
   tgggtgtggc ggaggcaggc gtgaccgttt gccgccctct cgctgctcta gaccaggtgg 24780
   aaactcaagc ctgcactcgg gaacagaacc gcatctgcac ctgcaggccc ggctggtact 24840
   gcgcgctgag caagcaggag gggtgccggc tgtgcgcgcc gctgcgcaag tgccgcccgg 24900
   gcttcggcgt ggccagacca ggtacggggt ggggctcagg tccttgggga cgcccatggg 24960
   cctctccttt gtagacatcc ttgcagtgtc acgggcatca acccattaat tagtccagca 25020
   gggagcactg ttagtggtgg ccaggtgcct gctacccatc cgtctgtcca cctgtccacc 25080
   gttcattctt cctgccagca ctcccgatta ggcacctctt atgtactgga accaggagac 25140
   ccagagatgg atcagaccaa gccctaagct aggaaagtta tgtgatgctg caagatgaac 25200
   tcacataact ctacagccca atcccgtgca tgtgtgtaca ggaatctgtg tgtgtgcatg 25260
   tgtgtacagg catctgtgtg tgtgtgtgtg tgtgtgtgtg tgtgtaaggg gtggaggtgc 25320
   agacagagct ccttgggccc ctcagacctc tcctagggct ctagtgccaa ggcccagctg 25380
   tcccgcagag tgtctgagtg gttgacaagt tcggattgtt ccctgaagga actgaaacat 25440
   cagacgtggt gtgcaagccc tgtgccccgg ggacgttctc caacacgact tcatccacgg 25500
   atatttgcag gccccaccag atgtgagtag ctgagtcctt tggttctgga ggagcaggga 25560
   ggggctgtcc ctgggtgact gtgggtccag gacacagagc agctcaccaa ccaccattgt 25620
   ccagactgct ttatctgagg gtggctccca ggataaatgg catggtgggc aggaccttgc 25680
   cctggaaggc aggactgggt gggtgcatgg ggaggtgggg gagggctagg agggggtggt 25740
   cctcagagag ggcacacatc gtcactctcc tatcctgcct gctggggccc gtgaatgagc 25800
   ccagccaccc cagccactct gtcccctgct gcctcctgac caagcctcct cctcctccag 25860
   ctgtaacgtg gtggccatcc ctgggaatgc aagcatggat gcagtctgca cgtccacgtc 25920
   ccccacccgg agtatggccc caggggcagt acacttaccc cagccagtgt ccacacgatc 25980
   ccaacacacg cagccaactc cagaacccag cactgctcca agcacctcct tcctgctccc 26040
   aatgggcccc agccccccag ctgaagggag cactggcgac ttcgctcttc cagttggtaa 26100
   gtcgcaagaa gtctcattca ttcactcctt ctgtctgcct gtctttctgt ctctctttct 26160
   tcctctccca tggttttact gagagcccac ggggggctgg accctgtgcc ctgtcctggg 26220
   atacaggtgg cagtgagact gctttctccc cacacctggt gctatcattc aaaatcccta 26280
   agtgcgtggg ctggatgcag tggctcaagc ctgtaatccc agtgctttgg gaggccaagg 26340
   aggctggagg attgcttgag gctaagagtt tgaggtcagt ctgggtaaca cagcgagact 26400
   ccatctctat aaaaaaatta aaaaattatt tgggtgggat ggtgcctatt gtcccagcta 26460
   ctcaggaggc tgaggtggga ggatcacatg agcccaggaa tttgaggctg cattgagctc 26520
   tgatcgtgcc actgcactcc agcctgggca atgagtgagg tcctatctca aaaacaaaac 26580
   aaaaccaaac aaaacccctg gataggggag aggtggggtc agcaattcac ctgaatatgc 26640
   agatggtctc cttgctgctg tttctgatga gcccattagg ctaagaagac agcgggctgg 26700
   gccaggtcac ctccaggggc ttccattctg gggtagaact gcatcaggcc agaggtctca 26760
   aactagtggc ccttaggctg aatgaggcac acagacatga ttgctttgta attggatttg 26820
   agaatctccg gcctagactc gcccctcatt tgcaatgacc cgaagcacct tgggtccctg 26880
   gcttgcctgg ctggcccctg gtacatttga gtttgttttc tgtagctgtc tgagcttctc 26940
   ttttctttct aggactgatt gtgggtgtga cagccttggg tctactaata ataggagtgg 27000
   tgaactgtgt catcatgacc caggtgaaaa gtaagagtcc atccttcctt ccttcatcca 27060
   cttgttcagg aagcttttgt ggggtgcctc ctgtgagtcc agcactgggc acagccttag 27120
   gggtcagaca gagctggata tgggggtcct tgttctgtag gagcttaagg cctggggtga 27180
   aggtacctct gctaacttaa ttactaaaag gcaagaggtg atcagggttt tctaaagggt 27240
   aggaggagtt caggattcct atccaggaag cctgggggat tcgggcttcc tgcagcaggt 27300
   ggtctctgag ctgggtaaga tcttgatggg tggagtctgt gcagggaagg acacgcatcc 27360
   tcagtggagg cgcggatggt atagatgggg gactggagaa tggttgggat ggtggcaggt 27420
   ggagttgggt gggggcttct gtggaccttg tctggtaggc gattggtccc cacagggctg 27480
   ggcctctctg ctggttctat ggggccccat actgccctcc tacttaggac agatgtgcct 27540
   gaggaagtca atctcttact tgtcccctct cctctttata gagaagccct tgtgcctgca 27600
   gagagaagcc aaggtggtga gtgtctccac tgccctctcc ccctcttccc ctggtctcct 27660
   tcccggcgtg ctgggctgtc acagaggaag cagtgaattc tgtcattggt ctgtccagtg 27720
   tcaggaggtg tgcagagccc tgggaggtgg aggaaaggcc ggggagtcag gcagccctcc 27780
   atgggctaag agaggctgtg agtgtgaaat gctccggcag agagcagatg ccttcgtggc 27840
   gttcgtggca ggcacgtggt cgtggaccca cagcgcaacc ttgggcagat gtgaaattgc 27900
   tctatggcta cttccttacc atgagatggg ggttgtgtct gctcgatcca gcctgcaggg 27960
   tggtgccatt cggtcagctg acaatgatgt ttctccttgc ctccattgtg tttacagagc 28020
   actttctcag agagcagtgg ttttcaaact gaattctggg atagcccagt gtcccacaga 28080
   gggcccttgg gcgtggggga agtggtcatc tctcaggttt gaggtagagg ctgtggggct 28140
   gggccctggg tcctcccaaa gcaactcacc tttgatctgt tttataaagt agggcctgcg 28200
   aaggcttttt gttagagaca gaccaacagt gtgtcatatc atagtatttc attcattctc 28260
   catcaggaag gcatttggct gcaagtaacc ccaaacctac aggtgacttt agaaaaaatg 28320
   gctttatttt tcttgcattg taagaagtca aggggttggt gtctgctgga atggcctcca 28380
   ctatcccctt gcatggtgcc agcaggaccc caggctctgt catctttagt gtgttgactt 28440
   ttgttctctt gtttgtggcc tcatgtcata agatggtaat catcacttca tatgtcacct 28500
   ctaagtttga ggtgggaaga aggggcaggt ggccaggtca gccatggtgc ccttatcagt 28560
   aaatcggatg ccttcccaga agccctgggt agacttcttt catctcactg accagacagt 28620
   gtcacatgac cacccctagc tgcgagggaa gctgggaaag agggtgtttt gcttttccag 28680
   cctctatgga ggaggaggac aagggagggg ggttggaagt gggtatgggg tgagccagaa 28740
   catacgtctg tcacagcctt gcacctggta ggcagggcag gttttttacc caatttgaca 28800
   gatgagaaaa cagagtctga tgagttttct cgacctcagg ggcaggtaga gaaagaactt 28860
   ctcaacctca tggatatttg agacaacatc ttacttaagg gaggccaagg tgggaggatt 28920
   gcttgagtct aggagttcaa gaccagcctg ggaaacacag tgagacccta tccctaaaca 28980
   aaattaaata aaataaatat ttttaaaaag gatcttggct gggcacggtg gctcactcct 29040
   gtaatcccag cactttggaa ggccgaggta gatggatcac ttgaggtcag gagttcaaga 29100
   ctagtctggc catcatggta aaaccccatc tctactaaaa acagaaaaat tagccaggcg 29160
   tggtggcacg tgcctgtagt ctcagctact cgggaggctg aggcaggaga gtcgcttgaa 29220
   cccgggcagt ggaggttgca atgagctgag atcacaccac tgctctccag cctgggcaat 29280
   agagcaagac tccatctcaa aagaaaaaat aaaataaaat aaataaataa aatatcttac 29340
   ttagttgtgg gggctgtccc gtgctattca gcagcatctt tggtctttat ttacttacat 29400
   gccagcctca ttcccactcc agtggtgaca atcaaaaatt ctccagacat caccaagtgt 29460
   cccctggagg gcaaaatcac ccctagtgga gaaccactgc tcgagggaac agctcagccc 29520
   tgggcagccc aatacccgaa gcttacacca ggtgtttgga atcagataga ttgatttcat 29580
   cccctgattt tggtgacaag aaaagagatg cagggaggaa aactgagttg ctcaaggtcg 29640
   caccgcagtg tgtctctgga ctcccatctc agtgctcttt gccctgccct acagtatttc 29700
   agtggggagg aaattttctc attccccggc gagcgagttt cactgctccc tttctccagc 29760
   ttcaattcag tgacactgac ggttgccgcc tggtggcgtg cgtagagcag gtgtctcccc 29820
   caagctcttc cgcagcagcc cagctgtcac cttccctttc cccaccaagc gtttcccaat 29880
   cctctgtgca ctgaagatgt cctgggtgtg gcctagcagc ctctgcttcc cttttccatt 29940
   ttttttttct ttctttcttt tttttttttt gagacagcta tgtcgcccag gctgcagtgc 30000
   agtggcgccc tcttggctca ctgcagcctc cgcctcccgg gttcgagtgg tcatcccacc 30060
   tcagcctccc aagtagatgg gactatgggc atgcgccacc atgcctggct aatttttata 30120
   tttttagtag agacggggtt ttgccatgtt ggccaggctg gtctcaaacc cctgacctca 30180
   agtgatctgc ccacctcggt ctcccaaagt actgggatga gaggcataag ccaccacgcc 30240
   tggccccttt cctttcactg ttgctctcag tgttcactga atgtgagtga ctcaagtatc 30300
   tgttgagtgc cccagtggtg tgtctagtgc agaaaaaaac ccccaaaccc tgatttgtgg 30360
   tgtttgctaa tttctgtggt ataaacactc acactgtagc cagtttccaa ctcccaagac 30420
   cttcctgaat atggagttgg gaggaaatct gcacaggcct gagcttatcc cagcacagcc 30480
   ctggctgagc cctgccaggc tctttgtgac tgagtcccca cgcatccctg tggggcaggg 30540
   agcatcatca cccccatttt acagaggagg aaactgagac tctgggaggt tccatttttc 30600
   ctgtaggatt agatcattag gtgagtggca tagcccaggt ttgaatcagg tttatgcctg 30660
   tgtcccacgc tactcctcaa ccagcagcta ctcctgataa gaggccagtg gagagtcctc 30720
   ttgagctggg ctgctgggct tagagtgtgg actcagcacc cacctgctcc tcctgccaca 30780
   gaaagaacgt ccagggcatg ggagacagtg gcgttccttt gtttagaaaa caccaacatg 30840
   tggccgggcg ccatggctca cgcctgtaat cccagcactt tgggaggccg aggggggcgg 30900
   atcacgaggt caggagatcg agaccatcct ggctaacaca gtgaaacccc atctctacta 30960
   aaaataaaaa aaattagccg ggcgtggtgg cgggcgcccg tagtcccagc tactccggag 31020
   gctgaggcag aatagcgtga acccgggagg tggagcttgc agtgtactga gatcgcacca 31080
   ctgcactcca gcctgggcga tagagcgaga ctccatctca aaaaaaaaaa aaaaaaaaac 31140
   caatatgtga gcttacccac aatcaccccc atggacacgg acacactcat gcacggaccc 31200
   acgtctgata acaggtgcga acatttatcg ggtgttgaac ggatgccagg cactgtgccc 31260
   agcgttcttt attctcacag ccatgctgcc acacgcaggc agtatgattc ttgtccccca 31320
   ttatatggcc caagaatcat gggcccagag aagttagtaa ctttcccagt gtggcacagc 31380
   taatgatcac ggcctgggac acgaacctag gagtgtctct cctcgccacc ccagcgcaca 31440
   cactcactac gtcactcaca gatatgcaca catgccccca acatagacac acgtgtgcac 31500
   gcttcacacc ctcacgcctg tagacacagg tgtgtgagcc ttgtgaacac acaaacagac 31560
   ggacacagca ggaacctgga ggacctggcc cgtggctctg ctgggtctct gcttctgccc 31620
   agcagggctg ggccaggagg ggccgggagc tgagggtgct ggctggctgg ctggctggct 31680
   ggaattctgt tttttttttt ttttttgaga aagagtcttg ctttgtcgcc caggctggag 31740
   tgcagtggca caatcttggc tcactgcaag ctccgcctcc cgggttcaca ccattctcct 31800
   gcctcagcct cccaagtagc tgggactaca ggcacccacc agctcgccca gctaattttt 31860
   tgtattttta gtagagacgg ggtttcacct tgttagccag gatggtctca atctcctgac 31920
   ctcgtgattc ggtgaccacc tcggcctccc aaagtgctgg gattacaggt gtgagccact 31980
   gcgcccggcc ctgggatcct gtgttttgaa tgaggctcct cagtactcgg ctctactggg 32040
   gtcccagccc aaggaatagg actcagcctg cttctgtgcc acctggggct gcttgaactt 32100
   tgcgacttgt ggcttgggag gagggaggtg gccgtgacct ttgggggttt ttgttctgcc 32160
   tggctgtagc caccagcaga gggggtgggg cacaggccag aaaaacccct tttgtggggt 32220
   tgtgaggagt gacaattggc tgcttctcct ccccttccag gctcagagca gggctggggg 32280
   gcagttgtgg gcagtgacca gggtcagacc acctgggcgg aggttcagca tgaacttgca 32340
   atgccctcca tctctccaaa actgggggac ccagcccagg gagggtgtgg gggttcctgg 32400
   ggaagctggt ctaggcttct gctcctgcca cggaccagct gtgtgatgct gggcacagga 32460
   tgcactttct ctgggcctcc gtggcctctt ggggatggct tgcacgagat ccctccagtc 32520
   ctgagtgaga ggctgtggcc ttggggaatt aagggtgcag gtggcgctca ggtgtccgag 32580
   aagccatggg agccgggggc tgcagggatt ggacagagag gaccctggta ctcgcatctg 32640
   ttctcagacc acatctggaa ttgtagctcc ctctggaggg aggcaggagg tctcagcctt 32700
   tcttgggggg cggtggcacc tgcgctgctc gctccacccc tgctctcacc tcccgctgca 32760
   gtgctggcga gccccatcag cccttcactc atctctaccc tccttctttc tgcctgggac 32820
   acttgttttc atcctgggca ggccaggggc cagggcagct gttgggaatg tggcctgtgc 32880
   catctccttt tttgctggga tcagaaaaca atcgcttaga attccaaggc aagggtgtga 32940
   gcgcctggcc agccagtggg aacagacaac agcctgggag aggaatttcc agcctctctt 33000
   cagtgtgcgt gtctggaaat ggggaccttg ccttgagcct ccagagttga aaccccagac 33060
   acccaggaaa ggccctttgg gatttagccc agccacagta tgtcctaacc gtgaccttgg 33120
   gcaagtaact caatctctcc gtgcctcagt ttccacaaag caaggataac actgggttgt 33180
   tggaagaatc aatatagata ttgtctggag ggatgtaggt acagtgcatg gcattgggtg 33240
   gcactcaaat gtcagctaat aatattatta ttattctacg ggaagaagac atcaggggaa 33300
   gttgcagagc agcctgtggg cggactctgg aacaagaggc tgaggcagtg cagcagaggg 33360
   tctcagacgt gagcgctctc tgccccggaa tgattgactg agcgcaaagg tctgcacgct 33420
   ttctctgtaa agggccagat ggtaggaatt tcaggctttg tggactgtat ggtctcagtg 33480
   acagctactc aaacctgcct ctgtagcaag aaagcagcta catgcataga cagcacacac 33540
   ccaactgaga gtggtgtgtt cctatctaat tgtgctactg gacacccaaa cttgagcttc 33600
   ccaccattcc atgtgccgca aattattctt tttttttgag atggagtttc actcttgttg 33660
   cccagcctgg agtgcagtgg ctcgatctcg cctcaccaca acctctgcct cccaggttca 33720
   agcgattctc ctgcctcagc ctcctgagta gctgggatta caggcatgag ccaccacgcc 33780
   agctaatttt gtatttttag tagagacggg gttttgccat gttggtcagg ctggtcccca 33840
   actcccttcc tcaggtgatc cgcctgcctc agcctcccaa agtgttggga ttacaggcat 33900
   gagccaccaa gcctggccac aaattattct taaacatttt tttttcaacc atttaaaaca 33960
   tgaaaaccag tggggtgtgg tggtgcacgc ctggtatccc agcactttgg gaggccaggg 34020
   taggaggatg gcttgagccc aggagtacaa gaccagcctg ggtaacatag cacaaccctg 34080
   tctctacaaa caatcgacaa caaaaaaatt agccaggagc agtgacacgt gcctgtggtc 34140
   tcagctactc aggaggttga ggcaggagga tcacttgagc ctggaaaatc gagggctata 34200
   gtcagctatg attgtgccac tccactcctg cctgagcaac agggtgagac cctgtctcaa 34260
   aaaaaaaaaa aaaaaaaaaa agtgaaaacc attcttagtg gcaggctgta ctgggcctct 34320
   gggcaatcat ttgcctagtt ctgatttaac aaactcttgt atggagttta ctatgtaata 34380
   ggcattgttt taagcacttt acaaatattc agccatctaa tcttcacaac aaccctatga 34440
   ggtaggctat tattctccct ttatagagta aaaaaaaaaa aaaaggcaca gagaggttaa 34500
   gtaacttgtc caaggtcaca cagcaagtga gtggtagagt catgatttgc acttgtgtgg 34560
   cctgggttta gagtccacac tcttggttgc taggctgggc catgtctccc tgtgcagatg 34620
   gggtgaagga aagctgcttt ccttctactc ccttatgcaa aataaggatg aaaatcctgc 34680
   cccacctcta agactatttg gtgaacaggg ccaggtattc tctgccctca taggacactc 34740
   tagtagagca gctgggtgct aataacagaa acacagaaac cacggagaat cacacctccc 34800
   aaaaagtgcc gtgtgtggaa agaacgtgca agggtgggca gaaacgtcac gtaaactgag 34860
   aagtgctgat ggcaggaggt gtggatggca gtgggaagga agaggggaag aaagagctgg 34920
   ctggtgggct gactgctctc ccctaccacc ccctgcccat ccagcctcac ttgcctgccg 34980
   ataaggcccg gggtacacag ggccccgagc agcagcacct gctgatcaca gcgccgagct 35040
   ccagcagcag ctccctggag agctcggcca gtgcgttgga cagaagggcg cccactcgga 35100
   accagccaca ggcaccaggc gtggaggcca gtggggccgg ggaggcccgg gccagcaccg 35160
   ggagctcagg taagaggtgg gagcacacct ggcttcttcc caagcctcct tggtctttct 35220
   cacctggttt ctgtcttagc catctcctcc tgagcctccc cgcagggtgg gacgaggcct 35280
   gagccacagg gaacttcctt cggttcgctg aacctaagtt ccctcccgcc tttgcccatg 35340
   ctgggcctat cacctcaaaa tcctcccttc tgtgggacaa ccccagcttg tccaagtccc 35400
   tggtatctgg gggaggagtt ttcctgaaac cttctccctg ctaccacccc cagctggcct 35460
   ggctgctcct ccgggctcac catgccctgg cctccctttc acaggactgt cagactgcat 35520
   gtacagacat tgtcttctcc tgtctcccac gccaggctgt gggacacctg gtgagcctgg 35580
   atcatctcat tcatccctgt atctgcaggc ccaacacggc ccagctactg ataattaatc 35640
   ataacaatcg cttctactta tggaaggcta cggaacagca ggcactgtac tgggcacttt 35700
   acatgcataa aactgacctg catgtcaatg ctaagagata gttcctgttg ttatcccatt 35760
   ttacagatga ggaaactgag ccccagagag gttaaacagc ttcttcaagg gcacatggct 35820
   agtaaacaga agagccagac tcacccctag gctgtcgggc tccagagccc tgggattgga 35880
   agatgaatga agaaatggtg gctccagggc tccactcact gcagtttgtt gctgggtctc 35940
   tttaggtctg aggcactggg actgtgggga ttgtgtccca tttatgcagg ctgcattgtg 36000
   ccctggacct ggtctatgac agatgtcact cctggttggc atctctaggg cccacactgg 36060
   aggggcccct gtacagggtc tgctggcctg tgcctctctc cctcttacct ggcagtgcca 36120
   gccagtgaga attcagggaa tgaccatgaa cttgggtcag tctgagatcc ttgcctggcc 36180
   actctggtgc cataagaatt tgggtgggat gcttaaccct gccaagcctt ggttttttca 36240
   cctagaggtg agctatagcg cctccttgcc agggctggtg agagatgggt gacattgtgc 36300
   ccagctgggc accagaccag ggccaggctc cctctgggcc gcctccaggt ggggactgat 36360
   ggctgcagcc cccaccacgc agccctcctc cagctcactc acccccagct gctccccagc 36420
   tcactgcgcc cctgcctctt gcctgcactc atgccacccc tgcctgccac acctgttcct 36480
   acctgccccc aactgcccac aggagctgac gtggccattt ctgtctgccc catcatagcc 36540
   cccagcctcc tcggtgggag gtctcatcag tgcccctcac tccatcctga gaactcccta 36600
   tgggagggcc tgggccagtg cctggaagag atcaggggcc ctacacactg ttgaatgaat 36660
   gaatgaatga gtgcgctcca attataaact cttagtcttt gcccagttct tggattcgtc 36720
   tgattttttt tttttaaatg gggtctcgct cgtcgcctag gctggagtgc agtggcgcag 36780
   tcttggctga cttatctgct caccgcagcc tccgcctccc aggttcaagt gatcctccca 36840
   cctcagcttc ccgagtaggc gggattacag gcatgtgcca tcacgcctgg ctaacttttg 36900
   tacttttagt agagatggag tttcaccatg ttggccagac tggtctcgaa atcctgacct 36960
   caggtgatcc atctgcctcg gcctcccgaa gggctgggat tacaggcgtg agccaacatg 37020
   cgcagcccat ctttctgatt tcttagctac acctggtgtg gctccctcct tgggccaggg 37080
   tggagccctg accatgtctg ccctcccctc tcccctctgc cccttctgct ctgtgctcct 37140
   tctcccgagt cccccagccc gtgtccctgg cctctgtctt ctctttctct ccctcccacc 37200
   cctaacacct ccctccactg tgggaacctg taaaccccag ggttgtgccc cttcatggtc 37260
   ccccatccac ccccgcaatg tctcatgctc gatatacaaa ggccatggtg actttgggtg 37320
   acatttgggt gctgtggagg ctcagggtgg aaatttcctt ccggccttgt gatttcaacc 37380
   ctcctccccc accacatgct tggggctgtt ttgagcacag caggttgcca gctccatcca 37440
   cctcccggct accctatccg agtagttgga gttagggaga accaggctgg ggtgagggca 37500
   ctcagcaggc ccctgcagca acagcagcag caactctcat tttctgaggg ggctacttac 37560
   tgtatgccag tcccttcata ttcatctcag caaacccacc gtccagtgcc tccccaacca 37620
   gttagaaaac tcagttgccc acaggggctg ggcaggaagg tgaggcaaac cttgggctgt 37680
   ccttggccgg atctcctgca tctggctccc aagggaagcc ataaatccag atttttaaat 37740
   gtaaacgcct gaattttaaa tgttggtaat caattcactt aaaaacatca ccaccaccac 37800
   caccaccacc accaacaaaa aaacccgtag acttgtccct gttacaggca ctaggaacac 37860
   agcagggaac aatcaaaaag tccctggtct ggccaggcaa ggtggctcat gcctgtaatc 37920
   tcagtacttc aggaggccaa ggcaggagga tcacttgagc ccaggagttc gagactagcc 37980
   tgggcaacat agcaagaccc ccgtctctac taaaaaaata aaaaaaaaag tccctaccct 38040
   cctgggttca gagtctggtt ggggacccca ggagctgggg gctctggaga tcaggagatc 38100
   acagaaatgg ggagggaccc agagagtggt ggataggatg ggaagtaaat gtctctagag 38160
   agggaggcca gggggtggag ggcgcttcgt ggaggaggtg gcctttgagc taaggcctga 38220
   gcactagaga agagctctct aggctgaggg agcggcctgt gcaaaggccc aggggacctg 38280
   aagggctcaa ggggctgtag cagggggtgg ggaatgtggc tggaaggaac cccatcaagg 38340
   tcttggagcg gcaggagagg gggtgggaga aggcaggctc cagatcagac agggcctggt 38400
   aggctgtagc aaggactgtg ggtttttgag cccccaagga agtgatctgc caggttcaag 38460
   ggccagctct ggctgctgat gggaaacaga tttcagaggg gtggggttga agccaggaca 38520
   gatggaggct gttcacaccc atccagatgg gagtgagggg aggcttccat agcccaccat 38580
   gcagcagcag ggcagggtga cccttgcaga agtcatcttt tgtttttgtt tgtttttgag 38640
   atggagtttg gctctttcgc ccaggctgga gtgaagtgac gtgatttcgg ctcactgcaa 38700
   cctccgtagc ctgggttcaa gctattctcc tgcctcagcc tcccgagtag ctgggattat 38760
   aggcacctgc caccataccc ggctaatttt tttttttgta ttttgagtag agacagagtt 38820
   tcaccatgtt ggccaggctg gtctcaaact cctgacctca ggcgatccac ctgccttggc 38880
   ctcccaaagt gctgggatta caggcgtgag ccaccctgcc tggtccagaa gtcatctttt 38940
   gaagggagac aaggcaggaa tgatggatgg gtgtgtgata tgagagaaag atgggtccga 39000
   ggctctgggc ccaagcagct gggtggatgg cagcaatggg aactgtgatg agcaggagag 39060
   gttttggatg cgagatggga gtagaatcaa gagttaagtt ggaggctgag cacggtggct 39120
   cacacctgta atctcagcgc tttgcgaggc tgaggtaggc agattctttg aggtcaggtg 39180
   ttcgagacca acccaggcaa cctggcgaaa ccctgtctct acaaaaaatt agcagggtgc 39240
   ggtggcctgt agtcccagct attcaggagg ctgatgtggg aggatcactt gaggccggga 39300
   ggcagaggtc acagtgagtt gagggagtga cacagcactc ttttgagacc ctgtctcaaa 39360
   aaaaaaaaaa aaaaagacag aagagacagg gtctcactat gttgcccagt ctggtcttga 39420
   actcctgggc tcaagcgatc ctacaaactt ggcctcccaa gtagacatct gttttatata 39480
   attggctcct cccatctctg gggtgattgg ggctgggtag gtagtgatgc tattcttatt 39540
   cggcagaggg gaaaatgagg cacatgcagg ttaagtgact tgctcaaggt cacacagcag 39600
   agctgggcta gaatcttggt ctcggctcct ggcccagtgc tctttcccat gtgtctgaat 39660
   ctgcatcttg ggcaggggtc cctgggcccc actcctggac ccccggactg acccccaccc 39720
   catcttgtgc ttagcagatt cttcccctgg tggccatggg acccaggtca atgtcacctg 39780
   catcgtgaac gtctgtagca gctctgacca cagctcacag tgctcctccc aagccagctc 39840
   cacaatggga gacacagatt ccagcccctc ggagtccccg aaggacgagc aggtcccctt 39900
   ctccaaggag gaatgtgcct ttcggtcaca gctggagacg ccagagaccc tgctggggag 39960
   caccgaagag aagcccctgc cccttggagt gcctgatgct gggatgaagc ccagttaacc 40020
   aggccggtgt gggctgtgtc gtagccaagg tgggctgagc cctggcagga tgaccctgcg 40080
   aaggggccct ggtccttcca ggcccccacc actaggactc tgaggctctt tctgggccaa 40140
   gttcctctag tgccctccac agccgcagcc tccctctgac ctgcaggcca agagcagagg 40200
   cagcgagttg tggaaagcct ctgctgccat ggcgtgtccc tctcggaagg ctggctgggc 40260
   atggacgttc ggggcatgct ggggcaagtc cctgactctc tgtgacctgc cccgcccagc 40320
   tgcacctgcc agcctggctt ctggagccct tgggtttttt gtttgtttgt ttgtttgttt 40380
   gtttgtttct ccccctgggc tctgccccag ctctggcttc cagaaaaccc cagcatcctt 40440
   ttctgcagag gggctttctg gagaggaggg atgctgcctg agtcacccat gaagacagga 40500
   cagtgcttca gcctgaggct gagactgcgg gatggtcctg gggctctgtg cagggaggag 40560
   gtggcagccc tgtagggaac ggggtccttc aagttagctc aggaggcttg gaaagcatca 40620
   cctcaggcca ggtgcagtgg ctcacgccta tgatcccagc actttgggag gctgaggcgg 40680
   gtggatcacc tgaggttagg agttcgagac cagcctggcc aacatggtaa aaccccatct 40740
   ctactaaaaa tacagaaatt agccgggcgt ggtggcgggc acctatagtc ccagctactc 40800
   agaagcctga ggctgggaaa tcgtttgaac ccgggaagcg gaggttgcag ggagccgaga 40860
   tcacgccact gcactccagc ctgggcgaca gagcgagagt ctgtctcaaa agaaaaaaaa 40920
   aagcaccgcc tccaaatgcc aacttgtcct tttgtaccat ggtgtgaaag tcagatgccc 40980
   agagggccca ggcaggccac catattcagt gctgtggcct gggcaagata acgcacttct 41040
   aactagaaat ctgccaattt tttaaaaaag taagtaccac tcaggccaac aagccaacga 41100
   caaagccaaa ctctgccagc cacatccaac cccccacctg ccatttgcac cctccgcctt 41160
   cactccggtg tgcctgcagc cccgcgcctc cttccttgct gtcctaggcc acaccatctc 41220
   ctttcaggga atttcaggaa ctagagatga ctgagtcctc gtagccatct ctctactcct 41280
   acctcagcct agaccctcct cctcccccag aggggtgggt tcctcttccc cactccccac 41340
   cttcaattcc tgggccccaa acgggctgcc ctgccacttt ggtacatggc cagtgtgatc 41400
   ccaagtgcca gtcttgtgtc tgcgtctgtg ttgcgtgtcg tgggtgtgtg tagccaaggt 41460
   cggtaagttg aatggcctgc cttgaagcca ctgaagctgg gattcctccc cattagagtc 41520
   agccttcccc ctcccagggc cagggccctg cagaggggaa accagtgtag ccttgcccgg 41580
   attctgggag gaagcaggtt gaggggctcc tggaaaggct cagtctcagg agcatgggga 41640
   taaaggagaa ggcatgaaat tgtctagcag agcaggggca gggtgataaa ttgttgataa 41700
   attccactgg acttgagctt ggcagctgaa ctattggagg gtgggagagc ccagccatta 41760
   ccatggagac aagaagggtt ttccaccctg gaatcaagat gtcagactgg ctggctgcag 41820
   tgacgtgcac ctgtactcag gaggctgagg ggaggatcac tggagcccag gagtttgagg 41880
   ctgcagcgag ctatgatcgc gccactacac tccagcctga gcaacagagt gagaccctgt 41940
   ctcttaaaga aaaaaaaagt cagactgctg ggactggcca ggtttctgcc cacattggac 42000
   ccacatgagg acatgatgga gcgcacctgc cccctggtgg acagtcctgg gagaacctca 42060
   ggcttccttg gcatcacagg gcagagccgg gaagcgatga atttggagac tctgtggggc 42120
   cttggttccc ttgtgtgtgt gtgttgatcc caagacaatg aaagtttgca ctgtatgctg 42180
   gacggcattc ctgcttatca ataaacctgt ttgtttta 42218
<210> 34
   <211> 110237
   <212> DNA
   <213> Homo sapiens
<400> 34
   ccctcctgga caaagcgttt ttcctcagcc ttcaccaccc atggaggagg tgtgattacc 60
   tctattcaca gaaaggagac tgggacccaa ccaaggcaag tcatttgctt cagaccacag 120
   agcaaagaat cattctgtca ttccagaaat atttgtcagg cgcccctggt gtgtcaggta 180
   ggtgcacctt gaacatttgt ttaatttgta cttaataaca ttttcaagag gacattacct 240
   ccttaagtga aaaatctgtg aggagttttc tatctttcta agtctttgtg gcacacttat 300
   cacatgcata gtactatttc tcaagttacc caggcacaca cagatgtatg gctttcagga 360
   agccatacat ctgtgtagct ctgaggccca gccttgttca acacttcact cagaagaatt 420
   gctggcctga tgaccaacaa taattgtcaa caacttaaaa cttcattgac ggctcctaaa 480
   aaaaatcagt gtgcatgtgt gtgtgtatgt gtgtgtgcgt gtgtgtgtgt gtgtgtgtat 540
   gagtgtgtgt ataacattca ttactgcaaa ctggaatgga gtggggctgg aaggcagccc 600
   agaagcaagg ctcagagcct ggagctgctg acctcttggg gacactgcag ccctggcctg 660
   gcctactttt ctcttctccc atatctggaa aatgaaagta gcctgacgta tccggggacc 720
   cggcccaaga ctccacccat tggcagctct tcacttgtat cttttcatat caaaaatggg 780
   aggtgacacc cagtttaagg aaaattccaa ggcatttgtc tcgactaatg tgaaagatga 840
   ttacagtggc cagaggactg ccaaggctcc ttctcaagct gcttgagtca atgagggtaa 900
   ggttttaaag ggcccgccct ggcttccttg ggcctgtagt ttgtgactgt gtcgttttca 960
   gagtgggaag gcccagagtt ggccccatgg aattctttct gcttctaggg tgtttgaaga 1020
   gatagaaagc cagggtaagg ttcaccgaac ctccaagcaa aaataaattt ccagaagggg 1080
   cccctttact ctgaggacat tgaatagcaa caaaaactac cactgaagag tcattttgtc 1140
   ccaggccctg tgctggaaac tttgaacacc tgtgttcacc cctccctcat gacactcatg 1200
   aagatatacc atggctgtct ttattttcta ggtgggaatc tcaggcttga gggttggggt 1260
   ggcttgggga aggtgtgtat ctaagaggta gctcttattt atttttactt ctagagatta 1320
   agacacattc tttttcaaga tagacatgga actgtcaaaa aggattaaaa gttaaataag 1380
   gataaggtta tgtaaattga tcttatgtaa aaaaaaacac taagatattt gaagactagg 1440
   catcattatg tgttatatca tgatttttaa aatgatgcta ccaagagtac ttcagaggtc 1500
   caaagctgaa atatatttgt atacttatat atataaagta agggatactt atttaaaacc 1560
   tcaaaaaatc agaagtatgt aaaggaagaa ataaaagctc cctcttccct tctgattcta 1620
   atttatatca aatttatatc tctatctgta gctcaatttg cctttttaac taaatagggt 1680
   gatatcacac atatcattcc ttaacacttt tctctcttaa cgacttctaa tggacatcta 1740
   tttgtggcac tgtatatagg gcatactttc ttgaaaaatc cattgcatgg tattttattc 1800
   ttgaataaat cacttgcatg gcattttatt gtaagattgt gtcataatgt atttattcag 1860
   ttccctggtg atggctttat catgtggctt ttggctgaaa gtatcagaaa gtggcccaaa 1920
   ctggcttaca taaatttacc atctcatgta actgaagttc aaagaaagag tggcttctga 1980
   gtgggttcca tactactagc ataatatttg gcatttccct gtatgtcccc tcctctgtgt 2040
   gttggttttg tttgaagatt ctttcccttt atggtggcaa aatggttttt gtagtggcac 2100
   caggctttac aacctcttgt gagggaagag ggagaaatgc catgccttaa tgtgccaaga 2160
   aggaggtgtg agatccaccc tggttgaacc ccatggtgcc ggtgtcctct cctagaccag 2220
   tggctgccaa gggccatctt ccgaggaagc acctggacta tacatcctcc acaatgggcc 2280
   cttagaattg gtccatattt ttcaaaattg caaataatgt gtaaaatcat tgtacttggc 2340
   cgggtgtggt agcttatgcc tgtaatccca gcactttggg aggccgaagt ggggggaatc 2400
   agctgaggtc aggagttcga gaccaccctg gctgacacgg caaaaccccg tctctagtaa 2460
   aaatacaaaa aaattagctg ggcatggtgg cctgtgcctg taatcctagc tactagggag 2520
   gctgaagcag gagaactgct tgaacccggg aggtggaggt tgcagtgagc cgagattgtg 2580
   ccattgcact ccagcctggg tgacagagca agactctgtg tcaccaaaaa aaaaaaaaaa 2640
   gaaaaaaatt cgtacacttt acatatagca ctgtttactt gggcaattat ctgtttagga 2700
   taaactagaa ataatattgc taagccaaat agaatatagg ttttaaaagt ttggcgcttt 2760
   atcctccaaa acagatttta atagtttaca cttgtaatag atgtacctgg tttcttatac 2820
   ctcacttact ggaatattag aaatcgttta gtctctacca aaacatggca tttttctatt 2880
   atcttaattc acatttcttt ggttattagt aatcttgaac atgtttttca cgtctattgg 2940
   ctttttgctt tttgcttatt gctttttgct tctttctttt ttcttctgtg gactgtttat 3000
   tcatgtcttt tgcagtattc tctcttggat ttgtgagaat tgtttattta ttataaacat 3060
   taaaccttga ttggtaatac atgttgcaaa tatttttccc taagttttca ttgatttgcc 3120
   aaacttagtt tttttttcca agttgtcttt tctatgcaaa agttttcgct tttttgtagg 3180
   tgaaactatg agaaattttc ctttaccatt tccgagttta atgtcatgct aagtccttcc 3240
   tgtcttggga agatgtgttc acagcccact gactgaaggc ccttcccggc cagttctgag 3300
   cttgagctga gtcaaatgta aacagctcct ggggctgtgg gtatagaaac tccctttctc 3360
   tttgggtctg gaataccatg atgccttttc cctgtcctca tttttcttaa atgttttcat 3420
   ttaatccagg cattatgtac tgggggatca actattcgca aaactcagta aaaggcggat 3480
   tcccaaggct ccactttgga tgcagtaggt ctggggccaa agcatgtgag aaaaaaagtg 3540
   tcagcattta ttcattccac agctatttca tgagggttca gggcatcatt ctggggacca 3600
   cggatacaat acggtgaaca tcatagacaa ggttcttatt ctcatgtagc ctaaattcta 3660
   ctgggggaga tggataatag gtgaacaaat aaacaaacaa actcatctgg caccaaaaag 3720
   tccactaggc aaagtgctga ctgtgaagaa aagctggggt ggaaagagag gccatggccc 3780
   ctgcaaaatg gaataggaac aaagatttaa tcatttgcct ctgaaaaatc tcagtgatga 3840
   gttccttccc tcccttccct caacctccag tgtagaggag actgatgatg attgacaggg 3900
   aagcactggt ggcttctcag aagtccatgt gcctcttcca ctgccttagt caccccttac 3960
   tctgcatttt cacagaatct cagtgaaatt gcttaaacct ctgagtacct gagagatact 4020
   cagggctctt tcttggatgc caaatagata aaatattgac tcttaaacat atgtctgaat 4080
   tcctcttgcc tttccaaggt aggagaccat accatactca tttgacagca ccatttacgg 4140
   tcaacaaatc tctaagctca aagaagagaa gaggcaaaaa gcaaccctac cttatgcaat 4200
   taaaaaaaaa tctcccttta tagagtttca ttgacccttt cttaaaaccc cctaaaatgg 4260
   acgtcaacaa tgcccctctt ttatgatgag aggatggagg agcaaacagg tcgagggact 4320
   tattccaatc gaacgtctca gtagctcagt cccaaggagc tctgccccag tagtgccact 4380
   tcaagtccca tgttcttttc atcttgtgag tgatgagcag cacgtggtct ctaggtttgc 4440
   aatataattt aggatttact ccttgggttt agttttggta actctcaaag aagttcaaat 4500
   attaagaatt ttgctagctg ttttagtaat ctattgctgt gtaactacct cttcccctac 4560
   ccccagtcca gaaaacaaca accatcttct cttttctcgt ggttctgtgg gcagttctgc 4620
   tccacatgac gtggtctggg gctgcagtcc tgtggatgct tctttgggct ggtgaggttc 4680
   catatggtgc tttcacacgt ctggtgcttt ggtgagacag tggggaggct gggctcagat 4740
   ggcaagctgt gatgactggg cctatctgcc catgtcatct ctgggcctct ccatatggtc 4800
   tttctgcatt ctctccagcc gggtggccta ttccttacat ggtagtttgg ggctcctaaa 4860
   agtgcagaca tggaagctgt tcaacctttg tgagatgctg ccccagagct agcctaacat 4920
   cccttcctct gccctctatg ggtaaagcag aagcaccaaa ttcaagctgg gaggaaaaca 4980
   aacttcctct ttcaaaggcg aaagtgacaa agaatttgca ggccgtttaa atccaccaca 5040
   ttgtccagtc aggagcaccc attttctcat tgaccttctg ctaagtgaag aggtagaagg 5100
   aattttcaca atggctttta gaaaagtaaa acaatacagg aagggatttt gctgtcattt 5160
   aaaacatggt ctgctccaga tggaggattt ggtgattttt attttcttct ttactaaaca 5220
   tttctgcatt tcatgaagtt tttcagtgca tcatttttat tatcagaaaa acaatgacat 5280
   tttattaaat atatatatat ttaactcatt aattatctaa ctcattgaat atatattttc 5340
   attctatatg tatatatatg tgtgtttaca tataaacgtg tgttatgcgt attctttata 5400
   catttttctc ttgttttttg agttggacct tggcaaagtc agtggtgaca cctggattac 5460
   cttgtgttaa ctttaatcag ttatgtgaca tccatgggat tggcaagtaa aacaagttat 5520
   tggtagtggg agtttgaata gatggcttct aaacaccctc ccagacactg taccttcaac 5580
   tctctgatct catccaatcc agaccaacac ttctttacta agtttctctg ttaatcagtt 5640
   tgcacaggaa aatgtttcca ttcaacacat atgtatgatg agcatgttgt gacactggct 5700
   ctgtgtagac atagtgggca ctgaggaaat ctggggatcc tgccattagg agcttgtgag 5760
   ctggcgggga gctatggtga tgcacaaaat tataacacat gctaaaagaa cacagttaaa 5820
   agagaaataa aatttaaatg ttaaagaggt gattaccagt tatttgaaaa aaaaattcac 5880
   ctctttccaa ggagattaga tttctacttt tagaattctt catacaaata cacatatttt 5940
   gagttcttga tctcagaacc ccattgcata tattgcatgc aactggtttg caagtttttg 6000
   gtacacaccc accccacaca tacctgattg cctactgcac ttcctagaaa tgtcgaggaa 6060
   gcctagatga cagaagttaa tttcttccca gggaatgtaa agcatatctt ggcagataag 6120
   aacgaaaata caggttaaag acagaagatt tcactgggag tggctatgag gagccattta 6180
   aatttctagc agcaaatggg tggcacaatt ggctttaaga tgatcaatct attggcagtg 6240
   tatcatagag cttcccaact tatctgtgcc agggagggat caacagtgca cacctgaaac 6300
   ttattacaga ctaatttcaa gtgatattgt actgcttcag gtatgatata agaactttat 6360
   aacgctttca tttctccctt cctggtcttt gtgttattat tgctgcacat tttccttcta 6420
   catatgttat gagacccaca atacattttt aattttgctt cagttatgtt ttgaaatgtt 6480
   ttaattaata aaaatatctt acttatgtac ccatgtagtt accatttcta ttgcttcgta 6540
   ttcttttttt ttttttttga gatggaatct cactctgtcg cctaggctgg aatgcagtgg 6600
   tgtgatctcg gctcactgca agctccgcct cccaggttca caccattctc ctgcctcagc 6660
   ctcccaagta gctgggacta caggcgcctg ccaccatgcc tggttaatat tttgtatttt 6720
   tagtagagac ggggtttcac cgtgttagcc aggatggtct caatctcctg acctcgtgat 6780
   ccacccacct catgtttcca tttggtataa ttttcttttt atctggaggg cttccttcaa 6840
   catttcttat agtgcaggtc tgctggtaac aaattatttc agtttataga tgtctgaaaa 6900
   tgacttcatt ttatcttcat ttctgaaaga catttttgct gcataaattt ttataggtct 6960
   agaattattt cttttagccc tttaaagata ttgatctagg gtcttttggc ttgcattgtt 7020
   tctaatgaga aatctgctgt cattcttttt tctttttttt gctcttcttt ggcagctttt 7080
   aagattttct ctttagcagg tattgtaagc atttgattat gatgtacctt agtgtagttg 7140
   tcttcatatt ttttgtatta gggcttgttg cccttcttgg atctgtgggt ttacagttac 7200
   tgtatttggg gggtttgtag ccattattta ttcaaatttt ttttctatta attcctctct 7260
   cataatcccc ccttgaggga cttccattac acatatattt ggatatttta cattgttcta 7320
   caattcatta atgctctgct cattttttca atcttctttt ctcaccgctt aattttgggt 7380
   agtttctatt gccatgtctt gaaatttact aatttttgtt tttctttctt tctttttatt 7440
   ttattttttt tgagagagag tcttgctctg ttgcccaggc tggagtatag tggtgcaatt 7500
   tcagctcact gcaacctcca cctcctggat tcaagcaatt ctcacatctc agcctcctga 7560
   gtagctggga ttacaggtgc ctgccaccat gctggtctaa tttttgtatt tttagagatg 7620
   atggggtttt gccattttgg ccaggctggt cttgaactcc tgacctcagg tgatccaccc 7680
   accttggcct cccaaagtgc tgggattata ggtgtgagcc gtggcacccg gccgaagttt     7740
   actaatcttt tcttttgtaa tgtgaatttg ccataataca atctaatgtg ttttctcaaa 7800
   tctaagatac agctgatttt atctcaatta gttattttac cttaaaaaaa tctcttttgt 7860
   gtctctgttc aacatgcttg atctttcctc tagcatcttg gccttataga atagtcataa 7920
   taaatgtttt catgtccttg cttgttaatt cttgctctgt gttagttctg catcagtttt 7980
   gattgattca tttttctcct cctgttggtt atattttcct gcttctttgc atgcatggta 8040
   attttttatt ggatcgtagg cattgtgagt ttgacattca ggttagacct aacatataca     8100
   gaacactcca cacaagaaca gtgaaataca catttttatc cactgcatgt ggactatttt 8160
   ccagggttga ccatatgtga agacataaaa caatgcttag tacactaaaa aaagagagaa 8220
   atcatacaaa gtatattctc tgatcacaac gaaatgaaat tagaaatcaa taaaagaaga 8280
   aaatttagga aagtcacaaa tatgtgaaaa ttaaacaata tactcttaaa tatcaaatgg 8340
   gtcaaagaag aaattaaaag agaaattaaa aatactgtaa gattaaagaa aatgaaagca 8400
   caatacacca agcttatggg atgcagctaa agctatacat agagaaaaaa ttaaagaaat 8460
   aaatgagtat attacaaaaa aggatcttaa atgacttatc attccactct aataaactaa 8520
   aaaagaagag aatactaaat gccaaatatc agaaggaagt tcataattaa gatcagagta 8580
   gaaatagata aaatggggaa tagaaaaaaa tagagaaaat caacaaacca aatgtaggtt 8640
   atcttctctg aaaagataaa caaaactggc aaagcattca ctggactgat caaaaattga 8700
   aagatgactc aaataactaa aatcaggaat taaagagggg accttacaga aataagaggg 8760
   attataaggg aatactacga aaaaattata tgccaactaa ttaaaaagcc tagttgaaag 8820
   gcacaaattc ctagaaagac acaactgaaa ctggatcaag aagaaataga taatctgaat 8880
   gggactataa taaataaaga cactgaatta gcagtcaaaa aacttcacac acacacacac 8940
   acacacacac acacacacac acaagaaaaa aaaaaggaaa aaagcccatg cccagatggt 9000
   ttcactggtg aattccacca aacatttaaa agagatgagc agcaatcctt cagaaaacta 9060
   tttcaaaaag ttgaaaagga agggacactt gcgaattcat cctagaaagt cagtattacc 9120
   ccggtaccaa caccagatga aaacatcata tgaaaagtaa aataaagacc aatatccctg 9180
   atgaatacag acatacaaat cctcagcaaa acactggaca tgaaatagag cagctataaa 9240
   agaatcatat gccatgacca agtgggattt atcctaagaa tgcaacatta tttcaacatc 9300
   caaaaatcaa tcaatgtaat gcaacatatt aatagaataa aggatggaat tgcataatca 9360
   tctcaataga tgcagacaaa atgtctgata aaatctgaca ccctttcaag attaaaaaca 9420
   acaacaacaa caacaacaaa ccaacaaaaa tcagcagagt agtaatagaa gaggactttc 9480
   ttaacctgat aatgggcatc caaaaaaccc cacagctaac attatactta atgatcaaag 9540
   accagtagct ttccctctgt gatcaggaag aagagaagga tgtttaatcc taccacttct 9600
   attcaacatt gtactaagaa tgctagctag ggaaatttgg caagaagaaa taaaattcat 9660
   attggaaaaa accagctcta ttctcaaatg acctggtctt gtatatagaa cattctaagg 9720
   aatccactaa aaactattag actactaaac aagctcagta agtttgcaag atacaaaatc 9780
   aatatatgaa aattaattgt atttttatat attagcaatg aaaaatttga aaatgaaatt 9840
   aagaaaacaa ttctacttac aatagcttca aaaagaataa aatagcataa acttaagaaa 9900
   agaagtataa gacttgtaca ctaaaagtat aaaatattat tgaaagtaat tttaaaatcg 9960
   ctcaattaat tgaaagacat cccatgttca tgaatcagaa gacttaactt ttttaaagat 10020
   ggcagtactc cccaaatgta tcccagattt atcacaatcc ttatgaatat ccaaattgcc 10080
   tttattgcag aaattgagaa actgctcata aaatacacaa ggaaatgcaa aggatggaga 10140
   atatccaaaa caatcttgaa aaataacaaa gtcaatgact tacactccca ttttaaaact 10200
   tactacaaag caacggtatc caagacagta ggatgttctc agagtataaa tataaaaatc 10260
   aataaaaata gaattaagaa ttcagaaata tgcccttacg tttatggtca attggttcca 10320
   agaaagatgc cagcaattca gtgggggaaa gaataatctt cttaataaaa ggtgatggga 10380
   catctggata tccacatgtg aagaaaaaat ttgcatctct atctcatact gtacacaaaa 10440
   atttactcaa aaagcaatca tagaactaaa cgtatgatct agaactgcaa aactcttaga 10500
   aggaaatata aatataaatc tttgtgacct tgaattgggc aatgcttcct taagtataca 10560
   gcgccagagc acaagcaaca acaaaataga taattgcact tcaccaaaat taaatccttt 10620
   catgttttaa agtactctct taagaaagtg aaaatatagc tcatggagtg ggataaaata 10680
   ttggcaaatt atctgataat gaacttgtat ccataatatg cacaaatctc tgataaatta 10740
   acaataaaaa gacaacccaa ttaaaaagtg agcaaaagat ttgaatggac atttcgctga 10800
   agaagataga taaagggttg ctaagcacat taaaagatgc tcgacattac tggtcattag 10860
   caaacgcaaa gcaaaacata ctaagatgcc atccactaga atgacgggat gaaagaggcc 10920
   agaaataata aatgttagtg aggacataga gaaactggta cactcattta ttactggaag 10980
   gaatgtaaga tggtacagct acttttaaaa tagtttgtgg ttctttgaaa tgttcaacat 11040
   agagttgcca tatgactcag caattccact cctaggtatc tatgtgacat aattaacaac 11100
   acaaaaatct gtacaccaat tttcatagca acattattca aaatagccaa aacatagaaa 11160
   caacccaaat gtccattaat ggatgaatgg attaacaata tgcagtatat ccaaacaacg 11220
   gaatattatt cagccatgga aaggagtgaa gtactaccta tacacagtac aacatagagg 11280
   aaccttgaaa gcattatgct aagtgagagg agctagttac agaagcccac acattgtata 11340
   attccattta tatggaatgc ttagaataag caaatttata gaatcagaaa gtagtatata 11400
   gattgccagg ggctagaaga aaggggaacg aagagtgatt gctaatgagt atgaagtttc 11460
   ttttgggggt gatgaaaatg ctctgaaatt ggatagtggt gatgggtgcg caactctatg 11520
   aatatactga aaccctttga attgtacact tttaaaaggg taattttatg ctgtgtgaat 11580
   gacatctcaa taaaattctc attaaaataa gaatatatga gagtatctat ttctaactgt 11640
   agagattttt gcaaatcctg cagaaatatg ctgaattagt tacaaccagc taagggagcc 11700
   tgggatggaa gagtttgaac cacacccaat ggcctgcatc tggtgctctc ccaagatcca 11760
   tggaaataat cttccaacct gcaatgtacc agggcatcta ccagcctggc tccacggtgg 11820
   tttcaggcat tgctctacaa atgatcaagt tttcattcta gagtacgaat gaggattgag 11880
   ttagactaac tgtacaaggg acaggaaata acagctcctt ccctgtcctg gggtcagtgg 11940
   tttgaaaagc accccatcta tcagtgaata tccagcttcc tgacaaagaa aagggctagg 12000
   ggatttgccc ccaccctgtt tagaaggcga aggcgaatta ggcctgaatg gcgacttgat 12060
   tctcatctga atatcagaga aacacatgtg ttgcaggcta cactggaagt cctaatggct 12120
   tcagtttgat agtggatttg ggcttaatgg ttgagcaact ccagtggctt cctctcttga 12180
   ttacgttggt ggctgtgaga tgaactaccc agtcatcagt catcattatc aatggcccat 12240
   ttgcctggga tgctttggaa gaatgaaaga gagtaaaggc agttctactt ttaaagaagt 12300
   taaaacatgg gctacagagc attttcgttt attgtttatc aaatgttagc ctgcctcaga 12360
   atacctggag ggcacagatt cctaggtccc acctctggag ctgctaagtc agttgatgtg 12420
   ggcaaataac ctaacacctt ggcattcact tccaggtttt cttcatttcc tcgtcctccc 12480
   tttccttctt caaatgttct cctgttcctt cttggcttta taactatctt tgtctctgct 12540
   tcccttccta tacattgcct taattaatcc tttaatttag attaaattaa aggataaata 12600
   taaattaaag gataaattaa aggataaatc taatccttta atttaaatat tatttctgga 12660
   gatatatttg ccatgtgatc cattttgttt gttagaaaaa cacaagagaa aaaaattgct 12720
   gataatcttc tcaaaaagga ttatcttgtt tgttctaaag ccaaggatgt atagtgcatc 12780
   ttggaatgtt ttggattgag gtgggacatt tgctttcttt ttgttttcaa acatcattaa 12840
   tttacttggt ataagaactt cagaatcagc ataaagtaaa agtagtttct taggaatagt 12900
   gtcatgataa ttccatgcac ttaacatgag gtaggtgata acggaagtag tggttttctt 12960
   gatttttctt attcaaactg cttcatcaaa caaaacaaaa cagaacaaac gccctggaga 13020
   cagagagtct tattgtgcca tagtatttct ctttctctct cttttttaaa tatttcaagc 13080
   gagcatgcca tcagcagggc tgtcttcact gagctatttg ttgctgggag atgttgtaga 13140
   ctgttgctcg gtcagtgcaa atgagagctg ctagcctcgc tgtaacctcg ctggctgggt 13200
   actctcagag attgttaaga gttttaaaat ttttctaatc actaagcatg ttaggaaacc 13260
   agagctttgt gtatgaagaa caacttcctc tcagggactg tactcaaggc tgaggtttgg 13320
   acctcagcca attttatgaa cgaacaggaa caatgtcaaa aacatcagga ttcctggtca 13380
   tgaggaccag agggggccac tccaatggga tggtaagctt ctgtagggct ggcacgtgac 13440
   tttgcacccc tgatgctccg catgaaggga ttttctgcac ccagcaagtg ctgactgatt 13500
   tcctgaggac acttagtcct actctaaact ctaagaaggt ctagggccac atcaggaaag 13560
   tgagattcat gcactgactc atttccacac ttcagaggac actttctttg agtgggtgtt 13620
   tggaaaaaaa tgcaaggtag atgtaattct ccacacatca gctgatgact cgtactgaca 13680
   gtgtctagaa tagaactttt tttttccttt tgggtaggta ccttctcccc agatggatgg 13740
   tgtgttcccc agagtgccga ggtatttctg ggtgaagtaa tattgttccc ttgtgtgtaa 13800
   tcctactagt tctctttcat gaagtagctg gaatgtctga gtggtcaagg gtaatccctg 13860
   agctggcctt gacacaaaca gatacgggct cttccatcct tgggtccctc tgttctgaca 13920
   cctgatgaca cacttaggag ttttcctcta gtacctaaaa ctatagttag taggtactta 13980
   gaaacaatta caagctgtgc taatgctttt taaaagtgga aatatcggcc aggtgtggtg 14040
   gctcacacct gcaatcccag cactttggga cgccgaggca ggtggatcac gaggtcagga 14100
   gttcaagacc agcctgacca atatggtgaa accccatctc tactaaaaat acaaaaatta 14160
   gccaggcatg gtgatgggcg cctgtaatcc cagctactca ggaggctgag gcaggagaat 14220
   tgcttgaacc tgggaggcag aggttgcagt gagccgagat cacgccactg cactccagct 14280
   taagcgactg agcaagactc cgcctcaaaa aaaaaaaagt ggaaatagca atatgtgctt 14340
   tagctaaata ttaattttgt ttttgccctg ccttttagct ctgtggtctt gaccaaggcc 14400
   tcatagcgac tctgaatctc ttgcgactct gaatctcttg cgactctgaa tctctcgcaa 14460
   tctcaatttt cttgtttgca aaactaagat gtagcagtta taatgtgagg cgcaaatgag 14520
   aaaggccata caaaaaccct aaccaaaaaa tccagtaaaa tgaagctcag ttaaactaaa 14580
   gacagcccgt tgacatcttt tgtgcgtttc ctccttggtt cccaggaatg ctggaaaaag 14640
   aggcatttgt tctaaacatg gaaatgcagc aaaataatga ttcttttatg gcctggtggt 14700
   ctggtttgac acaatcaata actaaaataa atacacacag cgtacagccc tgtaataata 14760
   gaggttgctc ggaatcaact cacagtagta aaaatcctgg ttgagaaatt atccccaaag 14820
   atgatatgat tttatcgccc cgtacaaagt taaccagtct tatacataat atggatcttt 14880
   tcaaactttt aaatgaagta taatgcatac acagaacagt gaactcatca tgggcaacta 14940
   aattaatttt cacaaaatca tattttgtga atatgctatg atttattaat acattgtact 15000
   gctgatgaac atttggatag tttctggttt ttggctaata aatatagggc tgctatgagt 15060
   atttttgtat acatactttg gttgacattt ctactatttt ctgttggtct tgtacctgtg 15120
   agtgaaatta ctgagtcata agatatgtat ttgttcctca gcagtagtta ctgctgccac 15180
   acagcatggt tctgctaata tgtacttcca gtgggaatgt ttgacagttc cagctgatgg 15240
   aaatctttac cagcgtttaa tattttctct tattcatttt caccattctg gtaagagtgt 15300
   cccgcaattt cattctggct ttaattgaat ttccccaatg gctaatgcag ttgaacactt 15360
   tttcaaatta ttttttggcc atttaaatat tttcttttgt taaggatcag ttcaaccttt 15420
   tgtttgtatt tctattgggt tacctagatt tttcttactg atttgcagtg gtttttaatg 15480
   cattctgggt aagaacactt tgtcagcagt atctattcca aatacctgct tcaactctgt 15540
   ggattgcatt tcatttccct aatggtgtct ttggatgaac acaccttgat ttatcaatag 15600
   tctttctttg attattagaa tattttgtgt ctgttttaca actatttgcc tactccaaag 15660
   tcataaaaat tttctcctgt gcatttcttt aaaggcttta ttgttttact tttcacattc 15720
   agatctgcag accaacaatt aagatttgga tatggtatta agcagtggct caaggttcaa 15780
   ttttcatttc tccatttgac tatccaaatg attcagtgcc atttagagaa atgaatactc 15840
   ttttctctct tcagttcagt ggcgtgttgg gcataaatca ggtaactatg taggtgtgta 15900
   tgagtctttt tctggactat tcattctatt gcattggcca gtttgtataa attttcactg 15960
   aaatgctgtc ctaattacta tagccctgat ataaaatgct atctagtagg gtaagtcttt 16020
   actattcctc aagattgcca tagctattat ttgcatttaa tattttcata tgaattttaa 16080
   aatcacctgt caattcccac aaaataccaa cagcgatttt gattgggatt gcaattaatt 16140
   tataaatcaa tttgggaata actgacaact ttacaacatc aaatctttca attattgaac 16200
   atgctacatt gtatagctcc atttataaga tattctttaa cttctcttgg caatttttaa 16260
   aatttttcag tgtagggaac ttacatgttt ttcatatttt cagaagaatc actgtctgtg 16320
   tcagcaatag ctttattttt aggtggttac tatttttagt gagcttgtac atgaagtaat 16380
   ttttgaaaca tttcatttat gacttgttcg ttgatagaaa taaaagtgat tttaatattg 16440
   accttttatc cactagcact gctaaacttg tttactgatt ttaataatct gtacatgctt 16500
   gtgaatattc cagaaaaaca atcatgccat ctgcaaataa tggcattaat ttttaaaggg 16560
   tggtttcact gggtaagatt tcttaattga cagtttagtt ttctttaggt gcttagagta 16620
   tgttgttcca atgccttttg gcctccgtac tttctcataa gaattcagtt gttaatcact 16680
   tcattgtatt tcctgtatgt gatgggtcgt ttttctctag ctgctctcag gatattttct 16740
   ttatcattgg cttttaacaa tttgcttatg atgtgctttc tttgtgatta ttctttgttg 16800
   ggttgtattg agcttgttga atatgtaaat taatattttt cactgtttga aaagttgtca 16860
   gtcattatta ctttaaatat tttctacgcc tttctccccc tctttttttt tggggatttt 16920
   aattacatat attttggcag agccaggggc tctgtttatt tttcaacaga ctttttttct 16980
   ttgtgatctt tcaactgtgt aaaaaactca ctagttcttt atctgtcact aatctgctct 17040
   gggaatcacc tcgtaaatta aaaaattctc gttactgttc tttccagctg tttttaaacg 17100
   ttatctagcc tccctttctc tctcaaagat tttatatttg ttcactcatc aatatcttat 17160
   ttaaaaatta tttgaacatg tttctcttta tttttttgag catattgata cacaacatgt 17220
   ggacctactc agagtgacct acatttttga agcccatatt ttttaagatg tagaattcca 17280
   gttttgcaat tattttccac tcacacatta aatatattat aggtgtatct cagagatatt 17340
   gtggatttgc ttccaaacca ctgcagaaaa gtgaatattg caataaagca agtcacaatt 17400
   tctttttttt ttggttaccc attgcatgta aaagttatgt ttacactata ctgtagtcta 17460
   ttaaatgtat ggtagcttta tgtctaaaaa acaatgtaca tatcttagtt aaaaatactt 17520
   tttcacttaa aaatgctaac aatgatctaa ggcttcagta agttgtaatc tttttgctag 17580
   tgggaggtct acccttgatg ttgatggctg ctgactgacc agagtggtgg ctgctgaagg 17640
   ttggggtagc tgtggcaatt tcttaaaata agacaacaat taaccatgcc acagcgatca 17700
   actcttctcc tcttgaaaga tttctctgta gcatgtgatg ctgtttgaca gcattttacc 17760
   caaagtagaa cttctttcaa gattggagtt aatcctctca aaccctgaca ctgctttctc 17820
   aattcagttt aggtaatatt cataatcctt ttgatatggt ttggctctgt gtccccaccc 17880
   aaatctaatg tcaaattgta attctcgtgc atcaggggag ggacctggtg ggaggtgact 17940
   ggatcatggg agctgttctc atgatagtga gtgtgagttc tcatgaggtt tgatggttta 18000
   aaggtgtggc atgttccccc ttgctctctc tccgtctctc ctgccaccgt gtaagacgtg 18060
   ccttgcttcc tcttcacctt cttccatgat tgcaagcttc ctgaggctgc tccagctatg 18120
   tggaactgtg ggtcaattaa acctctttcc ttcgtaaatt actcagttct ttctttatag 18180
   cagtgtgaaa atggactaat atacccatgt tgtcatttca gcaaatgttc acagcatctt 18240
   caccaagagt ggattccatg tcaagaaatc actttctttg ctcaaccata agaaacaact 18300
   cctcattcat ccaaatttgg tcatgagact gcagcaattg attccacctt caggctccac 18360
   ttttaattct agttctcttg ctattttcac cacatctgca gttacttcct ctactgaagt 18420
   cttgaacccc ttaaagtcat ccacgagggt tggagtcaac ttctaaactc ctgttaatgt 18480
   tgatatgttg acctcctctc attaaccatg aatgttctta atgacatcta gaatggtgaa 18540
   tcctttccag aaagttttcc atttactttt ccagttccat cagaaaaatc actatctatg 18600
   gcacctatag ccttacaaaa tgtatttctt aaataataag aattgaaagt caaaattacc 18660
   tcttgatcca atggaaattg tgttagcagg catgaaaaaa cattaatctc tttttacatc 18720
   ttcatcagag cccttggatg accagttccc ttgccaatga acagtgatat ttttacagga 18780
   atcttttttt ttttcccctg agaactagct ctcaacagtg ggcttaaaat attcagtaaa 18840
   ccatgctgta aacagatgtg ctgtcattca ggctttgttg ttaagtttat agcgcatagg 18900
   tagagtagat gtagcctaag ttttgaaatg agaaatggac gttggcgtca acttaaagtc 18960
   accagcttta ttagctccta ataagaaagt cagcttgtcc cttgaagctt caaaaccaga 19020
   cgttgacttc ttttctccac tataaaagtc ctagatggca ttttcttcca acagaaggct 19080
   gttttgtcta cattgcaaat ctgttgttta gtgtagccac cttcatcaat gatcttacct 19140
   agatatgtgt aacttgctgc agcttctaca ttaacactag ctgcttcatc ttgcactttt 19200
   gtgttatgga gatggcgtct ttccttcaac ctcatgaacc aacctctgct agcttccaac 19260
   ttttcttctg caggtttctc acctctctca gccttcctaa aattgaagag ttagggcctt 19320
   gctctgtgtt aggctttgga ttaagagaat gttgggtctg ttttgatcta ttattgacac 19380
   tactgaaact ttctccatat caacaataag gctgttttgc tttctgatca tttgtgtgtt 19440
   cactggagta gcacttttaa tgtccttcaa gtatttttac tttgcactca caacttttct 19500
   aaccatctgg tgcaagaggc ctagctttca tcccatcttg gctttcaaca tgcctaagct 19560
   tcatcatttc ttgtttttat taaagtgaga gaattgtgac tcttctttga atgcttagag 19620
   gtctttgtag ggctattaat tgacctaatt tcaatattgt tgcatctcag agaataggaa 19680
   ggagacagag agatggggga gtagctggtc tgtgggacag tcagaacaca tttattaact 19740
   tttctgtctt atgagtgtgg tttgtgtcac ctcaaaacaa ttacgatagt aacatcagat 19800
   attgctgatt gcagatcacc ataacatata ataataataa agtttgaaat agtgtgggaa 19860
   ttaccaaaat gtgacacaga gatatgaagt taccacatgc tgttggaaaa atggagctga 19920
   tagagccaga tgcatggttg ccacaaacct tcaatttgta aaacaaaaca aaaaccccca 19980
   cagtatctgt gaggtacaat aaagcaaagt gaaataaaat gaggtctgtt tgtacctcgt 20040
   tgtcttctag ttttcactgt ttctatcaag gaggcagttg taagtcttcc tctgaatgcc 20100
   tttgtttctc tgactgcttt taaattgttg ctattttgtc tgtggtattc aagagtttta 20160
   ttatgatggg tcaaggtgta gttatctttg gtttatcttt tttttttttt ttttgagaca 20220
   gagtctcact ctgttgctca ggctggagtg cagtggtacg atctcggctc actgcaagct 20280
   ccacctcccg ggttcacgcc attctcctgc ctcagcctcc tgagtagctg ggactacagg 20340
   tgcccgccac cacacccggc taattttttt gtatttttag tagagacagg gtttcaccat 20400
   gttagccagg atggtctcga tctcctgacc tcatgatccg cctgcctcag cctcccaaag 20460
   ggctaggatt acaggcatga gccactgcgc ccagcctatc cttttttttt caattttttt 20520
   tttttttttg taatttggcc ttctagtcct ttgtcccctc ctcatcacca ccaccaccta 20580
   ccatggggag ttttgggtgc aggaacctaa atccccacaa aggttctatg ttgggggtat 20640
   gtaataacaa ataagtcaat cagatcaaat attattttcc caccaacttg gaatttgcta 20700
   agccccatga aaatgtggtt caccagtttt gaaaaacgtt tggccactat ctcttcaact 20760
   gttgcttctg atttgctctt tctgtcttct tctaagatta attatacata tgttagactt 20820
   cttgagtata tcatgcatat ctcttacgct ctttttatta tttttctctc tatgcattgg 20880
   ttttgatatt ttctgttaaa ctacctccac atttgcaaaa cctgtggtct gctgtgttta 20940
   atcagctgtt aacccatcag ctgaattttc acttcagcta ttatattttt cagttctaga 21000
   ttgtctatta gattcctttt tatagatttc aatccattgt tggtattttt tatcctttta 21060
   cctgtttagt tcatgttttt ctgcatcttc ttgagggtat taatcacagt tatttacagt 21120
   cttagtttgc taactataat ttcttgatta catgtgagct gcttccatat tctgcttttt 21180
   tctcttaatt attagccata ttttctcgtc tctgtggtaa atttttaatt ttgccaaaca 21240
   atgtgtatga gaggacaata gaggcttcgg ctaatgttat taatgatcag ggagtttctt 21300
   cttttctgtt cagtagatgc aatgaggtat tggtaaactc agtccaacca gaattttcca 21360
   ttttagtaaa acacattaga acttggttta cttgtgtttc tcaattgaga cttttctggg 21420
   cttttcatag agagccttgt gagtctctgt tgccttagtt ccaaaaaaat tgtgggagaa 21480
   ccagatatac cctccagaga ttcacagccc agctctcaag tgatcagtct catagagatg 21540
   aaaaatctgg caaatgtttt gatggaaaac aggttatgtg cttgaggcag ggcttcttct 21600
   agtagttttt tttctttctt tcttttaatc tatcaatctt taacagcatc ttggatagcc 21660
   tatgagtgta tgtacttgac aaacattgcc tctttatggt tcacagtagt ttctaaaata 21720
   cattcacttt attggccatt atcctttgaa agctgatttt gtaatcttga taaagtggaa 21780
   gtggatttta tagtcttgag aaagtggaaa ttaacaaggc agcttcatgg ataatgtatc 21840
   cttttcttct ttctgaaaat tatacatact attttatata tttatggggt acatgtgata 21900
   ttttgttgca ttcatagaat gtgtaatgat caagtcagag tatgtggagt atctgccatc 21960
   ttgagtattt accattttta tacgttggga atatttcaag ttctctcttg taactacttt 22020
   gaaatagaca atacactgtt gcttactata gtcaccctac cttgctctcc aatagaacat 22080
   atgctttcta tctaactgta tgtttttacc cattgaccaa gctctcttta tccccgtctt 22140
   ttacccacac agccttccca gcctctacta tctattattc ttctctctac cttcatggga 22200
   ttaacttttt aagctcctat acagaataag aacatgcaaa atttgtcttt ctgtgtctgc 22260
   cttgttccac ttaatgtaat gacttccagt tccattcatg ttgctgcaaa tgacataaaa 22320
   tcattacttt ttaatggctg aatagaaggt ttttgatatg taataactta aagcttcatg 22380
   agattttact ctgccatttc caaagcttcc acatccattt ccagttacct acactatccc 22440
   agaactcagg aaatccctat tttgcaaact catcatttgt ttgagacccc cctcaagctt 22500
   ctgattgtca gagtagcccc acataaccat taaaagcttt gctgctttct ctttttccag 22560
   cagaggtcct ctgtgtgggt caagccaagt cttcagcttg cagccaaaat tggtaatgtt 22620
   ggcagagggg cgggaggctg gcaatcatca attctcatct taatcattgt cattgtctct 22680
   tctctgaaaa ttttgttttt ccgctcctag tcgttttctt agctctttga ttcttaaaaa 22740
   aaatacagct tcttcaattt atccatgttt gtccaatcat tgtagcatta tccagaatat 22800
   cctttatcaa ttaaatcctc ccaggaagca gaaggtcttg aaactcttat tttgatattc 22860
   atttcctgac caactataga aatctatgtt tcttaagtgc tctttaaacc aattcttttt 22920
   gggatttttc cctattcatt tcctcattaa tgagctaaat aacactcggg cctgttctaa 22980
   acgtaacatt gaaaattttg cacgcctcat ataaactccg agaacagggc aaaggggagc 23040
   atgtttatga cactaattct tctagcagtg aactcttgct gaaaaggtct ggaatttact 23100
   ggcaaaacag ggatggtggg agggcagcaa gagaagaaag gatgggggtg gagccgtgtc 23160
   caaaggaaga tgcttttccg ctctctagtt ccatgactat gatttttttt ttcagccaga 23220
   gtaagtgcat cttgtctata gaggtttact caggattgaa tgaaatttcg ccaagatgta 23280
   cttagagaag aaaactcaga ggccagaaag agagattgct ctatacctaa accaaaacta 23340
   catatagaca tctccaagac cttgagactt gaaggtgctg attagcagtt ttggaataag 23400
   tgggtgatta ttaacttgtt tccacttttg ttcattaaga attgataaga aaataatagt 23460
   tgccttagtg gaattgcctg ttattcaagt aactcacgta gaacgtttct gaggatttaa 23520
   gttcctatgg ccaaaactcc ccaaggtagt tagtggttga agttggtgaa ggagaaaaaa 23580
   aaggggtgga gaatcaaatg aagagcagtc attttaaggg acagaaaaga aaatgtcatc 23640
   aatctagctt atgttattga gaaagctagc aaaagtcctt tccggaaaac ccttgtgagt 23700
   cttcatttct ttggggtatt ttgaagtaga atgtctagat tagataacag gtatatgttt 23760
   aacttttggg gaaaatgtaa atttgtttcc caaagcagct gtaccatttt acattccctc 23820
   cagcagtatt tgagttccag ttcatccaca tcctcccttg cacttggaat ggttcaaact 23880
   ttccataata gccatcctaa gaggtatata gtggtaccgc attctggatt aatttgcatt 23940
   tccccaagga ctagtgatat tgagcatttt ttcgtgtgct tatttgccat ccatatatat 24000
   cttccttggt gaaggatctg tttatatttc ttgcccattt ttcaattggc ttatttattt 24060
   tcttgtattg ctttttaaga gttctttaca tattctggat gcaagtccag tggtgtcagc 24120
   cgccccactc cccagcacct ggtctggccc tacctgcagt gggacctccg cgtgaccctg 24180
   gtccccacct cctctgagtg tccgaggaga cacgcgcggg gcagcaggct cccggcagct 24240
   gcagtggggc atccccgcgg gaccagtcct cctgcagcag tgtggggtcc agccacgccc 24300
   ggttccagaa cctgcctccg tcctgagctt cacctgcagc agaaatacac catgatctgc 24360
   cctggggaaa acaaaacaaa acaaaaccaa ccaaccaacc aaccacacat ataaaacttt 24420
   aataggaagt ggaatagaag tgactacaaa atactaccat tcaatttcat catgcaagta 24480
   ctttaatatt ttcactcact ggcaaaataa agcacatgta actctaaatg tgaaataatt 24540
   ctgagtcttt gactaagtct tctaaagaaa gaataaaatg ctggtgaaaa cgtgaaattg 24600
   tgtagtgagg gatttatgac cttgtttatt atttttctac agatgttaag atgatcatga 24660
   ttaataccaa ttgaagcatt tacagaatgc tatttaggcc caatagtgat gttaaattac 24720
   agtatcagag aacctgatag atatggggtc tgattataaa gaaactttat tcaaaatcat 24780
   ctttgtcaaa gttttaacac agaagcaaaa gtcaaatatg ttcctacatg agatacaagc 24840
   tattcttgct aacagtacaa attctgtcct gtaagtatta gtattttcag gtttttcctt 24900
   ttctatgaaa cggttgaaag gaaaacttta gtacgctata actcataggc tactttgtgt 24960
   atttatcatt ttataaacaa atgtcttgac aattaagttg tgtatgaagg ccatcatcaa 25020
   gtcattaact cttatatcaa aacctattat tattcactga tttagataat tcaatttatg 25080
   tgtgcgttac caggttgcag tcagccaaca gacacagaac aggttaatag aaatatatat 25140
   atttttctta tcaggttttc ttttattgga acaggttgca tatatataca cacatataca 25200
   catatatgtg catatatgta tgtatatttc cattgatata atattctaga aaatgcagac 25260
   taatcactag taacagaaaa gcagatcagt catggcctgg ggaggggcaa gagtgacgaa 25320
   ttacaaaggg gcgtgagaaa aattttagga tggtggacat attcactatt ttggtggtgg 25380
   tgatggtttc atgggcttat acatatgaca atacttaaca gatacgccac ttcacagctg 25440
   tttcctaaga gtttaacctt tcagctttag gcccctcccc caacacggtg tcctttctca 25500
   ttgccacatc ataaaccagt ctttcccctg agtcacacac atgcaatcct tgcacagcaa 25560
   gtcatgatgg tgccctgtgt gtatgtgata gtgatgggaa aaggataaaa caaaacactc 25620
   aacttgtgtt atggcaaaaa gagattgcag tgatgataaa gaatgcagga aaggaagcca 25680
   atttatctgt taggaaaaaa cttccacttg cttataaatt ctgtttcctc tgatgaagtt 25740
   tcattataaa gtgttttctt tttctttaaa aacaagaaga tgagaggaca ttgattactt 25800
   aaaaattttt acattaaaga atactggaaa atttcagaga ataaatgtaa tgtataaggc 25860
   ccatataagg ccttaggttg aaaacaacat gcacccatga acttgctaag taatttgaga 25920
   aatagaacat aattggcatc tttgaagttt tctgtatacc tctctttcta cctccctgct 25980
   tccccccaga aatagctgct cttctgaacg tcagtgtttc tgtagtgtta gcatttatgt 26040
   ttgcatataa aaaatacatt attttacttc atttaaagat ttttaggccg ggcacggtgg 26100
   ctcacgcctg aaatcccagc actttgggag gccgaggcgg gcggatcatg aggttaggag 26160
   atcgagacca tcctggccaa catggtgaaa ccccatctct actaaaaata gaaaaattag 26220
   ctgggtgtgg tggtacgtgc ctgtaatccc agctactcgg gaggccgagg gaggagaatc 26280
   acttgaaccc gggaggcgga gcttgcagtg agccgagatc gtgccactgc actccagcct 26340
   ggcgatatag ccagactctg tctcaaaaaa aaagaaaaaa ttttataaaa ataatgcact 26400
   attcttcctt ttttcatttt acgttttgtt tttaagctgt attcctgtca atatatgtag 26460
   cacaacttag ttttcacact gtgtcctatt cagcgtgtaa atatactgta attgacgcat 26520
   tcattttcca ctcaatgaat ttggggactt ttacaggatt ttttgctatt atgaaaaatg 26580
   tttctctgag tgttcctcta ctcgcctcat agggtgtgta ctcatgatat tctttagaca 26640
   aagtttaagc tgaaccttca gataggcacg tgttcaaact gttttccaaa gtggttgcag 26700
   tgatttatat cacatcaaac agagtttcta cttgttgcca acatctggtt tgttagactc 26760
   tgaaagttgt ttgcctacct cggaaatgtg aaatagtttc tcatttgtgt ttctaatttc 26820
   cttgggtttt taagttaggt atcttttcat ttagtttatt ggcctttcag gttttctatt 26880
   ctgtaaaatg cctgtttttg tctttttttg cctatttaat attttttttt ctcttcctaa 26940
   ctgattctag gcattctaca tgtttgtgtt actaactctt tataagttat ttgtgttata 27000
   aatgtcttcc tcttctggct tgtcttttat ttttttaaat gatgtctttt ggtgagtgga 27060
   agtttttaat aataatgtag gcaaatatat taatctttat atattttaaa gcctttgagt 27120
   ctttttaaaa cttttaattt taccatcctg acattctctt agataaatat attcttttgt 27180
   attttattct taaagatggg agattttgcc ttagtatgag attcatctga agtggatctt 27240
   tttttttttt ttttctgcat gggatgaaat aaggaaccaa ttctgtttcc tcagcatatg 27300
   ctttgcctgt tgtcccagaa gctgttattg aacaacgtgc attgccacct ctgtcatgta 27360
   ctaactttcc ataagtattt gtcaggatag gaaatattac aatgaagttg caaagcttga 27420
   aatctcagtg acttagcaca acaaaagttt acgtctcaca gttgttaagt ctaaagttgg 27480
   tcattggctc tttttcgtgg ttcttttaga atggtgactt tatcggggat cggggctgct 27540
   tccttcctgc aaccctacaa gctaggaatt attttctttc atagcttcac atataagaga 27600
   aagataggga tgatggaaac tcacatgagc tcttaactgc cctggactag tggcttggtc 27660
   ccaaccataa acaagggagg gtgtcaaatt tggggagcat atatacatat ttaatgaaca 27720
   ttaattgtct gtaccatatc atctaagtgt ggacctcttt ctgtttcctc tattctgttc 27780
   ctggtctatt tgtatatcct tgcaataatc tcttaattgc taaagctttg taataagttt 27840
   tgatatctgc ttgtacaagt ccctcatcta gaaatattga tgaaagaaaa aagactataa 27900
   aatattagga atgaacaggg atgtataact atggacatgg caatatttaa agataagaga 27960
   ataccaagag tgactttaag ccaaaacatt tgaaatttta gataaaaaaa caaattccaa 28020
   gaaaaacata atgtatcaaa attgactaaa gaaaaaataa aaaaatctga atagtctcat 28080
   ataatattaa atatttgatt catattataa ctatcctaca aagaatagtt tgtataaaga 28140
   aaatagtata gtttctacaa agaaaatgtt ttacctaaat gatatttgag tgagttctac 28200
   caaatattca agcaatagat aatgtcaatc ttatacaaaa tttttagagg ataaaaatta 28260
   aaagaaacac attctaatat attatatgaa gctggaatac cattgacacc aaaactagaa 28320
   aatcccagta taagaaaata aaatcatatt tcaacttaga ggcacaaatc ttaaaatgaa 28380
   ataaaattat cagtgtataa cccgtggaga ttttaaaaag atgagaatcc atgaccgtgt 28440
   tacgattata ccagaaatat aaagatggct taatctcagg caatctatta atattatata 28500
   tcatatatag agtaagggaa aaactgttat taatagatgc aaaaatagtt tttcataata 28560
   tccaatatct attcttgata ctctcttggg aaaccaggaa gagaggaggt cattcttaac 28620
   ctgataaatg atgtctacta aaaacctaga agtaaatctt gtactctata aaacactggt 28680
   tctctttaaa tcaggagcaa tacaagaatt actattatga attagtttat ttaatgtttt 28740
   gctggagttg ctggccaatg cagcaagata agaaaataac aaaagtaaaa agcactgtaa 28800
   taaaatgaac agagctatta ttatttgaag ataatatgac ctgtccacat ggtaaaccaa 28860
   caaaaaatgc ttgcttttta ctgttgagaa gctgggtcat attcgacagt caagaattct 28920
   ggaataagtt gtgtttcatc aaatccctat ttaattgcat agtacactgc agtgaaagta 28980
   tgattatgaa agccatataa tagcataaaa actatttgtt aagtcaaaag agattacaaa 29040
   gatgtatgta cataggaaaa aaaagagtag gtgggaatgt tgcttaatgc taaagggggt 29100
   taggtggcaa gatcatgaat taattttcca tctattcttt atgtcccata tcttctgtgc 29160
   tgtggctata ttgtttacaa caaaagtatt tttaaaattt gtactaatga gaacctttgg 29220
   aatcttcctg cagatttagt gatgcttgtt gatttgtatc agagagctgt tgaagacaat 29280
   acagtttcac ctaacgttta actgcacaat aagtcagatg catttattgg gttgtgactc 29340
   atacttagaa cctgacatat aatgaatacc agtaagacat catcttacct tattaaacac 29400
   aataacttcc ttattcacta aaataactaa aaaaggcacc gcgcccaaag ttcacagaaa 29460
   tatacatcga ggcattgtga tagagtaaaa gagaagaaaa aggagttaaa caaagtctgt 29520
   agatccaaat cctagtcttg gttttcccat agtaggcttg gtttcctatt tcaaaatgtg 29580
   aatagtgaga aactgccctg tttataacat tgatttgtca tgagggcaaa acaaagaacc 29640
   caaaattaga gggcctagaa gagatggtag aagcagtttc cattggatgt tcgtacccat 29700
   ccagtggcct ttgtttgagt tcttccaggg atggggcaaa cactgtctct gcattgctga 29760
   gtgcttaatc tattgttatg aagatctttt tcacattggc ctcctggacg tctatcccat 29820
   ggccttcgtt ccatctctgg gttagggtac catggctcct agcatggtct ggtggctacc 29880
   aggtgtacag atatcaatcc tcttagtcta agggcctgtg ggagagccta gctggtcttc 29940
   ttcggccatg aacagccttt gttattgttt tctctgcatg ccatgacaag agcgtgtctg 30000
   agggcattct ccagtgatga cctcctttgt atcagaaggt acacttccac cagggcaact 30060
   acagcctcct accacacatc attcactgtg gacgtcttag tcagtcaaga ctctggcatt 30120
   tgtttggttt tgttttccac cagtgctttg ctttttcata tgccctgtcc tgtacttaac 30180
   aatatgaggt tagggaacta agaatgggat tttagatttg tctgtgttaa gtctcactat 30240
   gttagatttg gcctatggtt ctatcttaat ggcgtctttt gggaccctga tttgctctct 30300
   cagtgaattc acagttcatt ttaactccag gactcccaga atgtggtaag cttgcctgct 30360
   ctgcccttct gctgtagaaa cctgttctgc acctcccagc tccctcccca acccctggct 30420
   tggcctcgag gcattctcct ttcctgcatt caaggcctcc aggaatatta ccacacacca 30480
   tctgcacatc gtcgggttct tttggccaat gccaatttga taaatttttt tggcttcagt 30540
   gtccagttct gctttctaaa cttgtatttg cttcctttgg aaaatgtatt ccttctttct 30600
   accctccctc ctttcctgca ttcctctgct ctgtactctc cacaattgaa gagtgccatg 30660
   accatgtcca tgcccagcct ggctgcctct tttctgggag gagaggtcag ctccacacct 30720
   gagagccagg atggaaccaa atacctggaa ctgagcctga ggctttccag tagagcatac 30780
   tcttctaatt gactcattta ccattgagta tgggtgttta tacatgtatc caactctact 30840
   aaccatattc tttgttttgc tcacaagaca gatgtttgca gatacttaca tgaaattctg 30900
   attcactaag ttcacagcat ttaactgtgg taatctcacc atctaacaac ctcatcaaac 30960
   aaaagattgc atgagcttca ccagacctgg cctgttttta gtcatttcat gctttctctt 31020
   ggtgatcgtg gcttccttaa gtgctgaaag actatccctg taatcattca tctagaatct 31080
   ttcccattcc ttcatctgtg cttgcaaaat cccttaaact cagtaatggg tgtggaagct 31140
   gtctgtctgg aggtatgaag aattaaggga tggcattctc cccggaggtt tgatagaaag 31200
   ctggacacag tgcggtctgg aagagtcatg gggatgatac gaattgttca tgtatcattg 31260
   cctggaatag gagtatgata agcagccagg cactcaagtc agatccttag ggcctgacca 31320
   caaaggttac cagtggccat cggggatggc atggttaatt ctctagtgta aagagacaaa 31380
   ggttaaacta acagaatgtg tcacactgga aagcattttg gagatcaggt caattagcac 31440
   tccctgtgtt actgtccaca caagtcaccc agaaagctta gtcaaatata gatttgaatt 31500
   cagtggttct ggggtgggcc ctaatgttct gcatttccat cgtgctcctt ggtgatgctc 31560
   ttggcttatg ggccacactt tgtgtcccaa gagtttagac tatactgttt tgttttgttt 31620
   tttcagatgt agaaactgag atccagtgag atgaagtgat tttcccaaag atacatagat 31680
   gaagttgaag cttagctctt taacccaggt ctgctggcgt ccaggttggt gtctctctaa 31740
   ttatagaatt gctggactta ggaaagcaga agtctcctgc cctctcttaa tgacccatag 31800
   catgttgagg gggagccaag gttagattct agggttgcac atacttgcac aaacatgcac 31860
   acacatacac acatgctcat gcacacacat gcacacacat actgtacgtg cttcttccta 31920
   ttctcctttt tcaattggga ggtataagaa acacatggat tacagccaca ttaaagatat 31980
   tcataaatgc aaatcaattc tgaatgtgta aacttttcca taagaatatt ctgggaaaat 32040
   acttgagtta tttctgacct tttatgtata aacacagaca ttctggagtt tgtcaaagac 32100
   catgttactg ataaaattgt gttagaatga cgtcttctga tagtttaaat gttaaaagct 32160
   ctgggctcgg ctcagggaat tctatacttt agcattcata aaggacccta gagtttgaga 32220
   gattatccat taacttctga tattgaattt ttgttgccaa ctatgactat ttgttttccc 32280
   tgaactctgt aggggaacaa cacagctgac aaaacccagg caaaagcgtg tgtgtgggtg 32340
   cgtgtgggtg catgtgtgtg gcgtgtgtgt tcacacaggt taactaaaca gtgcagattc 32400
   ctccaatttt tctccttttt gagaagtagg aaggagcaag agagaagaaa tcatctggag 32460
   tggaaaagag ggctggaaaa gtccttgaac taataatctg ccaattaatc cagctgcttg 32520
   gaaatagcct ggaattggaa ggtcttttgg gtgctctctg gaaagggtac caggaagtat 32580
   gagacaatgg ctgctaggaa cttgcactgt tcctgagtag ataagacctg ttgtcaagta 32640
   aattgcggtg gatccctggt aacacttgaa gagcaatgca agcctgggct ccacctgggc 32700
   tctacccctg cctgactaag ggagcttggg gaaagtattt acataatctc aatcagtttt 32760
   ctcacctgta aaatgggcct aaagaattta aatttccatg caagtcaaaa ccataatgag 32820
   atatcatctc acaccagtca aaatggctat tactaaaaag tcaaaaaata agagatgcca 32880
   acaaggttgc agtgaaaagg gaatgcttat attctgcttg tgggaatgta aattagttca 32940
   gccactatga aaagcagttt ggtgattgct caaagaactt aaaacacaac taccatttga 33000
   ctcagccatc ccattgttgg gtatattccc acaggaatat taatcattct accataagac 33060
   atgatgttca ttacatcgct attctcaata gcaaagacat ggaatcaacc taaatgttca 33120
   tcagtggtag actggataaa gaaaatgtgg tacatgtaca ccgcggaata ttatgcagcc 33180
   atgaaaaaga acaagatcat gtcttttgca gcaacatgga tacaactgga ggccattctt 33240
   ctgagaacta acacagaaac agaaaatcaa atactgcatg gtctcactta taagtgagaa 33300
   ttaaacactg agttcatacg gacacaaaga aaggaaaaac agacaccagg acctacttga 33360
   ggatggagtg tgggaggagg gtgaggattg aaactaccta tagggtacca tacttattac 33420
   ctaggttaca aaaaaatctg tagaccaagc cgcctgaata cacaatttgc ctatataaca 33480
   gacctgcaca agtacccctg aaactaaaat aaaattaaaa ataaataagt aaatgaatat 33540
   ataatttaaa tttccttgaa gaatgcctgg cacctgtaag tgctcacttc tgctatctcc 33600
   gttatcatca cagagagaag gcacaggttc agagtcggga aaatcacccc aggatgcttg 33660
   agtggggtct gcttgagcaa atggcggtga aaactggaaa gaaggggaag gagcaaagag 33720
   gaggtgggct gtgtcgtgct gagtggtgtc aattggctag ggtaaggagt ggcagtggca 33780
   gaattgaggc tgcagagcat tgaagcagag gtttgagcag tgcctcttag ggtcagcagg 33840
   cggcccatcc aggtgagcac aaagcgctct tggaaagggt agcgggaaca atgacaggaa 33900
   agtgtgtgag accagatgtc tgaagacctt gaatgctaga attggaggct tgactcttcc 33960
   tccctacgat gcagattttt gagtagcgtt gtgataggct ggactctgta tcatgttgag 34020
   aatgttcatc ggagtgcaga gcacaggagg agctatgaga gaaataaaag aggcaggcca 34080
   ggtgcggtgg ctcatgcctg taaccccagc actttgggag gccaaggggg tggatcatct 34140
   gagttcagga gtttgagacc agcctgacca tcatggtgaa accccatctc tactaaaatt 34200
   acaaaaatta gccagacatg gtggcgggca tctataatcc cagctactcg ggctgaggca 34260
   cgagaatcac ttgaacctgg gaagcagggg ttgctgtgag ccgagattgt gacattgcac 34320
   tccagcctgg gagacagagc aagactgtct cagaaaaaaa aaaaaaaaaa aaaaagagga 34380
   ggcttggcag caccagctct cctaggtgga tctggctgca tttgtggaag ccagaggctc 34440
   cagctttcat ctccctgagc tgaggggtga tcagtggcca tcccacctcc agggtgtggt 34500
   gaagggagac ttccagagtt ctcagggcac atggcaggag tggtggccac catgagaagt 34560
   gggccagcat ctgggacacg agcagacctg aataaagagt cctgcagcat tgcctgggct 34620
   gactctaggg agattgctct aaggattggg aggtaggcag agagggaaag cttaggccac 34680
   cacaggcctg agactgacca gtcaagactc tgatgatggg aacatgataa tgagctggat 34740
   tcagccagca ggcttgtcca ggttcaacac aaagtcatga gaggaaagca ccagcttcgg 34800
   gaaactttag aggttaccgt gcagtggcgg gtgttggtgt ttcttaaggg tctggcacgt 34860
   ttgctgagtg attctggctg tctgcctggc tctgagcttc ctgttcatag ttccccagat 34920
   gtgtcacggc cgagggatta aagccctaag aggctgtgac acagccatct ccaaaacccc 34980
   actttctcct tcctttgagc ctccgtacca gctggggcgt ccggcaagat gtgagttgtc 35040
   actctgctgc ggcacagacc tgaattaaca actctagcta gggctgactt caaaaagcac 35100
   tttcgttttt taataaccaa catcagctca gcaggcttca tttgggaaaa gaaaccttgt 35160
   cggattaccc cgacattctc cacctcctgg gaggccagcc attcccaaat gccccaagga 35220
   tgaagaacgg agacggtaag atgattaatt gcactgcagg ttttttgttt ctgtattttt 35280
   ttccctcctt tttttgggtt tacttacaca ttaatgaggt atttaggaaa gacaaatgga 35340
   atctgataaa aggtaacgtt atattgtttg tcgaaacaac attttaagga tttcttgaag 35400
   ttatatggct ctgtaggcat gtttcacttt aagatgtgtt tctgagtcat attaggagaa 35460
   cgactgtgat tatctatgga agattaacta tgaaacatat gggaaatata ctttataact 35520
   gtatattact tataaaccat gaatagttac atgacttttg gtatgtggtg agagatgtta 35580
   agtgaaaggt gttgtaaatg aagtcgtttt ataagactgt tgttactgta agaattgaga 35640
   cttattttat cacttcccat taagcttcta cagtgttaga gaggcataac ccaatgttga 35700
   ctgaaggtgc tatgttttct ttttaattta ttttttattt tgtttgagat ggggtctcac 35760
   tttgtcaccc agactggagt acaatggcat gatcttggct cactgcaacc tccgcctccc 35820
   aggttcaagc gattctcctg cctcagcctc ccgagtagct gggactatag gcgcctgcca 35880
   ccatgccctg ctaacttttt gtatttttag tagagatggg ttttcactgt gttagccagg 35940
   atggtctcga tctcctgacc tcgtgatcca cctgcctggg cctcccaaag tgctgggatt 36000
   acaggcatga gccaccacgc ctggcctgaa tgtgctatgt tattaatggc agcctctatt 36060
   ggtttcattt aaaggtataa tgcattgtat tggcaaaact tgtacacgct ttcttcaata 36120
   aggacctcaa aggtaatctt aaaagcctga tcatattttt ctcccccctt cagaagcttt 36180
   gccaatgttg cacctatagc tatagagaag accatgcact ctcagagggg attataaaca 36240
   ttgccttgca tttttatcct tgatctccat aatttatcct tgatctccat ttatccttga 36300
   tcctcataat tcctactgat agtgttgttg tgcataagaa tgagaaaatg ggactgaacc 36360
   tgaaagatta aggaaagaaa ataacaaaac aaaaaggaaa acctaatgaa gcttatcatt 36420
   caattcaatt tataactcct tattgctcat gcaaaatgtg aatttttcgc tgtgttggtg 36480
   agggtgtgag gagtggatgg tggggaagga tgcttcaaaa aatacagcta aggaccaact 36540
   ggtttggtga gtcatggcat tcacccatga gacagctgga gaacagtgac gtgtgtgtct 36600
   ctgtgtgtgt gtgtctgtgt gtgtgtgcat ttgatgccag agattggtgt ttactataaa 36660
   tgtgttgatc tgttaggaaa aggaagaaga caaagttgtc cagaaagagc caagaaaact 36720
   tcttgagatg aatttgatct gcctaatggt agtgatgagt ttaaaaagat tcttatggcc 36780
   tttcctaaca ctacatgcta ttgttaatca ttgaactgac aaaccccgtt gagcacaaga 36840
   ctgttagttt acttaggagg aaaacttggc aggaacggtc aaagaatagc gagggtatac 36900
   ttctaaaata cgtccttgga acatttacac aaacagcaca agctcattgt caagaataaa 36960
   attttaaaaa atacatagag ataatagtaa aagacgagaa gtaaaaacac accactgtga 37020
   ggtaaacact gttaacattt tggtaggtgt atttgtctct gagtacattt acattttgaa 37080
   gaaatatatt tgtactctac atgcttttcg gttatctgct tttacctcac agcgatggag 37140
   catcttgtta tgagaatgag ttttctttta caaaagcttt tccatttcat tgcatagata 37200
   gtttattaaa tcctgttggc aggtagttga ttgtttttct cttattaaca gtactacaac 37260
   tatgttcttc tcttattaac agtactacaa ctaacaccct tgtagctaaa cctttgcatc 37320
   tccttgatgc aaaggttatc tcaacgagtt ttccttgaga taaatcccca gaggtagaat 37380
   ggtaggggtc aaagggcatg ggcattttaa aaactctaga tccctgctgc taaattaatc 37440
   ctaggaaagt ttaattaatt tatgtgccca ccagcactgt atgaacattt tccaactctc 37500
   tcatcagcat tgggttttat aattttttga aaagtgtgct actctgcatg cacacgtatg 37560
   tttattgtgg cactattcac aatagcaaag acttggaacc aacccaaatg tccatcagtg 37620
   atagactgga ttaagaaaat gtggcacata tacaccatgg aatactatgc agccatgaaa 37680
   aaggatgagt tcatgtcctt tgtagggaca tggatgaagc tggaaaccat catcctgagc 37740
   aaactatcac aaggacagaa aaccaaacgc cgcatattct cactcgtagg tgggaattga 37800
   acaatgagaa cacttggaca cagggtgggg aacatcacac accggggcct gttgtggggt 37860
   gggaggaggg gggagggata acattaggag acatacctaa tgtaaatgac aagttaacag 37920
   gtgcagcaca ccaacatggc acatgtatac atatgtaaca aacctgcatg ttgtgcacat 37980
   gtaccctaga acttaaagta tcacaataaa taaaagaaaa gtgtgctact ctgataatag 38040
   gcaaaggtat attttgttgt cttaatttac cttaaggaaa ccctttgcaa aatctgctgg 38100
   tttccagttc tgcccctttg ggttcagcct ctcgtctttt ttggttactc attgccaaag 38160
   cagtcacctc ttacagtaac ttctgccaac ctaatttctg aggactttga cctcttttga 38220
   tgtctctcag aaagtctgct gagagagcga gagaactgga tgcttttgct ctgaattttt 38280
   accgattgag taacctgggg taaacctcag tctttgtgat tttgtctcct ttttagctat 38340
   aaaatttaga cagaaggcct gtgctagcca cctcacaaga ttgtactggg aatataaaga 38400
   ggcaagatag aggcaagctt tttgaataga ggaaaagcaa aaatgcaaaa tggtattatt 38460
   attattgttg ttactctaaa gtttagtgat aaatctaacc ttctaaaata tatagtttct 38520
   ggagagctgc atgctgaaat tttgtcgtct tctaattcta aacttgggaa gtgtgtgtgt 38580
   gtgtgtatgt atgtgtgtgt gtgtgggggg gggtgtgtat aacatatttc tatgcttctt 38640
   ggttcttaga ttcagtagca gtgtttagtg tgttacagca gtgcatggat acattttaga 38700
   ttaaaaagat ttccattttg ttgcttattt attccattct tgtacaggaa aaccagccat 38760
   cctcctccta ttgcattctc atcatccatg ggctttgctc agctccccag tggactgaga 38820
   gctcctcaaa gacggggtcc ggtttcatgt gtatacctct ctccagggcc tgctttgggg 38880
   tctgacaccc agagggcgga tcaatcaatg tttctcaaat gagtcaatgt aagggaggta 38940
   gaaaaatatt tacccagttc tgtggagaaa agaatattcc aaaaaggcac cccagaagat 39000
   taaaaaaaaa cctaaatgga gcttttctag gggcaactgg ttatttattt cgtttagttt 39060
   gggtgagtct cagtttctat tgagtaaata tttaaagctc tgaaaggata ttaaacaaat 39120
   gaatgcaagc caaggatagg accaggaaaa tggagaaccc tgtctttata ggtgcaagtg 39180
   tcatggattt ggcatgaata tcaaaaactt taaggaggca tcgattttca ctctgactcc 39240
   cagctcagtg ctgggctgca aaaatcagga ccaaactgac atttagtaat ggtttgaact 39300
   tggtactgaa taataataat aataataata ataataataa taataataat aaaagctaat 39360
   atgtaataca gcacttgcta ggtaggagac ctggttttag gtaccgctca tataaaataa 39420
   tcagtactat tattttattt tgcagattaa gaaacggaag cacagagagt gatttgctag 39480
   ggttatacca gtagttattg cagagctgga attatgacgc agacaggctc actctggagc 39540
   ctgtgggtca gtgagggtag gtcggactga actttggaga caatgtctca tggttttagg 39600
   ccactggtga tgtttgtgct gtttatttgg gtatatgact accatttatt gagtcaccta 39660
   ctaaaggaac atgcactctt ctgggttctt taagtataat atcttgaatc tttctcgcaa 39720
   ccttgaaaca ttattccaac tctaggtcca gacaagctga gaccctgaga agttcagtaa 39780
   gtttcccaaa gctgcaggct agtgaggaga gatccatcag cctgcaccaa gtctgtctgc 39840
   cttctgagtc catgtgccac actggcctgg acaactgagt gctgatgtat ttggttcttc 39900
   catccaaagg tgccaagagt ccagttctac tgcatttgga ggtgttgaac ctggaaaact 39960
   cagccacaac aacccctcaa cttggggatc aggcagggct cagcctcagc ttctctgaac 40020
   ccacatgggc caatgtgaaa tggatttgat ttttcagacc agatcctccc tggcaggacc 40080
   atccaattta tcacaccaac ctagactcga tgggcgttta aaatattgag agatatttcc 40140
   ttatatttcc tctctattga ccccccaaat ctgcaccaag gaactccagc gaatgagatg 40200
   gagtatggtg agtgctagct tgggggccaa gggcggcttc gtttctcctg ggcgacttat 40260
   ctcccttttc tttgtttagt gtttccagtt ataacatgag ggaaaagact ataatttaaa 40320
   atggaatttt actttttttg gttaaacttt tccatatgat tttgaaactc tttaaatttt 40380
   tttgagtgaa tgacacatgg tttatttgct tttggagtgg taacacaatt taaaaggtga 40440
   aagtcttttt ctcttccctt tctgcagacg cttctttccc tcttcctcac caataggcga 40500
   gtcttgttaa tagtttctag ttttttcttc cacctctttt gcactcatag tgtgcaccat 40560
   tctatacctt gatccatagc tgtatcaaag gcggtgcatt ttctgcctct taatggacag 40620
   ttggattgtt tgcaatgttt tgttatttca ggcaatgttg cgaccagtgg cagcctgccc 40680
   tcgtaatttt gcagaagtgt ttccaagttg ttggttaaat tcctggaagc agaattgtgg 40740
   ggtcagaggc tgtgtacact tgtgattttg atatatattt cagaattacc caccccccca 40800
   ccccaccagg tcataccaat taacacccct accaggaatg tttgaaagaa tgcctctcca 40860
   tgtgctcgtc aacagtgcct gctaacaagc tggtttttat tagacttcgg cagtctcaaa 40920
   ggagaaagct gcaaacttag taagttttct aaattttcct atctcgtatc atgaccatgc 40980
   agtgtcagcc ccactgtggt ctctcgtggc aaatacaaca gtctgacagg tactttgttg 41040
   actcaggttg ggcatctatt caattggctt aaaaccattt gcgatcacaa cgtttttcat 41100
   ttggtgattg tgttttgagg ccttgctgtg tgtccagtca tgggatggca cagggatgcg 41160
   tggaccctgc cagggttgca tgaaacagct aggcagccgc gggagttggc acatgtgtgc 41220
   tgagcactgt gaaagaaaca cagagacaga aggctgcaga gacagcgagt agggagccac 41280
   ggtccgtgtt agcctggtga ttgggacggc tcgaggtgga gtgatgaagt ctgaggcatg 41340
   agaaggagcc agccagacag agggcagagg aagcagagca taaaaaccca gcatgagaag 41400
   cagaaagcag ctctatgtat ggccaatgaa gagagtggaa gggaggccaa ggtgagctgg 41460
   agacagaggc agaggccaga gcatggtggg tgtttagggc tgtggcaagg agtttggctt 41520
   ttattgtgag tttcaggttg actgtagtca tgggctgggg aagatggtgc caaggtctgt 41580
   aaactcttac cttgtagatg taaattctca cctgaataac accagggagg catggttcta 41640
   tacacaagca cctgtcagac tgtgttgtat ttgccacaag agatcacagt ggggccgctg 41700
   ctgcacatag ggctggcgac cagaacactg tatctcttgg ccaaccttgt cctccgtgtg 41760
   agacaagtgg agacatttta caaaaatacg ttctacgtct ctctggagta caactcagtt 41820
   tacaaagggc tttctcattc attatctcat gcattaataa actgactgat aattttgaca 41880
   gattattatt gcatgtccaa cctgtgctaa gccctgtgct aggtgctggg gatgatgaga 41940
   ggaagaagga gtggaagggg aaatagaaag atgaagaaaa ttcagagcag cttacagtcc 42000
   atctgcgaga tagaatcaaa tgtcaacaat gagaaggaga gcaggaaggg gacagtgaag 42060
   aggacccggc cacctctagg ttctggacca gaacacgggt aactctgccc caggggcctg 42120
   cttggtgcca gggagtcctt cagggatggg gtctcaggag acagctgcaa gtagggcttc 42180
   tctgacatcc ttttctccag gtgtaagtag ggttatcttt ctaactttag aaagatagag 42240
   gtgaccaact gagagctttc ctcagggctt ctgagtgggc aaagggcaga actggaactt 42300
   ttcctcccac ccctgaaatc tgaggccatg gctctctgtg ctcagaatag aacaaaattc 42360
   ctgaagccca accacctggg gaaggcattg aggagagtgt ctctattatg catcgtcttt 42420
   catgctggcc accctggttc catgatgatg aggctgcttt gtttatgagt ctgtagtgtt 42480
   ttcagggctc tggagaaggg gtgggctctc taccagtctt agggtcttgg tggctctaag 42540
   cccagaggtg cttccattgt gtgaagaaac acaggagacc gagacaaaat attgttgatc 42600
   agacactcag gcctatttcg gcggcaaaac tgtcctaagt ggtcatttat ggccagcaca 42660
   gagtggggat taacgtgtgt gtgtgtgtgt gtgtgtgtgt gagtgtgagt gtgtgtgttg 42720
   tgggaggtgt ttgggggagg tctcaggaat tgctggccct gaggtgatac taatggatca 42780
   ggtttccatg gacacaggaa catctgggca ggaagtagga agactgcaaa gtcagctcac 42840
   ccccttcagt gagcttcacc tcccagtcca gggaaaggag ggcagggctt gcttctgaga 42900
   agggactcct ggtgaattcc aggtactcaa cacagaattg ccccaatcag gtgtaacatg 42960
   ggatgggggt tgtcaaggcc taatgccagg taaaagagga agcaaaaggt gtgtttacca 43020
   tagaaaagtc tagaaatgta gtcataaaac ttgaaatgtg tctgtgttaa gtgtaatgga 43080
   aataaatact ttaatagttt tttttaggcg ttgggttttc ctttgtctgt gtacatagat 43140
   tgaatgtctg tgtgtccccc aagcccacca ctaaattcct atgttgaaac cctaattcca 43200
   gtgatggtgt taggaggtgg catgcttggg agattgttag gtcatgaggg tggagccctg 43260
   ataaatggga ttagtgcccc tgtaagaaga ggccagagaa ctagctagct ctctttctac 43320
   catgtgagga tacaatgaga agtcagctgt ctgcaaacca gaaagtgggc cctcaccaga 43380
   caccggatct gtcagaaccg tgatcttaca cttccagcct ccagaactgt gagacacaaa 43440
   tatttgttgt ttaagccacc caatctatga taatttgtta cagcagccca ggctaagaca 43500
   ctgcgttcaa tcagcctgtg acaatggtat aactatcttt aaagactttc agtaaggatt 43560
   ttagtaagtt tcaggtctat ttcctgaact aatgaacaag tgaatgagcc attgctatag 43620
   aataggctca aagcacattt gtgtcttttg tggaaatttg ccttctttta tcctttggtg 43680
   agaaattatt agcggtgaat gtacttgtaa gttatccatc ttttctccga aagtgctgaa 43740
   cgtcttaacc atgagtctct tcttgtctga gggaggcggt gttgcttttg gtgaccagag 43800
   ctgctcagtc atgtatacca ataacatatt ccaacatggg tcacaaaacc aaagcattcc 43860
   aaagccatga ttgtgccccc tgaatattga tgtggcttcc ctcattctat tcctggagtt 43920
   agtgagaggg tagaacttga ctggaaatcc tggaacaatt ttttgggact tttcattact 43980
   tgcgatttta tttgctgtct ccttgatctt gcttgccccg ctctaggctg attagcagcc 44040
   agtctcggag gctgcaccaa tgacgctcgg cctccttgcc ctggagcctg ggtacccacc 44100
   tcatctgcta ttctagaacc ggccaggatc agcagccatc catctcctcc ccatgatgtc 44160
   tgggagcttt tcacattgcc tctgttcctt acacaggctt tatgattttt gatttgtctc 44220
   taaggaaact gaggcttaaa aatagcaggt gagtttcttc aagtcgcggc agatcgataa 44280
   cagagtcaga atttgaacat gggtctgttg gattctctca gctctgtaca catcattatg 44340
   ccaaaatagt aaggaacatg aggaagggaa acactagttt atggaagagc tctttctcac 44400
   atgtccttca ggcagtcata tttgggcact ggtttttgag tccattcata tcaagcatgc 44460
   cattcaggct ctcttctgcc acttctaaga gttatccaaa ttggtgcatg gtacccataa 44520
   actagttttt aaaagagata tgattactga aatgatcagt atgtaatatt gaagcccaaa 44580
   tcaatacaaa tgaaatgcat atcataataa agtacaaatt caagtaaagt agggacttgg 44640
   tagatgtttc tctggaaagg ttaaacacac tgcgtggaat gcatggggaa acctggtttc 44700
   acaaatagta tcagctgtct gctaaagaat ctgtaaaacc acatgcgaag gttcacaggg 44760
   gttctttcct gtagggaaaa tatgtgatta ttgtcaacag tgaaggaatg tgtactggaa 44820
   cttaattgat ttaaaaagtc tcatgtgaac ctgccatgac aacctctttg aataagtttc 44880
   cctcaaggtt taagcatttc tcgagattaa cgtagtgggt ttctgggctg actcacaggg 44940
   agaaggggag gttacatttt tgctattgtg ataatgtggg aaattacact cggtatcact 45000
   cactgtgctt gaatctttat catcaacctc aaactctggg atttatgtaa ccgcaagatc 45060
   agaactctaa gcttcttttt ttctccagtt gaaatttatt ttaagttggt aaggaaatat 45120
   gacatatatc tatagaacct aggctcatag gaaggtaaaa aatgctttgg attggaagta 45180
   gaggcagacc tttactttag tctggaactg atgtcctgat tgtaaattgt tgttctaggg 45240
   aaacccagtg tgtgtgaatg taagcccctg gagttgagtt ccctttcttg gctgggcccc 45300
   agtctgccac gtcagtgctg aaaatggaca ttgctgctgc ctcggtgaca tacaaatagg 45360
   ggaaatgtct cctgaatgga attactcttt gtcatctagg ccagtagttg tcaaccctgg 45420
   ctgcatattg gaatcacctg ggagcctttt aaacactgat gcctggatcc taactccaga 45480
   agttctgatg tgatgggaat gcatgcagcc tggcttttta aagtcctcag gtgatcatac 45540
   tgtgcagtta gtgtaatgtg ggttgagaac cactgacccg agaagtagac agttgccgag 45600
   tcagtaaatt acccaagtaa acaagaagct ggttagacca agcctgtcca tagtgattgc 45660
   caaatcctgc ctggtgggga aatttaggac catttcctca gaaaactttg ttactggaag 45720
   ttatcaaggg ctctctacct agtaagtgtg ctttcagttt tttaaggcac tgtggatata 45780
   agttgtgtaa acgtagccac tttcatttat ttggcaaata tttaaacatc tgctctgttc 45840
   aagttatact caatctcagc ctacttttaa gcttattaac tgaacagcag gggagagaac 45900
   tcaagcaatg caaagtaggg gctgcaaagt gccatcagag agggggagat gaaaaacctc 45960
   aaggtttcag agcagggaga gatagttgcc tgtttctcct ggtcggaggg atgggaaaac 46020
   cctaaagaag tggtgcagta tttgggatag gccttggagg ggggagaagg cggaagaaga 46080
   agacttgagc tggaggagaa agaagggttg tattctgggt agaggaaact gcacgagcaa 46140
   gaaaagaggt agacattagg gaaacttgca aggaaatggg cacatagtaa acatgggtac 46200
   atcaagggcg aggagaggaa cacattccaa aaggtaggat caagggagac tgaggagcat 46260
   ctggaatgct atactgagtg gtctgggctt tgtttgggag gcagtggagc cttgcatggc 46320
   aggttttgac atccaacctt gcagcaaacg gcaaatagga gcttgctcag cagtatttat 46380
   atggcgaatc tggattattt agaaggaaat tgtgtttctg tctcactgtg tggcccatgt 46440
   aggttgcata gttgcagtat cttcaacttt gcattgacaa ggtgggtgcg ttgcactggg 46500
   tcgagatggt tcagatgtcc cccaatgcat tgctgctttc gacatctctc catttctcta 46560
   gcttctaaca ctttgtttgt ctctcttcaa ccaccatgct gagaaaaaag tttaagggct 46620
   tttcttgttc ccccacgtaa tcttggcaag taataacagt gaaccctgga gatggaaaga 46680
   agtgtggatg ttaggagact ggattgctag catggcatta acaatttttg gctatgttga 46740
   ctctcaagta aatatggcaa ctgcttttgc ttttcctttt caaaatacaa agataaaatt 46800
   taaggggaag tgagattttg gcataaatgg ctgactcttc tgcaaatagc atttctggat 46860
   taattttatt acatccaatt gctcttccat gtattccgat aattttattt gtaataaagt 46920
   tcataagtgc tgttggtgat tagccaaaaa aaagagtaaa gggaggtcaa cttttgtgta 46980
   gttgagatca ctttttctaa gtctgatttt aaattgggtg gtttgtactg tgttagcaat 47040
   gttagaggat acaaacaacc ataacaacaa aacagcaaca gcagcaggaa ggtcatccac 47100
   ccgtgctgaa aacctccttc ctcttgggtt cacttttcct tctagtgggg gaaatggtga 47160
   aatcacgtta gcactttcta ctcttggtcc tggtgtcttc tttatgacct cgtaacattt 47220
   tcctagataa aatttgcatg tctctaactt ctgagttcca cttcttactg ttctcatgtt 47280
   cattcattat tccattgtga cgtgtttggc tgagtttgaa agactctaat gataagtgga 47340
   gaaggaaaaa atgactttta taataaaagg taacacttaa tgacatgaat ctgagacaca 47400
   tgactaagta agaaaataaa atattgacgt cttggtttta aatattgggc aacaccatct 47460
   catgtttatc ctttttctca tcaaaccgca tgtccaattt tggattagga agacattctt 47520
   actgtactta tggtacgcct gcaatcatgg tgcctcctct gtctattttt gggttgacca 47580
   aataacaacg caaagcactg agtgccaacc accacaacaa ggacttggtc tgtatttgtc 47640
   catatgcttg cacttccccc aaggtggaca ctgtcatccc catgaatgga tgagggaaga 47700
   gaagctgaca gaacacagct ggaaagagca gggtgagagg tcactgtggg cctctcccat 47760
   cagtgatttg gtgctatttg tggcctctgc aggcatggac atatcctgtt gacattacaa 47820
   aataaaatgg atgcaaatat tattgtttag tgtcttccaa tttcttggat ccttaaatag 47880
   ctgccaacat cgctcttcag ttatttatgt acacaaagag ctatttctga atatccgtcc 47940
   taattttctt ctggcctgtt ccctgcatat taatgtattc caatgaatcc cacccattcc 48000
   aaagagaact gaagcaagct tgtggcttgt ttagatgtgg tccaagtctg tgtggtcccc 48060
   tcagggaggt gtagtaagga tttctcagaa ccggtgtttg cgtgtgaggg aagcctgctg 48120
   agcagagagc gtttacattg cccagtgttc cgagtgcttg agtttcaata gttaccttag 48180
   tttattagga ccttggaaga gtaccaaagc tgtgagttcc tgggagtttt ccgtcaaaat 48240
   cagtcctctg gcatggtaaa gaatatgttt gtaaaggaac aagaaaaaaa tatataattt 48300
   ttctaaatat atatatagga aatgtatata tatatacaca tataatatat atatagacaa 48360
   aataaaaata tatatttgat ctctggccct gattcccagc acagagctcc taacttcttg 48420
   gaatttcctg ggtgatggga gtcttttgtt ctaatgaagc tcctgggagg agaatggtca 48480
   ctagaaagaa cagattagaa gcttgggaat ttcagtccta cccccatcct ctgggaagac 48540
   ctggaggctg gagattgagt taatgatcag tgatgttcaa gtaacaaagt cttgtaagaa 48600
   tcccagaact ctggggtttg gggagctcca gggctgctga acatgtggag ggtagcccac 48660
   ccggagaggg caaggaagcg cacttcccct cccatacacc tcgccctgca tatctcttca 48720
   tctggctgtc ttctgtatcc tttattgtat tctttattac atagtaagct gggaaatgtg 48780
   tttccctgag gtctgtgagc catcccagca aattatgaaa tccaaggatg gggtcgtagg 48840
   aaccctggtt tacagctggt cagtcagaca tataggtgac aacttggcac ttgtatttgg 48900
   tatctgaaat ggggggcagt cttgtgggac tgaaccctta acctgaggtc tgtgctaact 48960
   ccagttagta tcagaattga attgaattgt gggacactcg gctggtgtca ttcagctggt 49020
   gtcagagtat tccttgacat cgcttcaatc cctggtcaca gaagtgtgtg gagtgcaggt 49080
   atagaaaagg aaaagtaggg ccgggtgtgg tggctcacgc ctgtaatccc agcactttgg 49140
   gaggccaagg tgggaggatc atgaggtcag gagatcaaga ccatcccggc taacatggtg 49200
   aaaccctgtc tctactaaaa ataggaaaaa attagccagg cgtggtggca ggcacctgta 49260
   gtcccagcta ctcgggaggc tgaggcagga gaatggtatg aacctgggaa gcagagcttg 49320
   cggtgagccg agatcgctcc actgctctcc agcttgggca acacagtgag actgtgtctc 49380
   aaaaaaaaaa aaaaaaaaaa aaagacaagg aaaaatagtt gttttttatc tatctcacgt 49440
   ttattttaga ttactcaaaa taagcatatc tgagcttata tctgctgtgc agagcaacga 49500
   aaacatttat gggagggtaa tggcggtaaa cactgtaact tcatgctgac gttaggtggt 49560
   aacttctctt cgataattca agctacctag tgataatgta agttctttat tgcacttcaa 49620
   aagtatttct ctgtccatgg agggcatttc ttctcaaaac actttgtact tccttatgaa 49680
   gttggaatat ttgctatgga aaccaaattg ttcttccttt ggcctgctga aaattagaaa 49740
   ataaattttg tgtggtcagt aggttattgt tttaaactta actacatgag attaacttct 49800
   ggaactgaga tgccaatgat gcaatttcag aatgcagttg ttgcactcat taaacaccac 49860
   agtttgggaa ggatccctga tccacttggt gctcagtggc tttctcagcc taacagggac 49920
   atagtttgaa gagtcttaaa caaaagttac ataagaaccg cagatgacta gctgttggtt 49980
   ccataggtag attttctttt attatcttag ttttcagctt tattgaggta caactgacaa 50040
   acaataattg tgtatattta aggtatacaa catgaggatt tgttgtatgt acacattatg 50100
   gaatgatcac cacaatcaag ctaacacatt catcacctta catagttacc tgttttgtgt 50160
   atgcatggtg agaacagcta aaatcaactc tcttagcaaa cttcaagtgt acgatacagt 50220
   attactcact atagtcacca cgttgtccat taggcccttc agaacttatt catttcatga 50280
   tgaaaaatgt atatattttg ataccatttc ccccttccct ccttggcagc caccactcta 50340
   ctctctgtaa atcattcttc ttgtcattag tcccctgtca gaactctgtc gtggcccctt 50400
   ccaaacctcc ccctgagcta tcaaagcttt catttactgc ctcggtataa ttaattttca 50460
   gtgcttattt ggccagatcg gacctacagc cctcagtggt ctttaaagtg aaaaaagtga 50520
   aaagtcttta gcatgaaacc tttgatgtgc atgttgtgtg agcgtatgtg tttgagtgtg 50580
   tgggtgttca tgtgtagttt catgaacgtg tgtgtgggtt ttcatgtgtg ttgagtcagt 50640
   gtgtatcata tgttgtatat gggtgttata tgtatggtgt gaatgtacgt gtttgttgtg 50700
   tgtgttttgt gtgtgttgca tgtgctctgt ggctgtgtgt tgcatgaatg tgtgtcgtgt 50760
   gtgcattgtg tgcacgtgcc aagtacctgg catgtatttt actaggtgct tagtaaacat 50820
   ttgtcacatg caggaaaggc acaattcctg cctccatgaa gctcactgtg taaactcaca 50880
   ggtgaattgt caattcctta ggcacagggt ctgcgttgtg ttcatttttc tgtccttcag 50940
   tgtcacacag aggcaggcaa ctccggcata aaatgtgggg ctcagatggg aaacaggtat 51000
   tgtataatga aacacaaatt tgctctcaat tcaggtttcc tgaagcagaa gcttgttttc 51060
   ttcaattcac ctttgcacac actacacaat cccattcact attatgtttg tcatgtatta 51120
   tccctccacg ctctgcaggt gggtagggat ttgttggatt tgttcctaga tacacctcaa 51180
   gtacctggaa cactgcctag tacatggtgt gcgcacaata aaaaattggt ttaatgatca 51240
   aatgtaagag taaatgagtt ccggcacatg tggtccacat ccgggttttt agcagaacat 51300
   aggtattatg atgtctgtca cagttcactc taaatctcag ctggtcttgg caagcagctg 51360
   cttggggact cttcctcttg ctcctgtcag gccctagtgt taatctgcat cctgcagggt 51420
   ttccctctta ggctgtcatc atggagctgg cccccgggag atggctcatc ctgcttggtc 51480
   aatggccacg ctgtgccctt gggtccttgc tgcagcctgt gcactgtatg ccattgaccc 51540
   aggggtctgg gacatccagc cacccggtgc tgaccaccat ggactttgct gttgcctgca 51600
   gatgtccgcc ccccaacccc ctggtttctc tccaggcatc ttctgttcct ctggtttctc 51660
   tcgcaacttc tatccccctc ctcggtgcct aggaattctt gtcagcttcc tccgctttgc 51720
   cagaggcagc ccttgcagct gtggagccac gtgtcactga gcactgcctt ctggaaagag 51780
   actgccattt ccaggctgct cctccaccag cccggtgact cagtttctac tccttcttct 51840
   ggagttgacc atcagagctg agctcttgcc ttccttcttt ctccttctgc agatcttccc 51900
   tctagctcta caggaagggc ctccctggct gtgtggtcag aaggtctcca ctgtccaagt 51960
   ccagcctggc ttactctagg gtttatgggt ccagtcttcc tccatgactc ttgagatgtc 52020
   aagcccagtt tggggagttc aaaaatacca ttttctctct ctcaacaaag cctagcacat 52080
   tttttaaaac cacattttaa catttctgaa attaggatgc atcttgcaat ggatagtagg 52140
   tcctagttat ttggcagcat tttttttctt attgtacata ttacagctaa cgatattttt 52200
   gcttaggctg ttgtcttgct atggatttaa actctatact ggagtttaaa gccatgcaac 52260
   acttttttgt tctcagcctt catcttcctc acctcaaggt aatagatgcc tctcctttaa 52320
   aaatgtgaag ctgccaggca accagaatca agaaaagggc agccctggaa gatatacgtt 52380
   tgtttcatac attcaaaaat aggattccca gtacctaaga tcaccctttg aatcaataca 52440
   catggggctg gaccacctta aggccatgga ctggtttcca tgctatactt ttaaataagg 52500
   tcaaagaaaa aatgtgtcca tctcacaggg gcctggcctt cctggaagaa gaaaacatac 52560
   tggtcagtaa ataagaatag ctgggccctt tggcagttat agatgtaatt ttccacgaat 52620
   aaggcagaat tgcattccac gcctctgtct catgagaaca taatgagtgt gagacatgga 52680
   gagactgcat ttctgagtca ggcctgctga gatacaggcc taaaactttt atactgaagt 52740
   cagagttttc tgtgtcagtt ctctctctct ccctgtctcc tatgcacaca tgcgcacaga 52800
   ataatgagtg agtggacatg aaatagagaa gtgctgtacc tggcaatagc aactgaatct 52860
   aaaaaaaaca gtttactaac aggcgtgagt gaggggcgct cagttatgag ttggtgattt 52920
   taagtatcag gatcaggtat gggccaggtt tgttcaagcg aaaaattgta gttaatattc 52980
   agaagggcct cgtgaaaccc aagagcaagg acatagccat gtttctggaa gaactgggac 53040
   cagccaggga ggttgtctgc cagggtctga aggaatcatt cttcatttct tgtctccatt 53100
   ccttgctttt ctctctctct ctttgaaaac tggctttcct tgttttcaga aaacacagtc 53160
   actctgcatc tcccaagtga tgttcagagc taaacacttt gttttgactc catctataac 53220
   acatatgcat taactccatg tatttcaatg taaaattctt ggggagagca tgtgaatgct 53280
   gtagctttgg gtctagcaaa tattcttggc cccaccagtc atggccaagg tgtcttttag 53340
   gctcatgcct gtagactgaa agcagggatc agggaaattg gtttacttta gtaagaaata 53400
   taggcatgct gagcaaatat caactgagta aacatcaact gaaatacata gaaatcatga 53460
   aagatggaat gcagtatact cactctattt ccttaaggta cttctgcatt ccgatgcaat 53520
   gtttgttgat tcaaaggata atgtaataaa atgccaaagt tttatcaatc agagacattt 53580
   gactgcacat gtaataattc catgctatct ttattatctg tgaaattgtg tttgtgtctt 53640
   atgggacaag tgtcatgaaa ctcattttga gaagcaagct gagattttgt aataaataca 53700
   tttagctttt ttttggaatg aggcataccc agaggtccat gtttaaaaca aattagagta 53760
   aatcaaagta gcatttacca taaatattaa cggtaaatga aaaggctagg ggaaatcttt 53820
   ttaaattgta tcaaaataat actttgttta tacataggac agccacagtg gctcacactt 53880
   gtaatcccag cactttggga ggccaaggca gacagatcac ctgaggtcag gagttccaga 53940
   ccagcctggc caacatggcg aaaccccacc tctactaaaa atacaaaaat gttagctggt 54000
   catggtggcg tgcacctata atcccagcta cttgggaggc tgaggcagga gaatctcttg 54060
   agccctagag gtggagtttg cagttagccg agatcacgcc attgcactcc agcctgggtg 54120
   acagagtgag actgtgtctt aataaaataa aataaaataa aataaaataa aataaaataa 54180
   tgtaacatga attcaatgta aaaaaattta gaaatatgcc tacagtgcct catatgggtc 54240
   tttcttggtt gataggaatg agacttggca gagattagga aggagatggt gaacaagagg 54300
   gagaaggaga gaggtgaaag ggatggaaag gggaagaggg aaagagaaag gggtttatct 54360
   tgtgagatca cctaagtaaa gtactcacac actacctcct ctattcttga ggcagggaaa 54420
   ggttagacaa gggtgctgtg ggagctggca gggtggacac ctgtattagt ctgtttctgc 54480
   actgctgtaa agaaataccc gagactgggc acagtggctc atacctgtgg tctcagcatt 54540
   ttgggaggct gaggtgggca gattgcccaa ggtcaggagt tcaagaccag cctgggcaac 54600
   atggtgaaac tccatctcta ctaaaaatag aaaaattagc caagcatggt ggtgcgcctg 54660
   taatcccagc tactcgggag gctgaggcag gagaatcgct tgaacccagg aggtggagga 54720
   tgcagtgagc tgagattgcg ccactgcact ccagcctggg tgacagagca agactccatc 54780
   tcccccccac cactgccccc ccaaaaaaga aatacttgag actgggtaat ttataaggaa 54840
   aagaggtata attggctcac aattccacat ggctggggaa gcctcgggaa acttataatc 54900
   attgtggaag gggaagcaag tgcatctgac atggtggcag gcgggagaag tgaagtgaga 54960
   gcacaggaaa aactgccact tttaaaacca tcagatctgg ccggatgtgg tggctcatgc 55020
   ctgtaatccc agcactttga gaggccgagg caggtgaatc acttaaggtc aggagtttgg 55080
   gaccagcctg gccaacatgg taaaaccctg tctctattaa atacaaaaat tagctgggtg 55140
   tggtggctgg cacttgtaat cccagctact cgggaagctg aggcaggaga atcacttgaa 55200
   cctgggaggc gggggttgca gtgagctgag attgtgccat tgcactctag cctgggtaac 55260
   agagcaagac tctgtctcaa aaaataaaaa aaaataagac ctcctgagac tcacccacta 55320
   tcacgagaac agcatggggg aaccaccgcc ataatccaat cacctcccat caggttcctc 55380
   ccttgacaca tggagattac aatccaagat gagatttggg tggggacaca gagccaaacc 55440
   atatcctcac ccttgcggct tgccaggctg atggcaggga acctctcatg gtggctcttc 55500
   ctcggctacc acacaaccgt ctagagtcca ttgattgttt agcacatgct gcctttactc 55560
   tgtagagtgt cttgctagga aaaccacagg agagaacaca tgcttgcaac tcccttcatc 55620
   atctttgcat caagaataat tcttgtctca acaacaaaca aacaaacgaa accaataacg 55680
   aaaattcttc atcatatttg catcaagagt aattctagct tggatttaga ctggcacaaa 55740
   caatgtctcc tgttcttctt ggtcgtagct cttgataaac ttattattgt ttcatggact 55800
   gggagcttcc tggggaagtg aaggggagag tttctggcat gggttgagga cagtcatgaa 55860
   ggtatgttca gaatgaagtc acatgtagca gtcaggatag ataggcaaga tacaaggaag 55920
   aagtggggta tctaaaaggg agaaagaaac acagaaagtt gggccttgtt aggcccttcc 55980
   agccccactg tggcactcac tgctggggta tcagctgaca tggttaggtg aagtcctcct 56040
   ggtggtcttg ggtgtaatat ttcaaagcag tgctcacatt aataaaaaca aggtctaatt 56100
   agttgtctgt gcttctaaag gaagactgag gaatgcgaag agggtctgta atacacagtt 56160
   tcccaggggc ccatccatga acaaaactct tttgtgatgc ctgggatgca ataagacacg 56220
   gaatttgaaa tggttcttcc cagaagactt attgctgagt aggtcacaaa ccattgacga 56280
   ggagtggaat acagtcaaga gtgaatgagt agacaatcag gaaaaatcaa agaagaaaat 56340
   agtgtcttca gctccaacca ttaccttttt tgagggactg attagaaggt ttagatcagt 56400
   ggttcttaac ttcaacagcc ttataatgct ctttttctta ataactattt cataatatcc 56460
   cctaagtgaa attcattgac aacataacct acttaagcat gatattaaaa atttaaatgt 56520
   attaccttag ctataatgta aagaagatat aaaaggtagt ccatattcag tttcatacat 56580
   acatgtgtag acacgactga gctagaagac agaaaggagt tgtcagatgc ttgagctata 56640
   cacagagaag tctttaatgc agcagctgca aatgcaggct gaaacagctt gtgcctcgct 56700
   ggagagccaa ataacatgag tggcattgct gttggggatg tggttttctt cctcccatgg 56760
   tgaaagatct tttataaact tacagaagaa aatcaagttt aacttgtcct caattgacat 56820
   ggtagctgct tttctggaaa atttatacgt attaaaactg tgcgaaaaat actttgtgtt 56880
   tatatgacaa acagagctag gcttcagact cagattttta tctccattga ggtctggtga 56940
   gatgtttgaa aggttttttt ttttttttaa ggatagagga caatttgttg tgctgtgctg 57000
   ttctaggtgt tgcaaaatgt ctagtagccc caacttctgc ctggtagatg cacacccaac 57060
   caaaaatact cccgcttgta tttaaaactc acttaggtta tagctaattc ccaggcactg 57120
   tctgtagaga ttctgctcag gtagggctct agaatgtcca agaggctgca cagctaggca 57180
   tgggaaccac ggattcaagc catccctctc tctgactcac cagatccctc tgacatatct 57240
   tgggccagtg gctgtccagc ctgggtttta aggagctggg tttcagtgaa gaatgtcagt 57300
   aggttcagtt ctcttcccag cccttttaat gcagaggccc ccttgtcagc ctagaaggct 57360
   gttctaagct cctgtgcaaa cccctcttgt gaacccttgg gtgtggccat cctggagatg 57420
   gttgactggt gatccagatt tcagcctctc ccttctctgc tttgcccagt gtcatgttca 57480
   ggcccccggc tttgtcaaaa gagagaaaca cttttccctg ggtctcaatc ctttgaagtt 57540
   ccattgggtg agtcagctcc ccacttagct tcagaaggat ccaggacagg ttgtcctaaa 57600
   attaatacct gcacattcat ggacagtgca tggtgagagg aggcaaagat ggcagagaca 57660
   actataactg gttgtggaaa agagttgagg gagagaagag aaaatattat taaatggaag 57720
   agattgaaaa atactgaaat caagtgcaag aacctttaat gggcagagtt aaatgacaca 57780
   tttattcatc caataaatat ataaaatgca tctaccacct actaggcttc attcaagaca 57840
   atgataatac ggcagtgtat aaaacagacc aaaacctacc ctcatgaagc ttacattcta 57900
   gagggggtag acagttaatt aacaagaaca agaagtaata tatatagtaa gtcagatggt 57960
   gacacatgct aggaaacccc agtagggact tcaggtaaga cagagatgtg tgtgtgtgac 58020
   tgtgtgtgtg tgtgtgtgtg tgtgtgtgtg tgtgtgtgta tccatgcaac atccaatgat 58080
   ctgggatacc tatttggcaa caagagagga aaatctcttt ggcaatagtg atttggatgt 58140
   tagtagtgta ggaggaatga gaaagccata ctgcaattat caggacagga aatgattcaa 58200
   gtttggatag aactttttaa caatgaaaaa tgaagagaaa caaaaccact ttcacatggc 58260
   aataattaaa tttctgagtt tacgtaaaga atcttatgaa acttctcatt ccaacctttt 58320
   gtttccttta agttctttat tatctttgag tgtttagatt tcaattctcg acagttcttc 58380
   acatattgaa tgagtttgct ctcctatcac acagagatca agttatctat gcattttaca 58440
   cttggttact ttagacttga gtatttgtgt cgaaataccc tcctttttaa atgtggtttt 58500
   ggttgatgct tttggaatga ggaatttatc agctgtggtg actcgtatat actctgttag 58560
   ccagaaatac tagcttactc tttacccgaa aactccctat tccaaaatga catcacacta 58620
   tgagtttatg cctaatgaag ctccagtaac atttgcttat ttgcatagta gaaatactgt 58680
   caggtacacg aattttcagt aattttctca atacctctat ggtattactg aataggtata 58740
   tcatgctcat tttatacgtt caaagaggct gagtatataa atacccaaat ataatgaatg 58800
   tttggcaggt aatgatcatc tctatcattg actatgttta aatacttttt atgtattaac 58860
   ttattctctc aacaacccaa taagctgggg actattgata acattatttt gccatcaagg 58920
   gaattgaggc ataagagaag ttaagtaact tgcttagtgc cacacagata gtatattgta 58980
   gagctgggat acatttgggg cagttgggct ccattgccta tgctagtaac catcattctg 59040
   cacttcctct agttctaaag agcaaatctc aaactcctac atgttttagg gtgattttta 59100
   actttacaca caatggcagt ggtcagtggt agaacagtaa aaggcagcaa tgtgaagggg 59160
   gaaaacgatc agttttggag agtgtgttgg atcccggttc agttatttta ctcattgggt 59220
   aatcttgggc aagttcctta acttctctag gatgtctgtt gctgtctaca aaacaagagt 59280
   agtagccagt gtggtggctc actcctgtaa tcccatagtt ttgggaggct gatacaggaa 59340
   gatcacttga ggctgagagt ttgtgcccag cttgggccac atagcaagac cctgtctcta 59400
   aagaataaaa aagtaaacaa taaataagct gggtgtggtg gtccaggcct gtagtcccag 59460
   ctacttggga ggctgaggtg agaggaatac ttgagcccag gagattgagg ctgcagtgag 59520
   ccaagatggt gctactgcac tccagcctgg gtgacagagt gagaccttgt ctcaacaaca 59580
   aacaaacaaa agaaatcaac aatgaaaccc caaaaaccta caacaacaac aacaacaaca 59640
   acaacaacaa caacaacaaa accaaaacaa gaactccttc ccaaagaaaa gtagtaaacc 59700
   attcatggtt ataagcatta aatgtgatgt cctattaaag tgtctaaatc atggtcagga 59760
   acccgatagg tgcttgaaat ctagaaattt cattcctaca taagggccaa ttattgagca 59820
   taggtcagat gaccagttaa gaaagctgcg gtgcagtaaa ttggaaaggg aattggtgat 59880
   gttggggaat tcagggcagg atttgtaccc cctaaaggag cagttcaaat ctcaatgatg 59940
   acttaggatg taggtcctta tggtgaacct gacaggggct gctgtgctgt gaagtattgc 60000
   tttgaaatcg gggtcagggg tgaagcagct gttgacgagg gtgcccctct cttctgacct 60060
   cttggcctgc cctttcttct tgctcctatg tagacacttt catgaactac acagtggtct 60120
   cagagggctg cagaagcttt atgtagagca gacgttcttg aactttcggt ctaaagacac 60180
   cttcgtactc ttaaaaactc tcagaacttt aaagagcttc tgtctacctg gttaattcta 60240
   ttgatattaa gcacattaga catgaaaact gagaaattaa aaaaataaat ttttaacaat 60300
   tatttaaaaa tgccgataaa ctcattgcct gttaacataa atctctttta taaaaacaat 60360
   tatttttaaa aaacgaaagt atcaaaaaga attacctttt tttactttgt ttacattgtt 60420
   ctggaaatct ctttaatgca ttgctcaaca gaatacagat aagttctcat atctgcttct 60480
   gcattcaatg ttggagaagt tctgcatcat gcggtctctg gaaaagtcca ctgtacgtgt 60540
   gtatgaaaaa gacacatacc atgtttgaat tattatgaaa gtagttttga ccttgaagac 60600
   tctgaaagga tctcaggaac ctttgagaac tgataatata acgtatcttt ttcttggtaa 60660
   taatgttttt gaacattctc agtattccct ctcagcttcc tcctggtgcc tgtgttcaag 60720
   caatcacaat tgctaatgat tcttcttgca aaattcttag gtttggcaac cctggaaata 60780
   gtagatttga tctttagctg tctccctcac tcttatcttt cctctttcct tgacagctgt 60840
   tctttccttt tcactcttgg tggcacaagc ttgggaggtt ttccatccag gctggtgcct 60900
   cttaacttct ctctctgcct ctagggcctg acagctgcca ggctgtcctt tatctgacat 60960
   tgttcacagt ttacttcctt cccccaaagt cttggttttg actaaaatgt attaattctt 61020
   ggttatgttt tataaagata tcaaaaactt tttcaaccaa agtttaaaaa agaatttgca 61080
   gggtgggggt gggcatgtgt aaagaatctc aggcatcttc actgatgaac ttcagaaact 61140
   gtgtcagtag atttctgatg ccagccagga ttgagtaata ggccctagat ttactttctc 61200
   ataagaaaca actaaattac tctacaaaat atgcaaaacc agggttttta agaattggac 61260
   atcagctaag gaaggatagt gatccctgag aggcagaaaa caaagccctg tgacttctcc 61320
   tgcttactga tgtgagagtt tccaggccat ggcacaggga agcgtgaccc aggcagagtc 61380
   agtgagaaga ctacccaaag tcagaaaaag cgcctcccta aaagattaga tgaaatagtg 61440
   gacaggagga ggaataattc ctattctcac cagccagact ggaaagtctc gtaattcata 61500
   gggatttgtt agaggactca gaagcatcaa gcttcagaag tgggataaaa tgagtcctag 61560
   agtgaatggt gctgtggtcc tgtctgccga gtcttaaaag caagatccaa aagaatcaaa 61620
   ctattctcaa taactcaaat gcatcctaag acacaaaaat attcagcatc taacaagtta 61680
   aaatccacaa tgtttggcat tcaaccaaaa aattaccagg tatgcaaata aggatgataa 61740
   taatgcagag gaaaatgagt caaaaccaac caagaactaa tagagatgtt agaattagca 61800
   gacaaggaca tggaaacagt ttttataact gtattccata aacacagagg taagtagaga 61860
   aacaaaagac gtgaaaatgc ctgcgtctaa cttctagaga taacaactac aatttctgag 61920
   gtaaaaaaaa tgactggatg agattaatag cagattagat attgcagaag aatagatcag 61980
   tgaatcgaaa gacataacaa tagaaactat aaaaaaaaac cacagaggag gaaaaacaaa 62040
   caagtaaaga acagagcatc acgaaacggt gggaaaattt caagtcacca aatacacatg 62100
   taattggagt cactcaaggg aaaaagggag tgacaggaaa aatatttgaa gaaataatgg 62160
   ccaggagttc tccaagctga actgaaaatt acaaacccac atacccaaga agttcagtga 62220
   accttaagaa acttgaggac aaacacatca tttcatcata attaaatttc ttaaaatcag 62280
   cgataaagaa aaaatcttag aagcctgagg gaaaaaatac gcctaccaag aaacagcagt 62340
   gacacagatg gtagcagatt cctcatagaa acaatgcaag ctagaagagt ttggagcatg 62400
   atctctaaca tactaaagga aaaaattgtc aaccaagaat tttatacaca gtgaaaatat 62460
   ctttcaaaaa caaagacaca ataaagccat ttcagatata taaaagctga attaatcact 62520
   aacagacctg cactacaaaa atgttagagg aaatccttca gacagaagga aaaggatact 62580
   agatggagat ctggatctat ccaaaggata gagcaccaga aatgccaaat tattatttaa 62640
   atgactttaa atgaaaatta tttaaacaga agtaataatg atgtagtgtg aggtttatag 62700
   gatatgtaaa agtaaaatga atgacaaaat agcacaaaag tcacgcaagg gaaaatgaaa 62760
   ctatactatt ttagggttcc tgtatgtagg gtgatacagt atcatttata ggaacagttt 62820
   tagcactaaa tacctgtttt aaaaagaaga aaattgtcaa attggtgacc atagcctcca 62880
   ccttaagtaa ctagaaaaag gaataaatta cacaccaaac aagcaaaaga taagacataa 62940
   taaagatcaa agcagaaagc aactaaatag aaaacagaaa tacaatcaag gacatcaatg 63000
   acaccaaagc tttgatgtca tttgatgaca cctttgagaa gatcaataaa attgataaac 63060
   ctttagctgg attgactgtg gattaaaaag agaagacaga acaatcaatg gtaggaacta 63120
   ggtaatatca ctatactaca ccacaaatag taaaagaata ataagggaat actaggaaca 63180
   aaattaggca taaaacatat acttttagat gaaatagata aattctttga aggacccaag 63240
   ctaccagctt cattcaaaaa gaaatagatg acttgactag ccctctatct actaaagaaa 63300
   ttgaatttgt agttaaaaat cttcccacaa ggcgcagtgg ctcacacctg taatcctagc 63360
   actttgggag gctgaagcag gaagatcacg aggtcaggag atcaagacca tcctggctaa 63420
   cacggtgaaa ccccatcgct actaaaaata caaaaaatta gctggacatg gtggcgggca 63480
   cctgtagtcc cagctactag ggaggctgag gcaagagaat ggcatgaacc caggaggcgg 63540
   agcttgcagt gagcccatat cgtgccactg cactccagcc tgggcgacag agcaagactc 63600
   tgtctcaaaa aaaaaaaaaa aaaaatcttc ccacaaggca aattccaggc ccagataaca 63660
   cgtcttgtga attctaccaa acatttaaag aaaatataat agtaattcta ctcaaactct 63720
   tccagaaaat tgaataggag taaatgcttc tcaactcatg agttctcccc aactctgtga 63780
   ggctaatatt acccagatat caaaaccaaa caggacatta catggaaagt acagaccagt 63840
   attcctcatg agcatagata taaaattcta aacaaaatag tagtaaatca aatccaacag 63900
   tatataaaaa agctaatgca tcgtgaccaa gtaggtatta tcccaagaat gtaagattgg 63960
   cttagcattg gaaaaatcca taaatgtaat tcactatatt agtgaaacta aaaaaagaaa 64020
   aacaatcata taattgtctc aacagatgca gaaaaagcac ttggcaaaat ccaacatcta 64080
   ttcctggtga aaactcttag caaactgaga aggaaacttc ttcaatccaa taagggatgt 64140
   gtacgaaagg tctatagcta acatcatact taatgaaagc tatcctccta ggatcaggaa 64200
   caaggcaaga acatccactt tccccacttc tatttaacag tgtattaagg gatctagctg 64260
   gtgcaaaaaa aaaaaaatca agatatgaaa taaaaggtat ccagattgga aaagaaaaga 64320
   aattctattt gtagacaaca tagtggtcta tgtaggaaac actatggaat ctattaaaaa 64380
   tctactagaa ctaataaatg agttttggag gttataagat caatatacaa aaatcagttg 64440
   tatactagca atgagcattc aggaattgaa atttaaaaac aatagccttc acaatagcat 64500
   ccagtaattt gaaaaacagg aataaatctg gccaaagata tgcaagatct gtacgtgaag 64560
   cggtgttgat ggcagagact attctgcccc tgagcccacg atgtgcctgg cactgttctt 64620
   gtcggtgcac tcaacccttc ctgtcttagg ctgctctctc tgcagtctgt tcagggtctt 64680
   tccaaattac cagctttttc tgggtacttc agatacctgc atcactactt tctttctttt 64740
   ctttcttttt tttttttgag acagaatttc gctcttgttg accaggctgg agcgcaatgg 64800
   cacagatttc ggctcaccgc aacctccgcc tcctgggttc aagtgattct tctgcctcag 64860
   ccttccgagt agctgggatt acaggcatgt gccaccacgc ccagctaatt ttgtattttt 64920
   agtagagacg ggttttctcc atgttggtca ggctggtctc gagctcccga gcttaggtga 64980
   tccgtccacc tcggcctcca aaagtgctag gattacaggc atgagccacc atgcccggcc 65040
   tgcatcacta ctttcaagaa gatagcagag aaaacagaaa aagcaatcaa tactttctca 65100
   atcaccatgt tcttcctgag ggtctctcct tctcttgaga ctgcagaagg aagaaactct 65160
   catcctagta aacttgtggc tttcctcttc ttttaaactt ttacatgctg ggactcattc 65220
   ttcgtgttct taggaaaacc tgggttctgg gtgcatacct acacctgttt ttcacgggca 65280
   tcttctaatt tagagactgt ttccctgtgt cattattttc atgcccagat ttagctggga 65340
   tattgctagg ttatttacaa gcacacagct aatatgtcaa aggaacaagg cagaggacca 65400
   taactcattt ttttttcttg gagaataaac aaaaaggaat gacaaaaatg tagaacatgg 65460
   taagatttta caattttatt ggctaatata atgctatcta aaatctgttt tttttaaaat 65520
   caaaaggtaa taaacactct gttgctgatt ttatgaacct atgcctggga accacagtgt 65580
   tgcctctgct ggatatcact gtgggggcag cagaactttg tgggcggtac ctacaataaa 65640
   aaatgcttta cgctgttctt tgtagctgac tgcttctgac attctgagca ttgatcgagt 65700
   tgcctatgaa tgttttggga caggggatga agacactcct cacccctaca aagtacatgc 65760
   attcctatct ctgaagctct tatatttatt tttactaaaa aattagaata taggcatgaa 65820
   tccatgtatt tacaaagtat tatgttaatg aatgtaacaa ttataatgtt attcaaactg 65880
   agaatctaat atacagagga tatgtgagag caggtaggat ttgatcaagc aatgaaatac 65940
   agaacgaatg atcacctggt tgatcaaaag gaaagaggaa gagtgtgaag aagattcttg 66000
   ctcgagtacg gatttaaaac gaccagattg tccattttgg atgaagactg gctttctaga 66060
   gaaggacttt tgttctagag tccctgaatt atctaaacct taagcagcta tgtccaatga 66120
   acattttgcg gtgaaggaat gtcctatatc tgttctgtct aatatgatag cctctagcaa 66180
   tgtgtgggta ttgagctctt gaaatgtggc tagtgcaact gagggattac attttttatt 66240
   tcatttaatt taaaataatt taaatagtca tgtttccaga ggctacatga attgcacagc 66300
   acagatctgg aggaataaaa gggaatccag tagccacctt ctgagactcc atgcctaagt 66360
   cacactcagg ggaactctgc ccaaaatctt gtccagttct gggtcctaag tctttcatgc 66420
   cattactggg gaattcactt gataggcttc tattattctc tgtctcagga ggactcaaga 66480
   tcagtggctt tggcctctga ggacaaacaa gcttatattc taaatggtat gctgagcttt 66540
   tcagcaggtc taactatccc agagtaggaa caactcagca ctgccatcca ggctcttaac 66600
   acagtaatct acccgtgata gggcaagaaa caaagaagga atcagtggtc ctatcgcctc 66660
   ttctcccaac ccctgggccc gaggcagaga actttcctag ctcccattgt ccctcagggc 66720
   aatcatggat aacagtattt tttttttttt tttttttgag acggagtctc actctgtcac 66780
   ccaggctaga gtgcagtggc acgatcttgg ctcattgcag cctctgcctc ctgggttcaa 66840
   gcgattctct tgccccagcc tcccaagtag ctgggattac aggtgtgcac caccatgcca 66900
   agctaatttt ttgtatcttt agtagagacg gggtttcccc atgttggcca ggctggtctc 66960
   gaacccctga cctcgtgatc taccctcctc ggcctcccaa agtgctggga ttacaggtgt 67020
   gagccaccgt gcccggctgg ataacagtaa ttcttgatgg aaagccattc acctattgct 67080
   gagctgttgc caagttgttt gttctgaaga aatacaagag gaatggttgg gtgtgaatgt 67140
   aatagttgga cattagggaa atgattaagc aaatatggca ctcctgcatc tgaccagatt 67200
   tttcttcttc ctttgctgat agtggcctgc tcttcactct tccaagtatc tttactagtg 67260
   gagacttacc tctcatgggc tctgttagtt cccagaaaat cactgaatga caacactaga 67320
   aacaaaatca ttctagttaa catttcctca aaagtacata gttatagcta cttcttttac 67380
   tcaaaatagt atattcaaaa ctaaacctat ttgaccatct catagctctc agcttttgtt 67440
   tgctggtcat aataataatc ttaaggaagc cctttgtaat tggacattgc tcagaggtga 67500
   tgctggagaa gggaggatag gtgttgttta attctgagac accttgcatg ctagactgaa 67560
   gttatacggt acaagataag ccatggagaa ctgtgacaca ttcctgagta cagtcattgt 67620
   tttgatgatc tattattttg ctagaatcta ccctgaaact tgatgactca aaacaacagt 67680
   gtattattat ctctcatgat tttgtgggtt gatgaggctc agctaggtgg ttctcatttg 67740
   gggcctgtca tgcagttgta gccagacagc cactaactag aattgactgg cctaacatcc 67800
   aagatggtgc actgccatgg ctggaatgta ctttggctga cagctggggc tgtggactgg 67860
   agcacctgta catggtatac atgtggctcg agcttcctaa cactatggtg gtctcgggac 67920
   agtcatggtc cttacctggc aagctctggg catcaagact gagtgttcta agagtcccag 67980
   gaagaacctg aaaggctttt tatgattgag ctcagaaatc ccagaaagtc aattccacca 68040
   tattctattg gttgaaattg tttgaacaag tcaataagcc agtccagatt caagaggagg 68100
   aaaattagac tttacctcta aattagatga gaagcaaaga atttgcagtt atctttagtt 68160
   taccacattg atcttattaa aaggaagcca cctagagaaa atacattgac agttatattc 68220
   agaaagatta aaaggttgaa aggctccaac acaaaaatct agattttgga aattgcttgt 68280
   gtaaaaggaa aagtgacaaa acttagagat caattacaaa aaattgcaga aaaccaaaga 68340
   tggtgggact gtgtggaatt acaagggaaa gagtattaca tttaagaata agtttaaaaa 68400
   gggttgtatt cttcctacat gtctggcata tgatggagaa cagaggttag aacaggcact 68460
   tcttggatga tgggaagggc tgccttttgg gtgaccatag acagttcagt tcctggtgct 68520
   tcaaatttta tgtgttaaag caatcagaag ccagtggcat gagagacaga ggccactaat 68580
   ctggttctgt taggtgtaac agtacttgga tgtcgccaag tctattggcc tctggaactt 68640
   gctgtgctat atctattaac atgggaaaaa ccaggtgcaa gaagcctctt tcactcattt 68700
   gccttcactg agcaagattc aagctgcaat tagcacaaac agttggctgg tttgcctgtc 68760
   acttatgtct tatcttcctc ctcttattaa ggaggttaag aaatggttta ttgcctatga 68820
   ccgtcttatg aaatatgtca cactttctca tcagctctta tttcctgttc acatgtggct 68880
   ttctggttgg taaaggtttt attaaatcag aggaatttga acggaaagat aatcatagta 68940
   gcccaccacc aattgcctag ggaaagggag agtgttgagt agattgaaat tgagaagttg 69000
   tagtgagaaa aagtaaggcg aaatgtagat aagaaaatag ttacttgaaa gtcagcagat 69060
   ggaaaatgta tgagaggagc gataaaaaac ctaggctgta gaaggaagtc catctaggac 69120
   agtggtttcc aatctttgtt gtggttctgt gagagtttac tggcagtgcc ataaataaca 69180
   aaaatatgtg tagcaaatta agaatcacag aggccctctg tgcaaaaatg taattttctt 69240
   tatttcttta caattgaagc caactagact taggaattct catttccacc tgatggtaga 69300
   aaaagatctc actaatttag agttgttttg tccccactgg ttcaagactc agtctctctg 69360
   ggggtagtaa cacaatctct tcagaattct ttccttccaa accctgcatt tgttttttct 69420
   gaaggatcaa tggagttctt tgggggtggt ggggctaaag tggaccgagc tgaagaatgg 69480
   ttgtcctggt gtcccgggag gggttctggt ccatgagtta tcctctgttc ttgaaaactt 69540
   cagctaggac catgcttgtc ccatcagact aggtccaagg gctctttggt cttcttagct 69600
   tctgcactgc cccctttgag tgtctgggga ctctcagctg ctttacttga acactctggg 69660
   atgcactgga cttttctcaa ggcagcctgt cctcaggccc aggcatatct gctgccaggc 69720
   ctgctgtttg gctcaggcca tgttggacac gggggctttg ctatgaccct ggtggtgtcc 69780
   agaactaggc tgggaagcca aatggcatcc ctctcttcca gtcttctcca tcaacctctt 69840
   cagtcaggag gaggccaggt gatgcagttt ggatatttgt ccctgcccaa atctcatgtt 69900
   aaaatgtaat ccccaatgtt ggaggtgggg gctggtggga gttgtttgga tcatgggggt 69960
   agatccctca tgaatggctt gggccatccc cttggtgata agcgagcttt tgctctgagt 70020
   tcacatgaga tctggtcatt taaaagtgag tggcacctcc cgcacctctc tctcgcttcc 70080
   tcctgctctg gccacgtgac gtacctgcac ctccttcacc ttctgtcatg attgttagtt 70140
   tcctgaggcc tcctcagaag cccaacagat ggcagtgcca tgcttcctgt atagcctaca 70200
   gaaccatgag agaattaaat ctcttttctt ataaatttcc cagtttcagg tattttttta 70260
   tagcaatgta ggaatggcct aacacaccag gatttctctt attaggtggt gctgaagagt 70320
   ggaagctctg agtcactcct cagaaacctt tcagggtcta gtaaagagca gctccctccc 70380
   tctgcttctc cccttcccac ctgctggtca actctgttcc ttggagtggg aacaaaaaca 70440
   aatagcttta cattaccata cattcttctg tactccgtgg tttatgatgt aaaccttatg 70500
   gtctaagttg tccaatactt tggctttgga ggttcaagga aaaaaaacat aaaatacttc 70560
   ttctttcaag cctagcctta atgtttataa tggacagtct ttcaaaatgt cctgccacat 70620
   gggccatagg agatgcttta tttttcaggc attggattat gttgtgttgg tcaatatgat 70680
   tttgtttcat gtgcattatc agaaactctt cccaaagagg gtcctgggat ctgatcagct 70740
   agacaagtgg aggagaaact gaaaaccctc aggggacagg gtctcagtta agtctcctga 70800
   atgataagca tttcagaggt gggagggaga caccccggaa ggtgtccaga ggctaacaat 70860
   gctatttctg tttcttgtaa gtttttgtcc aagatgtggg ccattggatt gcatcactag 70920
   catacaatgt cagtgtaagc agtaggcttt acactaaagc atgttgatgt gctcagagca 70980
   tttttgtggt gctgagacca gagcagatag ggatacaggg atgacagtct ccaccttacg 71040
   gaagcctcct acccagggga tggagtgaga caggcaggca caccagttat ccaaggaaag 71100
   gaaataaagg gaaggagaaa gtcagtgaaa gtggtcagga tggctgagtg agggagaagg 71160
   tgacttgaag gtcccagata gaacagtggt aataaggtga gaaagaggag gtcttccaat 71220
   tccatttctg tgttgggcgg gtgtttgaga gtcccacctg ggtctagagg cctgcatgtc 71280
   tagcaggaca ctgggtagga gtggctgaac ggggacaaca cttaaaaaag acaaaaatta 71340
   cggattttat ttctacatgg ttgcacaaat caatttcact ttctacttgt aaactgctta 71400
   aaaaccttca ttatgaaaat ggaaggacga atttatgttc accatcatag tgcttctggg 71460
   ctggttccac agcatggagc caggcccacc tgctgtagct ttcagaactg ccatttggac 71520
   acgtggttga agcagccttc agttggagga ttcacagtaa tggcatcact gtgggagctt 71580
   agaggaacaa ctgcttgcaa ccccagctgc actggaatta cctgggagct ttaaaaacac 71640
   tgccttttgg gcctcagccc cagagactct gacgtgactg atctgaacac aggagtgtca 71700
   tgtcctccgg tgattctgag tggagtcaag gctgtgagac cttctgctgt ataaaggaat 71760
   attgagagct gggattgtta aggaattctc ttaatgctac gtcactggcc tctgatgtaa 71820
   gcctgctttc tcgaggaaaa ggaaccataa aagcttctgg aaacagattg tttaagaatg 71880
   agaaattgtc atcatgccag gttgcaagac agcttttagg gcagatgagc ttctgctgct 71940
   cattccttca tcttttcctc cagcctggat ttgtccgggg gcagctgagg ctcaggttac 72000
   ctcaattctt gagtttctga agaggcatat ccaggggtgg gttacgagaa caagagcctg 72060
   cccagtctga aagggatgca ggaaggggcg gtgtgtacta gaaggaagga agctatcatc 72120
   atttattaaa actcttcttg gaggatggcc catgaagacc tccaaacaag ctggaggggc 72180
   cagtcacttg ctgaagacta gcgaagtgga gggggaaagc ccgagggagc tgcagactcg 72240
   accactgcgc cctcccctcc tctccctgca aggagcccaa ggtgtgatcc tgccaggcgt 72300
   tcgttcgctg catgaccttg gccatccttc cattcccctg ggtgtgaatt ctcattggca 72360
   aagtgagctg gtgggcataa gtgggtttta agtttaaaaa tttaaaaacc ctgtctgccc 72420
   ccaagtgtgg tatcaagatt ttatagtatg acacttaaat tgcttttttc atccgggcgc 72480
   gtaacagcaa caatgaaacc agcagataac gcgtgagtag tatcagctct gggcctggca 72540
   ctattttata tgtattagct catttttttt aaaaaactgt tttcaaccac tccatgagat 72600
   gagtgctctt atgatccctt tttcacagaa gaggaaacgg aggtacaaag aaattagtgc 72660
   acaaagccag tcggagagag cccctggcca ggcaccaagc tccagaggtc gctctggcga 72720
   gcgtttgctt cgggatctga tgccctggag tcgccaactc aattcgcggg tcgcagccag 72780
   gctccatggg ggtagtagag ccaggtcgta gtggctaggt gagttgtctc aactaactct 72840
   agtggagccg ccgcagccct ggaggagccg ggccgcgact cgagagcgcc cggcagctct 72900
   ccaatgcttt ggaaccggcg ggacccctgc gggctacccg gggcagggcg gtgtccgcag 72960
   gggtctgtcc agccgcgcct gctcctccgg gtggagagtt gggacacccg gccagctcgc 73020
   tcgcagcccg cggactggcc cagccgagcc gtctccgccc gttccccgcc cccgcagcgg 73080
   cggggctaga gccagagccg cgcccggcag ttcccggccg cgagggcggg cgcagcttgt 73140
   ggccggcggc cggagccgac tcggagcgcg cggcgccggc cgggaggagc cggagagcgg 73200
   ccgggccggg cggtgggggc gccggcctgc cccgcgcgcc ccagggagcg gcaggaatgt 73260
   gacaatcgcg cgcccgcgca ccgaagcact cctcgctcgg ctcctagggc tctcgcccct 73320
   ctgagctgag ccgggttccg cccggggctg ggatcccatc accctccacg gccgtccgtc 73380
   caggtacggg gaagctggag catccgggga ctaggtaggg gtgagacgga ccacagcctt 73440
   gacaccttcg agtcccccgc tctgcaggac gccgtgagcg tcctccctaa gttgcacttg 73500
   gttgtcagag tgtgttgtgt aagaagggga tggcggtgag gtctgcgtgt gtgtgtttct 73560
   tgtgtggagc aagagttaac gttgttcagg tcgtttgaag gaacttgcca gttgttctgg 73620
   aggggggttc taggtaggtg tgtgacttcc cgtgtgaatg gctttcaggt tttctatccg 73680
   gctacgttct acaggttcag gagcgtccct cttagaggtt aggctcagga ccgtcagtct 73740
   ccttaacaag gtaagggctg aggccttaac cacactgggg agggtaactt gttgcccctc 73800
   cagataaagg atggtgctag ggaatgtagc tacaagcaag taggcgctga gaagacactc 73860
   attatttaca acagtcatgc ccagcacttt acagtttata agcccttgac agcaactggt 73920
   cttttaccct ccctacagtc ttgtgaggag ggtgctgtca tcacccctgt tttacagcgg 73980
   gggaagcaga agtttaggtg accaacttgt tggaggatga tgtcctgaag gagggtaaga 74040
   cctctgcatg cagcccccct ctgtcttccc tcccactcgt cctgcctgcc agggctttct 74100
   cctctgcact gccgggagga aggaaggccg ctgggaaggc acgcagatgc tggctcctgc 74160
   tgggctgtct ctccactgcc ttatctttgg ggcagggtag cttctcagat ggccacattc 74220
   ctgtgattta gctttacatt ctaggaactg aggcaaggga caccatgaac ttgggctctt 74280
   gaggtcatgg agcaagggtc cattcgtcac gggaatgtga tggatgtctc agtcgctgcg 74340
   gtttggtgcc aggcttctgg acattgaatg tatgaaacag aactccatga ctattgatga 74400
   gctgaggaaa gccagaggtg gcatggggac gttcactgtc ctttttgcca tccagtttac 74460
   caagcggcta ttggcattgt tttctttctc aatcccttct aatttattgt gtgatatctc 74520
   atttgatatt tcatttaata ttctgtattc atatccgatg ttaacgttta tgctagacag 74580
   aaaggtagag cctttgagaa tggggtttgc tggggcaggg gtgtggaact ctgatttatt 74640
   ttcagtctct ggctagctgc caagctgctg tgtgtgaagc gtctcctgca cccgtccact 74700
   ctcatgtggc ctccatgctg ttcaggaaag gatatttgat ctgaattctc acatgacctt 74760
   ggcaggccca gagcagcacc cccatttcta ataggccctg cttgtatgtc ataatgtgac 74820
   atcaagtttt ttttttatgg ttctgaaacc tgtgacatct tttgagtcaa cctctcacca 74880
   ttgttgaaac actgcttaat agtagattca ctaatttatt aaagatttat tgactatcta 74940
   ctatgtgcca ggcactgtgg tgagtggtgg ggagacagcg gtgaatacga caagtgaaaa 75000
   cctctgtccc cttatggaca tgagtcttct tggtaagaca taggatgtca gcagtctctc 75060
   ttcaaaaggg gggtctttga aatccgcccc tttgtacaag ggtgtggatg cactggctac 75120
   ctagaggaca ccactcagca gccccaatgg agaggacgag gagatgatgg gcacagatgt 75180
   agctctgtgg ttccctagag aatcgcaggg ctgctattct ccaagtttgt ggtgtccctt 75240
   gcctcagttc ctaggatgtg gagctctagg aggtggagct acactctgtg gcttggccat 75300
   gcacagggac cagggcagat ggaaaccctg tggctgggat gcatcagtgc gagctgagtg 75360
   tccacctgca agctgggaca ttatggagaa gacatctcag taataagaga ggcaaagctg 75420
   gacaattgcg gtgaaactac catagcatag tgggtggaga agtacagaga tccttatatt 75480
   cttatgctac ataatggata tactaggttc ttgccatttt aaaagaagtt gtcaggggca 75540
   cctgtggctc acctgccatg gcacagctcc aggatgctac ccatctgttg ccgtgaatgg 75600
   gtgcttggtt tgcattttct tcttcttgag gttgtgaggt gggctggtgg cacaagacaa 75660
   ggatctgcac tgtcagcagc ctttttctcc tctggttgtc tgctgtgggt ccaggaaatg 75720
   ctgctggagc ctcccggcca tgcctagtgc agggagcatg tgggaagccc tggccccgag 75780
   ttatctcacc aggtactgaa aggggctgga gctctttaag gtcccagtcg cctgcatgtt 75840
   ggattctgtg ctgtctcctc ttcatgcaca tccaggagct cacacatggc ggcacgatgg 75900
   agacacctat cacttcccaa gaggactcat gcccacgagc ggacaggggg tctcataaat 75960
   taaaagtagg gtaaaaggaa aacaagagcg gagatgaagt ggagtctgaa atgaggcttg 76020
   acatgtgcag tgtaaaccct gatgacctag atgctgctaa agttactcaa gtggggccgt 76080
   gaatgtggag ggaagcctga gtcttagctc tgcctgcacc ttagcactgc ctgaagcact 76140
   cagggaaatg ccaggactgg atgcagagca aacaaactag catctctgga ggcgtgctgg 76200
   gcacgggtat tttcaaagcc ccccaggtag ccaaggttga caacataaat acataaatac 76260
   tctttcctgt aggcagtggg aaagaggaaa ccctgcaggg cacagccagc agtgtcttca 76320
   agatgaaagc aaagtgctca ggaaaaaaag cattcttggc acagagagaa gtgggaagtt 76380
   tcttttaaag atccctgaaa aagaggtgag aatagtgagt tttcggtggt ctcttgaaat 76440
   gaccctccct gtaagcacag ttcggtaatc atggttcagc gagctgtgaa caggaagagt 76500
   ctagccatgc tggtctctgc acatttggcg tcccaaatgt gagaactcat agatccctga 76560
   aaatacagcc aatgactaat gactaagagt taaatgaaaa gttagatggt gtcactttcc 76620
   atcagtgggt ctcacctcat taaaacccaa cacccccctt aatgcaaata ttttaaaaca 76680
   ccccttttat aattttgaaa cgatataata aaacttatct acaacataag tttaaaaaat 76740
   caatgtaata cttagatgtt aaggagaaat aaaagcaatt tgtattaaaa ttatgcattt 76800
   cagtgtgcag atgcttgagc taagctgaac tacaacctag tgaggtgagc cattgctttc 76860
   tatagtctgc aggacagctg gcatggggtg ggaacaggtg agtctcagtg gccatccaaa 76920
   tgccactgaa ggatgaatga ctctcggtta aggtcttgat ggtagccaaa ggccattctt 76980
   ctctcaattt tcacagtgat tgcattcctg aaaaatttag tgtgtataaa cttgtgccaa 77040
   caatatatgt atttcatatg aatgtagaag ggagttgggg ggctaggctt caaacattgt 77100
   aaatatgttt ccgtcagtat gaacgtctgt gagacctctg gaggtcatgc aggacaagtc 77160
   ttttctgagc agaactgcct gtgttgttcc ggatgtcaag catcgatggc ctccaatgct 77220
   aaatgcatcc tgatcaccca gggtcccctt ccccacaggt tccgaaatgc tccctcaagg 77280
   gccataccca ctccactgag aaccactgct ttccctgcat tgctgggtag tgagtgatta 77340
   aagtgccatt gcaggcgtgg gtgccgagat agcagggcac cggacatgtc taagccagcc 77400
   atcaggaatg cacttccgtt gcttgctgta ggatggtaga ggtcgagtta gaaatcagaa 77460
   aggttggtgc tttgtgtgtg ggggtgaagg gccacaaggg aactttgagg ggtggcagaa 77520
   atggtctaca tcctgaagta ttcctggtgc acacagttgt tgcaatttat ggaactgtcc 77580
   acgtaagttt tgtgtgtttc accatgagca atatggtgaa aaatatttac cccaataaaa 77640
   aaattataga agtgaaaaca atgtaacgat tcttgtagag aaggcttagt cctctgaaac 77700
   tgtctgtgga tcccaatctg agaaacagct gttcttgcct gactgggagg cagtgctcca 77760
   gggagaaagg tctctggttc tcagtcaact ttttgacaag tccagtgcaa tagtggcctt 77820
   ggcatcgtag accctggtgg ctggagagga gatgtgttgg agttataggg gaggctggga 77880
   agcacaaggc agggcattta gaaaggggac aaaggacaag agatgaggcc catccctgtc 77940
   tctgggttct ggttctgttg tccaggagcc ctagaaggtt tgtaggggtg agatggaaaa 78000
   gtcagacttc aacctagaac ctagtggtca agaggcttgg ggtaggtcca tccccatctg 78060
   tggcatctgg tgacggtgat cctgtctttc tgtgtctcat tgccactttt gcccttaaaa 78120
   tccacatgcc ttagttgaaa ccaccaatct tgtggctttt cgccttgtat tagctgaata 78180
   attgagattt catagcgaca cttgaaacct cttgggaaat ttttacttta tcttcatatc 78240
   cttgtgcatc ctcaatgata aaaataaaac tttccttctc ccatttacat gggtaaataa 78300
   gccattgata actatgaaca aggctttagg attcctctac tgataggagc attgccaggc 78360
   ttggtgtttc ctgggctttc tgagatggtg agtgagcttc tgtttccctg ctgtgggagt 78420
   gtttactcat agtttggtag agctgtttct ctgggggaaa atcagaggtg accacattga 78480
   acacacagtt gggtttttgg gttgtgtgca catgatagag cgtgggtgac taatctccac 78540
   gagatggaaa ccattgtgtc acctttggaa cacactacag tggtccccag cacgctctgt 78600
   ggttatttcc acactttcct gctctagctt gggtagggaa gttcctgggt gggctctgtg 78660
   atcattaggg gtaggtcact ccctctaatt tgccccacag gtatgagtgt gtggggctct 78720
   gatgcttcca tttctcatca gaattgcata gagattatta tggacattta cttaggacag 78780
   tgcctctcgg attttacctc ccagggattc cttctgggaa ataaaaagca aaggctcaac 78840
   ctagcactcc aaagcagccc actgcaagtg atgcttcttt gaagcctcat gttttgtctt 78900
   agatcgtgga gatttttctt tctgcttcat aatatagccg tgtttgctct aatagaaaaa 78960
   taataactcc cctgagaaaa tctttggatg aaattagtaa cctagggaga tagacttcag 79020
   gctctctggc tgtctgtagc ttctccctgg gaatttgtgg attctaagcc agggcttgtg 79080
   tgagtatgga ggtctaaagt tttttccata agtgtgtggt tctatggctt tgtaagtccc 79140
   ttttaccttc tttttcaaac tgacattttt gtttttaaca atgatgggga ctttttaata 79200
   gctgtatgaa tcttataaca aaggaataac acaggaggtc aaggctgcag tgagctgtaa 79260
   tcacaccact gcactgcagt ctgggtaaca gagcaagact ctgtctcaaa acaaacaaac 79320
   aaacaaacaa acaaacaaac aaacgggtaa caggaagaca tcataattat gggcttggtt 79380
   tcagtactgt aacacattcc ccgtacatgt ttccaccgat tttcagtaga acacatgggc 79440
   tgtgacattt catgagctaa agaacctgct ttagcccttg ttttttcagg actcattcat 79500
   ggtcacttct cagttatggc aaataagttg gagcctaatt ttctaagtat ctctccatta 79560
   catcagatct acacatatgc acatattggt taatatttgg gatgaggggc tgtattaaat 79620
   gatgctgtaa atgcatttac tcattactcc atgggaaggt agaagagtgt gttgctatca 79680
   ggtggaaatg tagaaagtgc agtgaggatt ttcttgtaaa tctcagattt ggggcttggc 79740
   tatgtcaggt ccctggaaga aggaggagtc atttactgcc gtctgttctc tcctcttggg 79800
   cctctgtaga gatttggccc tatgccttgg cgggctctgc tctgcagctt ggccagacac 79860
   atccctcagc atggcaagaa gtgtggaagg tcaagaggat gtgcccactt ggcctaatcc 79920
   taacccacca gcacccctgt tcttaaccat gtccctcatg tacctgggat ccctgaattc 79980
   cctccccaaa caccctggac cctaattcca gggcctgtgt ggtcattccc atggactcaa 80040
   ctgcctggtt catagactgg ccagggctgt acagaggggg atagacacag gtggacttcg 80100
   acacctacct gtgtgcatgc tcagggcccc ttccaatatg gaagggagct ggggagaggc 80160
   tagagacaga ggaatggaga ttaatgtagc tagacacagc aggagccatg cggtgggact 80220
   gctccgtgca ggtctcatgg tgaggacagc gcagcctatg ttttgacaga catggaatac 80280
   caggtgccag gatgagcttc ccccagctct gggcacctct gctgccgctg tccccagaag 80340
   tggacctcca gcaggttctg atactatctg gggtgcgcct gctgtgtgcc accttgcctg 80400
   tgcctagctt ctatcctaga aggcagggct cacaatgtgg gggaattcct caggaataag 80460
   aatggaattc ctcaacattg tggaggaacg ttgtgggaaa ttcctcaaga aaaagaagct 80520
   gtttaataaa tgcactgtgg ctacacagca ggaaaaatgc ctccttcccc tgcactgggc 80580
   cctaggcctt gaggaagaaa cctgggttcc tgtgacagcc cctctctccc cagctgtcac 80640
   ttttcccgtg cgtgcactgg gtatgaaatt ccttgtagag ggacaggaag gtgctgttgg 80700
   atgaggtcag tatttcctgg aaggggggct tctgttgggg gccaatatgc tcattgttaa 80760
   ttcttgtact taatttctac ttttgtaatt tgtttcacat tacagtttct cattctggga 80820
   ctagtaggaa attactttat gcctcaaata tgcattttca ttttgtcttt aatcaacaag 80880
   taactttgtg tgtgtgttgc acgtgctgct gtagtcacgt tgtgatacgc cagtgttacc 80940
   aaatacgtca tgatcaccaa acagcgatgg gggctgtatt tgggaagatc ttcacaattg 81000
   caagcttttg gatgtgattg tcacataaag ccgtggtgtg ctgcagtgaa tggacaaggc 81060
   ctccggaagt ggggatgacc agcctaggcc ttgggtcacc ctaggatctg gctgactccc 81120
   tgggtctgga catcagcatc tcagcactgc ccgctgtctt tcctggaagc tgctatggag 81180
   aggatgttag ccagtccagg ggtttcacac ataacataga tatccttgct tgaaggctga 81240
   acagggggaa ccgtatgcta tggcttatta atgtattgag taagttaatc cttttctctt 81300
   tcttgtttta ttttatgtgg aaaattcaat ctgaaaaata acagtggcat acacacatct 81360
   gtgacttttt aatgttactg gtattttcta aaggtacgtt ttgaatctta ctataagtca 81420
   tttaaaagaa ttacaatgga ctttgattaa ctagttctga ataagagagg tttctttacc 81480
   tagtatgcct tctggcagta acatttctta gtctctagtc ctattcctgt agttccctct 81540
   cctcagggtc atccccaccc ctcaactctt tcgacagcca tctgggtgtg aactctttat 81600
   ctaactcctt gtgtaggatt ctaccctctc ttatatgatt ccggccatct ccagacccca 81660
   gtgacggttc ccttgcaatc ctattctctg acctacaccc agtaccctta ttccatcctt 81720
   tctcttcttt gattctaggt tgggttttca tccccgtgtg tctcatgcaa aaatggtggg 81780
   taggtaggta ggtggattcc ttccctccct ccctctctcc ctccttcctt tcttccacaa 81840
   gtatttattg agctgttaca acagacatgc aagatgtgta ggatgggcct cctcatgggt 81900
   gtgggagagg ggagggtctg ctctgcactg ctttcttccc tggcccaggt agggaaggcc 81960
   aacaactagg gagctaccca atgttaccag agccaggggg cccagagcca gcagttcaag 82020
   gcacctcggt gcattgcacc agggagagcc tgtgtcactc gtcatgcctg gctttggagg 82080
   agcagatgtc tgcaagggcg ctgtgggaag cttctgatca gaatcagctc tgagatggtc 82140
   agaaatctca gaagcttcca ggctaggatt ggaagaattg acttccagac ccaggaggtg 82200
   ccatagaggt gcatggaggt ggaatggcac ggtgcttata aacggcaagg tagggagcca 82260
   gcgctggaca cagagccagc tgaacagtga aggcagaaga gtagggggcg ctgaccctca 82320
   tcccaggggt caggcagaag gggccgatgg gatgggtcag gtgctgcaga gatgttcagt 82380
   gggctggaga cagaggggtc ttggcatgga gaacctagga gggatagtgg cctgggcagt 82440
   gcatctcaga ggagcagtga gcttagaggg cacatggtgg tcgcttcagc aatggatgag 82500
   tggtggagac agtaagtata taaattctta agtgaagttt ctggggcaga aaaggaggga 82560
   aaatgaggcc attagaagag tgtgggggcc aggtgcagtg gctcatgcct gtaatcccag 82620
   cactttggga ggctgaggtg ggtggatcac gaggtcagga gatcgagacc atcctggcta 82680
   acacggtgaa accccgtctc tactaaaaat acaaaaaatt agccgggcgt ggtggcaggc 82740
   acctgtagtc ccagctactc aggaggctga ggcaggagaa tggcctggat gcgggaggca 82800
   gagcttgcag tgagcctaga tcgcaccact gcactccagc ctgggggaca gagcaagact 82860
   ccgtctcaaa aaaaaaaaaa aaaaaaaaaa aaaaaaaaaa aaaagaagag tgtggggagt 82920
   caccttctct gcaagaagct tcctgggcct cccaaataaa tccatccctc ccctccccct 82980
   ccccctctcc cattccatac attttggtgc cattcactat ataattccca gcagctgctt 83040
   tcatatctgg ggcatactca caggggtcag tcaggagggc actgtcctag gactgggagc 83100
   gggaggatgt gggggtctcc cctttgagtt tctgctatga tgtgattcca attgatgggc 83160
   caacacacct ctgatacctt gtgttatgct gtgtactgcc tcaaagtgaa taatagcttg 83220
   ggatttagtt tgtgtagtct aaggaaaata ctttgtgttg aactagtcag gctgtgttat 83280
   tgatggccat taaaaggcct tgtgcttagg tcttacaagc ttttcaaagg cccaaagaaa 83340
   tgattcagac aaaaaaaaaa attagactcc aaaatactaa aagaaaactg caaaaacagg 83400
   tgaattaatg gtgaaagatc tgtaggacat agcagtaagg ctaccacaac ccagcttttt 83460
   gaggttgtct taaattaatg tgatatgggg tgtaggtatg ttaacatgct tgataatgga 83520
   agagtgagga tacctgcttc ttaaacaggg cctgctcatt tttttctggc ctctagtaca 83580
   tttctttctg gcaaaactca agctctgttt tcacggagat gatgcactga gccatggcat 83640
   ccctaggtgg tttgattcat ttctgcaccc cggtgtctag caaagtgcca cgtgcatgtg 83700
   tttaatcaaa atagcttgtg gttgtcaagc tctttgtatt cttccctccc tccctgtgat 83760
   ttgatttcct gaaagtatcc tttccccaag gattatggaa catacacttg ttaaacactg 83820
   gacccatttt catgatcatt taaatgtcat gacctttctg acaccttgat atggtttggc 83880
   tctgtgtccc cacccaaatc tcatctaaat tataatcccc atgtgtccag ggagggatct 83940
   ggtgggaggt gattagatca tgggggcagt ttttcctatg ctgttctcat gatagtgagt 84000
   gaattctcat gagatctgat ggtttaaaag tgtttggcac ttcccccctt gctctctctc 84060
   ctgctgtcat gtaagacgtg cctacttcca cttccaccat gattgtaagt ttcctgaggc 84120
   ttccccagcc atgcggagct gtaagtcaat taaatcctct ttcctttata aattacccag 84180
   tctcagatat ttctttatag cagcgtgaaa atagactaat acatgcctct tgagatcctc 84240
   acagcaattg gtgcctttag acctcttcat tcacacttca ctcatgtgtt cttccttccc 84300
   caggtagacg caccctctga agatggtgac tccctcctga gaagctggac cccttggtaa 84360
   aagacaaggc cttctccaag gtaacagggc tgagtcaccc cagggtccca aggccggcct 84420
   tttctcacaa atagctgaac tcctccactc tcagatccag gggccctgga ctatctccca 84480
   ttgtatgcaa aaatatttta tttccgttga cacattactc tttggggcag cttgatggtt 84540
   ttattaaatt ttggaaggag gcttcagccc cagtaacagc caatgcctgt ttgctttccc 84600
   aggtcacctt gactgtgcta cagtagagac ctgggaaatg atgggttcca gtgctgtggc 84660
   caagctctcc ccagtttcaa ccccttccag gccttgctgt ccatttttac tccactgtca 84720
   ccacatgttc aaattcttgc tttctgctcg agtcagtact gctcacctcg ttccaagaga 84780
   tctgggtcta tgacactgtg ggtgacaccc tctgagcatc ttttccgagc tgtctgtggg 84840
   tacctgaacc tgccccccaa cctggccctt gccctacctg ccccgacctc agcgagtctg 84900
   gcccagtctg tccctgcttc tcccctctgg ccccaagtgt tctcaaatat gcccccgcct 84960
   gcctgtctgt cactgcagct ggcccagccc tcatcctctc tcccatgaac ctctgtggtg 85020
   gcctgctctc cctggctccc ctgtctccag tttatgttcc acacaggtgc caaaatgtct 85080
   gcaaaaaatg gagatttaac atacactagc ccttggctta aaacctgtac atgcttctct 85140
   gccacctgca aatgacagtc ccatttcctc agcaaacctc tgcatgttgt gggaacttat 85200
   ggcttctcag cctcccactc ggaacccacc tctggtttat gctccagaac atgatgttgt 85260
   tggaccctcc cctccacctc ctccaggctg agttagcctc tgtctcccct cctgccctgg 85320
   gcatggctct tccagtagac acccccacat cacagtctaa ctctgtgtcc acattccctg 85380
   caactgctga gctcctggag ccaggccctg ggaaccttgt tcctgttttt gttcccagag 85440
   ccaaatagat atttgatgta tgattgttga atgcagcagt gagtgaataa agacatgaat 85500
   ggttaactga aaacatttta tcaaaactgt tatttgtcat cagagcatgg gccggtttcc 85560
   aacagctaac atttaggagt gaccatttgc caggcactgt tctaagccct ttgcatgtgg 85620
   ccattcattt cacccacata gcgccatcaa tgacatgctg tcactgcatt gcttgtcaaa 85680
   agggggaact gaggcacgga ggctgagctt ttactcaaca tgccatctat gttgagggac 85740
   ttctgacttg gatagccgtt caactgcgag cactatggag gccagtcagg cctggccctg 85800
   ccttggaatt tctgtgttat tcagggacat aatggggttt gtgttgattt ttgttgggag 85860
   tgtgtgggtt aatctaggtg caaccctcac ctccttccct tctgatgtcc tcactcccat 85920
   tggtcatgtg acccttaacg cccatcccaa gctcagtgcc aattccttgc cccagggtcc 85980
   tggggattga catggatccc aaaagcccgc tgctgagagg acagggccac tcccaccccc 86040
   ctgactgttt cccagagcag ctctggggca gaagtaaact tggacccagg gggcataacc 86100
   ctttgccctt ttatagtcta ttagaggagg atgaggtctg acatcagctg gttcattgcc 86160
   ccttgtgccc cttgagcaca ctggggccca gggagagtca catccctgcc ttgtggtaca 86220
   ggccaccggc cctggacctg tgattaaaca ttgtccacaa ttaatttgga attattatct 86280
   cgtgatacac tccataaagt tcagtcacac atcttctttt tcttctcccc tgcctaatga 86340
   tcagacaagc caggtcttgt gactaaatga accgacagct ggcagtgttc atctgatttt 86400
   gcaatgccct tattttgttg ttgataaact aatgcaaata gctggttggc ctggaactgg 86460
   tgattgggaa acattttaac ccttctttcc ttcctctcta tcctaaagac ttaaaacaca 86520
   cttcacgcag tcacagaaag agctcataga ccagactgcg gggcttcaaa gccttctatc 86580
   ttatttaatc atctgtaaat agtttaaagg taaatatggt aataatacta gtattctaga 86640
   ctattttggg aagccagctg atgtgaataa agggtcagag ttaaaagata ttgaaggaat 86700
   gcagttttac tgctctgagg taggtttcaa ggcttgagcc aggcttagag atctttgtcc 86760
   tgatactggg gataaagtga agagaatatg aatgggtatg tctcgcatgg ttagaaaatc 86820
   tttttttttc ctgtatttgt ttaaaaagca ttttgtaact taaaaaaata cctatacacc 86880
   tttttttttt ttgagatgga gtctcactct gtcactcagg ctggagtgca gtggcaccat 86940
   cttggctcac tgcaaccccc gcctcctggg ttcaagcaat tctcccacct cagcctcccc 87000
   agtggctggg attacaggca tgcaccacca cacccagcta atttttgtac tttttggtag 87060
   agatggagtt tcatcatgtt ggccaggctt gtcttgaagt cctggcctca agtgatctgc 87120
   ctgccttggg ctcccaaagt actgggatta caggggtaaa ccaccacacc tgaccataat 87180
   acatcttaaa tagaacattt tccagctaat atttaaatga ctgcatgtca ttagaatctg 87240
   aatgagtgtt gtgttaaaat attttgtgtt ttcatattta tttacttact tatagctacg 87300
   gataagaata taggcaaatt taatctcccg taaaaattaa aaggcagatg ttttcaaagc 87360
   acagaattac atggtctact ctagacattg ctcctaaggt tgagcagagg ggataagagc 87420
   tttttctctt ttttcctttc ctcttttttt catacaaagt tcgttgtaga tattttagaa 87480
   agtatagaaa gccccgacaa ggggaaaccc actcaaccca attttaatgt tgttgtcatt 87540
   tcttttagtt tttttgggtg catttctatg tagctatatc ctattgtaca agcaatttag 87600
   gatcttgcaa tataaaatga aggcatgacc tttgagctgg ccaaaagtat gggctgattt 87660
   ttgcaaatgg gtcctggcag caggggacat aagtggggaa ccaatcccca cacaagcaga 87720
   ggtgagctgg cttaggggcg agtgcatagc tgactttgga tgtagactag atttggggaa 87780
   atttaaatgg ggtccaagac tggaaatgtg taatgggaca gaaggtagaa atgtttaaaa 87840
   tcttaagttg ttgagtttgt tctcaatttt gtgaaaactc tttatccctt tttgttcccc 87900
   accccatttt ctaaccagaa gtcaaatatg acaacattca tgatgtagaa aaaccagttc 87960
   atcagtgtga cttgtgggtg agggtgggga cagggccggt gttggtatat ttagttttag 88020
   agatttgctg gaaaagtaac atttttgctt ttgtctttct ttcgttcctc catttctcca 88080
   gaagaatatg aaagtgttac tcagacttat ttgtttcata gctctactga tttcttctct 88140
   ggaggctggt aagttaagta tttctttgtg ttcttgtctg ctaagaaaat ctgttattta 88200
   gaatacatga agtaccttcc ctaacagagt catttctcat actcgtctct gttgaatctc 88260
   tgggcacaga gcctcctggt catagacctg tactgctgga agttcctctc agcatcttcg 88320
   tctcattctc tcccatatag agtgggaaac tgaggcctag tgaggctgag agcccaaggt 88380
   catgtagcta gttttggaac aaaagctaaa atctaggtct ccaatctagg tccaggcctc 88440
   tttccacttg gctgctggtt gaaagtgtga tatgggaaat tctgactttc atttggagga 88500
   gatattatgt atagtcttta tgaacccaca gctggaagtc atcaagatac actacttttt 88560
   ctgtttcttt ccatttcttc attttctcta atcttttctc tttttgccag gtgctagcta 88620
   ctggggatac agcagggaac agtgcacaca gaaatacctg cctgcatagg gtttatattc 88680
   ttgtagaaga gagagataac agataagtag acatatattg tgtcatatga tggcaagtat 88740
   agggaggaaa aaagagtata aggttttggc attacagtgg gcagaaggtt aattttttga 88800
   atgtggccag aaaaaaacct ccctgagaag ctgacttttg agttgacctt aaggaagggg 88860
   aaaagagagc tatttggata tcagattggg gtgtggaaat attccagtca gcatgtgcaa 88920
   aggcccagag gccaagagta tttggcaggc tttcagaata aaaatatcag catgtctgga 88980
   gcatagtgag agatgaggga agtcataagg gaccaggctg tctgggtaat ggggggatgg 89040
   cagagggttg attatatgga atgttgtgcc cttttttagg gtcttggctt gtagtctggc 89100
   tgggatggga agacttcagg gttctttgtt taagaccaga gaggtgatct gacttacgtt 89160
   ttgattcctg ttttgagaat agatccgagg gagccaaaga gagaaataga gaagccagtg 89220
   aagaggcaag ccatgatgat ggctttctct aggcaagaaa tgttgatggc ttgtcaaagg 89280
   gtggcaagga gaagtgagaa gtagacaaat tttgaaatat tttgggaaca gaaccaagag 89340
   ggatttggag ttgggtcaga tgtgagatgt gagggaaaga gaagaatcaa gaattatctc 89400
   agggaatttt ggcctgcgca aacgcaaggt gaaattgcca ttgatgagat ggggaagatc 89460
   aggttttcag actggatgtg ttaagtttag ctgtccatta gatgtttaaa aggtgacttt 89520
   gactaggcag tctacagcct gagggaaagg tctgggttga atatataaaa ttggagacct 89580
   cagcaagcgg ctggtatgag aaccatgaga ctgaatggga tcatggagtg tagttaggaa 89640
   agataagaac tttaaggact gagccagaga ttgacttaaa ttccttggtc cagtgagttt 89700
   tctccccttt gctgaggggc tccatgtgtg ttggagcata ccttcaacat taaggctgtt 89760
   taaggcctta gccttcactt cctgcttgtg tatgacctca gagtcatcca gaaatgagag 89820
   cctggtgcct tctatgcaat ttcctgggca tgcacacagc cctgcataca tgcccaccct 89880
   tctgggttcc ccaggaatgt gtcagagctt ttcaaagccc tctggaggtt atggctctgg 89940
   tattgcagct ttaggcagtt gtaaaaaaca attaacactg agtttttcca gcaaacgcct 90000
   tggggatagt gcttttgctg tggagctctt ggtgaggtca ataacaccac caccctatga 90060
   atgtgacttt gaggacctgc cagtcaagtc aaagagtggc agtgctctgg aaatgggact 90120
   tttcgaggag ctccaaaccc tctctcttct tgcaagcctg ctggttttca aagcctttgt 90180
   gtctgcaaag ctcttgatat tcatggctgt tttagagctg ggaagagtag gatggaagtc 90240
   atttaagtta taaagctaca aaccctgctg atccgtgatt tggcatgttt ctgtttgttt 90300
   ccttattttc tagtaaatgc tcttctggtt actgcaagcc tttgattttt gaaatttttt 90360
   gccagtcttt ttgttgcctt tatggaggaa tgatttatga gacccccact ccaccatatt 90420
   ggaaatcttg caaattcaat ttctttgaat agaaaatagt attaatagat atagggcaat 90480
   ccaggctatc tacttcttct caagggagct ttaatagtct ggaaaatttg ctcatttcat 90540
   ttacgttatt aaatttattg gcataacatt gtttataata ttccctcact accctttaca 90600
   aattatttaa aaaagagatg tcatctcttt tttttcctga ttagtctggc taaagattta 90660
   tcaattttat tgattttgtg aaagaatctg cttttggtat ctttgatttt tcttcattgt 90720
   tcttctgttt tctatttcat tgatttcctc tctgatccat attactattt tcttcttact 90780
   tctgttatat taattatgtt ttttattttc tgtatcttac catattttga cattctggaa 90840
   gatgtttcat atctagggag agactgctcc accaggggct aagcagttcc taggtgtgtt 90900
   gtggacagct tacagcttgc ccttcatgtg agactgcttg acccaagatg gtaccccact 90960
   cacctcctta tccaactctc acacacccaa accaatgtta ccccagcctc aatcaacaca 91020
   gggctaggta ccagagaact agggacagcc tctatgccca aagcctgcaa aaatggttca 91080
   aatgggccaa tctgaaactg tttatgctgc cctgccttgc ctttcccatg ggaaccccag 91140
   taaagatgct ggcctaggct ttccttttgt ttctgcttct gcctcctaac ccaatctggt 91200
   gattctccct gtggttttgc gtggtatggc acaccccttc tgtccgaacc actaagtaat 91260
   acaaatcttt caattgcatt agtctctgtt ttgtcactca gtcaccttcg taaatgaaga 91320
   cccaggcata gaatctctgc ttgctgctta ctttggaatt actttgttct ttttctagtt 91380
   tcttaaggta gaagctcaag tcatcaattt gaaattttta aaaaatatat aggcatttaa 91440
   ggctagcagt ttccctctaa gtactgcttt cattgaatgc cataagtttt gatatgttat 91500
   gttttcctta tcattcagat acaaatactt tatattcttt ttgatttctt tgactcatga 91560
   gttatttagg agtatgttaa ttttcaaata tatagggatt ccttggatat ctttctatta 91620
   ttgatttcta atctaattcc attgtcacca gagaataagt tttgtctaat ttgaatcctt 91680
   ttaaatttac tgagtcttgg gttatgggga agaatatgga aaatttgcta agttttatgt 91740
   gtgcacttta aaaagaatgt aatgttctac tgttgttggt tggaattttc tgcaaatatc 91800
   aattatgtca aattggttga tagtgttgtt caaatcttct atgttgttac tgattatccg 91860
   tttaatttta ttgcttgttc tatcggtcct tgtgagtggg gtttctctag tttcttgcag 91920
   aaccagtttc tctagttctt gcagttctgt cagtttttgc ttcatgtatt ttgaagcact 91980
   gttagcagct acataaacat ttggtattat tgtgttcctg ttgattaatg acttctttat 92040
   ttttttaaaa tttatttatt tatttattca ttcatttttg ggacaaaagt ctctctgtgt 92100
   cacccaggct ggagtgcagt ggtgcgatct cggctcacag caacctctgc ctactgggtt 92160
   caagtgattc ttgtgcctca gcctcccaag tagctaggat tatgggtgcc caccaccaca 92220
   cctggctgat tttttttgta ttttagtaga ggtggagttt caccatgttg gccaggctgg 92280
   tctcaaactc ctgacctcag gtgactcacc cgcctcagcc ttgcaaagtg ctgggattac 92340
   aggcatgagc caccacgcct ggctgacttc tttattttta tgaaatgccc cttttttctt 92400
   gtaatatttt tactgtaaaa tctactttga taataatagt cactcaagct ttaactaatt 92460
   aatttttatt aaggtatagt tgacaaaatc gtgtatattt acagtgtaca atgtgatgtt 92520
   ttggtatatg tatacattgt gaaatggtta accctagcta attaatcaaa ctttcttttc 92580
   atgagtgtta gcctggcaaa tatttttctt ttctttttaa tctattttta cctttattta 92640
   taaaatgtct gtatgtaggt gtatgtgttt atgttaggca ccacatagtt gggcctttct 92700
   ttagccaatc tgatcatttc cgcctttcaa tgagagtgtt tagactattt gaaattttag 92760
   tgtgattatt catatattag ggtttaattc tgggatcttg ctatttgttt ttctatttgt 92820
   cccatcaatt ctgttatttt tttctctttt tttgctgcct tttagattaa ttggcatttt 92880
   taggattcat tttattttgc tttttggctt tttagtcata actgtttgtt gtgtatgtgt 92940
   ttttttaatg actgctctgg ggcttaaata gccatctcta agttatctct gtctgccttc 93000
   aggtaacact atacagtttc acatatagta tacaaagtta agagtatata cccatttctc 93060
   ctttcttgaa ctttgcccta ttatttccat atatgttact tctttatttg ttttaaactt 93120
   tatagcattt tattattttt tatttaaaca ctcagttttc tttaaagaga tttaaataat 93180
   aagaaagatg atcttataat tacccatttt tgttgctatt cattcttttg cgtagattca 93240
   ttttcatctt ataatattgt gctttttgaa ggaattaaat atttcttgta atgcaggtct 93300
   actggtgatg acattttaca gctttggcaa atctgaaaaa gtcagtattt tttcctaaat 93360
   tttaaatata ttttcatggg atatggaatt ctaggtagac tttttctttc agtactttaa 93420
   cgatattgtt ttgctctctt ctggcttgta ttgtttttgg aaagaaagct gcatcatttt 93480
   ttgtctttgt cctctgcaat aattgtgtca tttctcccgg gctacataga agattttatc 93540
   tttgtcactg gttttaaaca attttattat tatgtgtttg tagtttgctt tatgtttctt 93600
   ttgcttgggg ttcattgtga tatgggggcc tatgtataca tcatttttgt cacatcttga 93660
   aaaacttcaa atatgttttc tgttttccac atttttcttc ttcagtgatt catattacat 93720
   gtattgaagt tgtcctaagt aaaataattc cctgcccatt ttattctgtt atttttgtct 93780
   ctgtgcttaa ttttggataa tttctaatgc tgtgtcttca agttcactca tctttttttt 93840
   tcctgcagta tcaaatctgc cgtgaatccc atccattgtt ttttgtaact caacattatt 93900
   gttttcatct ctaaaagttt tttaaaatct ctttcatgtc ttcactaatc atgtttgatc 93960
   tttcctgtag catcttgaat aaatggaata caatagtaat aaactatttt aatttttaaa 94020
   tttattaggc ctattgtctg tgtcatttct gggtcagttt tgactgatcg atttttttcc 94080
   tccttatcgt tggttgtatg ttactgcttc tttgcatgcc taattatttt tttggatgtt 94140
   agacattgtg gattttacca tgttgggtgc tggatatttt cgtattcccc agaatgttct 94200
   tgagctttgt tctgccacat gattgttact tggaaatggt ttaatccttt cgggtcttgc 94260
   ttttaagctt aattaggatc aggaaaaagt ttattcttgg cctaattttc cctaatgact 94320
   gagtcagaac ccttctgggt actctgcctg atgccttatc attaatgagg ctttccaccc 94380
   tggctgttgg gaacagcaac tgtttccagc attgggtgtg ctccatgtgt tactccctcc 94440
   agttcgtttg gtagtcctcc ctctagcctt gggtcatttt cttatatgct tacactgact 94500
   gatactcagc tgaaaacttg ggggagactc tcagttatcc caagactctt tctgcgtgtc 94560
   gttatgcagc tctaagtact tgggtactct gccctgcaaa cttttctacc caggttctca 94620
   gctccatctc ctcaactcac tgggctttgc ctgtgttttt tctcggcaat gtcatggcct 94680
   agctgtaatt ttggacacct cctttcttct ccgtctttct gggatttctc tcctttgttt 94740
   tctgatgttc aatgtcttga atactgttgt ttcatatatt ttgtccattt tttagttgtt 94800
   catgtgggaa ggtaaatcca gtttctatta ctccatcttg tactgaaagg catgtctctc 94860
   ctcctacatt gtccccactt tttagatgag tgaaaagggg gtgaaggatg ttaaataact 94920
   tgccaggggt acagcacaca ggcagtaagt gatgcagctg gacttgaata tgcttctcag 94980
   actccccaga taatgatctt atcacaaggc tgtaaagctt ttatatgtaa tgtgaattga 95040
   tgtatttaat aatcaatgtg tttcttcgta gatattaaat caagacacac tggcagattt 95100
   ttatgtaaat tgcttccacc cttcttcctt ttaaagataa atgcaaggaa cgtgaagaaa 95160
   aaataatttt agtgtcatct gcaaatgaaa ttgatgttcg tccctgtcct cttaacccaa 95220
   atgaacacaa aggcactata acttggtata aagatgacag caagacacct gtatctacag 95280
   aacaagcctc caggattcat caacacaaag agaaactttg gtttgttcct gctaaggtgg 95340
   aggattcagg acattactat tgcgtggtaa ggtaagagag gaattagtat gttacaaaca 95400
   tgttctagtt tcctgcagtt gttaaggaca gaaatacttt taataatgct taacttacct 95460
   attttatttt attttagaaa ttcatcttac tgcctcagaa ttaaaataag tgcaaaattt 95520
   gtggagaatg agcctaactt atgttataat gcacaagcca tatttaagca gaaactaccc 95580
   gttgcaggag acggaggact tgtgtgccct tatatggagt tttttaaaaa tgaaaataat 95640
   gagttaccta aattacagtg gtataaggta attttatttt aaatatgaca tttcactttt 95700
   ccagaaaata aaatagttcc ctggacaata gaatgtatct gcaaagtacc tttttatttt 95760
   gtatattgtt ttctctttaa tggtgaaact tgatagggaa cctaaaaaga attgtaagac 95820
   tgcattcact taatttgaag cttaactaga aatttgtttg ctgttgtttt tatttaattt 95880
   aataagatgt aattgagaaa agtactagaa atagatgttt aaagagaagg ctgattataa 95940
   gcttaaggta ttgaagatgt aactatttat tacaaaacat actatgtgtg ttttattgaa 96000
   gatatggtct tcaaataacc tgctagtttc cataactgtc tgaaaagtgg gttgactagc 96060
   tttccatgga acaccccatg ttttggtaac tttaatctac ctagtctgat ggaacttaaa 96120
   tggggaatca tcttgtagag cttaagacgt actaaatacg taatatcaat gaagtgcagt 96180
   attgttaaga aactatatag tgttaacatt tttttcaaac atgtttttct tgcaaaataa 96240
   attggccttc acttcatagt acaagtctca tagacttgaa aacttaatgt cataggaata 96300
   tagttaactg tttccaaaaa gccagtatta aaacttacta tatgtaagga acttttccag 96360
   tcactttcta aaaatataac atttgctaag gtgaaaaaaa tggagctcct ttggcggtag 96420
   attggggaaa gttattggtt attggttttc aatgcttctc tctcccttta tctaggattg 96480
   caaacctcta cttcttgaca atatacactt tagtggagtc aaagataggc tcatcgtgat 96540
   gaatgtggct gaaaagcata gagggaacta tacttgtcat gcatcctaca catacttggg 96600
   caagcaatat cctattaccc gggtaataga atttattact ctaggtgagt catagctcca 96660
   gccctaaaag gtttagatct gggaaggtcc agagactgtg attctaatac tactttcatt 96720
   atccatgaag aaaccaaatc caagacgacc tcaatgatac cacaaaagtc acatggcttt 96780
   agttttgttt ttttgttact aaatactatg taatgttttt ccattcctgc ttcagatgta 96840
   gttctaatta aatagagaca ttatgagctg tgttttccaa ctgatttaag agggttgttc 96900
   tttggcatgg ctaaaaatgg ttcttactga aagctctggg gctccctctg gctggtatat 96960
   gggaatgttc tgagagcctg cacctgggga atctcagtga gtgtcagtgt ctagcagcaa 97020
   gcatcggtgt ggacactaat gagctttgga ataagtcaca tttccagaat ctacctcctg 97080
   ttcattctgt gttaggtgtt aacctctcta accctgtttc ctcttctgta acaagatgat 97140
   gcaatctccc aaggttattg ggaagattag agatatgggg tacacgaaac ttgtgtacct 97200
   ggtgcaaaga aatgcttggt gcaaaacagc tatgcaatta ataggtgtag gatgattgtt 97260
   cttgttaatt ttcttgtgtc tttcaattct gcgttaacca tgtttcatat tgaaaactat 97320
   atcacagcaa aatgaaacaa aatgcacaaa ttgccagggg atagtcagaa tgctcaaact 97380
   ttttgtaaat attgagattt tgtaagattt atgtaataaa ataagagtta ttccaatcag 97440
   aacttttttg caggttatag gggtgactat tagtagttat gctgggacaa caggtataaa 97500
   ttgggactat cccaagcaaa ctcagaatat gatcacccta taaatagctt cacttttcca 97560
   gttatataat actttatttt cctaaaaata tttttattaa ataaaagtta attccttgcc 97620
   atggtcccac aggcatcagt gaatcccaat tttcaatggt ctttgtatca aaccatgttc 97680
   ttgagttttt agcagcctgt cattgggtag ctctggtctg tctttcctga aacatagtaa 97740
   cctaaccagc aagtagaact tgagagagaa ctgggcactg gggactattt aagctaggtt 97800
   aagtgttttt tttttttttt ttttttttga gatggagtct cgctctgtca cccaggctgg 97860
   agtgcagtgg tgcgatctcc gctcactgca agctctgcct cccgggttca tgccattctt 97920
   ctgcctcagc ctcccgagta gctggaacta caggcgcccg ccaccacgcc cggctaattt 97980
   ttttgtattt ttagtagaga tggggtttca ccgtgttagc caggatggtc tcgatctcct 98040
   gacctcgtga tccgcccacc tctgcctcct taagtgctgg gattacaggt gtgagccacc 98100
   gcgcccggcc aggttaagtg tgtttttgat attctctgtt tccttttctg atcccatttg 98160
   tagaaacaat atactctact cggagtgagg taatattttg ttttctttcc aaggcccaca 98220
   ccaatgatgc ctagcacttt ttgttcactc aaggattcaa aaatatttat tgagcattta 98280
   ctatgtttca ggaactgtaa taggtataca ttagggaaaa aaacagagaa tatcctacct 98340
   tcatggtgtt cacttttttt ttgattgggg gaataccgag tgctcaattt tatagtttct 98400
   catagtttct tcatggccac agataaaccc ctgaaaccaa tcttttcata aataagtcag 98460
   attaatttct ggggcttctt gatacttttt tgcctatttt aataaattac agcattattc 98520
   cataaccctg tagttctcac tattgggtca ttaacacagt cattaaatgc aagcaggtct 98580
   agtattgact agcccttctg gccaataaca cccaattcaa ggaagtgact ttccactact 98640
   ctcgcctttg ctgacatgct ttcacttaag agaggcattt gtctggggac ccagggtcct 98700
   ggtgatgtgt gtgtttttca gtagcacccc actctatgaa ttcggaaggt ctagctctac 98760
   cccgggattc acagtgtgct gtggcctctg cctcagcttt cttccttcac tttctgcctc 98820
   ctgtctcctg gcatatgtgc tttgaacact tgatggaata tctggccaga agtcatttag 98880
   tatgttttgc taagtgttgt cgtcacttga gattctgatc tataagagac tgacaaacct 98940
   tatggatgtt tttctttcag aggaaaacaa acccacaagg cctgtgattg tgagcccagc 99000
   taatgagaca atggaagtag acttgggtaa gtgggcttca gtgagggtat gctggaatcg 99060
   gttttttttt tttaaaacat aagagtaaga taaattgtat ctttactata tataatctaa 99120
   gccatttact gtataaatct atcaatggag ggaaagtgag acaaattata tcttaatttt 99180
   ttgtatttct tgcctctatg gcaaacatct tgccatgtct gatatctcca gccctacttt 99240
   tctgtgaaca cttattacca tgaaaacacc aaccagacac caaggtcaaa aggcctagga 99300
   accagcaatg ccattgactg gtacaggatt ggatttcttt cttttttttt ttttcacaga 99360
   gttaaaatat ttaatgacaa aaatagggtt tgctgtaaaa gtgaatcaat acagtaggtg 99420
   ttacttatct ctgaattaaa caaaaattat atttgacatc ttaaagaact tctgaagaat 99480
   tgatcattgt acgacagaca tcagcagtgt tacaatttca ggactggatt tctcatgacc 99540
   agatccaagc tttgagtcgg aagagtaagg ctacaggccc caaagatggt agtcattcta 99600
   gaaaagtatt tttccaagtt agtgaacatt ttttattcaa gagtaaagaa agaaccccac 99660
   aagcatctag ctctgcactg tttagtgcag tagttactag gcgcatggag ttatggacat 99720
   gtacaattaa attaagtcaa ttgattcatt tagttaaatt aaaaattagt tcctctgtca 99780
   cattagccat atttcaaggg cttaatagcc acatgtgaca agtgactact gtacagacaa 99840
   gcacaaatat agaacatttc caacatcact gaaagttcta ttggaaacca ctgatcaaga 99900
   gagaaaaagt ggttctttgt gtcagaggac ccaaaatagg ttggccttgg aattgtcctc 99960
   tgtaatagta actgcatgac agcagtcaag aaccatacag aggttagaag gtatatgctt 100020
   gtgatctgtg aattctgtac ttggccaatt ctgtacttgt tatttatggt ggaaggtggt 100080
   agaaagacat tcttagacag aaacaaaaat gtcaaaactc aaaattaaat atgggccaat 100140
   aggctctaga acaatgtttc tcaaccatct ttttttgttt taaattgttc ccctttagag 100200
   tctaaaaagt cttcttccac ctaattctct ttcctgtctt taaatttcag taccacagtt 100260
   atcttttata tttgctgata tactgtggcc ctttggagta ccacaaacca tgtaatatct 100320
   aaggttttct tgcctcttcc ccaagaacca gtttttgccc tgctggccac agcccctgtt 100380
   gaaaatgcct gccctagaag tgtgtaattg agagctctga tgcctaggct gcattgatct 100440
   ctcaatctac aaaaaaaagc ttcaaattag gaaataatta ttttaatgta ttatattgac 100500
   agtggatcag aagcatatag taccctgtca taggattatc ctgaagggtg ttaaacaggc 100560
   aataagatcc aataccatta ctcctaaaat gacttagaaa aatagggatt gagagatatt 100620
   tccttaactt aataaataac atcagtgcaa gccaatagtc actaatattc ttcatggtga 100680
   aatactatag accttctcac taaagccaag agcaagacaa agatgcccat gtaaccacta 100740
   ttactgagca cagtctagaa gtgcctacta atgcaattga aggaaaaaaa caaggtatgc 100800
   ttaccacaag ggaaaaaaga catttttatt attgtttcct gatcatacca atcccaagaa 100860
   aattcccggg aaaaactaga attaattctg tataaattag tcagataata aattaacatt 100920
   tagaattaat gttctaaaat ataagacttg tttagcaata accagctaga aaacataata 100980
   ggaaacaaat gccatttgca ccagtaaatg tcttagaagt gtccataaaa tatgttagaa 101040
   aaactttgaa aatatgtgga atttataaga gaggaaaaaa ctaaaaaaac caaaaccctg 101100
   aatattaatc tcagaaaaac tgaaatttaa agcatgcatc cagccaggcg tggtggctca 101160
   agcctgtaat tccagcactt tggtaggcca aggtggacag atcacttgag gccaggtgtt 101220
   tgagaccagc ctggccaaga tgttgaaaca ccatctctac taaaaataca aaaattagct 101280
   gggtgtggtg gtgcacacct gtagttccag ctgctcaaga ggctgaggca tgagagtcac 101340
   ttgaacccag gaggagaagg ttacagtgag ccgagattgt gccactgcac ttccagcctg 101400
   agtgacagag cgagactgtg tctcattttt aaaaatggaa ttaaataatg gatagaggga 101460
   taacagtttt taaaaaggaa ggccgatgag caccaagcta atgaaaagca tgttgattca 101520
   ccgtgaattc aagaaatgta gatgaaaact taagtgacat tttcttctct caaaaggcaa 101580
   tgtctaacca aaaacacttg ttatctggtg gttttgggga aaatcgtgct cacatacact 101640
   attggagaga gggtagactg acacagcctc actaaagaga gggggttagc ttttaaaata 101700
   tgtttcttta attcaatctt gcttctagat tttaatactt caggatgtgt acaaataagg 101760
   ctattcatta aaatattaat cataatagtg aaaaattagg aaattgtgtt catcaatagg 101820
   aaatagttaa tcatagtgta tatgtattat ggaatgctaa gcagccatga cagagtctat 101880
   tttggaagac aaatcatgtg ggcccaaatt cccaatccac agtaattaca aaatagtagg 101940
   caaaaagacc ccatttcaaa aactgcacat gttcacagag aagataagga atgagacgca 102000
   taaggttact aatggtggtt ttctctgggt ggtgggttta gggtaatttt cctttttctt 102060
   tgtgcatttc tatatctaaa attttaattc aatagccatt tattaatcta gatagatcag 102120
   aaaaaatcaa ttaatgttaa gattaaaaat acattctttg caggatccca gatacaattg 102180
   atctgtaatg tcaccggcca gttgagtgac attgcttact ggaagtggaa tgggtcagta 102240
   attgatgaag atgacccagt gctaggggaa gactattaca ggtatgtatg ctaagagtta 102300
   ttcacatttt ggtgttaaat cccacgtgat attttatata accttgtcgg ttcagtttaa 102360
   aagcaagtgt tagttgctcc taacctttgc tgcctttttt tttttttttt tttttttttg 102420
   ctaagctaag tagaatttat aagatcttgt acatttaagg taaaaaatac ttgtcattgt 102480
   ggtgcaaatt acagaccact tagaagcctt tctcttacgt tatacatgtt gcaacatttc 102540
   aacgaagcaa ttatctttca ttattcttca tattcctcaa gtagcttaga agagactcat 102600
   taatgagctc aagaatcagg caatccactg ctctcgaaag cctctactgg tttcctactg 102660
   ctcatctatg ggacaaggat ctcctggctt cccatgacct ctccttcaca acacctgcaa 102720
   agcactttgt catctgcccc aatctcctct tgtttagtgt ctcctctgtg ccccatggag 102780
   aacacaatgc atttgtctac atttttcttc ttggaagtgg taattttttt gctttctctg 102840
   ctactgagcc tgttactgac agtggtcttg gggttataaa atctcagatt agtgttgcca 102900
   gcagtctgaa tgttgtgact gtgattgggc agggtgatta tttcagtggc aattttgtca 102960
   caaataagga aatacacagg gtatacaggt cttatttgta gttgatgcaa ttaacttaca 103020
   caagtttatt tactctctct ctcgaatagt gtggaaaatc ctgcaaacaa aagaaggagt 103080
   accctcatca cagtgcttaa tatatcggaa attgaaagta gattttataa acatccattt 103140
   acctgttttg ccaagaatac acatggtata gatgcagcat atatccagtt aatatatcca 103200
   ggtaaacaac aaactaattg aaatgcagtt ttgttttact gtagtaagat tccatgactt 103260
   tgaagtttga aatgccattt cctctgctta gaacacgctt cacttcctca ttacttgggt 103320
   aacttctatt tctcttttac tcttctgctg aactctctct tcttggaagc ttttctgtcc 103380
   tgctgcctgc ccttaaccca gctctcggcc caaagagagg aggttagggc ctctgtatgt 103440
   acttctgtag caccatatgt cacaggatct atggtacttc gttttctgta cttaactata 103500
   aattctgtga tggtggcaac catttatctt agtaatcatt ctatttccac agctaatggc 103560
   aagttaggtc tggtacattg tgggtaccca atgaactaca ttgatgatgc atagggtaag 103620
   actaaatatc cactttcaaa gaggacatca gaagtcactg ggtttcatga attggtataa 103680
   taaaacataa tattgaaagt atttgtgaaa cattttaagg taatttgtgg tttaattaaa 103740
   cataggctat tataattgcg tgcatggaag aatatctgaa agactctata aattaacttc 103800
   tatgtatgtt taaatactgg cagcatacaa atagaaatta catttaaaag ataacattta 103860
   tggtagcatc aaaaatcatc aaatacttaa gaataaatct agcaaaagct gttcaaaacc 103920
   tctgtaatac aaactaaaaa atattgagat aaattagaga agaactaaat ggaaggatat 103980
   acaatgttca tagattgaaa gactcgatat tatgaagata tcagttcttc cccaaatgaa 104040
   tctatggatt ccaaaaaatt tcaatcaaaa tctcagcagt atttgtacaa gtgagagtca 104100
   gtgtttgtat gggtgtgttt ttgtggacac ctgtaaaagt catagggaac tgtaaaggac 104160
   caagtatagt catggtggtc ttaaataaga aaaataaatc tggaggatta ttactacaga 104220
   catcatgatc ttgtatgaag cgacagtact taagaccgta agtgtgaact atcatttggg 104280
   ccagctggat atttgtctgg aaaatggtat aacttgattt ctgttttata tccaaatcat 104340
   ttaaattgga aaagtaaata aagcttttag attccctttt ctggatgctt ttacatggtt 104400
   cctgatattt agagggtact ttctttttct tccctctttc ttcttctcta gccccctccc 104460
   catcacctac tccatctgct ccccagcttc ctctttaatt attggataac taagtagaga 104520
   aggaggccag gagaatgtgg aaatgtgaaa cttccctaag cctccacctt attcgctttc 104580
   caccagtctt taactttacc caatggactc tctgacctca gggaactttt gtaggctttt 104640
   gagtgatttt aacttttccc tgttttactg tggaatgctc tatcctgtgg tcttcggtat 104700
   tatttcccat gttattgtat attatgctat ttaggtagaa acttcataga atgcacctta 104760
   gttttatcat tgctttgtac attagtatcc attacaaagt gataataatg aatgagttaa 104820
   tattgtctga atgcaggcca ttcccagata tgggctctct gagacgaaga tatttaattt 104880
   gctgtgctca aagttttaat tctttttggt tctaatattt tttctccttt ttttttcttt 104940
   ttgctatagt cactaatttc cagaagcaca tgattggtat atgtgtcacg ttgacagtca 105000
   taattgtgtg ttctgttttc atctataaaa tcttcaagat tgacattgtg ctttggtaca 105060
   gggattcctg ctatgatttt ctcccaataa aaggtataat tttgtattcc atgcagtatt 105120
   tcttgttgga gataagggat agttaggaga tgtagaagat gactaagagg gtttttacta 105180
   agagggtttc taaaatgaaa agtggcatat acaagaatac tagttattct tacacagaat 105240
   tatagtttta gaactcatta ggctgaatat cattgttttc taagttcctg cctcaacatt 105300
   catattggta gaataaacag gtatttagag catctgttta taaattcagt tatgcacaag 105360
   ccattggtag gccttgctct gtgatgaata ggacagacag agatcatacc ttaatgaaag 105420
   aaatgaaatg actagatctt catgcatcag ggaggttctc tctctccttt gtttaaactt 105480
   tgcgaaaaaa aaaaaaggaa taccacaata tctaaaaaaa aatgaatgga tagaattcag 105540
   ggctgagacc tctggggaag aagatgagga cagggagact ttaaggatgg ctctatacta 105600
   agagtatacg aggcagcctc caaatagaag aacaaatttg cagtaaaaca caagaaatag 105660
   aaaccttgta agagatgaac actttttaaa gggattatat ttttggtatt gctgcttcag 105720
   tcatgccatt gtataaaaaa agatgaatag ttcattatgt aatgaatgtt tgtgatactg 105780
   actttatcaa atctcttatt tttgccttgt ggttctaaat acattatgtt tttcctttag 105840
   cttcagatgg aaagacctat gacgcatata tactgtatcc aaagactgtt ggggaagggt 105900
   ctacctctga ctgtgatatt tttgtgttta aagtcttgcc tgaggtcttg gaaaaacagt 105960
   gtggatataa gctgttcatt tatggaaggg atgactacgt tggggaaggt atgtgtgtaa 106020
   tggaacagag taaaggctta ttgttgtaaa actacttagt aaaatgtgga ttccatcttt 106080
   ctagaagatc gtggcatagg ggtatatgtt tcacaatttt aagaacctct tcagaagtgt 106140
   atgatgattt ttacatttat taaatgcatt tacttctctc atattttgtc agagcaataa 106200
   ttaagtgatt ttcatttagt tttgaaaaac ttaatgacta gttaaaccca acagttgctt 106260
   tacaactgat acaattaaac aggagtggta gagatcaaat gattgaataa taagttttta 106320
   aaattccaac aaagttttct gagttctaaa tgaaatattt gtcttttgag ggccaatttt 106380
   tgagttagtc tataaaatgg gaggtttgca gtggctcatg ccttttatct cagtacaacc 106440
   ctgggaggcc agggtgagag ggtcacttga gcctaggagt ttgaggctgc agtgatccaa 106500
   gatcacgcca ctgcgctcca gtgtgagcaa cagaacaaga gactgtctct taaaattaat 106560
   taattaaaaa catgggaaac tcctttaatt ttaagtaaga caagaaaaag gcattaggag 106620
   ccatacggtt gtgaaaagcc ttgtgtggct ttggttcagg agagaatgat gataaataga 106680
   attttactta ctatattgtg cttcctgttt ttcagacatt gttgaggtca ttaatgaaaa 106740
   cgtaaagaaa agcagaagac tgattatcat tttagtcaga gaaacatcag gcttcagctg 106800
   gctgggtggt tcatctgaag agcaaatagc catgtataat gctcttgttc aggatggaat 106860
   taaagttgtc ctgcttgagc tggagaaaat ccaagactat gagaaaatgc cagaatcgat 106920
   taaattcatt aagcagaaac atggggctat ccgctggtca ggggacttta cacagggacc 106980
   acagtctgca aagacaaggt tctggaagaa tgtcaggtac cacatgccag tccagcgacg 107040
   gtcaccttca tctaaacacc agttactgtc accagccact aaggagaaac tgcaaagaga 107100
   ggctcacgtg cctctcgggt agcatggaga agttgccaag agttctttag gtgcctcctg 107160
   tcttatggcg ttgcaggcca ggttatgcct catgctgact tgcagagttc atggaatgta 107220
   actatatcat cctttatccc tgaggtcacc tggaatcaga ttattaaggg aataagccat 107280
   gacgtcaata gcagcccagg gcacttcaga gtagagggct tgggaagatc ttttaaaaag 107340
   gcagtaggcc cggtgtggtg gctcacgcct ataatcccag cactttggga ggctgaagtg 107400
   ggtggatcac cagaggtcag gagttcgaga ccagcccagc caacatggca aaaccccatc 107460
   tctactaaaa atacaaaaat gagctaggca tggtggcaca cgcctgtaat cccagctaca 107520
   cctgaggctg aggcaggaga attgcttgaa ccggggagac ggaggttgca gtgagccgag 107580
   tttgggccac tgcactctag cctggcaaca gagcaagact ccgtctcaaa aaaagggcaa 107640
   taaatgccct ctctgaatgt ttgaactgcc aagaaaaggc atggagacag cgaactagaa 107700
   gaaagggcaa gaaggaaata gccaccgtct acagatggct tagttaagtc atccacagcc 107760
   caagggcggg gctatgcctt gtctggggac cctgtagagt cactgaccct ggagcggctc 107820
   tcctgagagg tgctgcaggc aaagtgagac tgacacctca ctgaggaagg gagacatatt 107880
   cttggagaac tttccatctg cttgtatttt ccatacacat ccccagccag aagttagtgt 107940
   ccgaagaccg aattttattt tacagagctt gaaaactcac ttcaatgaac aaagggattc 108000
   tccaggattc caaagttttg aagtcatctt agctttccac aggagggaga gaacttaaaa 108060
   aagcaacagt agcagggaat tgatccactt cttaatgctt tcctccctgg catgaccatc 108120
   ctgtcctttg ttattatcct gcattttacg tctttggagg aacagctccc tagtggcttc 108180
   ctccgtctgc aatgtccctt gcacagccca cacatgaacc atccttccca tgatgccgct 108240
   cttctgtcat cccgctcctg ctgaaacacc tcccaggggc tccacctgtt caggagctga 108300
   agcccatgct ttcccaccag catgtcactc ccagaccacc tccctgccct gtcctccagc 108360
   ttcccctcgc tgtcctgctg tgtgaattcc caggttggcc tggtggccat gtcgcctgcc 108420
   cccagcactc ctctgtctct gctcttgcct gcacccttcc tcctcctttg cctaggaggc 108480
   cttctcgcat tttctctagc tgatcagaat tttaccaaaa ttcagaacat cctccaattc 108540
   cacagtctct gggagacttt ccctaagagg cgacttcctc tccagccttc tctctctggt 108600
   caggcccact gcagagatgg tggtgagcac atctgggagg ctggtctccc tccagctgga 108660
   attgctgctc tctgagggag aggctgtggt ggctgtctct gtccctcact gccttccagg 108720
   agcaatttgc acatgtaaca tagatttatg taatgcttta tgtttaaaaa cattccccaa 108780
   ttatcttatt taatttttgc aattattcta attttatata tagagaaagt gacctatttt 108840
   ttaaaaaaat cacactctaa gttctattga acctaggact tgagcctcca tttctggctt 108900
   ctagtctggt gttctgagta cttgatttca ggtcaataac ggtcccccct cactccacac 108960
   tggcacgttt gtgagaagaa atgacatttt gctaggaagt gaccgagtct aggaatgctt 109020
   ttattcaaga caccaaattc caaacttcta aatgttggaa ttttcaaaaa ttgtgtttag 109080
   attttatgaa aaactcttct actttcatct attctttccc tagaggcaaa catttcttaa 109140
   aatgtttcat tttcattaaa aatgaaagcc aaatttatat gccaccgatt gcaggacaca 109200
   agcacagttt taagagttgt atgaacatgg agaggacttt tggtttttat atttctcgta 109260
   tttaatatgg gtgaacacca acttttattt ggaataataa ttttcctcct aaacaaaaac 109320
   acattgagtt taagtctctg actcttgcct ttccacctgc tttctcctgg gcccgctttg 109380
   cctgcttgaa ggaacagtgc tgttctggag ctgctgttcc aacagacagg gcctagcttt 109440
   catttgacac acagactaca gccagaagcc catggagcag ggatgtcacg tcttgaaaag 109500
   cctattagat gttttacaaa tttaattttg cagattattt tagtctgtca tccagaaaat 109560
   gtgtcagcat gcatagtgct aagaaagcaa gccaatttgg aaacttaggt tagtgacaaa 109620
   attggccaga gagtgggggt gatgatgacc aagaattaca agtagaatgg cagctggaat 109680
   ttaaggaggg acaagaatca atggataagc gtgggtggag gaagatccaa acagaaaagt 109740
   gcaaagttat tccccatctt ccaagggttg aattctggag gaagaagaca cattcctagt 109800
   tccccgtgaa cttcctttga cttattgtcc ccactaaaac aaaacaaaaa acttttaatg 109860
   ccttccacat taattagatt ttcttgcagt ttttttatgg cattttttta aagatgccct 109920
   aagtgttgaa gaagagtttg caaatgcaac aaaatattta attaccggtt gttaaaactg 109980
   gtttagcaca atttatattt tccctctctt gcctttctta tttgcaataa aaggtattga 110040
   gccatttttt aaatgacatt tttgataaat tatgtttgta ctagttgatg aaggagtttt 110100
   ttttaacctg tttatataat tttgcagcag aagccaaatt ttttgtatat taaagcacca 110160
   aattcatgta cagcatgcat cacggatcaa tagactgtac ttattttcca ataaaatttt 110220
   caaactttgt actgtta 110237
<210> 35
   <211> 15775
   <212> DNA
   <213> Homo sapiens
<400> 35
   caagcttagc ctggccggga aacgggaggc gtggaggccg ggagcagccc ccggggtcat 60
   cgccctgcca ccgccgcccg attgctttag cttggaaatt ccggagctga agcggccagc 120
   gagggaggat gaccctctcg gcccgggcac cctgtcagtc cggaaataac tgcagcattt 180
   gttccggagg ggaaggcgcg aggtttccgg gaaagcagca ccgccccttg gcccccaggt 240
   ggctagcgct ataaaggatc acgcgcccca gtcgacgctg agctcctctg ctactcagag 300
   ttgcaacctc agcctcgcta tggctcccag cagcccccgg cccgcgctgc ccgcactcct 360
   ggtcctgctc ggggctctgt tcccaggtga gtcggggtgg ggattgccgt cgggccagtt 420
   ctccgaagcc ccgggaggac cggctcccgg gtcaggtcat gcatgcttag gtagctgttt 480
   atgggaagga ggggctagag acagcgattg aaaggcaaca gccagtaggt tcgaatccag 540
   accctgcata cctccacgtg tggccttggg ctatagattg cagctttaaa aaagggtagg 600
   gggttggaga tggaggggag gggcgggcct cgttttgttg cccaggccgg tcttgaactc 660
   cgggggtcta gccttacctc ctgcctcagc ctcccgagta gctgggatga gaggtgtgaa 720
   ccaccgcctt gcttggctag attgcgtctc ttacagtttc tcagctgtaa aacgggaaac 780
   gttatagcgg ccacctggca gggtatcttg gcccagcgca gcacctggcc ccaggactcg 840
   atcatgatgg tttgggaact tggctctgtg ccaacccaac aaggcttaag ggacccccac 900
   ccccctcaag atgtatattc tgttcctcat cctctctgcc cctggggaag tccagggctg 960
   cttctacttg ggggaattcc agagctgact tatccgtggc ccaaagctga gaagtgggac 1020
   gccccagcac accctccccc agctccagcc cagctaggga agagggaagg ggtcagaggg 1080
   tctttcatgg tggtgtaagt ttggggaacc aggagggtgg gagattgaca gcttggttaa 1140
   cagctcaaca aagcctgaga tccaggccag cacggtagtt catgccagta atcccaacac 1200
   tttaggagcc ccaggcgggc gaatcactta aggtcaggag tttgagacca gcctggccaa 1260
   catggcaaca tcccgtctct actaaaaata caaaaattag ctggcatggt ggtgggcgcc 1320
   tgtgatccca gctgctcggg aggctgaggg aggaaaatcc cttaagccca cgaggctgag 1380
   gttgcagtga accaagattg tgccactgca ctccagcctg ggagacatag cgagattctg 1440
   tctcaaaaaa caaagcgttc tgatccggac tcagacccag atcgcactgc tttctagctg 1500
   agtaaccatt tctctctatg aaatgggaat ggtcccagaa tctcccttgg agaatgtatg 1560
   gagccagtgt cctcacaccc ccatccaaga tagaacaaat ctgagacagg aatctttgag 1620
   tgaggcagtg ctgggctcag acattttttc ccaccttcgg aggcagcaga atctgaggga 1680
   cctgatccaa ataagcccct tctttctttc ttttcttttc tttttttttt tttttttttt 1740
   ttgagacgga gtctcactct gtcgcccagg ctggagtgca gtggcgtgat ctcggctcac 1800
   tgcaacctct gcctcccagg ttcaaacgat tctcctgcct cagcctccct gagtagctgg 1860
   gactacaggc atgtgccatc acacccggct aatcactgtg ttagccagga tggtctcgat 1920
   ttcctgacct catgatctgc ccaccctgcc tcccaaagtg ctgggattac atgcgtgagc 1980
   cacagtgccc accccgtaag ccccttcttt cttacctgca aggtagccag ttgctaccca 2040
   tcctgtgctg agttacttgt attagcaagg gatggggtgg ctatactcac ccaccttaca 2100
   gatggggaaa ttgaggccca aagaggggga aactacgtgt ctcagggagt gaggagccag 2160
   tctgattcct ggagggctga ctgtctccac ctgacttctt aggagggagg agggcaccaa 2220
   cttcacatta aaatctggtt ggacacagtg gctcacacct gtaatcctgg cattttggga 2280
   ggcttaggcg ggaggatcac ttgaggccag gagtttgaga ccagccttag caacatagtg 2340
   agaccccatc tctacaaaaa tgtttttcag ggccaggcgc ggtggctcac acctataatc 2400
   ccagcacttt gggaggctga ggcgggcgga ttacctgagg tcaggagttt gagaccagcc 2460
   tgaccaacat ggagaaaccc cgtctctact aaaagtacaa aattacccgg gcgtggtggc 2520
   gcatgcctgt aatcccagct actcgggagg ctgaggcagg agaatcgctt gaacctggga 2580
   ggcggaggtt gcggtgaact gagatcgtgc cattgcactc cagcctgggc aacaagagct 2640
   aaactccgtc tcaaaaaaaa aaaaatgttt ttcaaatatt agccgggtat ggtggtgtcc 2700
   tgtagtccca gctacttggg aggctgagat gggaggatca cttgagccca ggagttcaag 2760
   gttacagtga gctatgattg tgccactgta ttccagcctg ggtaacagag ggagacccgt 2820
   ttaaaaaaaa aaaagtgatg gctaaagtcc ttccatggct ccctattgcc ctcagtataa 2880
   agaacacatg tggctgggcg tggtggttca cgcctgtaat cccagcactt tgggaggctg 2940
   aggcgggcgg atcacttgag gccaggagtt tgagagcagg ctggccgacg tggcgaaacc 3000
   ccgtctctat taaaaataca aaaattagct gggcgtggtg gtgcttgcct gtaatcccag 3060
   atactctgga ggctgaggca ggagaatcac ttgaacccgg gaggcaaagg ttgcagtgag 3120
   ctgagattgc gccactgcac tccagtctga gtgacaaagc gagactccat ctcaaaaaaa 3180
   aaaaaaataa aagaacacat ctttagcatg gccttcagtg ctcacgggat cttcctgaat 3240
   taatctcccc ctcttcatcc ttgctcactc agctccagcc accctgcccc gggacatctg 3300
   tacttgcctg gaacttattt cccttttctc cggacagcca gccctttctc gtcatttaga 3360
   tctctgctga aacattaccc tgtcaccaaa gcactgtcta ttctatcacc ctgttttgtt 3420
   tttgtcaaag ctcatattaa catcagttat taattatctt gtttgctcat aatttttttt 3480
   tttttttttt tggagacaga gtctcgttct gttgctcagg ctggagtgca gtggcacaat 3540
   cttggctcac tgtaacctcc acctcccagg ttcaagtgat tcttgtgcct cagcctccca 3600
   aatagctagg actacaggca cgtcccacca tgcccagcta atttttgtat ttttagtgga 3660
   gacggggctt tgtcatgttg gccaggctga tctcaaattc ctgacctcaa gtgatctgcc 3720
   cgccttggcc tcccaaagtg ctgggattac aggcgtgagc caccacaccc ggcctgctca 3780
   tgaattttct ctttaacttc cacatcgaag gcaaagtatt gtcttgttaa ggctgtgcct 3840
   ccagcaccca gcacaggctg ggcgcacatt cccttgatga acctgatttg taatgcctgt 3900
   cgcctcttcc ctcgtttctt ctaggacctg gcaatgccca gacatctgtg tccccctcaa 3960
   aagtcatcct gccccgggga ggctccgtgc tggtgacatg cagcacctcc tgtgaccagc 4020
   ccaagttgtt gggcatagag accccgttgc ctaaaaagga gttgctcctg cctgggaaca 4080
   accggaaggt gtatgaactg agcaatgtgc aagaagatag ccaaccaatg tgctattcaa 4140
   actgccctga tgggcagtca acagctaaaa ccttcctcac cgtgtactgt gagtaactga 4200
   gcccggaggg ctggactagg cagacccggt gggagagacg tgcaggggca cctgcagagg 4260
   cctgggggaa tctttgccac ttgctcgtag ggtcaaggag gggctccttg cagggcaggt 4320
   ggggacatcc ttggaaagtc cctttgtgaa tttctttggg tacaattaaa gtatttacag 4380
   gctgggtgcg gtggctcatg cctgtaatcc cagcactttg agaggctgag gctggcggat 4440
   cacctgagat caggagttta agtttcgcca acatggcgaa accctgtctc tgctgaaaat 4500
   acaaaaatca gccgggcatg gtgtcaagcg cctgtaatcc cagctacttg gaaggctgag 4560
   gcaggagaac gcttgaacct gagaggcaga gattgcagtg agccgcgatc gtgccagtgc 4620
   actccagtct ggataacaga gcaagattcc atctcaagaa aaaaaaaatg ccatctctct 4680
   atgcctcact ctttgaacat atgacacggt cctgcttcag acactttaat aaaagatgca 4740
   aattaagcca agtgtggtgg cttgtaccta taatcccaac tactccagag gctgaggcag 4800
   aaggatggtt tgagcccagg agtttgagac cagcctgggc aacagagtga gaccctgttt 4860
   ctttcttttt tttttttttt tttttgagac ggagtctcac tctgtcgccc aggctggagt 4920
   gcagtggtgt gatctcggct cactgcaagc tccgcctccc gggttcacgc cattctcctg 4980
   cctcagcctc ccgagtagct gggactacag gcgcctgcca ccatgcccgg ctcatttttt 5040
   tgtattttta gtagagacgg ggtttcactg tgttagccag gatggtctca atctcctgac 5100
   ctagtggtcc gcccgcctcg gcctcccaaa gtgctgggat tacaggtgta agccactgtg 5160
   cccatccaag accctgtttc taccggaaaa aaaaagtaaa taatttagct gggcatcgtg 5220
   gtgtgcacct gtaatcccag ctgctcctga ggctgtgatg ggaggattgc tttaacccag 5280
   gggttcgaat cctaggagtt cgaatccatc ctaggcaaca tagcaaaacc ccatttttat 5340
   ttaaaaaaaa aaaaaaagat atgagttaaa actagccctg ggatggcatt tttcacatat 5400
   tggtaacaaa caaaagaatt gatggccggg cgcagtggct cacgcctgta atcccagcac 5460
   tttgggaggc cgaggcgggc ggatcacaag gtcaggagat cgagaccatc ctggctaaca 5520
   tggtgaaacc ccgtctctac taaaactaca aaaaattagc cgggcatggt ggcaggcgcc 5580
   tgtggtccca gctactcagg aggctgaggc aggagaatgg catgaacccg ggaggcagag 5640
   cttgcagtga gccaagatcg tgccactgca ctccagcctg ggcgacagag caagactcca 5700
   tctcaaaaaa aaaaaaaaaa aaaagaattg ataacagctg tgctgccaag gctattggaa 5760
   cgtaggaggt cctaggacag tgctgttggg agcataaata agcccaaccc tgtggcggga 5820
   aattgggcat cagttctcaa aatgtcatgg gctgggcacg gtggctcacg cctgtaatcc 5880
   cagcactttg ggaggctgag ggaggcggat cacttgaggt caggagttcg agaccagcct 5940
   gaccaccatg gagaaacccc gtctctatta aaaatacaaa aaaaactagc caggcatagt 6000
   ggcacatacc tgtaatccca gctactcggg aggctgaggc aggagaatct cttgaaactg 6060
   ggaggcagag gttgcggtga gctgagattg cgccactgca ctccagcttg ggcaacaaga 6120
   gcaaaactcc atctcaaaaa aaaaaaaaaa agaaataaaa gaaggtatgt tgaatatgag 6180
   tggtatgcca ccctcacatt agggaagggc agtttcgggg aggctgtatt tatgtataaa 6240
   atagccctaa aaggaagtgg gagaaatgac aatattagct ggctatgaga agagaggctg 6300
   ggaggctgtg ggagagggct tgggtgtgga gaattctttt tgttttttcc tttttttgag 6360
   acagagtttc actcttgttg cccaggctgg agtgcaatgg cacgatctca gctcaccgca 6420
   accttcacct cctgagttta agtgattctc cggcctcagc ctcccgacta gctgggatta 6480
   caggcatggg ccactacgcc tggcaaattt tgtattttta gtagagacag ggtttctcca 6540
   tgttggtcag gctggtcttg aactctgacc tcaggtgatc cgcccgcctc ggcctcccaa 6600
   agtgctggga ttacaacgtg agccactttg cctggctgag aattcttttt ttgttgttgt 6660
   ctttttgaga tggagttttg ctgtgtcccc agcctggagt gcaatggtgt aatctcagct 6720
   cactgcaacc tctccctccc gggttcaagc aattctcctg cctcagcctc ccaagtagct 6780
   ggaattacag gcgcccagca ccacgcccgg ctaatttttg tatttttagt agagacggga 6840
   tttcatcatg ttggccaggc tggtcttgat ctcctgacct tgtgatctgc ccgcctcggc 6900
   ctcccaaagt gctgggatta caggcgtgag ccactgcccc cagccgagaa tttctctttg 6960
   cgtccttcct actttgggga cttcgaatgg tgggaaagag ttatcaaggc caaaataagg 7020
   aattcaaatg aaaacaaaac aaaatcaaag aagaaaaaac agaagagcac tgggcaggct 7080
   aggcacgtgg ctcatgccta taatcccagt gatttagaag gccgaagtag aaggatcgct 7140
   tggaggccag gagttggaca ccagcctggg caacatagca agaccccata tctacaaaaa 7200
   ataaaaaacc taaccaagcg tgctggcata ctagtagtcc cagctactca ggaggctgag 7260
   gtgggaggat cacctgagcc tgggaggtcc aggctgtagt gagccgtgat gacaccactg 7320
   cactccagcc tgggtgacag agaaagaccc tgtctctaaa aaataaaaac tggccaagta 7380
   gctttgggat tagccttggg ttccagtccc agcaaggcct ttaatagctt gggacatgac 7440
   ttctgcattt actttgcaat caggtgagac ctcctctgat ggggaaaatg acacggtgag 7500
   tgacaaagga tgttctccta tcattgtgtc agggcaagga agcctctggg taaatgatca 7560
   aatgatcagc tttgcttctg atttggaggg tgggtgagca gatgctgacc ttcccaggtg 7620
   agggaagtcc ccgaacattc ccagcagctt ctggaaaccc cagggaaacc tctttgaagg 7680
   tcttttctgc atctctgcct gataggtctt tttttttttt tttttttttt tctttttttt 7740
   gacacacagt cttgctctgt cgcccaggct ggatcactgc aacctccatc tcccgggttc 7800
   aagcagttct cctgcctcat cctctccagt agctgggatt acaggcacct ggcaccacgc 7860
   ctggctaatt tttgtatttt tagtagagac agggtttcgc caagttggcc aggctggtct 7920
   cgaactctag acctctggtg atccacccgc ctcagcctcc cgaagtgctg ggattacagg 7980
   cttgagccac cacgcgcggc tctgcctgat agctgagagc atagaactcc aggtttgaga 8040
   cctggctctg ccacatttct ccctctatga ctgtgggtgc cccactttgc cttagttttt 8100
   acctctgtga aatggagcag atggctggca caggtagcaa aggagtaaaa gttatgtggg 8160
   agggtggtac ctgagagaga ctctagcttg gtcttgcccc acccctggtg taaacataaa 8220
   gaagcctccc tggatggctc aatcttctcc aaaaaggtta gaggtgtaat tcctagagga 8280
   ggcgaccact agctgggctt tgaaggatgt gtaggagttc ataaggacag gcattctggg 8340
   caggaggaac agcctgggca aaagttggga gcagggagaa atcttgatgg aggcaggagg 8400
   aggaggaggt aggttggtgt aggccaggtg cagtggctca cacctgtaat cccagtgctt 8460
   tgggaggcca aagcaagagg attgtttgag cccaggagtt cgagaccagc ctgggcaaca 8520
   tagcgagacc ctgtctctaa gaaaaaataa aaaaattagg gtacagtggc atatgcttgt 8580
   attctcaact actctggagg ctcaagtggg aggatcactt gaacccagga atttttttgt 8640
   ttttgttttt gtttttttga gatggagtct cactctgtgg cccaggctgg agtgcagtgg 8700
   tgccatcttg gctcactgca gcctcctcca cctcctgggt tcaaccgatt ctgctgcctg 8760
   agcctcccga gttgctggga ttaaggtgcc caccatcatg cccagctaat ttttttgtat 8820
   ttttagtaga gatagggttt accatgttag ccaggctggt cttgaacgcc tgaccgcaag 8880
   agatcctcct gcctcagttt cccaaagtgc tgggattata ggtgtgagcc actgagcctg 8940
   gtcaagccca ggaatttgag gttacagtca gctatgattg caccactgca ttccagccca 9000
   ggtgacagag agagacactg cccctaaaaa aaaaaaaaaa attgattgat gggaggaagg 9060
   gtgaggttgg cagagccttg aatgccaggt ggaggagctg ggactttcct tcttggggtg 9120
   atagggagtc atggagggtt tctgagcagg ccagggatta gatagctgaa ggctggattt 9180
   actggaagcc aatgagcagt tggctatggt ccttgtccac gcggcccatg ttgtgggcag 9240
   tgaccgtatt caagaaggga aggacagaca agtatttgaa tacttcagtg accaggattt 9300
   ggtaaaggac tgcaggtcag ggtcaagaag aggtgagagc aggacagact tcctccccgc 9360
   tgcaccaggc agctgagctg ggtttcctct aggggctgag gtttgagggt acctcaagtt 9420
   ctgcaagagt ctataggagg tggtaagaga gaagagctgg aggtcagagt tttcttgact 9480
   atatatatat atattttttt gtttttgttt ttaacagctt aacagctttc tgttttattt 9540
   ttagagacag ggtctcaggg tctcactttg tcacccaggc tggagtgcag tggtacaatc 9600
   gtagctgact gcagcctcaa actcccaggc tcaagaaatc ctcctcccac ccttagcctc 9660
   ctgagtagca gggactacag gtgtgagcca gcaggaagcc cagctggttt tttttttttc 9720
   tttggtgttt tttgtttgtt tgtttgagac cggagtttcg ctcttattgc ccaggctgaa 9780
   gtgcaatggc aggatcttag ctcaccacaa cctccgaccc ccaggatcaa gctattctct 9840
   tgcctcagcc acctgagtag ctgggattac aggcatgcga caccacacaa ggctaatttt 9900
   gtatttttag taaagacagg gtttctccat gttggtcagg ctggtctcga actcccaacc 9960
   tcagatgatc cacctgcctc ggcctcccaa agtgctgaga ttacaggcat gagccaccgt 10020
   gcccggcctt tttttttttt tttttttttt tgagacagag tctcactctg tcgcccaggc 10080
   aggagtgcag tggttcgatc tgggctcact gcaagctccg cctcccgggt tcactccatc 10140
   ctcctgcctt agcctcctga gtagctggga ctacaggcgc ccaccaccac gcctggctaa 10200
   ttttttgtat ttttagtaga gacggggttt caccgtgtta gccaggatgg tcttgatctc 10260
   ctgacctcgt gatccgcccg ccttggcctc ccaaagtgct gggattacag gcgtgagcca 10320
   ccatgtctgg cctggccagg ctggtcttga actcctgact tccggtgatc catctgttct 10380
   ggcctcccaa agtgctggga ttacaggcat aagccaccac gccatgccga agcccagctt 10440
   gtttttaatt tttttttttt tttttggaga aatgaggtct tgcaatgttg cccaagctag 10500
   ccttgaactc ctggcctcaa atgaccccgc cttggcatcc caaagtactg ggattacaga 10560
   tgtgagccac catgccccag ccttgctttc ttgagatacg atttagaata ccataagatt 10620
   catccctttt aagcacataa ttcaatgact tctgtacaaa caaccatgac tacaatctaa 10680
   ttttaaaata tttcaatcac tctaaaaaag aaacctcctg cttatgtaca gcgactctgt 10740
   ctacctctta agtgaattct cctaccttta atagccctat tttacagttc aggaaactga 10800
   ggttcagaga gacaaagtca cttacccaca gcaaagaagc aaggctgggt atcaaatgca 10860
   ggaccccccc ggtcctgatg cttttttttt tttttttttt ttcctctgag agagactctc 10920
   actctgtcac tcagtctaga gtacagtggc gcgatctcag ttcactgcaa tctctgcctc 10980
   ctgggctgaa gtaatccttt cctcacaagt aaacctcagc ctctcaagta gctgggacta 11040
   caggcacaca acaccacgcc tggctaattt ttgtattttt aggtagagac ggggtttcac 11100
   tatattggcc aggctggtct tgaactcctg acctcaggtg atccgcctgc ctcggccccc 11160
   caaagtgttg ggattacagg cgtgagccac cacacgcagc ctttttgtta ttagactctg 11220
   tcattactga cttttttttt tttttaatag aaacagggtc tttctttccc aggctaaagt 11280
   acagtggcat gatcacagtt cactatagcc ttaaactcct gggctcaagt gatcctcctg 11340
   cctcagcctc ccaagtagca gggactacag gtgtgcacca ccacacccag ttaaccattc 11400
   attcattcat tcattcattt attttgagat ggagtctcgc tctgtcacct aggctggagt 11460
   gcagtggcac gatctcagct cactgcaacc tccacctccc aggttcaaga gattctcctg 11520
   cctcagcctc ccgagtagct gagactacag gcgtgcacca ccatgccaga ctaatttttg 11580
   tatttttaat agagacgggg tttcactctg ttggtcaggc ttatctcgaa ctcctgactt 11640
   cgtggatcca ccctccttgg cctcccaaag tgctgggatt aaaggcgtga gccaccgcgc 11700
   cctgccaacc tttttttaat ttttcttaga gatgggggtc tccctatgtt gcccaggctt 11760
   gtcttgaact cctggcctca agtgaccctc ttgccttggc ctcacaaagt gctaggatta 11820
   cagcctgagc catcacacct ggccaacagg tttttttttt tgttttgttt tgttttttaa 11880
   agaatgtcta ggccaggctc atttactttc acctgtaatc ccagcacttt gggaggccgg 11940
   ggtgggcaga tcacttgagg tcaggaattc gagaccagcc tgggcaacat gctgaaaccc 12000
   cgtttctact aaaaatacaa aaattagctg ggtgtggtga cacgtgcctg taatcccagc 12060
   tactcaggag gttgaggcag gagaattgct tgaacccagg aggcagaggt tgcagtgagc 12120
   caagatcatg ccatcaccct ccagcctggg cgacagaagg agactcagtc taaaaactta 12180
   attaattaat taattaaaaa taaaaataca aaaattaacc tggtgtggtg gtgtgtgcct 12240
   gtaatctaag ctactcagga ggctgaggca ggagaatccc ctgaatccca gaggcagagg 12300
   ttgcagtgag ccaagatcga gccactgttt gcccagtcta gtgcactggg ctgctgaatt 12360
   tatttgacca gacacctagc aatagacttt gaagttcttt tccacttttc actctaagat 12420
   gctgctgtca tgaataagga atattttgat ccccttcaca aacactcggg gccctcttac 12480
   cagttttcac tgaagatctt gacattccta tctgcttagg tgtctgggcg tgtttggggg 12540
   agatactgaa gaggtagggc tcccaggcag gtgcagttcg tctgttaggc aggcagcaag 12600
   gtccacttca ccagacaccc ccacctctgt tttcctgcag ggactccaga acgggtggaa 12660
   ctggcacccc tcccctcttg gcagccagtg ggcaagaacc ttaccctacg ctgccaggtg 12720
   gagggtgggg caccccgggc caacctcacc gtggtgctgc tccgtgggga gaaggagctg 12780
   aaacgggagc cagctgtggg ggagcccgct gaggtcacga ccacggtgct ggtgaggaga 12840
   gatcaccatg gagccaattt ctcgtgccgc actgaactgg acctgcggcc ccaagggctg 12900
   gagctgtttg agaacacctc ggccccctac cagctccaga cctttggtga ggattgaaga 12960
   agccagcagg gagaaggtgg gggtggggta tcctgcaatg cggtgcctgt ggccacagga 13020
   tcttttgaga tgggtgtggc cccggctaag gggtgcatgt gttctaggcg tatgtgacct 13080
   aggctgctga gtggccctgg aagaggatct cgcaggaggg ggaatgaaat gccccagaga 13140
   agggcttcgg gacgtccatc cctgtctgct cacacctttc ttctctccct agtcctgcca 13200
   gcgactcccc cacaacttgt cagcccccgg gtcctagagg tggacacgca ggggaccgtg 13260
   gtctgttccc tggacgggct gttcccagtc tcggaggccc aggtccacct ggcactgggg 13320
   gaccagaggt tgaaccccac agtcacctat ggcaacgact ccttctcggc caaggcctca 13380
   gtcagtgtga ccgcagagga cgagggcacc cagcggctga cgtgtgcagt aatactgggg 13440
   aaccagagcc aggagacact gcagacagtg accatctaca gtaagaaggg gcaggggcgg 13500
   agtggggctt cttgggggtg tgacctgaac ccggggcggg gctcactgtg tgcctattcc 13560
   aggctttccg gcgcccaacg tgattctgac gaagccagag gtctcagaag ggaccgaggt 13620
   gacagtgaag tgtgaggccc accctagagc caaggtgacg ctgaatgggg ttccagccca 13680
   gccactgggc ccgagggccc agctcctgct gaaggccacc ccagaggaca acgggcgcag 13740
   cttctcctgc tctgcaaccc tggaggtggc cggccagctt atacacaaga accagacccg 13800
   ggagcttcgt gtcctgtgtg agtggggctg ctggtcaatg gcccctatcc cccaaggccc 13860
   aatctccctg aaggtcccat aaggtcttgc ctccaagtcc tgcccccacc cacctccatg 13920
   tcatctcatc gtgtttttcc agatggcccc cgactggacg agagggattg tccgggaaac 13980
   tggacgtggc cagaaaattc ccagcagact ccaatgtgcc aggcttgggg gaacccattg 14040
   cccgagctca agtgtctaaa ggatggcact ttcccactgc ccatcgggga atcagtgact 14100
   gtcactcgag atcttgaggg cacctacctc tgtcgggcca ggagcactca aggggaggtc 14160
   acccgcaagg tgaccgtgaa tgtgctctgt gagtgagccg gcgggcagag ctgggtgggg 14220
   gcaggggcca tggacctaat gcaatcctca ccgcctgttg tatcctcccc acagcccccc 14280
   ggtatgagat tgtcatcatc actgtggtag cagccgcagt cataatgggc actgcaggcc 14340
   tcagcacgta cctctataac cgccagcgga agatcaagaa atacagacta caacaggccc 14400
   aaaaagggac ccccatgaaa ccgaacacac aagccacgcc tccctgaacc tatcccggga    14460
   cagggcctct tcctcggcct tcccatattg gtggcagtgg tgccacactg aacagagtgg    14520
   aagacatatg ccatgcagct acacctaccg gccctgggac gccggaggac agggcattgt    14580
   cctcagtcag atacaacagc atttggggcc atggtacctg cacacctaaa acactaggcc    14640
   acgcatctga tctgtagtca catgactaag ccaagaggaa ggagcaagac tcaagacatg    14700
   attgatggat gttaaagtct agcctgatga gaggggaagt ggtgggggag acatagcccc    14760
   accatgagga catacaactg ggaaatactg aaacttgctg cctattgggt atgctgaggc    14820
   cccacagact tacagaagaa gtggccctcc atagacatgt gtagcatcaa aacacaaagg    14880
   cccacacttc ctgacggatg ccagcttggg cactgctgtc tactgacccc aacccttgat    14940
   gatatgtatt tattcatttg ttattttacc agctatttat tgagtgtctt ttatgtaggc    15000
   taaatgaaca taggtctctg gcctcacgga gctcccagtc ctaatcacat tcaaggtcac    15060
   caggtacagt tgtacaggtt gtacactgca ggagagtgcc tggcaaaaag atcaaatggg    15120
   gctgggactt ctcattggcc aacctgcctt tccccagaag gagtgatttt tctatcggca    15180
   caaaagcact atatggactg gtaatggtta caggttcaga gattacccag tgaggcctta    15240
   ttcctccctt ccccccaaaa ctgacacctt tgttagccac ctccccaccc acatacattt    15300
   ctgccagtgt tcacaatgac actcagcggt catgtctgga catgagtgcc cagggaatat    15360
   gcccaagcta tgccttgtcc tcttgtcctg tttgcatttc actgggagct tgcactatgc    15420
   agctccagtt tcctgcagtg atcagggtcc tgcaagcagt ggggaagggg gccaaggtat    15480
   tggaggactc cctcccagct ttggaagcct catccgcgtg tgtgtgtgtg tgtatgtgta    15540
   gacaagctct cgctctgtca cccaggctgg agtgcagtgg tgcaatcatg gttcactgca    15600
   gtcttgacct tttgggctca agtgatcctc ccacctcagc ctcctgagta gctgggacca    15660
   taggctcaca acaccacacc tggcaaattt gatttttttt ttttttccag agacggggtc     15720
   tcgcaacatt gcccagactt cctttgtgtt agttaataaa gctttctcaa ctgcc         15775
<210> 36
   <211> 27864
   <212> DNA
   <213> Homo sapiens
<400> 36
<210> 37
   <211> 18501
   <212> DNA
   <213> Homo sapiens
<400> 37
   gctttaatgg cattaggaca gtgggctggg cctgtttaga aactcatgta cctgactgta      60
   tttctcagcc tcagtctctg tgggatgcag aggttcagtg ctctcaacaa caagggccaa     120
   gaaatccaag tgtccgggcc cctgctccct ccaagggtca aggtaaagaa agcagcaagc     180
   actgtggtga gaagaatcag agggaggcaa tgaaaggaag agaagaagtc atgagggatg     240
   gagaccagaa acacagagag aggtaaggga gtgggggaag ttgagacagt tggcaggaca     300
   gcgcgtccac acccatgcac accaccaaga ttaaccccat aatcctctga tcttgtggaa     360
   gctcatttgt agtcttctag cccaaagaaa taaccgtttt tctgaaatgt taagcatcct     420
   tccaagggta tcagagtgat atggagaaat atggtaagac agttacagaa tcagagataa     480
   cgacactgat tctaaaagca ttattaccaa ggcattgttg cattaagact gttttttctt    540
   ttcttttaga gacagtatcc tgctacgttg cccaggcatg tctcagaccc ctgggtttag      600
   tgatcctcct gcctcagcct ccccaaatgc tgggattaca ggcatgagcc gggctgtttt      660
   taattctaag taatttataa tttacaatta cacctaacaa tggcttcaac aaggggtgtc      720
   caatcttttg ccttccctgg gccacactgg aagaagaaga attgtcttgg gccacacata      780
   aaatacacta acactaacga tacctgatga gctaaaaaaa aaaaaaaaaa aaaaaatccc      840
   ccaaaaatct gataatgttt taagaaagtt tatgaatttg tgttgggctg cattcaaagc      900
   catcttaggc catgggctgg acaagcttag cttagaacca agacgaaatt tatggtttac      960
   tttacaagaa ttttgcaaga gggtaatact aggttaggtt atatctggaa aatatcatca     1020
   agaactacat tcattttaat cttttctctt ttttatccct aatatggtga ctttttttcc    1080
   tggggattgc cacttcatgg ctccaagaaa gctaccacag ctcctgactt cacattacag     1140
   caggggtccc caacctccag gccatagacc aatacacaga ccgataccgg tctgtggcct     1200
   gttaggaagt gggctgcaca gcaggtgact ggcaggtgag caagagaagc ttcatctgta     1260
   tttatagcca ctccccattg ctggcattac tgcctgagct ctgcctcctg tcagattagt     1320
   ggcggcatta gattctcata acagtgtgaa ccctactgtg aactgtgcat gccagggatc     1380
   tgggttgtgt gctccttatg agaatctaat gcctgatgat ctgtcactgt ctcccatcac     1440
   tcccagatgg gactgtctag ttgcgggaaa gcaagctcag ggctcccacg gattctacat     1500
   cattgtgagt tgtataatta tttcattgtg tattacaatg taataataat aatagaaata     1560
   aagtctacaa taaatgcaat gtgcttaaat catcctgaaa ccatctaccc cgccacccca     1620
   tggagaaatt gttttttatg aaactggtcc ctggtgccaa aaatgttggg gactgctaca     1680
   ctccagcatc ccacaccagg aggatgggga tgagatggag aatcactgaa gatctttaat     1740
   cagaagggtg atatgttcat ttgtacttta taaacatttg tgtttgtatt ttataaacat     1800
   tttcttggga tcataacaac agatgcattt tagaagattc ccaaggtatg aagggtaaca     1860
   aatgaagacg tgcctaggag tcacatggaa gccaacactt tgtaagggga ttaaaataaa     1920
   gggaatggca gaggacatca ttatgcacag aggagaaggg cccaaatgtc aagagaaaac     1980
   aagagacaag ggactccaca gagagtaatt gccagaaaaa tttgtaggtc ttttgaagtc     2040
   tgactgttct tcctaaaacg ggggtcctgg gacaccccta cctgtttccc atgagccctc     2100
   ttttctaaaa cataagtagg ttttcttcct ggctaccaaa tggcctgtga ttagcacact     2160
   aggttgggca aaaatagagc caagtgtgtg ttccaaagtg actatggtga agaaaaaagc     2220
   gaaagtctta aaatggactt ccacttctgt gtttgcgtaa ggctaaaagg gaagatgtct     2280
   tagggttgac cggaatggga gtattcctgc ctatgctgca aaagtccctg actcttctgg     2340
   taactgaaac atgagaaatg gcctcggcga gcagcacagc ctcccagtaa agcactggac     2400
   tgaggtggta aagccagatt gcctggacta gaatcccagg actactgttc actagctccg 2460
   gacaagccag ctaatccagg ggtctcaaac atttccccca actttttttt tttgagacag 2520
   agtctcactg tgtcacccag gctggagtac ggtggtgtga tcttggctca ctgcagtttt 2580
   gcctcctagg ttcaagtgat tcccctgcct cagcctctcc agtagctggg attacaggtg 2640
   cccgccacca cgcctggcta atttttgtat tttttaagta gagatatggt tttgccacgt 2700
   tgaccaggct ggtcttgaac tcctgacctc aaacaatcac ttgccttggc ctcccaaagt 2760
   gctgggatta caggacggaa ccactgcact gggccacaaa catttcctat gaagagccag 2820
   atggtcaata tatttcattt ggcaggccat agggtctcca tcacaactcc ttggctctgc 2880
   tgttgtagcc tgaaaacagc cacaagcaaa gttgtaaatg aggagcatga ctgtatgtca 2940
   ataaaacttt atttaaaaca cgacaattgg acttcatata agttttgcat ggcacaaaat 3000
   acccttcatt tgattttctt tagccggtaa gaaatattaa agccattctt ggctctcagg 3060
   ccatacaaaa acaagcggtg gctagatttg acccctgatt taactgcact gggtcccaga 3120
   cacttcacct gtgaattgag agcaatgata cttacagagg tgttttctgg agcagaacaa 3180
   ataagatcag tgatacaaag cacttggcat gaattccagt gcaataagta ttatcaatat 3240
   cttagtaatt acataaacct ctctatctgc tgtttcacgt tctctgcggt gaacccatcc 3300
   atgcttaaaa cttgaaccac acctcagcta acattcacca gacccctgtc tctagctcag 3360
   acctgccctt gatgttcaga cacatcattt tgggttcctg tttcctttca aactcatctg 3420
   cagcctttgc tctcctcacc ttctcccaca actctgttaa ggactaaatt gtgtcacatt 3480
   ccccttcaaa ttcttaggtt gaagccctca ccccatgata tggtttggct gtgtcccaac 3540
   cctgatctca tcttgaattg tagctcccat aattcccacg tgttgtggga ggaaccaggt 3600
   gggagatagt tgtttcatgg ggacgatttc ccccataatg ttctcatggt agtgaatatg 3660
   tctcacgaga tctgacgatt ttatgagggg tttccccttt cacttggttc tcattctctt 3720
   ttgcctgctg ccatgtaaga tgtccctttg ttcttccttc gccttccgca atgattgcga 3780
   ggcctcccca gccacatgga actgtgagtc cattaaacct cttcccttta taaattaccc 3840
   agtcttgggt atgtcattat tagcagcatg agaattgacc aatacacccc agcatgtcag 3900
   aatgtgactg tatttgggga tacaaccttt aaagaggtaa ttagctgaaa ctgaggtcat 3960
   tagggtgggc cccaactcaa tatgactgat atccttatga aaagaggaga tccagacaca 4020
   gacaggcata gagggaaaac tgtgtgaaaa catgaggagg aggcggtatc tgcaagccaa 4080
   ggagagagga ctcaggagga atcaaccctg ctgacacctt gatcttaaac ttccagctga 4140
   taaaactgtg agaaaatata tttctattgt tgtagccacc cagccaatgg tattttgtta 4200
   tggcagctct aacaaactaa acactctgct tcttttcatt gactctcgtc tcaatgacag 4260
   cccccaaatc cacgcagtta ccttggtgca acagttctga agaaaatggc cacaatattc 4320
   ttggacactc cttccactga caggaaggca ggcatattgt atcgcctctc ccctggaacc 4380
   tgggtagggt tgggactact ttgaccaaag aacgtggtag aaatgacttc cacgtagctt 4440
   tcaaggctag gtcaaaaaag gctaggaagc atcttctggt tctcctggga cacgtgttct 4500
   ggaggaaggc agccattgtc ctgggacttt gattcctctg agaccaccat gctggaaagg 4560
   ctgcctgtag gcacttggtg caataccagc ctagttcagc atttgagtca ttccaaccca 4620
   ggcatctgac atgtgaatga aggagctcta gatgactcca attcacagtc ttctagtcac 4680
   cccccagacc tttgagtttt gggtggcaga gacaagcatc tctgtgcctt atcctaattc 4740
   ccaacccaca taatccatga acgtaatgaa atggttgttt tacatgctat gatttgggtg 4800
   gttatacagc aattatgaat tatgaatgtg ggcttcataa agacccttca tgcttcctca 4860
   ttctccagag ccatttggtc tccaagctca gtctaccaag cctggaaatc tctccctaaa 4920
   ctacacctcc tctctagcac actactccag cacaggcaat tattcgacaa gttcaccttg 4980
   atctctggca acttcttacc ctttaagatc tctgcctcca actgggagca ttctcagagc 5040
   catccctttg cagggggact ctagagcaat gttttctgga agcacacacc tgacaagtca 5100
   ctctcctgct taaatagctt ttaagcagaa gaaattgtaa gaaagaaatt tttttttaga 5160
   ccaatatcct tgatgaacat tgctgcaaaa atcctcaata aaatactgac aaaccaaatc 5220
   cagcagcaca tcaaaaagct tatccaccac gaccaagtgg gcttcatccc tgggatgcaa 5280
   ggctggttca atatacgcaa atcaataaat gtaatccagc atataaacag aaccaaagac 5340
   aaaaaccaca tgattatctc aatagatgca gaaaaggcct ttgacaaaat tcaacaaccc 5400
   ttcatgctaa aaactctcaa taaattgggt attgatggga cgtatctcaa aataataaga 5460
   gctatctatg acaaacccac agccaatatc atactgaatg ggcaaaaact agaagcattc 5520
   cctttgaaaa ctggcacaag acagggatgt cctctctcac cactcctatt caacatagtg 5580
   ttagaagttc tggccagggc aattaggcag gagaaggaaa taaagggtat tcaattagga 5640
   aaagaggaag tcaaattgtc cctgtctgca gatgacatga tggtatatct agaaaacccc 5700
   attgtctcag cccaaaatct ccttaagctg ataagcaact tcagcaaagt ctcaggatac 5760
   aaaatcaatg tacaaaaatc acaagcattc ttatacacca ataacagaca aacagagagc 5820
   caaatcatga gtgaactctc attcacaatt gcttcaaaga gaataaaata cttaggaatc 5880
   caacttacaa gggacgtgaa ggacctcttc aaggagaact acaaaccact gctcaatgaa 5940
   ataaaagagg acacaaacaa atggaagaac attccatgct catgggtagg aagaatcaat 6000
   atcgtgaaaa tggccatact gcccaaggta atttacagat tcactgccat ccccatcaag 6060
   ctaccaatga ctttcttcac agaattggaa aaaactactt taaagttcat atggaaccaa 6120
   aaaagagccc gcattgccaa gtcaatccta agccaaaaga acaaagctgg aggcatcacg 6180
   ctacctgact tcaaactata ctacaaggct acagtaacca aaacagcatg gtactggtac 6240
   caaaacagag atatagatca atggaacaga acagagccct cagaaataac gccgcatatc 6300
   tacaactatc tgatctttga caaacctgag aaaaacaagc aatggggaaa ggattcccta 6360
   tttaataaat ggtgctggga aaactggcta gccatatgta gaaagctgaa actggatccc 6420
   ttccttacac cttatacaaa aattaattca agatggatta aagacttaaa tgttagacct 6480
   aaaaccataa aaaccctaga agaaaaccta ggcattacca ttcaggacat aggcatgggc 6540
   aaggacttca tgtctaaaac accaaaagca atggcaacaa aagccaaaat tgacaaatgg 6600
   gatctaatta aactaaagag cttctgcaca gcaaaagaaa ctaccatcag agtgaacagg 6660
   caacctacaa aatgggaaaa agttttcaca acctactcat ctgacaaacg gctaatatcc 6720
   agaatctaca atgaactcaa acaaatttac aagaaaaaaa caaaaaactc tatcaaaaag 6780
   tgggcaaagg atatgaacag acacttctca aaagaagaca tttatgcagc caaaagacac 6840
   atgaaaaaat gctcatcatc actggccatc agagaaatgc aaatcaaaac cacaatgaga 6900
   taccatctca caccagttag aatggcaatc attaaaaagt caggaaaaaa caggtgctgg 6960
   aaaggggaga aataggaaca cttttacact gttggtggga ctgtaaacta gttcaaccat 7020
   tgtggaagtc agtgtggtga ttcctcaggg atctagaact agaaatacca tttgacccag 7080
   ccatcccatt actgggtata tacccaaagg actataaatc atgctgctgt aaagacacat 7140
   gcacacgtat gtttattgca gcactattca caatagcaaa gacttggaac caacccaaat 7200
   gtccaacaat gatagactgg attaagaaaa tgtggcacat atacaccatg gaatactatg 7260
   cagccataaa aaatgatgag ttcatgtcct ttgtagggac atggatgaaa ttggaaatca 7320
   tcattctcag taaactatca caaggacaaa aaaccaaaca ccgcatgttc tcactcatag 7380
   atgggaattg aacaatgagg aaacatggac acaggaaggg gaacatcaca ctctggggac 7440
   tgttgtgggg tgggggaggg ggggagggat agcattggga gatataccta atgctaaatg 7500
   acgagttaat gggtgcagca caccagcatg gcacatgtat acatatgtaa ctaacctgca 7560
   cattgtgcac atgtacccta aaacttaaag tataataata aaaatatata tatatataag 7620
   aaagaaattt taagaagaaa ttctaagttc tttttacagc attcaagacc aaccaagaaa 7680
   ttgcctctgc cgccctctac atacttaatg cttattgctg tctgccttat catctatgct 7740
   ctggtaaaca aaagtaacct gcttttttgt gacccctttc gtgggtattt ttactcccct 7800
   ctgacaagaa tgcattattt tttctccttt atctgagtct ttaagactca gcctacatgt 7860
   tccctcctcc ggatattgac tctagatccg tgaatctgag ttagtggttc cttttagagg 7920
   acctcacaag gagccaggca tctgtctatc actacgtgcc cccaccctat tgtaactaag 7980
   cactgcattc tcacctctct attcaggtgg tccgcagagc ccatgtctga ttgatctcta 8040
   tgtctccagc agccagcaag gaagcacctc tttagagacc tgcacctata caatacctac 8100
   caccttttat ttctcgatat gtgaactcca ttgagaacaa acgagtaaat gtaggtaatg 8160
   tgccttcttc ttttcttttc ttttcttttt ttttttagat ggagtctcgc tctgttgccc 8220
   aggctggagt ttagtggcac aatctcggct cactgtaacc tccgcctccc aggttcaagc 8280
   gattctgccg cctcagccta ccaagtagtt gggattacag gtgcccacca ccacgcccag 8340
   ctaatttttt ttgtattttt agtagaggtg gggtttcatg atgttggcta ggatggtttt 8400
   caactcctga cctcaagtga tccacccacc tcggcctccc aaagtgctag gattacaggt 8460
   gtgagccaca gcgcccagcc agtaatgtgc cttcttaagt tctgtgagcc attctaacaa 8520
   attatcagaa cagaggaagg ggttataaac atccccccac ccccgattta tagccagtca 8580
   gtcagaagta caggtggcca cctgggactt ggattggtgt ctgaagtgag gacagttttg 8640
   ggagagtgag ccctttaact tgtgggatct gacactaact ccaggtagac agcgtcggag 8700
   ctgaattgaa ttgtgagata cccagtggtg tccccagaga actggagaat tgcttgatat 8760
   ggaaaagacc cacacatttg atgccagaag tactgcataa gtcgagaatt gagtttgact 8820
   taatcatcat atttgggctt gattgcgtgg ctgaaactct tcccacttca gtaattgttt 8880
   ctttcatttt catgaaactc tgaagaagga agggctggac attcagattc cttgaccctt 8940
   gacatttgga agcatgaact ccagtctctc acagaaggct agaggtgaag gaacattcag 9000
   acacattggt ttctaagaag agtccgctga caacataccc aaggtgtctt ctgaaaatta 9060
   taagaaatcc tgagtttctg ttaggggatt ggctccagct ccattgtccc tcccccatca 9120
   ttcagtagtc tccgcgaaag cccttagagc cggtgttgct ccacaggaag ccaagaagca 9180
   cacaggaaaa ggagcttagc tgctggtaag ttggggactg acaatttaaa atttatcaat 9240
   ggaactcctg ctctctgaca aacacctcct aaattctaga gcagtgctgt aatggagcat 9300
   ccacttgcca caggtggctt ttgagtgcct atgtttctat cttgatctga aatgtgctta 9360
   agtgtaaaat atacttagtg tagtttaatc attagtatga tcaaaataat acaatttatc 9420
   tcgttaataa ctttcatttt ggtaacgtgt tccaatgatt ttttttattt gcggggttaa 9480
   atgaaacata ttattataat taatttctcc caatgatttt acttttttaa catggctact 9540
   agaaagttgt aagtcacgtg tgtggctggc atttagggct tgcattaccc tgtctctact 9600
   aaaaatgcaa acaacaaact agccaggcgt agtggtggga gcctgtaatc ccagctactc 9660
   aggaggctga ggcaggagaa tcgcttgaac ctaggaggcc gaggttgcag tgagccgata 9720
   tcgtgccatt gcactccagc ctgggcaaga agagtgaaac tctaactcaa agataaataa 9780
   acatgtgaag gagccaagtg tgaggagctt aagaggaaga gcatggtagg cagaaggaac 9840
   agagaaggca gaagccccga gatgacaatg agctgcatgt tcaaggaatc agagcccgtt 9900
   atgggtggag cccagtgtgt cccacgagag tgcagagatg aggtctgagg aatgcacagg 9960
   gcaggtccag gggtacttga gggaacttaa gataaggata agttagaagt ttggaagcca 10020
   ttagaaggtg ttgaaaatag catcaccatt ttattggacc tttttttttt ttttttttga 10080
   gacagaatct tgctctgtcg cccaggctga agtgcagtgg cgcagtctcg gctcactgca 10140
   aactctgcct ctcagggtca agcgattctc ctgcctcagc ctcccaagta gctgggatta 10200
   caggcacccg ccatcacgcc cagctaattt ttgtgttttt ttagtagaga cggggtttca 10260
   tcatgttggg caggttggtc ttgaactcct gacctcaggt gatccaccca cctcagcctc 10320
   ccaaagtgct gggattacag gcatgagcca ctcccggccc ttattggact tttttttttt 10380
   ttttgagaca gagttttgct cttgttgccc aggctggagt gcaatggcac gatctcagct 10440
   cactgcaacc tccgcctccc tggttcaagc gattctcctg cgtcagcctc ccgagcatct 10500
   ggaattacag gcacgtgcca ccacgcccag ctaattttgt atttttagta gagatggggt 10560
   ttcttcatgt tggccaggtt ggtctcgaac tcctgacctc tggtgaccca cctgcctcgg 10620
   cctcccaaag tgctgggatt acaggtgtga gccactgcac ctggccctta ttgaactttt 10680
   taaaagctca tttggtttcc ttctggagat tggattgtag ggcaggggaa agtaggacca 10740
   ggaacaacct atttgcaaag ttggcgcaaa cattcctgcc tgacaggacc atggacacag 10800
   gttgtagaga tagagatggc tctggctgtg cattcagcag attctgtaga tagaattaat 10860
   aggacttgga tgggattgtg gtgagagaaa gtgaaatgaa agataagttc tagtttggaa 10920
   gttttaacaa ctgaatgttt aaactcaaat agacacaaaa tattggaaga gtggcaggtt 10980
   tgggaggatg agacaatcaa ctgtttggtt gagccacgtt aggtttgaaa tgtctacggg 11040
   actcccgtgg ggagaggtta tatcagactg gagcaccaga gagaggccaa ggctgatagt 11100
   ttagatgaaa agagagcatg atattttaag ccctgagact ggataatatc acctatagaa 11160
   agactatata gagataagag aggtggggaa caagtaaaag ctgcgggaca ctcctaaatt 11220
   tagagtcaaa tttagagcag aaaatactag caaaggggac tgaaaagcgg tggccaattg 11280
   agcttcaaat gcaagtgaaa gtgtgttgtg tgtacattta tcatctcatg gcacaggaaa 11340
   aacgtgattt aaggagaagg aagcgatcca atgggaagaa gagatccaat ggatcctcta 11400
   tcacgaagat attgagataa gaaccaatat ggatttgcac ccactgcatt tgcagccttg 11460
   aggtcataag catcctcagg aaaatgcacc aggtcagtgc taaggactgg agtcaataca 11520
   gactggattc aggagaaaaa gtgaggcagg gaatcaaagg gagcaaacac agaaaactcc 11580
   ttccaaaatc tggctgcaaa ttcttttcat agaaagaatt ctctcaggag aaatagggtt 11640
   aacaccagaa aggaatatag tccaagatct ttttggttgt tttttgtttt gtttttgggg 11700
   gtggttttta ttttgttttt gtttagtttt gttgttgttt gtttgtttgt ttttgagacg 11760
   gagtcactgt tgcccaggct ggagtgcagt ggtgcgatct cagctcactg caacctccgc 11820
   cttccggtgt tcaagaggtc ttcctgcctc agcttcccaa gtagctggga ttgcaagcgc 11880
   gtgccatcac gtccagctaa tttttgtatt tttagtagag atgaggtttc accatgttgg 11940
   ccagtctggt ctcaaactcc tggcctcaag tgatccactt gcctcagctt ccctaaatgt 12000
   tgggattaca ggtgtgagcc acggcacctg gccaaggatg gtttcttttt aaagggaggg 12060
   gatatgattt atgtaatgtt tatgtaatag tgagaatgat ctagtagaaa taaaatcttg 12120
   agggtttgtt tttggcagaa aatggaagga caaatgcagg gggaaagttt gtgggaagat 12180
   gagagggcat cctgggccaa tgcagggagg gaaattattg gaaagaacaa gaagtcttta 12240
   ttcacagtga cagaggcagg tagactgagg gtcaggggag gaccggctga aaatttcaca 12300
   tctagttatt accattttct cattataatg tcagtcaagg gcatctgctg agagtgggaa 12360
   aaacagagta aatgaggtac tttttggaga aagcaggact gaaataatta aggtgtggga 12420
   tagcgagtgc gctggagaag tagagtaacc taatgaggaa ctgctgagtt ttcatttgaa 12480
   atctgtggtc ataaattcaa ttgcaaccct cgataattgt gaaagtcact tccgctctct 12540
   gagccgtact ttcctacctg ttacacaagg acagcacttg tcccaaagca agggtattgt 12600
   aatcagagat gataaaatat atagtgactg gcacatatta aggactcacc aaatagcaga 12660
   cctcataatc agtacaggta tcatctgtgg ttcgcagttc tcctccttac attacaacta 12720
   gggtggaccc aaaactttgc aatttttgcc ttgttaattt gtcctcacct gtcattgcac 12780
   aatcatcctt ccttgttgca tcaacatttt tttttttctg agacagagtc tcgctctttc 12840
   acccaggctg gagtgctgtg gcacgatctt ggctcactgc aacctctgcc tcccagactc 12900
   aagtgattct cctgcctcag cctcccaagt aactgggatt acaggcaccc accaccatgc 12960
   ccagctaatt tttgtatttt tagtagagac agggtttcac catgttggcc aggctggttt 13020
   caaattcctg gcctcaagtg atccacctgt cttggcctcc caaagtgcta ggattacagg 13080
   cgtgagccac tgcgcccagc ctgtatcaac attttaaaat gaatttgaaa ttatggatag 13140
   atcatgtctt ctaagtgtct tgtagaatgt ttggagtgtg gatagagaaa atctgagtct 13200
   gttgaggaac tatggggtgg aaaagggtta acagcacaat tctgttgtaa tcaatctggc 13260
   tgagctccaa ttctgtctct actctttcta cctggtaacg ttgaacaatt aacgcagccc 13320
   atctaagtct taacttcctc atctgtacaa tggttatact aattatcagg ctgctgcaaa 13380
   agtacttgtg gtttttgata ttattttcaa tggcaaaaac agcgttactt ttgcaccaac 13440
   ctaatatcac taagattaat caatattcac tgtgtctcag gagtgctcag tgttcacaca 13500
   tattaacaca gcccatccac agatcctgtg agaggcgttg atagggttaa aggaaaagct 13560
   cttggtgtgt gggtaatgtt cataagtaga aacaactatt gctatgagca tcgttcttgt 13620
   cctgaccttg aacttcataa tggattgagc cccacgacac agcacacagc ctggtgaggg 13680
   ccaagagttc ccatttgtca tagatatact cagtctagcc taaaagattt ccacatctat 13740
   tctcacttaa tcctcacctg aaagttagac ttcaccataa aattgtcctc gttctgcaga 13800
   gcagaaaact gaggctcaaa gagatatcac tgactaaaaa ctcatagtga ggagacatca 13860
   cagatgagat tagaagagag atgtagctga cttcagagcc cagtatatat atatatttta 13920
   gacagagtct cgctctgtcc cccaggctgg agtgcagtgg cacggctcac tgcaacctcc 13980
   gcctcccggg ttcaagcgat tctcctgcct cagcctcccg agtagctggg actgcaagtg 14040
   catgccacca ggcccggcta attttttgta ttttcggtag agacggggct tcaccgtatt 14100
   gcccagggtg gtctggaact cctgacctca ggcaatccac cccccttggc ctcccaaagt 14160
   gttgggatta caggcgtgag ccaccgtatc tggctgagcc cagtattctt tgtaacagtg 14220
   actctcaatt gggggtgact ttgcctccga ggggaccttt gacaatgttt atggaacctg 14280
   ttgattgtca tttctggaga aggagttgct actggtgtct gtggatagag gccagggggg 14340
   ctgctcaaca tcctactgtg tccagaacag tctgccacaa cgaagaatta cccagaccca 14400
   gatgtcaaag gtgccaaggt tgagaaaccc tgagctacat taaataacgt cccatggaat 14460
   acctcagtga aactcactgg taatagcagg aagaaaagga ggaaataagg aaaaggaaga 14520
   aggctaaaaa tcaccagact gaggcatagc cctatcctag taatcttggg ggaatccctt 14580
   tcaaaggcag gacttccgta tgcaaaggca gtggctctgg agttgttcat ctcggtttcc 14640
   gtatcttgct gcctccactg actggctggg catgatgttg aaaaaaacta cagaagtccc 14700
   cgggccatga attcccccag atgtgatggc tgtttcccaa tgttattatg agcattagat 14760
   gtgaacaagt gctatggaat tgctatttct cttaaagatg ctgaatagcc cagtctgaga 14820
   tgattctcaa attctcatgg ccatagcaaa tcatcctggc ttttacacat acagattcta 14880
   gaatgagatt tatggatata attgtttttc atttttatta taagttcagg ggtatatgtg 14940
   caggtttgtt atacaggtaa acttgtatcc tggtgttttc ttgtacagat tatttcatca 15000
   ctgagatatt aagcctaata cccactactt atttttcctg atcctgtccc tcccctcacc 15060
   ctccaccttc cggtaggccc cagtgttccc ctctttgtgt ccatgtgttc tcatcattta 15120
   cctcccattt gtaaatgaga acatgtggta tttggttttc tgttcctgca ttagtttgct 15180
   aaggataatg aatacaattt taacaggcag cctggatgtt tctgataaag tccacagacc 15240
   acggcataag aaaatgtgct caggtgacgt gggactttgt gttgaactgg atacaaatat 15300
   tacagatgtg atccggacca gaggcaggga ctacatgttt tgggtttttg ttcttaaaag 15360
   aaaaaaacaa aaacaaaaaa acttgatctg agaagctctg aagggtcata ttcttacaat 15420
   gttattgtcc acaatttagt cacgtgtcca ctaccaaagc aatcccttgc aaagtaaaag 15480
   ggattctggt tagtgctaat tagactagtc aggatttact cccaacatgg agaaaagaca 15540
   ccttctctgc agggtgagta actaaacaca attgtggttt ttcaagcaga gaagaatggg 15600
   gaaccgttgc tgggtgggca accagcaata tctgtcaagc gtaagggttt agaagtcaga 15660
   ccatgccaaa ctgaagtcct gaatctatta tctactacct aagtgacctt caacacattt 15720
   cacaattgcc ctgagctttc atttattcta atgaaaacta gaagtaatga atattacata 15780
   gctattacat aggccaaacc ggatcccata tgtagtaatt aacacaatgc atagtctcta 15840
   ataagaactt cacaagtatt aatacaactt attattctta tagtctttat cccacagaca 15900
   ccaaagctat acaaatcggc actgattatt tgcattccaa acagagaaga gctccacatc 15960
   aagcagggac agggaaaagc agagggtaga aagcagagat ggaactctct ttgctgactt 16020
   cctctagtgc agaatcctca ggatataaca tactactaca tgttaccagt gactcctcaa 16080
   aattctcttc aacaataacc tcaagccaaa ccctcaaact ttatcttgag tactagccaa 16140
   gcaataggtc aagggttctc acagtttgag ggacaggagt tgggagactg gaagaacctg 16200
   gggctgaaag agattgcaga gcttgccagc atgtttgcat tgaatggtgg gagacacaga 16260
   tgtgagcctt ctgattctga gtcatgtgcc ttcctcacaa tgctgaggct gcctatgctc 16320
   aattttccat tgtccataca acataagttc agtatgagcc ttgtaatcct cttggtgaag 16380
   tgatcagccc gataagcgaa atgcatattg ataatgatgg gaatatggag gactatccct 16440
   tcccaaagga gcatccaaga gaatgccaat tctatagcac cgttgtcaac aaggcttggt 16500
   agatagagtg ggtctcctgg taggagtggg agctttagtg gaagttgatg tgagctataa 16560
   atacagatgc tgtaagatgg ttccacagct gagaaatggc cattgttgga atgtttcagg 16620
   tgctgctggc aagatggaaa ccaacttctc cactcctctg aatgaatatg aagaagtgtc 16680
   ctatgagtct gctggctaca ctgttctgcg gatcctccca ttggtggtgc ttggggtcac 16740
   ctttgtcctc ggggtcctgg gcaatgggct tgtgatctgg gtggctggat tccggatgac 16800
   acgcacagtc accaccatct gttacctgaa cctggccctg gctgactttt ctttcacggc 16860
   cacattacca ttcctcattg tctccatggc catgggagaa aaatggcctt ttggctggtt 16920
   cctgtgtaag ttaattcaca tcgtggtgga catcaacctc tttggaagtg tcttcttgat 16980
   tggtttcatt gcactggacc gctgcatttg tgtcctgcat ccagtctggg cccagaacca 17040
   ccgcactgtg agtctggcca tgaaggtgat cgtcggacct tggattcttg ctctagtcct 17100
   taccttgcca gttttcctct ttttgactac agtaactatt ccaaatgggg acacatactg 17160
   tactttcaac tttgcatcct ggggtggcac ccctgaggag aggctgaagg tggccattac 17220
   catgctgaca gccagaggga ttatccggtt tgtcattggc tttagcttgc cgatgtccat 17280
   tgttgccatc tgctatgggc tcattgcagc caagatccac aaaaagggca tgattaaatc 17340
   cagccgtccc ttacgggtcc tcactgctgt ggtggcttct ttcttcatct gttggtttcc 17400
   ctttcaactg gttgcccttc tgggcaccgt ctggctcaaa gagatgttgt tctatggcaa 17460
   gtacaaaatc attgacatcc tggttaaccc aacgagctcc ctggccttct tcaacagctg 17520
   cctcaacccc atgctttacg tctttgtggg ccaagacttc cgagagagac tgatccactc 17580
   cctgcccacc agtctggaga gggccctgtc tgaggactca gccccaacta atgacacggc 17640
   tgccaattct gcttcacctc ctgcagagac tgagttacag gcaatgtgag gatggggtca 17700
   gggatatttt gagttctgtt catcctaccc taatgccagt tccagcttca tctacccttg 17760
   agtcatattg aggcattcaa ggatgcacag ctcaagtatt tattcaggaa aaatgctttt 17820
   gtgtccctga tttggggcta agaaatagac agtcaggcta ctaaaatatt agtgttattt 17880
   tttgtttttt gacttctgcc tataccctgg ggtaagtgga gttgggaaat acaagaagag 17940
   aaagaccagt ggggatttgt aagacttaga tgagatagcg cataataagg ggaagacttt 18000
   aaagtataaa gtaaaatgtt tgctgtaggt tttttatagc tattaaaaaa aatcagatta 18060
   tggaagtttt cttctatttt tagtttgcta agagttttct gtttcttttt cttacatcat 18120
   gagtggactt tgcattttat caaatgcatt ttctacatgt attaagatgg tcatattatt 18180
   cttcttcttt tatgtaaatc attataaata atgttcatta agttctgaat gttaaactac 18240
   tcttgaattc ctggaataaa ccacacttag tcctgatgta ctttaaatat ttatatctca 18300
   caggagttgg ttagaatttc tgtgtttatg tttatatact gttatttcac tttttctact 18360
   atccttgcta agttttcata gaaaataagg aacaaagaga aacttgtaat ggtctctgaa 18420
   aaggaattga gaagtaattc ctctgattct gttttctggt gttatatctt tattaaatat 18480
   tcagaaaaat tcaccagtga a 18501
<210> 38
   <211> 14156
   <212> DNA
   <213> Homo sapiens
<400> 38
<210> 39
   <211> 15540
   <212> DNA
   <213> Homo sapiens
<400> 39
   agaacatttc tttttcactt cccctttcag actccagaat ttgtttgccc tctagggtag      60
   aatccgccaa gctttgagag aaggctgtga ctgctgtgct ctgggcgcca gctcgctcca     120
   gggagtgatg ggaatcctgt cattcttacc tgtccttgcc actgagagtg actgggctga     180
   ctgcaagtcc ccccagcctt ggggtcatat gcttctgtgg acagctgtgc tattcctggg     240
   tgagtcaggg cccccaggag ggagagagga acgggaacct cagttgggga taaccctggg     300
   caaggatgag gggtggcatg  ggaagaccgt caggacaccc aggggaggga aggcaggctg     360
   gtgaagatga aaccctgatg actctgctgg ctttggaaag tcatccaatg tggcagagtt     420
   cagggttttt tcagtgtctt tcacccaatc ttactgaaca cccagactct tgatgttctg     480
   ttgctttcca catcagaacc cgctctgaga aaagtctagt aacctatact tgaatgataa     540
   atgaatggcc agagtgtttg ccttctttga aatttatgct tcacaaaaga ccaaatctta     600
   ggcaggaggg ccagtgctgc cttgggtaag gggtagggag  gaagttgtaa gatgcatttt     660
   tgggaggagg tggcagtggc cattctctgt gcccctgaaa ccactttgtg tagcagaagc     720
   ctattgtctt catgtgtaag aatattcccc taggcagaag cacagggaga tctgggttat     780
   ccagggaaaa gggactctga gaatacaaaa acctgggtaa gtctggagtc atctgttgtg     840
   ggaccagaga aatcatgcat gctgcttttg accacacaga ccatgatggg gaagtactgc     900
   atctgatcag caagagagac agggtctttg gccaaaatgg agggctcttt tctaaccatg     960
   ttctcagctg ttggataggg gacgacgact gattttgctg agtggtttga aaagcctagg    1020
   ggaggccaag taggggagac ttactgccag gactatctct ccaagctcag ttcctggggc    1080
   ttccaccctc acattctaga ggaaagcata ttgccactca gccaagttgt gtgatgtgat    1140
   tactagtgaa acgtgatcag ctctgggggc cctcggctct tggctgtcaa tgcaggatga    1200
   tactgggcta caggagaaag aggtaaggaa gacagagtat tcccccatga ataggatgga    1260
   atggattaaa gtagaagaga aagtaaagtg aactacctca tttgtcatgt ggggaagttc    1320
   aggcctagag aggtgatgtg cttctctacc acctgggcaa gactcctggg aaagatcttt    1380
   ggtatttttt cccatttgcc ttttactaag tcatcaaaga tttaccccat tttttccatt    1440
   cttattgggg ctgccacatt tacgtcttca tcacttgata actcaatttt tatagcaccc 1500
   tctgatgggg tcttgattga tcctgtctcc cctcccctgc atccatgctg cccaccaagg 1560
   ccacggcatc ttcaggtcca gctatggctg tgtcagtgca tttaataaat gccttccgtg 1620
   ctgctccatt gcccactgca aaaactctaa ttctgccctg cacactgtgt tctccattgc 1680
   accccacatg aatcttcttc tacagccagg cagtatgtac actgcacttt ctgtttttac 1740
   cacctttgct cctgctgact ctccaaattc tatgtgcagg cactgttcta ggacttggag 1800
   acttatcagt gaacatgact gacagccttc ctgaccggaa atacgaggtc aatgaactta 1860
   ttgtgaagca ataagtacac cgggtgggag gatatttggt atttactact atttattaac 1920
   tgtggaatgg atacataatt ggtgacttct ctctcctttc ctcatctttt cctttttttt 1980
   ctctcgctct tcctcttctt ttttactctc tctcccacta caccgtcccc cttgccaatc 2040
   ttacatactc aggtttttag ctaccacttc tatgtcaata actcctaaac ctatttctct 2100
   tgcttgcttc tgtcaagtat aattttatat ttccactgct ggttggacac ctctaaaatg 2160
   attctccagc tcaaaaacaa agcatattca aaatgcaact tatcatctcc tccttacaaa 2220
   tgttctcttc ctcctaacat ttgtatttgc gtaagcgcag ccaccctttt cctagttatc 2280
   caacttaaat cacccccaca gtctattaaa tgccaacttc tctctgccac ttaaatttct 2340
   tctgcatctg tttcctcttc ttcacctcct attgtcagta caactttggg ctctcttatc 2400
   tcccacctag attcaaccag tgaatgattt ccagagcaat ggcacatcaa attaatatgg 2460
   ctactgctct ctttgaattt atattctact gagggatata aacggtaagt aaacaaacaa 2520
   gaaggcataa gtacaaatgg aaaagggcta tgaagatgta aacggggtac tgtgataaga 2580
   ttagttgtgg ggaagagaag agagcagccc actttaaaca ggatgttatg taaagagttt 2640
   tctgaggggg tgacatttaa gctgagactt tcaacatggt aaggattcag ccatttggtg 2700
   atcaaggacg catttcaggg tagagggatt ggcatacaga aaatccccaa atttgaagaa 2760
   tgccaggaag gccagtgtta atgaggctca atgagtaggt gaaaagggat ctgagctgag 2820
   gctggagaag gaagcacgga cccaatttga tagggcttgc aggccatttc cattttattt 2880
   taagaccaaa gaaaggcaac tgaaggcttg taagttgggg agtgatatca tctgatttcg 2940
   tttttttttt ttttttaagg aactagcttc tttatggaaa atggattgta gggtgaccat 3000
   aagaagtgag gggacaaatt aagatatggg aaaaagctga tggtgactta aactgagtag 3060
   tgtctataaa gggggtgaag agaagttagt gggtgcaaga tctaagttgg aagcaggatc 3120
   aacaagactt cctggtgtgg aagggactgg attttgggag acagagaggt taaaagatgg 3180
   cttgcaggtt tctggcctat gtagctgggt ggataacgaa gatacatatt gacatgggga 3240
   agacggagag aaacagaatg tgtccacttg ctatgacaac atgaagccat tggtgatttt 3300
   gacaagaaaa gttttagtgg actgctgagg gccgagtcca aattgcagag aattagacag 3360
   tgaatgagag gagaagaaat caggacagcc tatgtaagaa aaacaatgga gaaatttagt 3420
   tttgaaaggg atgagacagg ccaggcacag tggctcatgc ctgtaatccc aacacttgga 3480
   gaggccgagg tgggtggatc acttaaggtc aggagttcaa gaccagcttg gccaacatgg 3540
   tgaaacacca tctctactaa aaatacaaaa attagccgga tgtggtggta atacaaaagt 3600
   tagccgggcg tggtggtgca tgcctgtaat cccagctgct tgggaagctg aggcaggaga 3660
   attgcttgaa tctgggaggc ggaggtcgta gtgcactgag atggtgccgc tgcactccag 3720
   cctgggtgaa gcaaacaaca acaacaacaa aaaccgagag aaatggggca gtagctggaa 3780
   ttatatgtgg tgtcaaggaa aagttttttt ttgttttttg tttttaatgg aagatactga 3840
   aataaattta ccattgatgg gaagatccag ctaagagaga aactggtgat gcagaggaga 3900
   aagtgaataa cagaaaggga aatctttgag aggattaaag ggaatgggat ccagagtact 3960
   tgctttgaga ctgaccatag acaggaggag agatgctttc tccagtgaaa tgcgaggaag 4020
   gagaagacag gtagagatgc agaaaagact ggtagattta gtcgggggac gatgtgagat 4080
   cttctgtgta acctcttttg ttttctctgt gaaatatgag gaaaggcatc agctaaaaat 4140
   gagagaggac tggaggcaga gggtgggatg agaaagcttg gggaatgagg aaaagatgag 4200
   tgtcactact cagagtagca gagcaaattt cttggaaaaa agtgccacaa tggcagttca 4260
   gtgtcctttt gaatgcggtg atcgcacatt tactgcgtca acagtttgcc ttcttgtgtg 4320
   gtaatttcaa taacacgcag ctgagcaagt aaaggcatgg cgaatacagg tgcatttggt 4380
   agagacagag aaaggcaacg aagttaaggg gatttgcaag gaggtaatta taatgatgcc 4440
   tcattgaatt caagctagat gggggagggt gggaggtaaa ggcaaacatg tgctcaagga 4500
   taggcagtgg taagatggat gctttggggt tcttgataag gtcaaagatt attctggtgg 4560
   ctgtacttga gcagagaaag ttcatctcca aagaaagaaa gtttactgat gatgagcagt 4620
   tcagggaatg aagaggccag ttgtttgatg ggttatccac atattctgga tcactacagt 4680
   catctctttt ttttttcttt ttctttttct ctgacctcat tcttccatca caattagttt 4740
   ctttcttaat taagctcttt ggcatttgcc ctgttccaat ctatttgaca ctgcactttc 4800
   agccccaatt tgaaaattct aagtgacttc ccactgtata ctgaaccaag gagtgaactt 4860
   ataaaaaaaa acttaaattt atttgaaatt caccatgtgt catgtagcac actaaatgcc 4920
   ttctctggat tatctcattt aatcctaaca accaccctat gaggtagata ctgctgtcat 4980
   tatcttcatt tacaagcaag gaaactgaag cagagaaaag ttaagtaatt tgtgaaaggt 5040
   cacttagcag tagcggagtc agcatttaaa ctcagtatgt gctttccccc actgccacac 5100
   cacctggcac taggagccct ccagagtatg acttctacct tgctttcctg ctttgtccct 5160
   cattattgga agtgcttgag gagagaccag aaatcaagta gaagttgggc tgcaacttgc 5220
   tgggaatgtt gccaagtact tgctgctcct tcccatggaa taccttttcc ctctcagcca 5280
   aaatcctatc agtcctggaa ggtccttgat gaacctgaaa ggtcccatct tctccatagg 5340
   attcccctcc ttatgaaccc caaaccctcc caaacacaga gccctgggat cttttctttc 5400
   tctgaacctc catagcattt tatccaaatt cctctaatgt catctctttt acatcagatt 5460
   acacatgtgc ttatctccac cgttagattc ttgccttcag tggaagtagg gacttaacgt 5520
   tcttttccct taggtgaact cagcatgcat tgcagggcag taggaagcca atgcaggttt 5580
   gttgattaat tttggatgca aaggaaggaa gtgattggtc caagtgtata tttcgagata 5640
   ttggagcatc gggaccagaa tccagttctg taaactttga agccaacact ttctccacat 5700
   tgctcccttt ttacctcttg gttctgattc catttccttc cgtacttttg tccctgaaaa 5760
   tatacatgca cttggaagct tcttgacttt ctctcagccc agatgcaaga gaaaaccttc 5820
   acccatggac agatggacac acacacacat acacacacac acacacacag agctctgatt 5880
   cacctaagga cccagctttg cttctttacc caataacagc acccacaaat ctttatctac 5940
   cctagcccac actttgcctc cactacggaa gctcttttct taacccagtg gcatccccct 6000
   acccctgcat gaagtcacag atctagtgcc cttttgcagt ctcttcttcc ttttatcaga 6060
   tatttctaat agcaaaaatc tggaagacct aaatgccaag caaaagagtt gttaaataaa 6120
   tcacaggaca gccaaatagt aggatatagt gtagccattt aaaatgacaa catgggcggg 6180
   gaacggtggc tcacacctgt aatcccagaa ttttgggagg ctgaggtggg tagatcactt 6240
   gaggtcagga gttccagacc agcctggcca acatggtgaa accccttctc tactaaaaat 6300
   acaaaactta gctgggtgtg gtggcgcggg cctgtaatcc cagctcttgg gaggctgagg 6360
   caggtgaatc acttgaacct gggaggcgga ggttgcagtg agccaagatc gcgttattgc 6420
   actccagcct gggtgacaga gtgagaccct gtccccccac aaaataaaat aaaataaaat 6480
   aaaataaaat aaaataaaat aaaataaaat gacaacatag aaacagattc atcttctaga 6540
   acttgcattg ctcagtgaag aatcaggtta aagaacagtt tttagagtat atcctcattt 6600
   acacttatgt atgtctgtat gtgaacagcg tgtgtctgtg tgtgatcaca gaaagaaatc 6660
   caacaggatg tatgccaagg tgataatatt ttgctcctgc gtattttcta tttttctgta 6720
   gtgatcatgg attacctgtg caatttctac cccctcccaa caaaagaaca aatcttgctc 6780
   tcaggataaa ctctggcaaa attatttata ctgatgcata aatcatatgt aaagcccaac 6840
   agagacctct ttccagaggc atatttattt taatcagtca agaggggtaa gccaaaggaa 6900
   aatgccacaa aacaggtgga agagggtgtt atatactttt atttcatttt tgtatgttac 6960
   tgggagttct cagtcctttt aaaagtgcag ctcatccagg cagtcccagg ggatatggcc 7020
   acgagggctg tgctgtaggt gaagggcgct ctgggcctgg gcagtctctg ggccaaagtg 7080
   ggcaggtgtc agttcagaca ctccactgtg ggctgatctt tgctttctgt cttacagctc 7140
   ctgttgctgg gacacctggt aagtactcca cccacagcct ttccttccct cgggtcccct 7200
   tttcctctcc tcccagtgcc ctgtctggag gtgctctaaa tgcaccagca actgagccca 7260
   gtaccaagtc agggccagga aagggtaatt gctcccgaaa gatcccctct ccctcttcca 7320
   ctctcctcct tccttttcct ccttccacgg aggcaagtgg taggcagcca cggtgttcaa 7380
   atgggggagc tgtcacagaa aatgagaact ctgatctctc tctcaactgc cttccgctgt 7440
   ccaaataaaa ccaagggctt cctctctcca aataaaattc aggcacatgc aaaatcacat 7500
   ggcctgcata tgttgtcccc ctgtgttgct aaatccttga ctgtgcatgt ttctgagagc 7560
   tccaaggcct gttccctgac agagtggtct ctgcatacct ctggctacac tttgccgggc 7620
   acttctgtgt ttgtgccact cagcctccac cgctccagga ctggccctcc ggcttttctc 7680
   cctctctctt tatccttctg aggccagaga ggctcgtgta tgcttgggtg tgaggacggg 7740
   ccatcttgac ctcgttttta aagtatacag aatgacatta ggtcagaacg gaaacctgat 7800
   atccaactcc catgcctgcc atctccacca gagcccccac ttatcttcag ccagccccag 7860
   gggaaattta tccactagtg tctaaatgcc tttgtttatc ggggagaaaa gttagtctcg 7920
   aaagagtatg tatgtatggg gtgtctgtgt gtctgtgctg gtggggagag gttggtgggg 7980
   agagagagaa agaactggtg aggatatctc aggtaaagta tatgtatttc gagggtgttt 8040
   ctcttgcctg ctccctcctt cttccctgtt gccctgtcgg agtcctgggc tccctggggc 8100
   cgttgtggtg ggatgtatga gcaaaagcaa ctgccttcag ttgcagaacc attctgggcc 8160
   tggctcggct tttggtgccc ctagtaggcc tacaggtgct tttttgtctg agattcaggg 8220
   cctctcaagc tcctgggctt cctcttcttc atgctacctc ctctctctgc ccctcagcag 8280
   ctcccccaaa ggctgtgctg aaactcgagc cccagtggat caacgtgctc caggaggact 8340
   ctgtgactct gacatgccgg gggactcaca gccctgagag cgactccatt cagtggttcc 8400
   acaatgggaa tctcattccc acccacacgc agcccagcta caggttcaag gccaacaaca 8460
   atgacagcgg ggagtacacg tgccagactg gccagaccag cctcagcgac cctgtgcatc 8520
   tgactgtgct ttctggtcag tggaggaagg ccccagggtg gacctgggag ggccaggacg 8580
   gatgaaatct gctttcaggc agaggtttgc aggaaagggg ggtggcctgc ttactgggaa 8640
   gtatcgctgt gagttgcctc agcacatatc agtggttgtt tttgcctcag ttctgattga 8700
   acagaagaag gtttcaaggc caaaaacagg cagccaagtg tgagagaagc agaaggaaat 8760
   ccctactgca taaaacccat ttccatttta atggcagaat tgaaaagcac agaccacaac 8820
   tgaatcctag ccctggaaat gactcactat acaacatgat gaattcattt aacccttgag 8880
   tttccatttc ttcacctgct ccgtggggca ctaacgcctc cctcagaggc ttctggtgag 8940
   aatcagtgtt tccctgcccc cgccccgccc tccatgcccc ttctccacgt tctcactgtg 9000
   ctaggtgctc ttctctgtct ttctcttcca ccagcctgtg ggaaacctga gatgaaagtc 9060
   gtgtcttacc catctttgta tttccagcat ctgaaactgg gcagagctta ataaatattt 9120
   tgctggagag gttgatgatc ttacaaagct cccattgaaa ggtggctctc tgtaaagcaa 9180
   agttacaatg agattgtgat gaacattgtc cttgtggctt ttcacttagt cccctccctt 9240
   cacctgaaga gcaaattttc ctcaaaagta cacagcaaac gaatgaccca ctggtgacac 9300
   tgctgccttt agaccctgct ggaaagaagc tccacattta ttaacattcc cgaagtaaat 9360
   ttatcaggta gcattcatca ggtaacattt gttgcacatt catgactttt ctactgtcca 9420
   caaaggcata tgtccttatc atatgcggac tcctcggtca cactggattc ttccttccct 9480
   cctcgacatg gaagagatgg catcttaggg tctcttgtgt tcttcctgca gaggcctgtc 9540
   gggcaggaaa aggctgcagc tgccttcctg ggagaaggag gagatgagtg tatcctgaac 9600
   acctattatg tgctaggggc tattgtagat acatgacact atcatgctca ttttcacgaa 9660
   tgaggaaact gaggctcaga agacttaaat tatttgccca agagttcata aatgacagag 9720
   ccagcattag agtccaggac tgtctgattt cagacctaag ctgttccctc tgcacatcgt 9780
   gtcccaccag taaggaagat ctgggtctca gagctgagcc aagacctccc gggtcctctg 9840
   cggttttttg tgtctttcag agtggctggt gctccagacc cctcacctgg agttccagga 9900
   gggagaaacc atcgtgctga ggtgccacag ctggaaggac aagcctctgg tcaaggtcac 9960
   attcttccag aatggaaaat ccaagaaatt ttcccgttcg gatcccaact tctccatccc 10020
   acaagcaaac cacagtcaca gtggtgatta ccactgcaca ggaaacatag gctacacgct 10080
   gtactcatcc aagcctgtga ccatcactgt ccaaggtatg cggagtctgc caagatgtaa 10140
   ggaggggaga agaggggatg gacaagggct gaggtcacat gggcctacat ggaggtctga 10200
   gaaaggccac agcgcaaaat tgggcactgg agcaaagagg agtggtgtgg aggcctggct 10260
   aagtattgac caatgagcag gagtaggggc cagagcttgg agccctcagg tgataggtga 10320
   ccaggctgtt gttccacttt gaaatgcagg ccccagacta aggacggcag cgaagcagag 10380
   ctccctcgtt ggtgcagagg ttccctaagc tcctgggcat tcctaagaac tgaggtttgc 10440
   ctttattctt ctcatggctc atgttacagc cattcactcc agaaagcctg gcacgtcatg 10500
   gaccgttcaa ggctgtgctc catagagtaa tgatgcctcc agctatgcga ggctttgggc 10560
   ccacccttcc cactgcccct gagggctaag gggagccctt ccctctgttc ctgcctgctc 10620
   acaaatgtac ctttattagt ttggtggaga aacctcggta agcaggaggc ataagtccag 10680
   ccacagaaac cctgtgcaga tgaggctggg gatgtagtga gtgctgcaga agtgagtgac 10740
   tcagacacag aagagcttcg ggtgacaagc actaggacat agcattggat gggggggagg 10800
   tgggacaagg agagtactgc ctgtcctgat gtctgtcttc cctagctccc agctcttcac 10860
   cgatggggat cattgtggct gtggtcactg ggattgctgt agcggccatt gttgctgctg 10920
   tagtggcctt gatctactgc aggaaaaagc ggatttcagg tttgtagctc ctcccggtcc 10980
   ctttggttat cagtttccac ttggcccagg ccctaacccc agacattgcc agaatccctc 11040
   tctttgggct agatacacat tcagatctag gcccgtattg tattatagtc attcattcgt 11100
   ttattagatc attcatttga caagacttga acaaactagc ttatgtgtca gccaatgcgt 11160
   gagacatggt gggtgatgcc aagagaagtc cagtccctgc ccttggggag ttctcagcat 11220
   tgtgaggaag acagggtcat tataataaga tgggataaga gcaatgatag ggacatgaaa 11280
   agcctaggat agaagtacag aggaggctcc atagcccaac aagggctgag gcagttggag 11340
   aaggtcttgg ggagagcagt gtagtgtagt gatggagtgc acatagtggg gccagactgc 11400
   ctgagttcaa atctagttct acagcatacc aactatatga cccaagtgag tcactccacc 11460
   tctctgtgcc tcagctgtct caagtggaaa atggggacac taataggact tacctcagag 11520
   ggttgccatg gggatgaaag aggctgatgc tcttacacag tgcctggtag gcagtaacag 11580
   cacacaaaca gggctgttgc cattctggag gaggtggtgt gtgagggcag cgcttagcta 11640
   gggaaggagg accctccagg cactgggcca aggcagggat tgaagccggg tggggctcat 11700
   atgggtgtcg aagggggagt agagagggta ctgcaagcca ccaagaaaga aagaatcctg 11760
   ctaacacctc acaaagcact tttcaattgg ttcatagcca acacctcagt tactgatgat 11820
   aagtaaatac agagaaacag agagagagag actgagacac aggggtgttt ttaaaaggca 11880
   gagcaagcct cagacagatg ccatggcgtg gactttctga cactcctggg catccccatg 11940
   ggtgagctga attctgcctc tggaccagcc cttttccagg cgggagcagc ctctgagcag 12000
   gggagctggg ggtgggagga caggaaacat ctgccagagt gaaggcctga gctggtccca 12060
   tccaaccctg gccctggtcc ttggtcctga ggactcaggc cccaccgcct aatcctacta 12120
   acctcctgtg tgcccctccc agctctccca ggataccctg agtgcaggga aatgggagag 12180
   accctccctg agaaaccagg tgagtacagg ttgtctcagg gattcagtga tggctcacca 12240
   gggctgccgg ctggactgga gccagggaga aggggcttgt gcaagttcag ctgggagcca 12300
   gggagggagc agcagtggga ggatggctgg ggctcaaatc gcttggtcag ctcttagtct 12360
   aactcctggg cctaaagacg acctctctgg agatgagaag agaaacacca gtcccagata 12420
   cagaagagag ggctgtgtcc gaatttctgg taccaggaat ctggtgatat ttccagagca 12480
   agaaatcaga gatactttgg tcttcctgtg gagctttggc agagctggca aaggatgggg 12540
   ttaggggctg taagactgag gccaatagga cgtgggaggc aggagtctaa ggggaaagga 12600
   ggaggaggcc tggaaacccc ttgatttgct gaggaatctg cctctgtgga gggatggggt 12660
   ggagtggcca ggctcagctg tctgtggaac actaggagga ggtttgggct tgagaaaatt 12720
   ctgggggact aggggcacta gtgggctcta ctctgtgggc aaaactcagt tctgatgccc 12780
   atgcactcaa ggttgactga attttggcct ggttctgccc tcatcacaca gtggagccct 12840
   gagcaagtca gtctccctga tggggttcag tctcctcact gaggaaatga gtgggtggcc 12900
   agggtgacct ctcaggtctt ctagatttga cattctgaga accaggggcc acccaagccc 12960
   tggcccaggt gggagtgtgt aaaggaactt ccaggaggat gccaggcaac ttccagactg 13020
   tagttaaagt cagagcacaa aggaaaccag tgctttctct gggggcttcc atgacagtaa 13080
   gaacaggcag aaaaaagagg ctgtcaaaga ccccacctca gatggctgga gtatgattca 13140
   gagcagagcc aacagattta agagcttgag caaaatatca cttgttcagc tgtgcttgca 13200
   gcaaggtcgc atgtctgctg gccaggccca gtggtgatag gggcctgtgt tcacaccagg 13260
   gccaaaacag atctggaaaa aacaaagtcc aagcaactgg attatccatc ttgttgtgtg 13320
   gttctgtctg cactggcata agcacatttc acaaggcact tttgcagcca ggcattgggg 13380
   ctgcataaat ggttttttaa aaatcctttc ccaaaaggca aagctgggtt gctgggattc 13440
   ttgggaaatg taatagaatg ggaattcaga atgcggttta ctgcagaatt tacagcatgg 13500
   caataaaggc tctctatgtt cagggggtga gagaagagac tagatgcatt gtctcagatg 13560
   tctcctgact tcctgagaaa tgccactttg cagatatggg gctgcttttc acactggcta 13620
   agtcagttca tggcaaaata tgagggaatt cagtgtgttt cagacactaa ccaaagtcca 13680
   tccttccttt ctccttccgt ccctcccttc ctccctttct cactcactct ctctttcttt 13740
   ttaatgccat ggagtaccat atttttcaaa gttccaacaa agatgcacta catttagaca 13800
   atgtggaaat ggccctcagc cttctctccc tgaagtctgt ggccagcaaa aatctggact 13860
   ttacttcact ctgatgctga acacccccct cccccacctc ttccccaata tctcagatcc 13920
   caagcctccc tggtttgctt ctaggaaagc tcagcaattc cctgaaaaga atgcagctca 13980
   agtgactgct ccactttttc accttggcct ttgcggaagg ctgtggctgg gctaccctat 14040
   gcatcgatca atgaggtcat atttacccag tgcttggcct agaggcccaa gacagggtca 14100
   gcagtgcttg ctttggctct agtttgggcg ttggttttgc agcctcagca tcagcacagg 14160
   caggccctct tctccagcag tgctctggct gatatttctt ctttttccca cagccaatcc 14220
   cactaatcct gatgaggctg acaaagttgg ggtgagtgat cccagccatc tccccctccc 14280
   ttctcccctg ttgccttttc tgttgccttc tttcctaggc cctctctgtg gatccttact 14340
   gctggtttct gccttctcta ggctgagaac acaatcacct attcacttct catgcacccg 14400
   gatgctctgg aagagcctga tgaccagaac cgtatttagt ctccattgtc ttgcattggg 14460
   atttgagaag aaaatcagag agggaagatc tggtatttcc tggcctaaat tccccttggg 14520
   gaggacaggg agatgctgca gttccaaaag agaaggtttc ttccagagtc atctacctga 14580
   gtcctgaagc tccctgtcct gaaagccaca gacaatatgg tcccaaataa ccgactgcac 14640
   cttctgtgct tcagctcttc ttgacatcaa ggctcttccg ttccacatcc acacagccaa    14700
   tccaattaat caaaccactg ttattaacag ataatagcaa cttgggaaat gcttatgtta    14760
   caggttacgt gagaacaatc atgtaaatct atatgatttc agaaatgtta aaatagacta    14820
   acctctacca gcacattaaa agtgattgtt tctgggtgat aaaattattg atgattttta    14880
   ttttctttat ttttctataa agatcatata ttacttttat aataaaacat tataaaaaca    14940
   acattctgtt taccttttca aggctgtatt ggttggagtg tagactgaac tgcctggggt    15000
   ctgtttctct tcagtgatga gactcttagg aaggcaggaa tggataggat agggggagga    15060
   gaggagagat ggggatttag aatgtagagt gagtgcccct tttcttaaaa ctgaatacag    15120
   tcacgcacca cataatgatg tttagttcaa caacagactg catatatgat ggtgatccca    15180
   taaaattata ataccatatt tctattgtac cttttctatt cctatgttta gatatatgag    15240
   tacttaccat tgtgttacaa ttgcctaaag tattcagtac agtagcatgc tgtacaggtt    15300
   tgtagcctag gggcaatagg ctatacgcta cagcctaggt gtgtagtagg ccacaccatt    15360
   taggtttgta taagtacctg ctatgatgtt cacacaacaa aattgcctgc atttctcaaa    15420
   atgtatcccc atatttcaac aatgcatgac tgtactcttc tgccaatgac cttgtattct    15480
   tgtttccatg tcttcttctc tttcctccta tggcaaataa aacactgttt tgcaacacaa    15540

## Description

L'invention concerne des marqueurs membranaires et leur utilisation dans le cadre de l'identification de cellules infectées constituants le réservoir viral, notamment des cellules infectées par des rétrovirus.

Les traitements antirétroviraux améliorent considérablement le pronostic vital des patients infectés par le virus de l'immunodéficience humaine (VIH) en altérant notamment la capacité du virus à se multiplier.

Ces traitements ne sont cependant pas suffisamment efficaces pour être arrêtés car ils n'éliminent pas les réservoirs cellulaires du virus et leur interruption conduit quasi systématiquement à une réapparition du virus dans le sang, preuve que l'infection persiste.

La persistance de ces réservoirs viraux latents est donc un obstacle majeur au concept de guérison de l'infection par VIH et est devenue une question majeure dans les domaines cliniques et scientifiques.

Des travaux récents ont montré que les lymphocytes T CD4 quiescents une fois infectés chez les patients, constituent les principaux réservoirs de virus à même de persister sous traitements durant des années [Chun et al., 1997. Proc Natl Acad Sci U S A 94, 13193-13197; Finzi et al., 1997 Science 278, 1295-1300; Chomont et al., 2009 Nat Med 15, 893-900.]

Une méthode de détection de réservoirs de cellules infectées par un virus de l'immunodéficience chez les mammifères, basée sur la détection du marqueur p24, a été décrite (Petitjean et al., 2007 Journal of Clinical Virology 39(1): 1-8).

Toutefois, aucun marqueur permettant de différencier les cellules réservoir du VIH des cellules non infectées n'a été identifié à ce jour.

En l'absence de marqueurs permettant d'identifier ces réservoirs de virus, différentes stratégies visant à cibler leur persistance en les « purgeant » du virus qu'ils hébergent ont été testées. Elles n'ont cependant pas permis de reproduire chez les patients, leur efficacité observée en laboratoire. Il est possible que les conditions in vitro dans lesquelles ont été testées ces stratégies soient trop éloignées du « contexte patient ».

Aussi, existe-t-il un besoin d'identifier ces cellules afin d'éradiquer complètement l'infection virale.

L'invention se propose de pallier ces manques de l'état de l'art.

Un des buts de l'invention est de proposer l'utilisation de nouveaux marqueurs permettant d'identifier les cellules réservoirs.

Un autre but de l'invention est de fournir des méthodes d'identification et d'éradication des cellules réservoirs du virus.

L'invention concerne l'utilisation d'un marqueur cellulaire, exprimé à la surface des cellules lymphoïdes, et éventuellement myéloïdes, pour la détection de cellules réservoirs d'au moins un lentivirus, notamment un lentivirus, lesdites cellules réservoirs étant des cellules infectées par ledit lentivirus et étant insensibles aux agents thérapeutiques spécifiques dudit lentivirus. L'invention concerne en outre l'utilisation d'un moyen de détection d'un marqueur cellulaire, exprimé à la surface des cellules lymphoïdes, et éventuellement myéloïdes, pour la détection de cellules réservoirs d'au moins un lentivirus, notamment un lentivirus, lesdites cellules réservoirs étant des cellules infectées par ledit lentivirus et étant insensibles aux agents thérapeutiques spécifiques dudit lentivirus.

L'invention repose sur la constatation surprenante faite par les inventeurs que les marqueurs cellulaires, et notamment certains marqueurs des clusters de différentiations CD, sont spécifiquement exprimés, ou exprimés à un certain niveau par rapport au niveau d'expression desdits marqueurs dans une cellule non infectée par un lentivirus, par les cellules réservoirs d'au moins un lentivirus.

Par une approche décrite dans les exemples ci-après, les inventeurs ont mis en évidence que certains marqueurs CD spécifiques étaient particulièrement intéressants pour détecter les cellules réservoirs de lentivirus.

Dans l'invention, on entend par cellules réservoirs des cellules dans lesquelles des formes virales compétentes pour la réplication s'accumulent et persistent avec une cinétique de renouvellement plus lente que celle des formes virales se répliquant activement.

Les marqueurs particulièrement intéressants sont les suivants : le marqueur CD123 (Gene ID: 3563), le marqueur CD87 (Gene ID: 5329), le marqueur CD89 (Gene ID: 2204), le marqueur CD172a (Gene ID: 140885), le marqueur CD112 (Gene ID: 5819), le marqueur CD213a1 (Gene ID: 3597), le marqueur CD120b (Gene ID: 7133), le marqueur CD121a (Gene ID: 3554), le marqueur CD54 (Gene ID: 3383), le marqueur CD11c (Gene ID: 3687), le marqueur FPRL1 (Gene ID: 2358) et le marqueur CD32 (CD32a Gene ID: 2212; CD32b Gene ID: 2213).

Le marqueur ou antigène CD123 est également connu sous le nom de sous-unité alpha du récepteur de l'interleukine-3 humain. Il s'agit d'une glycoprotéine transmembranaire de type I et un membre de la superfamille des récepteurs de cytokines. Le CD123 forme un hétérodimère avec l'antigène CD131 (la sous-unité bêta du récepteur de l'interleukine 3) pour former le récepteur de l'interleukine-3. Au sein du récepteur, c'est l'antigène CD123 qui confère la spécificité vis-à-vis de la cytokine. Par exemple chez l'homme, cet antigène est codé par le gène contenu dans la séquence SEQ ID NO : 27. L'antigène est notamment constitué de la séquence d'acides aminés représentée par la séquence SEQ ID NO : 1, codée elle-même par la molécule d'acide nucléique (cDNA) représentée par la séquence SEQ ID NO : 14.

Le marqueur ou antigène CD87 est également connu comme le récepteur de l'urokinase activateur du plasminogène ou uPAR. Le CD87 est un acteur majeur impliqué dans la migration de l'endothélium angiogénique. Par exemple chez l'homme, cet antigène est codé par le gène contenu dans la séquence SEQ ID NO : 28. L'antigène est notamment constitué de la séquence d'acides aminés représentée par la séquence SEQ ID NO : 2, codée elle-même par la molécule d'acide nucléique (cDNA) représentée par la séquence SEQ ID NO : 15.

Le marqueur CD89 est un récepteur des régions constantes Fc des immunoglobulines de classe A (IgA). Récepteur glycoprotéique transmembranaire, il est exprimé à la surface des cellules de lignée myéloïde, telles que les cellules neutrophiles, les monocytes, les macrophages et les éosinophiles, au sein desquelles il participe à la médiation de la réponse immune contre les pathogènes. Par exemple chez l'homme, cet antigène est codé par le gène contenu dans la séquence SEQ ID NO : 29. L'antigène est notamment constitué de la séquence d'acides aminés représentée par la séquence SEQ ID NO : 3, codée elle-même par la molécule d'acide nucléique (cDNA) représentée par la séquence SEQ ID NO : 16.

L'antigène CD172a ou SIRP α (en anglais Signal regulatory protein a) est une glycoprotéine membranaire majoritairement exprimée par les cellules myéloïdes, les cellules souches et les neurones. Cet antigène agit comme un récepteur inhibiteur qui interagit avec l'antigène CD47 visant à réguler la phagocytose des cellules. Par exemple chez l'homme, cet antigène est codé par le gène contenu dans la séquence SEQ ID NO : 30. L'antigène est notamment constitué de la séquence d'acides aminés représentée par la séquence SEQ ID NO : 4, codée elle-même par la molécule d'acide nucléique (cDNA) représentée par la séquence SEQ ID NO : 17.

L'antigène CD112 est une glycoprotéine membranaire également connu sous le nom de nectine-2. Il est également connu sous les noms de PVRL2 (en anglais Poliovirus receptor-related 2) ou HVEB (en anglais herpesvirus entry mediator B), du fait de son implication dans l'entrée des virus de la polio ou de l'herpès Par exemple chez l'homme, cet antigène est codé par le gène contenu dans la séquence SEQ ID NO : 31. L'antigène est notamment constitué de la séquence d'acides aminés représentée par la séquence SEQ ID NO : 5, codée elle-même par la molécule d'acide nucléique (cDNA) représentée par la séquence SEQ ID NO : 18.

L'antigène CD213a1 est aussi connu comme étant la chaine alpha 1 du récepteur à l'interleukine 13 (IL13RA1). Cette sous-unité du récepteur participe à la transduction du signal par la voie JAK/STAT. Par exemple chez l'homme, cet antigène est codé par le gène contenu dans la séquence SEQ ID NO : 32. L'antigène est notamment constitué de la séquence d'acides aminés représentée par la séquence SEQ ID NO : 6, codée elle-même par la molécule d'acide nucléique (cDNA) représentée par la séquence SEQ ID NO : 19.

L'antigène CD120 correspond au récepteur 2 du facteur nécrosant de tumeurs (en anglais tumor necrosis factor receptor 2 -TNFR2). Par exemple chez l'homme, cet antigène est codé par le gène contenu dans la séquence SEQ ID NO : 33. L'antigène est notamment constitué de la séquence d'acides aminés représentée par la séquence SEQ ID NO : 7, codée elle-même par la molécule d'acide nucléique (cDNA) représentée par la séquence SEQ ID NO : 20.

L'antigène CD121a correspond au récepteur de type1 à l'interleukine 1 (IL1R1) et participe à la réponse immune et inflammatoire liée à l'IL1. Par exemple chez l'homme, cet antigène est codé par le gène contenu dans la séquence SEQ ID NO : 34. L'antigène est notamment constitué de la séquence d'acides aminés représentée par la séquence SEQ ID NO : 8, codée elle-même par la molécule d'acide nucléique (cDNA) représentée par la séquence SEQ ID NO : 21.

L'antigène CD54 est également connu sous le nom de molécule d'adhésion intercellulaire 1 (en anglais Intercellular Adhesion Molecule 1 ICAM-1). ICAM-1 est une glycoprotéine de la surface cellulaire exprimée sur les cellules endothéliales et les cellules du système immunitaire interagissant avec les intégrines. ICAM-1 est également utilisé par les rhinovirus pour leur entrée dans les cellules cibles. Par exemple chez l'homme, cet antigène est codé par le gène contenu dans la séquence SEQ ID NO : 35. L'antigène est notamment constitué de la séquence d'acides aminés représentée par la séquence SEQ ID NO : 9, codée elle-même par la molécule d'acide nucléique (cDNA) représentée par la séquence SEQ ID NO : 22.

L'antigène CD11c est une protéine transmembranaire de type I fortement exprimé sur les cellules dendritiques, mais également exprimé par les monocytes, les macrophages les neutrophiles et certaines cellules lymphocytaires B. Par exemple chez l'homme, cet antigène est codé par le gène contenu dans la séquence SEQ ID NO : 36. L'antigène est notamment constitué de la séquence d'acides aminés représentée par la séquence SEQ ID NO : 10, codée elle-même par la molécule d'acide nucléique (cDNA) représentée par la séquence SEQ ID NO : 23.

L'antigène FPRL1 est également connu sous le nom de N-formyl peptide receptor 2. C'est un récepteur de faible affinité pour les N-formyl-methionyl peptides qui sont des chimioattractants des neutrophiles induisant par la suite leur activation. Par exemple chez l'homme, cet antigène est codé par le gène contenu dans la séquence SEQ ID NO : 37. L'antigène est notamment constitué de la séquence d'acides aminés représentée par la séquence SEQ ID NO : 11, codée elle-même par la molécule d'acide nucléique (cDNA) représentée par la séquence SEQ ID NO : 24.

L'antigène CD32 est un récepteur membranaire des immunoglobulines G exprimé par une grande majorité de cellules lymphocytaires B. Le CD32 participe à la régulation de la production des immunoglobulines lors d'une surproduction. Par exemple chez l'homme, cet antigène est codé par le gène contenu dans la séquence SEQ ID NO : 38 ou SEQ ID NO : 39. L'antigène est notamment constitué de la séquence d'acides aminés représentée par la séquence SEQ ID NO : 12 (CD32a) ou SEQ ID NO : 13 (CD32b), codée elles-mêmes respectivement par la molécule d'acide nucléique (cDNA) représentée par la séquence SEQ ID NO : 25 ou SEQ ID NO : 26.

Dans l'invention, lorsqu'il est fait mention des molécules CD (cluster de différentiation) ces molécules seront indifféremment appelées « marqueur », « antigène » ou « molécules ».

Aussi, l'invention concerne avantageusement l'utilisation du marqueur CD89 ou d'un moyen de détection du marqueur susmentionné pour la détection de cellules réservoirs d'un lentivirus, lesdites cellules réservoirs étant telles que définies ci-dessus.

Dans l'invention, les lentivirus infectant les cellules réservoirs recherchées sont avantageusement des lentivirus responsables d'une immunodéficience chez les mammifères notamment les singes, notamment les hominidés, en particulier l'homme, et les félins, notamment le chat (i.e. les virus VIH 1 et 2, VIS et VIF respectivement).

Aussi, l'invention concerne avantageusement l'utilisation du marqueur CD89 pour la détection de cellules réservoirs d'un virus de l'immunodéficience chez les mammifères, notamment un lentivirus, lesdites cellules réservoirs étant des cellules infectées par ledit virus de l'immunodéficience chez les mammifères et insensibles aux agents thérapeutiques anti-virus de l'immunodéficience chez les mammifères.

Dans l'invention, il est possible d'utiliser l'un des marqueurs susmentionnés, ou une combinaison des marqueurs susmentionnés.

Il est notamment particulièrement avantageux d'utiliser le marqueur CD89, seul ou en combinaison avec l'un au moins des marqueurs susmentionnés pour la sélection susmentionnée.

Aussi, l'invention concerne avantageusement l'utilisation d'au moins le marqueur de différentiation CD89, notamment l'utilisation du seul marqueur CD89, pour la détection de cellules réservoirs d'un virus de l'immunodéficience chez les mammifères, notamment un lentivirus, lesdites cellules réservoirs étant des cellules infectées par ledit virus de l'immunodéficience chez les mammifères et insensibles aux agents thérapeutiques anti-virus de l'immunodéficience chez les mammifères, ces cellules étant responsables du rebond viral après arrêt de la thérapie, notamment la trithérapie ou multithérapie chez l'homme.

Dans un mode de réalisation avantageux, l'invention concerne l'utilisation susmentionnée, où ledit marqueur CD89 consiste en la protéine de séquence SEQ ID NO:3.

Comme démontré dans les exemples ci-après, les inventeurs ont identifié le marqueur CD89 comme étant spécifiquement exprimé à la surface des cellules réservoirs, cellules infectées par les lentivirus notamment responsables d'une immunodéficience chez les mammifères.

Dans l'invention, on entend par « insensibles aux agents thérapeutiques » le fait, notamment pour les cellules infectées par le virus de l'immunodéficience, de ne pas répondre significativement à l'effet d'un traitement médicamenteux. L'insensibilité aux agents thérapeutiques peut se caractériser par une absence complète de réponse, due à une latence virale ou à l'inaccessibilité aux agents thérapeutiques, ce qui signifie que l'effet d'un ou plusieurs médicaments est essentiellement le même que celui d'une composition n'ayant aucun agent thérapeutique (véhicule pharmaceutiquement acceptable, excipient, eau, ou encore un placébo). L'insensibilité peut également se caractériser par une réponse qui n'est pas significativement distincte de celle obtenue avec composition n'ayant aucun agent thérapeutique.

Les cellules réservoirs de l'invention sont dites insensibles aux agents thérapeutiques antiviraux, car, avec les thérapies actuelles utilisées pour l'éradication des virus, ces cellules ne sont ni tuées, ni détruites, ni induites dans un processus d'apoptose ou de nécrose, ni reconnues comme faisant partie du non soi par le système immunitaire. Dans le cas particulier des virus de l'immunodéficience tels que le VIH (VIH1 ou VIH2), le VIS ou le VIF, les cellules réservoirs ne sont pas éliminées par les agents antirétroviraux actuellement utilisés (inhibiteurs d'entrée, inhibiteurs de transcriptase inverse, anti protéases, nucléotides modifiés...)

L'invention concerne en outre une méthode de détection de cellules réservoirs de cellules infectées par au moins un virus de l'immunodéficience chez les mammifères ou des cellules réservoirs de lentivirus, ladite méthode comprenant une étape de mise en contact, notamment *in vitro* ou ex *vivo,* de cellules d'un mammifère infecté par ledit virus de l'immunodéficience chez les mammifères et notamment traités avec une thérapie antirétrovirale spécifique dudit virus de l'immunodéficience chez les mammifères, avec un agent permettant la détection des cellules lymphocytaires exprimant à leur surface au moins le marqueur CD89.

Par « cellules réservoirs de cellules infectées par au moins un virus » on entend dans l'invention une sous-population de cellules infectée par ledit au moins un virus, cette population faisant partie de la population des cellules infectées. Ces cellules réservoirs étant telles que définies précédemment. « Cellules réservoirs de cellules infectées par au moins un virus » signifie également cellules réservoirs d'au moins un virus.

Dans la méthode susmentionnée, il est proposé de détecter les cellules réservoirs de lentivirus, en mettant en contact des cellules lymphocytaires d'un sujet (humain ou animal) avec un agent capable de mettre en évidence spécifiquement des cellules exprimant l'un au moins des marqueurs susmentionnés. Pour ce faire, les méthodes immunologiques classiques d'isolement des cellules lymphocytaires, telle que la cytométrie de flux bien connue de l'homme du métier, peuvent être utilisées.

L'agent permettant la détection dudit marqueur est avantageusement un ou plusieurs anticorps. Cet anticorps, ou ces anticorps si la détection simultanée de plusieurs marqueurs est recherchée, ou encore si plusieurs anticorps du même marqueur sont utilisés, peuvent être couplés à un des agents permettant la détection du complexe immun entre les anticorps et leurs cibles. Par exemple, les anticorps peuvent être couplés à des molécules luminescentes, fluorescentes, ou à des enzymes catalysant une réaction dont les produits sont visibles par l'expérimentateur. Ces techniques de détection des complexes immuns sont très largement connues de l'état de la technique.

Avantageusement, l'invention concerne une méthode de détection de cellules réservoirs de cellules infectées par au moins un virus de l'immunodéficience, notamment un virus, chez les mammifères, ladite méthode comprenant une étape de mise en contact, notamment in vitro ou ex vivo, de cellules d'un mammifère infectées par ledit virus de l'immunodéficience chez les mammifères et traitées avec une thérapie antirétrovirale spécifique dudit virus de l'immunodéficience chez les mammifères, avec un agent permettant la détection des cellules lymphocytaires exprimant à leur surface au moins le marqueur de différentiation CD89.

Dans un mode de réalisation avantageux, l'invention concerne la méthode susmentionnée, où ledit marqueur CD89 consiste en la protéine de séquence SEQ ID NO:3.

Avantageusement, l'invention concerne une méthode telle que définie ci-dessus, dans laquelle ledit agent permettant la détection des cellules lymphocytaires ou myéloïdes exprimant à sa surface le marqueur de différentiation CD89 est un anticorps anti-CD89.

Dans un mode de réalisation avantageux, l'invention concerne une méthode telle que définie ci-dessus, dans laquelle ledit virus de l'immunodéficience chez les mammifères est le virus de l'immunodéficience humaine VIH, notamment le virus VIH1 ou le virus VIH2, de l'immunodéficience simienne VIS ou de l'immunodéficience féline VIF.

Dans un mode de réalisation avantageux, l'invention concerne une méthode telle que définie ci-dessus, dans laquelle les cellules lymphocytaires exprimant à leur surface le marqueur de différentiation CD89 sont des cellules lymphocytaires TCD4, notamment des cellules TCD4 quiescentes. Ces lymphocytes quiescents sont caractérisés par le fait qu'ils ne se divisent pas, ont une durée de vie longue (semaine-années), un taux de transcription bas et une activité métabolique qui leur est propre.

L'invention concerne également un kit, ou trousse, de détection de cellules réservoirs d'un virus de l'immunodéficience chez les mammifères comprenant au moins un agent détectant, ou permettant la détection, le marqueur CD89 à la surface des cellules et au moins une composition permettant de déterminer la présence d'ADN dudit virus de l'immunodéficience chez les mammifères dans le génome desdites cellules.

Le kit de détection susmentionné comprend donc des moyens de détecter l'expression membranaire d'au moins un des marqueurs susmentionnés, et des moyens de détection de l'insertion dans le génome de la cellule l'ADN d'un lentivirus responsable d'une immunodéficience. Les moyens de détection des marqueurs sont ceux décrits précédemment, notamment des anticorps spécifiques desdits marqueurs.

Avantageusement, l'invention concerne également un kit, ou trousse, de détection de cellules réservoirs d'un virus de l'immunodéficience chez les mammifères comprenant au moins un agent détectant le marqueur de différentiation CD89 à la surface des cellules et au moins une composition permettant de déterminer la présence d'ADN dudit virus de l'immunodéficience chez les mammifères dans le génome desdites cellules.

Dans un mode de réalisation avantageux, l'invention concerne le kit susmentionné, où ledit marqueur CD89 consiste en la protéine de séquence SEQ ID NO : 3.

Avantageusement, l'invention concerne un kit tel que défini ci-dessus, dans lequel ladite composition permettant de déterminer la présence d'ADN dudit virus de l'immunodéficience chez les mammifères dans le génome desdites cellules comprend des oligonucléotides spécifiques de séquences du génome dudit virus, notamment pour la mise en œuvre d'une réaction de polymérisation en chaine, ou PCR.

Au vu de la connaissance accumulée depuis la découverte du virus VIH, du virus VIS et du virus VIF, l'homme du métier est à même d'identifier des oligonucléotides permettant d'identifier la présence desdits virus dans le génome de la cellule. L'utilisation de couples d'oligonucléotides permettant la mise en œuvre d'une réaction de PCR est particulièrement avantageuse, mais l'homme du métier peut, s'il le souhaite, faire appel à d'autres techniques de biologie moléculaire telles que l'hybridation de Southern (en anglais Southern blot).

Dans un autre aspect, l'invention concerne une méthode de pronostic, notamment *in vitro,* de la rechute d'une immunodéficience chez les mammifères suite à l'arrêt d'un traitement avec des agents thérapeutiques anti-virus responsable de ladite immunodéficience chez les mammifères, ladite méthode comprenant une étape de quantification des cellules lymphocytaires exprimant à leur surface l'un au moins le marqueur CD89 et présentant dans leur génome de l'ADN d'un virus responsable de ladite immunodéficience chez les mammifères.

La méthode de pronostic de l'invention est basée sur la détection, ou l'absence de détection de cellules réservoirs de lentivirus.

Après l'arrêt du traitement antiviral dirigé contre le VIH, le virus émerge à nouveau et la pathologie (l'immunodéficience) réapparait. Si le patient est traité à la fois avec un traitement antiviral, et un traitement visant à éradiquer les cellules réservoir, il est nécessaire, après l'arrêt du traitement antiviral de déterminer les chances qu'à le patient à voir ré-émerger la maladie, du fait de la réactivation du virus. Aussi, dans le contexte de l'invention, avec les moyens décrits pour détecter les cellules réservoirs, il sera possible de mesurer la quantité de cellules réservoirs résiduelles après les traitements susmentionnés (anti-virus et anti cellules réservoirs). Si le patient dispose d'une très faible quantité de cellules réservoirs, ou s'il ne dispose plus de cellules réservoirs, dans ce cas le pronostic sera favorable : la maladie aura peu de chances de ré-émerger à court terme. A l'inverse, si de nombreuses cellules réservoirs sont encore présentes, le diagnostic ne sera pas favorable, et la rechute du patient sera rapide.

Avantageusement, l'invention concerne par ailleurs une méthode de pronostic, notamment *in vitro,* de la rechute d'une immunodéficience chez les mammifères suite à l'arrêt d'un traitement avec des agents thérapeutiques anti-virus responsable de ladite immunodéficience chez les mammifères, ladite méthode comprenant une étape de quantification des cellules lymphocytaires exprimant à leur surface au moins le marqueur CD89 et présentant dans leur génome de l'ADN d'un virus responsable de ladite immunodéficience chez les mammifères.

Dans un mode de réalisation avantageux, l'invention concerne la méthode susmentionnée, où ledit marqueur CD89 consiste en la protéine de séquence SEQ ID NO:3.

L'invention concerne également une méthode de diagnostic, notamment *in vitro,* de la rémission complète d'une immunodéficience chez les mammifères après l'arrêt d'un traitement avec des agents thérapeutiques anti-virus responsable de ladite immunodéficience chez les mammifères, ladite méthode comprenant une étape de de détection de l'absence de cellules lymphocytaires exprimant à leur surface l'un au moins le marqueur CD89 et présentant dans leur génome de l'ADN d'un virus de l'immunodéficience chez les mammifères.

Dans cette méthode de diagnostic, si le patient n'a plus de cellules réservoirs du VIH dans son organisme, il sera possible de poser le diagnostic de rémission complète de l'infection virale, et donc de l'immunodéficience qui est associée. La détection de l'absence de cellules réservoirs est bien évidemment effectuée par les moyens de détection selon l'invention.

Avantageusement, l'invention concerne également une méthode de diagnostic, notamment *in vitro,* de la rémission complète d'une immunodéficience chez les mammifères après l'arrêt d'un traitement avec des agents thérapeutiques anti-virus responsable de ladite immunodéficience chez les mammifères, ladite méthode comprenant une étape de détection de l'absence de cellules lymphocytaires présentant dans leur génome de l'ADN d'un virus de l'immunodéficience chez les mammifères et exprimant à leur surface au moins le marqueur CD89.

Dans un mode de réalisation avantageux, l'invention concerne la méthode susmentionnée, où ledit marqueur CD89 consiste en la protéine de séquence SEQ ID NO:3.

L'invention concerne également une méthode de détection de cellules réservoirs de cellules infectées par un virus de l'immunodéficience chez les mammifères, ladite méthode comprenant une étape de mise en contact, *in vitro* ou *ex vivo,* de cellules d'un mammifère infectées par ledit virus de l'immunodéficience chez les mammifères, et notamment traitées avec une thérapie antirétrovirale spécifique dudit virus de l'immunodéficience chez les mammifères et/ou une thérapie visant à éradiquer lesdites cellules réservoirs, avec un agent permettant la détection des cellules lymphocytaires exprimant à leur surface un marqueur de différentiation susmentionné, notamment le marqueur CD89.

L'invention concerne par ailleurs une méthode d'évaluation de l'efficacité d'un traitement visant à éradiquer des cellules réservoirs de cellules d'un mammifère infectées par ledit virus de l'immunodéficience chez les mammifères, ladite méthode comprenant une étape de détection in vitro ou ex vivo desdites cellules de réservoir avec un agent permettant la détection de la présente la quantité ou l'absence de cellules lymphocytaires exprimant à leur surface le marqueur de différentiation CD89.

Avantageusement, l'invention concerne la méthode susmentionnée, comprenant en outre une étape de classification desdits mammifères infectés selon la présence, l'absence ou la quantité de cellules réservoir détectées.

Dans cette méthode, les cellules réservoirs sont recherchées à l'issue d'un traitement visant à les détruire. Si les cellules réservoir sont toujours présentes dans un nombre substantiellement identique à celui avant ledit traitement visant à les éradiquer, le traitement n'a alors pas fonctionné. Si par contre le nombre de cellules réservoir a significativement diminué, dans ce cas le traitement est opérationnel, notamment lorsqu'il n'est plus possible d'identifier de cellules réservoir.

Par ailleurs l'invention concerne un Anticorps multispécifique reconnaissant d'une part au moins un épitope du marqueur CD89 et au moins un marqueur caractéristique des cellules lymphocytaires, notamment les cellules lymphocytaires, notamment les cellules lymphocytaires T.

Plus avantageusement, l'invention concerne l'anticorps multispécifique susmentionné, où ledit anticorps est un anticorps bispécifique, reconnaissant spécifiquement l'antigène CD89 et l'antigène CD3.

Avantageusement, les anticorps susmentionnés sont dans le cadre de l'invention monoclonaux, notamment humain ou humanisé, de singe ou siminisé, ou de chat ou felinisés.

Par anticorps multispécifique on entend dans l'invention un anticorps capable de reconnaître un premier antigène par son premier CDR et un second antigène par son second CDR.

Avantageusement, l'anticorps défini ci-dessus est un anticorps bifonctionnel, ou bispécifique, reconnaissant l'antigène CD89 et l'antigène CD3.

Par anticorps bifonctionnel, ou bispécifique, on entend dans l'invention, un anticorps recombinant présentant une partie hyper variable reconnaissant un épitope déterminé, et une seconde partie hypervariable reconnaissant un second épitope, différent du premier. Ainsi, un anticorps bifonctionnel, ou bispécifique, est capable de reconnaître simultanément deux protéines différentes. Ces anticorps peuvent également se trouver sous la forme de leurs fragments tels que des scFv bispécifiques, des bi scFv bispécifiques, des minicorps (minibody en anglais) bi spécifiques ou encore des diacorps (diabody en anglais) bispécifiques.

Ces anticorps, ou leurs fragments, bispécifiques sont avantageusement humanisés pour une utilisation en thérapie humaine. L'humanisation, bien connue de l'état de l'art, consiste à remplacer toute la structure de l'anticorps par des molécules de séquence humaine auxquelles sont greffées les régions hypervariables d'intérêt, généralement issues d'anticorps monoclonaux de souris.

Aussi, de manière plus avantageuse, l'invention concerne un anticorps monoclonal, notamment humanisé, ou l'un de ses fragments susmentionnés, capable de reconnaître simultanément, d'une part le marqueur CD89 et d'autre part le marqueur CD3.

Dans l'invention, les anticorps utilisés peuvent être couplés à des agents actifs tels que des toxines (par exemple la ricine ou le DM1 dérivé maytansine), des radioisotopes (par exemple l'yttrium 90 ou ⁹⁰Y (émetteur pur de rayonnement bêta moins) et l'iode 131 ou ¹³¹I (émetteur bêta moins et gamma)), des agents biologiques, des drogues ou encore des enzymes. L'efficacité thérapeutique repose alors principalement sur l'agent couplé, l'anticorps jouant le rôle de vecteur pour guider l'agent vers la cible.

L'invention concerne également un anticorps bispécifique dirigé d'une part contre le marqueur CD89 et d'autre part contre un marqueur spécifique des cellules T, notamment le marqueur CD3, pour son utilisation dans le cadre du traitement d'une infection par un lentivirus, notamment une infection liée au VIH ou au VIS.

De manière plus avantageuse, l'invention concerne un anticorps bispécifique dirigé contre le CD89 et le CD3 pour son utilisation dans le cadre du traitement d'une infection par un lentivirus, notamment une infection liée au VIH ou au VIS ou au VIF.

L'invention concerne en outre une composition comprenant au moins un agent reconnaissant les cellules lymphocytaires exprimant le marqueur CD89, pour son utilisation pour l'éradication de cellules réservoirs de cellules infectées par un virus de l'immunodéficience chez les mammifères.

Avantageusement, l'invention concerne la composition susmentionnée, où ledit agent est au moins un anticorps reconnaissant le marqueur CD89, notamment un anticorps monospécifique du marqueur CD89, ou un anticorps multispécifique reconnaissant au moins un épitope du marqueur CD89 et au moins un marqueur caractéristique des cellules lymphocytaires, notamment les cellules lymphocytaires T, pour son utilisation susmentionnée.

Comme indiqué ci-dessus, les anticorps couplés à des agents toxiques peuvent être avantageux pour éradiquer les cellules réservoirs.

De manière avantageuse, l'invention concerne une composition pour son utilisation susmentionnée, dans laquelle l'anticorps spécifique et la combinaison d'agents antirétroviraux sont utilisées simultanément, séparément ou étalée dans le temps.

Il est également décrit une méthode de traitement des infections par des lentivirus, notamment des lentivirus provoquant une immunodéficience chez les mammifères, comprenant l'administration à un mammifère infecté, notamment un humain infecté par le VIH, une dose efficace de la composition susmentionnée.

L'invention concerne par ailleurs un anticorps reconnaissant spécifiquement le marqueur CD89 exprimés sur les cellules lymphocytaires réservoir des lentivirus, pour son utilisation dans le cadre du traitement d'une infection par un lentivirus, notamment une infection liée au VIH, au VIS ou au VIF.

Avantageusement, l'anticorps susmentionné est un anticorps anti CD89, éventuellement couplé à un agent cytotoxique, ou un anticorps au moins bispécifique reconnaissant outre le marqueur CD89 et au moins un autre marqueur spécifique des cellules lymphocytaires, notamment le marqueur CD3.

L'invention concerna aussi une composition susmentionnée, pour son utilisation dans le cadre du traitement d'une infection par un lentivirus, notamment une infection liée au VIH, au VIS ou au VIF.

Avantageusement, l'invention concerne une composition pour son utilisation susmentionnée, dans laquelle l'anticorps spécifique et la combinaison d'agents antirétroviraux sont utilisées simultanément, séparément ou étalée dans le temps.

L'invention sera mieux comprise à la lumière des données expérimentales exposées ci-après et des figures qui les illustrent.

### Légende des figures

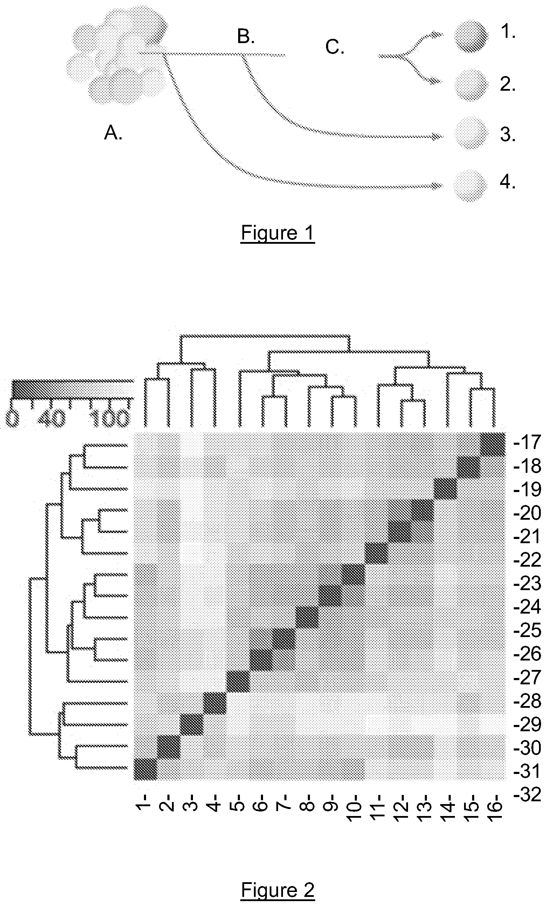

La **Figure 1** représente l'approche expérimentale utilisée par les inventeurs pour obtenir des lymphocytes TCD4 infectés de manière latente. A. : Les cellules mononuclées de sang périphérique (PBMC en anglais) d'individus sains (non infectés par le VIH) sont prélevées à t0. Sans plus de traitements elles constituent les cellules contrôles 4 (Non infectées ; NI). Le reste des PBMC est traité avec la protéine Vpx (VLP-Vpx ; B.). Les cellules qui ne sont pas traitées subséqemment constituent le second témoin 3.de cellules traitées avec Vpx et non infectées.
   Enfin les cellules restrantes sont infectées avec un virus VIH permettant l'expression de manière constitutive de la GFP (C.). La fraction des cellules exposée au VIH se divise alors en deux catégories : les cellules exposées non infectées n'exprimant pas la GFP (2.) et les cellules exposées au virus ; infectées et exprimant la GFP (1.).
   Les différentes cellules sont triées à l'aide d'un cytomètre de flux en vu de l'extraction de leurs ARN, le séquençage desdits ARN extraits, d'analyses bio-informatique et de la validation des candidats par cytométrie de flux.
La **figure 2** est un graphique coloré (ici en noir et blanc) de regroupement hiérarchique effectuée sur la distance euclidienne et calculée en utilisant les comptages d'expression génique transformés et logarithmiquement régularisés (en anglais "regularized-log transformed gene expression counts"), entre chaque sous-ensembles de cellules 1. à 3. et non infectées (4.). Le graphique représente les résultats pour des PBMC issus de quatre donneurs différents.
   1- : Cellules 1. issues de l'individu 2 ; 2- : Cellules 1. issues de l'individu 4 ; 3- : Cellules 1. issues de l'individu 1 ; 4- : Cellules 1. issues de l'individu 3 ; 5- : Cellules 4. issues de l'individu 4 ; 6- : Cellules 3. issues de l'individu 1 ; 7- : Cellules 2. issues de l'individu 1 ; 8- : Cellules 4. issues de l'individu 1 ; 9- : Cellules 3. issues de l'individu 2 ; 10- : Cellules 2. issues de l'individu 2 ; 11- : Cellules 4. issues de l'individu 3 ; 12- : Cellules 3. issues de l'individu 4 ; 13- : Cellules 2. issues de l'individu 4 ; 14- : Cellules 4. issues de l'individu 2 ; 15- : Cellules 3. issues de l'individu 3 ; 16- : Cellules 2. issues de l'individu 3 ; 17- : Cellules 2. issues de l'individu 3 ; 18- : Cellules 3. issues de l'individu 3 ; 19- : Cellules 4. issues de l'individu 2 ; 20- : Cellules 2. issues de l'individu 4 ; 21- : Cellules 3. issues de l'individu 4 ; 22- : Cellules 4. issues de l'individu 3 ; 23- : Cellules 2. issues de l'individu 2 ; 24- : Cellules 3. issues de l'individu 2 ; 25- : Cellules 4. issues de l'individu 1 ; 26- : Cellules 2. issues de l'individu 1 ; 27- : Cellules 3. issues de l'individu 1 ; 28- : Cellules 4. issues de l'individu 4 ; 29- : Cellules 1. issues de l'individu 3 ; 30- : Cellules 1. issues de l'individu 1 ; 31- : Cellules 1. issues de l'individu 4 et 32- : Cellules 1. issues de l'individu 2.

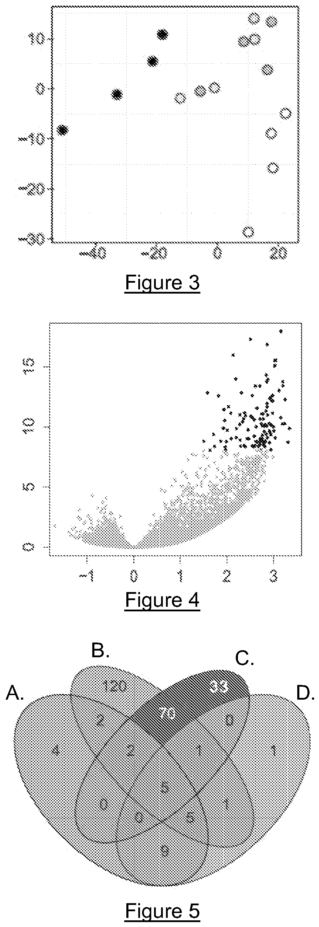

La **figure 3** est un graphique montrant l'analyse en composantes principales des "regularized-log transformed gene expression counts" pour les sous-groupes 1. (points noirs), 2. (points gris foncés), 3. (points gris clairs) et 4.(points blancs) de 4 donneurs sains. Les points de deux premières composantes principales sont présentés. L'axe des abscisses représente PC2 : 17% de la variance et l'axe des ordonnées représente PC1 : 58% de la variance.
La **figure 4** représente un graphique Volcano présentant la significativité (False Discovery Rate, FDR) et la modification d'expression génique entre les cellules TCD4 infectées (1.) et les cellules TCD4 exposées non infectées (2.). Les points noirs indiquent les gènes sélectionnés du fait de leur sur-expression significative dans la fraction 1. comparée aux cellules 2. (FDR <10-8). L'axe des abscisses représente le logarithme de base 2 de la modification d'expression et l'axe des ordonnées représente le logarithme de base 10 de FDR.
La **figure 5** représente un diagramme de Venn montrant les 253 gènes exprimés de manière différentielle entre les 4 groupes et notamment 2. vs 1. (FDR <10-8). Les intersections grises foncées indiquent des gènes sélectionnés pour une analyse ultérieure. A : 4. vs 3. ; B : 4. vs 1. ; C : 2. vs 1. et D : 4. vs 2.

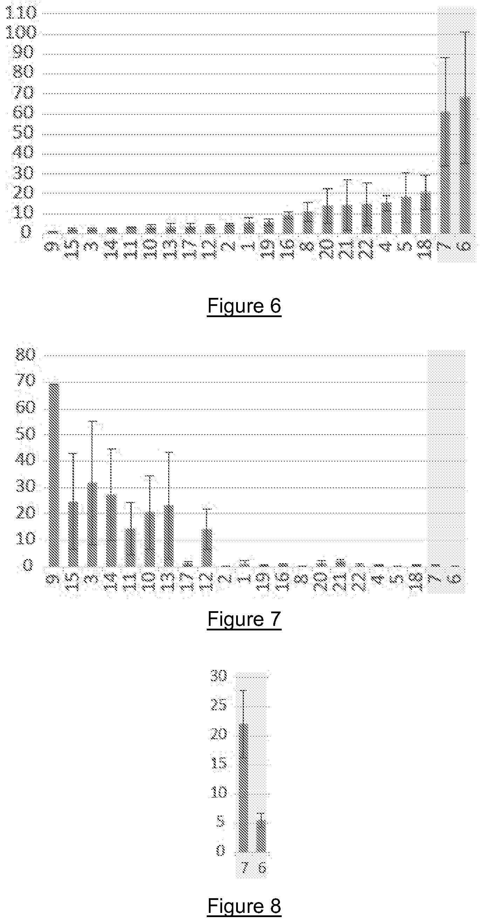

La **figure 6** représente un histogramme montrant les résultats de cytométrie d'augmentation d'expression (axe des abscisses) pour chacun des marqueurs évalués (gènes 1 à 22 sur les 111 candidats potentiels) sur les cellules TCD4 1. et 2.
   Les marqueurs sont les suivants : 9 : aqp9, 15 : mucl1, 3 : ca12, 14 : vnn3, 11 : eaat1, 10 : c22orf42, 13 : gpr91, 17 : cd66d, 12 : step1b, 2 : gjb2, 1 : colec12, 19 : cd80, 16 : niacr1, 8 : cd354, 20 : cd116, 21 : scarf1, 22 :llrk2, 4 : cd300c, 5 : clec4d, 18 : tlr2, 7 : cd32 et 6 : fprl1
La **figure 7** représente un histogramme montrant le pourcentage des cellules TCD4 totales 2. exprimant chaque marqueur afin de mettre en évidence l'expression des candidats les plus fiables sur les cellules 1. Les marqueurs sont les suivants : 9 : aqp9, 15 : mucl1, 3 : ca12, 14 : vnn3, 11 : eaat1, 10 : c22orf42, 13 : gpr91, 17 : cd66d, 12 : step1b, 2 : gjb2, 1 : colec12, 19 : cd80, 16 : niacr1, 8 : cd354, 20 : cd116, 21 : scarf1, 22 :llrk2, 4 : cd300c, 5 : clec4d, 18 : tlr2, 7 : cd32 et 6 : fprl1.
La **figure 8** représente un histogramme montrant le pourcentage de cellules TCD4 total 1. exprimant le marqueur #7 (CD32) et le marqueur #6 (fprl1).

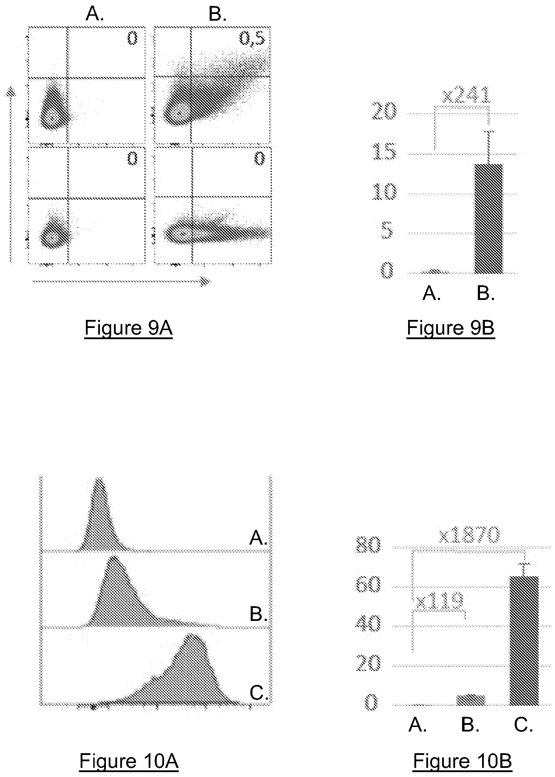

Les **figures 9A et 9B** représentent les profils d'expression du marqueur #7 (CD32) dans le modèle de latence virale développé par les inventeurs.
   La figure 9A représente les résultats de cytométrie des cellules TCD4 infectées de façon latente générées comme représenté sur la figure 1. L'expression du marqueur #7 (CD32) a été évaluée par cytométrie en flux à la surface des cellules TCD4 infectées (1.; B.) et des cellules TCD4 quiescentes non infectées 2. (A.) (n=3). Les pourcentages GFP+/marker #7+ (CD32+) sont indiqués sur chaque panel. L'axe des abscisses représente l'intensité de fluorescence de la GFP, et l'axe des ordonnées représente l'intensité de fluorescence de marqueur CD32.
   La figure 9B représente un histogramme indiquent le pourcentage de cellules exprimant le marqueur #7+ (CD32+) entre cellules 2. (GFPneg ; A.) et 4 (GFPpos ; B.).
Les **figures 10A et 10B** montrent l'intensité de fluorescence du marqueur #7 (CD32) est corrélée à l'intensité de l'expression de la GFP dans les cellules TCD4 infectées (n = 3).
   La figure 10A montre des représentations du nombre de cellules en fonction de l'intensité de fluorescence du marqueur CD32 pour trois sous-groupes des cellules : A. les cellules n'exprimant pas la GFP, B. : les cellules exprimant faiblement la GFP et C : les cellules exprimant fortement la GFP.
   La figure 10B représente un graphique le pourcentage de marqueur CD32 sur les cellules TCD4 pour les 3 catégories A. B. et C. décrites à la figure 10A.

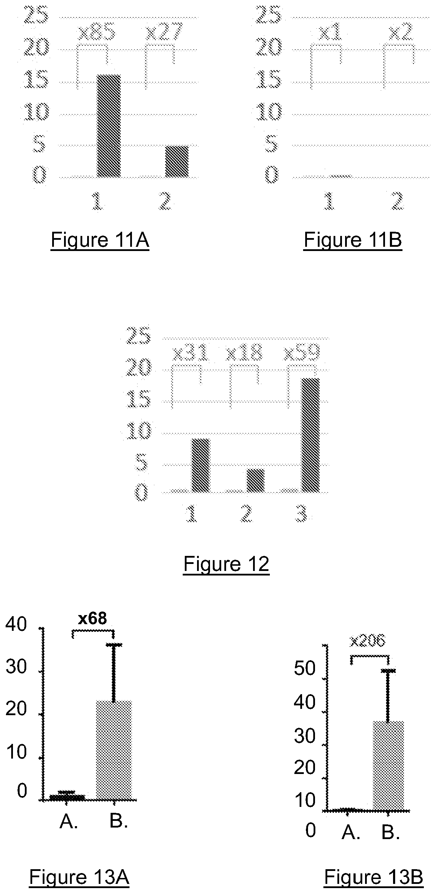

Les **figures 11A et 11B** représentent des graphiques montrant l'expression du marqueur #7 (CD32) à la surface populations de cellules TCD4 non stimulées (11A) ou stimulées par la voie TCR (n=2) (11B). Les barres noires représentent le pourcentage de cellules GFP positives et les barres grises le pourcentage de cellules GFP négatives.
La **figure 12** représente un graphique montrant que l'utilisation de particules virales pseudotypés VSV-G permet de mettre en évidence une induction de l'expression du marqueur #7 (CD32) sur des cellules telles que les TCD8 (n = 2). La moyenne et les IQR sont présentés sur chaque histogramme ainsi que l'augmentation entre les cellules 2. (GFPneg) et 4. (GFP+) lorsque cela est nécessaire.
Les **figures 13** **A et 13B** montrent l'analyse par cytométrie de l'induction du marqueur #7 (CD32) à la surface des cellules TCD4 quiescentes infectées (GFP+) en comparaison avec des cellules non infectées ou traitées avec VLP-Vpx seul.
   Des PBMC de donneurs sains (n=3) ont été infectées par les virus SIVmac239 (13A) et VIH-2 (13B). Les résultats de l'analyses FACS sont représentés en % de cellules exprimant le marqueur #7 (CD32) dans les populations non-infectées (GFP- ; A.) et infectées (GFP+ ; B.). Les histogrammes présentent la moyenne et la déviation standard pour les expériences réalisées avec SIVmac239 et VIH-2.

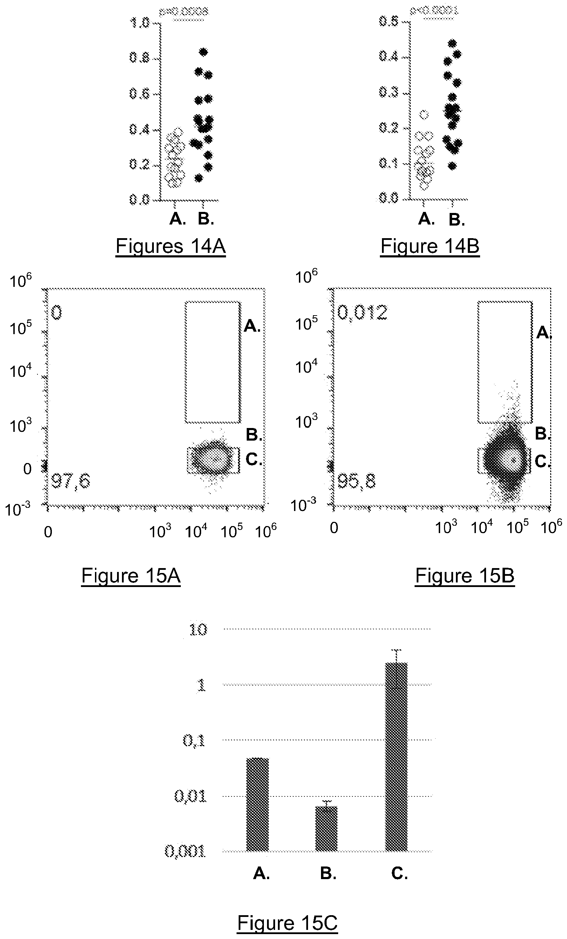

Les **figures 14A et 14B** représentent des histogrammes montrant la comparaison du niveau d'expression ex *vivo* du marqueur #7 (CD32) à la surface des cellules TCD4 de patients infectés par le VIH-1 et viro-supprimés (B) en comparaison de donneurs sains (A).
   La figure 14A représente le pourcentage de cellules CD32+ sur le total des cellules TCD4.
   La figure 14A représente le pourcentage de cellules CD32+ sur le total des cellules TCD4 immatures DR+.
Les **figures 15A à 15** **C** montrent les résultats d'expression du marqueur CD32 chez un premier patient.
   La figure 15A montre des résultats de cytométrie de flux montrant les différents niveaux d'expression ex vivo du marqueur #7 (CD32) sur les lymphoctytes TCD4 (sélection CD3+/CD4+) chez un patient avirémique (n=2), à l'aide d'un isotype. L'axe des abscisses représente le niveau de fluorescence du CD3 et l'axe des ordonnées ne niveau de fluorescence de CD32.
   La figure 15B montre des résultats de cytométrie de flux montrant les différents niveaux d'expression ex vivo du marqueur #7 (CD32) sur les lymphoctytes TCD4 (selection CD3+/CD4+) chez un patient avirémique (n=2), à l'aide d'un anticorps anti CD32. A: cellules exprimant fortement le CD32, B : cellules exprimant faiblement le CD32 et C : Cellules n'exprimant pas le CD32. L'axe des abscisses représente l'intensité de fluorescence du marqueur CD3, et l'axe des ordonnées représente l'intensité de fluorescence du marqueur CD32.
   La figure 15 C représente un histogramme montrant le nombre de copies d'ADN VIH-1 total par cellule quantifiées par qPCR (normalisation avec le gène de la béta-globine) sur les cellules : A : cellules CD4+ ; B : cellules CD4+ CD32-, C : CD4+ CD32 exprimé fortement.

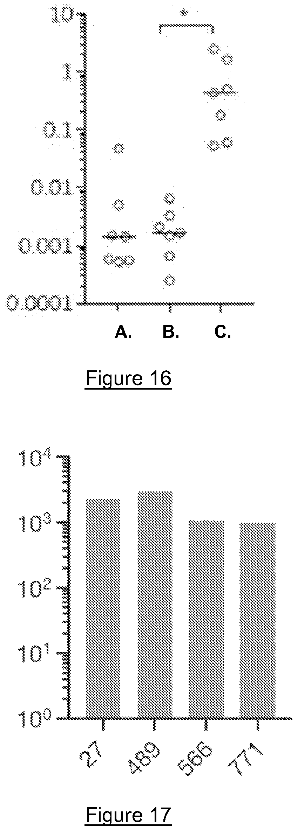

La **figure 16** est un graphique représentant le nombre de copie d'ADN VIH-1 par cellule, quantifié par qPCR, chez 7 patients avirémiques dans les copulations de cellules TCD4 totales (A), les populations TCD4 CD32- (B) et TCD4 CD32+ (C). Un test de Wilcoxon entre les fractions T CD4+ CD32- et CD32+ montre un enrichissement significatif en ADN VIH-1 dans la fraction CD32+ (p=0.0156).
La **Figure 17** est un histogramme montrant le logarithme des unités infectieuses par million des populations de cellules T CD4 CD32+ issues de 4 patients (27, 439, 566 et 771) après réactivation du virus HIV.

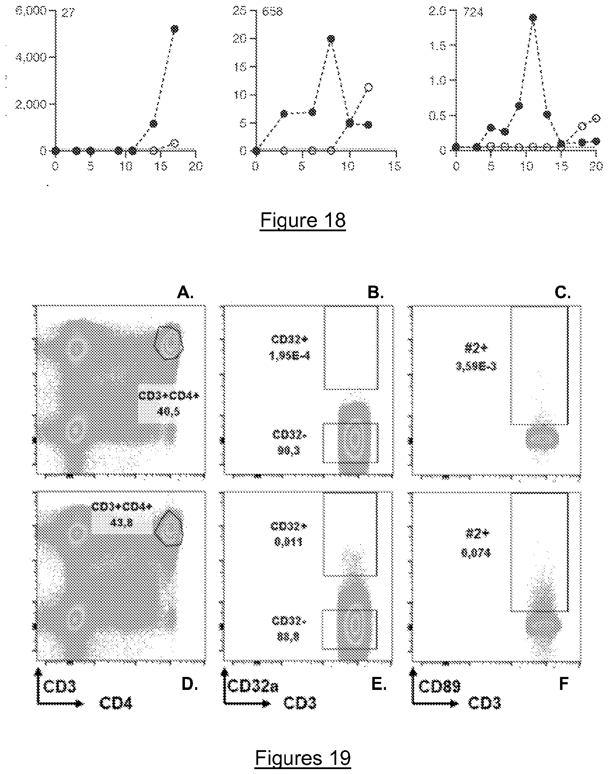

La **Figure 18** représente 3 courbes montrant la réactivation virale au cours du temps (exprimée en jours, axe des abscisses) à partir de populations de cellules T CD4 totales (ronds noirs) ou T CD4 déplétées en cellules CD32 (ronds gris) à partir de trois échantillons de patients différents et indépendants. L'axe des ordonnées représente la quantité de p24 en pg.mL⁻¹.
Les **Figures 19A à 19C** représentent des résultats de cytométrie de flux montrant les contrôles isotypiques (contrôles négatifs) des marquages de surface CD32 et CD89.
   La figure 19A représente des résultats de cytométrie de flux sur des cellules de patients et détectées avec les marqueurs CD3 et CD4. La population CD4+CD3+ est marquée par une fenêtre (40,5% de la population).
   La figure 19B représente des résultats de cytométrie de flux sur les cellules de la fenêtre de la figure 19A, et les marquages de contrôle isotypique pour le marqueur CD32, détectées à l'aide des marqueurs CD3 et isotype contrôle. Les cellules marquées CD3 et isotype sont détéctées pour leur positivité ou négativité à l'antigène CD32
   La figure 19C représente des résultats de cytométrie de flux sur les cellules de la fenêtre du bas (CD32-) de la figure 19B. Les cellules marquées CD3 et isotype sont détectées pour leur positivité ou négativité à l'antigène CD89.
Les **Figures 19D à 19F** représentent des résultats de cytométrie de flux montrant les marquages de surface CD32 et CD89.
   La figure 19D représente des résultats de cytométrie de flux sur des cellules de patients et détectées avec les marqueurs CD3 et CD4. La population CD4+CD3+ est marquée par une fenêtre (43,8% de la population).
   La figure 19E représente des résultats de cytométrie de flux sur les cellules de la fenêtre de la figure 19A, et détectées à l'aide des marqueurs CD3 et CD32. Les cellules CD32+ (fenêtre du haut) et CD32- (fenêtre du bas - 88,8%) sont indiquées.
   La figure 19F représente des résultats de cytométrie de flux sur les cellules de la fenêtre du bas (CD32-) de la figure 19B. Les cellules marquées CD3 et CD89 sont détectées pour leur positivité ou négativité à l'antigène CD89. La fenêtre représente les cellules CD89+ CD32- (0,074%).

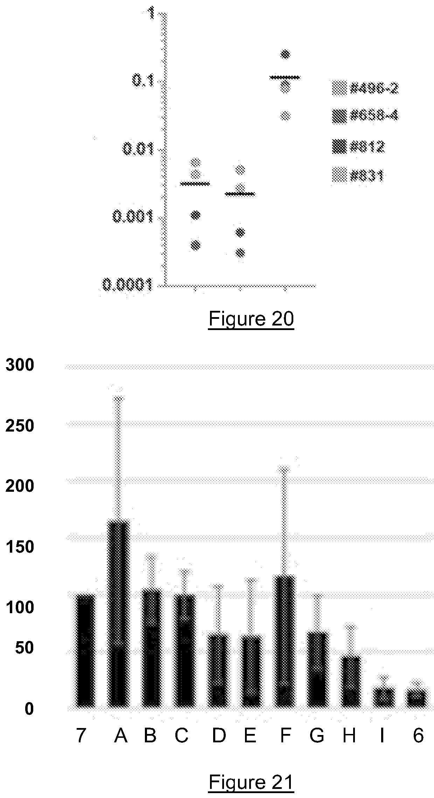

La **Figure 20** est un graphique représentant le nombre de copies d'ADN de VIH par cellule à partir de populations totales de T CD4 (A), de sous-population de cellules T CD4+CD32-CD89- (B) et de sous populations de cellules T CD4+CD32-CD89+(C) de 4 patients indépendants. La quantification des ADN viraux présents dans chacune de ces fractions a été réalisé par qPCR ADN VIH-1. Chez les patients testés, le marqueur CD89 identifie bien un réservoir de cellules infectées (médiane approximative de 0,1 copies d'ADN VIH-1 par cellule T CD4+ CD32a- CD89+)
La **Figure 21** est un histogramme montrant le rapport de marqueurs A, B, C, D, E (CD89), F, G, H et I par rapport au CD32 dans des cellules de patients GFP+ ayant subi une réactivation du VIH. La réactivation du virus est mesurée par RNAseq.

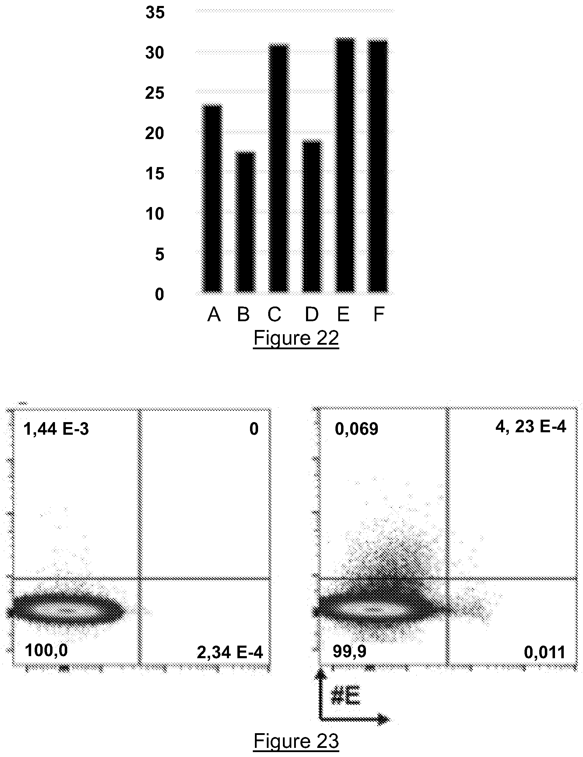

La **Figure 22** est un histogramme montrant le rapport de marqueurs A, B, C, D, E (CD89), dans ces cellules CD32- dans des cellules de patients GFP+ ayant subi une réactivation du VIH. Ces marqueurs sont mutuellement exclusifs avec CD32.
La **Figure 23** montre des résultats de cytométrie de flux de cellules T CD4 de patient (patient 812) marquées avec des anticorps CD32 et CD89 (image de droite bas) ou des isotypes (image de gauche) et la détection des cellules simplement (CD32 ou CD89) et doublement (CD32 et CD89) marquées. On note que les deux populations sont exclusives et qu'aucun double marquage n'est détecté.

### Exemples

### Exemple1 - identification de marqueurs spécifiques des cellules réservoirs

L'invention a pour objectif d'identifier et valider un marqueur spécifique des cellules infectées à l'aide de modèle *in vitro* et l'exploration phénotypique ex *vivo* des cellules primaires de patients infectés par le VIH-1 sous traitement antiviral (anti rétroviral) efficace. La mise en évidence in vitro et ex vivo d'une expression spécifique notamment du marqueur CD32 à la surface des cellules infectées peut être exploitée afin de cibler et d'éliminer le réservoir viral chez les patients infectés par le VIH-1, et ainsi proposer une thérapie efficace permettant d'éradiquer définitivement le virus chez les patients infectés.

### Matériel et méthodes :

### 1. Production virale et VLPs.

Les VLP contenant Vpx et les particules virales ont été produites en suivant le protocole standard de transfection phosphate calcium d'ADN dans les cellules 293T. Les VLP-Vpx ont été produits en co-transfectant 8µg de plasmide pSIV3+ et 2 µg de plasmide pMD2-G VSV-G. Le milieu de culture a été remplacé 16h post-transfection avant de récupérer les VLPs 48h plus tard, les centrifuger les filtrer sur filtre 0.45mm et les concentrer 100X par ultracentrifugation. Les particules virales VIH-1-CMV-eGFP ont été produites en co-transfectant 5µg du plasmide pHRET, 5µg de plasmide de packaging psPAX2 et 2µg de plasmide pMD2-G. Après concentration, le titre p24 du stock viral a été mesuré par ELISA et le titre infectieux (MOI) par titrage sur cellules 293T.

### 2.Infection et « sorting » des lymphocytes T CD4+ quiescents infectés in vitro.

Les cellules mononuclées du sang périphérique de donneurs sains ont été isolées par gradient de densité (Ficoll), puis cultivées en plaque 24 puits en présence de VLP-Vpx pendant 12h à une concentration de 2.10⁶ cellules/puit dans 300µL final de milieu complet (RPMI 10 %SVF). Les cellules ont ensuite été infectées par ajout de VIH-1-CMV-eGFP (1µg p24 équivalent à une MOI de 10X). Comme contrôle, des cellules ont été cultivées exclusivement en présence de VLP-Vpx, de VIH-1-CMV-egFP, ou ont été laissées non traitées. Trois jours post-infection, les cellules T CD4+ quiescentes (CD69- HLA-DR-) infectées (XH+), les cellules T CD4+ quiescentes traitées exclusivement avec du VIH-1-CMV-eGFP (XH-) et les contrôles (X ou NT) ont été isolées à l'aide d'un trieur. Les cellules triées ont été resuspendues dans du tampon RA1 complété en bétamercaptoéthanol et stockées à -80°C avant extraction de ARN totaux.

### 3.Séquençage des ARN totaux et analyse bio-informatique.

Les ARN totaux issus des fractions XH+, XH-, X et NT ont été extraits en utilisant le GE Healthcare Illustra RNA mini kit. La qualité des ARN a été analysée sur le Bio-analyseur 2100 d'Agilent et par RNA Nanochip. Une libraire Illumina a ensuite été constituée. Les échantillons ont été multiplexés avant séquençage. Une analyse en composantes principales des "regularized-log transformed gene expression counts" pour les différentes fractions.

### 4.Isolement des cellules mononuclées du sang périphérique des patients VIH-1.

Les cellules mononuclées du sang périphérique de patients VIH-1 traités efficacement (charge virale <20 copies d'ARN VIH-1/mL de sang) ont été isolées par gradient de densité (Ficoll).

### 5.Cytométrie de flux et « sorting » des sous-populations de lymphocytes T CD4+.

Les cellules issues des infections *in vitro,* du sang périphérique de donneurs sains et de patients VIH-1 ont été marquées à l'aide d'anticorps anti-CD3, anti-CD4, anti-CD32, anti-HLA-DR, anti-CD69 et analysées par FACS. Les cellules fraiches de patients VIH-1 ont été marquées à l'aide d'anticorps anti-CD3, anti-CD4, anti-CD32, anti-HLA-DR et d'un contrôle isotypique IgG2 afin d'être triées sur SH800 (Sony) en fonction de l'expression du marqueur CD32 (T CD4+ totaux ; T CD4+ CD32- ; T CD4+ CD32low ; T CD4+ CD32+). Pour chaque sous-population, une partie des cellules triées a été conservée à -80°C en culot sec pour une quantification de l'ADN VIH-1 total et une seconde partie a été mise en culture pour des tests d'inductibilté et d'amplification virale.

### 6. Quantification des ADN VIH-1 totaux.

Les ADN des différentes fractions isolées à partir de sang de patients VIH-1 ont été purifiés à l'aide du QIAamp DNA micro kit, Qiagen). La concentration en ADN a été déterminée par qPCR béta-globine (DNA control kit, Roche). Le nombre de copie d'ADN VIH-1 total par cellule a été déterminé par qPCR ultrasensible (Bicentric).

### Descriptif détaillé :

Afin d'identifier des candidats marqueurs, les inventeurs ont développé un modèle *in vitro* permettant pour la première fois d'infecter directement des lymphocytes TCD4 quiescentes issues de donneurs sains. En effet, ces cellules ne sont pas permissives à l'infection par le VIH-1 sans signal d'activation préalable (activation via le TCR ou PHA/IL2). L'identification par les inventeurs de la protéine SAMHD1, responsable de la restriction dans ces cellules, leur a permis de développer un traitement par des VLP contenant la protéine Vpx (codée par le virus SIVmac251) permettant la levée de restriction et l'infection directe, sans recours à un signal d'activation. A l'aide de ce modèle **(****Figure 1****)** où des PBMC de donneurs sains ont été traités par VLP-Vpx puis infectés par un VIH-1-CMV-GFP, les ARN totaux des cellules infectées (GFP+) ont été extraits afin de réaliser des expériences de RNA-seq sur les ARN messagers. Des PBMC traitées par VLP-Vpx mais non-infectées ainsi que des cellules non infectées ont été utilisées comme contrôle. Une analyse statistique et bio-informatique a permis de déterminer l'impact de l'infection par le VIH-1 sur le programme transcriptionnel des lymphocytes TCD4 quiescents.

En effet, les analyses en composante principale PCA **(****Figure 2****)** et le regroupement hiérarchique HC **(****Figure 3****)** ont été réalisées à partir des résultats RNA-seq. Les deux analyses PCA et HC effectuées sur quatre donneurs ont permis de mettre en évidence que les cellules traités VLP-Vpx et infectés (VLP-Vpx GFP+) formaient un cluster distinct des cellules contrôles (VLP-Vpx GFP-, VLP-Vpx seul et non infectés). Ces résultats indiquent que les cellules VLP-Vpx GFP+ de différents donneurs partagent une signature qui les distingue des autres populations. Lors de l'infection, le VIH-1 module donc l'expression génique des lymphocytes TCD4 quiescents et pourrait se traduire par un profil phénotypique les distinguant des cellules non infectées.

Les inventeurs se sont donc intéressés aux gènes exprimés de façon différentielle (gènes DE) et notamment aux gènes up-régulés lors de l'infection latente **(****Figures 4 et 5**). Ainsi, 22 candidats marqueurs correspondant à des protéines de surface ont été sélectionnés pour validation *in vitro.* En utilisant le modèle *in vitro* sur des PBMC de donneurs sains, l'expression de 22 marqueurs a été testée par analyse FACS **(****Figure 6****)** sur les lymphocytes TCD4 quiescent infectés. Les résultats sont présentés en pourcentage d'expression des marqueurs (#1 à #22) sur les cellules infectées GFP+ et non infectées (GFP-). L'analyse a mis en lumière une expression spécifique du marqueur CD32 (marqueur #7) sur les cellules infectées en comparaison des cellules contrôle **(****Figures 7 et 8****).** L'indentification d'un candidat marqueur s'est donc focalisée sur le marqueur CD32.

La **figure 21** montre que 9 autres marqueurs sont également exprimés à la surface des cellules T CD4 de patients infectés et qui sont susceptible de rebond viral.

La poursuite de l'analyse sur de nouveaux donneurs a permis de confirmer l'induction de l'expression spécifique du marqueur CD32 dans les cellules infectées de manière latente avec enrichissement dans le niveau d'expression de ce marqueur dans les population GFPhigh **(****Figures 9A et 9B et 10A et 10B****).** Les inventeurs ont également montré que l'induction du marqueur CD32 est limitée à la population quiescente. En effet, des cellules stimulées par l'intermédiaire de la voie TCR (anti-CD3/anti-CD28) n'expriment pas le marqueur CD32 **(****Figures 11A et 11B****).** L'utilisation d'un virus pseudotypé par l'enveloppe du virus VSV a permis de montrer que l'induction de ce marqueur sur d'autres populations infectées telles que les CD8 **(****Figure 12****).** De plus, nous avons déterminé si les lentivirus HIV2 et SIV induisent l'expression de CD32 comme le VIH-1. Les **figures 13A et 13B** montrent que SIV ainsi que HIV2 sont capables d'induire l'expression de CD32 à la surface des cellules T CD4 quiescentes infectées en présence de Vpx.

Après avoir validé le marqueur CD32 in vitro, les inventeurs ont cherché à établir sa relevance ex *vivo.* Ainsi, une analyse phénotypique par FACS du niveau d'expression de ce marqueur sur les lymphocytes TCD4 a été réalisée sur les PBMC de patients infectés et traité efficacement avec des antiviraux en comparaison à des donneurs sains. Nous avons pu mettre en évidence une expression significativement plus élevée du CD32 chez les patients **(****Figure 14A et 14B****).**

Enfin, les inventeurs ont exploré le niveau d'enrichissement en ADN-VIH-1 dans les différents sous-groupes de lymphocytes TCD4 exprimant différentiellement le marqueur CD32 (TCD4 totaux, TCD4 CD32-, TCD4 CD32low et TCD4 CD32+) de deux patients viro-supprimés. Après tri de ces différentes populations **(****Figures 15A à 15C****),** les ADN génomiques ont été extraits et les ADN VIH-1 totaux ont été quantifiés par qPCR dans les différentes fractions. Les résultats obtenus chez les deux patients montrent un fort enrichissement en ADN VIH-1 dans les fractions TCD4 exprimant le marqueur CD32 en comparaison avec les TCD4 n'exprimant pas ce marqueur **(****Figure 16****).**

L'ensemble de ces résultats *in vitro* et ex *vivo* ont ainsi permis de valider le CD32 comme marqueur spécifique des cellules infectées par le VIH-1. L'identification du CD32 permet d'établir de nouvelles stratégies visant à cibler directement des cellules infectées par le VIH-1 et de permettre la purge du réservoir viral et la guérison du SIDA.

### Exemple 2

Les sous-populations cellulaires isolées à partir du sang de patients VIH-1 ont été mise en culture en absence ou en présence d'agents activateurs tels que PHA ou des billes anti-CD3, anti-CD28 et anti-CD2 (Miltenyi) en présence d'IL2 (50 UI/mL). Les fractions T CD4⁺ et T CD4⁺ CD32⁻ ont été cultivées en plaque de 24 puits à la concentration de 10⁶ cellules par mL de milieu complet et les surnageant récupérés tous les 2 jours pour test ELISA p24.

Les résultats sont présentés dans le tableau suivant et à la **figure 17** :

| Patient | Unités infectieuses par million (IUPM) de cellules CD4 (95% IC) | IUPM de cellules CD4 CD32a+ (95% IC) |
|---|---|---|
| 27 | 2,2 (0,51 à 9,44) | 4977 (533 à 46400) |
| 489 | 5,5 (1,33 à 23,01) | 16422 (1841 à 146000) |
| 566 | 2,2 (0,51 à 9,44) | 2326 (249 à 21700) |
| 771 | 2,2 (0,51 à 9,44) | 2158 (231 à 20100) |

Cette première expérience a pour but de mettre en évidence que la production de nouvelles particules virales à partir des lymphocytes T CD4+ totaux contenant la fraction CD32+ est plus faible que celle de cellules T CD4+ CD32+, ce qui montre que les réservoirs viraux sont des cellules CD32a+. Les résultats obtenus montrent un enrichissement de 3000x du nombre d'IUPM dans les cellules T CD4+ CD32a+ par rapport aux lymphocytes T CD4+ totaux.

Dans une seconde expérience, des lymphocytes T CD4+ isolés à partir du sang de 3 patients ont été activés polyclonalement (anti-CD3/antiCD28 plus IL2) *in vitro* comme contrôle de production virale. En parallèle et pour ces mêmes patients, des lymphocytes T CD4+ déplétées en cellules exprimant le marqueur CD32a ont également été isolées puis activées dans les mêmes conditions in vitro.

Les fractions T CD4+ CD32low et CD32+ ont été cultivées en plaque de 96 puits à fond rond avant que les surnageants de culture ne soient prélevés tous les 3 jours pour être ajouter sur 2000 cellules MT4C5 pour un test d'amplification virale par ELISA p24 ultrasensible SIMOA. Cette seconde expérience vise à démontrer que le virus dont la production a été induite par activation des fractions T CD4+ CD32+ et T CD4+ CD32low est capable d'établir une infection productive en co-culture.

Cette expérience a pour but de mettre en évidence qu'il est possible d'induire la production de nouvelles particules virales à partir des lymphocytes T CD4+ totaux contenant la fraction CD32+ à l'inverse de cellules T CD4+ déplétée en cellules CD32+.

Les résultats sont présentés à la **figure 18****.**

Il résulte de la comparaison des cinétiques de réplication virale entre les cellules T CD4+ (ronds noirs) totales et les cellules T CD4+ CD32a- (ronds gris), que la déplétion des cellules T CD4+ CD32a+ entraine un retard considérable dans la réplication virale. Ces résultats confirment que les cellules T CD4+ CD32a+ contribuent de façon significative au réservoir total des cellules infectées et compétentes pour la réplication virale.

### Exemple 3

Dans cet exemple, les inventeurs ont tenté de comprendre comment les populations de cellules T CD4 issues de patents, et déplétées en CD32, peuvent toujours réactiver le virus VIH après activation.

D'autres marqueurs décrits précédemment ont été testés, et ont identifié d'autres réservoirs que ceux exprimant le CD32.

Notamment les inventeurs ont identifié que des populations de cellules T CD4 CD89+ constituaient des réservoirs viraux. Le même protocole que celui de l'exemple 1 a été utilisé.

Chez 4 patients, les marquages à l'aide d'anticorps spécifiques ont permis de mettre en évidence l'expression de CD89 à la surface des lymphocytes T CD4+. Pour démontrer que l'expression du marqueur CD89 à la surface de ces cellules était liée à l'infection de celles-ci, les lymphocytes T CD4+ totaux mais aussi les cellules T CD4+ CD32a- CD89-et T CD4+ CD32a- CD89+ ont été isolées à partir de ces mêmes patients comme décrit précédemment (stratégie de fenêtrage pour un patient représentatif). Les résultats sont présentés aux **Figures 19** **A à 19F.**

Suivant le même protocole qu'à l'exemple 1 la quantification des ADN viraux présents dans chacune de ces fractions a été réalisé par qPCR ADN VIH-1 **(****Figure 20****).** Chez les patients testés, le marqueur CD89 identifie bien un réservoir de cellules infectées (médiane approximative de 0,1 copies d'ADN VIH-1 par cellule T CD4+ CD32a- CD89+).

### Exemple 4

Pour poursuivre l'étude, les inventeurs ont enfin testé si les différents candidats marqueurs étaient coexprimés sur les cellules identifiées comme étant des réservoirs viraux.

Comme le montre la **Figure 22****,** 6 marqueurs, dont le marqueur CD89 sont exprimés à la surface des cellules T CD4 qui n'expriment pas de CD32, ces marqueurs représentant de 17 à 30 % des cellules infectés par le VIH et donc susceptibles de le réactiver.

Afin d'identifier si le marqueur CD89 constitue un autre réservoir que celui identifié pour CD32, les inventeurs ont testé sur des populations cellules de patients la coexpression de CD32 et de CD89.

Les résultats sont présentés à la **Figure 23**.

Ces résultats montrent que les deux marqueurs CD32 et CD89 sont mutuellement exclusifs, et identifient chacun un réservoir distinct.

### Exemple 5

Les anticorps de la présente invention, dirigés contre CD32 peuvent être produits par diverses techniques connues de l'homme du métier, notamment celles décrites ci- après.

Des souris Balb/c sont immunisées avec la protéine humaine entière CD32 ou le fragment extracellulaire fusionné au domaine Fc des immunoglobulines humaines. Les souris sont injectées par administration sous-cutanée avec 10µg de la protéine ou du fragment au jour 0, jour 14 et jour 28 en présence d'adjuvant complet de Freund (première injection) ou adjuvant incomplet (deuxième et troisième injections). Les splénocytes de la souris sont fusionnés avec des cellules de myélomes murins (PX63.Ag8.653; ATCC, Rockville, MD) selon le protocole précédemment décrit (Salhi et al. Biochem. J. 2004). Les cellules sont cultivées en plaques de culture (10⁵ cellules par puits) dans un milieu HAT permettant la sélection des hybridomes. Après 12 jours, les surnageants sont récupérés et testés pour leur liaison à CD32 par ELISA. Les cellules sont alors soumises à une étape de sous-clonage par dilution limite, les clones positifs sont ensuite soumis à un deuxième cycle de sous-clonage par dilution limite afin d'isoler après ELISA les clones purifiés présentant l'affinité la plus élevée. Ces clones sont ensuite cultivés à plus grande échelle pour produire les anticorps *in vitro.* Les surnageants sont ensuite purifiés sur colonne de chromatographie d'affinité protéine G.

La technique du phage display utilisant des vecteurs modifiés tels que décrits dans le document WO 2007/074496 ou la sélection sur phage display suivie de celle de Biopanning (Krebber et al, (1997) ; WO 2006/117699) est également une autre alternative à l'obtention d'anticorps de haute affinité dirigés contre CD32.

Le séquençage des hybridomes sélectionnés ou la séquence obtenue déjà connue dans les phages sélectionnés permettent ensuite de cloner les régions variables ou plus particulièrement les CDRs responsables de la liaison spécifique à l'épitope dans un plasmide permettant, après transfection dans des cellules productrices comme des CHO, la production et l'obtention d'anticorps chimériques, humanisés ou humains.

Des anticorps anti-CD3 sont obtenus en procédant à travers les mêmes étapes. La création des anticorps humanisés ou humains monospécifiques anti-CD32 ou bispécifiques anti-CD32/CD3 consiste ensuite à utiliser les séquences obtenues précédemment (hybridomes ayant la meilleure affinité pour l'antigène correspondant (CD32 et CD3 respectivement) ou bactériophages). Dans le cas où les anticorps proviennent d'hybridomes, les CDR sont modifiés par mutagenèse de manière à optimiser les acides aminés importants pour la reconnaissance de l'antigène et les acides aminés charpentes permettant un bon repliement des CDR. L'étape d'humanisation consiste à comparer les séquences issues de l'hybridome murin avec une banque de données de séquences d'anticorps humains « Kabat data base ». Les acides aminés potentiellement immunogènes du fait de leur nature murine sont ensuite modifiés. Après l'humanisation, si les anticorps avaient été obtenus par hybridomes, ou dès obtention de la séquence dans le cas de la technique du phage display, les séquences codantes pour les régions variables lourdes (VH) et légères (VL) humanisées/humaines dirigées contre les deux antigènes (CD32 et CD3) sont clonées en fusion dans un vecteur d'expression eucaryote pour permettre la production en cellules CHO.

L'invention n'est pas limitée aux modes de réalisation présentés et d'autres modes de réalisation apparaîtront clairement à l'homme du métier.

## Revendications

1. Méthode de détection de cellules réservoirs de cellules infectées par un virus de l'immunodéficience chez les mammifères, ladite méthode comprenant une étape de mise en contact, *in vitro* ou ex *vivo,* de cellules d'un mammifère infectées par ledit virus de l'immunodéficience chez les mammifères, et notamment traitées avec une thérapie antirétrovirale spécifique dudit virus de l'immunodéficience chez les mammifères, avec un agent permettant la détection des cellules lymphocytaires exprimant à leur surface le marqueur de différentiation CD89.

2. Méthode selon la revendication 1, dans laquelle ledit agent permettant la détection des cellules lymphocytaires exprimant à sa surface le marqueur de différentiation CD89 est un anticorps anti-CD89.

3. Méthode selon la revendication 1 ou 2, dans laquelle ledit virus de l'immunodéficience chez les mammifères est le virus de l'immunodéficience humaine VIH, de l'immunodéficience simienne VIS ou de l'immunodéficience féline VIF.

4. Méthode selon l'une quelconque des revendications 1 à 3, dans laquelle les cellules lymphocytaires exprimant à leur surface le marqueur de différentiation CD89 sont des cellules lymphocytaires TCD4, notamment des cellules TCD4 quiescentes.

5. Kit de détection de cellules réservoirs d'un virus de l'immunodéficience chez les mammifères comprenant au moins un agent détectant le marqueur de différentiation CD89 à la surface des cellules et au moins une composition permettant de déterminer la présence d'ADN dudit virus de l'immunodéficience chez les mammifères dans le génome desdites cellules.

6. Kit selon la revendication 5, dans lequel ladite composition permettant de déterminer la présence d'ADN dudit virus de l'immunodéficience chez les mammifères dans le génome desdites cellules comprend des oligonucléotides spécifiques de séquences du génome dudit virus, notamment pour la mise en œuvre d'une réaction de polymérisation en chaine.

7. Méthode de pronostic *in vitro* de la rechute d'une immunodéficience chez les mammifères suite à l'arrêt d'un traitement avec des agents thérapeutiques anti-virus responsable de ladite immunodéficience chez les mammifères, ladite méthode comprenant une étape de quantification des cellules lymphocytaires exprimant à leur surface le marqueur CD89 et présentant dans leur génome de l'ADN d'un virus responsable de ladite immunodéficience chez les mammifères.

8. Méthode de diagnostic, *in vitro,* de la rémission complète d'une immunodéficience chez les mammifères après l'arrêt d'un traitement avec des agents thérapeutiques anti-virus responsable de ladite immunodéficience chez les mammifères, ladite méthode comprenant une étape de détection de l'absence de cellules lymphocytaires présentant dans leur génome de l'ADN d'un virus de l'immunodéficience chez les mammifères et exprimant à leur surface au moins le marqueur CD89.

9. Méthode de détection de cellules réservoirs de cellules infectées par un virus de l'immunodéficience chez les mammifères, ladite méthode comprenant une étape de mise en contact, *in vitro* ou ex *vivo,* de cellules d'un mammifère infectées par ledit virus de l'immunodéficience chez les mammifères, et notamment traitées avec une thérapie antirétrovirale spécifique dudit virus de l'immunodéficience chez les mammifères et/ou une thérapie visant à éradiquer lesdites cellules réservoirs, avec un agent permettant la détection des cellules lymphocytaires exprimant à leur surface le marqueur de différentiation CD89.

10. Méthode d'évaluation de l'efficacité d'un traitement visant à éradiquer des cellules réservoirs de cellules d'un mammifère infectées par ledit virus de l'immunodéficience chez les mammifères, ladite méthode comprenant une étape de détection in vitro ou ex vivo desdites cellules de réservoir avec un agent permettant la détection de la présente la quantité ou l'absence de cellules lymphocytaires exprimant à leur surface le marqueur de différentiation CD89,
ladite méthode comprenant avantageusement une étape de classification desdits mammifères infectés selon la présence, l'absence ou la quantité de cellules réservoir détectées

11. Composition comprenant au moins un agent reconnaissant les cellules lymphocytaires exprimant le marqueur CD89, notamment un anticorps monospécifique du marqueur CD89, ou un anticorps multispécifique reconnaissant au moins un épitope du marqueur CD89 et au moins un marqueur caractéristique des cellules lymphocytaires, notamment les cellules lymphocytaires T, pour son utilisation pour l'éradication de cellules réservoirs de cellules infectées par un virus de l'immunodéficience chez les mammifères.

12. Anticorps
- reconnaissant spécifiquement le marqueur CD89 exprimé sur les cellules lymphocytaires réservoir des lentivirus, éventuellement couplé à un agent cytotoxique, ou
- au moins bispécifique reconnaissant outre le marqueur CD89 et au moins un autre marqueur spécifique des cellules lymphocytaires, notamment le marqueur CD3
pour son utilisation dans le cadre du traitement d'une infection par un lentivirus, notamment une infection liée au VIH, au VIS ou au VIF.

## Patentansprüche

1. Verfahren zum Nachweis von Zellen, die ein Reservoir von Zellen darstellen, die mit einem Virus der Immunschwäche bei Säugetieren infiziert sind, wobei das Verfahren einen Schritt umfasst, in dem Zellen eines Säugetiers, das mit dem Virus der Immunschwäche bei Säugetieren infiziert ist, wobei sie insbesondere mittels einer antiretroviralen Therapie behandelt wurden, die spezifisch für die Immunschwäche bei Säugetieren ist, *in vitro* oder ex *vivo* mit einem Mittel in Kontakt gebracht werden, das einen Nachweis von lymphozytenartigen Zellen ermöglicht, die auf ihrer Oberfläche den Zelltypmarker CD89 exprimieren.

2. Verfahren nach Anspruch 1, wobei es sich bei dem Mittel, das einen Nachweis von lymphozytenartigen Zellen ermöglicht, die auf ihrer Oberfläche den Zelltypmarker CD89 exprimieren, um einen anti-CD89-Antikörper handelt.

3. Verfahren nach Anspruch 1 oder 2, wobei es sich bei dem Virus der Immunschwäche bei Säuretieren um das Virus der menschlichen Immunschwäche HIV, der simianen Immunschwäche SIV oder der felinen Immunschwäche FIV handelt.

4. Verfahren nach einem beliebigen der Ansprüche 1 bis 3, wobei es sich bei den lymphozytenartigen Zellen, die auf ihrer Oberfläche den Zelltypmarker CD89 exprimieren, um lymphozytenartige TCD4-Zellen, insbesondere um ruhende TCD4-Zellen handelt.

5. Kit zum Nachweis von Zellen, die ein Reservoir eines Virus der Immunschwäche bei Säugetieren darstellen, wobei es mindestens ein Mittel zum Nachweis des Zelltypmarkers CD89 auf der Oberfläche der Zellen und mindestens eine Zusammensetzung umfasst, die es ermöglicht zu bestimmen, ob DNA des Virus der Immunschwäche bei Säugetieren im Genom der Zellen vorliegt.

6. Kit nach Anspruch 5, wobei die Zusammensetzung, die es ermöglicht zu bestimmen, ob DNA des Virus der Immunschwäche bei Säugetieren im Genom der Zellen vorliegt, Oligonukleotide umfasst, die spezifisch für Sequenzen des Genoms des Virus sind, insbesondere zur Durchführung einer Polymerisationskettenreaktion.

7. Verfahren zur in-vitro-Prognose für Rückfälle einer Immunschwäche bei Säugetieren, nachdem die Behandlung mit therapeutischen Mitteln eingestellt wurde, die sich gegen das Virus richten, das für die Immunschwäche bei Säugetieren verantwortlich ist, wobei das Verfahren einen Schritt des Quantifizierens von lymphozytenartigen Zellen umfasst, die auf ihrer Oberfläche den Marker CD89 exprimieren und in deren Genom DNA eines Virus vorliegt, das für die Immunschwäche bei Säugetieren verantwortlich ist.

8. Verfahren zur in-vitro-Diagnose für die vollständige Remission einer Immunschwäche bei Säugetieren, nachdem die Behandlung mit therapeutischen Mitteln eingestellt wurde, die sich gegen das Virus richten, das für die Immunschwäche bei Säugetieren verantwortlich ist, wobei das Verfahren einen Schritt des Nachweises der Abwesenheit von lymphozytenartigen Zellen umfasst, in deren Genom DNA eines Virus der Immunschwäche bei Säugetieren vorliegt und die auf ihrer Oberfläche zumindest den Marker CD89 exprimieren.

9. Verfahren zum Nachweis von Zellen, die ein Reservoir von Zellen darstellen, die mit einem Virus der Immunschwäche bei Säugetieren infiziert sind, wobei das Verfahren einen Schritt umfasst, in dem Zellen eines Säugetiers, das mit dem Virus der Immunschwäche bei Säugetieren infiziert ist, wobei sie insbesondere mittels einer antiretroviralen Therapie, die spezifisch für die Immunschwäche bei Säugetieren ist, und/oder einer Therapie behandelt wurden, die darauf abzielt, die Reservoir-Zellen zu auszumerzen, *in vitro* oder ex *vivo* mit einem Mittel in Kontakt gebracht werden, das einen Nachweis von lymphozytenartigen Zellen ermöglicht, die auf ihrer Oberfläche den Zelltypmarker CD89 exprimieren.

10. Verfahren zur Untersuchung der Wirksamkeit einer Behandlung, die darauf abzielt, Zellen ausmerzen, die ein Reservoir von Zellen darstellen, die mit einem Virus der Immunschwäche bei Säugetieren infiziert sind, wobei das Verfahren einen Schritt umfasst, in dem die Reservoir-Zellen *in vitro* oder ex *vivo* mit einem Mittel nachgewiesen werden, das es ermöglicht, die Menge oder die Abwesenheit von lymphozytenartigen Zellen nachzuweisen, die auf ihrer Oberfläche den Zelltypmarker CD89 exprimieren, wobei das Verfahren vorteilhafterweise einen Schritt des Einstufens der infizierten Säugetiere in Abhängigkeit vom nachgewiesenen Vorliegen, von der nachgewiesenen Abwesenheit oder von der nachgewiesenen Menge der Reservoir-Zellen umfasst.

11. Zusammensetzung, die mindestens ein Mittel umfasst, das die lymphozytenartigen Zellen erkennt, die den Marker CD89 exprimieren, insbesondere einen monospezifischen Antikörper des Markers CD89 oder einen multispezifischen Antikörper, der mindestens ein Epitop des Markers CD89 erkennt, und mindestens einen Marker, der charakteristisch für lymphozytenartigen Zellen, insbesondere für lymphozytenartige T-Zellen ist, um sie dazu zu verwenden, Zellen auszumerzen, die ein Reservoir von Zellen darstellen, die mit einem Virus der Immunschwäche bei Säugetieren infiziert sind.

12. Antikörper
- der auf spezifische Weise den Marker CD89 erkennt, der auf den lymphozytenartigen Zellen exprimiert wird, die ein Reservoir von Lentiviren darstellt, wobei er möglicherweise an ein zytotoxisches Mittel gekoppelt ist, oder
- der mindestens bispezifisch ist, wobei er neben dem Marker CD89 mindestens einen weiteren Marker erkennt, der spezifisch für lymphozytenartige Zellen ist, insbesondere den Marker CD3,
um ihn im Rahmen der Behandlung einer Infektion mit einem Lentivirus, insbesondere einer Infektion in Verbindung mit dem HIV, dem SIV oder dem FIV, zu verwenden.

## Claims

1. A method for the detection of cellular reservoirs of cells infected with a mammalian immunodeficiency virus, said method comprising a step of bringing, *in vitro* or *ex vivo,* cells of a mammal infected with said mammalian immunodeficiency virus and in particular treated with a specific anti-retroviral therapy for said mammalian immunodeficiency virus into contact with an agent allowing the detection of lymphocyte cells expressing the CD89 differentiation marker on their surface.

2. The method according to claim 1, wherein said agent that makes it possible to detect lymphocyte cells expressing the differentiation marker CD89 on their surface is an anti-CD89 antibody.

3. The method according to claim 1 or claim 2, wherein said mammalian immunodeficiency virus is the human immunodeficiency virus, HIV, simian immunodeficiency virus, SIV, or feline immunodeficiency virus, FIV.

4. The method according to any of claims 1 to 3, wherein the lymphocyte cells expressing the differentiation marker CD89 on their surface are TCD4 lymphocyte cells, in particular quiescent TCD4 cells.

5. A kit for detecting cellular reservoirs of a mammalian immunodeficiency virus, comprising at least one agent detecting the CD89 differentiation marker on the surface of the cells and at least one composition making it possible to determine the presence of DNA of said mammalian immunodeficiency virus in the genome of said cells.

6. The kit according to claim 5, wherein said composition that makes it possible to determine the presence of DNA of said mammalian immunodeficiency virus in the genome of said cells comprises specific oligonucleotides of sequences of the genome of said virus, in particular for the implementation of a polymerase chain reaction.

7. A method for making a prognosis in vitro on the relapse of a mammalian immunodeficiency following the cessation of treatment with antiviral therapeutic agents responsible for said mammalian immunodeficiency, said method comprising a step of quantifying the lymphocyte cells expressing on their surface the CD89 marker, and having in their genome DNA of a virus responsible for said mammalian immunodeficiency.

8. A method for making a diagnosis, in vitro, on the complete remission of mammalian immunodeficiency following the cessation of treatment with antiviral therapeutic agents responsible for said mammalian immunodeficiency, said method comprising a step of detecting the absence of lymphocyte cells having in their genome DNA of a mammalian immunodeficiency virus and expressing on their surface at least the CD89 marker.

9. A method for the detection of cellular reservoirs of cells infected with a mammalian immunodeficiency virus, said method comprising a step of bringing, in vitro or ex vivo, cells of a mammal infected with said mammalian immunodeficiency virus and in particular treated with a specific anti-retroviral therapy for said mammalian immunodeficiency virus and/or a therapy aiming to eradicate said cellular reservoirs into contact with an agent allowing the detection of the lymphocyte cells expressing the CD89 differentiation marker on their surface.

10. A method for the evaluation of the efficacy of a treatment aiming to eradicate cellular reservoirs of mammalian cells infected with said mammalian immunodeficiency virus, said method comprising a step of detecting, in vitro or ex vivo, said cellular reservoirs using an agent allowing the detection of the presence, quantity or absence of lymphocyte cells expressing the CD89 differentiation marker on their surface.

11. A composition comprising at least one agent that recognizes the lymphocyte cells expressing the CD89 marker, in particular a monospecific antibody of the CD89 marker, or a multi-specific antibody that recognizes at least one epitope of the CD89 marker and at least one marker characteristic of the lymphocyte cells, in particular the T lymphocyte cells, for its use in eradicating cellular reservoirs of cells infected by a mammalian immunodeficiency virus.

12. An antibody that
- specifically recognizes the CD89 marker expressed on the lymphocyte cellular reservoirs of lentiviruses, potentially coupled to a cytotoxic agent, or
- an antibody that is at least bispecific and recognizes further to CD89 marker a marker that is specific of lymphocytes cells, in particular the CD3 marker,
for its use in the treatment of an infection by a lentivirus, in particular an infection linked to HIV, SIV or FIV.
